# EUROPÄISCHE PATENTSCHRIFT

(11) **EP 2 298 769 B1**
(45) Veröffentlichungstag und Bekanntmachung des Hinweises auf die Patenterteilung: **30.10.2013**
(21) Anmeldenummer: 10180922.6
(22) Anmeldetag: 21.02.2002
(51) Int. Cl.: C07D 473/04, C07D 473/06, C07D 473/08, C07D 473/10, C07D 473/12

(54) **Xanthinderivate, deren Herstellung und deren Verwendung als Arzneimittel**
Xanthin derivatives, their production and utilisation as medicine
Dérivé de xanthine, sa fabrication et son utilisation en tant que médicament

(30) Priorität: 24.02.2001 DE 10109021; 10.04.2001 DE 10117803; 17.08.2001 DE 10140345; 30.01.2002 DE 10203486
(43) Veröffentlichungstag der Anmeldung: 23.03.2011
(62) Teilanmeldung aus: 08154072.6
(73) Patentinhaber: Boehringer Ingelheim Pharma GmbH & Co. KG, 55216 Ingelheim am Rhein (DE)
(72) Erfinder: Himmelsbach, Frank, 88441 MITTELBIBERACH (DE); Mark, Michael, 88400 BIBERACH (DE); Eckhardt, Matthias, 88400 BIBERACH (DE); Langkopf, Elke, 88447 WARTHAUSEN (DE); Maier, Roland, 88400 BIBERACH (DE); Lotz, Ralf, 88433 SCHEMMERHOFEN (DE)
(74) Vertreter: Simon, Elke Anna Maria

(56) Entgegenhaltungen:
- US-A- 5 223 499
- US-A- 5 753 635

## Beschreibung

Gegenstand der vorliegenden Erfindung sind Verbindungen der allgemeinen Formel 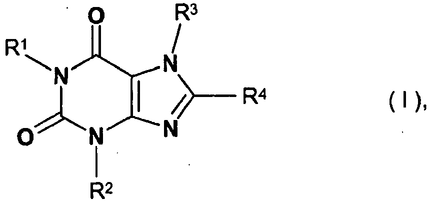 in der
R¹ eine C₁₋₈-Alkylgruppe,
eine C₃₋₈-Alkenylgruppe,
eine C₃₋₄-Alkenylgruppe, die durch eine C₁₋₂-Alkyloxy-carbonyl-, Aminocarbonyl-, C₁-₃-Alkylamino-carbonyl-, Di-(C₁₋₃-alkyl)-amino-carbonyl-, Pyrrolidin-1-ylcarbonyl-, Piperidin-1-ylcarbonyl- oder Morpholin-4-ylcarbonyl-gruppe substituiert ist,
eine C₃₋₈-Alkinylgruppe,
eine durch eine Gruppe Rₐ substituierte C₁₋₆-Alkylgruppe, wobei
   Rₐ eine C₃₋₇-Cycloalkyl-, Heteroaryl-, Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-yl-carbonyl-, Piperazin-1-ylcarbonyl-, 4-Methylpiperazin-1-ylcarbonyl- oder 4-Ethylpiperazin-1-ylcarbonylgruppe bedeutet,
eine durch eine Phenylgruppe substituierte C₁₋₆-Alkylgruppe, wobei der Phenylring durch die Gruppen R¹⁰ bis R¹⁴ substituiert ist und
   R¹⁰ ein Wasserstoffatom,
   ein Fluor-, Chlor-, Brom- oder lodatom,
   eine C₁₋₄-Alkyl-, Hydroxy-, oder C₁₋₄-Alkyloxygruppe,
   eine Nitro-, Amino-, C₁₋₃-Alkylamino-, Di-(C₁₋₃-alkyl)amino-, Cyan-C₁₋₃-alkyl-amino-, [N-(Cyan-C₁₋₃-alkyl)-N-C₁₋₃-alkyl-amino]-, C₁₋₃-Alkyloxy-carbonyl-C₁₋₃-alkylamino-, Pyrrolidin-1-yl-, Piperidin-1-yl-, Morpholin-4-yl-, Piperazin-1-yl-, oder 4-(C₁₋₃-Alkyl)-piperazin-1-yl-Gruppe,
   eine C₁₋₃-Alkyl-carbonylamino-, Arylcarbonylamino-, Aryl-C₁₋₃-alkyl-carbonyl-amino-, C₁₋₃-Alkyloxy-carbonylamino-, Aminocarbonylamino-, C₁₋₃-Alkyl-aminocarbonylamino-, Di-(C₁₋₃-alkyl)aminocarbonylamino-, Pyrrolidin-1-yl-carbonylamino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonyl-amino-, C₁₋₃-Alkyl-sulfonylamino-, Bis-(C₁₋₃-alkylsulfonyl)-amino-, Amino-sulfonylamino-, C₁₋₃-Alkylamino-sulfonylamino-, Di-(C₁₋₃-alkyl)amino-sulfonyl-amino-, Pyrrolidin-1-yl-sulfonylamino-, Piperidin-1-yl-sulfonylamino-, Mor-pholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)thiocarbonylamino-, (C₁₋₃-Alkyloxy-carbonylamino)carbonylamino-, Arylsulfonylamino- oder Aryl-C₁₋₃-alkyl-sulfonylaminogruppe,
   eine N-(C₁₋₃-Alkyl)-C₁₋₃-alkyl-carbonylamino-, N-(C₁₋₃-Alkyl)-arylcarbonyl-amino-, N-(C₁₋₃-Alkyl)-aryl-C₁₋₃-alkyl-carbonylamino-, N-(C₁₋₃-Alkyl)-C₁₋₃-alkyloxy-carbonylamino-, N-(Aminocarbonyl)-C₁₋₃-alkylamino-, N-(C₁₋₃-Alkyl-aminocarbonyl)-C₁₋₃-alkylamino -, N-[Di-(C₁₋₃-alkyl)aminocarbonyl]-C₁₋₃-alkylamino-, N-(C₁₋₃-Alkyl)-C₁₋₃-alkyl-sulfonylamino-, N-(C₁₋₃-Alkyl)-aryl-sulfonylamino-, oder N-(C₁₋₃-Alkyl)-aryl-C₁₋₃-alkyl-sulfonylaminogruppe,
   eine 2-Oxo-imidazolidin-1-yl-, 2,4-Dioxo-imidazolidin-1-yl-, 2,5-Dioxo-imidazolidin-1-yl- oder 2-Oxo-hexahydropyrimidin-1-ylgruppe, in der das Stickstoffatom in 3-Stellung jeweils durch eine Methyl- oder Ethylgruppe substituiert sein kann,
   eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl-oder 4-(C₁₋₃-Alkyl)-piperazin-1-yl-carbonylgruppe,
   eine C₁₋₃-Alkyl-carbonyl- oder eine Arylcarbonylgruppe,
   eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1-yl-carbonyl-C₁₋₃-alkyl- oder 4-(C₁₋₃-Alkyl)-piperazin-1-yl-carbonyl-C₁₋₃-alkyl-gruppe,
   eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy-, C₁₋₃-Alkyl-aminocarbonyl-C₁₋₃-alkyloxy-, Di-(C₁₋₃-alkyl)-aminocarbonyl-C₁₋₃-alkyloxy-, Pyrrolidin-1-yl-carbonyl-C₁₋₃-alkyl-oxy-, Piperidin-1-yl-carbonyl-C₁₋₃-alkyloxy-, Morpholin-4-yl-carbonyl-C₁₋₃-alkyl-oxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
   eine Hydroxy-C₁₋₃-alkyl-, C₁₋₃-Alkyloxy-C₁₋₃-alkyl-, Amino-C₁₋₃-alkyl-, C₁₋₃-Alkyl-amino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl-, 4-(C₁₋₃-Alkyl)-piperazin-1-yl-C₁₋₃-alkylgruppe,
   eine Hydroxy-C₁₋₃-alkyloxy-, C₁₋₃-Alkyloxy-C₁₋₃-alkyloxy-, C₁₋₃-Alkylsulfanyl-C₁₃-alkyloxy-, C₁₋₃-Alkylsulfinyl-C₁₋₃-alkyloxy-, C₁₋₃-Alkylsulfonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy-, 4-(C₁₋₃-Alkyl)-piperazin-1-yl-C₁₋₃-alkyloxygruppe,
   eine Mercapto-, C₁₋₃-Alkylsulfanyl-, C₁₋₃-Akysulfinyl-, C₁₋₃-Alkylsulfonyl-, C₁₋₃-Alkylsulfonyloxy-, Arylsulfonyloxy-, Trifluormethylsulfanyl-, Trifluormethylsulfinyl- oder Trifluormethylsulfonylgruppe,
   eine Sulfo-, Aminosulfonyl-, C₁₋₃-Alkyl-aminosulfonyl-, Di-(C₁₋₃-alkyl)-amino-sulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonyl-gruppe,
   eine durch 1 bis 3 Fluoratome substituierte Methyl- oder Methoxygruppe,
   eine durch 1 bis 5 Fluoratome substituierte Ethyl- oder Ethoxygruppe,
   eine C₂₋₄-Alkenyl- oder C₂₋₄-Alkinylgruppe,
   eine C₃₋₄-Alkenyloxy- oder C₃₋₄-Alkinyloxygruppe,
   eine C₃₋₆-Cycloalkyl- oder C₃₋₆-Cycloalkyloxygruppe,
   eine C₃₋₆-Cycloalkyl-C₁₋₃-alkyl- oder C₃₋₆-Cycloalkyl-C₁₋₃-alkyloxygruppe oder
   eine Aryl-, Aryloxy-, Aryl-C₁₋₃-alkyl- oder Aryl-C₁₋₃-alkyloxygruppe,
   R¹¹ und R¹², die gleich oder verschieden sein können, jeweils ein Wasserstoffatom, ein Fluor-, Chlor-, Brom- oder lodatom, eine C₁₋₃-Alkyl-, Trifluormethyl-, Hydroxy-, oder C₁₋₃-Alkyloxygruppe oder eine Cyangruppe, oder
   R¹¹ zusammen mit R¹², sofern diese an benachbarte Kohlenstoffatome gebunden sind, auch eine Methylendioxy-, Difluormethylendioxy-oder eine geradkettige C₃₋₅-Alkylengruppe,
      und
   R¹³ und R¹⁴, die gleich oder verschieden sein können, jeweils ein Wasserstoffatom, ein Fluor-, Chlor- oder Bromatom, eine Trifluormethyl-, C₁₋₃-Alkyl- oder C₁₋₃-Alkyloxygruppe bedeuten,
eine Phenyl-C₁₋₄-alkylgruppe, in der der Alkylteil durch eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-Gruppe substituiert ist und der Phenylteil durch die Gruppen R¹⁰ bis R¹⁴ substituiert ist, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴ wie vorstehend erwähnt definiert sind und
   A eine Carbonyl-, Cyaniminomethylen-, Hydroxyiminomethylen- oder C₁₋₃-Alkyloxyiminomethylen-Gruppe, m die Zahl 0, 1 oder 2 und n die Zahl 1, 2 oder 3 bedeuten,
eine Phenylcarbonylmethylgruppe, in der der Phenylteil durch R¹⁰ bis R¹⁴ substituiert ist, wobei R¹⁰ bis R¹⁴ wie vorstehend erwähnt definiert sind und der Methylteil durch eine C₁₋₃-Alkylgruppe substituiert ist,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, m und n wie vorstehend erwähnt definiert sind und
   B eine Methylengruppe, die durch eine Hydroxy-, C₁₋₃-Alkyloxy-, Amino-, C₁₋₃-Alkylamin-, Di-(C₁₋₃-alkyl)-amino-, Mercapto-, C₁₋₃-Alkylsulfanyl-, C₁₋₃-Alkyl-sulfinyl- oder C₁₋₃-Alkylsulfonylgruppe substituiert ist und gegebenenfalls zu-sätzlich durch eine Methyl- oder Ethylgruppe substituiert ist, bedeutet,
eine Naphthyl-C₁₋₃-alkylgruppe, in der der Naphthylteil durch die Gruppen R¹⁰ bis R¹⁴ substituiert ist, wobei R¹⁰ bis R¹⁴ wie vorstehend erwähnt definiert sind,
eine Naphthyl-(CH₂)ₘ-A-(CH₂)ₙ-Gruppe, in der der Naphthylteil durch R¹⁰ bis R¹⁴ substituiert ist, wobei R¹⁰ bis R¹⁴, A, m und n wie vorstehend erwähnt definiert sind,
eine Naphthyl-(CH₂)ₘ-B-(CH₂)ₙ-Gruppe, in der der Naphthylteil durch R¹⁰ bis R¹⁴ substituiert ist, wobei R¹⁰ bis R¹⁴, B, m und n wie vorstehend erwähnt definiert sind,
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₃-Alkyloxycarbonyl-, Aminocarbonyl-, C₁₋₃-Alkylaminocarbonyl-, Di-(C₁₋₃-alkyl)aminocarbonyl-, Pyrrolidin-1-yl-carbonyl-, Piperidin-1-yl-carbonyl- oder Morpholin-4-yl-carbonyl-, Piperazin-1-yl-carbonyl-, 4-Methylpiperazin-1-yl-carbonyl- oder 4-Ethylpiperazin-1-yl-carbonyl-Gruppe bedeutet und A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴ und m wie vorstehend erwähnt sind und D eine Sauerstoff- oder Schwefelatom, eine Imino-, C₁₋₃-Nkylimino-, Sulfinyl- oder Sulfonylgruppe bedeutet,
eine Naphthyl-(CH₂)ₘ-D-C₁₋₃-alkylgruppe, in der der Naphthylteil durch die Gruppen R¹⁰ bis R¹⁴ substituiert ist, wobei R¹⁰ bis R¹⁴, D und m wie vorstehend erwähnt sind, oder
eine durch eine Gruppe R_{b} substituierte C₂₋₆-Alkylgruppe, wobei
   R_{b} durch mindestens zwei Kohlenstoffatome vom Ringstickstoffatom in 1-Stellung des Xanthingerüstes isoliert ist und R_{b} eine Hydroxy-, C₁₋₃-Alkyloxy-, Mercapto-, C₁₋₃-Alkylsulfanyl-, C₁₋₃-Alkylsulfinyl-, C₁₋₃-Alkylsulfonyl-, Amino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl- oder 4-(C₁₋₃-Alkyl)-piperazin-1-ylgruppe bedeutet,
R² ein Wasserstoffatom,
eine C₁₋₈-Alkylgruppe,
eine C₂₋₆-Alkenylgruppe,
eine C₃₋₆-Alkinylgruppe,
eine durch eine Gruppe Rₐ substituierte C₁₋₆-Alkylgruppe, wobei Rₐ wie vorstehend erwähnt definiert ist,
eine Tetrahydrofuran-3-yl-, Tetrahydropyran-3-yl-, Tetrahydropyran-4-yl-, Tetrahydrofuranyl-C₁₋₃-alkyl- oder Tetrahydropyranyl-C₁₋₃-alkyl-gruppe,
eine durch eine Phenylgruppe substituierte C₁₋₆-Alkylgruppe, wobei der Phenylring durch die Gruppen R¹⁰ bis R¹⁴ substituiert ist und R¹⁰ bis R¹⁴ wie vorstehend erwähnt definiert sind,
eine durch die Gruppen R¹⁰ bis R¹⁴ substituierte Phenylgruppe, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴, A, m und n wie vorstehend erwähnt definiert sind,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, B, m und n wie vorstehend erwähnt definiert sind,
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹, A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴, m und D wie vorstehend erwähnt sind,
eine durch eine Gruppe R_{b} substituierte C₂₋₆-Alkylgruppe, wobei
   R_{b} durch mindestens zwei Kohlenstoffatome vom Ringstickstoffatom in 3-Stellung des Xanthingerüstes isoliert ist und wie vorstehend erwähnt definiert ist,
oder eine C₃₋₆-Cycloalkylgruppe,
R³ eine durch die Gruppe R_{c} substituierte C₁₋₄-Alkylgruppe, wobei
   R_{c} eine gegebenenfalls durch eine oder zwei C₁₋₃-Alkylgruppen substituierte C₃₋₇-Cycloalkylgruppe, oder
   eine gegebenenfalls durch eine oder zwei C₁₋₃-Alkylgruppen substituierte C₅₋₇-Cycloalkenylgruppe, bedeutet,
eine C₃₋₈-Alkenylgruppe;
eine durch ein Fluor-, Chlor- oder Bromatom, oder eine Trifluormethylgruppe substituierte C₃₋₆-Alkenylgruppe,
eine C₃₋₈-Alkinylgruppe, oder
eine Aryl-C₂₋₄-alkenylgruppe,
und
R⁴ eine Azetidin-1-yl- oder Pyrrolidin-1-ylgruppe, die in 3-Stellung durch eine RₑNR_{d}-Gruppe substituiert ist und zusätzlich durch eine oder zwei C₁₋₃-Alkylgruppen substituiert sein kann, wobei
   Rₑ ein Wasserstoffatom oder eine C₁₋₃-Alkylgruppe und
   R_{d} ein Wasserstoffatom, eine C₁₋₃-Alkylgruppe, eine R_{f}-C₁₋₃-alkylgruppe oder eine R_{g}-C₂₋₃-alkylgruppe bedeutet, wobei
   R_{f} eine Carboxy-, C₁₋₃-Alkyloxy-carbonyl-, Aminocarbonyl-, C₁₋₃-Alkylamino-carbonyl-, Di-(C₁₋₃-alkyl)-aminocarbonyl-, Pyrrolidin-1-yl-carbonyl-, 2-Cyan-pyrrolidin-1-yl-carbonyl-, 2-Carboxypyrrolidin-1-yl-carbonyl-, 2-Methoxycarbo-nylpyrrolidin-1-yl-carbonyl-, 2-Ethoxycarbonylpyrrolidin-1-yl-carbonyl-, 2-Aminocarbonylpyrrolidin-1-yl-carbonyl-, 4-Cyanthiazolidin-3-yl-carbonyl-, 4-Carboxythiazolidin-3-yl-carbonyl-, 4-Methoxycarbonylthiazolidin-3-yl-carbonyl-, 4-Ethoxycarbonylthiazolidin-3-yl-carbonyl-, 4-Aminocarbonylthiazolidin-3-yl-carbonyl-, Piperidin-1-yl-carbonyl-, Morpholin-4-yl-carbonyl-, Piperazin-1-yl-carbonyl-, 4-Methyl-piperazin-1-yl-carbonyl- oder 4-Ethyl-piperazin-1-yl-carbonyl-gruppe bedeutet und
   R_{g}, das mindestens durch zwei Kohlenstoffatome vom Stickstoffatom der RₑNR_{d}-Gruppe getrennt ist, eine Hydroxy-, Methoxy- oder Ethoxygruppe bedeutet,
eine Piperidin-1-yl- oder Hexahydroazepin-1-ylgruppe, die in 3-Stellung oder in 4-Stellung durch eine RₑNR_{d}-Gruppe substituiert ist und zusätzlich durch eine oder zwei C₁₋₃-Alkylgruppen substituiert sein kann, wobei Rₑ und R_{d} wie vorstehend erwähnt definiert sind,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonylGruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-amino-gruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoff-atome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl-oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine R¹⁹-C₃₋₄-alkyl-gruppe, in der der C₃₋₄-Alkylteil geradkettig ist und durch den Rest R¹⁵ substituiert sein kann und zusätzlich durch eine oder zwei C₁₋₃-Alkylgruppen substituiert sein kann, wobei R¹⁵ eine C₁₋₆-Alkylgruppe, eine C₃₋₆-Cycloalkyl-, C₃₋₆-Cycloalkyl-C₁₋₃-alkyl, Aryl- oder Aryl-C₁₋₃-alkylgruppe und R¹⁹ eine Amino-, C₁₋₃-Alkylamino- oder Di-(C₁₋₃-alkyl)-aminogruppe darstellt,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino-oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimi-dinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1 H-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 1,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl- oder 3,4-Dihydro-3-oxo-2H-benzo[1,4]oxazinyl-Gruppe zu verstehen ist,
   wobei die vorstehend erwähnten Heteroarylgruppen durch R¹⁰ bis R¹⁴ substituiert sein können, wobei R¹⁰ bis R¹⁴ wie vorstehend erwähnt definiert sind,
wobei, soweit nichts anderes erwähnt wurde, die vorstehend erwähnten Alkyl-, Alkenyl- und Alkinylgruppen geradkettig oder verzweigt sein können,
oder die am Ringstickstoffatom in 9-Stellung des Xanthingerüstes N-oxidierten oder methylierten oder ethylierten Derivate,
oder die Derivate, in denen die 2-Oxo-, die 6-Oxo- oder die 2-Oxo- und die 6-Oxogruppe des Xanthingerüstes durch Thioxogruppen ersetzt sind,
deren Tautomere, Enantiomere, Diastereomere, deren Gemische oder deren Salze;
zur Verwendung in der Kombinationstherapie mit anderen Wirkstoffen.

Weitere Gegenstände der vorliegenden Erfindung sind den vorliegenden Ansprüchen zu entnehmen.

US5735635 offenbart Xanthinderivate mit antithrombotischer Wirkung, während US5223499 Xanthinderivate beschreibt, welche als Angiotensin II Antagonisten Verwendung finden.

In den internationalen Anmeldungen WO 02/02560, WO 02/24698, WO 03/004496 und WO 03/024965 werden strukturähnliche Verbindungen beschrieben.

Weiterer Gegenstand der vorliegenden Beschreibung sind substituierte Xanthine der allgemeinen Formel 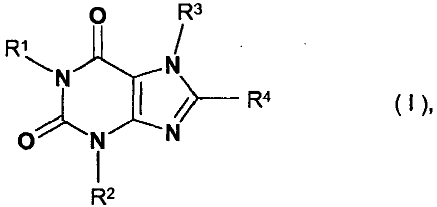 deren Tautomere, deren Stereoisomere, deren Gemische und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

In der obigen Formel I bedeuten
R¹ ein Wasserstoffatom,
eine C₁₋₈-Alkylgruppe,
eine C₃₋₈-Alkenylgruppe,
eine C₃₋₄-Alkenylgruppe, die durch eine C₁₋₂-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl- oder Morpholin-4-ylcarbonyl-gruppe substituiert ist,
eine C₃₋₈-Alkinylgruppe,
eine durch eine Gruppe Rₐ substituierte C₁₋₆-Alkylgruppe, wobei
Rₐ eine C₃₋₇-Cycloalkyl-, Heteroaryl-, Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonylgruppe bedeutet,
eine durch eine Phenylgruppe substituierte C₁₋₆-Alkylgruppe, wobei der Phenylring durch die Gruppen R¹⁰ bis R¹⁴ substituiert ist und
R¹⁰ ein Wasserstoffatom,
ein Fluor-, Chlor-, Brom- oder Iodatom,
eine C₁₋₄-Alkyl-, Hydroxy-, oder C₁₋₄-Alkyloxygruppe,
eine Nitro-, Amino-, C₁₋₃-Alkylamino-, Di-(C₁₋₃-alkyl)amino-, Cyan-C₁₋₃-alkylamino-, [N-(Cyan-C₁₋₃-alkyl)-N-C₁₋₃-alkyl-amino]-, C₁₋₃-Alkyloxy-carbonyl-C₁₋₃-alkylamino-, Pyrrolidin-1-yl-, Piperidin-1-yl-, Morpholin-4-yl-, Piperazin-1-yl-oder 4-(C₁₋₃-Alkyl)-piperazin-1-yl-Gruppe,
eine C₁₋₃-Alkyl-carbonylamino-, Arylcarbonylamino-, Aryl-C₁₋₃-alkyl-carbonyl-amino-, C₁₋₃-Alkyloxy-carbonylamino-, Aminocarbonylamino-, C₁₋₃-Alkyl-aminocarbonylamino-, Di-(C₁₋₃-alkyl)aminocarbonylamino-, Pyrrolidin-1-yl-carbonyl-amino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonylamino-, C₁₋₃-Alkyl-sulfonylamino-, Bis-(C₁₋₃-alkylsulfonyl)-amino-, Aminosulfonylamino-, C₁₋₃-Alkyl-amino-sulfonylamino-, Di-(C₁₋₃-alkyl)amino-sulfonylamino-, Pyrrolidin-1-yl-sulfonylamino-, Piperidin-1-yl-sulfonylamino-, Morpholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)thiocarbonylamino-, (C₁₋₃-Alkyloxy-carbonylamino)carbonyl-amino-, Arylsulfonylamino- oder Aryl-C₁₋₃-alkyl-sulfonylaminogruppe,
eine N-(C₁₋₃-Alkyl)-C₁₋₃-alkyl-carbonylamino-, N-(C₁₋₃-Alkyl)-arylcarbonylamino-, N-(C₁₋₃-Alkyl)-aryl-C₁₋₃-alkyl-carbonylamino-, N-(C₁₋₃-Alkyl)-C₁₋₃-alkyloxy-carbonylamino-, N-(Aminocarbonyl)-C₁₋₃-alkylamino-, N-(C₁₋₃-Alkyl-amino-carbonyl)-C₁₋₃-alkylamino-, N-[Di-(C₁₋₃-alkyl)aminocarbonyl]-C₁₋₃-alkylamino-, N-(C₁₋₃-Alkyl)-C₁₋₃-alkyl-sulfonylamino-, N-(C₁₋₃-Alkyl)-arylsulfonylamino- oder N-(C₁₋₃-Alkyl)-aryl-C₁₋₃-alkyl-sulfonylaminogruppe,
eine 2-Oxo-imidazolidin-1-yl-, 2,4-Dioxo-imidazolidin-1-yl-, 2,5-Dioxo-imidazo-lidin-1-yl-oder2-Oxo-hexahydropyrimidin-1-yl gruppe, in der das Stickstoffatom in 3-Stellung jeweils durch eine Methyl- oder Ethylgruppe substituiert sein kann,
eine Cyan-, Carboxy-, C₁₋₃-Alkyloxy-carbonyl-, Aminocarbonyl-, C₁₋₃-Alkyl-amino-carbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl- oder 4-(C₁₋₃-Alkyl)-piperazin-1-yl-carbonylgruppe,
eine C₁₋₃-Alkyl-carbonyl- oder eine Arylcarbonylgruppe,
eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1-yl-carbonyl-C₁₋₃-alkyl- oder 4-(C₁₋₃-Alkyl)-piperazin-1-yl-carbonyl-C₁₋₃-alkylgruppe,
eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy-, C₁₋₃-Alkyl-aminocarbonyl-C₁₋₃-alkyloxy-, Di-(C₁₋₃-alkyl)-aminocarbonyl-C₁₋₃-alkyloxy-, Pyrrolidin-1-yl-carbonyl-C₁₋₃-alkyl-oxy-, Piperidin-1-yl-carbonyl-C₁₋₃-alkyloxy-, Morpholin-4-yl-carbonyl-C₁₋₃-alkyl-oxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
eine Hydroxy-C₁₋₃-alkyl-, C₁₋₃-Alkyloxy-C₁₋₃-alkyl-, Amino-C₁₋₃-alkyl-, C₁₋₃-Alkyl-amino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl-, 4-(C₁₋₃-Alkyl)-piperazin-1-yl-C₁₋₃-alkylgruppe,
eine Hydroxy-C₁₋₃-alkyloxy-, C₁₋₃-Alkyloxy-C₁₋₃-alkyloxy-, C₁₋₃-Alkylsulfanyl-C₁₋₃-alkyloxy-, C₁₋₃-Alkylsulfinyl-C₁₋₃-alkyloxy-, C₁₋₃-Alkylsulfonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy-, 4-(C₁₋₃-Alkyl)-piperazin-1-yl-C₁₋₃-alkyloxygruppe,
eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Arylsulfonyloxy-, Trifluormethylsulfanyl-, Trifluormethylsulfinyl-oder Trifluormethylsulfonylgruppe,
eine Sulfo-, Aminosulfonyl-, C₁₋₃-Alkyl-aminosulfonyl-, Di-(C₁₋₃-Alkyl)-amino-sulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonyl-gruppe,
eine durch 1 bis 3 Fluoratome substituierte Methyl- oder Methoxygruppe,
eine durch 1 bis 5 Fluoratome substituierte Ethyl- oder Ethoxygruppe,
eine C₂₋₄-Alkenyl- oder C₂₋₄-Alkinylgruppe,
eine C₃₋₄-Alkenyloxy- oder C₃₋₄-Alkinyloxygruppe,
eine C₃₋₆-Cycloalkyl- oder C₃₋₆-Cycloalkyloxygruppe,
eine C₃₋₆-Cycloalkyl-C₁₋₃-alkyl- oder C₃₋₆-Cycloalkyl-C₁₋₃-alkyloxygruppe oder
eine Aryl-, Aryloxy-, Aryl-C₁₋₃-alkyl- oder Aryl-C₁₋₃-alkyloxygruppe,
R¹¹ und R¹², die gleich oder verschieden sein können, jeweils ein Wasserstoffatom, ein Fluor-, Chlor-, Brom- oder lodatom, eine C₁₋₃-Alkyl-, Trifluormethyl-, Hydroxy-, oder C₁₋₃-Alkyloxygruppe oder eine Cyangruppe, oder
R¹¹ zusammen mit R¹², sofern diese an benachbarte Kohlenstoffatome gebunden sind, auch eine Methylendioxy-, Difluormethylendioxy- oder eine geradkettige C₃₋₅-Alkylengruppe und
R¹³ und R¹⁴, die gleich oder verschieden sein können, jeweils ein Wasserstoffatom, ein Fluor-, Chlor- oder Bromatom, eine Trifluormethyl-, C₁₋₃-Alkyl- oder C₁₋₃-Alkyloxygruppe bedeuten,
eine Phenyl-C₁₋₄-alkylgruppe, in der der Alkylteil durch eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-Gruppe substituiert ist und der Phenylteil durch die Gruppen R¹⁰ bis R¹⁴ substituiert ist, wobei R¹⁰ bis R¹⁴ wie vorstehend erwähnt definiert sind,
eine durch die Gruppen R¹⁰ bis R¹⁴ substituierte Phenylgruppe, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴ wie vorstehend erwähnt definiert sind und
A eine Carbonyl-, Cyaniminomethylen-, Hydroxyiminomethylen- oder C₁₋₃-Alkyloxyiminomethylen-Gruppe, m die Zahl 0, 1 oder 2 und n die Zahl 1, 2 oder 3 bedeuten,
eine Phenylcarbonylmethylgruppe, in der der Phenylteil durch R¹⁰ bis R¹⁴ substituiert ist, wobei R¹⁰ bis R¹⁴ wie vorstehend erwähnt definiert sind und der Methylteil durch eine C₁₋₃-Alkylgruppe substituiert ist,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, m und n wie vorstehend erwähnt definiert sind und
B eine Methylengruppe, die durch eine Hydroxy-, C₁₋₃-Alkyloxy-, Amino-, C₁₋₃-Alkylamino-, Di-(C₁₋₃-alkyl)-amino-, Mercapto-, C₁₋₃-Alkylsulfanyl-, C₁₋₃-Alkyl-sulfinyl- oder C₁₋₃-Alkylsulfonylgruppe substituiert ist und gegebenenfalls zusätzlich durch eine Methyl- oder Ethylgruppe substituiert ist, bedeutet,
eine Naphthyl-C₁₋₃-alkylgruppe, in der der Naphthylteil durch die Gruppen R¹⁰ bis R¹⁴ substituiert ist, wobei R¹⁰ bis R¹⁴ wie vorstehend erwähnt definiert sind,
eine Naphthyl-(CH₂)ₘ-A-(CH₂)ₙ-Gruppe, in der der Naphthylteil durch R¹⁰ bis R¹⁴ substituiert ist, wobei R¹⁰ bis R¹⁴, A, m und n wie vorstehend erwähnt definiert sind,
eine Naphthyl-(CH₂)m-B-(CH₂)ₙ-Gruppe, in der der Naphthylteil durch R¹⁰ bis R¹⁴ substituiert ist, wobei R¹⁰ bis R¹⁴, B, m und n wie vorstehend erwähnt definiert sind,
eine [1,4]Naphthochinon-2-yl-, Chromen-4-on-3-yl-, 1-Oxoindan-2-yl-, 1,3-Dioxo-indan-2-yl- oder 2,3-Dihydro-3-oxo-benzofuran-2-ylgruppe
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₃-Alkyloxycarbonyl-, Aminocarbonyl-, C₁₋₃-Alkylaminocarbonyl-, Di-(C₁₋₃-alkyl)aminocarbonyl-, Pyrrolidin-1-yl-carbonyl-, Piperidin-1-yl-carbonyl- oder Morpholin-4-yl-carbonyl-, Piperazin-1-yl-carbonyl-, 4-Methylpiperazin-1-yl-carbonyl- oder 4-Ethylpiperazin-1-yl-carbonyl-Gruppe bedeutet und A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴ und m wie vorstehend erwähnt sind und D eine Sauerstoff- oder Schwefelatom, eine Imino-, C₁₋₃-Alkylimino-, Sulfinyl- oder Sulfonylgruppe bedeutet,
eine Naphthyl-(CH₂)ₘ-D-C₁₋₃-alkylgruppe, in der der Naphthylteil durch die Gruppen R¹⁰ bis R¹⁴ substituiert ist, wobei R¹⁰ bis R¹⁴, D und m wie vorstehend erwähnt sind,
eine durch eine Gruppe R_{b} substituierte C₂₋₆-Alkylgruppe, wobei
R_{b} durch mindestens zwei Kohlenstoffatome vom Ringstickstoffatom in 1-Stellung des Xanthingerüstes isoliert ist und
R_{b} eine Hydroxy-, C₁₋₃-Alkyloxy-, Mercapto-, C₁₋₃-Alkylsulfanyl-, C₁₋₃-Alkylsulfinyl-, C₁₋₃-Alyklsulfonyl-, Amino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl- oder 4-(C₁₋₃-Alkyl)-piperazin-1-ylgruppe bedeutet,
eine C₃₋₆-Cycloalkylgruppe,
oder eine Amino- oder Arylcarbonylaminogruppe,
R² ein Wasserstoffatom,
eine C₁₋₈-Alkylgruppe,
eine C₂₋₆-Alkenylgruppe,
eine C₃₋₆-Alkinylgruppe,
eine durch eine Gruppe Rₐ substituierte C₁₋₆-Alkylgruppe, wobei Rₐ wie vorstehend erwähnt definiert ist,
eine Tetrahydrofuran-3-yl-, Tetrahydropyran-3-yl-, Tetrahydropyran-4-yl-, Tetrahydrofuranyl-C₁₋₃-alkyl- oder Tetrahydropyranyl-C₁₋₃-alkyl-gruppe,
eine durch eine Phenylgruppe substituierte C₁₋₆-Alkylgruppe, wobei der Phenylring durch die Gruppen R¹⁰ bis R¹⁴ substituiert ist und R¹⁰ bis R¹⁴ wie vorstehend erwähnt definiert sind,
eine durch die Gruppen R¹⁰ bis R¹⁴ substituierte Phenylgruppe, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴, A, m und n wie vorstehend erwähnt definiert sind,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, B, m und n wie vorstehend erwähnt definiert sind,
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹, A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴, m und D wie vorstehend erwähnt sind,
eine durch eine Gruppe R_{b} substituierte C₂₋₆-Alkylgruppe, wobei
R_{b} durch mindestens zwei Kohlenstoffatome vom Ringstickstoffatom in 3-Stellung des Xanthingerüstes isoliert ist und wie vorstehend erwähnt definiert ist,
oder eine C₃₋₆-Cycloalkylgruppe,
R³ eine C₁₋₈-Alkylgruppe,
eine durch die Gruppe R_{c} substituierte C₁₋₄-Alkylgruppe, wobei
R_{c} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇-Cycloalkenylgruppe,
eine Arylgruppe oder
eine Furanyl-, Thienyl-, Oxazolyl-, Isoxazolyl-, Thiazolyl-, Isothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder lodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom oder eine Trifluormethylgruppe substituierte C₃₋₆-Alkenylgruppe,
eine C₃₋₈-Alkinylgruppe,
eine Arylgruppe oder
eine Aryl-C₂₋₄-alkyenylgruppe,
und
R⁴ eine Azetidin-1-yl- oder Pyrrolidin-1-ylgruppe, die in 3-Stellung durch eine RₑNR_{d}-Gruppe substituiert ist und zusätzlich durch eine oder zwei C₁₋₃-Alkylgruppen substituiert sein kann, wobei
Rₑ ein Wasserstoffatom oder eine C₁₋₃-Alkylgruppe und
R_{d} ein Wasserstoffatom, eine C₁₋₃-Alkylgruppe, eine R_{f}-C₁₋₃-alkylgruppe oder eine R_{g}-C₂₋₃-alkylgruppe bedeutet, wobei
   R_{f} eine Carboxy-, C₁₋₃-Alkyloxy-carbonyl-, Aminocarbonyl-, C₁₋₃-Alkylamino-carbonyl-, Di-(C₁₋₃-alkyl)-aminocarbonyl-, Pyrrolidin-1-yl-carbonyl-, 2-Cyanpyrrolidin-1-yl-carbonyl-, 2-Carboxypyrrolidin-1-yl-carbonyl-, 2-Methoxycarbonylpyrrolidin-1-yl-carbonyl-, 2-Ethoxy-carbonylpyrrolidin-1-yl-carbonyl-, 2-Aminocarbonylpyrrolidin-1-yl-carbonyl-, 4-Cyanthiazolidin-3-yl-carbonyl-, 4-Carboxythiazolidin-3-yl-carbonyl-, 4-Methoxycarbonylthiazolidin-3-yl-carbonyl-, 4-Ethoxy-carbonylthiazolidin-3-yl-carbonyl-, 4-Aminocarbonylthiazolidin-3-yl-carbonyl-, Piperidin-1-yl-carbonyl-, Morpholin-4-yl-carbonyl-, Piperazin-1-yl-carbonyl-, 4-Methyl-piperazin-1-yl-carbonyl- oder 4-Ethyl-piperazin-1-yl-carbonylgruppe bedeutet und
   R_{g}, das mindestens durch zwei Kohlenstoffatome vom Stickstoffatom der RₑNR_{d}-Gruppe getrennt ist, eine Hydroxy-, Methoxy- oder Ethoxygruppe bedeutet,
eine Piperidin-1-yl- oder Hexahydroazepin-1-ylgruppe, die in 3-Stellung oder in 4-Stellung durch eine RₑNR_{d}-Gruppe substituiert ist und zusätzlich durch eine oder zwei C₁₋₃-Alkylgruppen substituiert sein kann, wobei Rₑ und R_{d} wie vorstehend erwähnt definiert sind,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonylGruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-amino-gruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diaze-pan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cycloalkyl-C₁₋₂-alkyl)-N-(C₁₋₂-alkyl)-aminogruppe, in der der Cycloalkyl-teil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl-C₁₋₂-alkyl)-N-(C₁₋₂-alkyl)-aminogruppe, in der der Cycloalkyl-teil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine durch die Reste R¹⁵ und R¹⁶ substituierte Aminogruppe, in der
R¹⁵ eine C₁₋₆-Alkylgruppe, eine C₃₋₆-Cycloalkyl-, C₃₋₆-Cycloalkyl-C₁₋₃-alkyl-, Aryl-oder Aryl-C₁₋₃-alkylgruppe und
R¹⁶ eine R¹⁷-C₂₋₃-alkylgruppe darstellt, wobei der C₂₋₃-Alkylteil geradkettig ist und durch ein bis vier C₁₋₃-Alkylgruppen, die gleich oder verschieden sein können, substituiert sein kann, oder durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert sein kann und
R¹⁷ eine Amino-, C₁₋₃-Alkylamino- oder Di-(C₁₋₃-alkyl)-aminogruppe darstellt, wobei, falls R³ eine Methylgruppe bedeutet, R¹⁷ keine Di-(C₁₋₃-alkyl)-amino-gruppe darstellen kann,
eine durch den R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch die Reste R¹⁵ und R²⁰ substituierte Aminogruppe, in der
R¹⁵ und R²⁰ wie vorstehend erwähnt definiert sind, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und durch den Rest R¹⁵ substituiert sein kann und zusätzlich durch eine oder zwei C₁₋₃-Alkylgruppen substituiert sein kann, wobei R¹⁵ wie vorstehend erwähnt definiert ist und R¹⁹ eine Amino-, C₁₋₃-Alkylamino- oder Di-(C₁₋₃-alkyl)-aminogruppe darstellt,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino-oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₙ ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinyl-gruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimi-dinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1 H-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 1,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl- oder 3,4-Dihydro-3-oxo-2H-benzo[1,4]oxazinyl-Gruppe zu verstehen ist,
wobei die vorstehend erwähnten Heteroarylgruppen durch R¹⁰ bis R¹⁴ substituiert sein können, wobei R¹⁰ bis R¹⁴ wie vorstehend erwähnt definiert sind,
wobei, soweit nichts anderes erwähnt wurde, die vorstehend erwähnten Alkyl-, Alkenyl- und Alkinylgruppen geradkettig oder verzweigt sein können,
sowie die am Ringstickstoffatom in 9-Stellung des Xanthingerüstes N-oxidierten oder methylierten oder ethylierten Derivate,
sowie die Derivate, in denen die 2-Oxo-, die 6-Oxo- oder die 2-Oxo- und die 6-Oxogruppe des Xanthingerüstes durch Thioxogruppen ersetzt sind,
mit der Maßgabe, daß die Verbindungen, in denen
R¹ ein Wasserstoffatom, eine Methyl-, Propyl-, 2-Hydroxypropyl-, Aminocarbonylmethyl- oder Benzylgruppe,
R² eine Methylgruppe,
R³ eine C₁₋₈-Alkylgruppe, eine gegenenfalls durch ein Fluor-, Chlor- oder Bromatom oder eine Methylgruppe substituierte Benzylgruppe, eine 1-Phenylethyl- oder 2-Phenylethylgruppe, eine 2-Propen-1-yl-, 2-Buten-1-yl-, 3-Chlor-2-buten-1-yl- oder 2-Methyl-2-propen-1-ylgruppe
und
R⁴ eine Piperazin-1-ylgruppe darstellen, ausgeschlossen sind,
und mit der Maßgabe, daß die Verbindungen, in denen
R¹ ein Wasserstoffatom oder eine Methylgruppe,
R² ein Wasserstoffatom oder eine Methylgruppe,
R³ eine Methylgruppe
und
R⁴ eine 3-Aminopropyl-, 3-[Di-(C₁₋₃-alkyl)amino]-propyl-, 1-Phenyl-3-[di-(C₁₋₃-alkyl)-amino]-propyl-, 1-Phenyl-3-methyl-3-(dimethylamino)-propyl-, 1-(4-Chlorphenyl)-3-(dimethylamino)-propyl-, 1-Phenyl-2-methyl-3-(dimethylamino)-propyl-, 1-(3-Methoxyphenyl)-3-(dimethylamino)-propyl- oder eine 4-Aminobutylgruppe darstellen, ausgeschlossen sind,
und mit der Maßgabe, daß die Verbindung
1,3,7-Trimethyl-8-(1-aminocyclohexyl)-xanthin
ausgeschlossen ist,
deren Tautomere, Enantiomere, Diastereomere, deren Gemische und deren Salze.

Die bei der Definition der vorstehend erwähnten Reste erwähnten Carboxygruppen können durch eine in-vivo in eine Carboxygruppe überführbare Gruppe oder durch eine unter physiologischen Bedingungen negativ geladene Gruppe ersetzt sein, desweiteren können die bei der Definition der vorstehend erwähnten Reste erwähnten Amino- und Iminogruppen durch einen in-vivo abspaltbaren Rest substituiert sein. Derartige Gruppen werden beispielsweise in der WO 98/46576 und von N.M. Nielsen et al. in International Journal of Pharmaceutics 39, 75-85 (1987) beschrieben.

Unter einer in-vivo in eine Carboxygruppe überführbare Gruppe ist beispielsweise eine Hydroxymethylgruppe, eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkyloxy-carbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl-C₃₋₅-alkinol mit der Maßgabe, daß keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel

Rₚ-CO-O-(R_{q}CRᵣ)-OH,

in dem
Rₚ eine C₁₋₈-Alkyl-, C₅₋₇-Cycloalkyl-, C₁₋₈-Alkyloxy-, C₅₋₇-Cydoalkyloxy-, Phenyl- oder Phenyl- C₁₋₃-alkylgruppe,
Rq ein Wasserstoffatom, eine C₁₋₃-Alkyl-, C₅₋₇-Cycloalkyl- oder Phenylgruppe und
Rᵣ ein Wasserstoffatom oder eine C₁₋₃-Alkylgruppe darstellen,
unter einer unter physiologischen Bedingungen negativ geladenen Gruppe wie eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆Alkylsulfonylaminocarbonyl-, Phenylsulfonylamino-carbonyl-, Benzylsulfonylaminocarbonyl- oder Perfluor-C₁₋₆-alkylsulfonylamino-carbonylgruppe
und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Brom- oder Jodatome, durch C₁₋₃-Alkyl- oder C₁₋₃-Alkyloxygruppen mono- oder disubstituierte Phenylcarbonylgruppe, wobei die Substituenten gleich oder verschieden sein können, eine Pyridinoylgruppe oder eine C₁₋₁₆-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine C₁₋₁₆-Alkyloxy-carbonyl- oder C₁₋₁₆-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-C₁₋₆-alkyloxycarbonyl-gruppe wie die Benzyloxycarbonyl-, Phenylethoxycarbonyl- oder Phenylpropoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkyloxy-carbonyl-, C₁₋₃-Alkyloxy-C₂₋₄-alkyloxy-C₂₋₄-alkyloxycarbonyl-, Rₚ-CO-O-(R_{q}CRᵣ)-O-CO-, C₁₋₆-Alkyl-CO-NH-(RₛCRₜ)-O-CO- oder C₁₋₆-Alkyl-CO-O-(RₛCRₜ)-(RₛCRₜ)-O-CO-Gruppe, in denen Rₚ bis Rᵣ wie vorstehend erwähnt definiert sind,
   Rₛ und Rt, die gleich oder verschieden sein können, Wasserstoffatome oder C₁₋₃-Alkylgruppen darstellen,
zu verstehen.

Desweiteren schließen die in den vor- und nachstehenden Definitionen erwähnten gesättigten Alkyl- und Alkyloxyteile, die mehr als 2 Kohlenstoffatome enthalten, soweit nichts Anderes erwähnt wurde, auch deren verzweigte Isomere wie beispielsweise die Isopropyl-, tert.Butyl-, Isobutylgruppe etc. ein.

Für R¹ und R² kommt beispielsweise jeweils die Bedeutung eines Wasserstoffatoms, einer Methyl-, Ethyl-, Propyl-, 2-Propyl-, Butyl-, 2-Butyl-, 2-Methylpropyl-, 2-Propen-1-yl-, 2-Propin-1-yl-, Cyclopropylmethyl-, Benzyl-, 2-Phenylethyl-, Phenylcarbonyl-methyl-, 3-Phenylpropyl-, 2-Hydroxyethyl-, 2-Methoxyethyl-, 2-Ethoxyethyl-, 2-(Dimethylamino)ethyl-, 2-(Di-ethylamino)ethyl-, 2-(Pyrrolidino)ethyl-, 2-(Piperidino)ethyl-, 2-(Morpholino)ethyl-, 2-(Piperazino)ethyl-, 2-(4-Methylpiperazino)ethyl-, 3-Hydroxypropyl-, 3-Methoxy-propyl-, 3-Ethoxypropyl-, 3-(Dimethylamino)propyl-, 3-(Diethylamino)propyl-, 3-(Pyrrolidino)propyl-, 3-(Piperidino)propyl-, 3-(Morpholino)propyl-,3-(Piperazino)-propyl-, 3-(4-Methylpiperazino)propyl-, Carboxymethyl-, (Methoxycarbonyl)methyl-, (Ethoxycarbonyl)methyl-, 2-Carboxyethyl-, 2-(Methoxycarbonyl)ethyl-, 2-(Ethoxy-carbonyl)ethyl-, 3-Carboxypropyl-, 3-(Methoxycarbonyl)propyl-, 3-(Ethoxycarbonyl)-propyl-, (Aminocarbonyl)methyl-, (Methylaminocarbonyl)methyl-, (Dimethylaminocarbonyl)methyl-, (Pyrrolidinocarbonyl)methyl-, (Piperidinocarbonyl)methyl-, (Morpholinocarbonyl)methyl-, 2-(Aminocarbonyl)ethyl-, 2-(Methylaminocarbonyl)ethyl-, 2-(Dimethylaminocarbonyl)ethyl-, 2-(Pyrrolidinocarbonyl)ethyl-, 2-(Piperidinocarbonyl)-ethyl-, 2-(Morpholinocarbonyl)ethyl-, Cyanmethyl- oder 2-Cyanethylgruppe in Betracht.

Für R³ kommt beispielsweise die Bedeutung einer Methyl-, Ethyl-, Propyl-, 2-Propyl-, Butyl-, 2-Butyl-, 2-Methylpropyl-, Pentyl-, 2-Methylbutyl-, 3-Methylbutyl-, 2,2-Dimethylpropyl-, Cyclopropylmethyl-, (1-Methylcyclopropyl)methyl-, (2-Methylcyclo-propyl)methyl-, Cyclobutylmethyl-, Cyclopentylmethyl-, Cyclohexylmethyl-, 2-(Cyclo-propyl)ethyl-,
2-Propen-1-yl-, 2-Methyl-2-propen-1-yl-, 3-Phenyl-2-propen-1-yl-, 2-Buten-1-yl-, 4,4,4-Trifluor-2-buten-1-yl-, 3-Buten-1-yl-, 2-Chlor-2-buten-1-yl-, 2-Brom-2-buten-1-yl-, 3-Chlor-2-buten-1-yl-, 3-Brom-2-buten-1-yl-, 2-Methyl-2-buten-1-yl-, 3-Methyl-2-buten-1-yl-, 2,3-Dimethyl-2-buten-1-yl-, 3-Trifluormethyl-2-buten-1-yl-, 3-Methyl-3-buten-1-yl-,
1-Cyclopenten-1-ylmethyl-, (2-Methyl-1-cyclopenten-1-yl)methyl-, 1-Cyclohexen-1-ylmethyl-, 2-(1-Cyclopenten-1-yl)ethyl-, 2-Propin-1-yl-, 2-Butin-1-yl, 3-Butin-1-yl, Phenyl-, Methylphenyl-, Benzyl-, eine Fluorbenzyl-, Chlorbenzyl-, Brombenzyl-, Methylbenzyl-, Methoxybenzyl-, 1-Phenylethyl-, 2-Phenylethyl-, 3-Phenylpropyl-, 2-Furanylmethyl, 3-Furanylmethyl-, 2-Thienylmethyl- odert 3-Thienylmethylgruppe in Betracht.

Für R⁴ kommt beispielsweise die Bedeutung einer 3-Aminopyrrolidin-1-yl-, 3-Amino-piperidin-1-yl-, 3-(Methylamino)-piperidin-1-yl-, 3-(Ethylamino)-piperidin-1-yl-, 3-(Di-methylamino)-piperidin-1-yl-, 3-(Diethylamino)-piperidin-1-yl-, 3-[(2-Hydroxyethyl)-amino]-piperidin-1-yl-,
3-[N-Methyl-N-(2-hydroxyethyl)-amino]-piperidin-1-yl-, 3-[(3-Hydroxypropyl)amino]-piperidin-1-yl-, 3-[N-Methyl-N-(3-hydroxypropyl)-amino]-piperidin-1-yl-, 3-[(Carboxy-methyl)amino]-piperidin-1-yl-, 3-[(Methoxycarbonylmethyl)amino]-piperidin-1-yl-, 3-[(Ethoxycarbonylmethyl)amino]-piperidin-1-yl-, 3-[N-Methyl-N-(methoxycarbonyl-methyl)-amino]-piperidin-1-yl-,
3-[N-Methyl-N-(ethoxycarbonylmethyl)-amino]-piperidin-1-yl-, 3-[(2-Carboxyethyl)-amino]-piperidin-1-yl-, 3-{[2-(Methoxycarbonyl)ethyl]amino}-piperidin-1-yl-,
3-{[2-(Ethoxycarbonyl)ethyl]amino}-piperidin-1-yl-, 3-{N-Methyl-N-[2-(methoxy-carbonyl)ethyl]-amino}-piperidin-1-yl-, 3-{N-Methyl-N-[2-(ethoxycarbonyl)ethyl]-amino}-piperidin-1-yl-, 3-[(Aminocarbonylmethyl)amino]-piperidin-1-yl-, 3-[(Methyl-aminocarbonylmethyl)amino]-piperidin-1-yl-, 3-[(Dimethylaminocarbonylmethyl)-amino]-piperidin-1-yl-, 3-[(Ethylaminocarbonylmethyl)amino]-piperidin-1-yl-, 3-[(Diethylaminocarbonylmethyl)amino]-piperidin-1-yl-, 3-[(Pyrrolidin-1-ylcarbonylme-thyl)amino]-piperidin-1-yl-, 3-[(2-Cyanpyrrolidin-1-ylcarbonylmethyl)amino]-piperidin-1-yl-,
3-[(4-Cyanthiazolidin-3-ylcarbonylmethyl)amino]-piperidin-1-yl-, 3-[(2-Aminocarbonyl-pyrrolidin-1-ylcarbonylmethyl)amino]-piperidin-1-yl-, 3-[(2-Carboxypyrrolidin-1-yl-carbonylmethyl)amino]-piperidin-1-yl-, 3-[(2-Methoxycarbonylpyrrolidin-1-ylcarbonyl-methyl)amino]-piperidin-1-yl-, 3-[(2-Ethoxycarbonylpyrrolidin-1-ylcarbonylmethyl)-amino]-piperidin-1-yl-, 3-[(Piperidin-1-ylcarbonylmethyl)amino]-piperidin-1-yl-, 3-[(Morpholin-4-ylcarbonylmethyl)amino]-piperidin-1-yl-, 3-Amino-2-methyl-piperidin-1-yl-, 3-Amino-3-methyl-piperidin-1-yl-, 3-Amino-4-methyl-piperidin-1-yl-, 3-Amino-5-methyl-piperidin-1-yl-, 3-Amino-6-methyl-piperidin-1-yl-, 2-Amino-8-aza-bicyclo[3.2.1]oct-8-yl-, 6-Amino-2-aza-bicyclo[2.2.2]oct-2-yl-, 4-Aminopiperidin-1-yl-, 3-Amino-hexahydroazepin-1-yl-, 4-Amino-hexahydroazepin-1-yl-, Piperazin-1-yl-, [1,4]Diazepan-1-yl-, 3-Aminocyclopentyl-, 3-Aminocyclohexyl-, 3-(Methylamino)-cyclohexyl-,
3-(Ethylamino)-cyclohexyl-, 3-(Dimethylamino)-cyclohexyl-, 3-(Diethylamino)-cyclohexyl-, 4-Aminocyclohexyl-, (2-Aminocyclopropyl)amino-, (2-Aminocyclo-butyl)amino-, (3-Aminocyclobutyl)amino-, (2-Aminocyclopentyl)amino-, (3-Aminocyclopentyl)amino-, (2-Aminocyclohexyl)amino- oder (3-Aminocyclohexyl)aminogruppe in Betracht.

Eine besonders zu erwähnende Untergruppe betrifft diejenigen Verbindungen der allgemeinen Formel I, in denen R¹ bis R⁴ wie oben erwähnt definiert sind, mit der zusätzlichen Maßgabe, daß die Verbindungen, in denen R⁴ eine gegebenenfalls substituierte Piperazin-1-yl- oder [1,4]Diazepan-1-ylgruppe darstellt, ausgeschlossen sind, deren Tautomere, Enantiomere, Diastereomere, deren Gemische und deren Salze.

Eine zweite besonders zu erwähnende Untergruppe betrifft diejenigen Verbindungen der allgemeinen Formel I, in denen
R¹ ein Wasserstoffatom,
eine C₁₋₆-Alkylgruppe,
eine C₃₋₆-Alkenylgruppe,
eine C₃₋₄-Alkenylgruppe, die durch eine C₁₋₂-Alkyloxy-carbonylgruppe substituiert ist,
eine C₃₋₆-Alkinylgruppe,
eine C₃₋₆-Cycloalkyl-C₁₋₃-alkyl-Gruppe,
eine Phenylgruppe, die durch ein Fluor-, Chlor- oder Bromatom oder durch eine Methyl-, Trifluormethyl-, Hydroxy- oder Methoxygruppe substituiert sein kann,
eine Phenyl-C₁₋₄-alkyl-Gruppe, in der der Phenylteil durch R¹⁰ bis R¹² substituiert ist, wobei
R¹⁰ ein Wasserstoffatom, ein Fluor-, Chlor- oder Bromatom,
eine C₁₋₄-Alkyl-, Trifluormethyl-, Hydroxymethyl-, C₃₋₆-Cycloalkyl-, Ethinyl- oder Phenylgruppe,
eine Hydroxy-, C₁₋₄-Alkyloxy-, Difluormethoxy-, Trifluormethoxy-, 2,2,2-Trifluorethoxy-, Phenoxy-, Benzyloxy-, 2-Propen-1-yloxy-, 2-Propin-1-yloxy-, Cyan-C₁₋₂-alkyloxy-, C₁₋₂-Alkylsulfonyloxy-, Phenylsulfonyloxy-, Carboxy-C₁₋₃-alkyloxy-, C₁₋₃-Alkyloxy-carbonyl-C₁₋₃-alkyloxy-, Aminocarbonyl-C₁₋₃-alkyloxy-, C₁₋₂-Alkyl-aminocarbonyl-C₁₋₃-alkyloxy-, Di-(C₁₋₂-alkyl)aminocarbonyl-C₁₋₃-alkyloxy-, Pyrrolidin-1-yl-carbonyl-C₁₋₃-alkyloxy-, Piperidin-1-ylcarbonyl-C₁₋₃-alkyloxy-, Morpholin-4-ylcarbonyl-C₁₋₃-alkyloxy-, Methylsulfanylmethoxy-, Methylsulfinylmethoxy-, Methylsulfonylmethoxy-, C₃₋₆-Cycloalkyloxy- oder C₃₋₆-Cycloalkyl-C₁₋₂-alkyloxygruppe,
eine Carboxy-, C₁₋₃-Alkyloxycarbonyl-, Carboxy-C₁₋₃-alkyl-, C₁₋₃-Alkyloxy-carbonyl-C₁₋₃-alkyl-, Aminocarbonyl-, C₁₋₂-Alkylaminocarbonyl-, Di-(C₁₋₂-alkyl)aminocarbonyl-, Morpholin-4-ylcarbonyl- oder Cyanogruppe,
eine Nitro-, Amino-, C₁₋₂-Alkylamino-, Di-(C₁₋₂-alkyl)amino-, Cyan-C₁₋₂-alkylamino-, [N-(Cyan-C₁₋₂-alkyl)-N-C₁₋₂-alkyl-amino]-, C₁₋₂-Alkyloxy-carbonyl-C₁₋₂-alkylamino-, C₁₋₂-Alkyl-carbonylamino-, C₁₋₂-Alkyloxy-carbonylamino-, C₁₋₃-Alkylsulfonylamino-, Bis-(C₁₋₂-alkylsulfonyl)-amino-, Aminosulfonylamino-, C₁₋₂-Alkylamino-sulfonylamino-, Di-(C₁₋₂-alkyl)amino-sulfonylamino-, Morphol in-4-yl-sulfonylamino-, (C₁₋₂-Alkylamino)thiocarbonylamino-, (C₁₋₂-Alkyloxy-carbonylamino)carbonylamino-, Aminocarbonylamino-, C₁₋₂-Alkyl-aminocarbonylamino-, Di-(C₁₋₂-alkyl)aminocarbonylamino- oder Morpholin-4-ylcarbonylamino-Gruppe,
eine 2-Oxo-imidazolidin-1-yl-, 3-Methyl-2-oxo-imidazolidin-1-yl-, 2,4-Dioxo-imidazolidin-1-yl-, 3-Methyl-2,4-dioxo-imidazolidin-1-yl-, 2,5-Dioxo-imidazo-lidin-1-yl-, 3-Methyl-2,5-dioxo-imidazolidin-1-yl-, 2-Oxo-hexahydropyrimidin-1-yl- oder 3-Methyl-2-oxo-hexahydropyrimidin-1-yl-Gruppe,
oder
eine C₁₋₂-Alkylsulfanyl-, C₁₋₂-Alkylsulfinyl-, C₁₋₂-Alkylsulfonyl-, Aminosulfonyl-, C₁₋₂-Alkylaminosulfonyl- oder Di-(C₁₋₂-alkyl)aminosulfonylgruppe,
und R¹¹ und R¹², die gleich oder verschieden sein können, ein Wasserstoff-, Fluor-, Chlor- oder Bromatom oder
eine Methyl-, Cyan-, Trifluormethyl- oder Methoxygruppe,
oder, R¹¹ zusammen mit R¹², sofern diese an benachbarte Kohlenstoffatome gebunden sind, auch eine Methylendioxy-, Difluormethylendioxy-, 1,3-Propylen- oder 1,4-Butylen-Gruppe bedeuten,
eine Phenyl-C₁₋₃-alkylgruppe, in der der Alkylteil durch eine Carboxy-, C₁₋₂-Alkyloxy-carbonyl-, Aminocarbonyl-, C₁₋₂-Alkylaminocarbonyl- oder Di-(C₁₋₂-alkyl)aminocarbonyl-Gruppe substituiert ist,
eine Phenyl-C₂₋₃-alkenylgruppe, wobei der Phenylteil durch ein Fluor-, Chlor- oder Bromatom oder durch eine Methyl-, Trifluormethyl- oder Methoxygruppe substituiert sein kann,
eine Phenyl-(CH₂)ₘ-A-(CH₂)ₙ-Gruppe, in der der Phenylteil durch R¹⁰ bis R¹² substituiert ist, wobei R¹⁰ bis R¹² wie vorstehend erwähnt definiert sind und
A eine Carbonyl-, Hydroxyiminomethylen- oder C₁₋₂-Alkyloxyiminomethylen-Gruppe, m die Zahl 0 oder 1 und n die Zahl 1 oder 2 bedeuten,
eine Phenylcarbonylmethylgruppe, in der der Phenylteil durch R¹⁰ bis R¹² substituiert ist, wobei R¹⁰ bis R¹² wie vorstehend erwähnt definiert sind und der Methylteil durch eine Methyl- oder Ethylgruppe substituiert ist,
eine Phenylcarbonylmethylgruppe, in der zwei benachbarte Wasserstoffatome des Phenylteiles durch eine -O-CO-NH-, -NH-CO-NH-, -N=CH-NH-, -N=CH-O- oder -O-CH₂-CO-NH- Brücke ersetzt sind, wobei die vorstehend erwähnten Brücken durch eine oder zwei Methylgruppen substituiert sein können,
eine Phenyl-(CH₂)ₘ-B-(CH₂)ₙ-Gruppe, in der der Phenylteil durch R¹⁰ bis R¹² substituiert ist, wobei R¹⁰ bis R¹², m und n wie vorstehend erwähnt definiert sind und
B eine Methylengruppe, die durch eine Hydroxy- oder C₁₋₂-Alkyloxygruppe substituiert ist und gegebenenfalls zusätzlich durch eine Methylgruppe substituiert ist, bedeutet,
eine Naphthylmethyl- oder Naphthylethylgruppe, wobei der Naphthylteil jeweils durch R¹⁰ bis R¹² substituiert ist, wobei R¹⁰ bis R¹² wie vorstehend erwähnt definiert sind,
eine [1,4]Naphthochinon-2-yl-, Chromen-4-on-3-yl- oder 1-Oxoindan-2-ylgruppe,
eine Heteroaryl-C₁₋₃-alkylgruppe, wobei unter dem Begriff Heteroaryl eine Pyrrolyl-, Imidazolyl-, Triazolyl-, Furanyl-, Thienyl-, Oxazolyl-, Isoxazolyl-, Thiazolyl-, Isothiazolyl-, Pyridyl-, Pyridazinyl-, Pyrimidinyl-, Pyrazinyl-, Indolyl-, Benzimidazolyl-, 2,3-Dihydro-2-oxo-1*H*-benzimidazolyl-, Indazolyl-, Benzofuranyl-, 2,3-Dihydrobenzofuranyl-, Benzoxazolyl-, Dihydro-2-oxo-benzoxazolyl-, Benzoisoxazolyl-, Benzothiophenyl-, Benzothiazolyl-, Benzoisothiazolyl-, Chinolinyl-, 1,2-Dihydro-2-oxochinolinyl-, Isochinolinyl-, 1,2-Dihydro-1-oxo-isochinolinyl-, Cinnolinyl-, Chinazolinyl-, 1,2-Dihydro-2-oxo-chinazolinyl-, 1,2-Dihydro-1-oxo-phthalazin-4-yl-, Cumarinyl- oder 3,4-Dihydro-3-oxo-2H-benzo[1,4]oxazinyl-Gruppe zu verstehen ist,
wobei die vorstehend erwähnten Heteroarylgruppen an Kohlenstoffatomen durch ein Fluor-, Chlor- oder Bromatom, durch eine Methyl- Trifluormethyl-, Cyan-, Aminocarbonyl-, Aminosulfonyl-, Methylsulfonyl-, Nitro-, Amino-, Acetylamino-, Methylsulfonylamino-, Methoxy-, Difluormethoxy- oder Trifluormethoxygruppe substituiert sein können und die Iminogruppen der vorstehend erwähnten Heteroarylgruppen durch Methyl- oder Ethylgruppen substituiert sein können,
eine Furanyl-A-CH₂-, Thienyl-A-CH₂-, Thiazolyl-A-CH₂- oder Pyridyl-A-CH₂-Gruppe, wobei A wie vorstehend erwähnt definiert ist,
eine Furanyl-B-CH₂-, Thienyl-B-CH₂-, Thiazolyl-B-CH₂- oder Pyridyl-B-CH₂-Gruppe, wobei B wie vorstehend erwähnt definiert ist,
eine C₁₋₄-Alkyl-A-(CH₂)ₙ-Gruppe, wobei A und n wie vorstehend erwähnt definiert sind,
eine C₃₋₆-Cycloalkyl-(CH₂)ₘ-A-(CH₂)ₙ-Gruppe, wobei A, m und n wie vorstehend erwähnt definiert sind,
eine C₃₋₆-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₂-Nkyloxycarbonyl-, Aminocarbonyl-, C₁₋₂-Alkylaminocarbonyl-, Di-(C₁₋₂-alkyl)aminocarbonyl-, Pyrrolidin-1-yl-carbonyl-, Piperidin-1-yl-carbonyl- oder Morpholin-4-yl-carbonyl-Gruppe bedeutet und A und n wie vorstehend erwähnt definiert sind,
eine Phenyl-D-C₁₋₃-alkylgruppe, in der der Phenylteil gegebenenfalls durch ein Fluor, Chlor- oder Bromatom, eine Methyl-, Trifluormethyl- oder Methoxygruppe substituiert ist und D eine Sauerstoff- oder Schwefelatom, eine Sulfinyl- oder Sulfonylgruppe bedeutet,
eine durch eine Gruppe Rₐ substituierte C₁₋₄-Alkylgruppe, wobei
Rₐ eine Cyano-, Carboxy-, C₁₋₃-Alkyloxy-carbonyl-, Aminocarbonyl-, C₁₋₂-Alkyl-aminocarbonyl-, Di-(C₁₋₂-alkyl)aminocarbonyl-, Pyrrolidin-1-yl-carbonyl-, Piperidin-1-ylcarbonyl- oder Morpholin-4-ylcarbonyl-Gruppe bedeutet,
eine durch eine Gruppe R_{b} substituierte C₂₋₄-Alkylgruppe, wobei
R_{b} eine Hydroxy-, C₁₋₃-Alkyloxy-, Amino-, C₁₋₃-Alkylamino-, Di-(C₁₋₃-alkyl)-amino-, Pyrrolidin-1-yl-, Piperidin-1-yl-, Morpholin-4-yl, Piperazin-1-yl-, 4-Methyl-piperazin-1-yl- oder 4-Ethyl-piperazin-1-yl-Gruppe darstellt und durch mindestens zwei Kohlenstoffatome vom Ringstickstoffatom in 1-Stellung des Xanthingerüstes isoliert ist,
oder eine Amino- oder Benzoylaminogruppe,
R² ein Wasserstoffatom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Phenylgruppe, die gegebenenfalls durch ein Fluor-, Chlor- oder Bromatom oder durch eine Methyl-, Trifluormethyl-, Hydroxy-, Methoxy-, Difluormethoxy- oder Trifluormethoxygruppe substituiert ist,
eine Phenyl-C₁₋₄-alkylgruppe, in der der Phenylteil gegebenenfalls durch ein Fluor-, Chlor- oder Bromatom, eine Methyl-, Trifluormethyl-, Dimethylamino-, Hydroxy-, Methoxy-, Difluormethoxy- oder Trifluormethoxygruppe substituiert ist,
eine Phenyl-C₂₋₃-alkenylgruppe, wobei der Phenylteil durch ein Fluor-, Chlor- oder Bromatom oder durch eine Methyl-, Trifluormethyl- oder Methoxygruppe substituiert sein kann,
eine Phenylcarbonyl-C₁₋₂-alkylgruppe, in der der Phenylteil gegebenenfalls durch ein Fluor-, Chlor- oder Bromatom, eine Methyl-, Trifluormethyl-, Hydroxy-, Methoxy-, Difluormethoxy- oder Trifluormethoxygruppe substituiert ist,
eine Heteroaryl-C₁₋₃-alkylgruppe, wobei der Begriff Heteroaryl wie vorstehend erwähnt definiert ist,
eine Furanylcarbonylmethyl-, Thienylcarbonylmethyl-, Thiazolylcarbonylmethyl- oder Pyridylcarbonylmethylgruppe,
eine C₁₋₄-Alkyl-carbonyl-C₁₋₂-alkyl-Gruppe,
eine C₃₋₆-Cycloalkyl-carbonyl-C₁₋₂-alkyl-Gruppe,
eine Phenyl-D-C₁₋₃-alkylgruppe, in der der Phenylteil gegebenenfalls durch ein Fluor-, Chlor- oder Bromatom, eine Methyl-, Trifluormethyl-, Hydroxy-, Methoxy-, Difluormethoxy- oder Trifluormethoxygruppe substituiert ist, und D wie vorstehend erwähnt definiert ist, oder
eine durch eine Gruppe Rₐ substituierte C₁₋₄-Alkylgruppe, wobei Rₐ wie vorstehend erwähnt definiert ist, oder
eine durch eine Gruppe R_{b} substituierte C₂₋₄-Alkylgruppe, wobei R_{b} wie vorstehend erwähnt definiert ist und durch mindestens zwei Kohlenstoffatome vom Ringstickstoffatom in 3-Stellung des Xanthingerüstes isoliert ist,
R³ eine durch die Gruppe R_{c} substituierte C₁₋₃-Alkylgruppe, wobei
R_{c} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇-Cycloalkenylgruppe oder
eine Arylgruppe oder
eine Furanyl-, Thienyl-, Oxazolyl-, Isoxazolyl-, Thiazolyl-, Isothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder lodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom, oder eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine RₑNR_{d}-Gruppe substituiert ist und zusätzlich durch eine oder zwei C₁₋₃-Alkylgruppen substituiert sein kann, wobei
Rₑ ein Wasserstoffatom oder eine C₁₋₃-Alkylgruppe und
R_{d} ein Wasserstoffatom oder eine C₁₋₃-Alkylgruppe bedeutet,
eine Piperidin-1-yl- oder Hexahydroazepin-1-ylgruppe, die in 3-Stellung oder in 4-Stellung durch eine RₑNR_{d}-Gruppe substituiert ist und zusätzlich durch eine oder zwei C₁₋₃-Alkylgruppen substituiert sein kann, wobei Rₑ und R_{d} wie vorstehend erwähnt definiert sind,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diaze-pan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cycloalkyl-C₁₋₂-alkyl)-N-(C₁₋₂-alkyl)-aminogruppe, in der der Cycloalkyl-teil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl-C₁₋₂-alkyl)-N-(C₁₋₂-alkyl)-aminogruppe, in der der Cycloalkyl-teil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine durch die Reste R¹⁵ und R¹⁶ substituierte Aminogruppe, in der
R¹⁵ eine C₁₋₃-Alkylgruppe und
R¹⁶ eine R¹⁷-C₂₋₃-alkylgruppe darstellt, wobei der C₂₋₃-Alkylteil geradkettig ist und durch ein bis vier C₁₋₃-Alkylgruppen, die gleich oder verschieden sein können, substituiert sein kann, oder durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert sein kann und
R¹⁷ eine Amino-, C₁₋₃-Alkylamino- oder Di-(C₁₋₃-alkyl)-aminogruppe darstellt,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch die Reste R¹⁵ und R²⁰ substituierte Aminogruppe, in der
R¹⁵ und R²⁰ wie vorstehend erwähnt definiert sind, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und durch den Rest R¹⁵ substituiert sein kann und zusätzlich durch eine oder zwei C₁₋₃-Alkylgruppen substituiert sein kann, wobei R¹⁵ wie vorstehend erwähnt definiert ist und R¹⁹ eine Amino-, C₁₋₃-Alkylamino- oder Di-(C₁₋₃-alkyl)-aminogruppe darstellt,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino-oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt und
wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische und deren Salze.
Eine dritte besonders zu erwähnende Untergruppe betrifft diejenigen Verbindungen der allgemeinen Formel I, in denen
R¹, R² und R³ wie oben erwähnt definiert sind und
R⁴ eine Azetidin-1-yl- oder Pyrrolidin-1-ylgruppe, die in 3-Stellung durch eine RₑNR_{d}-Gruppe substituiert ist und zusätzlich durch eine oder zwei C₁₋₃-Alkylgruppen substituiert sein kann, wobei
Rₑ ein Wasserstoffatom oder eine C₁₋₃-Alkylgruppe und
R_{d} ein Wasserstoffatom oder eine C₁₋₃-Alkylgruppe bedeutet,
eine Piperidin-1-yl- oder Hexahydroazepin-1-ylgruppe, die in 3-Stellung oder in 4-Stellung durch eine RₑNR_{d}-Gruppe substituiert ist und zusätzlich durch eine oder zwei C₁₋₃-Alkylgruppen substituiert sein kann, wobei Rₑ und R_{d} wie vorstehend erwähnt definiert sind,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cycloalkyl-C₁₋₂-alkyl)-N-(C₁₋₂-alkyl)-aminogruppe, in der der Cycloalkyl-teil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)aminO-C₁₋₃-alkylgruppe substituiert ist,
eine N-(C₃₋₇-Cycloalkyl-C₁₋₂-alkyl)-N-(C₁₋₂-alkyl)-aminogruppe, in der der Cycloalkyl-teil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine durch die Reste R¹⁵ und R¹⁶ substituierte Aminogruppe, in der
R¹⁵ eine C₁₋₄-Alkylgruppe und
R¹⁶ eine R¹⁷-C₂₋₃-alkylgruppe darstellt, wobei der C₂₋₃-Alkylteil geradkettig ist und durch ein bis vier C₁₋₃-Alkylgruppen, die gleich oder verschieden sein können, substituiert sein kann, oder durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert sein kann und
R¹⁷ eine Amino-, C₁₋₃-Alkylamino- oder Di-(C₁₋₃-alkyl)-aminogruppe darstellt,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch die Reste R¹⁵ und R²⁰ substituierte Aminogruppe, in der
R¹⁵ und R²⁰ wie vorstehend erwähnt definiert sind, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und durch den Rest R¹⁵ substituiert sein kann und zusätzlich durch eine oder zwei C₁₋₃-Alkylgruppen substituiert sein kann, wobei R¹⁵ wie vorstehend erwähnt definiert ist und R¹⁹ eine Amino-, C₁₋₃-Alkylamino- oder Di-(C₁₋₃-alkyl)-aminogruppe darstellt,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino- oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten, deren Tautomere, Enantiomere, Diastereomere, deren Gemische und deren Salze.
Bevorzugte Verbindungen der obigen allgemeinen Formel I sind diejenigen, in denen
R¹ ein Wasserstoffatom,
eine C₁₋₆-Alkylgruppe,
eine C₃₋₆-Alkenylgruppe,
eine C₃₋₄-Alkenylgruppe, die durch eine C₁₋₂-Alkyloxy-carbonylgruppe substituiert ist,
eine C₃₋₆-Alkinylgruppe,
eine C₃₋₆-Cycloalkyl-C₁₋₃-alkyl-Gruppe,
eine Phenylgruppe, die durch ein Fluor-, Chlor- oder Bromatom oder durch eine Methyl-, Trifluormethyl-, Hydroxy- oder Methoxygruppe substituiert sein kann,
eine Phenyl-C₁₋₄-alkyl-Gruppe, in der der Phenylteil durch R¹⁰ bis R¹² substituiert ist, wobei
R¹⁰ ein Wasserstoffatom, ein Fluor-, Chlor- oder Bromatom,
eine C₁₋₄-Alkyl-, Trifluormethyl-, Hydroxymethyl-, C₃₋₆-Cycloalkyl-, Ethinyl- oder Phenylgruppe,
eine Hydroxy-, C₁₋₄-Alkyloxy-, Difluormethoxy-, Trifluormethoxy-, 2,2,2-Trifluorethoxy-, Phenoxy-, Benzyloxy-, 2-Propen-1-yloxy-, 2-Propin-1-yloxy-, Cyan-C₁₋₂-alkyloxy-, C₁₋₂-Alkylsulfonyloxy-, Phenylsulfonyloxy-, Carboxy-C₁₋₃-alkyloxy-, C₁₋₃-Alkyloxy-carbonyl-C₁₋₃-alkyloxy-, Aminocarbonyl-C₁₋₃-alkyloxy-, C₁₋₂-Alkyl-aminocarbonyl-C₁₋₃-alkyloxy-, Di-(C₁₋₂-alkyl)aminocarbonyl-C₁₋₃-alkyloxy-, Pyrrolidin-1-yl-carbonyl-C₁₋₃-alkyloxy-, Piperidin-1-ylcarbonyl-C₁₋₃-alkyloxy-, Morpholin-4-ylcarbonyl-C₁₋₃-alkyloxy-, Methylsulfanylmethoxy-, Methylsulfinylmethoxy-, Methylsulfonylmethoxy-, C₃₋₆-Cycloalkyloxy- oder C₃₋₆-Cycloalkyl-C₁₋₂-alkyloxygruppe,
eine Carboxy-, C₁₋₃-Alkyloxycarbonyl-, Carboxy-C₁₋₃-alkyl-, C₁₋₃-Alkyloxy-carbonyl-C₁₋₃-alkyl-, Aminocarbonyl-, C₁₋₂Alkylaminocarbonyl-, Di-(C₁₋₂-alkyl)aminocarbonyl-, Morpholin-4-ylcarbonyl- oder Cyanogruppe,
eine Nitro-, Amino-, C₁₋₂-Alkylamino-, Di-(C₁₋₂-alkyl)amino-, Cyan-C₁₋₂-alkylamino-, [N-(Cyan-C₁₋₂-alkyl)-N-C₁₋₂-alkyl-amino]-, C₁₋₂-Alkyloxy-carbonyl-C₁₋₂-alkylamino-, C₁₋₂-Alkyl-carbonylamino-, C₁₋₂-Alkyloxy-carbonylamino-, C₁₋₃-Alkylsulfonylamino-, Bis-(C₁₋₂-alkylsulfonyl)-amino-, Aminosulfonylamino-, C₁₋₂-Alkylamino-sulfonylamino-, Di-(C₁₋₂-alkyl)amino-sulfonylamino-, Morpholin-4-yl-sulfonylamino-, (C₁₋₂-Alkylamino)thiocarbonylamino-, (C₁₋₂-Alkyloxy-carbonylamino)carbonylamino-, Aminocarbonylamino-, C₁₋₂-Alkyl-aminocarbonylamino-, Di-(C₁₋₂-alkyl)aminocarbonylamino- oder Morpholin-4-ylcarbonylamino-Gruppe,
eine 2-Oxo-imidazolidin-1-yl-, 3-Methyl-2-oxo-imidazolidin-1-yl-, 2,4-Dioxo-imidazolidin-1-yl-, 3-Methyl-2,4-dioxo-imidazolidin-1-yl-, 2,5-Dioxo-imidazo-lidin-1-yl-, 3-Methyl-2,5-dioxo-imidazolidin-1-yl-, 2-Oxo-hexahydropyrimidin-1-yl- oder 3-Methyl-2-oxo-hexahydropyrimidin-1-yl-Gruppe,
oder
eine C₁₋₂-Alkylsulfanyl-, C₁₋₂-Alkylsulfinyl-, C₁₋₂Alkylsulfonyl-, Aminosulfonyl-, C₁₋₂-Alkylaminosulfonyl- oder Di-(C₁₋₂-alkyl)aminosulfonylgruppe,
und R¹¹ und R¹², die gleich oder verschieden sein können, ein Wasserstoff-, Fluor-, Chlor- oder Bromatom oder
eine Methyl-, Cyan-, Trifluormethyl- oder Methoxygruppe,
oder, R¹¹ zusammen mit R¹², sofern diese an benachbarte Kohlenstoffatome gebunden sind, auch eine Methylendioxy-, Difluormethylendioxy-, 1,3-Propylen- oder 1,4-Butylen-Gruppe bedeuten,
eine Phenyl-C₁₋₃-alkylgruppe, in der der Alkylteil durch eine Carboxy-, C₁₋₂-Alkyloxy-carbonyl-, Aminocarbonyl-, C₁₋₂-Alkylaminocarbonyl- oder Di-(C₁₋₂-alkyl)aminocarbonyl-Gruppe substituiert ist,
eine Phenyl-C₂₋₃-alkenylgruppe, wobei der Phenylteil durch ein Fluor-, Chlor- oder Bromatom oder durch eine Methyl-, Trifluormethyl- oder Methoxygruppe substituiert sein kann,
eine Phenyl-(CH₂)ₘ-A-(CH₂)ₙ-Gruppe, in der der Phenylteil durch R¹⁰ bis R¹² substituiert ist, wobei R¹⁰ bis R¹² wie vorstehend erwähnt definiert sind und
A eine Carbonyl-, Hydroxyiminomethylen- oder C₁₋₂-Alkyloxyiminomethylen-Gruppe, m die Zahl 0 oder 1 und n die Zahl 1 oder 2 bedeuten,
eine Phenylcarbonylmethylgruppe, in der der Phenylteil durch R¹⁰ bis R¹² substituiert ist, wobei R¹⁰ bis R¹² wie vorstehend erwähnt definiert sind und der Methylteil durch eine Methyl- oder Ethylgruppe substituiert ist,
eine Phenylcarbonylmethylgruppe, in der zwei benachbarte Wasserstoffatome des Phenylteiles durch eine -O-CO-NH-, -NH-CO-NH-, -N=CH-NH-, -N=CH-O- oder -O-CH₂-CO-NH- Brücke ersetzt sind, wobei die vorstehend erwähnten Brücken durch eine oder zwei Methylgruppen substituiert sein können,
eine Phenyl-(CH₂)ₘ-B-(CH₂)ₙ-Gruppe, in der der Phenylteil durch R¹⁰ bis R¹² substituiert ist, wobei R¹⁰ bis R¹², m und n wie vorstehend erwähnt definiert sind und
B eine Methylengruppe, die durch eine Hydroxy- oder C₁₋₂-Alkyloxygruppe substituiert ist und gegebenenfalls zusätzlich durch eine Methylgruppe substituiert ist, bedeutet,
eine Naphthylmethyl- oder Naphthylethylgruppe, wobei der Naphthylteil jeweils durch R¹⁰ bis R¹² substituiert ist, wobei R¹⁰ bis R¹² wie vorstehend erwähnt definiert sind,
eine [1,4]Naphthochinon-2-yl-, Chromen-4-on-3-yl- oder 1-Oxoindan-2-ylgruppe,
eine Heteroaryl-C₁₋₃-alkylgruppe, wobei unter dem Begriff Heteroaryl eine Pyrrolyl-, Imidazolyl-, Triazolyl-, Furanyl-, Thienyl-, Oxazolyl-, Isoxazolyl-, Thiazolyl-, Isothiazolyl-, Pyridyl-, Pyridazinyl-, Pyrimidinyl-, Pyrazinyl-, Indolyl-, Benzimidazolyl-, 2,3-Dihydro-2-oxo-1*H*-benzimidazolyl-, Indazolyl-, Benzofuranyl-, 2,3-Dihydrobenzofuranyl-, Benzoxazolyl-, Dihydro-2-oxo-benzoxazolyl-, Benzoisoxazolyl-, Benzothiophenyl-, Benzothiazolyl-, Benzoisothiazolyl-, Chinolinyl-, 1,2-Dihydro-2-oxo-chinolinyl-, Isochinolinyl-, 1,2-Dihydro-1-oxo-isochinolinyl-, Cinnolinyl-, Chinazolinyl-, 1,2-Dihydro-2-oxo-chinazolinyl-, 1,2-Dihydro-1-oxo-phthalazin-4-yl-, Cumarinyl- oder 3,4-Dihydro-3-oxo-2*H*-benzo[1,4]oxazinyl-Gruppe zu verstehen ist,
wobei die vorstehend erwähnten Heteroarylgruppen an Kohlenstoffatomen durch ein Fluor-, Chlor- oder Bromatom, durch eine Methyl- Trifluormethyl-, Cyan-, Aminocarbonyl-, Aminosulfonyl-, Methylsulfonyl-, Nitro-, Amino-, Acetylamino-, Methylsulfonylamino-, Methoxy-, Difluormethoxy- oder Trifluormethoxygruppe substituiert sein können und die Iminogruppen der vorstehend erwähnten Heteroarylgruppen durch Methyl- oder Ethylgruppen substituiert sein können,
eine Furanyl-A-CH₂-, Thienyl-A-CH₂-, Thiazolyl-A-CH₂- oder Pyridyl-A-CH₂-Gruppe, wobei A wie vorstehend erwähnt definiert ist,
eine Furanyl-B-CH₂-, Thienyl-B-CH₂-, Thiazolyl-B-CH₂- oder Pyridyl-B-CH₂-Gruppe, wobei B wie vorstehend erwähnt definiert ist,
eine C₁₋₄-Alkyl-A-(CH₂)ₙ-Gruppe, wobei A und n wie vorstehend erwähnt definiert sind,
eine C₃₋₆-Cycloalkyl-(CH₂)ₘ-A-(CH₂)ₙ-Gruppe, wobei A, m und n wie vorstehend erwähnt definiert sind,
eine C₃₋₆-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₂-Alkyloxycarbonyl-, Aminocarbonyl-, C₁₋₂-Alkylaminocarbonyl-, Di-(C₁₋₂-alkyl)aminocarbonyl-, Pyrrolidin-1-yl-carbonyl-, Piperidin-1-yl-carbonyl- oder Morpholin-4-yl-carbonyl-Gruppe bedeutet und A und n wie vorstehend erwähnt definiert sind,
eine Phenyl-D-C₁₋₃-alkylgruppe, in der der Phenylteil gegebenenfalls durch ein Fluor, Chlor- oder Bromatom, eine Methyl-, Trifluormethyl- oder Methoxygruppe substituiert ist und D eine Sauerstoff- oder Schwefelatom, eine Sulfinyl- oder Sulfonylgruppe bedeutet,
eine durch eine Gruppe Rₐ substituierte C₁₋₄-Alkylgruppe, wobei
Rₐ eine Cyano-, Carboxy-, C₁₋₃-Alkyloxy-carbonyl-, Aminocarbonyl-, C₁₋₂-Alkyl-aminocarbonyl-, Di-(C₁₋₂-alkyl)aminocarbonyl-, Pyrrolidin-1-yl-carbonyl-, Piperidin-1-ylcarbonyl- oder Morpholin-4-ylcarbonyl-Gruppe bedeutet,
eine durch eine Gruppe R_{b} substituierte C₂₋₄-Alkylgruppe, wobei
R_{b} eine Hydroxy-, C₁₋₃-Alkyloxy-, Amino-, C₁₋₃-Alkylamino-, Di-(C₁₋₃-alkyl)-amino-, Pyrrolidin-1-yl-, Piperidin-1-yl-, Morpholin-4-yl, Piperazin-1-yl-, 4-Methyl-piperazin-1-yl- oder 4-Ethyl-piperazin-1-yl-Gruppe darstellt und durch mindestens zwei Kohlenstoffatome vom Ringstickstoffatom in 1-Stellung des Xanthingerüstes isoliert ist,
oder eine Amino- oder Benzoylaminogruppe,
R² ein Wasserstoffatom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Phenylgruppe, die gegebenenfalls durch ein Fluor-, Chlor- oder Bromatom oder durch eine Methyl-, Trifluormethyl-, Hydroxy-, Methoxy-, Difluormethoxy- oder Trifluormethoxygruppe substituiert ist,
eine Phenyl-C₁₋₄-alkylgruppe, in der der Phenylteil gegebenenfalls durch ein Fluor-, Chlor- oder Bromatom, eine Methyl-, Trifluormethyl-, Dimethylamino-, Hydroxy-, Methoxy-, Difluormethoxy- oder Trifluormethoxygruppe substituiert ist,
eine Phenyl-C₂₋₃-alkenylgruppe, wobei der Phenylteil durch ein Fluor-, Chlor- oder Bromatom oder durch eine Methyl-, Trifluormethyl- oder Methoxygruppe substituiert sein kann,
eine Phenylcarbonyl-C₁₋₂-alkylgruppe, in der der Phenylteil gegebenenfalls durch ein Fluor-, Chlor- oder Bromatom, eine Methyl-, Trifluormethyl-, Hydroxy-, Methoxy-, Difluormethoxy- oder Trifluormethoxygruppe substituiert ist,
eine Heteroaryl-C₁₋₃-alkylgruppe, wobei der Begriff Heteroaryl wie vorstehend erwähnt definiert ist,
eine Furanylcarbonylmethyl-, Thienylcarbonylmethyl-, Thiazolylcarbonylmethyl- oder Pyridylcarbonylmethylgruppe,
eine C₁₋₄-Alkyl-carbonyl-C₁₋₂-alkyl-Gruppe,
eine C₃₋₆-Cycloalkyl-carbonyl-C₁₋₂-alkyl-Gruppe,
eine Phenyl-D-C₁₋₃-alkylgruppe, in der der Phenylteil gegebenenfalls durch ein Fluor-, Chlor- oder Bromatom, eine Methyl-, Trifluormethyl-, Hydroxy-, Methoxy-, Difluormethoxy- oder Trifluormethoxygruppe substituiert ist, und D wie vorstehend erwähnt definiert ist, oder
eine durch eine Gruppe Rₐ substituierte C₁₋₄-Alkylgruppe, wobei Rₐ wie vorstehend erwähnt definiert ist, oder
eine durch eine Gruppe R_{b} substituierte C₂₋₄-Alkylgruppe, wobei R_{b} wie vorstehend erwähnt definiert ist und durch mindestens zwei Kohlenstoffatome vom Ringstickstoffatom in 3-Stellung des Xanthingerüstes isoliert ist,
R³ eine C₂₋₆-Alkylgruppe,
eine C₃₋₇-Alkenylgruppe,
eine C₃₋₅-Alkenylgruppe, die durch ein Fluor- Chlor- oder Bromatom oder eine Trifluormethylgruppe substituiert ist,
eine C₃₋₆-Alkinylgruppe,
eine durch die Gruppe R_{c} substituierte C₁₋₃-Alkylgruppe, wobei
R_{c} eine gegebenenfalls durch eine oder zwei Methylgruppen substituierte C₃₋₆-Cycloalkylgruppe,
eine gegebenenfalls durch eine oder zwei Methylgruppen substituierte C₅₋₆-Cycloalkenylgruppe,
eine gegebenenfalls durch ein Fluor-, Chlor-, Brom- oder lodatom, durch eine Methyl-, Trifluormethyl-, Cyan-, Nitro-, Amino-, Hydroxy-, Methoxy-, Difluormethoxy- oder Trifluormethoxy gruppe substituierte Phenylgruppe,
eine Phenylgruppe, die durch zwei Fluoratome substituiert ist,
eine Naphthylgruppe oder
eine gegebenenfalls durch eine Methyl- oder Trifluormethylgruppe substituierte Furanyl-, Thienyl-, Oxazolyl-, Isoxazolyl-, Thiazolyl-, Isothiazolyl- oder Pyridylgruppe bedeutet,
eine gegebenenfalls durch ein Fluor-, Chlor- oder Bromatom, durch eine Methyl-, Trifluormethyl-, Cyan-, Hydroxy-, Methoxy-, Difluormethoxy- oder Trifluormethoxygruppe substituierte Phenylgruppe,
eine Phenylgruppe, die durch zwei Methylgruppen substituiert ist,
eine Naphthylgruppe
oder eine Phenyl-C₂₋₃-alkenyl-gruppe
und
R⁴ eine Pyrrolidin-1-ylgruppe, die in 3-Stellung durch eine Amino-, Methylamino- oder Dimethylaminogruppe substituiert ist,
eine Azetidin-1-ylgruppe, die durch eine Aminomethylgruppe substituiert ist,
eine Pyrrolidin-1-ylgruppe, die durch eine Aminomethylgruppe substituiert ist,
eine Piperidin-1-ylgruppe, die in 3-Stellung oder in 4-Stellung durch eine Amino-, Methylamino-, Dimethylamino- oder [(2-Cyan-pyrrolidin-1-yl-)carbonylmethyl]-aminogruppe substituiert ist, wobei der Piperidin-1-yl-Teil zusätzlich durch eine Methyl- oder Ethylgruppe substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine 3-Amino-piperidin-1-ylgruppe, in der ein Wasserstoffatom in 2-Stellung zusammen mit einem Wasserstoffatom in 6-Stellung durch eine -CH₂-CH₂-Brücke ersetzt ist,
eine 3-Amino-piperidin-1-ylgruppe, in der ein Wasserstoffatom in 4-Stellung zusammen mit einem Wasserstoffatom in 6-Stellung durch eine -CH₂-CH₂-Brücke ersetzt ist,
eine Piperidin-1-ylgruppe, die durch eine Aminomethylgruppe substituiert ist,
eine Piperidin-3-yl- oder Piperidin-4-ylgruppe,
eine Piperidin-3-yl- oder Piperidin-4-ylgruppe, die in 1-Stellung durch eine Aminogruppe substituiert ist,
eine Hexahydroazepin-1-yl-gruppe, die in 3-Stellung oder in 4-Stellung durch eine Aminogruppe substituiert ist,
eine gegebenenfalls am Kohlenstoffgerüst durch eine oder zwei Methylgruppen substituierte Piperazin-1-yl- oder [1,4]Diazepan-1-ylgruppe,
eine 3-Imino-piperazin-1-yl-, 3-Imino-[1,4]diazepan-1-yl- oder 5-Imino-[1,4]diazepan-1-ylgruppe,
eine [1,4]Diazepan-1-ylgruppe, die in 6-Stellung durch eine Aminogruppe substituiert ist,
eine C₃₋₆-Cycloalkyl-aminogruppe, in der der Cycloalkylteil durch eine Amino-, Methylamino- oder Dimethylaminogruppe substituiert ist, wobei die beiden Stickstoffatome am Cycloalkylteil durch mindestens zwei Kohlenstoffatome voneinander isoliert sind,
eine N-(C₃₋₆-Cycloalkyl)-N-(C₁₋₂-alkyl)-aminogruppe, in der der Cycloalkylteil durch eine Amino-, Methylamino- oder Dimethylaminogruppe substituiert ist, wobei die beiden Stickstoffatome am Cycloalkylteil durch mindestens zwei Kohlenstoffatome voneinander isoliert sind,
eine C₃₋₆-Cycloalkyl-aminogruppe, in der der Cycloalkylteil durch eine Aminomethyl- oder Aminoethylgruppe substituiert ist,
eine N-(C₃₋₆-Cycloalkyl)-N-(C₁₋₂-alkyl)-aminogruppe, in der der Cycloalkylteil durch eine Aminomethyl- oder Aminoethylgruppe substituiert ist,
eine C₃₋₆-Cycloalkyl-C₁₋₂-alkyl-aminogruppe, in der der Cycloalkylteil durch eine Amino-, Aminomethyl- oder Aminoethylgruppe substituiert ist,
eine N-(C₃₋₆-Cycloalkyl-C₁₋₂-alkyl)-N-(C₁₋₂-alkyl)-aminogruppe, in der der Cycloalkylteil durch eine Amino-, Aminomethyl- oder Aminoethylgruppe substituiert ist,
eine durch die Reste R¹⁵ und R¹⁶ substituierte Aminogruppe, in der
R¹⁵ eine C₁₋₄-Alkylgruppe und
R¹⁶ eine 2-Aminoethyl-, 2-(Methylamino)ethyl- oder 2-(Dimethylamino)ethylgruppe darstellt, wobei der Ethylteil jeweils durch eine oder zwei Methyl- oder Ethylgruppen oder durch eine Aminocarbonyl-, C₁₋₂-Alkyl-aminocarbonyl-, Di-(C₁₋₂-alkyl)aminocarbonyl-, Pyrrolidin-1-yl-carbonyl-, Piperidin-1-ylcarbonyl-oder Morpholin-4-ylcarbonyl-Gruppe substituiert sein kann,
eine Aminogruppe, in der das Stickstoffatom durch eine Pyrrolidin-3-yl-, Piperidin-3-yl-, Piperidin-4-yl-, Pyrrolidin-2-ylmethyl-, Pyrrolidin-3-ylmethyl-, Piperidin-2-ylmethyl-, Piperidin-3-ylmethyl- oder Piperidin-4-ylmethylgruppe substituiert ist,
eine C₁₋₂-Alkylaminogruppe, in der das Stickstoffatom durch eine Pyrrolidin-3-yl-, Piperidin-3-yl-, Piperidin-4-yl-, Pyrrolidin-2-ylmethyl-, Pyrrolidin-3-ylmethyl-, Piperidin-2-ylmethyl-, Piperidin-3-ylmethyl- oder Piperidin-4-ylmethylgruppe substituiert ist,
eine 3-Amino-propyl-, 3-Methylamino-propyl- oder 3-Dimethylamino-propylgruppe, in der der Propylteil durch eine oder zwei Methylgruppen substituiert sein kann,
eine 4-Amino-butyl-, 4-Methylamino-butyl- oder 4-Dimethylamino-butylgruppe, in der der Butylteil durch eine oder zwei Methylgruppen substituiert sein kann,
eine C₁₋₂-Alkylgruppe, die durch eine 2-Pyrrolidinyl-, 3-Pyrrolidinyl-, 2-Piperidinyl-, 3-Piperidinyl- oder 4-Piperidinylgruppe substituiert ist,
eine 3-Amino-2-oxo-piperidin-5-yl- oder 3-Amino-2-oxo-1-methyl-piperidin-5-yl-Gruppe,
eine C₃₋₆-Cycloalkylgruppe, die durch eine Amino-, Aminomethyl- oder Aminoethylgruppe substituiert ist oder
eine C₃₋₆-Cycloalkyl-C₁₋₂-alkylgruppe, in der der Cycloalkylteil durch eine Amino-, Aminomethyl- oder Aminoethylgruppe substituiert ist, bedeuten,
wobei soweit nichts anderes erwähnt wurde, die vorstehend erwähnten Alkyl-, Alkenyl- und Alkinylgruppen geradkettig oder verzweigt sein können,
mit der Maßgabe, daß die Verbindungen, in denen
R¹ ein Wasserstoffatom, eine Methyl-, Propyl-, 2-Hydroxypropyl-, Aminocarbonylmethyl- oder Benzylgruppe,
R² eine Methylgruppe,
R³ eine C₁₋₅-Alkylgruppe, eine gegenenfalls durch ein Fluor-, Chlor- oder Bromatom oder eine Methylgruppe substituierte Benzylgruppe, eine 1-Phenylethyl- oder 2-Phenylethylgruppe, eine 2-Propen-1-yl-, 2-Buten-1-yl-, 3-Chlor-2-buten-1-yl- oder 2-Methyl-2-propen-1-ylgruppe
und
R⁴ eine Piperazin-1-ylgruppe darstellen, ausgeschlossen sind,
deren Tautomere, Enantiomere, Diastereomere, deren Gemische und deren Salze.

Eine besonders zu erwähnende Untergruppe der bevorzugten Verbindungen der Formel I betrifft diejenigen Verbindungen der allgemeinen Formel I, in denen R¹ bis R⁴ wie oben erwähnt definiert sind, mit der zusätzlichen Maßgabe, daß die Verbindungen, in denen R⁴ eine gegebenenfalls substituierte Piperazin-1-yl- oder [1,4]Diazepan-1-ylgruppe darstellt, ausgeschlossen sind, deren Tautomere, Enantiomere, Diastereomere, deren Gemische und deren Salze.

Eine zweite besonders zu erwähnende Untergruppe der bevorzugten Verbindungen der Formel I betrifft diejenigen Verbindungen der allgemeinen Formel I, in denen
R¹ ein Wasserstoffatom,
eine C₁₋₄-Alkylgruppe,
eine C₃₋₅-Alkenylgruppe,
eine 2-Propen-1-ylgruppe, die durch eine Methoxycarbonylgruppe substituiert ist,
eine C₃₋₅-Alkinylgruppe,
eine Phenyl-C₁₋₄-alkyl-Gruppe, in der der Phenylteil durch R¹⁰ bis R¹² substituiert ist, wobei
R¹⁰ ein Wasserstoffatom, ein Fluor-, Chlor- oder Bromatom,
eine Methyl-, Ethyl-, Trifluormethyl-, oder Ethinylgruppe,
eine Hydroxy-, Methoxy-, Ethoxy-, Difluormethoxy-, Trifluormethoxy-, 2,2,2-Trifluorethoxy-, Phenoxy-, Benzyloxy-, 2-Propen-1-yloxy-, 2-Propin-1-yloxy-, Cyan-C₁₋₂-alkyloxy-, C₁₋₂-Alkyl-sulfonyloxy-, Phenylsulfonyloxy-, Carboxy-C₁₋₂-alkyloxy-, C₁₋₂-Alkyloxy-carbonyl-C₁₋₂-alkyloxy-, Aminocarbonyl-C₁₋₂-alkyloxy-, C₁₋₂-Alkyl-aminocarbonyl-C₁₋₂-alkyloxy-, Di-(C₁₋₂-alkyl)aminocarbonyl-C₁₋₂-alkyloxy-, Pyrrolidin-1-ylcarbonyl-C₁₋₂-alkyloxy-, Piperidin-1-ylcarbonyl-C₁₋₂-alkyloxy-, Morpholin-4-ylcarbonyl-C₁₋₂-alkyloxy-gruppe,
eine Carboxy-, C₁₋₂-Alkyloxy-carbonyl-, Aminocarbonyl-, C₁₋₂-Alkylaminocar-bonyl-, Di-(C₁₋₂-alkyl)aminocarbonyl-, Morpholin-4-ylcarbonyl- oder Cyanogruppe,
eine Nitro-, Amino-, C₁₋₂-Alkylamino-, Di-(C₁₋₂-alkyl)amino-, Cyan-C₁₋₂-alkylamino-, [N-(Cyan-C₁₋₂-alkyl)-N-methyl-amino]-, C₁₋₂-Alkyloxy-carbonyl-C₁₋₂-alkylamino-, C₁₋₂-Alkyl-carbonylamino-, C₁₋₂-Alkyloxy-carbonylamino-, C₁₋₂-Alkylsulfonylamino-, Bis-(C₁₋₂-alkylsulfonyl)-amino-, Aminosulfonylamino-, C₁₋₂-Alkylamino-sulfonylamino-, Di-(C₁₋₂-alkyl)amino-sulfonylamino-, Morpholin-4-yl-sulfonylamino-, (C₁₋₂-Alkylamino)thiocarbonylamino-, (C₁₋₂-Alkyloxy-carbonylamino)carbonylamino-, Aminocarbonylamino-, C₁₋₂-Alkyl-aminocarbonylamino-, Di-(C₁₋₂-alkyl)aminocarbonylamino- oder Morpholin-4-yl-carbonylamino-Gruppe,
eine 2-Oxo-imidazolidin-1-yl-, 3-Methyl-2-oxo-imidazolidin-1-yl-, 2,4-Dioxo-imidazolidin-1-yl-, 3-Methyl-2,4-dioxo-imidazolidin-1-yl-, 2,5-Dioxo-imidazo-lidin-1-yl-, 3-Methyl-2,5-dioxo-imidazolidin-1-yl-, 2-Oxo-hexahydropyrimidin-1-yl- oder 3-Methyl-2-oxo-hexahydropyrimidin-1-yl-Gruppe,
oder
eine C₁₋₂-Alkylsulfanyl-, C₁₋₂-Alkylsulfinyl-, C₁₋₂-Alkylsulfonyl-, Aminosulfonyl-, C₁-₂-Alkylaminosulfonyl- oder Di-(C₁₋₂-alkyl)aminosulfonylgruppe,
und R¹¹ und R¹², die gleich oder verschieden sein können, ein Wasserstoff-, Fluor-, Chlor- oder Bromatom oder
eine Methyl-, Cyan- oder Methoxygruppe,
oder, R¹¹ zusammen mit R¹², sofern diese an benachbarte Kohlenstoffatome gebunden sind, auch eine Methylendioxy-Gruppe bedeuten,
eine Phenylmethylgruppe, in der der Methylteil durch eine Carboxy-, Methoxycarbonyl- oder Aminocarbonylgruppe substituiert ist,
eine 2-Phenylethylgruppe, in der der Ethylteil durch eine Carboxy-, Methoxycarbonyl- oder Aminocarbonylgruppe substituiert ist,
eine 2-Phenylethylgruppe, in der der Ethylteil in 2-Stellung durch eine Hydroxy-, Methoxy-, Hydroxyimino- oder Methoxyiminogruppe substituiert ist,
eine 2-Phenylethylgruppe, in der der Ethylteil in 2-Stellung durch eine Hydroxygruppe und eine Methylgruppe substituiert ist,
eine Phenylcarbonylmethylgruppe, in der der Phenylteil durch R¹⁰ bis R¹² substituiert ist, wobei R¹⁰ bis R¹² wie vorstehend erwähnt definiert sind,
eine 1-(Phenylcarbonyl)ethyl- oder 2-(Phenylcarbonyl)ethylgruppe,
eine 2-Phenylethenylgruppe,
eine Phenylsulfanylmethyl- oder Phenylsulfinylmethylgruppe,
eine 2-(Phenyloxy)ethylgruppe,
eine Naphthylmethyl- oder Naphthylethylgruppe, wobei der Naphthylteil jeweils durch eine Methyl-, Nitro-, Amino-, Acetylamino-, Methylsulfonylamino-, Cyan-, Aminocarbonyl- oder Aminosulfonylgruppe substituiert sein kann,
eine [1,4]Naphthochinon-2-yl-, Chromen-4-on-3-yl- oder 1-Oxoindan-2-ylgruppe
eine Oxazolylmethyl-, Isoxazolylmethyl-, Thiazolylmethyl-, Pyridylmethyl-, Benzo-furanylmethyl-, 2,3-Dihydrobenzofuranylmethyl-, Benzo[d]isoxazolylmethyl-, Benzo-[d]isothiazolylmethyl-, (1*H*-Indazol-3-yl)methyl-, Chinolinylmethyl-, (1,2-Dihydro-2-oxo-chinolin-4-yl)methyl-, Isochinolinylmethyl-, (1,2-Dihydro-1-oxo-isochinolin-4-yl)methyl-, Cinnolinylmethyl-, Chinazolinylmethyl-, (1,2-Dihydro-2-oxo-chinazolin-4-yl)methyl-, (1,2-Dihydro-1-oxo-phthalazin-4-yl)methyl- oder Cumarinylmethyl-Gruppe, wobei der heterocyclische Teil jeweils durch eine Methylgruppe substituiert sein kann,
eine Chinolinylmethyl- oder Isochinolinylmethylgruppe, wobei der heterocyclische Teil jeweils durch eine Cyan-, Nitro-, Amino-, Acetylamino-, Methylsulfonylamino-, Aminocarbonyl- oder Aminosulfonylgruppe substituiert ist,
eine Pyrrolylethyl-, Triazolylethyl-, Thienylethyl-, Thiazolylethyl- oder Pyridylethylgruppe, wobei der heterocyclische Teil jeweils durch eine Methylgruppe substituiert sein kann,
eine Furanylcarbonylmethyl-, Thienylcarbonylmethyl-, Thiazolylcarbonylmethyl- oder Pyridylcarbonylmethylgruppe,
eine Methylgruppe, die durch eine Cyclopropyl-, Cyan-, Carboxy-, Aminocarbonyl- oder Methoxycarbonylgruppe substituiert ist,
eine Ethylgruppe, die in 2-Stellung durch eine Hydroxy-, Methoxy-, Dimethylamino-, Carboxy- oder Methoxycarbonylgruppe substituiert ist, oder
eine Propylgruppe, die in 3-Stellung durch eine Hydroxy-, Dimethylamino-, Carboxy-oder Methoxycarbonylgruppe substituiert ist,
eine 2-Oxopropylgruppe oder
eine Amino- oder Benzoylaminogruppe,
R² ein Wasserstoffatom,
eine C₁₋₆-Alkylgruppe,
eine Ethenylgruppe,
eine 2-Propen-1-yl- oder 2-Propin-1-ylgruppe,
eine C₃₋₆-Cycloalkylgruppe,
eine Tetrahydrofuran-3-yl-, Tetrahydropyran-3-yl-, Tetrahydropyran-4-yl-, Tetrahydrofuranylmethyl- oder Tetrahydropyranylmethylgruppe,
eine Phenylgruppe,
eine Phenyl-C₁₋₄-alkylgruppe, wobei der Phenylteil durch ein Fluor- oder Chloratom, eine Methyl-, Dimethylamino-, Hydroxy-, Methoxy- oder Trifluormethoxygruppe substituiert sein kann,
eine Phenylcarbonylmethylgruppe, wobei der Phenylteil durch ein Fluor- oder Chloratom, eine Hydroxy-, Methoxy- oder Trifluormethoxygruppe substituiert sein kann,
eine 2-Phenylethenylgruppe,
eine 2-(Phenyloxy)ethylgruppe,
eine Pyridylmethyl- oder Pyridylethylgruppe,
eine Methylgruppe, die durch eine C₃₋₆-Cycloalkyl-, Cyan-, Carboxy- oder Methoxycarbonylgruppe substituiert ist, oder
eine Ethylgruppe, die in 2-Stellung durch eine C₃₋₆-Cycloalkyl-, Cyan-, Carboxy-, Methoxycarbonyl-, Hydroxy-, Methoxy- oder Dimethylaminogruppe substituiert ist,
oder eine Propylgruppe, die in 3-Stellung durch eine C₃₋₆-Cycloalkyl-, Cyan-, Carboxy-, Methoxycarbonyl-, Hydroxy-, Methoxy- oder Dimethylaminogruppe substituiert ist,
R³ eine C₄₋₆-Alkenylgruppe,
eine 1-Cyclopenten-1-ylmethyl- oder 1-Cyclohexen-1-ylmethylgruppe,
eine 1-Cydopenten-1-ylmethylgruppe, in der der 1-Cyclopenten-1-yl-Teil durch eine Methylgruppe substituiert ist,
eine 2-Propin-1-yl-, 2-Butin-1-yl- oder 2-Pentin-1-ylgruppe,
eine Phenylgruppe, die durch ein Fluoratom oder eine Cyan-, Methyl- Methoxy- oder Trifluormethylgruppe substituiert sein kann,
eine Phenylgruppe, die durch zwei Methylgruppen substituiert ist,
eine Benzylgruppe, in der der Phenylteil durch ein oder zwei Fluoratome, ein Chlor-, Brom- oder lodatom, oder eine Methyl-, Methoxy-, Cyan-, Nitro- oder Aminogruppe substituiert sein kann,
eine Furanylmethyl- oder Thienylmethylgruppe,
eine Cyclopropylmethylgruppe oder
eine Cyclopropylmethylgruppe, in der der Cyclopropylteil durch eine Methylgruppe substituiert ist, und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Aminocarbonyl-, Methylaminocarbonyl-, Dimethylaminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist,
eine 3-Amino-2-oxo-piperidin-5-yl- oder 3-Amino-2-oxo-1-methyl-piperidin-5-yl-Gruppe,
eine [1,4]Diazepan-1-ylgruppe, die in 6-Stellung durch eine Aminogruppe substituiert ist,
eine Cyclohexylgruppe, die in 3-Stellung durch eine Aminogruppe substituiert ist,
eine 2-Amino-cyclohexylaminogruppe,
oder eine durch die Reste R¹⁵ und R¹⁶ substituierte Aminogruppe, in der
R¹⁵ eine Methyl- oder Ethylgruppe und
R¹⁶ eine 2-Aminoethylgruppe darstellt, wobei der Ethylteil durch eine oder zwei Methylgruppen oder durch eine Aminocarbonyl-, Methylaminocarbonyl-, Dimethylaminocarbonyl- oder Pyrrolidin-1-ylcarbonylgruppe substituiert sein kann, bedeuten,
wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische und deren Salze.

Eine dritte besonders zu erwähnende Untergruppe der bevorzugten Verbindungen der Formel I betrifft diejenigen Verbindungen der allgemeinen Formel I, in denen
R¹, R² und R³ wie oben erwähnt definiert sind und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Aminocarbonyl-, Methylaminocarbonyl-, Dimethylaminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist,
eine 3-Amino-2-oxo-piperidin-5-yl- oder 3-Amino-2-oxo-1-methyl-piperidin-5-yl-Gruppe,
eine Cyclohexylgruppe, die in 3-Stellung durch eine Aminogruppe substituiert ist,
eine 2-Amino-cyclohexylaminogruppe,
oder eine durch die Reste R¹⁵ und R¹⁶ substituierte Aminogruppe darstellt, in der
R¹⁵ eine Methyl- oder Ethylgruppe und
R¹⁶ eine 2-Aminoethylgruppe darstellt, wobei der Ethylteil durch eine oder zwei Methylgruppen oder durch eine Aminocarbonyl-, Methylaminocarbonyl-, Dimethylaminocarbonyl- oder Pyrrolidin-1-ylcarbonylgruppe substituiert sein kann,
wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische und deren Salze.

Besonders bevorzugte Verbindungen der obigen allgemeinen Formel I sind diejenigen, in denen
R¹ ein Wasserstoffatom,
eine C₁₋₄-Alkylgruppe,
eine C₃₋₅-Alkenylgruppe,
eine 2-Propen-1-ylgruppe, die durch eine Methoxycarbonylgruppe substituiert ist,
eine C₃₋₅-Alkinylgruppe,
eine Phenylgruppe,
eine Phenyl-C₁₋₄-alkylgruppe, in der der Phenylteil durch ein oder zwei Fluoratome, ein oder zwei Chloratome, ein Bromatom, eine bis drei Methylgruppen, eine Butyl-, Trifluormethyl-, Hydroxy-, Methoxy-, Nitro-, Amino-, Carboxy- oder Ethoxycarbonylgruppe substituiert sein kann,
eine 2-Phenylethylgruppe, in der der Ethylteil in 2-Stellung durch eine Hydroxy-, Methoxy- oder Hydroxyiminogruppe substituiert ist,
eine Phenylcarbonylmethylgruppe, in der der Phenylteil durch ein Fluoratom oder durch eine Methyl-, Aminocarbonyl-, Aminosulfonyl-, Cyan-, Hydroxy-, Methoxy-, Phenoxy-, Benzyloxy-, 2-Propen-1-yloxy-, 2-Propin-1-yloxy-, Cyanmethoxy-, (Methoxycarbonyl)methoxy-, (Aminocarbonyl)methoxy-, (Methylaminocarbonyl)-methoxy-, (Dimethylaminocarbonyl)methoxy-, Methylsulfonyloxy-, Phenylsulfonyloxy-, Nitro-, Amino-, (Methoxycarbonyl)methylamino-, Acetylamino-, Methoxycarbonylamino-, Methylsulfonylamino-, Bis-(methylsulfonyl)-amino-, Aminocarbonylamino-, Dimethylaminocarbonylamino-, (Methylamino)thiocarbonylamino-, (Ethoxycarbonyl-amino)carbonylamino- oder Cyanmethylamino-Gruppe substituiert sein kann,
eine Phenylcarbonylmethylgruppe, in der der Phenylteil durch zwei Methoxygruppen oder durch ein Bromatom und durch eine Dimethylaminogruppe substituiert ist,
eine 2-(Phenylcarbonyl)ethylgruppe,
eine 2-Phenylethenylgruppe,
eine 2-(Phenoxy)ethylgruppe,
eine Phenylsulfanylmethyl- oder Phenylsulfinylmethylgruppe,
eine Naphthylmethyl- oder Naphthylethylgruppe,
eine Isoxazolylmethyl-, Thiazolylmethyl-, Pyridylmethyl-, Benzo[d]isoxazolylmethyl-, Benzo[d]isothiazolylmethyl-, (1*H*-Indazol-3-yl)methyl-, Chinolinylmethyl- oder Isochinolinylmethylgruppe, wobei der heterocyclische Teil jeweils durch eine Methylgruppe substituiert sein kann,
eine Isochinolinylmethylgruppe, in der der Isochinolinylteil durch eine Nitro- oder Aminogruppe substituiert ist,
eine (1,2-Dihydro-2-oxo-chinolin-4-yl)methylgruppe,
eine Chromen-4-on-3-ylgruppe,
eine Pyrrolylethyl-, Triazolylethyl-, Thienylethyl-, Thiazolylethyl- oder Pyridylethylgruppe, wobei der heterocyclische Teil jeweils durch eine Methylgruppe substituiert sein kann,
eine Thienylcarbonylmethylgruppe,
eine Methylgruppe, die durch eine Cyclopropyl-, Cyan-, Carboxy-, Aminocarbonyl- oder Methoxycarbonylgruppe substituiert ist,
eine Ethylgruppe, die in 2-Stellung durch eine Hydroxy-, Methoxy-, Dimethylamino-, Carboxy- oder Methoxycarbonylgruppe substituiert ist,
eine Propylgruppe, die in 3-Stellung durch eine Hydroxy-, Dimethylamino-, Carboxy-oder Methoxycarbonylgruppe substituiert ist,
eine 2-Oxopropylgruppe oder
eine Amino- oder Benzoylaminogruppe,
R² ein Wasserstoffatom,
eine C₁₋₆-Alkylgruppe,
eine Ethenylgruppe,
eine 2-Propen-1-yl- oder 2-Propin-1-ylgruppe,
eine Phenylgruppe,
eine Phenyl-C₁₋₄-alkylgruppe, wobei der Phenylteil durch ein Fluoratom, eine Methyl- oder Methoxygruppe substituiert sein kann,
eine Phenylcarbonylmethylgruppe,
eine 2-Phenylethenylgruppe,
eine Methylgruppe, die durch eine Cyclopropyl-, Cyan-, Carboxy- oder Methoxy-carbonylgruppe substituiert ist, oder
eine Ethylgruppe, die in 2-Stellung durch eine Cyan-, Hydroxy-, Methoxy- oder Dimethylaminogruppe substituiert ist,
R³ eine C₄₋₆-Alkenylgruppe,
eine 1-Cyclopenten-1-ylmethyl- oder 1-Cyclohexen-1-ylmethylgruppe,
eine 2-Propin-1-yl-, 2-Butin-1-yl- oder 2-Pentin-1-ylgruppe,
eine Phenylgruppe, die durch ein Fluoratom oder eine Cyan-, Methyl- oder Trifluormethylgruppe substituiert sein kann,
eine Phenylgruppe, die durch zwei Methylgruppen substituiert ist,
eine Naphthylgruppe,
eine Benzylgruppe, in der der Phenylteil durch ein oder zwei Fluoratome, ein lodatom oder eine Cyan-, Nitro- oder Aminogruppe substituiert sein kann,
eine Naphthylmethylgruppe,
eine 2-Phenylethenylgruppe,
eine Furanylmethyl- oder Thienylmethylgruppe oder
eine Cyclopropylmethylgruppe und
R⁴ eine Pyrrolidin-1-ylgruppe, die in 3-Stellung durch eine Aminogruppe substituiert ist,
eine Azetidin-1-ylgruppe, die durch eine Aminomethylgruppe substituiert ist,
eine Pyrrolidin-1-ylgruppe, die durch eine Aminomethylgruppe substituiert ist,
eine Piperidin-1-ylgruppe, die in 3-Stellung oder in 4-Stellung durch eine Amino-, Methylamino-, Dimethylamino- oder [(2-Cyan-pyrrolidin-1-yl)carbonylmethyl]-aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Pyrrolidin-1-yl-carbonylgruppe substituiert ist,
eine 3-Amino-piperidin-1-ylgruppe, in der der Piperidin-1-yl-Teil in 4-Stellung zusätzlich durch eine Hydroxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Piperidin-1-ylgruppe, die durch eine Aminomethylgruppe substituiert ist,
eine Piperidin-3-yl- oder Piperidin-4-yl-gruppe,
eine 1-Amino-piperidin-3-yl- oder 1-Amino-piperidin-4-ylgruppe,
eine Hexahydroazepin-1-yl-gruppe, die in 3-Stellung oder in 4-Stellung durch eine Aminogruppe substituiert ist,
eine Piperazin-1-yl- oder [1,4]Diazepan-1-ylgruppe,
eine [1,4]Diazepan-1-ylgruppe, die in 6-Stellung durch eine Aminogruppe substituiert ist,
eine 3-Aminopropylgruppe,
eine Cyclohexylgruppe, die durch eine Aminogruppe substituiert ist,
eine 2-Amino-cyclopropylaminogruppe,
eine 2-Amino-cyclobutylaminogruppe,
eine 2-Amino-cyclopentylamino- oder 3-Amino-cyclopentylaminogruppe,
eine 2-Amino-cyclohexylamino-, 2-(Methylamino)-cyclohexylamino- oder 3-Amino-cyclohexylaminogruppe,
eine N-(2-Aminocyclohexyl)-methylaminogruppe,
eine durch die Reste R¹⁵ und R¹⁶ substituierte Aminogruppe, in der
R¹⁵ eine Methyl- oder Ethylgruppe und
R¹⁶ eine 2-Aminoethyl- 2-(Methylamino)ethyl- oder 2-(Dimethylamino)ethylgruppe darstellt, wobei der Ethylteil durch eine oder zwei Methylgruppen oder durch eine Aminocarbonyl-, Methylaminocarbonyl-, Dimethylaminocarbonyl- oder Pyrrolidin-1-ylcarbonylgruppe substituiert sein kann,
oder eine Amino- oder Methylaminogruppe, in der das Stickstoffatom durch eine Pyrrolidin-3-yl-, Piperidin-3-yl-, Piperidin-4-yl- oder Piperidin-2-ylmethylgruppe substituiert ist, bedeuten,
wobei soweit nichts anderes erwähnt wurde, die vorstehend erwähnten Alkyl- und Alkenylgruppen geradkettig oder verzweigt sein können,
mit der Maßgabe, daß die Verbindungen
3-Methyl-7-(2-buten-1-yl)-8-(piperazin-1-yl)-xanthin,
3-Methyl-7-(2-methyl-2-propen-1-yl)-8-(piperazin-1-yl)-xanthin,
3-Methyl-7-benzyl-8-(piperazin-1-yl)-xanthin,
1,7-Dibenzyl-3-methyl-8-(piperazin-1-yl)-xanthin und
1,3-Dimethyl-7-(4-fluorbenzyl)-8-(piperazin-1-yl)-xanthin
ausgeschlossen sind,
deren Tautomere, Enantiomere, Diastereomere, deren Gemische und deren Salze.

Eine besonders zu erwähnende Untergruppe der besonders bevorzugten Verbindungen der Formel I betrifft diejenigen Verbindungen der allgemeinen Formel I, in denen R¹ bis R⁴ wie oben erwähnt definiert sind, mit der zusätzlichen Maßgabe, daß die Verbindungen, in denen R⁴ eine gegebenenfalls substituierte Piperazin-1-yl- oder [1,4]Diazepan-1-ylgruppe darstellt, ausgeschlossen sind, deren Tautomere, Enantiomere, Diastereomere, deren Gemische und deren Salze.

Eine zweite besonders zu erwähnende Untergruppe der besonders bevorzugten Verbindungen der Formel I betrifft diejenigen Verbindungen der allgemeinen Formel I, in denen
R¹ ein Wasserstoffatom,
eine C₁₋₄-Alkylgruppe,
eine C₃₋₅-Alkenylgruppe,
eine 2-Propen-1-ylgruppe, die durch eine Methoxycarbonylgruppe substituiert ist,
eine C₃₋₅-Alkinylgruppe,
eine Phenyl-C₁₋₄-alkylgruppe, in der der Phenylteil durch ein oder zwei Fluoratome, ein oder zwei Chloratome, ein Bromatom, eine bis drei Methylgruppen, eine Trifluormethyl-, Hydroxy-, Methoxy-, Nitro-, Amino-, Carboxy- oder Ethoxycarbonylgruppe substituiert sein kann,
eine 2-Phenylethylgruppe, in der der Ethylteil in 2-Stellung durch eine Hydroxy-, Methoxy- oder Hydroxyiminogruppe substituiert ist,
eine Phenylcarbonylmethylgruppe, in der der Phenylteil durch ein Fluoratom oder durch eine Methyl-, Aminocarbonyl-, Aminosulfonyl-, Cyan-, Hydroxy-, Methoxy-, Phenoxy-, Benzyloxy-, 2-Propen-1-yloxy-, 2-Propin-1-yloxy-, Cyanmethoxy-, (Methoxycarbonyl)methoxy-, (Aminocarbonyl)methoxy-, (Methylaminocarbonyl)-methoxy-, (Dimethylaminocarbonyl)methoxy-, Methylsulfonyloxy-, Phenylsulfonyloxy-, Nitro-, Amino-, (Methoxycarbonyl)methylamino-, Acetylamino-, Methoxycarbonylamino-, Methylsulfonylamino-, Bis-(methylsulfonyl)-amino-, Aminocarbonylamino-, Dimethylaminocarbonylamino-, (Methylamino)thiocarbonylamino-, (Ethoxycarbonyl-amino)carbonylamino- oder Cyanmethylamino-Gruppe substituiert sein kann,
eine Phenylcarbonylmethylgruppe, in der der Phenylteil durch zwei Methoxygruppen oder durch ein Bromatom und durch eine Dimethylaminogruppe substituiert ist,
eine 2-(Phenylcarbonyl)ethylgruppe,
eine 2-Phenylethenylgruppe,
eine 2-(Phenoxy)ethylgruppe,
eine Phenylsulfanylmethyl- oder Phenylsulfinylmethylgruppe,
eine Naphthylmethyl- oder Naphthylethylgruppe,
eine Isoxazolylmethyl-, Thiazolylmethyl-, Pyridylmethyl-, Benzo[d]isoxazolylmethyl-, Benzo[d]isothiazolylmethyl-, (1*H*-Indazol-3-yl)methyl-, Chinolinylmethyl- oder Isochinolinylmethylgruppe, wobei der heterocyclische Teil jeweils durch eine Methylgruppe substituiert sein kann,
eine Isochinolinylmethylgruppe, in der der Isochinolinylteil durch eine Nitro- oder Aminogruppe substituiert ist,
eine (1,2-Dihydro-2-oxo-chinolin-4-yl)methylgruppe,
eine Pyrrolylethyl-, Triazolylethyl-, Thienylethyl-, Thiazolylethyl- oder Pyridylethylgruppe, wobei der heterocyclische Teil jeweils durch eine Methylgruppe substituiert sein kann,
eine Thienylcarbonylmethylgruppe,
eine Methylgruppe, die durch eine Cyclopropyl-, Cyan-, Carboxy-, Aminocarbonyl- oder Methoxycarbonylgruppe substituiert ist,
eine Ethylgruppe, die in 2-Stellung durch eine Hydroxy-, Methoxy-, Dimethylamino-, Carboxy- oder Methoxycarbonylgruppe substituiert ist,
eine Propylgruppe, die in 3-Stellung durch eine Hydroxy-, Dimethylamino-, Carboxy- oder Methoxycarbonylgruppe substituiert ist,
eine 2-Oxopropylgruppe oder
eine Amino- oder Benzoylaminogruppe,
R² ein Wasserstoffatom,
eine C₁₋₆-Alkylgruppe,
eine Ethenylgruppe,
eine 2-Propen-1-yl- oder 2-Propin-1-ylgruppe,
eine Phenylgruppe,
eine Phenyl-C₁₋₄-alkylgruppe, wobei der Phenylteil durch ein Fluoratom, eine Methyl- oder Methoxygruppe substituiert sein kann,
eine Phenylcarbonylmethylgruppe,
eine 2-Phenylethenylgruppe,
eine Methylgruppe, die durch eine Cyclopropyl-, Cyan-, Carboxy- oder Methoxycarbonylgruppe substituiert ist, oder
eine Ethylgruppe, die in 2-Stellung durch eine Cyan-, Hydroxy-, Methoxy- oder Dimethylaminogruppe substituiert ist,
R³ eine C₄₋₆-Alkenylgruppe,
eine 1-Cyclopenten-1-ylmethyl- oder 1-Cyclohexen-1-ylmethylgruppe,
eine 2-Propin-1-yl-, 2-Butin-1-yl- oder 2-Pentin-1-ylgruppe,
eine Phenylgruppe, die durch ein Fluoratom oder eine Cyan-, Methyl- oder Trifluormethylgruppe substituiert sein kann,
eine Phenylgruppe, die durch zwei Methylgruppen substituiert ist,
eine Benzylgruppe, in der der Phenylteil durch ein oder zwei Fluoratome, ein lodatom oder eine Cyan-, Nitro- oder Aminogruppe substituiert sein kann,
eine Furanylmethyl- oder Thienylmethylgruppe oder
eine Cyclopropylmethylgruppe und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Pyrrolidin-1-yl-carbonylgruppe substituiert ist,
eine 3-Amino-piperidin-1-ylgruppe, in der der Piperidin-1-yl-Teil in 4-Stellung zusätzlich durch eine Hydroxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist,
eine [1,4]Diazepan-1-ylgruppe, die in 6-Stellung durch eine Aminogruppe substituiert ist,
eine Cyclohexylgruppe, die in 3-Stellung durch eine Aminogruppe substituiert ist,
eine 2-Amino-cyclohexylaminogruppe,
oder eine durch die Reste R¹⁵ und R¹⁶ substituierte Aminogruppe, in der
R¹⁵ eine Methyl- oder Ethylgruppe und
R¹⁶ eine 2-Aminoethylgruppe darstellt, wobei der Ethylteil durch eine oder zwei Methylgruppen oder durch eine Aminocarbonyl-, Methylaminocarbonyl-, Dimethylaminocarbonyl- oder Pyrrolidin-1-ylcarbonylgruppe substituiert sein kann, bedeutet,
wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische und deren Salze.

Eine dritte besonders zu erwähnende Untergruppe der besonders bevorzugten Verbindungen der Formel I betrifft diejenigen Verbindungen der allgemeinen Formel I, in denen
R¹, R² und R³ wie oben erwähnt definiert sind und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Pyrrolidin-1-yl-carbonylgruppe substituiert ist,
eine 3-Amino-piperidin-1-ylgruppe, in der der Piperidin-1-yl-Teil in 4-Stellung zusätzlich durch eine Hydroxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist,
eine Cyclohexylgruppe, die in 3-Stellung durch eine Aminogruppe substituiert ist,
eine 2-Amino-cyclohexylaminogruppe,
oder eine durch die Reste R¹⁵ und R¹⁶ substituierte Aminogruppe bedeutet, in der
R¹⁵ eine Methyl- oder Ethylgruppe und
R¹⁶ eine 2-Aminoethylgruppe darstellt, wobei der Ethylteil durch eine oder zwei Methylgruppen oder durch eine Aminocarbonyl-, Methylaminocarbonyl-, Dimethylaminocarbonyl- oder Pyrrolidin-1-ylcarbonylgruppe substituiert sein kann,
wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische und deren Salze.
Als eine weitere Untergruppe von Verbindungen der allgemeinen Formel I sind diejenigen zu nennen, in denen
R¹ ein Wasserstoffatom,
eine C₁₋₈-Alkylgruppe,
eine C₃₋₈-Alkenylgruppe,
eine C₃₋₈-Alkinylgruppe,
eine durch eine Gruppe Rₐ substituierte C₁₋₆-Alkylgruppe, wobei
Rₐ eine C₃₋₇-Cycloalkyl-, Heteroaryl-, Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonylgruppe bedeutet,
eine durch eine Phenylgruppe substituierte C₁₋₆-Alkylgruppe, wobei der Phenylring durch die Gruppen R¹⁰ bis R¹⁴ substituiert ist und
R¹⁰ ein Wasserstoffatom,
ein Fluor-, Chlor-, Brom- oder lodatom,
eine C₁₋₄-Alkyl-, Hydroxy-, oder C₁₋₄-Alkyloxygruppe,
eine Nitro-, Amino-, C₁₋₃-Alkylamino-, Di-(C₁₋₃-alkyl)amino-, Pyrrolidin-1-yl-, Piperidin-1-yl-, Morpholin-4-yl-, Piperazin-1-yl-, 4-(C₁₋₃-Alkyl)-piperazin-1-yl-, C₁₋₃-Alkyl-carbonylamino-, Arylcarbonylamino-, Aryl-C₁₋₃-alkyl-carbonylamino-, C₁₋₃-Alkyloxy-carbonylamino-, Aminocarbonylamino-, C₁₋₃-Alkyl-aminocarbonylamino-, Di-(C₁₋₃-alkyl)aminocarbonylamino-,C₁₋₃-Alkyl-sulfonylamino-, Aryl-sulfonylamino- oder Aryl-C₁₋₃-alkyl-sulfonylaminogruppe,
eine N-(C₁₋₃-Alkyl)-C₁₋₃-alkyl-carbonylamino-, N-(C₁₋₃-Alkyl)-arylcarbonylamino-, N-(C₁₋₃-Alkyl)-aryl-C₁₋₃-alkyl-carbonylamino-, N-(C₁₋₃-Alkyl)-C₁₋₃-alkyloxy-carbonylamino-, N-(Aminocarbonyl)-C₁₋₃-alkylamino-, N-(C₁₋₃-Alkyl-aminocarbonyl)-C₁₋₃-alkylamino -, N-[Di-(C₁₋₃-alkyl)aminocarbonyl]-C₁₋₃-alkylamino-, N-(C₁₋₃-Alkyl)-C₁₋₃-alkyl-sulfonylamino-, N-(C₁₋₃-Alkyl)-aryl-sulfonylamino-, oder N-(C₁₋₃-Alkyl)-aryl-C₁₋₃-alkyl-sulfonylaminogruppe,
eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl-oder 4-(C₁₋₃-Alkyl)-piperazin-1-yl-carbonylgruppe,
   eine C₁₋₃-Alkyl-carbonyl- oder eine Arylcarbonylgruppe,
   eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1-yl-carbonyl-C₁₋₃-alkyl- oder 4-(C₁₋₃-Alkyl)-piperazin-1-yl-carbonyl-C₁₋₃-alkyl-gruppe,
eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy-, C₁₋₃-Alkyl-aminocarbonyl-C₁₋₃-alkyloxy-, Di-(C₁₋₃-alkyl)-aminocarbonyl-C₁₋₃-alkyloxy-, Pyrrolidin-1-yl-carbonyl-C₁₋₃-alkyl-oxy-, Piperidin-1-yl-carbonyl-C₁₋₃-alkyloxy-, Morpholin-4-yl-carbonyl-C₁₋₃-alkyl-oxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
eine Hydroxy-C₁₋₃-alkyl-, C₁₋₃-Alkyloxy-C₁₋₃-alkyl-, Amino-C₁₋₃-alkyl-, C₁₋₃-Alkyl-amino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl-, 4-(C₁₋₃-Alkyl)-piperazin-1-yl-C₁₋₃-alkylgruppe,
eine Hydroxy-C₁₋₃-alkyloxy-, C₁₋₃-Alkyloxy-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy-, 4-(C₁₋₃-Alkyl)-piperazin-1-yl-C₁₋₃-alkyloxygruppe,
eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Trifluormethylsulfanyl-, Trifluormethylsulfinyl- oder Trifluormethylsulfonylgruppe,
eine Sulfo-, Aminosulfonyl-, C₁₋₃-Alkyl-aminosulfonyl-, Di-(C₁₋₃-alkyl)-amino-sulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonyl-gruppe,
eine durch 1 bis 3 Fluoratome substituierte Methyl- oder Methoxygruppe,
eine durch 1 bis 5 Fluoratome substituierte Ethyl- oder Ethoxygruppe,
eine C₂₋₄-Alkenyl- oder C₂₋₄-Alkinylgruppe,
eine 2-Propen-1-yloxy- oder 2-Propin-1-yloxygruppe,
eine C₃₋₆-Cycloalkyl- oder C₃₋₆-Cycloalkyloxygruppe,
eine C₃₋₆-Cycloalkyl-C₁₋₃-alkyl- oder C₃₋₆-Cycloalkyl-C₁₋₃-alkyloxygruppe oder
eine Aryl-, Aryloxy-, Aryl-C₁₋₃-alkyl- oder Aryl-C₁₋₃-alkyloxygruppe,
R¹¹ und R¹², die gleich oder verschieden sein können, jeweils ein Wasserstoffatom, ein Fluor-, Chlor-, Brom- oder lodatom, eine C₁₋₃-Alkyl-, Trifluormethyl-, Hydroxy-, oder C₁₋₃-Alkyloxygruppe oder eine Cyangruppe, oder
R¹¹ zusammen mit R¹², sofern diese an benachbarte Kohlenstoffatome gebunden sind, auch eine Methylendioxy-,Difluormethylendioxy-, geradkettige C₃₋₅-Alkylen-, -CH=CH-CH=CH-, -CH=CH-CH=N- oder -CH=CH-N=CH-Gruppe und
R¹³ und R¹⁴, die gleich oder verschieden sein können, jeweils ein Wasserstoffatom, ein Fluor-, Chlor- oder Bromatom, eine Trifluormethyl-, C₁₋₃-Alkyl- oder C₁₋₃-Alkyloxygruppe bedeuten,
eine durch die Gruppen R¹⁰ bis R¹⁴ substituierte Phenylgruppe, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴ wie vorstehend erwähnt definiert sind und
A eine Carbonyl-, Cyaniminomethylen-, Hydroxyiminomethylen- oder C₁₋₃-Alkyloxyiminomethylen-Gruppe, m die Zahl 0, 1 oder 2 und n die Zahl 1, 2 oder 3 bedeuten,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, m und n wie vorstehend erwähnt definiert sind und
B eine Methylengruppe, die durch eine Hydroxy-, C₁₋₃-Alkyloxy-, Amino-, C₁₋₃-Alkylamino-, Di-(C₁₋₃-alkyl)-amino-, Mercapto-, C₁₋₃-Alkyisulfanyl-, C₁₋₃-Alkylsulfinyl- oder C₁₋₃-Alkylsulfonylgruppe substituiert ist und gegebenenfalls zusätzlich durch eine Methyl- oder Ethylgruppe substituiert ist, bedeutet,
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₃-Alkyloxycarbonyl-, Aminocarbonyl-, C₁₋₃-Alkylaminocarbonyl-, Di-(C₁₋₃-alkyl)aminocarbonyl-, Pyrrolidin-1-yl-carbonyl-, Piperidin-1-yl-carbonyl- oder Morpholin-4-yl-carbonyl-, Piperazin-1-yl-carbonyl-, 4-Methylpiperazin-1-yl-carbonyl- oder 4-Ethylpiperazin-1-yl-carbonyl-Gruppe bedeutet und A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴ und m wie vorstehend erwähnt sind und D
eine Sauerstoff- oder Schwefelatom, eine Imino-, C₁₋₃-Alkylimino-, Sulfinyl- oder Sulfonylgruppe bedeutet,
eine durch eine Gruppe R_{b} substituierte C₂₋₆-Alkylgruppe, wobei
R_{b} durch mindestens zwei Kohlenstoffatome vom Ringstickstoffatom in 1-Stellung des Xanthingerüstes isoliert ist undR_{b} eine Hydroxy-, C₁₋₃-Alkyloxy-, Mercapto-, C₁₋₃-Alkylsuffanyl-, C₁₋₃-Alkylsulfinyl-, C₁₋₃-Alkylsulfonyl-, Amino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl- oder 4-(C₁₋₃-Alkyl)-piperazin-1-ylgruppe bedeutet,
oder eine C₃₋₆-Cycloalkylgruppe,
R² ein Wasserstoffatom,
eine C₁₋₈-Alkylgruppe,
eine C₃₋₆-Alkenylgruppe,
eine C₃₋₆-Alkinylgruppe,
eine durch eine Gruppe Rₐ substituierte C₁₋₆-Alkylgruppe, wobei Rₐ wie vorstehend erwähnt definiert ist,
eine durch eine Phenylgruppe substituierte C₁₋₆-Alkylgruppe, wobei der Phenylring durch die Gruppen R¹⁰ bis R¹⁴ substituiert ist und R¹⁰ bis R¹⁴ wie vorstehend erwähnt definiert sind,
eine durch die Gruppen R¹⁰ bis R¹⁴ substituierte Phenylgruppe, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴, A, m und n wie vorstehend erwähnt definiert sind,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, B, m und n wie vorstehend erwähnt definiert sind,
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹, A und n wie vorstehend erwähnt definiert sind,
eine Phenyl-(CH2)ₘ-D-C₁₋₃-alkylgruppe, in der der Phenylteil durch die Gruppen R¹⁰ bis R¹⁴ substituiert ist, wobei R¹⁰ bis R¹⁴, m und D wie vorstehend erwähnt sind,
eine durch eine Gruppe R_{b} substituierte C₂₋₆-Alkylgruppe, wobei
R_{b} durch mindestens zwei Kohlenstoffatome vom Ringstickstoffatom in 3-Stellung des Xanthingerüstes isoliert ist und wie vorstehend erwähnt definiert ist,
oder eine C₃₋₆-Cycloalkylgruppe,
R³ eine C₁₋₈-Alkylgruppe,
eine durch die Gruppe R_{c} substituierte C₁₋₄-Alkylgruppe, wobei
R_{c} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇-Cycloalkenylgruppe odereine Aryl- oder Heteroarylgruppe bedeutet,
eine C₃₋₈-Alkenylgruppe,
eine durch ein Fluor-, Chlor- oder Bromatom, oder eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine RₑNR_{d}-Gruppe substituiert ist und zusätzlich durch eine oder zwei C₁₋₃-Alkylgruppen substituiert sein kann, wobei
Rₑ ein Wasserstoffatom oder eine C₁₋₃-Alkylgruppe und
R_{d} ein Wasserstoffatom, eine C₁₋₃-Alkylgruppe, eine R_{f}-C₁₋₃-alkylgruppe oder eine R_{g}-C₂₋₃-alkylgruppe bedeutet, wobei
R_{f} eine Carboxy-, C₁₋₃-Alkyloxy-carbonyl-, Aminocarbonyl-, C₁₋₃-Alkylamino-carbonyl-, Di-(C₁₋₃-alkyl)-aminocarbonyl-, Pyrrolidin-1-yl-carbonyl-, 2-Cyan-pyrrolidin-1-yl-carbonyl-, 2-Carboxypyrrolidin-1-yl-carbonyl-, 2-Methoxy-carbonylpyrrolidin-1-yl-carbonyl-, 2-Ethoxycarbonylpyrrolidin-1-yl-carbonyl-, 2-Aminocarbonylpyrrolidin-1-yl-carbonyl-, 4-Cyanthiazolidin-3-yl-carbonyl-, 4-Carboxythiazolidin-3-yl-carbonyl-, 4-Methoxycarbonylthiazolidin-3-yl-carbonyl-, 4-Ethoxycarbonylthiazolidin-3-yl-carbonyl-, 4-Aminocarbonylthiazolidin-3-yl-carbonyl-, Piperidin-1-yl-carbonyl-, Morpholin-4-yl-carbonyl-, Piperazin-1-yl-carbonyl-, 4-Methyl-piperazin-1-yl-carbonyl- oder 4-Ethyl-piperazin-1-yl-carbonyl-gruppe bedeutet und
R_{g}, das mindestens durch zwei Kohlenstoffatome vom Stickstoffatom der RₑNR_{d}-Gruppe getrennt ist, eine Hydroxy-, Methoxy- oder Ethoxygruppe bedeutet,
eine Piperidin-1-yl- oder Hexahydroazepin-1-ylgruppe, die in 3-Stellung oder in 4-Stellung durch eine RₑNR_{d}-Gruppe substituiert ist und zusätzlich durch eine oder zwei C₁₋₃-Alkylgruppen substituiert sein kann, wobei Rₑ und R_{d} wie vorstehend erwähnt definiert sind,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1 yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁-₃-Alkylamino-C₁-₃-alkyl- oder eine Di-(C₁₋₃-alkyl)amino-C₁₋₃-alkylgruppe substituiert ist,
eine C₃-₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine N-(C₃₋₇-Cycloalkyl)-N-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eineC₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-,C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine N-(C₃₋₇-Cycloalkyl-C₁₋₂-alkyl)-N-(C₁₋₂-alkyl)-aminogruppe in der der Cycloalkyl-teil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl-C₁₋₂-alkyl)-N-(C₁₋₂-alkyl)-aminogruppe, in der der Cycloalkyl-teil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine durch die Reste R¹⁵ und R¹⁶ substituierte Aminogruppe, in der
R¹⁵ eine C₁₋₆-Alkylgruppe, eine C₃₋₆-Cycloalkyl-, C₃₋₆-Cycloalkyl-C₁₋₃-alkyl-, Aryl- oder Aryl-C₁₋₃-alkylgruppe und
R¹⁶ eine R¹⁷-C₂₋₃-alkylgruppe darstellt, wobei der C₂₋₃-Alkylteil geradkettig ist und durch ein bis vier C₁₋₃-Alkylgruppen, die gleich oder verschieden sein können, substituiert sein kann und
R¹⁷ eine Amino-, C₁₋₃-Alkylamino- oder Di-(C₁₋₃-alkyl)-aminogruppe darstellt, wobei, falls R³ eine Methylgruppe bedeutet, R¹⁷ keine Di-(C₁₋₃-alkyl)-aminogruppe darstellen kann,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch die Reste R¹⁵ und R²⁰ substituierte Aminogruppe, in der
R¹⁵ und R²⁰ wie vorstehend erwähnt definiert sind, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und durch den Rest R¹⁵ substituiert sein kann und zusätzlich durch eine oder zwei C₁₋₃-Alkylgruppen substituiert sein kann, wobei R¹⁵ wie vorstehend erwähnt definiert ist und R¹⁹ eine Amino-, C₁₋₃-Alkylamino- oder Di-(C₁₋₃-alkyl)-aminogruppe darstellt ,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino-oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinyl-gruppe zu verstehen ist, in der eine bis drei Methingruppen durch Stickstoffatome ersetzt sind,
wobei die fünfgliedrigen Gruppen oder Molekülteile jeweils durch eine C₁₋₃-Alkyl- oder Trifluormethylgruppe substituiert sein können und
die sechsgliedrigen Gruppen oder Molekülteile jeweils durch eine oder zwei C₁₋₃-Alkylgruppen oder durch ein Fluor-, Chlor-, Brom- oder lodatom, durch eine Trifluormethyl-, Hydroxy-, C₁₋₃-Alkyloxy-, Difluormethoxy- oder Trifluormethoxygruppe substituiert sein können,
wobei, soweit nichts anderes erwähnt wurde, die vorstehend erwähnten Alkyl-, Alkenyl- und Alkinylgruppen geradkettig oder verzweigt sein können,
sowie die am Ringstickstoffatom in 9-Stellung des Xanthingerüstes N-oxidierten oder methylierten oder ethylierten Derivate,
mit der Maßgabe, daß die Verbindungen, in denen
R¹ ein Wasserstoffatom, eine Methyl-, Propyl-, 2-Hydroxypropyl-, Aminocarbonylmethyl- oder Benzylgruppe,
R² eine Methylgruppe,
R³ eine C₁₋₈-Alkylgruppe, eine gegenenfalls durch ein Fluor-, Chlor- oder Bromatom oder eine Methylgruppe substituierte Benzylgruppe, eine 1-Phenylethyl- oder 2-Phenylethylgruppe, eine 2-Propen-1-yl-, 2-Buten-1-yl-, 3-Chlor-2-buten-1-yl- oder 2-Methyl-2-propen-1-ylgruppe
und
R⁴ eine Piperazin-1-ylgruppe darstellen, ausgeschlossen sind,
und mit der Maßgabe, daß die Verbindungen, in denen
R¹ ein Wasserstoffatom oder eine Methylgruppe,
R² ein Wasserstoffatom oder eine Methylgruppe,
R³ eine Methylgruppe
und
R⁴ eine 3-Aminopropyl-, 3-[Di-(C₁₋₃-alkyl)aminol-propyl-, 1-Phenyl-3-[di-(C₁₋₃-alkyl)-amino]-propyl-, 1-Phenyl-3-methyl-3-(dimethylamino)-propyl-, 1-(4-Chlorphenyl)-3-(dimethylamino)-propyl-, 1-Phenyl-2-methyl-3-(dimethylamino)-propyl-, 1-(3-Methoxyphenyl)-3-(dimethylamino)-propyl- oder eine 4-Aminobutylgruppe darstellen, ausgeschlossen sind,
und mit der Maßgabe, daß die Verbindung
1,3,7-Trimethyl-8-(1-aminocyclohexyl)-xanthin
ausgeschlossen ist,
deren Isomere und deren Salze.

Beispielsweise seien folgende bevorzugte Verbindungen erwähnt:
(1) 1,3-Dimethyl-7-benzyl-8-(3-amino-pyrrolidin-1-yl)-xanthin,
(2) 1,3-Dimethyl-7-(3-methyl-2-buten-1-yl)-8-(3-amino-pyrrolidin-1-yl)-xanthin,
(3) 1,3-Dimethyl-7-benzyl-8-(3-amino-piperidin-1-yl)-xanthin,
(4) 1,3-Dimethyl-7-(3-methyl-2-buten-1-yl)-8-[(trans-2-amino-cyclohexyl)amino]-xanthin,
(5) 1,3-Dimethyl-7-(3-methyl-2-buten-1-yl)-8-(3-amino-piperidin-1-yl)-xanthin,
(6) 1,3-Dimethyl-7-(3-methyl-2-buten-1-yl)-8-(4-amino-piperidin-1-yl)-xanthin,
(7) 1,3-Dimethyl-7-(3-methyl-2-buten-1-yl)-8-[(cis-2-amino-cyclohexyl)amino]-xanthin,
(8) 1,3-Dimethyl-7-(2-butin-1-yl)-8-(3-amino-piperidin-1-yl)-xanthin,
(9) 1,3-Dimethyl-7-[(1-cyclopenten-1-yl)methyl]-8-(3-amino-piperidin-1-yl)-xanthin,
(10) 1,3-Dimethyl-7-(2-thienylmethyl)-8-(3-amino-piperidin-1-yl)-xanthin,
(11) 1,3-Dimethyl-7-(3-fluorbenzyl)-8-(3-amino-piperidin-1-yl)-xanthin,
(12) 1,3-Dimethyl-7-(2-fluorbenzyl)-8-(3-amino-piperidin-1-yl)-xanthin,
(13) 1,3-Dimethyl-7-(4-fluorbenzyl)-8-(3-amino-piperidin-1-yl)-xanthin,
(14) 1,3-Dimethyl-7-(2-buten-1-yl)-8-(3-amino-piperidin-1-yl)-xanthin,
(15) 1,3-Bis-(cyclopropylmethyl)-7-benzyl-8-(3-amino-piperidin-1-yl)-xanthin,
(16) (R)-1,3-Dimethyl-7-(3-methyl-2-buten-1-yl)-8-(3-amino-piperidin-1-yl)-xanthin,
(17) (S)-1,3-Dimethyl-7-(3-methyl-2-buten-1-yl)-8-(3-amino-piperidin-1-yl)-xanthin,
(18) 1,3-Dimethyl-7-(3-methyl-2-buten-1-yl)-8-(3-amino-hexahydroazepin-1-yl)-xanthin,
(19) 1,3-Dimethyl-7-(3-methyl-2-buten-1-yl)-8-(4-amino-hexahydroazepin-1-yl)-xanthin,
(20) 1,3-Dimethyl-7-(3-methyl-2-buten-1-yl)-8-(cis-3-amino-cyclohexyl)-xanthin-hydrochlorid,
(21) 1,3-Dimethyl-7-(3-methyl-2-buten-1-yl)-8-(3-methylamino-piperidin-1-yl)-xanthin,
(22) 1-(2-Phenylethyl)-3-methyl-7-(3-methyl-2-buten-1-yl)-8-(3-amino-piperidin-1-yl)-xanthin ,
(23) 1,3-Dimethyl-7-(3-methyl-2-buten-1-yl)-8-[N-(2-aminoethyl)-methylamino]-xanthin,
(24) 1-[2-(Thiophen-2-yl)-ethyl]-3-methyl-7-(3-methyl-2-buten-1-yl)-8-(3-amino-piperidin-1-yl)-xanthin,
(25) 1-[2-(Thiophen-3-yl)-ethyl]-3-methyl-7-(3-methyl-2-buten-1-yl)-8-(3-amino-piperidin-1-yl)-xanthin,
(26) 1-[2-(2-Methyl-phenyl)-ethyl]-3-methyl-7-(3-methyl-2-buten-1-yl)-8-(3-amino-piperidin-1-yl)-xanthin,
(27) 1-[2-(3-Methyl-phenyl)-ethyl]-3-methyl-7-(3-methyl-2-buten-1-yl)-8-(3-amino-piperidin-1-yl)-xanthin,
(28) 1-[2-(3-Methoxy-phenyl)-ethyl]-3-methyl-7-(3-methyl-2-buten-1-yl)-8-(3-amino-piperidin-1 -yl)-xanthin,
(29) 1-((E)-2-Phenyl-vinyl)-3-methyl-7-(3-methyl-2-buten-1-yl)-8-(3-amino-piperidin-1-yl)-xanthin,
(30) 1-(2-Phenyl-ethyl)-3-methyl-7-(3-methyl-2-buten-1-yl)-8-((S)-3-amino-piperidin-1-yl)-xanthin,
(31) 1-(2-Phenyl-ethyl)-3-methyl-7-(3-methyl-2-buten-1-yl)-8-((*R*)-3-amino-piperidin-1-yl)-xanthin,
(32) 1-[2-(2-Methoxy-phenyl)-2-oxo-ethyl]-3-methyl-7-(3-methyl-2-buten-1-yl)-8-(3-amino-piperidin-1-yl)-xanthin,
(33) 1-[2-(Thiophen-3-yl)-2-oxo-ethyl]-3-methyl-7-(3-methyl-2-buten-1-yl)-8-(3-amino-piperidin-1-yl)-xanthin,
(34) 1-(2-Phenyl-2-oxo-ethyl)-3-methyl-7-(3-methyl-2-buten-1-yl)-8-((*S*)-3-amino-piperidin-1-yl)-xanthin,
(35) 1-(2-Phenyl-2-oxo-ethyl)-3-methyl-7-(3-methyl-2-buten-1-yl)-8-((*R*)-3-amino-piperidin-1-yl)-xanthin,
36) 1-[(Isochinolin-1-yl)methyl]-3-methyl-7-(3-methyl-2-buten-1-yl)-8-((*R*)-3-amino-piperidin-1-yl)-xanthin,
(37) 1-[(Isochinolin-1-yl)methyl]-3-methyl-7-(3-methyl-2-buten-1-yl)-8-((*S*)-3-amino-piperidin-1-yl)-xanthin und
(38) 1-[(1-Naphthyl)methyl]-3-methyl-7-(3-methyl-2-buten-1-yl)-8-(3-amino-piperidin-1-yl)-xanthin
sowie deren Salze.

Erfindungsgemäß erhält man die Verbindungen der allgemeinen Formel I nach an sich bekannten Verfahren, beispielsweise nach folgenden Verfahren:
a) Zur Herstellung von Verbindungen der allgemeinen Formel I, in der R⁴ einer der eingangs erwähnten, über ein Stickstoffatom mit dem Xanthingerüst verknüpften Reste ist:
   Umsetzung einer Verbindung der allgemeinen Formel 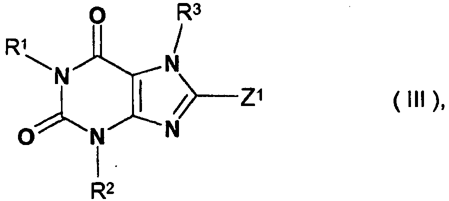 in der
      R¹ bis R³ wie eingangs erwähnt definiert sind und Mercapto-, Z¹ eine Austrittsgruppe wie ein Halogenatom, eine substituierte Hydroxy-, Mercapto-, Sulfinyl-, Sulfonyl- oder Sulfonyloxygruppe wie ein Chlor- oder Bromatom, eine Methansulfonyl- oder Methansulfonyloxygruppe darstellt, mit einer Verbindung der allgemeinen Formel

      H - R⁴' (IV),

      in der
      R⁴' einen der für R⁴ eingangs erwähnten Reste darstellt, der über ein Stickstoffatom mit dem Xanthingerüst der allgemeinen Formel I verknüpft ist.
      Die Umsetzung wird zweckmäßigerweise in einem Lösungsmittel wie Isopropanol, Butanol, Tetrahydrofuran, Dioxan, Toluol, Chlorbenzol, Dimethylformamid, Dimethylsulfoxid, Methylenchlorid, Ethylenglycolmonomethylether, Ethylenglycoldiethylether oder Sulfolan gegebenenfalls in Gegenwart einer anorganischen oder tertiären organischen Base, z.B. Natriumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid oder einem Katalysator auf Palladiumbasis bei Temperaturen zwischen -20 und 180°C, vorzugsweise jedoch bei Temperaturen zwischen -10 und 120°C, durchgeführt. Die Umsetzung kann jedoch auch ohne Lösungsmittel oder in einem Überschuß der eingesetzten Verbindung der allgemeinen Formel IV durchgeführt werden.
b) Zur Herstellung einer Verbindung der allgemeinen Formel I, in der R⁴ gemäß der eingangs erwähnten Definition eine Aminogruppe oder eine gegebenenfalls im Alkylteil substituierte Alkylaminogruppe enthält:
   Entschützung einer Verbindung der allgemeinen Formel 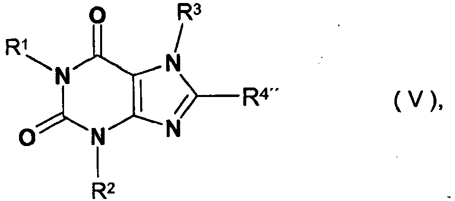 in der R¹, R² und R³ wie eingangs definiert sind und
      R⁴" eine N-tert.-Butyloxycarbonylaminogruppe oder eine N-tert.-Butyloxycarbonyl-N-alkylaminogruppe enthält, wobei der Alkylteil der N-tert.-Butyloxycarbonyl-N-alkyl-aminogruppe wie eingangs erwähnt substituiert sein kann.
      Die Abspaltung des tert.-Butyloxycarbonylrestes erfolgt vorzugsweise durch Behandlung mit einer Säure wie Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol oder Diethylether bei Temperaturen zwischen 0 und 80°C.
c) Zur Herstellung einer Verbindung der allgemeinen Formel I, in der R² gemäß der eingangs erwähnten Definition ein Wasserstoffatom darstellt:
   Entschützung einer Verbindung der allgemeinen Formel 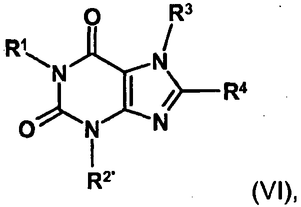 in der R¹, R³ und R⁴ wie eingangs definiert sind und R²' eine Schutzgruppe wie eine Methoxymethyl-, Benzyloxymethyl-, Methoxyethoxymethyl- oder 2-(Trimethylsilyl)-ethyloxymethyl-Gruppe darstellt.
      Die Abspaltung des Schutzrestes erfolgt beispielsweise mit Hilfe einer Säure wie Essigsäure, Trifluoressigsäure, Salzsäure, Schwefelsäure oder eines sauren lonenaustauschers in einem Lösemittel wie Methylenchlorid, Tetrahydrofuran, Methanol, Ethanol oder Isopropanol oder deren Gemischen, wobei die 2-(Trimethylsilyl)-ethyloxymethyl-Gruppe auch mit Hilfe von Fluorwasserstoffsäure oder einem Salz der Fluorwasserstoffsäure wie dem Tetrabutylammoniumfluorid abgespalten werden kann.

Erhält man erfindungsgemäß eine Verbindung der allgemeinen Formel I, die eine Amino-, Alkylamino- oder Iminogruppe enthält, so kann diese mittels Acylierung oder Sulfonylierung in eine entsprechende Acyl- oder Sulfonylverbindung der allgemeinen Formel I übergeführt werden;
erhält man eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden;
erhält man eine Verbindung der allgemeinen Formel I, die eine Nitrogruppe enthält, so kann diese mittels Reduktion in eine entsprechende Aminoverbindung übergeführt werden;
erhält man eine Verbindung der allgemeinen Formel I, die eine Iminogruppe enthält, so kann diese mittels Nitrosierung und anschließender Reduktion in eine entsprechende N-Amino-iminoverbindung übergeführt werden;
erhält man eine Verbindung der allgemeinen Formel I, die eine C₁₋₃-Alkyloxy-carbonylgruppe enthält, so kann diese mittels Esterspaltung in die entsprechende Carboxyverbindung übergeführt werden;
erhält man eine Verbindung der allgemeinen Formel I, in der R¹ eine Carbonylgruppe enthält, so kann diese beispielsweise mittels Reaktion mit Hydroxylamin in ein entsprechendes Oxim der allgemeinen Formel I übergeführt werden;
erhält man eine Verbindung der allgemeinen Formel I, die eine Carboxygruppe enthält, so kann diese mittels Veresterung in einen entsprechenden Ester der allgemeinen Formel I übergeführt werden; oder
erhält man eine Verbindung der allgemeinen Formel I, die eine Carboxy- oder Estergruppe enthält, so kann diese durch Umsetzung mit einem Amin in ein entsprechendes Amid der allgemeinen Formel I übergeführt werden.

Die nachträgliche Veresterung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Esterbildung kann auch durch Umsetzung einer Verbindung, die eine Carboxygruppe enthält, mit einem entsprechenden Alkylhalogenid erfolgen.

Die nachträgliche Acylierung oder Sulfonylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem entsprechenden Acyl- oder Sulfonylderivat gegebenenfalls in Gegenwart einer tertiären organischen Base oder in Gegenwart einer anorganischen Base oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Alkylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem Alkylierungsmittel wie einem entsprechenden Halogenid oder Sulfonsäureester, z.B. mit Methyljodid, Ethylbromid, Dimethylsulfat oder Benzylchlorid, gegebenenfalls in Gegenwart einer tertiären organischen Base oder in Gegenwart einer anorganischen Base zweckmäßigerweise bei Temperaturen zwischen 0 und 150°C, vorzugsweise bei Temperaturen zwischen 0 und 100°C, durchgeführt.

Die nachträgliche reduktive Alkylierung wird mit einer entsprechenden Carbonylverbindung wie Formaldehyd, Acetaldehyd, Propionaldehyd, Aceton oder Butyraldehyd in Gegenwart eines komplexen Metallhydrids wie Natriumborhydrid, Lithiumborhydrid, Natriumtriacetoxyborhydrid oder Natriumcyanoborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 1 bis 5 bar durchgeführt. Die Methylierung kann auch in Gegenwart von Ameisensäure als Reduktionsmittel bei erhöhten Temperaturen, z.B. bei Temperaturen zwischen 60 und 120°C, durchgeführt werden.

Die nachträgliche Reduktion einer Nitrogruppe erfolgt beispielsweise mit Wasserstoff und einem Katalysator wie Palladium auf Aktivkohle, Platindioxid oder Raney-Nickel, oder mit Hilfe anderer Reduktionsmittel wie Eisen oder Zink in Gegenwart einer Säure wie Essigsäure.

Die nachträgliche Nitrosierung einer Iminogruppe mit nachfolgender Reduktion zur N-Amino-iminoverbindung erfolgt beispielsweise so, daß die Iminoverbindung mit einem Alkylnitrit wie Isoamylnitrit nitrosiert wird und die gebildete N-Nitrosoiminoverbindung anschließend direkt zur N-Amino-iminoverbindung reduziert wird, wozu sich beispielsweise Zink in Gegenwart einer Säure wie Essigsäure eignet.

Die nachträgliche Spaltung einer C₁₋₃-Alkyloxycarbonylgruppe zur Carboxygruppe erfolgt beispielsweise hydrolytisch mit einer Säure wie Salzsäure oder Schwefelsäure oder eines Alkalihydroxids wie Lithiumhydroxid, Natriumhydroxid oder Kaliumhydroxid.

Die nachträgliche Amidbildung wird durch Umsetzung eines entsprechenden reaktionsfähigen Carbonsäurederivates mit einem entsprechenden Amin gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan, wobei das eingesetzte Amin gleichzeitig als Lösungsmittel dienen kann, gegebenenfalls in Gegenwart einer tertiären organischen Base oder in Gegenwart einer anorganischen Base oder mit einer entsprechenden Carbonsäure in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert-Butyl-, Trityl-, Benzyl- oder Tetrahydropyranylgruppe,
als Schutzreste für eine Carboxygruppe die Trimethylsilyl-, Methyl-, Ethyl-, tert.-Butyl-, Benzyl- oder Tetrahydropyranylgruppe,
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln III bis VI sind entweder literaturbekannt oder man erhält diese nach an sich literaturbekannten Verfahren (siehe Beispiele I bis XXXI).

Beispielsweise erhält man eine Ausgangsverbindung der allgemeinen Formel III durch Umsetzung eines in 8-Stellung halogenierten Theophyllinderivats mit einem entsprechend substituierten Alkylhalogenid.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:

Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Diese Zelllinie wurde von der American Type Culture Collection (ATCC HTB 37) erworben. Die Differenzierung der Zellen, um die DPP-IV Expression zu induzieren, wurde nach der Beschreibung von Reiher et al. in einem Artikel mit dem Titel "Increased expression of intestinal cell line Caco-2", erschienen in Proc. Natl. Acad. Sci. Vol. 90, Seiten 5757-5761 (1993), durchgeführt. Der Zellextrakt wurde von in einem Puffer (10mM Tris HCl, 0.15 M NaCl, 0.04 t.i.u. Aprotinin, 0.5% Nonidet-P40, pH 8.0) solubilisierten Zellen durch Zentrifugation bei 35,000 g für 30 Minuten bei 4°C (zur Entfernung von Zelltrümmern) gewonnen.

Der DPP-IV Assay wurde wie folgt durchgeführt:

50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung (Beispiel Nr.) | DPP IV-Hemmung IC₅₀ [nM] |
|---|---|
| 1 (2) | 82 |
| 1(6) | 230 |
| 1(15) | 624 |
| 1(16) | 78 |
| 1(19) | 2770 |
| 1(21) | 124 |
| 1(25) | 56 |
| 1(27) | 125 |
| 1(28) | 166 |
| 1(30) | 2050 |
| 1(34) | 205 |
| 1(35) | 95 |
| 1(55) | 142 |
| 1(60) | 57 |
| 1(62) | 167 |
| 1(70) | 32 |
| 1(97) | 212 |
| 1(121) | 10 |
| 2(1) | 22 |
| 2(22) | 66 |
| 2(28) | 5 |
| 2(56) | 64 |
| 2(77) | 22 |
| 2(85) | 17 |
| 2(88) | 6 |
| 2(113) | 20 |
| 2(119) | 2 |
| 2(127) | 22 |
| 2(131) | 127 |
| 2(136) | 3 |
| 6 | 55 |

Die erfindungsgemäß hergestellten Verbindungen sind gut verträglich, da beispielsweise nach oraler Gabe von 30 mg/kg der Verbindung des Beispiels 1 (2) an Ratten keine toxischen Nebenwirkungen beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ I und Typ II, diabetische Komplikationen, metabolische Azidose oder Ketose, Insulinresistenz, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Ebenso sind sie zur Prävention und Therapie von chronischen entzündlichen Darmerkrankungen geeignet. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Des weiteren sind die Substanzen geeignet, Mangelzustände von Wachstumshormon, die mit Minderwuchs einhergehen, zu beeinflussen.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570), alpha-Glucosidasehemmer (z.B. Acarbose, Voglibose), alpha2-Antagonisten, Insulin und Insulinanaloga, GLP-1 und GLP-1 Analoga (z.B. Exendin-4) oder Amylin. Daneben Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, Cholesterolresorptionsinhibitoren wie zum Beispiel Ezetimibe, gallensäurebindende Substanzen wie zum Beispiel Colestyramin, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin oder ß3-Agonisten wie SB-418790 oder AD-9677.
Daneben ist eine Kombination mit Medikamenten zur Beeinflussung des Bluthochdrucks wie z.B. All Antagonisten oder ACE Inhibitoren, Diuretika, ß-Blocker und andere oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

### Herstellung der Ausgangsverbindungen:

### Beispiel I

### 1,3-Dimethyl-7-benzyl-8-chlor-xanthin

Eine Mischung aus 20 g 8-Chlortheophyllin, 150 ml Dimethylformamid, 10,2 ml Benzylbromid und 15,5 ml N-Ethyl-diisopropylamin wird über Nacht bei Raumtemperatur gerührt. Das Reaktionsgemisch wird auf 600 ml Wasser gegossen. Der Feststoff wird abgesaugt, mit Wasser und Diethylether gewaschen und getrocknet.
Ausbeute: 14,6 g (51 % der Theorie)
Schmelzpunkt: 155°C
R_{f}-Wert: 0.84 (Kieselgel, Essigester/Methanol = 9:1)

Analog Beispiel I werden folgende Verbindungen erhalten:
(1) 1,3-Dimethyl-7-(3-methyl-2-buten-1-yl)-8-chlor-xanthin
   Schmelzpunkt: 104 °C
   Massenspektrum (EI): m/z = 282, 284 [M]⁺
(2) 1,3-Dimethyl-7-(2-butin-1-yl)-8-chlor-xanthin
   Schmelzpunkt: 105-108 °C
   R_{f}-Wert: 0.55 (Kieselgel, Methylenchlorid/Methanol = 20:1)
(3) 1,3-Dimethyl-7-[(1-cyclopenten-1-yl)methyl]-8-chlor-xanthin
   R_{f}-Wert: 0.50 (Kieselgel, Methylenchlorid/Methanol = 20:1)
(4) 1,3-Dimethyl-7-(2-thienylmethyl)-8-chlor-xanthin
   R_{f}-Wert: 0.35 (Kieselgel, Methylenchlorid/Methanol = 50:1)
   Massenspektrum (EI): m/z = 310, 312 [M]⁺
(5) 1,3-Dimethyl-7-(3-fluorbenzyl)-8-chlor-xanthin
   R_{f}-Wert: 0.60 (Kieselgel, Methylenchlorid/Methanol = 20:1)
(6) 1,3-Dimethyl-7-(2-fluorbenzyl)-8-chlor-xanthin
   Massenspektrum (EI): m/z = 322, 324 [M]⁺
(7) 1,3-Dimethyl-7-(3-methyl-2-buten-1-yl)-8-(cis-3-tert.-butyloxycarbonylamino-cyclohexyl)-xanthin
   Massenspektrum (ESI⁺): m/z = 446 [M+H]⁺
(8) 1,3-Dimethyl-7-(4-fluorbenzyl)-8-chlor-xanthin
   R_{f}-Wert: 0.60 (Kieselgel, Methylenchlorid/Methanol = 20:1)
(9) 1,3-Dimethyl-7-(2-buten-1-yl)-8-chlor-xanthin
   R_{f}-Wert: 0.70 (Kieselgel, Methylenchlorid/Methanol = 10:1)
(10) 3-Methyl-7-(3-methyl-2-buten-1-yl)-8-chlor-xanthin
   Schmelzpunkt: 226-228°C
   R_{f}-Wert: 0.66 (Kieselgel, Methylenchlorid/Methanol = 9:1)
   Massenspektrum (ESI⁺): m/z = 269, 271 [M+H]⁺
(11) 3-Methyl-7-(3-methyl-2-buten-1-yl)-8-brom-xanthin
   Massenspektrum (ESI⁺): m/z = 313, 315 [M+H]⁺
   R_{f}-Wert: 0.48 (Kieselgel, Methylenchlorid/Methanol = 10:1)
(12) 1,3-Dimethyl-7-(3-methyl-2-buten-1-yl)-8-[3-(tert.-butyloxycarbonylamino)-propyl]-xanthin
   Massenspektrum (ESI⁺): m/z = 406 [M+H]⁺
(13) 1,3-Dimethyl-7-(3-methyl-2-buten-1-yl)-8-[1-(tert.-butyloxycarbonyl)-piperidin-4-yl]-xanthin
   Durchführung in Gegenwart von Kaliumcarbonat in Dimethylformamid bei 60°C.
   Massenspektrum (ESI⁺): m/z = 432 [M+H]⁺
(14) 1,3-Dimethyl-7-(3-methyl-2-buten-1-yl)-8-[trans-2-(tert.-butyloxycarbonylamino)-cyclohexyl]-xanthin
   Massenspektrum (ESI⁺): m/z = 446 [M+H]⁺
(15) 1,3-Dimethyl-7-(2-pentin-1-yl)-8-chlor-xanthin
   Massenspektrum (ESI⁺): m/z = 281, 283 [M+H]⁺
(16) 3-Methyl-7-benzyl-8-chlor-xanthin
   Massenspektrum (ESI⁺): m/z = 291, 293 [M+H]⁺
(17) 3-Methyl-7-cyclopropylmethyl-8-chlor-xanthin
   Massenspektrum (EI): m/z = 254, 256 [M]⁺
(18) 3-Methyl-7-(2-butin-1-yl)-8-chlor-xanthin
   Massenspektrum (ESI⁺): m/z = 253, 255 [M+H]⁺
(19) 1,3-Dimethyl-7-(3-methyl-2-buten-1-yl)-8-brom-xanthin
   Massenspektrum (ESI⁺): m/z = 327, 329 [M+H]⁺
(20) 1,3-Dimethyl-7-(3-methyl-2-buten-1-yl)-8-[3-(tert.-butyloxycarbonylamino)-cyclohexyl]-xanthin (cis/trans-Gemisch)
   Massenspektrum (ESI⁺): m/z = 446 [M+H]⁺
(21) 1,3-Dimethyl-7-[(thiophen-3-yl)-methyl]-8-chlor-xanthin
   R_{f}-Wert: 0.42 (Kieselgel, Cyclohexan/Essigester = 1:1)
(22) 1,3-Dimethyl-7-[(thiophen-2-yl)-methyl]-8-chlor-xanthin
   ¹H-NMR (300 MHz, CDCl₃): charakteristische Signale bei 3.40 und 3.52 ppm (jeweils s, jeweils 3H), 5.70 ppm (s, 2H), 6.95 ppm (m, 1 H) und 7.25 ppm (m, 2H)
(23) 1,3-Dimethyl-7-[(furan-3-yl)-methyl]-8-chlor-xanthin
   R_{f}-Wert: 0.44 (Kieselgel, Essigester/Hexan = 1:1)
(24) 1,3-Dimethyl-7-[(furan-2-yl)-methyl]-8-chlor-xanthin
   R_{f}-Wert: 0.50 (Kieselgel, Essigester/Hexan = 1:1)
(25) 1,3-Dimethyl-7-(2-propin-1-yl)-8-chlor-xanthin
   R_{f}-Wert: 0.33 (Kieselgel, Essigester/Hexan = 1:1)
(26) 1,3-Dimethyl-7-(2,3-dimethyl-2-buten-1-yl)-8-chlor-xanthin
   R_{f}-Wert: 0.51 (Kieselgel, Essigester/Hexan = 1:1)
(27) 1,3-Dimethyl-7-((E)-2-methyl-2-buten-1-yl)-8-chlor-xanthin
   R_{f}-Wert: 0.57 (Kieselgel, Essigester/Hexan = 1:1)
(28) 1,3-Dimethyl-7-[(cyclohexen-1-yl)-methyl]-8-chlor-xanthin
   R_{f}-Wert: 0.62 (Kieselgel, Essigester/Hexan = 1:1)
(29) 1,3-Dimethyl-7-[(cyclopenten-1-yl)-methyl]-8-chlor-xanthin
   R_{f}-Wert: 0.54 (Kieselgel, Essigester/Hexan = 1:1)
(30) 1,3-Dimethyl-7-((Z)-2-methyl-2-buten-1-yl)-8-(piperazin-1-yl)-xanthin
   R_{f}-Wert: 0.51 (Kieselgel, Essigester = 1:1)
(31) 1,3-Dimethyl-7-(3-methyl-2-buten-1-yl)-8-[1-(tert.-butyloxycarbonyl)-piperidin-3-yl]-xanthin
   Durchführung in Gegenwart von Kaliumcarbonat
   Massenspektrum (ESI⁺): m/z = 432 [M+H]⁺
(32) 1,3-Dimethyl-7-[(2-naphthyl)methyl]-8-chlor-xanthin
   Durchführung in Gegenwart von Kaliumcarbonat
   R_{f}-Wert: 0.60 (Kieselgel, Cyclohexan/Essigester = 1:1)
   Massenspektrum (ESI⁺): m/z =377, 379 [M+Na]⁺
(33) 1,3-Dimethyl-7-[(1-naphthyl)methyl]-8-chlor-xanthin
   Durchführung in Gegenwart von Kaliumcarbonat
   R_{f}-Wert: 0.60 (Kieselgel, Cyclohexan/Essigester = 1:1)
   Massenspektrum (ESI⁺): m/z = 355, 357 [M+H]⁺
(34) 1,3-Dimethyl-7-(2-cyano-benzyl)-8-chlor-xanthin
   Durchführung in Gegenwart von Kaliumcarbonat
   R_{f}-Wert: 0.60 (Kieselgel, Cyclohexan/Essigester = 1:1)
   Massenspektrum (ESI⁺): m/z = 330, 332 [M+H]⁺
(35) 1,3-Dimethyl-7-(3-cyano-benzyl)-8-chlor-xanthin
   Durchführung in Gegenwart von Kaliumcarbonat
   R_{f}-Wert: 0.60 (Kieselgel, Cyclohexan/Essigester = 1:1)
   Massenspektrum (ESI⁺): m/z = 330, 332 [M+H]⁺
(36) 1,3-Dimethyl-7-(3,5-difluor-benzyl)-8-chlor-xanthin
   Durchführung in Gegenwart von Kaliumcarbonat
   R_{f}-Wert: 0.60 (Kieselgel, Cyclohexan/Essigester = 1:1)
   Massenspektrum (EI): m/z = 340, 342 [M]⁺
(37) 1,3-Dimethyl-7-(4-cyano-benzyl)-8-chlor-xanthin
   Durchführung in Gegenwart von Kaliumcarbonat
   R_{f}-Wert: 0.60 (Kieselgel, Cyclohexan/Essigester = 1:1)
   Massenspektrum (EI): m/z = 329, 331 [M]⁺
(38) 1,3-Dimethyl-7-(3-nitro-benzyl)-8-chlor-xanthin
   Durchführung in Gegenwart von Kaliumcarbonat
   R_{f}-Wert: 0.60 (Kieselgel, Cyclohexan/Essigester = 1:1)
   Massenspektrum (ESI⁺): m/z = 350, 352 [M+H]⁺
(39) 1,3-Dimethyl-7-(4-nitro-benzyl)-8-chlor-xanthin
   Durchführung in Gegenwart von Kaliumcarbonat
   R_{f}-Wert: 0.60 (Kieselgel, Cyclohexan/Essigester = 1:1)
(40) 3-Methyl-7-(2-cyano-benzyl)-8-chlor-xanthin
   R_{f}-Wert: 0.60 (Kieselgel, Cyclohexan/Essigester = 1:1)
   Massenspektrum (ESI⁺): m/z = 316, 318 [M+H]⁺
(41) 1,3-Dimethyl-7-(2-nitro-benzyl)-8-chlor-xanthin
   Durchführung in Gegenwart von Kaliumcarbonat
   R_{f}-Wert: 0.60 (Kieselgel, Cyclohexan/Essigester = 1:1)
(42) 1,3-Dimethyl-7-(2-iod-benzyl)-8-chlor-xanthin
   Durchführung in Gegenwart von Kaliumcarbonat.
   Massenspektrum (ESI⁺): m/z = 431, 433 [M+H]⁺

### Beispiel II

### (R)-1,3-Dimethyl-7-(3-methyl-2-buten-1-yl)-8-[3-(tert.-butyloxycarbonylamino)-piperidin-1-yl]-xanthin

Eine Mischung aus 1 g 1,3-Dimethyl-7-(3-methyl-2-buten-1-yl)-8-chlor-xanthin, 1,32 g (R)-3-tert.-Butyloxycarbonylamino-piperidin, 1 ml Triethylamin und 10 ml Dimethylformamid wird zweieinhalb Tage bei 50°C gerührt. Das Reaktionsgemisch wird mit 100 ml Wasser verdünnt und anschließend mit Essigester extrahiert. Die organische Phase wird getrocknet, eingeengt und der Rückstand mit Diethylether verrührt. Der Feststoff wird abgesaugt und getrocknet.
Ausbeute: 1,0 g (63 % der Theorie)
Schmelzpunkt: 164°C
R_{f}-Wert: 0.36 (Aluminiumoxid, Cyclohexan/Essigester = 1:1)

Analog Beispiel II werden folgende Verbindungen erhalten:
(1) (S)-1,3-Dimethyl-7-(3-methyl-2-buten-1-yl)-8-[3-(tert.-butyloxycarbonylamino)-piperidin-1-yl]-xanthin
   Schmelzpunkt: 164°C
   Massenspektrum (ESI⁻): m/z = 445 [M-H]⁻
(2) 1,3-Dimethyl-7-(3-methyl-2-buten-1-yl)-8-[3-(tert.-butyloxycarbonylamino)-hexahydroazepin-1-yl]-xanthin
   Schmelzpunkt: 154°C
   Massenspektrum (ESI⁻): m/z = 459 [M-H]⁻
(3) 1,3-Dimethyl-7-(3-methyl-2-buten-1-yl)-8-[4-(tert.-butyloxycarbonylamino)-hexahydroazepin-1-yl]-xanthin
   Massenspektrum (ESI⁻): m/z = 459 [M-H]⁻
   R_{f}-Wert: 0.67 (Kieselgel, Essigester)
(4) 1,3-Dimethyl-7-(3-methyl-2-buten-1-yl)-8-[3-(tert.-butyloxycarbonylamino)-4-methyl-piperidin-1-yl]-xanthin
   Massenspektrum (ESI⁺): m/z = 461 [M+H]⁺
   R_{f}-Wert: 0.88 (Kieselgel, Essigester/Methanol = 5:1)
(5) 1-Methyl-3-(4-methoxy-benzyl)-7-benzyl-8-[(S)-3-(tert.-butyloxycarbonylamino)-piperidin-1-yl]-xanthin
   Massenspektrum (ESI⁺): m/z = 575 [M+H]⁺
   R_{f}-Wert: 0.74 (Kieselgel, Methylenchlorid/Methanol = 95:5)
(6) 1,3-Dimethyl-7-(3-methyl-2-buten-1-yl)-8-{N-[2-(tert.-butyloxycarbonylamino)-ethyl]-N-ethyl-amino}-xanthin
   Massenspektrum (ESI⁺): m/z = 435 [M+H]⁺
(7) 1-Methyl-3-hexyl-7-benzyl-8-[(S)-3-(tert.-butyloxycarbonylamino)-piperidin-1-yl]-xanthin
   Schmelzpunkt: 152-159°C
   Massenspektrum (ESI⁺): m/z = 539 [M+H]⁺
(8) 1-Methyl-3-(2-trimethylsilanyl-ethoxymethyl)-7-benzyl-8-(3-amino-piperidin-1-yl)-xanthin
   Durchführung mit Kaliumcarbonat bei 120°C
   Massenspektrum (ESI⁺): m/z = 485 [M+H]⁺
(9) 1-Methyl-3-(2-hydroxy-ethyl)-7-benzyl-8-[(S)-3-(tert.-butyloxycarbonylamino)-piperidin-1-yl]-xanthin
   Durchführung mit Kaliumcarbonat bei 110°C
   R_{f}-Wert: 0.41 (Kieselgel, Methylenchlorid/Methanol/konz. wässriges Ammoniak = 9:1:0.1)
   Massenspektrum (ESI⁺): m/z = 499 [M+H]⁺
(10) 1-(2-Phenyl-ethyl)-3-methyl-7-(3-methyl-2-buten-1-yl)-8-[(S)-3-(tert.-butyloxycarbonylamino)-piperidin-1-yl]-xanthin
   Durchführung mit Hünigbase bei 100°C
   Massenspektrum (ESI⁺): m/z = 537 [M+H]⁺
(11) 1-(2-Phenyl-ethyl)-3-methyl-7-(3-methyl-2-buten-1-yl)-8-[(R)-3-(tert.-butyloxycarbonylamino)-piperidin-1-yl]-xanthin
   Massenspektrum (ESI⁺): m/z = 537 [M+H]⁺
(12) 1,3-Dimethyl-7-(3-methyl-2-buten-1-yl)-8-{2-[(tert.-butyloxycarbonylamino)methyl]-piperidin-1-yl}-xanthin
   Durchführung mit Kaliumcarbonat und Natriumjodid in Dimethylsulfoxid bei 120°C R_{f}-Wert: 0.73 (Kieselgel, Essigester)
   Massenspektrum (ESI⁺): m/z = 461 [M+H]⁺
(13) 1,3-Dimethyl-7-(3-methyl-2-buten-1-yl)-8-{[1-(tert.-butyloxycarbonyl)-pyrrolidin-3-yl]amino}-xanthin
   Durchführung mit Natriumcarbonat in Dimethylsulfoxid bei 130°C R_{f}-Wert: 0.50 (Kieselgel, Essigester)
   Massenspektrum (ESI⁺): m/z = 433 [M+H]⁺
(14) 1,3-Dimethyl-7-(3-methyl-2-buten-1-yl)-8-{N-[1-(tert.-butyloxycarbonyl)-piperidin-3-yl]-N-methyl-amino)-xanthin
   Durchführung mit Hünigbase, 4-Dimethylaminopyridin und Natriumcarbonat in Dimethylsulfoxid bei 150°C
   R_{f}-Wert: 0.62 (Kieselgel, Essigester)
   Massenspektrum (ESI⁺): m/z = 461 [M+H]⁺
(15) 3-Methyl-7-(3-methyl-2-buten-1-yl)-8-[(S)-3-(tert.-butyloxycarbonylamino)-piperidin-1-yl]-xanthin
   R_{f}-Wert: 0.30 (Kieselgel, Methylenchlorid/Methanol = 9:1)
   Massenspektrum (ESI⁺): m/z = 433 [M+H]⁺
(16) 1,3-Dimethyl-7-(3-methyl-2-buten-1-yl)-8-{[1-(tert.-butyloxycarbonyl)-piperidin-4-yl]amino}-xanthin
   Durchführung mit Hünigbase und 4-Dimethylaminopyridin in Dimethylsulfoxid bei 100°C
   R_{f}-Wert: 0.81 (Kieselgel, Methylenchlorid/Methanol/konz. wässriges Ammoniak = 9:1:0.1)
(17) 1,3-Dimethyl-7-(3-methyl-2-buten-1-yl)-8-{[1-(tert.-butyloxycarbonyl)-piperidin-3-yl]amino}-xanthin
   Durchführung mit Hünigbase und 4-Dimethylaminopyridin in Dimethylsulfoxid bei 100°C
   R_{f}-Wert: 0.37 (Kieselgel, Essigester/Hexan = 7:3)
(18) 3-Methyl-7-(3-methyl-2-buten-1-yl)-8-[3-(tert.-butyloxycarbonylamino)-piperidin-1-yl]-xanthin
   R_{f}-Wert: 0.49 (Kieselgel, Petrolether/Essigester/Methanol = 5:4:1)
   Massenspektrum (ESI⁺): m/z = 433 [M+H]⁺
(19) 1,3-Dimethyl-7-(3-methyl-2-buten-1-yl)-8-{N-[1-(tert.-butyloxycarbonyl)-pyrrolidin-3-yl]-N-methyl-amino}-xanthin
   Durchführung mit Natriumcarbonat in Dimethylsulfoxid bei 160°C
   R_{f}-Wert: 0.68 (Kieselgel, Methylenchlorid/Methanol/konz. wässriges Ammoniak = 90:10:1)
   Massenspektrum (ESI⁺): m/z = 447 [M+H]⁺
(20) 1-[2-(2-Nitro-phenyl)-ethyl]-3-methyl-7-(3-methyl-2-buten-1-yl)-8-[3-(tert.-butyloxycarbonylamino)-piperidin-1-yl]-xanthin
   R_{f}-Wert: 0.34 (Kieselgel, Petrolether/Essigester/Methanol = 7:2:1)
   Massenspektrum **(ESI⁺):** m/z = 582 [M+H]⁺
(21) 1-[2-(3,5-Difluor-phenyl)-ethyl]-3-methyl-7-(3-methyl-2-buten-1-yl)-8-[3-(tert.-butyloxycarbonylamino)-piperidin-1-yl]-xanthin
   R_{f}-Wert: 0.38 (Kieselgel, Petrolether/Essigester/Methanol = 7:2:1)
   Massenspektrum (ESI⁺): m/z = 573 [M+H]⁺
(22) 1-[2-(2,6-Difluor-phenyl)-ethyl]-3-methyl-7-(3-methyl-2-buten-1-yl)-8-[3-(tert.-butyloxycarbonylamino)-piperidin-1-yl]-xanthin
   R_{f}-Wert: 0.38 (Kieselgel, Petrolether/Essigester/Methanol = 7:2:1)
   Massenspektrum (ESI⁺): m/z = 573 [M+H]⁺
(23) 3-Methyl-7-(3-methyl-2-buten-1-yl)-8-[(R)-3-(tert.-butyloxycarbonylamino)-piperidin-1-yl]-xanthin
   Massenspektrum (ESI⁺): m/z = 433 [M+H]⁺
(24) 1-[2-(3,5-Dimethyl-phenyl)-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 565 [M+H]⁺
(25) 1,3-Dimethyl-7-(3-methyl-2-buten-1-yl)-8-[cis-2-(tert.-butyloxycarbonylamino)-cyclopropylamino]-xanthin
   R_{f}-Wert: 0.41 (Kieselgel, Essigester)
   Massenspektrum (ESI⁺): m/z = 419 [M+H]⁺
(26) 3-Methyl-7-(2-cyano-benzyl)-8-[3-(tert.-butyloxycarbonylamino)-piperidin-1-yl]-xanthin
   Durchführung mit Natriumcarbonat in Dimethylsulfoxid
   Massenspektrum (ESI⁻): m/z = 478 [M-H]⁻
(27) 1-(2-Phenyl-2-oxo-ethyl)-3-methyl-7-(3-methyl-2-buten-1-yl)-8-[4-(tert.-butyloxycarbonyl)-piperazin-1-yl]-xanthin
   Durchführung mit Kaliumcarbonat bei 100°C
   R_{f}-Wert: 0.70 (Kieselgel, Cyclohexan/Essigester = 1:1)
   Massenspektrum (ESI⁺): m/z = 537 [M+H]⁺
(28) 1-[2-(3-Nitro-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 596 [M+H]⁺
(29) 1-(2-Phenyl-2-oxo-ethyl)-3-methyl-7-(3-methyl-2-buten-1-yl)-8-(4-(tert.-butyloxycarbonyl)-homopiperazin-1-yl]-xanthin
   R_{f}-Wert: 0.70 (Kieselgel, Cyclohexan/Essigester = 1:1)
(30) 1,3-Dimethyl-7-(3-methyl-2-buten-1-yl)-8-{4-[(tert.-butyloxycarbonylamino)-methyl]-piperidin-1-yl}-xanthin
   Durchführung in 1-Methyl-2-pyrrolidon bei 135°C.
   R_{f}-Wert: 0.69 (Kieselgel, Essigester)
   Massenspektrum (ESI⁺): m/z = 461 [M+H]⁺
(31) 1,3-Dimethyl-7-(3-methyl-2-buten-1-yl)-8-{3-[(tert.-butyloxycarbonylamino)-methyl]-piperidin-1-yl}-xanthin
   Durchführung in 1-Methyl-2-pyrrolidon bei 135°C.
   R_{f}-Wert: 0.74 (Kieselgel, Essigester)
   Massenspektrum (ESI⁺): m/z = 461 [M+H]⁺
(32) 1,3-Dimethyl-7-(3-methyl-2-buten-1-yl)-8-[trans-2-(tert.-butyloxycarbonylamino)-cyclobutylamino]-xanthin
   Durchführung in Gegenwart von Hünigbase in 1-Methyl-2-pyrrolidon bei 135°C.
   R_{f}-Wert: 0.65 (Kieselgel, Essigester/Petrolether = 8:2)
   Massenspektrum (ESI⁺): m/z = 433 [M+H]⁺
(33) 1,3-Dimethyl-7-(3-methyl-2-buten-1-yl)-8-{N-[(S)-2-(tert.-butyloxycarbonylamino)-1-methyl-ethyl]-N-methyl-amino}-xanthin
   Durchführung mit Natriumcarbonat in Dimethylsulfoxid
   R_{f}-Wert: 0.69 (Kieselgel, Essigester)
   Massenspektrum (ESI⁺): m/z = 435 [M+H]⁺
(34) 1,3-Dimethyl-7-(3-methyl-2-buten-1-yl)-8-{N-[(R)-2-(tert.-butyloxycarbonylamino)-1-methyl-ethyl]-N-methyl-amino}-xanthin
   Durchführung mit Natriumcarbonat in Dimethylsulfoxid
   R_{f}-Wert: 0.32 (Kieselgel, Cyclohexan/Essigester = 1:1)
   Massenspektrum (ESI⁺): m/z = 435 [M+H]⁺
(35) 1,3-Dimethyl-7-(3-methyl-2-buten-1-yl)-8-[cis-2-(tert.-butyloxycarbonylamino)-cyclohexylamino]-xanthin Durchführung mit Natriumcarbonat in Dimethylsulfoxid

   R_{f}-Wert: 0.35 (Kieselgel, Cyclohexan/Essigester = 1:1)
   Massenspektrum (ESI⁺): m/z = 461 [M+H]⁺
(36) 1,3-Dimethyl-7-(3-methyl-2-buten-1-yl)-8-[6-(tert.-butyloxycarbonylamino)-[1,4]diazepan-1-yl]-xanthin
   Durchführung mit Natriumcarbonat in Dimethylsulfoxid
   R_{f}-Wert: 0.08 (Kieselgel, Methylenchlorid/Methanol = 95:5)
(37) 1-[(Pyridin-2-yl)methyl]-3-methyl-7-(3-methyl-2-buten-1-yl)-8-[3-(tert.-butyloxycarbonylamino)-piperidin-1-yl]-xanthin
   Durchführung mit Natriumcarbonat in Dimethylsulfoxid
   R_{f}-Wert: 0.43 (Kieselgel, Essigester)
   Massenspektrum (ESI⁺): m/z = 524 [M+H]⁺
(38) 1,3-Dimethyl-7-(3-methyl-2-buten-1-yl)-8-[trans-2-(tert.-butyloxycarbonylamino)-cyclopentylamino]-xanthin
   Durchführung in Gegenwart von Hünigbase in 1-Methyl-2-pyrrolidon bei 135°C.
   Schmelzpunkt: 177-179°C
   Massenspektrum (ESI⁺): m/z = 447 [M+H]⁺
(39) 1,3-Dimethyl-7-(3-methyl-2-buten-1-yl)-8-[3-(tert.-butyloxycarbonylamino)-cyclohexylamino]-xanthin (cis/trans-Gemisch)
   Durchführung in Gegenwart von Hünigbase in 1-Methyl-2-pyrrolidon bei 135°C.
   R_{f}-Wert: 0.36 (Kieselgel, Essigester/Petrolether = 1:1)
   Massenspektrum (ESI⁻): m/z = 459 [M-H]⁻
(40) 1,3-Dimethyl-7-(3-methyl-2-buten-1-yl)-8-[cis-2-(tert.-butyloxycarbonylamino)-cyclopentylamino]-xanthin
   Schmelzpunkt: 175-178°C
   Massenspektrum (ESI⁻): m/z = 445 [M-H]⁻
(41) 1-[(Isochinolin-1-yl)methyl]-3-methyl-7-(3-methyl-2-buten-1-yl)-8-[3-(tert.-butyloxycarbonylamino)-piperidin-1-yl]-xanthin
   Durchführung mit Natriumcarbonat in Dimethylsulfoxid
   R_{f}-Wert: 0.51 (Kieselgel, Methylenchlorid/Methanol = 95:5)
(42) 1,3-Dimethyl-7-(3-methyl-2-buten-1-yl)-8-[cis-3-(tert.-butyloxycarbonylamino)-cyclopentylamino]-xanthin
   Durchführung in Gegenwart von Hünigbase in 1-Methyl-2-pyrrolidon bei 135°C.
   R_{f}-Wert: 0.23 (Kieselgel, Essigester/Petrolether = 1:1)
   Massenspektrum (ESI⁺): m/z = 447 [M+H]⁺
(43) 1-[(Pyridin-3-yl)methyl]-3-methyl-7-(3-methyl-2-buten-1-yl)-8-[3-(tert.-butyloxycarbonylamino)-piperidin-1-yl]-xanthin
   Durchführung mit Natriumcarbonat in Dimethylsulfoxid
   R_{f}-Wert: 0.44 (Kieselgel, Methylenchlorid/Methanol = 95:5)
   Massenspektrum (ESI⁺): m/z = 524 [M+H]⁺
(44) 1-[(Pyridin-4-yl)methyl]-3-methyl-7-(3-methyl-2-buten-1-yl)-8-[3-(tert.-butyloxycarbonylamino)-piperidin-1-yl]-xanthin
   Durchführung mit Natriumcarbonat in Dimethylsulfoxid
   R_{f}-Wert: 0.28 (Kieselgel, Essigester)
   Massenspektrum (ESI⁺): m/z = 524 [M+H]⁺
(45) 1-[(Isochinolin-1-yl)methyl]-3-methyl-7-(3-methyl-2-buten-1-yl)-8-[(R)-3-(tert.-butyloxycarbonylamino)-piperidin-1-yl]-xanthin
   Durchführung mit Kaliumcarbonat in Dimethylsulfoxid
   R_{f}-Wert: 0.37 (Kieselgel, Essigester)
   Massenspektrum (ESI⁺): m/z = 574 [M+H]⁺
(46) 1-[(Isochinolin-1-yl)methyl]-3-methyl-7-(3-methyl-2-buten-1-yl)-8-[(S)-3-(tert.-butyloxycarbonylamino)-piperidin-1-yl]-xanthin
   Durchführung mit Kaliumcarbonat in Dimethylsulfoxid
   R_{f}-Wert: 0.37 (Kieselgel, Essigester)
   Massenspektrum (ESI⁺): m/z = 574 [M+H]⁺
(47) 1-(2-Phenyl-2-oxo-ethyl)-3-methyl-7-(3-methyl-2-buten-1-yl)-8-[3-(tert.-butyloxycarbonylamino)-3-methyl-piperidin-1-yl]-xanthin
   R_{f}-Wert: 0.51 (Kieselgel, Cyclohexan/Essigester/Methanol = 6:3:1)
   Massenspektrum (ESI⁺): m/z = 565 [M+H]⁺
(48) 1,3-Dimethyl-7-(3-methyl-2-buten-1-yl)-8-[3-(tert.-butyloxycarbonylamino)-3-methyl-piperidin-1-yl]-xanthin
   R_{f}-Wert: 0.48 (Kieselgel, Cyclohexan/Essigester/Methanol = 6:3:1)
   Massenspektrum (EI): m/z = 460 [M]⁺
(49) 1,3-Dimethyl-7-(3-methyl-2-buten-1-yl)-8-{N-[2-(tert.-butyloxycarbonylamino)-3-dimethylamino-3-oxo-propyl]-N-methyl-amino}-xanthin
   R_{f}-Wert: 0.48 (Kieselgel, Methylenchlorid/Methanol = 9:1)
   Massenspektrum (ESI⁺): m/z = 492 [M+H]⁺
(50) 1,3-Dimethyl-7-(3-methyl-2-buten-1-yl)-8-{N-[2-(tert.-butyloxycarbonylamino)-3-amino-3-oxo-propyl]-N-methyl-amino}-xanthin
   R_{f}-Wert: 0.40 (Kieselgel, Methylenchlorid/Methanol = 9:1)
   Massenspektrum (EI): m/z = 463 [M]⁺
(51) 1-[2-(2-Nitro-phenyl)-2-oxo-ethyl]-3-methyl-7-(3-methyl-2-buten-1-yl)-8-[3-(tert.-butyloxycarbonylamino)-piperidin-1-yl]-xanthin
   Durchführung mit Natriumcarbonat in Dimethylsulfoxid.
   Massenspektrum (ESI⁺): m/z = 596 [M+H]⁺
(52) 1-[(Isochinolin-4-yl)methyl]-3-methyl-7-(3-methyl-2-buten-1-yl)-8-[3-(tert.-butyloxycarbonylamino)-piperidin-1-yl]-xanthin
   Durchführung mit Natriumcarbonat in Dimethylsulfoxid.
   R_{f}-Wert: 0.48 (Kieselgel, Essigester)
   Massenspektrum (ESI⁺): m/z = 574 [M+H]⁺
(53) 1-[(1-Methyl-1*H*-indazol-3-yl)methyl]-3-methyl-7-(3-methyl-2-buten-1-yl)-8-[3-(tert.-butyloxycarbonylamino)-piperidin-1-yl]-xanthin
   Durchführung mit Natriumcarbonat in Dimethylsulfoxid.
   Massenspektrum (ESI⁺): m/z = 577 [M+H]⁺
(54) 1,3-Dimethyl-7-(3-methyl-2-buten-1-yl)-8-{N-[2-(tert.-butyloxycarbonylamino)-3-oxo-3-(pyrrolidin-1-yl)-propyl]-N-methyl-aminol-xanthin
   Durchführung mit Hünigbase in N-Methylpyrrolidinon.
   Schmelzpunkt: 173-175°C
   Massenspektrum (ESI⁺): m/z = 518 [M+H]⁺
(55) 1,3-Dimethyl-7-(3-methyl-2-buten-1-yl)-8-{N-[2-(tert.-butyloxycarbonylamino)-3-methylamino-3-oxo-propyl]-N-methyl-amino}-xanthin
   Durchführung mit Hünigbase in N-Methylpyrrolidinon.
   Massenspektrum (ESI⁺): m/z = 478 [M+H]⁺
(56) 1-[2-(2-Hydroxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 567 [M+H]⁺
(57) 1-Methyl-3-[2-(4-methoxy-phenyl)-ethyl]-7-(2-cyano-benzyl)-8-[3-(tert.-butyloxy-carbonylamino)-piperidin-1-yl]-xanthin
   Durchführung in Gegenwart von Natriumcarbonat in Dimethylsulfoxid.
   Rf-Wert: 0.50 (Kieselgel, Methylenchlorid/Methanol = 9:1)
   Massenspektrum (ESI⁺): m/z = 614 [M+H]⁺
(58) 1-Methyl-3-(2-phenyl-ethyl)-7-(2-cyano-benzyl)-8-[3-(tert.-butyloxycarbonyl-amino)-piperidin-1-yl]-xanthin
   Durchführung in Gegenwart von Natriumcarbonat in Dimethylsulfoxid.
   Massenspektrum (ESI⁺): m/z = 584 [M+H]⁺
(59) 1-[(Chinolin-4-yl)methyl]-3-methyl-7-(3-methyl-2-buten-1-yl)-8-[3-(tert.-butyloxy-carbonylamino)-piperidin-1-yl]-xanthin
   Durchführung in Gegenwart von Natriumcarbonat in Dimethylsulfoxid.
   R_{f}-Wert: 0.50 (Kieselgel, Essigester)
   Massenspektrum (ESI⁺): m/z = 574 [M+H]⁺
(60) 1,3-Dimethyl-7-(3-methyl-2-buten-1-yl)-8-[endo-6-(tert.-butyloxycarbonylamino)-2-aza-bicyclo[2.2.2]oct-2-yl]-xanthin
   Durchführung in Gegenwart von Kaliumcarbonat und Hünigbase in Dimethylsulfoxid.
   R_{f}-Wert: 0.52 (Kieselgel, Cyclohexan/Essigester = 1:1)
   Massenspektrum (ESI⁺): m/z = 473 [M+H]⁺
(61) 1-[(Chinolin-8-yl)methyl]-3-methyl-7-(3-methyl-2-buten-1-yl)-8-[3-(tert.-butyloxy-carbonylamino)-piperidin-1-yl]-xanthin
   Durchführung in Gegenwart von Natriumcarbonat in Dimethylsulfoxid.
   R_{f}-Wert: 0.73 (Kieselgel, Essigester)
   Massenspektrum (ESI⁺): m/z = 574 [M+H]⁺
(62) 1,3-Dimethyl-7-(3-methyl-2-buten-1-yl)-8-[exo-6-(tert.-butyloxycarbonylamino)-2-aza-bicyclo[2.2.2]oct-2-yl]-xanthin
   Durchführung in Gegenwart von Kaliumcarbonat und Hünigbase in Dimethylsulfoxid.
   R_{f}-Wert: 0.45 (Kieselgel, Cyclohexan/Essigester = 1:1)
   Massenspektrum (ESI⁺): m/z = 473 [M+H]⁺
(63) 1-[2-(3-Cyano-phenyl)-2-oxo-ethyl]-3-methyl-7-(3-methyl-2-buten-1-yl)-8-[3-(tert.-butyloxycarbonylamino)-piperidin-1-yl]-xanthin
   Durchführung in Gegenwart von Natriumcarbonat in Dimethylsulfoxid.
   R_{f}-Wert: 0.33 (Kieselgel, Cyclohexan/Essigester = 1:1)
   Massenspektrum (ESI⁺): m/z = 576 [M+H]⁺
(64) 1-[2-(3-Aminosulfonyl-phenyl)-2-oxo-ethyl]-3-methyl-7-(3-methyl-2-buten-1-yl)-8-[3-(tert.-butyloxycarbonylamino)-piperidin-1-yl]-xanthin
   Durchführung in Gegenwart von Natriumcarbonat in Dimethylsulfoxid.
   R_{f}-Wert: 0.15 (Kieselgel, Cyclohexan/Essigester = 1:1)
   Massenspektrum (ESI⁻): m/z = 628 [M-H]⁻
(65) 1-[2-(3-Aminocarbonyl-phenyl)-2-oxo-ethyl]-3-methyl-7-(3-methyl-2-buten-1-yl)-8-[3-(tert.-butyloxycarbonylamino)-piperidin-1-yl]-xanthin
   Durchführung in Gegenwart von Natriumcarbonat in Dimethylsulfoxid.
   R_{f}-Wert: 0.36 (Kieselgel, Methylenchlorid/Methanol = 9:1)
   Massenspektrum (ESI⁺): m/z = 594 [M+H]⁺

### Beispiel III

### 3-(tert.-Butyloxycarbonylamino)-hexahydroazepin

2 g 1-Benzyl-3-(tert.-butyloxycarbonylamino)-hexahydroazepin in 20 ml Methanol werden 24 Stunden bei Raumtemperatur und einem Wasserstoffdruck von 3 bar in Gegenwart von 200 mg Palladium auf Aktivkohle (10% Pd) hydriert. Danach wird vom Katalysator abgesaugt und das Filtrat zur Trockene eingeengt.
Ausbeute: 1,3 g (90 % der Theorie)
Schmelzpunkt: 78°C
Massenspektrum (ESI⁺): m/z = 215 [M+H]⁺

Analog Beispiel III werden folgende Verbindungen erhalten:
(1) (S)-3-(tert.-Butyloxycarbonylamino)-piperidin
   Schmelzpunkt: 122°C
   Massenspektrum (ESI⁺): m/z = 201 [M+H]⁺
(2) (R)-3-(tert.-Butyloxycarbonylamino)-piperidin
   das Ausgangsmaterial, (*R*)-1-Benzyl-3-(tert.-butyloxycarbonylamino)-piperidin, wurde analog dem literaturbekannten (S)-Enantiomer hergestellt (Moon, Sung-Hwan; Lee, Sujin; Synth.Commun.; 28; 21; 1998; 3919-3926)
   Schmelzpunkt: 119°C
   Massenspektrum (ESI⁺): m/z = 201 [M+H]⁺
(3) 4-(tert.-Butyloxycarbonylamino)-hexahydroazepin
   Massenspektrum (ESI⁺): m/z = 215 [M+H]⁺
   R_{f}-Wert: 0.02 (Aluminiumoxid, Cyclohexan/Essigester = 1:1)
(4) 3-(tert.-Butyloxycarbonylamino)-4-methyl-piperidin
   Das Rohprodukt wird direkt zur Verbindung des Beispiels II (4) weiter umgesetzt.
(5) 6-(tert.-Butyloxycarbonylamino)-[1,4]diazepan
   Das Ausgangsmaterial 1,4-Dibenzyl-6-(tert.-butyloxycarbonylamino)-[1,4]diazepan wurde analog J. Heterocycl. Chem. 1995, 32, 637-642 hergestellt.
   Das Rohprodukt wird direkt zur Verbindung des Beispiels II (36) weiter umgesetzt.
(6) 2-(tert.-Butyloxycarbonylamino)-3-methylamino-propionsäure-dimethylamid
   R_{f}-Wert: 0.40 (Kieselgel, Methylenchlorid/Methanol/konz. wässriges Ammoniak = 40:10:1)
   Massenspektrum (ESI⁺): m/z = 246 [M+H]⁺
(7) 2-(tert.-Butyloxycarbonylamino)-3-methylamino-propionsäure-amid
   R_{f}-Wert: 0.20 (Kieselgel, Methylenchlorid/Methanol/konz. wässriges Ammoniak = 40:10:1)
   Massenspektrum (ESI⁺): m/z = 218 [M+H]⁺
(8) 2-(tert.-Butyloxycarbonylamino)-3-methylamino-1-(pyrrolidin-1-yl)-propan-1-on
   Es wird Palladium(II)hydroxid als Katalysator eingesetzt.
   Massenspektrum (ESI⁺): m/z = 272 [M+H]⁺
(9) 2-(tert.-Butyloxycarbonylamino)-1,3-bis(methylamino)-propan-1-on
   Es wird Palladium(II)hydroxid als Katalysator eingesetzt.
   Massenspektrum (ESI⁺): m/z = 232 [M+H]⁺
(10) endo-6-(tert.-Butyloxycarbonylamino)-2-aza-bicyclo[2.2.2]octan
   R_{f}-Wert: 0.25 (Kieselgel, Methylenchlorid/Methanol/konz. wässriges Ammoniak = 90:10:0.1)
   Massenspektrum (ESI⁺): m/z = 227 [M+H]⁺
(11) exo-6-(tert.-Butyloxycarbonylamino)-2-aza-bicyclo[2.2.2]octan
   R_{f}-Wert: 0.27 (Kieselgel, Methylenchlorid/Methanol/konz. wässriges Ammoniak = 90:10:1)
(12) 1-(tert.-Butyloxycarbonyl)-3-amino-4-hydroxy-piperidin
   R_{f}-Wert: 0.17 (Kieselgel, Methylenchlorid/Methanol/konz. wässriges Ammoniak = 90:10:1)
   Massenspektrum (ESI⁺): m/z = 217 [M+H]⁺

### Beispiel IV

### 1-Benzyl-3-(tert.-butyloxycarbonylamino)-hexahydroazepin

Hergestellt durch Umsetzung von 1-Benzyl-3-amino-hexahydroazepin mit Pyrokohlensäure-di-tert.-butylester
Schmelzpunkt: 48-50°C
Massenspektrum (ESI⁺): m/z = 305 [M+H]⁺

Analog Beispiel IV werden folgende Verbindungen erhalten:
(1) 1-Benzyl-4-(tert.-butyloxycarbonylamino)-hexahydroazepin
   Massenspektrum (ESI⁺): m/z = 305 [M+H]⁺
   R_{f}-Wert: 0.79 (Aluminiumoxid, Cyclohexan/Essigester = 1:1)
(2) 3-(tert.-Butyloxycarbonylamino)-4-methyl-pyridin
   Durchführung mit Natrium-bis-(trimethylsilyl)-amid/Pyrokohlensäure-di-tert.-butylester in Tetrahydrofuran bei 0°C.
   R_{f}-Wert: 0.45 (Kieselgel, Essigester)
(3) 1-(tert.-Butyloxycarbonyl)-3-[(2,2,2-trifluoro-acetyl)amino]-pyrrolidin
   Durchführung mit Triethylamin in Tetrahydrofuran
   R_{f}-Wert: 0.77 (Kieselgel, Methylenchlorid/Methanol/konz. wässriges Ammoniak = 90:10:1)
   Massenspektrum (ESI⁺): m/z = 281 [M+H]⁺
(4) trans-2-Amino-1-(tert.-butyloxycarbonylamino)-cyclobutan
   Durchführung mit Pyrokohlensäure-di-tert.-butylester in Gegenwart von 1 N Natronlauge in Methanol bei 0°C.
   R_{f}-Wert: 0.60 (Kieselgel, Methylenchlorid/Methanol/konz. wässriges Ammoniak = 90:10:0.1)
   Massenspektrum (ESI⁺): m/z = 187 [M+H]⁺
(5) (*S*)-1-(tert.-Butyloxycarbonylamino)-2-methylamino-propan
   Durchführung mit Pyrokohlensäure-di-tert.-butylester in Gegenwart von Hünigbase in Methanol.
   Massenspektrum (ESI⁺): m/z = 189 [M+H]⁺
   R_{f}-Wert: 0.30 (Kieselgel, Methylenchlorid/Methanol/konz. wässriges Ammoniak = 90:10:1)
(6) (R)-1-(tert.-Butyloxycarbonylamino)-2-methylamino-propan
   Durchführung mit Pyrokohlensäure-di-tert.-butylester in Gegenwart von Hünigbase in Methanol.
   Massenspektrum (ESI⁺): m/z = 189 [M+H]⁺
(7) 1,3-Dimethyl-7-(3-methyl-2-buten-1-yl)-8-[2-(tert.-butyloxycarbonylamino)-2-methyl-propylamino]-xanthin
   Durchführung mit Pyrokohlensäure-di-tert.-butylester in Gegenwart von Hünigbase in Methanol.
   R_{f}-Wert: 0.82 (Kieselgel, Methylenchlorid/Methanol/konz. wässriges Ammoniak = 90:10:1)
(8) cis-3-Amino-1-(tert.-butyloxycarbonylamino)-cyclopentan
   Durchführung mit Pyrokohlensäure-di-tert.-butylester in Gegenwart von 1 N Natronlauge in Methanol.
   R_{f}-Wert: 0.63 (Kieselgel, Methylenchlorid/Methanol/konz. wässriges Ammoniak = 40:10:1)
   Massenspektrum (ESI⁺): m/z = 201 [M+H]⁺
(9) endo-6-(tert.-Butyloxycarbonylamino)-2-benzyl-2-aza-bicyclo[2.2.2]octan
   R_{f}-Wert: 0.53 (Aluminiumoxid, Cyclohexan/Essigester = 9:1)
   Massenspektrum (ESI⁺): m/z = 317 [M+H]⁺
(10) exo-6-(tert.-Butyloxycarbonylamino)-2-benzyl-2-aza-bicyclo[2.2.2]octan
   R_{f}-Wert: 0.37 (Aluminiumoxid, Cyclohexan/Essigester = 9:1)
   Massenspektrum (ESI⁺): m/z = 317 [M+H]⁺

### Beispiel V

### 1,3-Dimethyl-8-(cis-3-tert.-butyloxycarbonylamino-cyclohexyl)-xanthin

hergestellt aus der Verbindung des Beispiels VI durch Behandlung mit 4N Natronlauge in Methanol bei 100°C im Bombenrohr Massenspektrum (ESI⁺): m/z = 378 [M+H]⁺

Analog Beispiel V wird folgende Verbindung erhalten:
(1) 1,3-Dimethyl-8-[3-(tert.-butyloxycarbonylamino)propyl]-xanthin
   Massenspektrum (ESI⁺): m/z = 338 [M+H]⁺
(2) 1,3-Dimethyl-8-[1-(tert.-butyloxycarbonyl)-piperidin-4-yl]-xanthin
(3) 1,3-Dimethyl-8-[trans-2-(tert.-butyloxycarbonylamino)-cyclohexyl]-xanthin
   Massenspektrum (ESI⁺): m/z = 378 [M+H]⁺
(4) 1,3-Dimethyl-8-[3-(tert.-butyloxycarbonylamino)-cyclohexyl]-xanthin
   (cis/trans-Gemisch)
   Massenspektrum (ESI⁺): m/z = 378 [M+H]⁺
(5) 1,3-Dimethyl-8-[1-(tert.-butyloxycarbonyl)-piperidin-3-yl]-xanthin
   Massenspektrum (ESI⁺): m/z = 364 [M+H]⁺

### Beispiel VI

### 1,3-Dimethyl-5-[(cis-3-tert.-butyloxycarbonylamino-cyclohexyl)-carbonylamino]-6-amino-uracil

hergestellt aus 5,6-Diamino-1,3-dimethyluracil und cis-3-tert.-Butyloxycarbonyl-amino-cyclohexancarbonsäure in Gegenwart von O-(Benzotriazol-1-yl)-N,N,N',N'-tetramethyluroniumhexafluorophosphat und N-Ethyl-diisopropylamin in Dimethylformamid bei Raumtemperatur
Massenspektrum (ESI⁺): m/z = 396 [M+H]⁺

Analog Beispiel VI wird folgende Verbindung erhalten:
(1) 1,3-Dimethyl-5-{[3-(tert.-butyloxycarbonylamino)propyl]-carbonylamino}-6-aminouracil
(2) 1,3-Dimethyl-5-{[1-(tert.-butyloxycarbonyl)-piperidin-4-yl]-carbonylamino}-6-amino-uracil
   Durchführung mit O-(Benzotriazol-1-yl)-N,N,N',N'-tetramethyluroniumtetrafluoroborat und N-Hydroxybenzotriazol
   Massenspektrum (ESI⁺): m/z = 382 [M+H]⁺
(3) 1,3-Dimethyl-5-({trans-2-[(fluoren-9-ylmethoxycarbonyl)amino]-cyclohexyl}-carbonylamino)-6-amino-uracil
   Durchführung mit O-(Benzotriazol-1-yl)-N,N,N',N'-tetramethyluroniumtetrafluoroborat
   Massenspektrum (ESI⁺): m/z = 518 [M+H]⁺
(4) 1,3-Dimethyl-5-{[3-(tert.-butyloxycarbonylamino)-cyclohexyl]-carbonylamino}-6-amino-uracil (cis/trans-Gemisch)
   Durchführung mit O-(Benzotriazol-1-yl)-N,N,N',N'-tetramethyluroniumtetrafluoroborat
   Massenspektrum (ESI⁺): m/z = 396 [M+H]⁺
(5) 1,3-Dimethyl-5-{[1-(tert.-butyloxycarbonyl)-piperidin-3-yl]-carbonylamino}-6-amino-uracil
   Durchführung mit O-(Benzotriazol-1-yl)-N,N,N',N'-tetramethyluroniumtetrafluoroborat Massenspektrum (ESI⁺): m/z = 382 [M+H]⁺
(6) 2-(tert.-Butyloxycarbonylamino)-3-(N-benzyl-N-methyl-amino)-propionsäure-dimethylamid
   Durchführung mit Dimethylamin in Gegenwart von O-(Benzotriazol-1-yl)-N,N,N',N'-tetramethyluroniumtetrafluoroborat und Hydroxybenzotriazol in Tetrahydrofuran. R_{f}-Wert: 0.80 (Kieselgel, Methylenchlorid/Methanol/konz. wässriges Ammoniak = 40:10:1)
   Massenspektrum (ESI⁺): m/z = 336 [M+H]⁺
(7) 2-(tert.-Butyloxycarbonylamino)-3-(N-benzyl-N-methyl-amino)-propionsäure-amid Durchführung mit Ammoniumcarbonat in Gegenwart von O-(Benzotriazol-1-yl)-N,N,N',N'-tetramethyluroniumtetrafluoroborat und Hydroxybenzotriazol in Tetrahydrofuran.
   R_{f}-Wert: 0.75 (Kieselgel, Methylenchlorid/Methanol/konz. wässriges Ammoniak = 40:10:1)
   Massenspektrum (ESI⁺): m/z = 308 [M+H]⁺
(8) 2-(tert.-Butyloxycarbonylamino)-3-(N-benzyl-N-methyl-amino)-1-(pyrrolidin-1-yl)-propan-1-on
   Durchführung mit Pyrrolidin in Gegenwart von O-(Benzotriazol-1-yl)-N,N,N',N'-tetramethyluroniumtetrafluoroborat und Hydroxybenzotriazol in Tetrahydrofuran.
   R_{f}-Wert: 0.40 (Kieselgel, Methylenchlorid/Methanol = 9:1)
   Massenspektrum (ESI⁺): m/z = 362 [M+H]⁺
(9) 2-(tert.-Butyloxycarbonylamino)-3-(N-benzyl-N-methyl-amino)-1-dimethylamino-propan-1-on
   Durchführung mit Methylamin (40%ige wässrige Lösung) in Gegenwart von O-(Benzotriazol-1-yl)-N,N,N',N'-tetramethyluroniumtetrafluoroborat und Hydroxybenzotriazol in Tetrahydrofuran.
   R_{f}-Wert: 0.40 (Kieselgel, Methylenchlorid/Methanol = 9:1)
   Massenspektrum (ESI⁺): m/z = 322 [M+H]⁺
(10) 1-(tert.-Butyloxycarbonyl)-3-{[(9H-fluoren-9-ylmethoxy)carbonyl]amino}-3-(pyrrolidin-1-ylcarbonyl)-piperidin
   Durchführung mit Pyrrolidin in Gegenwart von O-(Benzotriazol-1-yl)-N,N,N',N'-tetra-methyluroniumtetrafluoroborat, Hydroxybenzotriazol und Hünigbase in Dimethylformamid. Das Ausgangsmaterial 1-(tert.-Butyloxycarbonyl)-3-{[(9H-fluoren-9-ylmethoxy)carbonyl]amino}-piperidin-3-yl-carbonsäure ist bei Pharmacore, Inc.
   (USA) erhältlich.
   R_{f}-Wert: 0.52 (Aluminiumoxid, Methylenchlorid/Methanol = 9:1)
   Massenspektrum (ESI⁺): m/z = 520 [M+H]⁺

### Beispiel VII

### 1,3-Bis-(cyclopropylmethyl)-7-benzyl-8-chlor-xanthin

hergestellt aus der Verbindung des Beispiels VIII durch Umsetzung mit N-Chlorsuccinimid in 1,2-Dichlorethan unter Rückfluß
Massenspektrum (ESI⁺): m/z = 407, 409 [M+Na]⁺

Analog Beispiel VII werden folgende Verbindungen erhalten:
(1) 1-Methyl-3-(cyclopropylmethyl)-7-benzyl-8-chlor-xanthin
   Massenspektrum (ESI⁺): m/z = 345, 347 [M+H]⁺
(2) 1,3-Diethyl-7-benzyl-8-chlor-xanthin
   Massenspektrum (ESI⁺): m/z = 355, 357 [M+Na]⁺
(3) 1-Methyl-3-ethyl-7-benzyl-8-chlor-xanthin
   Massenspektrum (ESI⁺): m/z = 341, 343 [M+Na]⁺
(4) 1-Methyl-3-(4-methoxy-benzyl)-7-benzyl-8-chlor-xanthin
   Schmelzpunkt: 172-175°C
   Massenspektrum (ESI⁺): m/z = 411, 413 [M+H]⁺
(5) 1-Methyl-3,7-dibenzyl-8-chlor-xanthin
   R_{f}-Wert: 0.72 (Kieselgel, Methylenchlorid/Methanol/konz. wässriges Ammoniak = 98:2:1)
   Massenspektrum (ESI⁺): m/z = 381, 383 [M+H]⁺
(6) 1-Methyl-3-[(methoxycarbonyl)-methyl]-7-benzyl-8-chlor-xanthin
   R_{f}-Wert: 0.83 (Kieselgel, Methylenchlorid/Methanol/konz. wässriges Ammoniak = 95:5:1)
   Massenspektrum (ESI⁺): m/z = 363, 365 [M+H]⁺
(7) 1-Methyl-3-isopropyl-7-benzyl-8-chlor-xanthin
   R_{f}-Wert: 0.69 (Kieselgel, MethylenchloridlMethanol/konz. wässriges Ammoniak = 98:2:1)
   Massenspektrum (EI): m/z = 332, 334 [M]⁺
(8) 1-Methyl-3-hexyl-7-benzyl-8-chlor-xanthin
   R_{f}-Wert: 0.68 (Kieselgel, Methylenchlorid/Methanol/konz. wässriges Ammoniak = 98:2:1)
   Massenspektrum (ESI⁺): m/z = 375, 377 [M+H]⁺
(9) 1-Methyl-3-(2-trimethylsilanyl-ethoxymethyl)-7-benzyl-8-chlor-xanthin
   Massenspektrum (ESI⁺): m/z = 421, 423 [M+H]⁺
(10) 1-Methyl-3-(2-methoxy-ethyl)-7-benzyl-8-chlor-xanthin
   R_{f}-Wert: 0.84 (Kieselgel, Methylenchlorid/Methanol/konz. wässriges Ammoniak = 9:1:0.1)
   Massenspektrum (ESI⁺): m/z = 349, 351 [M+H]⁺
(11) 1-Methyl-3-cyanomethyl-7-benzyl-8-chlor-xanthin
   R_{f}-Wert: 0.90 (Kieselgel, Methylenchlorid/Methanol/konz. wässriges Ammoniak = 95:5:1)
   Massenspektrum (ESI⁺): m/z = 352 [M+Na]⁺
(12) 1-Methyl-3-(2-hydroxy-ethyl)-7-benzyl-8-chlor-xanthin
   R_{f}-Wert: 0.48 (Kieselgel, Methylenchlorid/Methanol/konz. wässriges Ammoniak = 9:1:0.1)
   Massenspektrum (ESI⁺): m/z = 335, 337 [M+H]⁺
(13) 1-Methyl-3-(2-trimethylsilanyl-ethoxymethyl)-7-benzyl-8-chlor-xanthin
   Massenspektrum (ESI⁺): m/z = 421, 423 [M+H]⁺
(14) 1-Methyl-3-(2-trimethylsilanyl-ethoxymethyl)-7-(2-cyano-benzyl)-8-chlor-xanthin
   Massenspektrum (ESI⁺): m/z = 468, 470 [M+Na]⁺

### Beispiel VIII

### 1,3-Bis-(cyclopropylmethyl)-7-benzyl-xanthin

hergestellt aus 7-Benzyl-xanthin durch Umsetzung mit Cyclopropylmethylbromid in Dimethylformamid in Gegenwart von Cäsiumcarbonat
Massenspektrum (ESI⁺): m/z = 351 [M+H]⁺

Analog Beispiel VIII werden folgende Verbindungen erhalten:
(1) 3-(Cyclopropylmethyl)-7-benzyl-xanthin
   Massenspektrum (ESI⁺): m/z = 297 [M+H]⁺
(2) 1,3-Diethyl-7-benzyl-xanthin
   Durchführung mit Kaliumcarbonat
   Massenspektrum (ESI⁺): m/z = 321 [M+Na]⁺
(3) 3-Ethyl-7-benzyl-xanthin
   Durchführung mit Kaliumcarbonat
   Massenspektrum (ESI⁺): m/z = 293 [M+Na]⁺
(4) 3-(4-Methoxy-benzyl)-7-benzyl-xanthin
   Durchführung mit 1,8-Diazabicyclo[5.4.0]undec-7-en
   Massenspektrum (ESI⁺): m/z = 363 [M+H]⁺
(5) 3,7-Dibenzyl-xanthin
   Durchführung mit 1,8-Diazabicyclo[5.4.0]undec-7-en Schmelzpunkt: 184-187°C
   Massenspektrum (ESI⁺): m/z = 333 [M+H]⁺
(6) 3-[(Methoxycarbonyl)-methyl]-7-benzyl-xanthin
   Durchführung mit 1,8-Diazabicyclo[5.4.0]undec-7-en
   R_{f}-Wert: 0.21 (Kieselgel, Methylenchlorid/Methanol/konz. wässriges Ammoniak = 95:5:1)
   Massenspektrum (ESI⁺): m/z = 315 [M+H]⁺
(7) 3-Isopropyl-7-benzyl-xanthin
   Durchführung mit 1,8-Diazabicyclo[5.4.0]undec-7-en
   Schmelzpunkt: 215-218°C
   Massenspektrum (ESI⁺): m/z = 285 [M+H]⁺
(8) 3-Hexyl-7-benzyl-xanthin
   Durchführung mit 1,8-Diazabicyclo[5.4.0]undec-7-en
   R_{f}-Wert: 0.52 (Kieselgel, Methylenchlorid/Methanol/konz. wässriges Ammoniak = 9:1:0.1)
   Massenspektrum (ESI⁺): m/z = 327 [M+H]⁺
(9) 3-(2-Trimethylsilanyl-ethoxymethyl)-7-benzyl-xanthin
   Durchführung mit 1,8-Diazabicyclo[5.4.0]undec-7-en
   Massenspektrum (ESI⁺): m/z = 373 [M+H]⁺
(10) 3-(2-Methoxy-ethyl)-7-benzyl-xanthin
   Durchführung mit 1,8-Diazabicyclo[5.4.0]undec-7-en
   R_{f}-Wert: 0.45 (Kieselgel, Methylenchlorid/Methanol/konz. wässriges Ammoniak = 9:1:0.1)
   Massenspektrum (ESI⁺): m/z = 301 [M+H]⁺
(11) 3-Cyanomethyl-7-benzyl-xanthin
   Durchführung mit 1,8-Diazabicyclo[5.4.0]undec-7-en
   R_{f}-Wert: 0.41 (Kieselgel, Methylenchlorid/Methanol/konz. wässriges Ammoniak = 9:1:0.1)
   Massenspektrum (ESI⁻): m/z = 280 [M-H]⁻
(12) 3-(2-Hydroxy-ethyl)-7-benzyl-xanthin
   Durchführung mit 1,8-Diazabicyclo[5.4.0]undec-7-en
   R_{f}-Wert: 0.28 (Kieselgel, Methylenchlorid/Methanol/konz. wässriges Ammoniak = 9:1:0.1)
   Massenspektrum (ESI⁺): m/z = 287 [M+H]⁺
(13) 3-(2-Trimethylsilanyl-ethoxymethyl)-7-benzyl-xanthin
   Durchführung mit 1,8-Diazabicyclo[5.4.0]undec-7-en
   R_{f}-Wert: 0.30 (Kieselgel, Methylenchlorid/Methanol = 98:2) Massenspektrum (ESI⁺): m/z = 373 [M+H]⁺
(14) 3-[(Methoxycarbonyl)methyl]-7-(3-methyl-2-buten-1-yl)-8-[3-(tert.-butyloxycarbonylamino)-piperidin-1-yl]-xanthin
   Durchführung mit 1,8-Diazabicyclo[5.4.0]undec-7-en
   R_{f}-Wert: 0.31 (Kieselgel, Methylenchlorid/Methanol/konz. wässriges Ammoniak = 90:10:1)
   Massenspektrum (ESI⁺): m/z = 491 [M+H]⁺
(15) 3-(2-Trimethylsilanyl-ethoxymethyl)-7-(2-cyano-benzyl)-xanthin
   Durchführung in Gegenwart von 1,8-Diazabicyclo[5.4.0]undec-7-en.
   Massenspektrum (ESI⁺): m/z = 420 [M+Na]⁺

### Beispiel IX

### 1-Ethyl-3-methyl-7-(3-methyl-2-buten-1-yl)-8-brom-xanthin

hergestellt aus 3-Methyl-7-(3-methyl-2-buten-1-yl)-8-brom-xanthin durch Umsetzung mit Ethylbromid in Gegenwart von Kaliumcarbonat in Dimethylformamid bei 70°C
Massenspektrum (ESI⁺): m/z = 341, 343 [M+H]⁺
Retentionszeit: 1,48 min (HPLC, Multosphere 100FBS, 50 mm, 50% Acetonitril)

Analog Beispiel IX werden folgende Verbindungen erhalten:
(1) 1-Propyl-3-methyl-7-(3-methyl-2-buten-1-yl)-8-brom-xanthin
   Massenspektrum (ESI⁺): m/z = 355, 357 [M+H]⁺
(2) 1-Butyl-3-methyl-7-(3-methyl-2-buten-1-yl)-8-brom-xanthin
   Massenspektrum (ESI⁺): m/z = 369, 371 [M+H]⁺
(3) 1-(2-Propyl)-3-methyl-7-(3-methyl-2-buten-1-yl)-8-brom-xanthin
   Retentionszeit: 2,11 min (HPLC, Multosphere 100FBS, 50 mm, 50% Acetonitril)
(4) 1-(2-Methylpropyl)-3-methyl-7-(3-methyl-2-buten-1-yl)-8-brom-xanthin
   Retentionszeit: 2,46 min (HPLC, Multosphere 100FBS, 50 mm, 50% Acetonitril)
(5) 1-(2-Propen-1-yl)-3-methyl-7-(3-methyl-2-buten-1-yl)-8-brom-xanthin
   Retentionszeit: 1,55 min (HPLC, Multosphere 100FBS, 50 mm, 50% Acetonitril)
   Massenspektrum (ESI⁺): m/z = 353, 355 [M+H]⁺
(6) 1-(2-Pröpin-1-yl)-3-methyl-7-(3-methyl-2-buten-1-yl)-8-brom-xanthin
   Retentionszeit: 1,20 min (HPLC, Multosphere 100FBS, 50 mm, 50% Acetonitril)
   Massenspektrum (ESI⁺): m/z = 351, 353 [M+H]⁺
(7) 1-(Cyclopropylmethyl)-3-methyl-7-(3-methyl-2-buten-1-yl)-8-brom-xanthin
   Retentionszeit: 2,19 min (HPLC, Multosphere 100FBS, 50 mm, 50% Acetonitril)
   Massenspektrum (ESI⁺): m/z = 367, 369 [M+H]⁺
(8) 1-Benzyl-3-methyl-7-(3-methyl-2-buten-1-yl)-8-brom-xanthin
   Retentionszeit: 2,40 min (HPLC, Multosphere 100FBS, 50 mm, 50% Acetonitril)
   Massenspektrum (ESI⁺): m/z = 403, 405 [M+H]⁺
(9) 1-(2-Phenylethyl)-3-methyl-7-(3-methyl-2-buten-1-yl)-8-brom-xanthin
   Retentionszeit: 3,29 min (HPLC, Multosphere 100FBS, 50 mm, 50% Acetonitril)
(10) 1-(3-Phenylpropyl)-3-methyl-7-(3-methyl-2-buten-1-yl)-8-brom-xanthin
   Retentionszeit: 2,95 min (HPLC, Multosphere 100FBS, 50 mm, 50% Acetonitril)
(11) 1-(2-Hydroxyethyl)-3-methyl-7-(3-methyl-2-buten-1-yl)-8-brom-xanthin
   Retentionszeit: 2,35 min (HPLC, Multosphere 100FBS, 50 mm, 20% Acetonitril)
(12) 1-(2-Methoxyethyl)-3-methyl-7-(3-methyl-2-buten-1-yl)-8-brom-xanthin
   Retentionszeit: 2,54 min (HPLC, Multosphere 100FBS, 50 mm, 30% Acetonitril)
(13) 1-(3-Hydroxypropyl)-3-methyl-7-(3-methyl-2-buten-1-yl)-8-brom-xanthin
   Retentionszeit: 2,52 min (HPLC, Multosphere 100FBS, 50 mm, 20% Acetonitril)
(14) 1-[2-(Dimethylamino)ethyl]-3-methyl-7-(3-methyl-2-buten-1-yl)-8-brom-xanthin
   Retentionszeit: 2,73 min (HPLC, Multosphere 100FBS, 50 mm, 5% Acetonitril)
(15) 1-[3-(Dimethylamino)propyl]-3-methyl-7-(3-methyl-2-buten-1-yl)-8-brom-xanthin Retentionszeit: 2,79 min (HPLC, Multosphere 100FBS, 50 mm, 5% Acetonitril)
(16) 1-Methyl-3-(cyclopropylmethyl)-7-benzyl-xanthin
   Durchführung mit Methyljodid bei Raumtemperatur
   Massenspektrum (ESI⁺): m/z = 311 [M+H]⁺
(17) 1-Methyl-3-ethyl-7-benzyl-xanthin
   Durchführung mit Methyljodid bei Raumtemperatur
(18) 1-Methyl-3-(4-methoxy-benzyl)-7-benzyl-xanthin
   Durchführung mit Methyljodid bei Raumtemperatur
   Massenspektrum (ESI⁺): m/z = 377 [M+H]⁺
(19) 1-Methyl-3,7-dibenzyl-xanthin
   Durchführung mit Methyljodid bei Raumtemperatur
   R_{f}-Wert: 0.51 (Kieselgel, Methylenchlorid/Methanol/konz. wässriges Ammoniak = 95:5:1)
   Massenspektrum (ESI⁺): m/z = 347 [M+H]⁺
(20) 1-Methyl-3-[(methoxycarbonyl)-methyl]-7-benzyl-xanthin
   Durchführung mit Methyljodid bei Raumtemperatur
   Schmelzpunkt: 182°C
   Massenspektrum (ESI⁺): m/z = 329 [M+H]⁺
(21) 1-Methyl-3-isopropyl-7-benzyl-xanthin
   Durchführung mit Methyljodid bei Raumtemperatur
   R_{f}-Wert 0.66 (Kieselgel, Methylenchlorid/Methanol/konz. wässriges Ammoniak = 9:1:0.1)
   Massenspektrum (ESI⁺): m/z = 299 [M+H]⁺
(22) 1-Methyl-3-hexyl-7-benzyl-xanthin
   Durchführung mit Methyljodid bei Raumtemperatur
   R_{f}-Wert: 0.77 (Kieselgel, Methylenchlorid/Methanol/konz. wässriges Ammoniak = 95:5:1)
   Massenspektrum (ESI⁺): m/z = 341 [M+H]⁺
(23) 1-Methyl-3-(2-trimethylsilanyl-ethoxymethyl)-7-benzyl-xanthin
   Durchführung mit Methyljodid bei Raumtemperatur
(24) 1-Methyl-3-(2-methoxy-ethyl)-7-benzyl-xanthin
   Durchführung mit Methyljodid bei Raumtemperatur
   R_{f}-Wert: 0.70 (Kieselgel, Methylenchlorid/Methanol/konz. wässriges Ammoniak = 9:1:0.1)
   Massenspektrum (ESI⁺): m/z = 315 [M+H]⁺
(25) 1-Methyl-3-cyanomethyl-7-benzyl-xanthin
   Durchführung mit Methyljodid bei Raumtemperatur
   R_{f}-Wert: 0.74 (Kieselgel, Methylenchlorid/Methanol/konz. wässriges Ammoniak = 9:1:0.1)
   Massenspektrum (ESI⁺): m/z = 296 [M+H]⁺
(26) 1-Methyl-3-(2-hydroxy-ethyl)-7-benzyl-xanthin
   Durchführung mit Methyljodid bei Raumtemperatur
   R_{f}-Wert: 0.44 (Kieselgel, Methylenchlorid/Methanol/konz. wässriges Ammoniak = 9:1:0.1)
   Massenspektrum (ESI⁺): m/z = 301 [M+H]⁺
(27) 1-Methyl-3-(2-trimethylsilanyl-ethoxymethyl)-7-benzyl-xanthin
   Durchführung mit Methyljodid bei Raumtemperatur
   R_{f}-Wert: 0.44 (Kieselgel, Methylenchlorid/Methanol = 95:5)
   Massenspektrum (ESI⁺): m/z = 387 [M+H]⁺
(28) 1-(2-Phenyl-ethyl)-3-methyl-7-benzyl-8-chlor-xanthin
   Durchführung mit 2-Phenyl-ethylbromid bei 60°C
   Massenspektrum (ESI⁺): m/z = 395, 397 [M+H]⁺
(29) 1-(2-Phenyl-ethyl)-3-methyl-7-cyclopropylmethyl-8-chlor-xanthin
   Durchführung mit 2-Phenyl-ethylbromid bei 60°C
   Massenspektrum (ESI⁺): m/z = 359, 361 [M+H]⁺
(30) 1-(2-Phenyl-ethyl)-3-methyl-7-(2-butin-1-yl)-8-chlor-xanthin
   Massenspektrum (ESI⁺): m/z = 357, 359 [M+H]⁺
(31) 1-(2-Phenyl-ethyl)-3-methyl-7-(3-methyl-2-buten-1-yl)-8-chlor-xanthin
   Massenspektrum (ESI⁺): m/z = 395, 397 [M+Na]⁺
(32) 1-[(Methoxycarbonyl)-methyl]-3-methyl-7-(3-methyl-2-buten-1-yl)-8-[(S)-3-(tert.-butyloxycarbonylamino)-piperidin-1-yl]-xanthin
   Durchführung mit Bromessigsäuremethylester bei 50°C
   Schmelzpunkt: 143-145°C
   Massenspektrum (ESI⁺): m/z = 505 [M+H]⁺
(33) 1-[3-(Methoxycarbonyl)-propyl]-3-methyl-7-(3-methyl-2-buten-1-yl)-8-[(S)-3-(tert.-butyloxycarbonylamino)-piperidin-1-yl]-xanthin
   Durchführung mit 4-Brombuttersäuremethylester bei 50°C
   Schmelzpunkt: 130-131 °C
   Massenspektrum (ESI⁺): m/z = 533 [M+H]⁺
(34) 1-{2-[4-(Ethoxycarbonyl)-phenyl]-ethyl}-3-methyl-7-(3-methyl-2-buten-1-yl)-8-[(S)-3-(tert.-butyloxycarbonylamino)-piperidin-1-yl]-xanthin
   Durchführung mit 4-(2-Brom-ethyl)-benzoesäureethylester bei 50°C
   R_{f}-Wert: 0.40 (Kieselgel, Cyclohexan/Essigester = 1:1)
   Massenspektrum (ESI⁺): m/z = 609 [M+H]⁺
(35) 1-[2-(Methoxycarbonyl)-ethyl]-3-methyl-7-(3-methyl-2-buten-1-yl)-8-[(S)-3-(tert.-butyloxycarbonylamino)-piperidin-1-yl]-xanthin
   Durchführung mit 3-Brompropionsäuremethylester bei 50°C
   R_{f}-Wert: 0.35 (Kieselgel, Cyclohexan/Essigester = 1:1)
   Massenspektrum (ESI⁺): m/z = 519 [M+H]⁺
(36) 1-Cyanomethyl-3-methyl-7-(3-methyl-2-buten-1-yl)-8-brom-xanthin
   R_{f}-Wert: 0.58 (Kieselgel, Petrolether/Essigester/Methanol = 6:3.5:0.5)
   Massenspektrum (ESI⁺): m/z = 352, 354 [M+H]⁺
(37) 1-(2-Phenyl-2-oxo-ethyl)-3-methyl-7-(3-methyl-2-buten-1-yl)-8-[3-(tert.-butyloxycarbonylamino)-piperidin-1-yl]-xanthin
   R_{f}-Wert: 0.30 (Kieselgel, Petrolether/Essigester/Methanol = 7:2.5:0.5)
   Massenspektrum (ESI⁺): m/z = 551 [M+H]⁺
(38) 1-[2-(2-Methoxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 581 [M+H]⁺
(39) 1-[2-(Thiophen-3-yl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 557 [M+H]⁺
(40) 1-[2-(4-Methoxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 581 [M+H]⁺
(41) 1-(2-Phenyl-2-oxo-ethyl)-3-methyl-7-(3-methyl-2-buten-1-yl)-8-[(S)-3-(tert.-butyloxycarbonylamino)-piperidin-1-yl]-xanthin
(42) 1-(2-Phenyl-2-oxo-ethyl)-3-methyl-7-(3-methyl-2-buten-1-yl)-8-[(R)-3-(tert.-butyloxycarbonylamino)-piperidin-1-yl]-xanthin
   Massenspektrum (ESI⁺): m/z = 551 [M+H]⁺
(43) 1-(Phenylsulfanylmethyl)-3-methyl-7-(3-methyl-2-buten-1-yl)-8-[3-(tert.-butyloxycarbonylamino)-piperidin-1-yl]-xanthin
   R_{f}-Wert: 0.30 (Kieselgel, Petrolether/Essigester/Methanol = 7:2:1)
   Massenspektrum (ESI⁺): m/z = 555 [M+H]⁺
(44) 1-[2-(3-Methoxy-phenyl)-2-oxo-ethyl]-3-methyl-7-(3-methyl-2-buten-1-yl)-8-[3-(tert.-butyloxycarbonylamino)piperidin-1-yl]-xanthin
   R_{f}-Wert: 0.30 (Kieselgel, Petrolether/Essigester/Methanol = 7:2:1)
(45) 1-[2-(4-Methyl-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.20 (Kieselgel, Petrolether/Essigester/Methanol = 7:2:1)
   Massenspektrum (ESI⁺): m/z = 565 [M+H]⁺
(46) 1-(2-Methoxycarbonyl-2-propen-1-yl)-3-methyl-7-(3-methyl-2-buten-1-yl)-8-[3-(tert.-butyloxycarbonylamino)-piperidin-1-yl]-xanthin
   R_{f}-Wert: 0.15 (Kieselgel, Petrolether/Essigester/Methanol = 75:20:5)
   Massenspektrum (ESI⁺): m/z = 531 [M+H]⁺
(47) 1-(3-Oxo-3-phenyl-propyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 565 [M+H]⁺
(49) 1-(2-Oxo-propyl)-3-methyl-7-(3-methyl-2-buten-1-yl)-8-[3-(tert.-butyloxycarbonylamino)-piperidin-1-yl]-xanthin
   R_{f}-Wert: 0.10 (Kieselgel, Petrolether/Essigester/Methanol = 6:3:1)
   Massenspektrum (ESI⁺): m/z = 489 [M+H]⁺
(50) 1-(2-Phenyl-2-oxo-ethyl)-3-methyl-7-(2-cyano-benzyl)-8-[3-(tert.-butyloxycarbonylamino)-piperidin-1-yl]-xanthin
   Massenspektrum (ESI⁺): m/z = 598 [M+H]⁺
(51) 1-(2-Phenyl-ethyl)-3-methyl-7-(2-cyano-benzyl)-8-[3-(tert.-butyloxycarbonylamino)-piperidin-1-yl]-xanthin
   R_{f}-Wert: 0.50 (Kieselgel, Cyclohexan/Essigester = 1:1)
   Massenspektrum (ESI⁺): m/z = 584 [M+H]⁺
(52) 1-(3-Methoxycarbonyl-2-propen-1-yl)-3-methyl-7-(3-methyl-2-buten-1-yl)-8-[3-(tert.-butyloxycarbonylamino)-piperidin-1-yl]-xanthin
   Massenspektrum (ESI⁺): m/z = 531 [M+H]⁺
(53) 1-[2-(2,5-Dimethoxy-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.31 (Kieselgel, Cyclohexan/Essigester/Methanol = 6:3:1)
(54) 1-[2-(4-Fluor-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.40 (Kieselgel, Petrolether/Essigester/Methanol = 6:3:1)
(55) 1-[2-(3-Hydroxy-phenyl)-2-oxo-ethyl]-3-methyl-7-(3-methyl-2-buten-1-yl)-8-[3-(tert.-butyloxycarbonylamino)-piperidin-1-yl]-xanthin
   (Durch Umsetzung von Beispiel II(18) mit 2-Brom-1-(3-(tert.-butyl-dimethyl-silanyloxy)-phenyl]-ethanon in Gegenwart von Kalium-tert.-butylat in Dimethylformamid bei Raumtemperatur)
   Massenspektrum (ESI⁺): m/z = 567 [M+H]⁺
(56) 1-(3-Methoxycarbonyl-2-propen-1-yl)-3-methyl-7-(2-cyano-benzyl)-8-[3-(tert.-butyloxycarbonylamino)-piperidin-1-yl]-xanthin
   R_{f}-Wert: 0.50 (Kieselgel, Cyclohexan/Essigester = 1:1)
   Massenspektrum (ESI⁺): m/z = 600 [M+Na]⁺
(57) 1-[(Pyridin-2-yl)methyl]-3-methyl-7-(2-cyano-benzyl)-8-[3-(tert.-butyloxycarbonylamino)-piperidin-1-yl]-xanthin
   Massenspektrum (ESI⁺): m/z = 571 [M+H]⁺
(58) 1-(2-Phenyl-2-oxo-ethyl)-3-[(methoxycarbonyl)methyl]-7-(3-methyl-2-buten-1-yl)-8-[3-(tert.-butyloxycarbonylamino)-piperidin-1-yl]-xanthin
   R_{f}-Wert: 0.68 (Kieselgel, Methylenchlorid/Methanol/konz. wässriges Ammoniak = 90:10:1)
   Massenspektrum (ESI⁺): m/z = 609 [M+H]⁺
(59) 1-(2-Phenyl-2-oxo-ethyl)-3-methyl-7-(3-methyl-2-buten-1-yl)-8-chlor-xanthin
   R_{f}-Wert: 0.55 (Kieselgel, Cyclohexan/Essigester/Methanol = 6:3:1)
   Massenspektrum (ESI⁺): m/z = 387, 389 [M+H]⁺
(60) 1-[2-(3-Allyloxycarbonylamino-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.40 (Kieselgel, Cyclohexan/Essigester/Methanol = 6:3:1)
   Massenspektrum (ESI⁺): m/z = 650 [M+H]⁺
(61) 1-[2-(3-Nitro-phenyl)-2-oxo-ethyl]-3-methyl-7-(3-methyl-2-buten-1-yl)-8-chlor-xanthin
   Massenspektrum (ESI⁺): m/z = 432, 434 [M+H]⁺
(62) 1-[2-(2-Brom-5-dimethylamino-phenyl)-2-oxo-ethyl]-3-methyl-7-(3-methyl-2-buten-1-yl)-8-[3-(tert.-butyloxycarbonylamino)-piperidin-1-yl]-xanthin
(63) 1-[(Thiazol-2-yl)methyl]-3-methyl-7-(3-methyl-2-buten-1-yl)-8-[3-(tert.-butyloxycarbonylamino)-piperidin-1-yl]-xanthin
   R_{f}-Wert: 0.34 (Kieselgel, Methylenchlorid/Methanol = 95:5)
   Massenspektrum (ESI⁺): m/z = 530 [M+H]⁺
(64) 1-[(Benzo[d]isothiazol-3-yl)methyl]-3-methyl-7-(3-methyl-2-buten-1-yl)-8-[3-(tert.-butyloxycarbonylamino)-piperidin-1-yl]-xanthin
   R_{f}-Wert: 0.40 (Kieselgel, Cyclohexan/Essigester = 1:1)
   Massenspektrum (ESI⁺): m/z = 580 [M+H]⁺
(65) 1-[(Isoxazol-3-yl)methyl]-3-methyl-7-(3-methyl-2-buten-1-yl)-8-[3-(tert.-butyloxycarbonylamino)-piperidin-1-yl]-xanthin
   R_{f}-Wert: 0.20 (Kieselgel, Essigester)
   Massenspektrum (ESI⁺): m/z = 514 [M+H]⁺
(66) 1-[(1-Naphthyl)methyl]-3-methyl-7-(3-methyl-2-buten-1-yl)-8-[3-(tert.-butyloxycarbonylamino)-piperidin-1-yl]-xanthin
   R_{f}-Wert: 0.41 (Kieselgel, Cyclohexan/Essigester = 1:1)
   Massenspektrum (ESI⁺): m/z = 595 [M+Na]⁺
(67) 1-[(Benzo[d]isoxazol-3-yl)methyl]-3-methyl-7-(3-methyl-2-buten-1-yl)-8-[3-(tert.-butyloxycarbonylamino)-piperidin-1-yl]-xanthin
   R_{f}-Wert: 0.60 (Kieselgel, Methylenchlorid/Methanol = 95:5)
   Massenspektrum (ESI⁺): m/z = 564 [M+H]⁺
(68) 1-Cyanomethyl-3-methyl-7-(2-cyano-benzyl)-8-[3-(tert.-butyloxycarbonylamino)-piperidin-1-yl]-xanthin
   R_{f}-Wert: 0.40 (Kieselgel, Cyclohexan/Essigester = 1:1)
   Massenspektrum (ESI⁺): m/z = 541 [M+Na]⁺
(69) 1-[2-(2-Nitro-phenyl)-2-oxo-ethyl]-3-methyl-7-(3-methyl-2-buten-1-yl)-8-chlor-xanthin
   R_{f}-Wert: 0.25 (Kieselgel, Cyclohexan/Essigester/Methanol = 7:2:1)
   Massenspektrum (ESI⁺): m/z = 432, 434 [M+H]⁺
(70) 1-[(6-Methyl-pyridin-2-yl)methyl]-3-methyl-7-(3-methyl-2-buten-1-yl)-8-[3-(tert.-butyloxycarbonylamino)-piperidin-1-yl]-xanthin
   Durchführung in Gegenwart von Natriumiodid.
   R_{f}-Wert: 0.47 (Kieselgel, Essigester)
   Massenspektrum (ESI⁺): m/z = 538 [M+H]⁺,
(71) 1-Cyanomethyl-3-methyl-7-(2-cyano-benzyl)-8-[3-(tert.-butyloxycarbonylamino)-piperidin-1-yl]-xanthin
   R_{f}-Wert: 0.40 (Kieselgel, Cyclohexan/Essigester = 1:1)
(72) 1-[2-(2-Methoxy-phenyl)-2-oxo-ethyl]-3-methyl-7-(3-methyl-2-buten-1-yl)-8-chlor-xanthin
   Massenspektrum (ESI⁺): m/z = 417, 419 [M+H]⁺
(73) 1-Methyl-3-(2-trimethylsilanyl-ethoxymethyl)-7-(2-cyano-benzyl)-xanthin
   Massenspektrum (ESI⁺): m/z = 412 [M+H]⁺
(74) 1-[(3-Methyl-pyridin-2-yl)methyl]-3-methyl-7-(3-methyl-2-buten-1-yl)-8-[3-(tert.-butyloxycarbonylamino)-piperidin-1-yl]-xanthin
   R_{f}-Wert: 0.27 (Kieselgel, Essigester)
   Massenspektrum (ESI⁺): m/z = 538 [M+H]⁺
(75) 1-[(5-Methyl-pyridin-2-yl)methyl]-3-methyl-7-(3-methyl-2-buten-1-yl)-8-[3-(tert.-butyloxycarbonylamino)-piperidin-1-yl]-xanthin
   R_{f}-Wert: 0.45 (Kieselgel, Essigester)
   Massenspektrum (ESI⁺): m/z = 538 [M+H]⁺
(76) 1-[(4-Methyl-pyridin-2-yl)methyl]-3-methyl-7-(3-methyl-2-buten-1-yl)-8-[3-(tert.-butyloxycarbonylamino)-piperidin-1-yl]-xanthin
   R_{f}-Wert: 0.26 (Kieselgel, Essigester)
   Massenspektrum (ESI⁺): m/z = 538 [M+H]⁺
(77) 1-[(5-Nitro-isochinolin-1-yl)methyl]-3-methyl-7-(3-methyl-2-buten-1-yl)-8-[3-(tert.-butyloxycarbonylarnino)-piperidin-1-yl]-xanthin
   R_{f}-Wert: 0.54 (Kieselgel, Methylenchlorid/Methanol = 95:5)
(78) 1-[(2-Oxo-1,2-dihydro-chinolin-4-yl)methyl]-3-methyl-7-(3-methyl-2-buten-1-yl)-8-[3-(tert.-butyloxycarbonylamino)-piperidin-1-yl]-xanthin
   R_{f}-Wert: 0.38 (Kieselgel, Methylenchlorid/Methanol = 95:5)
   Massenspektrum (ESI⁺): m/z = 590 [M+H]⁺
(79) 1-[2-(3-Cyano-phenyl)-2-oxo-ethyl]-3-methyl-7-(3-methyl-2-buten-1-yl)-8-chlor-xanthin
   R_{f}-Wert: 0.52 (Kieselgel, Cyclohexan/Essigester = 1:1)
   Massenspektrum (ESI⁺): m/z = 434, 436 [M+Na]⁺
(80) 1-[2-(3-Aminosulfonyl-phenyl)-2-oxo-ethyl]-3-methyl-7-(3-methyl-2-buten-1-yl)-8-chlor-xanthin
   R_{f}-Wert: 0.25 (Kieselgel, Cyclohexan/Essigester = 1:1)
   Massenspektrum (ESI⁺): m/z = 466, 468 [M+H]⁺
(81) 1-[2-(3-Aminocarbonyl-phenyl)-2-oxo-ethyl]-3-methyl-7-(3-methyl-2-buten-1-yl)-8-chlor-xanthin
   R_{f}-Wert: 0.10 (Kieselgel, Cyclohexan/Essigester = 1:1)
   Massenspektrum (ESI⁺): m/z = 430, 432 [M+H]⁺
(82) 1-(2-Phenoxy-ethyl)-3-methyl-7-(3-methyl-2-buten-1-yl)-8-[3-(tert.-butyloxy-carbonylamino)-piperidin-1-yl]-xanthin
   R_{f}-Wert: 0.75 (Kieselgel, Cyclohexan/Essigester = 1:4)
   Massenspektrum (ESI⁺): m/z = 553 [M+H]⁺

### Beispiel X

### 1-Benzyl-3-(tert-butyloxycarbonylamino)-4-methyl-piperidin

hergestellt durch katalytische Hydrierung von 1-Benzyl-3-(tert.-butyloxycarbonylamino)-4-methyl-pyridinium-bromid in Methanol in Gegenwart von Platindioxid und einem Wasserstoffdruck von 4 bar.
Massenspektrum (EI): m/z = 304 [M]⁺

### Beispiel XI

### 1-Benzyl-3-(tert.-butyloxycarbonylamino)-4-methyl-pyridinium-bromid

hergestellt durch Umsetzung von 3-(tert.-Butyloxycarbonylamino)-4-methyl-pyridin mit Benzylbromid in Toluol
Schmelzpunkt: 200-201 °C

### Beispiel XII

### 1-[2-(2,4,6-Trimethyl-phenyl)-ethyl]-3-methyl-7-(3-methyl-2-buten-1-yl)-8-brom-xanthin

hergestellt durch Umsetzung von 3-Methyl-7-(3-methyl-2-buten-1-yl)-8-brom-xanthin mit 2-(2,4,6-Trimethyl-phenyl)-ethanol in Gegenwart von Triphenylphosphin und Diisopropylazodicarboxylat in Tetrahydrofuran bei Raumtemperatur
R_{f}-Wert: 0.40 (Kieselgel, Methylenchlorid/Essigester = 15:1)
Massenspektrum (ESI⁺): m/z = 459, 461 [M+H]⁺

Analog Beispiel XII werden folgende Verbindungen erhalten:
(1) 1-[2-(2,4-Dichlor-phenyl)-ethyl]-3-methyl-7-(3-methyl-2-buten-1-yl)-8-brom-xanthin
   R_{f}-Wert: 0.40 (Kieselgel, Methylenchlorid/Essigester = 15:1)
   Massenspektrum (EI): m/z = 484, 486, 488 [M]⁺
(2) 1-[2-(Thiophen-2-yl)-ethyl]-3-methyl-7-(3-methyl-2-buten-1-yl)-8-brom-xanthin
   R_{f}-Wert: 0.50 (Kieselgel, Methylenchlorid/Essigester =15:1)
   Massenspektrum (EI): m/z = 422, 424 [M]⁺
(3) 1-[2-(Thiophen-3-yl)-ethyl]-3-methyl-7-(3-methyl-2-buten-1-yl)-8-brom-xanthin Schmelzpunkt: 173.8-174.5°C
   Massenspektrum (ESI⁺): m/z = 445, 447 [M+Na]⁺
(4) 1-[2-(4-tert.-Butyl-phenyl)-ethyl]-3-methyl-7-(3-methyl-2-buten-1-yl)-8-brom-xanthin
   R_{f}-Wert: 0.85 (Kieselgel, Methylenchlorid/Methanol = 30:1)
   Massenspektrum (ESI⁺): m/z = 473, 475 [M+H]⁺
(5) 1-[2-(4-Fluor-phenyl)-ethyl]-3-methyl-7-(3-methyl-2-buten-1-yl)-8-brom-xanthin
   R_{f}-Wert: 0.70 (Kieselgel, Methylenchlorid/Essigester = 15:1)
(6) 1-[2-(4-Methoxy-phenyl)-ethyl]-3-methyl-7-(3-methyl-2-buten-1-yl)-8-brom-xanthin
   R_{f}-Wert: 0.70 (Kieselgel, Methylenchlorid/Essigester = 15:1)
(7) 1-[2-(2-Fluor-phenyl)-ethyl]-3-methyl-7-(3-methyl-2-buten-1-yl)-8-chlor-xanthin
   R_{f}-Wert: 0.75 (Kieselgel, Methylenchlorid/Essigester = 20:1)
   Massenspektrum (ESI⁺): m/z = 391, 393 [M+H]⁺
(8) 1-[2-(2-Methyl-phenyl)-ethyl]-3-methyl-7-(3-methyl-2-buten-1-yl)-8-chlor-xanthin
   R_{f}-Wert: 0.60 (Kieselgel, Methylenchlorid/Essigester = 20:1)
   Massenspektrum (ESI⁺): m/z = 387, 389 [M+H]⁺
(9) 1-[2-(3-Methyl-phenyl)-ethyl]-3-methyl-7-(3-methyl-2-buten-1-yl)-8-chlor-xanthin
   R_{f}-Wert: 0.80 (Kieselgel, Methylenchlorid/Essigester = 20:1)
   Massenspektrum (EI): m/z = 386, 388 [M]⁺
(10) 1-[2-(1-Naphthyl)-ethyl]-3-methyl-7-(3-methyl-2-buten-1-yl)-8-chlor-xanthin
   R_{f}-Wert: 0.70 (Kieselgel, Methylenchlorid/Essigester = 20:1)
   Massenspektrum (ESI⁺): m/z = 423, 425 [M+H]⁺
(11) 1-[2-(2-Naphthyl)-ethyl]-3-methyl-7-(3-methyl-2-buten-1-yl)-8-chlor-xanthin
   R_{f}-Wert: 0.72 (Kieselgel, Methylenchlorid/Essigester = 20:1)
   Massenspektrum (ESI⁺): m/z = 423, 425 [M+H]⁺
(12) 1-(4-Phenyl-butyl]-3-methyl-7-(3-methyl-2-buten-1-yl)-8-chlor-xanthin
   Massenspektrum (ESI⁺): m/z = 401, 403 [M+H]⁺
(13) 1-[2-(3-Trifluormethyl-phenyl)-ethyl]-3-methyl-7-(3-methyl-2-buten-1-yl)-8-chlor-xanthin
   R_{f}-Wert: 0.55 (Kieselgel, Petrolether/Essigester/Methanol = 75:20:5)
   Massenspektrum (ESI⁺): m/z = 463, 465 [M+Na]⁺
(14) 1-[2-(Pyridin-2-yl)-ethyl]-3-methyl-7-(3-methyl-2-buten-1-yl)-8-brom-xanthin
   Massenspektrum (ESI⁺): m/z = 417, 419 [M+H]⁺
(15) 1-(2-(Pyrrol-1-yl)-ethyl]-3-methyl-7-(3-methyl-2-buten-1-yl)-8-chlor-xanthin
   R_{f}-Wert: 0.40 (Kieselgel, Petrolether/Essigester/Methanol = 75:20:5)
   Massenspektrum (ESI⁺): m/z = 384, 386 [M+Na]⁺
(16) 1-[2-([1,2,3]Triazol-1-yl)-ethyl]-3-methyl-7-(3-methyl-2-buten-1-yl)-8-chlor-xanthin
   R_{f}-Wert: 0.22 (Kieselgel, Petrolether/Essigester/Methanol = 7:2:1)
   Massenspektrum (ESI⁺): m/z = 364, 366 [M+H]⁺
(17) 1-[2-(Pyridin-4-yl)-ethyl]-3-methyl-7-(3-methyl-2-buten-1-yl)-8-chlor-xanthin
   R_{f}-Wert: 0.15 (Kieselgel, Petrolether/Essigester/Methanol = 7:2:1)
   Massenspektrum (ESI⁺): m/z = 374, 376 [M+H]⁺
(18) 1-(3-Butin-1-yl)-3-methyl-7-(3-methyl-2-buten-1-yl)-8-brom-xanthin
   R_{f}-Wert: 0.45 (Kieselgel, Petrolether/Essigester = 7:3)
   Massenspektrum (ESI⁺): m/z = 387, 389 [M+Na]⁺
(19) 1-(3-Buten-1-yl)-3-methyl-7-(3-methyl-2-buten-1-yl)-8-brom-xanthin
   R_{f}-Wert: 0.45 (Kieselgel, Petrolether/Essigester = 7:3)
   Massenspektrum (ESI⁺): m/z = 389, 391 [M+Na]⁺
(20) 1-(4-Pentin-1-yl)-3-methyl-7-(3-methyl-2-buten-1-yl)-8-chlor-xanthin
   R_{f}-Wert: 0.37 (Kieselgel, Petrolether/Essigester/Methanol = 80:15:5)
   Massenspektrum (EI): m/z = 378, 380 [M]⁺
(21) 1-(4-Penten-1-yl)-3-methyl-7-(3-methyl-2-buten-1-yl)-8-brom-xanthin
   R_{f}-Wert: 0.30 (Kieselgel, Petrolether/Essigester = 8:2)
   Massenspektrum (ESI⁺): m/z = 381, 383 [M+H]⁺
(22) 1-{2-[4-(tert.-Butyl-dimethyl-silanyloxy)-phenyl]-ethyl}-3-methyl-7-(3-methyl-2-buten-1-yl)-8-[(S)-3-(tert.-butyloxycarbonylamino)-piperidin-1-yl]-xanthin
   R_{f}-Wert: 0.68 (Kieselgel, Cyclohexan/Essigester = 3:1)
   Massenspektrum (ESI⁺): m/z = 667 [M+H]⁺
(23) 1-{2-[3-(tert.-Butyl-dimethyl-silanyloxy)-phenyl]-ethyl}-3-methyl-7-(3-methyl-2-buten-1-yl)-8-[(S)-3-(tert.-butyloxycarbonylamino)-piperidin-1-yl]-xanthin
   R_{f}-Wert: 0.60 (Kieselgel, Cyclohexan/Essigester = 1:1)
   Massenspektrum (ESI⁺): m/z = 667 [M+H]⁺
(24) 1-[2-(Pyridin-3-yl)-ethyl]-3-methyl-7-(3-methyl-2-buten-1-yl)-8-brom-xanthin
   R_{f}-Wert: 0.17 (Kieselgel, Petrolether/Essigester/Methanol/konz. wässriges Ammoniak = 7:2:1:0.1)
   Massenspektrum (ESI⁺): m/z = 418, 420 [M+H]⁺
(25) 1-[2-(4-Methyl-thiazol-5-yl)-ethyl]-3-methyl-7-(3-methyl-2-buten-1-yl)-8-brom-xanthin
   R_{f}-Wert: 0.55 (Kieselgel, Petrolether/Essigester/Methanol = 5:4:1)
   Massenspektrum (ESI⁺): m/z = 438, 440 [M+H]⁺
(26) 1-[2-(3-Methoxy-phenyl)-ethyl]-3-methyl-7-(3-methyl-2-buten-1-yl)-8-brom-xanthin
   R_{f}-Wert: 0.60 (Kieselgel, Petrolether/Essigester/Methanol = 7:2.5:0.5)
   Massenspektrum (ESI⁺): m/z = 447, 449 [M+H]⁺
(27) 1-[2-(3-Brom-phenyl)-ethyl]-3-methyl-7-(3-methyl-2-buten-1-yl)-8-brom-xanthin
   R_{f}-Wert: 0.60 (Kieselgel, Petrolether/Essigester/Methanol = 7:2.5:0.5)
   Massenspektrum (EI): m/z = 494, 496, 498 [M]⁺
(28) 1-[2-(3-Chlor-phenyl)-ethyl]-3-methyl-7-(3-methyl-2-buten-1-yl)-8-brom-xanthin
   R_{f}-Wert: 0.60 (Kieselgel, Petrolether/Essigester/Methanol = 7:2.5:0.5)
   Massenspektrum (EI): m/z = 450, 452, 454 [M]⁺
(29) 1-[2-(2-Chlor-phenyl)-ethyl]-3-methyl-7-(3-methyl-2-buten-1-yl)-8-chlor-xanthin
   R_{f}-Wert: 0.65 (Kieselgel, Petrolether/Essigester/Methanol = 7:2.5:0.5)
   Massenspektrum (ESI⁺): m/z = 407, 409, 411 [M+H]⁺
(30) 1-[2-(2-Methoxy-phenyl)-ethyl]-3-methyl-7-(3-methyl-2-buten-1-yl)-8-chlor-xanthin
   R_{f}-Wert: 0.65 (Kieselgel, Petrolether/Essigester/Methanol = 7:2.5:0.5)
   Massenspektrum (ESI⁺): m/z = 403, 405 [M+H]⁺
(31) 1-[2-(2-Trifluormethyl-phenyl)-ethyl]-3-methyl-7-(3-methyl-2-buten-1-yl)-8-brom-xanthin
   R_{f}-Wert: 0.55 (Kieselgel, Petrolether/Essigester = 8:2)
   Massenspektrum (ESI⁺): m/z = 485, 487 [M+H]⁺
(32) 1-[2-(2-Brom-phenyl)-ethyl]-3-methyl-7-(3-methyl-2-buten-1-yl)-8-chlor-xanthin
   R_{f}-Wert: 0.55 (Kieselgel, Petrolether/Essigester = 8:2)
   Massenspektrum (ESI⁺): m/z = 451, 453, 455 [M+H]⁺
(33) 1-[2-(3-Fluor-phenyl)-ethyl]-3-methyl-7-(3-methyl-2-buten-1-yl)-8-chlor-xanthin
   R_{f}-Wert: 0.60 (Kieselgel, Petrolether/Essigester = 8:2)
   Massenspektrum (ESI⁺): m/z = 391, 393 [M+H]⁺
(34) 1-[2-(3-Nitro-phenyl)-ethyl]-3-methyl-7-(3-methyl-2-buten-1-yl)-8-chlor-xanthin
   R_{f}-Wert: 0.45 (Kieselgel, Petrolether/Essigester/Methanol = 7:2:1)
   Massenspektrum (ESI⁺): m/z = 440, 442 [M+Na]⁺
(35) 1-[2-(4-Methyl-phenyl)-ethyl]-3-methyl-7-(3-methyl-2-buten-1-yl)-8-chlor-xanthin
   R_{f}-Wert: 0.50 (Kieselgel, Petrolether/Essigester/Methanol = 7:2:1) Massenspektrum (ESI⁺): m/z = 387, 389 [M+H]⁺
(36) 1-[2-(2-Nitro-phenyl)-ethyl]-3-methyl-7-(3-methyl-2-buten-1-yl)-8-chlor-xanthin
   R_{f}-Wert: 0.85 (Kieselgel, Petrolether/Essigester/Methanol = 6:3:1)
   Massenspektrum (ESI⁺): m/z = 418, 420 [M+H]⁺
(37) 1-[2-(3,5-Difluor-phenyl)-ethyl]-3-methyl-7-(3-methyl-2-buten-1-yl)-8-chlor-xanthin
   R_{f}-Wert: 0.50 (Kieselgel, Petrolether/Essigester = 7:3)
   Massenspektrum (EI): m/z = 408, 410 [M]⁺
(38) 1-[2-(2,6-Difluor-phenyl)-ethyl]-3-methyl-7-(3-methyl-2-buten-1-yl)-8-chlor-xanthin
   R_{f}-Wert: 0.50 (Kieselgel, Petrolether/Essigester = 7:3)
   Massenspektrum (ESI⁺): m/z = 409, 411 [M+H]⁺
(39) 1-[2-(3,5-Dimethyl-phenyl)-ethyl]-3-methyl-7-(3-methyl-2-buten-1-yl)-8-chlor-xanthin
   R_{f}-Wert: 0.58 (Kieselgel, Petrolether/Essigester = 7:3)
   Massenspektrum (ESI⁺): m/z = 401, 403 [M+H]⁺
(40) 1-(2-Phenyl-propyl)-3-methyl-7-(3-methyl-2-buten-1-yl)-8-chlor-xanthin
   R_{f}-Wert: 0.60 (Kieselgel, Petrolether/Essigester/Methanol = 7:2:1)
   Massenspektrum (ESI⁺): m/z = 387, 389 [M+H]⁺
(41) 1-(2-Methoxy-2-phenyl-ethyl)-3-methyl-7-(3-methyl-2-buten-1-yl)-8-chlor-xanthin
   R_{f}-Wert: 0.70 (Kieselgel, Petrolether/Essigester/Methanol = 7:2:1)
   Massenspektrum (ESI⁺): m/z = 425, 427 [M+Na]⁺
(42) 1-[(Pyridin-2-yl)methyl]-3-methyl-7-(3-methyl-2-buten-1-yl)-8-chlor-xanthin
   R_{f}-Wert: 0.14 (Kieselgel, Petrolether/Essigester = 1:1)
   Massenspektrum (ESI⁺): m/z = 360, 362 [M+H]⁺
(43) 1-[(Isochinolin-1-yl)methyl]-3-methyl-7-(3-methyl-2-buten-1-yl)-8-chlor-xanthin
   R_{f}-Wert: 0.31 (Kieselgel, Cyclohexan/Essigester = 1:1)
   Massenspektrum (ESI⁺): m/z = 410, 412 [M+H]⁺
(44) 1-[(Pyridin-3-yl)methyl]-3-methyl-7-(3-methyl-2-buten-1-yl)-8-chlor-xanthin
   R_{f}-Wert: 0.10 (Kieselgel, Methylenchlorid/Methanol = 98:2)
   Massenspektrum (ESI⁺): m/z = 360, 362 [M+H]⁺
(45) 1-[(Pyridin-4-yl)methyl]-3-methyl-7-(3-methyl-2-buten-1-yl)-8-chlor-xanthin
   R_{f}-Wert: 0.24 (Kieselgel, Methylenchlorid/Methanol = 95:2)
   Massenspektrum (ESI⁺): m/z = 360, 362 [M+H]⁺
(46) 1-[(Isochinolin-4-yl)methyl]-3-methyl-7-(3-methyl-2-buten-1-yl)-8-chlor-xanthin
   R_{f}-Wert: 0.28 (Kieselgel, Essigester/Petrolether = 2:1)
   Massenspektrum (ESI⁺): m/z = 410, 412 [M+H]⁺
(47) 1-[(1-Methyl-1H-indazol-3-yl)methyl]-3-methyl-7-(3-methyl-2-buten-1-yl)-8-chlor-xanthin
   Massenspektrum (ESI⁺): m/z = 413, 415 [M+H]⁺
(48) 1-[(Chinolin-4-yl)methyl]-3-methyl-7-(3-methyl-2-buten-1-yl)-8-chlor-xanthin
   R_{f}-Wert: 0.39 (Kieselgel, Essigester)
   Massenspektrum (ESI⁺): m/z = 410, 412 [M+H]⁺
(49) 1-[(Chinolin-8-yl)methyl]-3-methyl-7-(3-methyl-2-buten-1-yl)-8-chlor-xanthin
   R_{f}-Wert: 0.74 (Kieselgel, Essigester)
   Massenspektrum (ESI⁺): m/z = 410, 412 [M+H]⁺

### Beispiel XIII

### 1,3-Dimethyl-5-[trans-2-(tert.-butyloxycarbonylamino)-cyclohexyl]-carbonylamino}-6-amino-uracil

hergestellt durch Behandeln von 1,3-Dimethyl-5-({trans-2-[(fluoren-9-ylmethoxy-carbonyl)amino]-cyclohexyl}-carbonylamino)-6-amino-uracil mit Piperidin in Dimethylformamid und anschließende Umsetzung mit Pyrokohlensäure-di-tert.-butylester
Massenspektrum (ESI⁺): m/z = 396 [M+H]⁺

### Beispiel XIV

### 1-Methyl-3-(2-propin-1-yl)-7-benzyl-8-chlor-xanthin

hergestellt durch Umsetzung von 1-Methyl-7-benzyl-8-chlor-xanthin mit Propargylbromid in Gegenwart von Kaliumcarbonat in Dimethylformamid bei Raumtemperatur
Schmelzpunkt: 169-172°C
Massenspektrum (EI): m/z = 328, 330 [M]⁺

Analog Beispiel XIV werden folgende Verbindungen erhalten:
(1) 1-Methyl-3-(2-propen-1-yl)-7-benzyl-8-chlor-xanthin
   R_{f}-Wert: 0.83 (Kieselgel, Methylenchlorid/Methanol = 95:5)
   Massenspektrum (EI): m/z = 330, 332 [M]⁺
(2) 1-Methyl-3-(2-phenyl-ethyl)-7-benzyl-8-chlor-xanthin
   Schmelzpunkt: 174-179°C
   Massenspektrum (ESI⁺): m/z = 395, 397 [M+H]⁺
(3) 1-Phenyl-3-methyl-7-(3-methyl-2-buten-1-yl)-8-[(3-(tert.-butyloxycarbonylamino)-piperidin-1-yl]-xanthin
   R_{f}-Wert: 0.66 (Aluminiumoxid, Essigester/Petrolether = 8:2)
   Massenspektrum (ESI⁺): m/z = 509 [M+H]⁺
(4) 1-Methyl-3-(2-dimethylamino-ethyl)-7-benzyl-8-chlor-xanthin
   R_{f}-Wert: 0.30 (Kieselgel, Methylenchlorid/Methanol/konz. wässriges Ammoniak = 9:1:0.1)
   Massenspektrum (ESI⁺): m/z = 362, 364 [M+H]⁺
(5) 1,3-Bis(2-phenyl-ethyl)-7-(3-methyl-2-buten-1-yl)-8-[3-(tert.-butyloxycarbonylamino)-piperidin-1-yl]-xanthin
   R_{f}-Wert: 0.79 (Kieselgel, Petrolether/Essigester = 4:6)
   Massenspektrum (ESI⁺): m/z = 627 [M+H]⁺
(6) 1-(2-Phenyl-ethyl)-3-cyanomethyl-7-(3-methyl-2-buten-1-yl)-8-[3-(tert.-butyloxycarbonylamino)-piperidin-1-yl]-xanthin
   R_{f}-Wert: 0.74 (Kieselgel, Essigester/Petrolether = 6:4)
   Massenspektrum (ESI⁺): m/z = 562 [M+H]⁺
(7) 1-(2-Phenyl-ethyl)-3-[(methoxycarbonyl)-methyl]-7-(3-methyl-2-buten-1-yl)-8-[3-(tert.-butyloxycarbonylamino)-piperidin-1-yl]-xanthin
   R_{f}-Wert: 0.65 (Kieselgel, Essigester/Petrolether = 6:4)
   Massenspektrum (ESI⁺): m/z = 595 [M+H]⁺
(8) 1-(2-Phenyl-ethyl)-3-(2-dimethylamino-ethyl)-7-(3-methyl-2-buten-1-yl)-8-[3-(tert.-butyloxycarbonylamino)-piperidin-1-yl]-xanthin
   R_{f}-Wert: 0.39 (Kieselgel, Methylenchlorid/Methanol/konz. wässriges Ammoniak = 90:10:1)
   Massenspektrum (ESI⁺): m/z = 594 [M+H]⁺
(9) 1-(2-Phenyl-ethyl)-3-(2-propin-1-yl)-7-(3-methyl-2-buten-1-yl)-8-[3-(tert.-butyloxycarbonylamino)-piperidin-1-yl]-xanthin
   R_{f}-Wert: 0.77 (Kieselgel, Essigester/Petrolether = 6:4)
   Massenspektrum (ESI⁺): m/z = 561 [M+H]⁺
(10) 1-Methyl-3-(2-phenyl-2-oxo-ethyl)-7-(3-methyl-2-buten-1-yl)-8-[3-(tert.-butyloxycarbonylamino)-piperidin-1-yl]-xanthin
   R_{f}-Wert: 0.69 (Kieselgel, Methylenchlorid/Methanol/konz. wässriges Ammoniak = 95:5:1)
   Massenspektrum (ESI⁺): m/z = 551 [M+H]⁺
(11) 1-Methyl-3-cyanomethyl-7-(3-methyl-2-buten-1-yl)-8-[3-(tert.-butyloxycarbonylamino)-piperidin-1-yl]-xanthin
   R_{f}-Wert: 0.80 (Kieselgel, Methylenchlorid/Methanol/konz. wässriges Ammoniak = 90:10:1)
   Massenspektrum (ESI⁺): m/z = 472 [M+H]⁺
(12) 1-Methyl-3-(2-phenyl-ethyl)-7-(3-methyl-2-buten-1-yl)-8-[3-(tert.-butyloxycarbonylamino)-piperidin-1-yl]-xanthin
   R_{f}-Wert: 0.88 (Kieselgel, Methylenchlorid/Methanol/konz. wässriges Ammoniak = 90:10:1)
   Massenspektrum (ESI⁺): m/z = 537 [M+H]⁺
(13) 1-Methyl-3-(2-dimethylamino-ethyl)-7-(3-methyl-2-buten-1-yl)-8-[3-(tert.-butyloxycarbonylamino)-piperidin-1-yl]-xanthin
   R_{f}-Wert: 0.21 (Kieselgel, Methylenchlorid/Methanol/konz. wässriges Ammoniak = 90:10:1)
   Massenspektrum (ESI⁺): m/z = 504 [M+H]⁺
(14) 1-Methyl-3-isopropyl-7-(3-methyl-2-buten-1-yl)-8-[3-(tert.-butyloxycarbonylamino)-piperidin-1-yl]-xanthin
   R_{f}-Wert: 0.54 (Kieselgel, Methylenchlorid/Methanol/konz. wässriges Ammoniak = 95:5:1)
(15) 1-Methyl-3-(2-cyano-ethyl)-7-(3-methyl-2-buten-1-yl)-8-[3-(tert.-butyloxycarbonylamino)-piperidin-1-yl]-xanthin
   R_{f}-Wert: 0.59 (Kieselgel, Methylenchlorid/Methanol/konz. wässriges Ammoniak = 90:10:1)
(16) 1-Methyl-3-[2-(4-methoxy-phenyl)-ethyl]-7-(3-methyl-2-buten-1-yl)-8-[3-(tert.-butyloxycarbonylamino)-piperidin-1-yl]-xanthin
   R_{f}-Wert: 0.88 (Kieselgel, Methylenchlorid/Methanol/konz. wässriges Ammoniak = 90:10:1)
   Massenspektrum (ESI⁺): m/z = 567 [M+H]⁺
(17) 1-Methyl-3-[2-(3-methoxy-phenyl)-ethyl]-7-(3-methyl-2-buten-1-yl)-8-(3-(tert.-butyloxycarbonylamino)-piperidin-1-yl]-xanthin
   R_{f}-Wert: 0.76 (Kieselgel, Methylenchlorid/Methanol/konz. wässriges Ammoniak = 90:10:1)
   Massenspektrum (ESI⁺): m/z = 567 [M+H]⁺
(18) 1-Methyl-3-[2-(2-methoxy-phenyl)-ethyl]-7-(3-methyl-2-buten-1-yl)-8-[3-(tert.-butyloxycarbonylamino)-piperidin-1-yl]-xanthin
   R_{f}-Wert: 0.68 (Kieselgel, Methylenchlorid/Methanol/konz. wässriges Ammoniak = 90:10:1)
(19) 1-Methyl-3-[2-(3-methyl-phenyl)-ethyl]-7-(3-methyl-2-buten-1-yl)-8-[3-(tert.-butyloxycarbonylamino)-piperidin-1-yl]-xanthin
   R_{f}-Wert: 0.81 (Kieselgel, Methylenchlorid/Methanol/konz. wässriges Ammoniak = 90:10:1)
   Massenspektrum (ESI⁺): m/z = 551 [M+H]⁺
(20) 1-Methyl-3-[2-(4-methyl-phenyl)-ethyl]-7-(3-methyl-2-buten-1-yl)-8-[3-(tert.-butyloxycarbonylamino)-piperidin-1-yl]-xanthin
   R_{f}-Wert: 0.81 (Kieselgel, Methylenchlorid/Methanol/konz. wässriges Ammoniak = 90:10:1)
   Massenspektrum (ESI⁺): m/z = 551 [M+H]⁺
(21) 1-Methyl-3-[2-(2-methyl-phenyl)-ethyl]-7-(3-methyl-2-buten-1-yl)-8-[3-(tert.-butyloxycarbonylamino)-piperidin-1-yl]-xanthin
   R_{f}-Wert: 0.72 (Kieselgel, Methylenchlorid/Methanol/konz. wässriges Ammoniak = 90:10:1)
(22) 1-Methyl-3-[2-(2-fluor-phenyl)-ethyl]-7-(3-methyl-2-buten-1-yl)-8-[3-(tert.-butyloxycarbonylamino)-piperidin-1-yl]-xanthin
   R_{f}-Wert: 0.89 (Kieselgel, Methylenchlorid/Methanol/konz. wässriges Ammoniak = 90:10:1)
   Massenspektrum (ESI⁺): m/z = 555 [M+H]⁺
(23) 1-Methyl-3-(4-phenyl-butyl)-7-(3-methyl-2-buten-1-yl)-8-[3-(tert.-butyloxycarbonylamino)-piperidin-1-yl]-xanthin
   R_{f}-Wert: 0.65 (Kieselgel, Methylenchlorid/Methanol/konz. wässriges Ammoniak = 90:10:1)
   Massenspektrum (ESI⁺): m/z = 565 [M+H]⁺
(24) 1-Methyl-3-(3-phenyl-propyl)-7-(3-methyl-2-buten-1-yl)-8-[3-(tert.-butyloxycarbonylamino)-piperidin-1-yl]-xanthin
   R_{f}-Wert: 0.84 (Kieselgel, Methylenchlorid/Methanol/konz. wässriges Ammoniak = 90:10:1)
   Massenspektrum (ESI⁺): m/z = 551 [M+H]⁺
(25) 1-Methyl-3-[2-(4-fluor-phenyl)-ethyl]-7-(3-methyl-2-buten-1-yl)-8-[3-(tert.-butyloxycarbonylamino)-piperidin-1-yl]-xanthin
   R_{f}-Wert: 0.80 (Kieselgel, Methylenchlorid/Methanol/konz. wässriges Ammoniak = 98:2:1)
   Massenspektrum (ESI⁺): m/z = 555 [M+H]⁺
(26) 1-Methyl-3-[2-(3-fluor-phenyl)-ethyl]-7-(3-methyl-2-buten-1-yl)-8-[3-(tert.-butyloxycarbonylamino)-piperidin-1-yl]-xanthin
   R_{f}-Wert: 0.82 (Kieselgel, Methylenchlorid/Methanol/konz. wässriges Ammoniak = 90:10:1)
   Massenspektrum (ESI⁺): m/z = 555 [M+H]⁺
(27) 1-Methyl-3-(2-phenyl-ethyl)-7-(2-cyano-benzyl)-8-chlor-xanthin
   Massenspektrum (ESI⁺): m/z = 420, 422 [M+H]⁺

### Beispiel XV

### 1-Methyl-7-benzyl-8-chlor-xanthin

hergestellt durch Behandeln von 1-Methyl-3-(2-trimethylsilanyl-ethoxymethyl)-7-benzyl-8-chlor-xanthin mit Trifluoressigsäure in Methylenchlorid bei Raumtemperatur R_{f}-Wert: 0.10 (Kieselgel, Methylenchlorid/Methanol = 98:2)

Analog Beispiel XV wird folgende Verbindung erhalten:
1) 1-Methyl-7-(2-cyano-benzyl)-8-chlor-xanthin
   Massenspektrum (ESI⁺): m/z = 338, 340 [M+Na]⁺

### Beispiel XVI

### 1,3-Dimethyl-7-(3-methyl-phenyl)-8-chlor-xanthin

hergestellt durch Umsetzung von 8-Chlor-theophyllin mit 3-Methylphenylboronsäure in Gegenwart von wasserfreiem Kuper(II)acetat, Pyridin und Molsieb 4Å in Methylenchlorid bei Raumtemperatur
Massenspektrum (ESI⁺): m/z = 305, 307 [M+H]⁺

Analog Beispiel XVI werden folgende Verbindungen erhalten:
(1) 1,3-Dimethyl-7-((E)-1-hexen-1-yl)-8-chlor-xanthin
   Massenspektrum (ESI⁺): m/z = 297, 299 [M+H]⁺
(2) 1,3-Dimethyl-7-((E)-2-phenyl-vinyl-8-chlor-xanthin
   Massenspektrum (ESI⁺): m/z = 317, 319 [M+H]⁺
(3) 1,3-Dimethyl-7-(2-naphthyl)-8-chlor-xanthin
   R_{f}-Wert: 0.60 (Kieselgel, Cyclohexan/Essigester = 1:1)
   Massenspektrum (ESI⁺): m/z = 341, 343 [M+H]⁺
(4) 1,3-Dimethyl-7-phenyl-8-chlor-xanthin
   R_{f}-Wert: 0.60 (Kieselgel, Cyclohexan/Essigester = 1:1)
   Massenspektrum (ESI⁺): m/z = 291, 293 [M+H]⁺
(5) 1,3-Dimethyl-7-(3,5-dimethyl-phenyl)-8-chlor-xanthin
   R_{f}-Wert: 0.60 (Kieselgel, Cyclohexan/Essigester = 1:1)
   Massenspektrum (ESI⁺): m/z = 319, 321 [M+H]⁺
(6) 1,3-Dimethyl-7-(4-methyl-phenyl)-8-chlor-xanthin
   R_{f}-Wert: 0.60 (Kieselgel, Cyclohexan/Essigester = 1:1)
   Massenspektrum (ESI⁺): m/z = 305, 307 [M+H]⁺
(7) 1,3-Dimethyl-7-(3-trifluormethyl-phenyl)-8-chlor-xanthin
   R_{f}-Wert: 0.60 (Kieselgel, Cyclohexan/Essigester = 1:1)
   Massenspektrum (ESI⁺): m/z = 381, 383 [M+Na]⁺
(8) 1,3-Dimethyl-7-(3-cyano-phenyl)-8-chlor-xanthin
   R_{f}-Wert: 0.50 (Kieselgel, Cyclohexan/Essigester = 1:1)
   Massenspektrum (ESI⁺): m/z = 338, 340 [M+Na]⁺
(9) 1,3-Dimethyl-7-(3-fluor-phenyl)-8-chlor-xanthin
   R_{f}-Wert: 0.50 (Kieselgel, Cyclohexan/Essigester = 1:1)
   Massenspektrum (EI): m/z = 308, 310 [M]⁺

### Beispiel XVII

### cis-N-Methyl-cyclohexan-1,2-diamin

hergestellt durch Behandeln von cis-N-(tert.-Butyloxycarbonyl)-cyclohexan-1,2-diamin mit Lithiumaluminiumhydrid in Tetrahydrofuran unter Rückfluß
R_{f}-Wert: 0.10 (Kieselgel, Methylenchlorid/Methanol/konz. wässriges Ammoniak = 90:10:1)
Massenspektrum (ESI⁺): m/z = 129 [M+H]⁺

### Beispiel XVIII

### 1-(tert.-Butyloxycarbonyl)-3-methylamino-piperidin

hergestellt durch Behandeln von 1-(tert.-Butyloxycarbonyl)-3-[N-(2,2,2-trifluoro-acetyl)-N-methyl-amino]-piperidin mit 2N Natronlauge in Methanol bei Raumtemperatur
R_{f}-Wert: 0.40 (Kieselgel, Methylenchlorid/Methanol/konz. wässriges Ammoniak = 90:10:1)
Massenspektrum (ESI⁺): m/z = 215 [M+H]⁺

Analog Beispiel XVIII werden folgende Verbindungen erhalten:
(1) 1-(tert.-Butyloxycarbonyl)-3-methylamino-pyrrolidin
   R_{f}-Wert: 0.42 (Kieselgel, Methylenchlorid/Methanol/konz. wässriges Ammoniak = 90:10:1)
   Massenspektrum (ESI⁺): m/z = 201 [M+H]⁺
(2) 2-[3-(tert.-Butyloxycarbonylamino)-piperidin-1-yl]-3-benzyl-4-ethoxycarbonyl-5-methylamino-3H-imidazol
   Durchführung mit Natriumethylat in Ethanol.
   R_{f}-Wert: 0.60 (Kieselgel, Petrolether/Essigester = 1:1)

### Beispiel XIX

### 1-(tert.-Butyloxycarbonyl)-3-[N-(2,2,2-trifluoro-acetyl)-N-methyl-amino]-piperidin

hergestellt durch Umsetzung von 1-(tert.-Butyloxycarbonyl)-3-[(2,2,2-trifluoroacetyl)amino]-piperidin mit Natriumhydrid und Methyljodid in Tetrahydrofuran bei Raumtemperatur
R_{f}-Wert: 0.78 (Kieselgel, Methylenchlorid/Methanol = 95:5)

Analog Beispiel XIX werden folgende Verbindungen erhalten:
(1) 1-(tert.-Butyloxycarbonyl)-3-[N-(2,2,2-trifluoro-acetyl)-N-methyl-amino]-pyrrolidin
(2) 2-[3-(tert.-Butyloxycarbonylamino)-piperidin-1-yl]-3-benzyl-4-ethoxycarbonyl-5-[N-(2,2,2-trifluoro-acetyl)-N-methyl-amino]-3H-imidazol
Durchführung mit Kaliumcarbonat in Dimethylformamid.
R_{f}-Wert: 0.60 (Kieselgel, Petrolether/Essigester = 1:1)

### Beispiel XX

### 1-(tert.-Butyloxycarbonyl)-3-[(2,2,2-trifluoro-acetyl)amino]-piperidin

hergestellt durch Umsetzung von 3-Amino-1-(tert.-butyloxycarbonyl)-piperidin mit Trifluoressigsäuremethylester in Methanol bei Raumtemperatur
R_{f}-Wert: 0.73 (Kieselgel, Methylenchlorid/Methanol/konz. wässriges Ammoniak = 90:10:1)
Massenspektrum (ESI⁻): m/z = 295 [M-H]⁻

Analog Beispiel XX wird folgende Verbindung erhalten:
(1) 2-[3-(tert.-Butyloxycarbonylamino)-piperidin-1-yl]-3-benzyl-4-ethoxycarbonyl-5-[(2,2,2-trifluoro-acetyl)amino]-3H-imidazol
   Durchführung mit Trifluoressigsäureanhydrid in Gegenwart von 4-Dimethylaminopyridin in Methylenchlorid bei Raumtemperatur.
   R_{f}-Wert: 0.70 (Kieselgel, Petrolether/Essigester = 1:1)

### Beispiel XXI

### (S)-2-Amino-1-methylamino-propan-dihydrochlorid

hergestellt durch Behandeln von (S)-Alaninmethylamid-hydrochlorid mit Lithiumaluminiumhydrid in Tetrahydrofuran unter Rückfluß und Fällung des nach der Aufarbeitung erhaltenen Produktes als Dihydrochlorid
R_{f}-Wert: 0.08 (Kieselgel, Methylenchlorid/Methanol/konz. wässriges Ammoniak = 90:10:1)
Massenspektrum (ESI⁻): m/z = 159, 161, 163 [M+HCl+Cl]⁻

Analog Beispiel XXI wird folgende Verbindung erhalten:
(1) (R)-2-Amino-1-methylamino-propan-dihydrochlorid
   Massenspektrum (EI): m/z = 88 [M]⁺

### Beispiel XXII

### 1-Phenyl-7-(3-methyl-2-buten-1-yl)-8-[3-(tert.-butyloxycarbonylamino)-piperidin-1-yl]-xanthin

hergestellt durch Behandeln von 2-[3-(tert.-Butyloxycarbonylamino)-piperidin-1-yl]-3-(3-methyl-2-buten-1-yl)-4-ethoxycarbonyl-5-[(phenylaminocarbonyl)amino]-3H-imidazol mit Kalium-tert.-butylat in Ethanol unter Rückfluß
R_{f}-Wert: 0.75 (Aluminiumoxid, Methylenchlorid/Methanol/konz. wässriges Ammoniak = 9:1:0.1)
Massenspektrum (ESI⁺): m/z = 495 [M+H]⁺

Analog Beispiel XXII werden folgende Verbindungen erhalten:
(1) 1-(2-Phenyl-ethyl)-7-(3-methyl-2-buten-1-yl)-8-[3-(tert.-butyloxycarbonylamino)-piperidin-1-yl]-xanthin
   R_{f}-Wert: 0.71 (Kieselgel, Essigester)
   Massenspektrum (ESI⁺): m/z = 523 [M+H]⁺
(2) 1-Methyl-7-(3-methyl-2-buten-1-yl)-8-[3-(tert.-butyloxycarbonylamino)-piperidin-1-yl]-xanthin
   Durchführung mit Natriumethylat in Ethanol bei Raumtemperatur Schmelzpunkz: 182-185°C
   Massenspektrum (ESI⁺): m/z = 433 [M+H]⁺
(3) 1-Amino-7-(3-methyl-2-buten-1-yl)-8-[3-(tert.-butyloxycarbonylamino)-piperidin-1-yl]-xanthin
   (Verunreinigt mit 1-Amino-7-(3-methyl-butyl)-8-[3-(tert.-butyloxycarbonylamino)-piperidin-1-yl]-xanthin)
   Durchführung mit Natriumethylat in Ethanol bei Raumtemperatur
   R_{f}-Wert: 0.26 (Kieselgel, Methylenchlorid/Methanol/konz. wässriges Ammoniak = 90:10:1)
   Massenspektrum (ESI⁺): m/z = 434 [M+H]⁺
(4) 7-(3-Methyl-2-buten-1-yl)-8-[3-(tert.-butyloxycarbonylamino)-piperidin-1-yl]-xanthin
   R_{f}-Wert: 0.24 (Kieselgel, Methylenchlorid/Methanol/konz. wässriges Ammoniak = 90:10:1)
   Massenspektrum (ESI⁺): m/z = 419 [M+H]⁺
(5) Kalium-{3-methyl-7-benzyl-8-[3-(tert.-butyloxycarbonylamino)-piperidin-1-yl]-xanthin}-2-thiolat
   Durchführung in n-Butanol bei 105°C.
   R_{f}-Wert: 0.90 (Aluminiomoxid, Methylenchlorid/Methanol =10:1)

### Beispiel XXIII

### 2-[3-(tert.-Butyloxycarbonylamino)-piperidin-1-yl]-3-(3-methyl-2-buten-1-yl)-4-ethoxycarbonyl-5-[(phenyl-aminocarbonyl)amino]-3H-imidazol

hergestellt durch Umsetzung von 2-[3-(tert.-Butyloxycarbonylamino)-piperidin-1-yl]-3-(3-methyl-2-buten-1-yl)-4-ethoxycarbonyl-5-amino-3*H*-imidazol mit Phenylisocyanat in 1,2-Dimethoxyethan unter Rückfluß
Massenspektrum (ESI⁺): m/z = 541 [M+H]⁺

Analog Beispiel XXIII werden folgende Verbindungen erhalten:
(1) 2-[3-(tert.-Butyloxycarbonylamino)-piperidin-1-yl]-3-(3-methyl-2-buten-1-yl)-4-ethoxycarbonyl-5-{[(2-phenyl-ethyl)-aminocarbonyl]amino}-3H-imidazol
   R_{f}-Wert: 0.70 (Kieselgel, Essigester)
   Massenspektrum (ESI⁺): m/z = 569 [M+H]⁺
(2) 2-[3-(tert.-Butyloxycarbonylamino)-piperidin-1-yl]-3-(3-methyl-2-buten-1-yl)-4-ethoxycarbonyl-5-[(methyl-aminocarbonyl)amino]-3*H*-imidazol
   Durchführung bei 130°C in der Roth-Bombe
   Massenspektrum (ESI⁺): m/z = 479 [M+H]⁺
(3) 2-[3-(tert.-Butyloxycarbonylamino)-piperidin-1-yl]-3-(3-methyl-2-buten-1-yl)-4-ethoxycarbonyl-5-{[(ethoxycarbonylamino)carbonyl]amino}-3H-imidazol
   R_{f}-Wert: 0.29 (Kieselgel, Methylenchlorid/Methanol/konz. wässriges Ammoniak = 90:10:1)
   Massenspektrum (ESI⁺): m/z = 537 [M+H]⁺
(4) 1-[2-(3-{[(Ethoxycarbonylamino)carbonyl]amino}-phenyl)-2-oxo-ethyl]-3-methyl-7-(3-methyl-2-buten-1-yl)-8-[3-(tert.-butyloxycarbonylamino)-piperidin-1-yl]-xanthin
   Durchführung in Gegenwart von Triethylamin in einem Gemisch aus Methylenchlorid und Dimethylformamid bei Raumtemperatur.
   R_{f}-Wert: 0.41 (Kieselgel, Cyclohexan/Essigester = 1:2)
(5) 2-[3-(tert.-Butyloxycarbonylamino)-piperidin-1-yl]-3-benzyl-4-ethoxycarbonyl-5-{N-[(ethoxycarbonylamino)thiocarbonyl]-N-methyl-amino}-3H-imidazol
   Durchführung mit Ethoxycarbonylisothiocyanat in Tetrahdrofuran unter Rückfluß.
   R_{f}-Wert: 0.35 (Kieselgel, Petrolether/Essigester = 1:1)

### Beispiel XXIV

### 2-[3-(tert.-Butyloxycarbonylamino)-piperidin-1-yl]-3-(3-methyl-2-buten-1-yl)-4-ethoxycarbonyl-5-amino-3H-imidazol

hergestellt durch Umsetzung von Cyanimino-[N-(3-methyl-2-buten-1-yl)-N-(ethoxycarbonylmethyl)-amino]-[3-(tert.-butyloxycarbonylamino)-piperidin-1-yl]-methan mit Natrium in Ethanol unter Rückfluß
R_{f}-Wert: 0.26 (Aluminiumoxid, Essigester/Petrolether = 8:2)
Massenspektrum (ESI⁺): m/z = 422 [M+H]⁺

Analog Beispiel XXIV wird folgende Verbindung erhalten:
(1) 2-[3-(tert.-Butyloxycarbonylamino)-piperidin-1-yl]-3-benzyl-4-ethoxycarbonyl-5-amino-3*H*-imidazol
   R_{f}-Wert: 0.40 (Kieselgel, Essigester/Petrolether = 4:1)

### Beispiel XXV

### Cyanimino-[N-(3-methyl-2-buten-1-yl)-N-(ethoxycarbonylmethyl)-amino]-[3-(tert.-butyloxycarbonylamino)-piperidin-1-yl]-methan

hergestellt durch Umsetzung von Cyanimino-[(ethoxycarbonylmethyl)amino]-[3-(tert.-butyloxycarbonylamino)-piperidin-1-yl]-methan mit 1-Brom-3-methyl-2-buten in Gegenwart von Kaliumcarbonat in Aceton bei Raumtemperatur
Massenspektrum (ESI⁺): m/z = 422 [M+H]⁺

Analog Beispiel XXV wird folgende Verbindung erhalten:
(1) Cyanimino-[N-benzyl-N-(ethoxycarbonylmethyl)-amino]-[3-(tert.-butyloxycarbonyl-amino)-piperidin-1-yl]-methan
   Durchführung mit Bromessigsäureethylester in Gegenwart von Kaliumcarbonat in Dimethylformamid.
   R_{f}-Wert: 0.70 (Kieselgel, Essigester/Petrolether = 4:1)

### Beispiel XXVI

### Cyanimino-[(ethoxycarbonylmethyl)amino]-[3-(tert.-butyloxycarbonylamino)-piperidin-1-yl]-methan

hergestellt durch Umsetzung von Cyanimino-[(ethoxycarbonylmethyl)amino]-phenyloxy-methan mit 3-(tert.-Butyloxycarbonylamino)-piperidin in Isopropanol bei 70°C
R_{f}-Wert: 0.45 (Aluminiumoxid, Essigester)
Massenspektrum (ESI⁺): m/z = 354 [M+H]⁺

Analog Beispiel XXVI wird folgende Verbindung erhalten:
(1) Cyanimino-benzylamino-[3-(tert.-butyloxycarbonylamino)-piperidin-1-yl]-methan
   Durchführung in Dimethylformamid bei 80°C.
   R_{f}-Wert: 0.56 (Aluminiumoxid, Methylenchlorid/Methanol = 40:1)

### Beispiel XXVII

### Cyanimino-[(ethoxycarbonylmethyl)amino]-phenyloxy-methan

hergestellt durch Umsetzung von Diphenylcyanocarbonimidat mit Aminoessigsäureethylester-hydrochlorid in Gegenwart von Triethylamin in Isopropanol bei Raumtemperatur (analog R. Besse et al., Tetrahedron 1990, 46, 7803-7812)
Massenspektrum (ESI⁺): m/z = 248 [M+H]⁺

Analog Beispiel XXVII wird folgende Verbindung erhalten:
(1) Cyanimino-benzylamino-phenyloxy-methan
   R_{f}-Wert: 0.20 (Kieselgel, Petrolether/Essigester = 3:1)
   Massenspektrum (ESI⁺): m/z = 252 [M+H]⁺

### Beispiel XXVIII

### 1-((E)-2-Phenyl-vinyl)-3-methyl-7-(3-methyl-2-buten-1-yl)-8-brom-xanthin

hergestellt durch Umsetzung von 3-Methyl-7-(3-methyl-2-buten-1-yl)-8-brom-xanthin mit (E)-2-Phenyl-vinyl-boronsäure in Gegenwart von wasserfreiem Kuper(II)acetat und Pyridin in Methylenchlorid bei Raumtemperatur.
R_{f}-Wert: 0.70 (Kieselgel, Petrolether/Essigester/Methanol = 6:3:1)
Massenspektrum (ESI⁺): m/z = 415, 417 [M+H]⁺

### Beispiel XXIX

### 1,3-Dimethyl-7-((E)-2-hexen-1-yl)-8-chlor-xanthin

hergestellt durch Umsetzung von 8-Chlor-theophyllin mit (E)-2-Hexen-1-ol in Gegenwart von Triphenylphosphin und Diisopropylazodicarboxylat in Tetrahydrofuran bei Raumtemperatur
Massenspektrum (EI): m/z = 296, 298 [M]⁺

### Beispiel XXX

### 1-(Phenylsulfinylmethyl)-3-methyl-7-(3-methyl-2-buten-1-yl)-8-[3-(tert.-butyloxycarbonylamino)-piperidin-1-yl]-xanthin

hergestellt durch Oxidation von 1-(Phenylsulfanylmethyl)-3-methyl-7-(3-methyl-2-buten-1-yl)-8-[3-(tert.-butyloxycarbonylamino)-piperidin-1-yl]-xanthin mit
Wasserstoffperoxid in Hexafluorisopropanol
R_{f}-Wert: 0.40 (Kieselgel, Petrolether/Essigester/Methanol = 6.5:2:1.5)
Massenspektrum (ESI⁺): m/z = 571 [M+H]⁺

### Beispiel XXXI

### 1,3-Dimethyl-7-(3-methyl-2-buten-1-yl)-8-(1-nitroso-piperidin-4-yl)-xanthin _

hergestellt durch Behandeln von 1,3-Dimethyl-7-(3-methyl-2-buten-1-yl)-8-(piperidin-4-yl)-xanthin mit Isoamylnitrit in Tetrahydrofuran bei 60°C.
Das Rohprodukt wird sofort weiter umgesetzt (siehe Beispiel 8).
(1) 1,3-Dimethyl-7-(3-methyl-2-buten-1-yl)-8-(1-nitroso-piperidin-3-yl)-xanthin
   Massenspektrum (ESI⁺): m/z = 361 [M+H]⁺

### Beispiel XXXII

### 1,3-Dimethyl-7-((E)-1-buten-1-yl)-8-chlor-xanthin

hergestellt durch Behandeln von 1,3-Dimethyl-7-(2-methansulfonyloxy-butyl)-8-chlor-xanthin mit 1,8-Diazabicyclo[5.4.0]undec-7-en in Dioxan unter Rückfluß.
Massenspektrum (ESI⁺): m/z = 269, 271 [M+H]⁺

### Beispiel XXXIII

### 1,3-Dimethyl-7-(2-methansulfonyloxy-butyl)-8-chlor-xanthin

hergestellt durch Umsetzung von 1,3-Dimethyl-7-(2-hydroxy-butyl)-8-chlor-xanthin mit Methansulfonsäurechlorid in Methylenchlorid in Gegenwart von Triethylamin.
Massenspektrum (ESI⁺): m/z = 365, 367 [M+H]⁺

Analog Beispiel XXXIII werden folgende Verbindungen erhalten:
(1) 1-[2-(3-Methansulfonyloxy-phenyl)-2-oxo-ethyl]-3-methyl-7-(3-methyl-2-buten-1-yl)-8-[3-(tert.-butyloxycarbonylamino)piperidin-1-yl]-xanthin
   Massenspektrum (ESI⁺): m/z = 645 [M+H]⁺
(2) 1-(2-{3-[Bis(methansulfonyl)-amino]-phenyl}-2-oxo-ethyl)-3-methyl-7-(3-methyl-2-buten-1-yl)-8-[3-(tert.-butyloxycarbonylamino)-piperidin-1-yl]-xanthin
(3) 1-[2-(3-Methansulfonylamino-phenyl)-2-oxo-ethyl]-3-methyl-7-(3-methyl-2-buten-1-yl)- 8-[3-(tert.-butyloxycarbonylamino)-piperidin-1-yl]-xanthin
   Durchführung mit Pyridin als Hilfsbase.
   Massenspektrum (ESI⁺): m/z = 644 [M+H]⁺
(4) 1-[2-(2-Methansulfonyloxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 645 [M+H]⁺
(5) 1-(2-{2-[Bis(methansulfonyl)-amino]-phenyl}-2-oxo-ethyl)-3-methyl-7-(3-methyl-2-buten-1-yl)-8-[3-(tert.-butyloxycarbonylamino)-piperidin-1-yl]-xanthin
   Durchführung in Dichlorethan mit zwei Äquivalenten Methansulfonsäurechlorid.
   Massenspektrum (ESI⁺): m/z = 722 [M+H]⁺

### Beispiel XXXIV

### 1,3-Dimethyl-7-(2-hydroxy-butyl)-8-chlor-xanthin

hergestellt durch Umsetzung von 8-Chlor-theophyllin mit 2-Ethyl-oxiran in Dimethylformamid in Gegenwart von Hünigbase bei 65°C.
Massenspektrum (ESI⁺): m/z = 287, 289 [M+H]⁺

### Beispiel XXXV

### 1-(2-Phenyl-ethyl)-3-vinyl-7-(3-methyl-2-buten-1-yl)-8-[3-(tert.-butyloxycarbonylamino)-piperidin-1-yl]-xanthin

135 mg 1-(2-Phenyl-ethyl)-7-(3-methyl-2-buten-1-yl)-8-[3-(tert.-butyloxycarbonylamino)-piperidin-1-yl]-xanthin, 84 µl Vinyltrimethoxysilan, 53 mg wasserfreises Kupfer(II)acetat und 0.53 ml einer 1 M Lösung von Tetrabutylammoniumfluorid in Tetrahydrofuran werden in 5 ml Methylenchlorid suspendiert und mit 200 mg Molekularsieb 4Å versetzt. Dann werden 43 µl Pyridin zugegeben und das türkisgrüne Reaktionsgemisch wird drei Tage bei Raumtemperatur gerührt. Anschließend wird es mit Methylenchlorid verdünnt und über Talkum abgesaugt. Das Filtrat wird im Vakuum eingeengt und das Rohrprodukt wird chromatographisch über eine Kieselgelsäule mit Cyclohexan/Essigester (8:2 auf 1:1) als Laufmittel gereinigt.
Ausbeute: 32 mg (23 % der Theorie)
R_{f}-Wert: 0.50 (Kieselgel, Cyclohexan/Essigester = 2:1)
Massenspektrum (EI): m/z = 548 [M]⁺

### Beispiel XXXVI

### 1-(2-Phenyl-ethyl)-3-((E)-2-phenyl-vinyl)-7-(3-methyl-2-buten-1-yl)-8-[3-(tert.-butyloxycarbonylamino)-piperidin-1-yl]-xanthin

hergestellt durch Umsetzung von 1-(2-Phenyl-ethyl)-7-(3-methyl-2-buten-1-yl)-8-[3-(tert.-butyloxycarbonylamino)-piperidin-1-yl]-xanthin mit (E)-2-Phenylvinyl-boronsäure in Methylenchlorid in Gegenwart von wasserfreiem Kupfer(II)acetat, Pyridin und Molekularsieb 4Å bei Raumtemperatur.
R_{f}-Wert: 0.71 (Kieselgel, Petrolether/Essigester = 6:4)
Massenspektrum (ESI⁺): m/z = 625 [M+H]⁺

Analog Beispiel XXXVI werden folgende Verbindungen erhalten:
(1) 1-Methyl-3-phenyl-7-(3-methyl-2-buten-1-yl)-8-[3-(tert.-butyloxycarbonylamino)-piperidin-1-yl]-xanthin
   R_{f}-Wert: 0.86 (Kieselgel, Methylenchlorid/Methanol/konz. wässriges Ammoniak = 95:5:1)
   Massenspektrum (ESI⁺): m/z = 509 [M+H]⁺
(2) 1-Methyl-3-((E)-2-phenyl-vinyl)-7-(3-methyl-2-buten-1-yl)-8-[3-(tert.-butyloxy-carbonylamino)-piperidin-1-yl]-xanthin
   Schmelzpunkt: 201-202.5°C
   Massenspektrum (ESI⁺): m/z = 535 [M+H]⁺

### Beispiel XXXVII

### 1-(2-Hydroxy-2-phenyl-ethyl)-3-methyl-7-(3-methyl-2-buten-1-yl)-8-[3-(tert.-butyloxycarbonylamino)-piperidin-1-yl]-xanthin

hergestellt durch Behandeln von 1-(2-Phenyl-2-oxo-ethyl)-3-methyl-7-(3-methyl-2-buten-1-yl)-8-[3-(tert.-butyloxycarbonylamino)-piperidin-1-yl]-xanthin mit Natriumborhydrid in Methanol bei Raumtemperatur.
R_{f}-Wert: 0.30 (Kieselgel, Petrolether/Essigester/Methanol = 60:35: 5)

### Beispiel XXXVIII

### 1-Phenylcarbonylamino-7-(3-methyl-2-buten-1-yl)-8-[3-(tert.-butyloxycarbonylamino)-piperidin-1-yl]-xanthin

hergestellt durch Umsetzung von 1-Amino-7-(3-methyl-2-buten-1-yl)-8-[3-(tert.-butyloxycarbonylamino)-piperidin-1-yl]-xanthin (verunreinigt mit 1-Amino-7-(3-methyl-butyl)-8-[3-(tert.-butyloxycarbonylamino)-piperidin-1-yl]-xanthin) mit Benzoylchlorid in Gegenwart von Pyridin in Methylenchlorid bei Raumtemperatur. Das erhaltene Produkt ist mit 1-Phenylcarbonylamino-7-(3-methyl-butyl)-8-[3-(tert.-butyloxycarbonylamino)-piperidin-1-yl]-xanthin verunreinigt.
R_{f}-Wert: 0.16 (Kieselgel, Methylenchlorid/Methanol/konz. wässriges Ammoniak = 90:10:1)
Massenspektrum (ESI⁺): m/z = 538 [M+H]⁺

### Beispiel XXXIX

### 2-[3-(tert.-Butyloxycarbonylamino)-piperidin-1-yl]-3-(3-methyl-2-buten-1-yl)-4-ethoxycarbonyl-5-hydrazinocarbonylamino-3H-imidazol

hergestellt durch Umsetzung von 2-[3-(tert.-Butyloxycarbonylamino)-piperidin-1-yl]-3-(3-methyl-2-buten-1-yl)-4-ethoxycarbonyl-5-ethoxycarbonylamino-3H-imidazol mit Hydrazin-hydrat in Xylol bei 150°C. Das erhaltene Produkt ist mit 2-[3-(tert.-Butyloxycarbonylamino)-piperidin-1-yl]-3-(3-methyl-butyl)-4-ethoxycarbonyl-5-hydrazinocarbonylamino-3H-imidazol verunreinigt.
R_{f}-Wert: 0.10 (Kieselgel, Methylenchlorid/Methanol/konz. wässriges Ammoniak = 90:10:1)

### Beispiel XL

### 2-[3-(tert.-Butyloxycarbonylamino)-piperidin-1-yl]-3-(3-methyl-2-buten-1-yl)-4-ethoxycarbonyl-5-ethoxycarbonylamino-3H-imidazol

hergestellt durch Umsetzung von 2-[3-(tert.-Butyloxycarbonylamino)-piperidin-1-yl]-3-(3-methyl-2-buten-1-yl)-4-ethoxycarbonyl-5-amino-3H-imidazol mit Chlorameisensäureethylester in Gegenwart von 0.5 N Natronlauge in Methylenchlorid bei 50°C.
Schmelzpunkt: 129-131 °C
Massenspektrum (ESI⁺): m/z = 494 [M+H]⁺

### Beispiel XLI

### 1-[2-(3-Allyloxy-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Umsetzung von 1-[2-(3-Hydroxy-phenyl)-2-oxo-ethyl]-3-methyl-7-(3-methyl-2-buten-1-yl)-8-[3-(tert.-butyloxycarbonylamino)-piperidin-1-yl]-xanthin mit Allylbromid in Gegenwart von Kaliumcarbonat in Dimethylformamid bei Raumtemperatur.
Massenspektrum (ESI⁺): m/z = 607 [M+H]⁺

Analog Beispiel XLI werden folgende Verbindungen erhalten:
(1) 1-{2-Oxo-2-[3-(2-propin-1-yloxy)-phenyl]-ethyl}-3-methyl-7-(3-methyl-2-buten-1-yl)-8-[3-(tert.-butyloxycarbonylamino)-piperidin-1-yl]-xanthin
   Massenspektrum (ESI⁺): m/z = 627 [M+Na]⁺
(2) 1-(2-{3-[(Methoxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ES1⁺): m/z = 639 [M+H]⁺
(3) 1-[2-(3-Cyanomethoxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 606 [M+H]⁺
(4) 1-[2-(3-Benzyloxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 657 [M+H]⁺
(5) 1-[2-(3-Phenylsulfonyloxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 707 [M+H]⁺
(6) 1-(2-{2-[(Methoxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 639 [M+H]⁺
(7) 1-[2-(2-Cyanomethoxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 606 [M+H]⁺
(8) 1-(2-{3-[(Dimethylaminocarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   R_{f}-Wert: 0.25 (Kieselgel, Cyclohexan/Essigester/Methanol = 5:4:1)
   Massenspektrum (ESI⁺): m/z = 652 [M+H]⁺
(9) 1-(2-{3-[(Methylaminocarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   R_{f}-Wert: 0.24 (Kieselgel, Cyclohexan/Essigester/Methanol = 5:4:1) Massenspektrum (ESI⁺): m/z = 638 [M+H]⁺
(10) 1-(2-{3-[(Aminocarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   R_{f}-Wert: 0.30 (Kieselgel, Cyclohexan/Essigester/Methanol = 5:4:1)
   Massenspektrum (ESI⁺): m/z = 624 [M+H]⁺

### Beispiel XLII

### 1-[2-(3-Phenyloxy-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Umsetzung von 1-[2-(3-Hydroxy-phenyl)-2-oxo-ethyl]-3-methyl-7-(3-methyl-2-buten-1-yl)-8-[3-(tert.-butyloxycarbonylamino)-piperidin-1-yl]-xanthin mit Phenylboronsäure in Methylenchlorid in Gegenwart von wasserfreiem Kupfer(II)acetat, Pyridin und Molsieb 4Å bei Raumtemperatur.
Massenspektrum (ESI⁺): m/z = 643 [M+H]⁺

### Beispiel XLIII

### 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Behandeln von 1-[2-(3-Allyloxycarbonylamino-phenyl)-2-oxo-ethyl]-3-methyl-7-(3-methyl-2-buten-1-yl)-8-(3-(tert.-butyloxycarbonylamino)-piperidin-1-yl]-xanthin mit Tetrakis(triphenylphosphin)palladium(0) und 5,5-Dimethyl-1,3-cyclohexandion in Tetrahydrofuran bei Raumtemperatur.
R_{f}-Wert: 0.22 (Kieselgel, Cyclohexan/Essigester/Methanol/konz. wässriges
Ammoniak = 60:30:10:1)

### Beispiel XLIV

### 1-(3-Allyloxycarbonylamino-phenyl)-2-brom-ethan-1-on und 1-(3-Allyloxycarbonylamino-phenyl)-2-chlor-ethan-1-on

hergestellt durch Umsetzung von 1-(3-Amino-phenyl)-2-brom-ethan-1-on-hydrobromid mit Chlorameisensäureallylester in Methylenchlorid in Gegenwart von Hünigbase. Es wird ein Gemisch aus Chlor- und Brom-Verbindung erhalten.
R_{f}-Wert: 0.50 (Kieselgel, Cyclohexan/Essigester/Methanol = 6:3:1)
Massenspektrum (ESI⁻): m/z = 252, 254 [M1-H]⁻; 296, 298 [M2-H]⁻

### Beispiel XLV

### 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Behandeln von 1-[2-(3-Nitro-phenyl)-2-oxo-ethyl]-3-methyl-7-(3-methyl-2-buten-1-yl)-8-[3-(tert.-butyloxycarbonylamino)-piperidin-1-yl]-xanthin mit Eisenpulver in einem Gemisch aus Ethanol, Wasser und Eisessig (80:25:10) bei 100°C.
R_{f}-Wert: 0.55 (Kieselgel, Cyclohexan/Essigester/Methanol/konz. wässriges Ammoniak = 50:30:20:1)
Massenspektrum (ESI⁺): m/z = 566 [M+H]⁺

Analog Beispiel XLV werden folgende Verbindungen erhalten:
(1) 1-(2-(2-Amino-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 566 [M+H]⁺
(2) 1-[(5-Amino-isochinolin-1-yl)methyl]-3-methyl-7-(3-methyl-2-buten-1-yl)-8-[3-(tert.-butyloxycarbonylamino)-piperidin-1-yl]-xanthin
   R_{f}-Wert: 0.53 (Kieselgel, Methylenchlorid/Methanol/konz. wässriges Ammoniak = 90:10:1)
   Massenspektrum (ESI⁺): m/z = 589 [M+H]⁺

### Beispiel XLVI

### 2-Brom-1-(3-dimethylamino-phenyl)-ethan-1-on und 2-Brom-1-(2-brom-5-dimethylamino-phenyl)-ethan-1-on

hergestellt durch Behandeln von 1-(3-Dimethylamino-phenyl)-ethan-1-on mit Brom in Gegenwart von Essigsäure in Essigester unter Rückfluß. Es wird ein Gemisch aus Mono- und Dibrom-Verbindung erhalten.
Massenspektrum (ESI⁺): m/z = 242, 244 [M1+H]⁺; 320, 322, 324 [M2+H]⁺

### Beispiel XLVII

### 1-[2-(3-Methoxycarbonylamino-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Umsetzung von 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin mit Chlorameisensäuremethylester in Gegenwart von Triethylamin in einem Gemisch aus Methylenchlorid und Dimethylformamid (3:1) bei Raumtemperatur.
Massenspektrum (ESI⁺): m/z = 624 [M+H]⁺

Analog Beispiel XLVII wird folgende Verbindung erhalten:
(1) 1-(2-{3-[(Dimethylaminocarbonyl)amino]-phenyl}-2-oxo-ethyl)-3-methyl-7-(3-methyl-2-buten-1-yl)-8-[3-(tert.-butyloxycarbonylamino)-piperidin-1-yl]-xanthin Umsetzung erfolgt mit Dimethylcarbamoylchlorid in Gegenwart von Kaliumcarbonat in Dimethylformamid bei 75°C.
R_{f}-Wert: 0.30 (Kieselgel, Cyclohexan/Essigester/Methanol = 6:4:1) Massenspektrum (EI): m/z = 636 [M]⁺

### Beispiel XLVIII

### 1-[2-(3-Acetylamino-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Umsetzung von 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin mit Acetylchlorid in Gegenwart von Pyridin in einem Gemisch aus Methylenchlorid und Dimethylformamid (3:1) bei Raumtemperatur.
Massenspektrum (ESI⁺): m/z = 608 [M+H]⁺

Analog Beispiel XLVIII wird folgende Verbindung erhalten:
(1) 1-[2-(2-Acetylamino-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 608 [M+H]⁺

### Beispiel XLIX

### 1-[2-(3-Cyanomethylamino-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Umsetzung von 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin mit Bromacetonitril in Gegenwart von Hünigbase in Dimethylformamid bei 70°C.
R_{f}-Wert: 0.18 (Kieselgel, Cyclohexan/Essigester = 1:2)

### Beispiel L

### 1,3-Dimethyl-7-(3-methyl-2-buten-1-yl)-8-{cis-N-[2-(tert.-butyloxycarbonylamino)-cyclohexyl]-N-methyl-amino}-xanthin

hergestellt durch Behandeln von 1,3-Dimethyl-7-(3-methyl-2-buten-1-yl)-8-[cis-2-(tert.-butyloxycarbonylamino)-cyclohexylamino]-xanthin mit Natriumhydrid in Dimethylformamid bei 0°C und anschließende Umsetzung mit Methyliodid bei 0°C bis Raumtemperatur.
R_{f}-Wert: 0.42 (Kieselgel, Cyclohexan/Essigester = 1:1)

Analog Beispiel L wird folgende Verbindung erhalten:
(1) 1,3-Dimethyl-7-(3-methyl-2-buten-1-yl)-8-{N-[2-(tert.-butyloxycarbonylamino)-2-methyl-propyl]-N-methyl-amino}-xanthin
   R_{f}-Wert: 0.62 (Kieselgel, Methylenchlorid/Methanol = 95:5)
   Massenspektrum (ESI⁺): m/z = 449 [M+H]⁺

### Beispiel LI

### 2-(tert.-Butyloxycarbonylamino)-3-(N-benzyl-N-methyl-amino)-propionsäure

hergestellt durch Umsetzung von 3-(tert.-Butyloxycarbonylamino)-oxetan-2-on mit N-Benzyl-N-methyl-amin in Acetonitril bei Raumtemperatur.
R_{f}-Wert: 0.40 (Kieselgel, Methylenchlorid/Methanol = 9:1)
Massenspektrum (ESI⁺): m/z = 309 [M+H]⁺

### Beispiel LII

### 1-(2-{3-[(Methylamino)thiocarbonylamino]-phenyl}-2-oxo-ethyl)-3-methyl-7-(3-methyl-2-buten-1-yl)-8-[3-(tert.-butyloxycarbonylamino)-piperidin-1-yl]-xanthin

hergestellt durch Umsetzung von 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin mit Methylisothiocyanat in Dimethylformamid bei 90°C.
R_{f}-Wert: 0.34 (Kieselgel, Cyclohexan/Essigester/Methanol = 7:2:1)
Massenspektrum (ESI⁺): m/z = 639 [M+H]⁺

Analog Beispiel LII wird folgende Verbindung erhalten:
(1) 1-(2-{3-[(Aminocarbonyl)amino]-phenyl}-2-oxo-ethyl)-3-methyl-7-(3-methyl-2-buten-1-yl)-8-[3-(tert.-butyloxycarbonylamino)-piperidin-1-yl]-xanthin
   Umsetzung erfolgt mit Trimethylsilylisocyanat.
Massenspektrum (ESI⁺): m/z = 609 [M+H]⁺

### Beispiel LIII

### 1-(2-{3-[(Methoxycarbonyl)methylamino]-phenyl}-2-oxo-ethyl)-3-methyl-7-(3-methyl-2-buten-1-yl)-8-[(3-(tert.-butyloxycarbonylamino)-piperidin-1-yl]-xanthin

hergestellt durch Umsetzung von 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin mit Bromessigsäuremethylester in Gegenwart von Kaliumcarbonat in Dimethylformamid bei 80°C.
R_{f}-Wert: 0.38 (Kieselgel, Cyclohexan/Essigester = 3:7)
Massenspektrum (ESI⁺): m/z = 638 [M+H]⁺

### Beispiel LIV

### 1-[2-(2-Hydroxy-phenyl)-2-oxo-ethyl]-3-methyl-7-(3-methyl-2-buten-1-yl)-8-chlor-xanthin

hergestellt durch Behandeln von 1-[2-(2-Methoxy-phenyl)-2-oxo-ethyl]-3-methyl-7-(3-methyl-2-buten-1-yl)-8-chlor-xanthin mit Bortribromid in Methylenchlorid. Das gewünschte Produkt ist mit ca. 20 % 1-[2-(2-Hydroxy-phenyl)-2-oxo-ethyl]-3-methyl-7-(3-brom-3-methyl-butyl)-8-chlor-xanthin verunreinigt.
Massenspektrum (ESI⁺): m/z = 403, 405 [M+H]⁺

### Beispiel LV

### 1-Methyl-3-[2-(4-methoxy-phenyl)-ethyl]-7-(2-cyano-benzyl)-8-chlor-xanthin

hergestellt durch Umsetzung von 1-Methyl-7-(2-cyano-benzyl)-8-chlor-xanthin mit 2-(4-Methoxy-phenyl)-ethanol in Gegenwart von Triphenylphosphin und Azodicarbonsäurediethylester in Tetrahydrofuran bei Raumtemperatur.
Massenspektrum (ESI⁺): m/z = 450 [M+H]⁺

### Beispiel LVI

### 7-(2-Cyano-benzyl)-xanthin

hergestellt durch Behandeln von 16.68 g 2-Amino-7-(2-cyano-benzyl)-1,7-dihydro-purin-6-on mit 17.00 g Natriumnitrit in einem Gemisch aus 375 ml konz. Essigsäure, 84 ml Wasser und 5.2 ml konz. Salzsäure bei 50°C.
Ausbeute: 8.46 g (50 % der Theorie)
Massenspektrum (ESI⁺): m/z = 268 [M+H]⁺

### Beispiel LVII

### 2-Amino-7-(2-cyano-benzyl)-1,7-dihydro-purin-6-on

hergestellt durch Umsetzung von 20.00 g Guanosin-hydrat mit 22.54 g 2-Cyano-benzylbromid in Dimethylsulfoxid bei 60°C und anschließende Behandlung mit 57 ml konz. Salzsäure.
Ausbeute: 18.00 g (97% der Theorie)
Massenspektrum (ESI⁺): m/z = 267 [M+H]⁺

### Beispiel LVIII

### 1-(4-Oxo-4H-chromen-3-yl)-3-methyl-7-(3-methyl-2-buten-1-yl)-8-[(3-(tert.-butyloxycarbonylamino)-piperidin-1-yl]-xanthin

hergestellt durch Umsetzung von 1-[2-(2-Hydroxy-phenyl)-2-oxo-ethyl]-3-methyl-7-(3-methyl-2-buten-1-yl)-8-[3-(tert.-butyloxycarbonylamino)-piperidin-1-yl]-xanthin mit Dimethylformamid-dimethylacetal in Gegenwart von Pyridin in Toluol unter Rückfluß.
Massenspektrum (ESI⁺): m/z = 577 [M+H]⁺

### Beispiel LIX

### endo-6-Amino-2-benzyl-2-aza-bicyclo[2.2.2]octan und exo-6-Amino-2-benzyl-2-aza-bicyclo[2.2.2]octan

hergestellt durch Umsetzung von 2-Benzyl-2-aza-bicyclo[2.2.2]octan-6-on (R. F. Borne et al., J. Het. Chem. 1973, 10, 241) mit Ammoniumacetat in Gegenwart von Eisessig und Molsieb 4Å in Methanol und anschließende Behandlung mit Natrium-cyanoborhydrid bei Raumtemperatur. Es wird ein Gemisch aus endo- und exo-Verbindung erhalten, welches nach der Umsetzung mit Pyrokohlensäure-di-tert.-butylester chromatographisch getrennt wird (siehe Bsp. IV(9)).
Massenspektrum (ESI⁺): m/z = 217 [M+H]⁺

### Beispiel LX

### 3-Amino-3-(pyrrolidin-1-ylcarbonyl)-piperidin x Trifluoressigsäure

hergestellt durch Behandeln von 1-(tert.-Butyloxycarbonyl)-3-amino-3-(pyrrolidin-1-ylcarbonyl)-piperidin mit Trifluoressigsäure in Methylenchlorid bei Raumtemperatur.

Analog Beispiel LX wird folgende Verbindung erhalten:
(1) 3-Amino-4-hydroxy-piperidin x Trifluoressigsäure
Massenspektrum (EI): m/z = 116 [M]⁺

### Beispiel LXI

### 1-(tert.-Butyloxycarbonyl)-3-amino-3-(pyrrolidin-1-ylcarbonyl)-piperidin

hergestellt durch Behandeln von 1-(tert.-Butyloxycarbonyl)-3-{[(9H-fluoren-9-ylmethoxy)carbonyl]amino}-3-(pyrrolidin-1-ylcarbonyl)-piperidin mit Diethylamin in Tetrahydrofuran bei Raumtemperatur.
Schmelzpunkt: 108.5°C

### Beispiel LXII

### 1-(tert.-Butyloxycarbonyl)-3-benzylamino-4-hydroxy-piperidin und 1-(tert.-Butyloxycarbonyl)-4-benzylamino-3-hydroxy-piperidin

hergestellt durch Umsetzung von 3.10 g 3-(tert.-Butyloxycarbonyl)-7-oxa-3-aza-bicyclo[4.1.0]heptan mit 1.7 ml Benzylamin in 30 ml Ethanol unter Rückfluß. Die entstandenen Regioisomere können chromatographisch über eine Kieselgelsäule mit Essigester/Methanol/konz. wässrigem Ammoniak (90:10:1) als Laufmittel getrennt werden:
1-(tert.-Butyloxycarbonyl)-4-benzylamino-3-hydroxy-piperidin
   Ausbeute: 0.68 g (14% der Theorie)
   R_{f}-Wert: 0.68 (Kieselgel, Essigester/Methanol/konz. wässriges Ammoniak = 90:10:1) Massenspektrum (ESI⁺): m/z = 307 [M+H]⁺
1-(tert.-Butyloxycarbonyl)-3-benzylamino-4-hydroxy-piperidin
   Ausbeute: 1.13 g (24% der Theorie)
   R_{f}-Wert: 0.56 (Kieselgel, Essigester/Methanol/konz. wässriges Ammoniak = 90:10:1) Massenspektrum (ESI⁺): m/z = 307 [M+H]⁺

### Beispiel LXIII

### 1,3-Dimethyl-2-thioxo-7-benzyl-8-[3-(tert.-butyloxycarbonylamino)-piperidin-1-yl]-xanthin

hergestellt durch Umsetzung von Kalium-{3-methyl-7-benzyl-8-[3-(tert.-butyloxy-carbonylamino)-piperidin-1-yl]-xanthin}-2-thiolat mit Dimethylsulfat in einem Gemisch aus Wasser und Dimethylformamid. Das gewünschte Produkt wird chromatographisch von ebenfalls enstandenem 2-Methylsulfanyl-3-methyl-7-benzyl-8-(3-amino-piperidin-1-yl)-xanthin abgetrennt.
Massenspektrum (EI): m/z = 484 [M]⁺

### Herstellung der Endverbindungen:

### Beispiel 1

### 1,3-Dimethyl-7-benzyl-8-(3-amino-pyrrolidin-1-yl)-xanthin

Eine Mischung aus 200 mg 1 ,3-Dimethyl-7-benzyl-8-chlor-xanthin, 420 mg 3-Amino-pyrrolidin-dihydrochlorid, 0,92 ml Triethylamin und 2 ml Dimethylformamid wird 2 Tage bei 50°C gerührt. Das Reaktionsgemisch wird mit 20 ml Wasser verdünnt und zweimal mit je 10 ml Essigester extrahiert. Die organische Phase wird mit gesättigter Kochsalzlösung gewaschen, getrocknet und eingeengt. Der Rückstand wird mit Diethylether/Diisopropylether (1:1) zur Kristallisation gebracht. Der Feststoff wird abgesaugt und getrocknet.
Ausbeute: 92 mg (40 % der Theorie)
Schmelzpunkt: 150 °C
Massenspektrum (ESI⁺): m/z = 355 [M+H]⁺
R_{f}-Wert: 0.08 (Kieselgel, Essigester/Methanol/konz. wässriges Ammoniak = 9:1:0,1) Analog Beispiel 1 werden folgende Verbindungen erhalten:
(1) 1,3-Dimethyl-7-(3-methyl-2-buten-1-yl)-8-(3-amino-pyrrolidin-1-yl)-xanthin Schmelzpunkt: 119 °C
   Massenspektrum (ESI⁺): m/z = 333 [M+H]⁺
   R_{f}-Wert: 0.07 (Kieselgel, Essigester/Methanol/konz. wässriges Ammoniak = 9:1:0,1)
(2) 1,3-Dimethyl-7-benzyl-8-(3-amino-piperidin-1-yl)-xanthin
   Massenspektrum (ESI⁺): m/z = 369 [M+H]⁺
   R_{f}-Wert: 0.06 (Kieselgel, Essigester/Methanol/konz. wässriges Ammoniak = 9:1:0,1)
(3) 1,3-Dimethyl-7-(3-methyl-2-buten-1-yl)-8-[(trans-2-amino-cyclohexyl)amino]-xanthin
   Massenspektrum (ESI⁺): m/z = 361 [M+H]⁺
(4) 1,3-Dimethyl-7-(3-methyl-2-buten-1-yl)-8-(3-amino-piperidin-1-yl)-xanthin
   Massenspektrum (ESI⁺): m/z = 347 [M+H]⁺
(5) 1,3-Dimethyl-7-(3-methyl-2-buten-1-yl)-8-(4-amino-piperidin-1-yl)-xanthin
   Massenspektrum (ESI⁺): m/z = 347 [M+H]⁺
(6) 1,3-Dimethyl-7-(3-methyl-2-buten-1-yl)-8-[(cis-2-amino-cyclohexyl)amino]-xanthin
   Massenspektrum (ESI⁺): m/z = 361 [M+H]⁺
(7) 1,3-Dimethyl-7-(2-butin-1-yl)-8-(3-amino-piperidin-1-yl)-xanthin Massenspektrum (ESI⁺): m/z = 331 [M+H]⁺
   R_{f}-Wert: 0.08 (Kieselgel, Essigester/Methanol/konz. wässriges Ammoniak = 9:1:0,1)
(8) 1,3-Dimethyl-7-[(1-cyclopenten-1-yl)methyl]-8-(3-amino-piperidin-1-yl)-xanthin
   Massenspektrum (ESI⁺): m/z = 359 [M+H]⁺
   R_{f}-Wert: 0.09 (Kieselgel, Essigester/Methanol/konz. wässriges Ammoniak = 9:1:0,1)
(9) 1,3-Dimethyl-7-(2-thienylmethyl)-8-(3-amino-piperidin-1-yl)-xanthin
   Massenspektrum (ESI⁺): m/z = 375 [M+H]⁺
   R_{f}-Wert: 0.08 (Kieselgel, Essigester/Methanol/konz. wässriges Ammoniak = 9:1:0,1)
(10) 1,3-Dimethyl-7-(3-fluorbenzyl)-8-(3-amino-piperidin-1-yl)-xanthin
   Massenspektrum (ESI⁺): m/z = 387 [M+H]⁺
   R_{f}-Wert: 0.08 (Kieselgel, Essigester/Methanol/konz. wässriges Ammoniak = 9:1:0,1)
(11) 1,3-Dimethyl-7-(2-fluorbenzyl)-8-(3-amino-piperidin-1-yl)-xanthin
   Massenspektrum (ESI⁺): m/z = 387 [M+H]⁺
   R_{f}-Wert: 0.08 (Kieselgel, Essigester/Methanol/konz. wässriges Ammoniak = 9:1:0,1)
(12) 1,3-Dimethyl-7-(4-fluorbenzyl)-8-(3-amino-piperidin-1-yl)-xanthin
   Massenspektrum (ESI⁺): m/z = 387 [M+H]⁺
(13) 1,3-Dimethyl-7-(2-buten-1-yl)-8-(3-amino-piperidin-1-yl)-xanthin
   Massenspektrum (ESI⁺): m/z = 333 [M+H]⁺
(14) 1,3-Bis-(cyclopropylmethyl)-7-benzyl-8-(3-amino-piperidin-1-yl)-xanthin
   Massenspektrum (ESI⁺): m/z = 449 [M+H]⁺
(15) 3-Methyl-7-(3-methyl-2-buten-1-yl)-8-(3-amino-piperidin-1-yl)-xanthin
   Massenspektrum (ESI⁺): m/z = 333 [M+H]⁺
(16) 1-Ethyl-3-methyl-7-(3-methyl-2-buten-1-yl)-8-(3-amino-piperidin-1-yl)-xanthin
   Massenspektrum (ESI⁺): m/z = 361 [M+H]⁺
(17) 1-Propyl-3-methyl-7-(3-methyl-2-buten-1-yl)-8-(3-amino-piperidin-1-yl)-xanthin
   Massenspektrum (ESI⁺): m/z = 375 [M+H]⁺
(18) 1-Butyl-3-methyl-7-(3-methyl-2-buten-1-yl)-8-(3-amino-piperidin-1-yl)-xanthin
   Massenspektrum (ESI⁺): m/z = 389 [M+H]⁺
(19) 1-(2-Propyl)-3-methyl-7-(3-methyl-2-buten-1-yl)-8-(3-amino-piperidin-1-yl)-xanthin
   Massenspektrum (ESI⁺): m/z = 375 [M+H]⁺
(20) 1-(2-Methylpropyl)-3-methyl-7-(3-methyl-2-buten-1-yl)-8-(3-amino-piperidin-1-yl)-xanthin
   Massenspektrum (ESI⁺): m/z = 389 [M+H]⁺
(21) 1-(2-Propen-1-yl)-3-methyl-7-(3-methyl-2-buten-1-yl)-8-(3-amino-piperidin-1-yl)-xanthin
   Massenspektrum (ESI⁺): m/z = 373 [M+H]⁺
(22) 1-(2-Propin-1-yl)-3-methyl-7-(3-methyl-2-buten-1-yl)-8-(3-amino-piperidin-1-yl)-xanthin
   Massenspektrum (ESI⁺): m/z = 371 [M+H]⁺
(23) 1-(Cyclopropylmethyl)-3-methyl-7-(3-methyl-2-buten-1-yl)-8-(3-amino-piperidin-1-yl)-xanthin
   Massenspektrum (ESI⁺): m/z = 387 [M+H]⁺
(24) 1-Benzyl-3-methyl-7-(3-methyl-2-buten-1-yl)-8-(3-amino-piperidin-1-yl)-xanthin
   Massenspektrum (ESI⁺): m/z = 423 [M+H]⁺
(25) 1-(2-Phenylethyl)-3-methyl-7-(3-methyl-2-buten-1-yl)-8-(3-amino-piperidin-1-yl)-xanthin
   Massenspektrum (ES⁺): m/z = 437 [M+H]⁺
(26) 1-(3-Phenylpropyl)-3-methyl-7-(3-methyl-2-buten-1-yl)-8-(3-amino-piperidin-1-yl)-xanthin
   Massenspektrum (ES⁺): m/z = 451 [M+H]⁺
(27) 1-(2-Hydroxyethyl)-3-methyl-7-(3-methyl-2-buten-1-yl)-8-(3-amino-piperidin-1-yl)-xanthin
   Massenspektrum (ESI⁺): m/z = 377 [M+H]⁺
(28) 1-(2-Methoxyethyl)-3-methyl-7-(3-methyl-2-buten-1-yl)-8-(3-amino-piperidin-1-yl)-xanthin
   Massenspektrum (ESI⁺): m/z = 391 [M+H]⁺
(29) 1-(3-Hydroxypropyl)-3-methyl-7-(3-methyl-2-buten-1-yl)-8-(3-amino-piperidin-1-yl)-xanthin
   Massenspektrum (ESI⁺): m/z = 391 [M+H]⁺
(30) 1-[2-(Dimethylamino)ethyl]-3-methyl-7-(3-methyl-2-buten-1-yl)-8-(3-amino-piperidin-1-yl)-xanthin
   Massenspektrum (ESI⁺): m/z = 404 [M+H]⁺
(31) 1-[3-(Dimethylamino)propyl]-3-methyl-7-(3-methyl-2-büten-1-yl)-8-(3-amino-piperidin-1-yl)-xanthin
   Massenspektrum (ESI⁺): m/z = 418 [M+H]⁺
(32) 1-Methyl-3-(cyclopropylmethyl)-7-benzyl-8-(3-amino-piperidin-1-yl)-xanthin
   Massenspektrum (ESI⁺): m/z = 409 [M+H]⁺
(33) 1,3-Diethyl-7-benzyl-8-(3-amino-piperidin-1-yl)-xanthin
   Massenspektrum (ESI⁺): m/z = 397 [M+H]⁺
(34) 1-Methyl-3-ethyl-7-benzyl-8-(3-amino-piperidin-1-yl)-xanthin
   Massenspektrum (ESI⁺): m/z = 383 [M+H]⁺
(35) 1,3-Dimethyl-7-(3-methyl-2-buten-1-yl)-8-[N-(2-aminoethyl)-methylamino]-xanthin
   Massenspektrum (ESI⁺): m/z = 321 [M+H]⁺
(36) 1-[2-(2,4,6-Trimethyl-phenyl)-ethyl]-3-methyl-7-(3-methyl-2-buten-1-yl)-8-(3-amino-piperidin-1-yl)-xanthin
   Schmelzpunkt: 153-154.5°C
   Massenspektrum (ESI⁺): m/z = 479 [M+H]⁺
(37) 1-[2-(2,4-Dichlor-phenyl)-ethyl]-3-methyl-7-(3-methyl-2-buten-1-yl)-8-(3-amino-piperidin-1-yl)-xanthin
   Schmelzpunkt: 130-132°C
   Massenspektrum (ESI⁺): m/z = 505, 507, 509 [M+H]⁺
(38) 1-[2-(Thiophen-2-yl)-ethyl]-3-methyl-7-(3-methyl-2-buten-1-yl)-8-(3-amino-piperidin-1-yl)-xanthin
   R_{f}-Wert: 0.20 (Kieselgel, Essigester/Methanol/konz. wässriges Ammoniak = 5:1:0.1)
   Massenspektrum (ESI⁺): m/z = 443 [M+H]⁺
(39) 1-[2-(Thiophen-3-yl)-ethyl]-3-methyl-7-(3-methyl-2-buten-1-yl)-8-(3-amino-piperidin-1-yl)-xanthin
   R_{f}-Wert: 0.20 (Kieselgel, Essigester/Methanol/konz. wässriges Ammoniak = 5:1:0.1)
   Massenspektrum (ESI⁺): m/z = 443 [M+H]⁺
(40) 1-[2-(4-tert.-Butyl-phenyl)-ethyl]-3-methyl-7-(3-methyl-2-buten-1-yl)-8-(3-amino-piperidin-1-yl)-xanthin
   R_{f}-Wert: 0.25 (Kieselgel, Essigester/Methanol/konz. wässriges Ammoniak = 5:1:0.1)
   Massenspektrum (ESI⁺): m/z = 493 [M+H]⁺
(41) 1-[2-(4-Fluor-phenyl)-ethyl]-3-methyl-7-(3-methyl-2-buten-1-yl)-8-(3-amino-piperidin-1-yl)-xanthin
   R_{f}-Wert: 0.20 (Kieselgel, Essigester/Methanol/konz. wässriges Ammoniak = 5:1:0.1)
   Massenspektrum (ESI⁺): m/z = 455 [M+H]⁺
(42) 1-[2-(4-Methoxy-phenyl)-ethyl]-3-methyl-7-(3-methyl-2-buten-1-yl)-8-(3-amino-piperidin-1-yl)-xanthin
   R_{f}-Wert: 0.18 (Kieselgel, Essigester/Methanol/konz. wässriges Ammoniak = 5:1:0.1)
   Massenspektrum (ESI⁺): m/z = 467 [M+H]⁺
(43) 1-Methyl-3,7-dibenzyl-8-(3-amino-piperidin-1-yl)-xanthin
   Massenspektrum (ESI⁺): m/z = 445 [M+H]⁺
(44) 1-Methyl-3-[(methoxycarbonyl)-methyl]-7-benzyl-8-(3-amino-piperidin-1-yl)-xanthin
   R_{f}-Wert: 0.27 (Kieselgel, Methylenchlorid/Methanol/konz. wässriges Ammoniak = 9:1:0.1)
   Massenspektrum (ESI⁺): m/z = 427 [M+H]⁺
(45) 1,3-Dimethyl-7-(3-methyl-2-buten-1-yl)-8-[N-(2-methylamino-ethyl)-N-methyl-amino]-xanthin
   Massenspektrum (ESI⁺): m/z = 335 [M+H]⁺
(46) 1,3-Dimethyl-7-(3-methyl-2-buten-1-yl)-8-[N-(2-dimethylamino-ethyl)-N-methyl-amino]-xanthin
   Massenspektrum (ESI⁺): m/z = 349 [M+H]⁺
(47) 1-Methyl-3-isopropyl-7-benzyl-8-(3-amino-piperidin-1-yl)-xanthin
   R_{f}-Wert: 0.32 (Kieselgel, Methylenchlorid/Methanol/konz. wässriges Ammoniak = 9:1:0.1)
   Massenspektrum (ESI⁺): m/z = 397 [M+H]⁺
(48) 1,3-Dimethyl-7-(2-pentin-1-yl)-8-(3-amino-piperidin-1-yl)-xanthin
   Massenspektrum (ESI⁺): m/z = 345 [M+H]⁺
(49) 1-Methyl-3-(2-methoxy-ethyl)-7-benzyl-8-(3-amino-piperidin-1-yl)-xanthin
   R_{f}-Wert: 0.31 (Kieselgel, Methylenchlorid/Methanol/konz. wässriges Ammoniak = 9:1:0.1)
   Massenspektrum (ESI⁺): m/z = 413 [M+H]⁺
(50) 1-Methyl-3-cyanomethyl-7-benzyl-8-(3-amino-piperidin-1-yl)-xanthin
   R_{f}-Wert: 0.24 (Kieselgel, Methylenchlorid/Methanol/konz. wässriges Ammoniak = 9:1:0.1)
   Massenspektrum (ESI⁺): m/z = 394 [M+H]⁺
(51) 1-[2-(2-Fluor-phenyl)-ethyl]-3-methyl-7-(3-methyl-2-buten-1-yl)-8-(3-amino-piperidin-1-yl)-xanthin
   R_{f}-Wert: 0.30 (Kieselgel, Methylenchlorid/Methanol/konz. wässriges Ammoniak = 10:1:0.1)
   Massenspektrum (ESI⁺): m/z = 455 [M+H]⁺
(52) 1-[2-(2-Methyl-phenyl)-ethyl]-3-methyl-7-(3-methyl-2-buten-1-yl)-8-(3-amino-piperidin-1-yl)-xanthin
   R_{f}-Wert: 0.34 (Kieselgel, Methylenchlorid/Methanol/konz. wässriges Ammoniak = 10:1:0.1)
   Massenspektrum (ESI⁺): m/z = 451 [M+H]⁺
(53) 1-Methyl-3-(2-propin-1-yl)-7-benzyl-8-(3-amino-piperidin-1-yl)-xanthin
   R_{f}-Wert: 0.23 (Kieselgel, Methylenchlorid/Methanol/konz. wässriges Ammoniak = 9:1:0.1)
   Massenspektrum (ESI⁺): m/z = 393 [M+H]⁺
(54) 1-Methyl-3-(2-propen-1-yl)-7-benzyl-8-(3-amino-piperidin-1-yl)-xanthin
   R_{f} Wert: 0.31 (Kieselgel, Methylenchlorid/Methanol/konz. wässriges Ammoniak = 9:1:0.1)
   Massenspektrum (ESI⁺): m/z = 395 [M+H]⁺
(55) 1-[2-(3-Methyl-phenyl)-ethyl]-3-methyl-7-(3-methyl-2-buten-1-yl)-8-(3-amino-piperidin-1-yl)-xanthin
   R_{f}-Wert: 0.20 (Kieselgel, Essigester/Methanol/konz. wässriges Ammoniak = 7:3:0.1)
   Massenspektrum (ESI⁺): m/z = 451 [M+H]⁺
(56) 1-[2-(1-Naphthyl)-ethyl]-3-methyl-7-(3-methyl-2-buten-1-yl)-8-(3-amino-piperidin-1-yl)-xanthin
   R_{f}-Wert: 0.30 (Kieselgel, Methylenchlorid/Methanol/konz. wässriges Ammoniak = 15:1:0.1)
   Massenspektrum (ESI⁺): m/z = 487 [M+H]⁺
(57) 1-[2-(2-Naphthyl)-ethyl]-3-methyl-7-(3-methyl-2-buten-1-yl)-8-(3-amino-piperidin-1-yl)-xanthin
   R_{f}-Wert: 0.25 (Kieselgel, Essigester/Methanol/konz. wässriges Ammoniak = 7:3:0.1)
   Massenspektrum (ESI⁺): m/z = 487 [M+H]⁺
(58) 1-(4-Phenyl-butyl)-3-methyl-7-(3-methyl-2-buten-1-yl)-8-(3-amino-piperidin-1-yl)-xanthin
   R_{f}-Wert: 0.22 (Kieselgel, Essigester/Methanol/konz. wässriges Ammoniak = 7:3:0.1)
   Massenspektrum (ESI⁺): m/z = 465 [M+H]⁺
(59) 1-[2-(3-Trifluormethyl-phenyl)-ethyl]-3-methyl-7-(3-methyl-2-buten-1-yl)-8-(3-amino-piperidin-1-yl)-xanthin
   R_{f}-Wert: 0.30 (Kieselgel, Essigester/Methanol/konz. wässriges Ammoniak = 7:3:0.1)
   Massenspektrum (ESI⁺): m/z = 505 [M+H]⁺
(60) 1-[2-(Pyridin-2-yl)-ethyl]-3-methyl-7-(3-methyl-2-buten-1-yl)-8-(3-amino-piperidin-1-yl)-xanthin
   Schmelzpunkt: 117-120°C
   Massenspektrum (ESI⁺): m/z = 438 [M+H]⁺
(61) 1-[2-(Pyrrol-1-yl)-ethyl]-3-methyl-7-(3-methyl-2-buten-1-yl)-8-(3-amino-piperidin-1-yl)-xanthin
   Schmelzpunkt: 136-138.6°C
   Massenspektrum (ESI⁺): m/z = 426 [M+H]⁺
(62) 1,3-Dimethyl-7-(3-methyl-phenyl)-8-(3-amino-piperidin-1-yl)-xanthin
   Massenspektrum (ESI⁺): m/z = 369 [M+H]⁺
(63) 1-[2-([1,2,3]Triazol-1-yl)-ethyl]-3-methyl-7-(3-methyl-2-buten-1-yl)-8-(3-amino-piperidin-1-yl)-xanthin
   R_{f}-Wert: 0.15 (Kieselgel, Essigester/Methanol/konz. wässriges Ammoniak = 7:3:0.1)
   Massenspektrum (ESI⁺): m/z = 428 [M+H]⁺
(64) 1-[2-(Pyridin-4-yl)-ethyl]-3-methyl-7-(3-methyl-2-buten-1-yl)-8-(3-amino-piperidin-1-yl)-xanthin
   R_{f}-Wert: 0.12 (Kieselgel, Essigester/Methanol/konz. wässriges Ammoniak = 7:3:0.1)
   Massenspektrum (ESI⁺): m/z = 438 [M+H]⁺
(65) 1-(3-Butin-1-yl)-3-methyl-7-(3-methyl-2-buten-1-yl)-8-(3-amino-piperidin-1-yl)-xanthin
   Schmelzpunkt: 150-152°C
   Massenspektrum (ESI⁺): m/z = 385 [M+H]⁺
(66) 1-(3-Buten-1-yl)-3-methyl-7-(3-methyl-2-buten-1-yl)-8-(3-amino-piperidin-1-yl)-xanthin
   Schmelzpunkt: 111-112.6°C
   Massenspektrum (ESI⁺): m/z = 387 [M+H]⁺
(67) 1-(4-Pentin-1-yl)-3-methyl-7-(3-methyl-2-buten-1-yl)-8-(3-amino-piperidin-1-yl)-xanthin
   R_{f}-Wert: 0.12 (Kieselgel, Essigester/Methanol/konz. wässriges Ammoniak = 8:2:0.1)
   Massenspektrum (ESI⁺): m/z = 399 [M+H]⁺
(68) 1-(2-Phenyl-ethyl)-3-methyl-7-benzyl-8-(3-amino-piperidin-1-yl)-xanthin
   Massenspektrum (ESI⁺): m/z = 459 [M+H]⁺
(69) 1-(2-Phenyl-ethyl)-3-methyl-7-cyclopropylmethyl-8-(3-amino-piperidin-1-yl)-xanthin
   Massenspektrum (ESI⁺): m/z = 423 [M+H]⁺
(70) 1-Methyl-3-(2-phenyl-ethyl)-7-benzyl-8-(3-amino-piperidin-1-yl)-xanthin
   R_{f}-Wert: 0.23 (Kieselgel, Methylenchlorid/Methanol/konz. wässriges Ammoniak = 9:1:0.1)
   Massenspektrum (ESI⁺): m/z = 459 [M+H]⁺
(71) 1-(2-Phenyl-ethyl)-3-methyl-7-(2-butin-1-yl)-8-(3-amino-piperidin-1-yl)-xanthin
   Massenspektrum (ESI⁺): m/z = 421 [M+H]⁺
(72) 1-(4-Penten-1-yl)-3-methyl-7-(3-methyl-2-buten-1-yl)-8-(3-amino-piperidin-1-yl)-xanthin
   R_{f}-Wert: 0.18 (Kieselgel, Essigester/Methanol/konz. wässriges Ammoniak = 7:3:0.1)
   Massenspektrum (ESI⁺): m/z = 401 [M+H]⁺
(73) 1,3-Dimethyl-7-benzyl-8-(homopiperazin-1-yl)-xanthin
   R_{f}-Wert: 0.33 (Kieselgel, Methylenchlorid/Methanol/konz. wässriges Ammoniak = 90:10:0.1)
   Massenspektrum (ESI⁺): m/z = 369 [M+H]⁺
(74) 1,3-Dimethyl-7-(3-methyl-2-buten-1-yl)-8-{[(piperidin-2-yl)methyl]-amino}-xanthin
   R_{f}-Wert: 0.24 (Kieselgel, Methylenchlorid/Methanol/konz. wässriges Ammoniak = 90:10:1)
   Massenspektrum (ESI⁺): m/z = 361 [M+H]⁺
(75) 1,3-Dimethyl-7-(3-methyl-2-buten-1-yl)-8-{(R)-[2-(aminomethyl)-pyrrolidin-1-yl]}-xanthin
   R_{f}-Wert: 0.27 (Kieselgel, Methylenchlorid/Methanol/konz. wässriges Ammoniak = 90:10:1)
   Massenspektrum (ESI⁺): m/z = 347 [M+H]⁺
(76) 1,3-Dimethyl-7-(3-methyl-2-buten-1-yl)-8-{(S)-[2-(aminomethyl)-pyrrolidin-1-yl]}-xanthin
   Schmelzpunkt: 112-115°C
   Massenspektrum (ESI⁺): m/z = 347 [M+H]⁺
(77) 1,3-Dimethyl-7-(3-methyl-2-buten-1-yl)-8-[cis-(2-methylamino-cyclohexyl)-amino]-xanthin
   Schmelzpunkt: 172.5-175°C
   Massenspektrum (ESI⁺): m/z = 375 [M+H]⁺
(78) 1,3-Dimethyl-7-(3-methyl-2-buten-1-yl)-8-(homopiperazin-1-yl)-xanthin
   R_{f}-Wert: 0.31 (Kieselgel, Essigester/Methanol/konz. wässriges Ammoniak = 90:10:1)
   Massenspektrum (ESI⁺): m/z = 347 [M+H]⁺
(79) 1,3-Dimethyl-7-(3-methyl-2-buten-1-yl)-8-[N-((S)-2-amino-propyl)-N-methyl-amino]-xanthin
   Durchführung mit Natriumcarbonat und Hünigbase in Dimethylsulfoxid bei 150°C in der Roth-Bombe
   R_{f}-Wert: 0.31 (Kieselgel, Methylenchlorid/Methanol/konz. wässriges Ammoniak = 90:10:1)
   Massenspektrum (ESI⁺): m/z = 335 [M+H]⁺
(80) 1,3-Dimethyl-7-(3-methyl-2-buten-1-yl)-8-(piperazin-1-yl)-xanthin
   R_{f}-Wert: 0.42 (Kieselgel, Methylenchlorid/Methanol = 9:1)
   Massenspektrum (ESI⁺): m/z = 333 [M+H]⁺
(81) 1,3-Dimethyl-7-(3-methyl-2-buten-1-yl)-8-[N-((R)-2-amino-propyl)-N-methyl-amino]-xanthin
   Durchführung mit Natriumcarbonat und Hünigbase in Dimethylsulfoxid bei 150°C in der Roth-Bombe
   Schmelzpunkt: 101-104.5°C
   Massenspektrum (ESI⁺): m/z = 335 [M+H]⁺
(82) 1-[2-(Pyridin-3-yl)-ethyl]-3-methyl-7-(3-methyl-2-buten-1-yl)-8-(3-amino-piperidin-1-yl)-xanthin
   Massenspektrum (ESI⁺): m/z = 438 [M+H]⁺
   R_{f}-Wert: 0.18 (Kieselgel, Essigester/Methanol/konz. wässriges Ammoniak = 7:3:0.1)
(83) 1-[2-(4-Methyl-thiazol-5-yl)-ethyl]-3-methyl-7-(3-methyl-2-buten-1-yl)-8-(3-amino-piperidin-1-yl)-xanthin
   Massenspektrum (ESI⁺): m/z = 458 [M+H]⁺
   R_{f}-Wert: 0.14 (Kieselgel, Essigester/Methanol/konz. wässriges Ammoniak = 7:3:0.1)
(84) 1-Methyl-3-(2-dimethylamino-ethyl)-7-benzyl-8-(3-amino-piperidin-1-yl)-xanthin
   R_{f}-Wert: 0.18 (Kieselgel, Methylenchlorid/Methanol/konz. wässriges Ammoniak = 9:1:0.1)
   Massenspektrum (ESI⁺): m/z = 426 [M+H]⁺
(85) 1-Cyanomethyl-3-methyl-7-(3-methyl-2-buten-1-yl)-8-(3-amino-piperidin-1-yl)-xanthin
   R_{f}-Wert: 0.33 (Kieselgel, Essigester/Methanol/konz. wässriges Ammoniak = 7:3:0.1)
   Massenspektrum (ESI⁺): m/z = 372 [M+H]⁺
(86) 1-[2-(3-Methoxy-phenyl)-ethyl]-3-methyl-7-(3-methyl-2-buten-1-yl)-8-(3-amino-piperidin-1-yl)-xanthin
   Schmelzpunkt: 118.5-119.5°C
   Massenspektrum (ESI⁺): m/z = 467 [M+H]⁺
(87) 1-[2-(3-Brom-phenyl)-ethyl]-3-methyl-7-(3-methyl-2-buten-1-yl)-8-(3-amino-piperidin-1-yl)-xanthin
   Schmelzpunkt: 116.5-117.5°C
   Massenspektrum (ESI⁺): m/z = 515, 517 [M+H]⁺
(88) 1-[2-(3-Chlor-phenyl)-ethyl]-3-methyl-7-(3-methyl-2-buten-1-yl)-8-(3-amino-piperidin-1-yl)-xanthin
   R_{f}-Wert: 0.21 (Kieselgel, Methylenchlorid/Methanol/konz. wässriges Ammoniak = 9:1:0.1)
   Massenspektrum (ESI⁺): m/z = 471, 473 [M+H]⁺
(89) 1,3-Dimethyl-7-((E)-1-hexen-1-yl)-8-(3-amino-piperidin-1-yl)-xanthin
   Massenspektrum (ESI⁺): m/z = 361 [M+H]⁺
(90) 1-((E)-2-Phenyl-vinyl)-3-methyl-7-(3-methyl-2-buten-1-yl)-8-(3-amino-piperidin-1-yl)-xanthin
   R_{f}-Wert: 0.11 (Kieselgel, Essigester/Methanol/konz. wässriges Ammoniak = 7:3:0.1)
   Massenspektrum (ESI⁺): m/z = 435 [M+H]⁺
(91) 1-[2-(2-Chlor-phenyl-ethyl]-3-methyl-7-(3-methyl-2-buten-1-yl)-8-(3-amino-piperidin-1-yl)-xanthin
   R_{f}-Wert: 0.25 (Kieselgel, Essigester/Methanol/konz. wässriges Ammoniak = 7:3:0.1)
   Massenspektrum (ESI⁺): m/z = 471, 473 [M+H]⁺
(92) 1,3-Dimethyl-7-((E)-2-phenyl-vinyl)-8-(3-amino-piperidin-1-yl)-xanthin
   Massenspektrum (ESI⁺): m/z = 381 [M+H]⁺
(93) 1-[2-(2-Methoxy-phenyl)-ethyl]-3-methyl-7-(3-methyl-2-buten-1-yl)-8-(3-amino-piperidin-1-yl)-xanthin
   R_{f}-Wert: 0.15 (Kieselgel, Essigester/Methanol/konz. wässriges Ammoniak = 7:3:0.1)
   Massenspektrum (ESI⁺): m/z = 467 [M+H]⁺
(94) 1-[2-(2-Trifluormethyl-phenyl)-ethyl]-3-methyl-7-(3-methyl-2-buten-1-yl)-8-(3-amino-piperidin-1-yl)-xanthin
   R_{f}-Wert: 0.16 (Kieselgel, Essigester/Methanol/konz. wässriges Ammoniak = 7:3:0.1)
   Massenspektrum (ESI⁺): m/z = 505 [M+H]⁺
(95) 1-[2-(2-Brom-phenyl)-ethyl]-3-methyl-7-(3-methyl-2-buten-1-yl)-8-(3-amino-piperidin-1-yl)-xanthin
   R_{f}-Wert: 0.15 (Kieselgel, Essigester/Methanol/konz. wässriges Ammoniak = 7:3:0.1)
   Massenspektrum (ESI⁺): m/z = 515, 517 [M+H]⁺
(96) 1-(2-Phenyl-ethyl)-3-methyl-7-(3-methyl-2-buten-1-yl)-8-(piperazin-1-y1)-xanthin
   Massenspektrum (ESI⁺): m/z = 423 [M+H]⁺
(97) 1-(2-Phenyl-ethyl)-3-methyl-7-(3-methyl-2-buten-1-yl)-8-(homopiperazin-1-yl)-xanthin
   Massenspektrum (ESI⁺): m/z = 437 [M+H]⁺
(98) 1-[2-(3-Fluor-phenyl)-ethyl]-3-methyl-7-(3-methyl-2-buten-1-yl)-8-(3-amino-piperidin-1-yl)-xanthin
   Schmelzpunkt: 126.8-127.5°C
   Massenspektrum (ESI⁺): m/z = 455 [M+H]⁺
(99) 1-[2-(3-Nitro-phenyl)-ethyl]-3-methyl-7-(3-methyl-2-buten-1-yl)-8-(3-amino-piperidin-1-yl)-xanthin
   Schmelzpunkt: 120.8-122°C
   Massenspektrum (ESI⁺): m/z = 482 [M+H]⁺
(100) 1-[2-(4-Methyl-phenyl)-ethyl]-3-methyl-7-(3-methyl-2-buten-1-yl)-8-(3-amino-piperidin-1-yl)-xanthin

   Schmelzpunkt: 129-130.2°C
   Massenspektrum (ESI⁺): m/z = 451 [M+H]⁺
(101) 1,3-Dimethyl-7-(3-methyl-2-buten-1-yl)-8-(3-aminomethyl-pyrrolidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 347 [M+H]⁺
(102) 1,3-Dimethyl-7-[(thiophen-3-yl)-methyl]-8-(piperazin-1-yl)-xanthin
   (Durchführung in Tetrahydrofuran bei 60°C)
   R_{f}-Wert: 0.14 (Kieselgel, Methylenchlorid/Methanol = 9:1)
   Massenspektrum (ESI⁺): m/z = 361 [M+H]⁺
(103) 1,3-Dimethyl-7-[(thiophen-2-yl)-methyl]-8-(piperazin-1-yl)-xanthin
   (Durchführung in Tetrahydrofuran bei 60°C)
   R_{f}-Wert: 0.19 (Kieselgel, Methylenchlorid/Methanol = 9:1)
   Massenspektrum (ESI⁺): m/z = 361 [M+H]⁺
(104) 1,3-Dimethyl-7-[(furan-3-yl)-methyl]-8-(piperazin-1-yl)-xanthin
   (Durchführung in Tetrahydrofuran bei 60°C)
   R_{f}-Wert: 0.13 (Kieselgel, Methylenchlorid/Methanol = 9:1) Massenspektrum (ESI⁺): m/z = 345 [M+H]⁺
(105) 1,3-Dimethyl-7-[(furan-2-yl)-methyl]-8-(piperazin-1-yl)-xanthin
   (Durchführung in Tetrahydrofuran bei 60°C)
   R_{f}-Wert: 0.13 (Kieselgel, Methylenchlorid/Methanol = 9:1)
   Massenspektrum (ESI⁺): m/z = 345 [M+H]⁺
(106) 1,3-Dimethyl-7-(2-propin-1-yl)-8-(piperazin-1-yl)-xanthin
   (Durchführung in Tetrahydrofuran bei 60°C)
   R_{f}-Wert: 0.16 (Kieselgel, Methylenchlorid/Methanol = 9:1)
   Massenspektrum (ESI⁺): m/z = 303 [M+H]⁺
(107) 1,3-Dimethyl-7-(2,3-dimethyl-2-buten-1-yl)-8-(piperazin-1-yl)-xanthin
   (Durchführung in Tetrahydrofuran bei 60°C)
   R_{f}-Wert: 0.24 (Kieselgel, Methylenchlorid/Methanol = 9:1)
   Massenspektrum (ESI⁺): m/z = 347 [M+H]⁺
(108) 1,3-Dimethyl-7-((E)-2-methyl-2-buten-1-yl)-8-(piperazin-1-yl)-xanthin
   (Durchführung in Tetrahydrofuran bei 60°C)
   R_{f}-Wert: 0.27 (Kieselgel, Methylenchlorid/Methanol = 9:1)
   Massenspektrum (ESI⁺): m/z = 333 [M+H]⁺
(109) 1,3-Dimethyl-7-[(1-cyclohexen-1-yl)-methyl]-8-(piperazin-1-yl)-xanthin
   (Durchführung in Tetrahydrofuran bei 60°C)
   R_{f}-Wert: 0.17 (Kieselgel, Methylenchlorid/Methanol = 9:1)
   Massenspektrum (ESI⁺): m/z = 359 [M+H]⁺
(110) 1,3-Dimethyl-7-[(1-cyclopenten-1-yl)-methyl]-8-(piperazin-1-yl)-xanthin
   (Durchführung in Tetrahydrofuran bei 60°C)
   R_{f}-Wert: 0.19 (Kieselgel, Methylenchlorid/Methanol = 9:1)
   Massenspektrum (ESI⁺): m/z = 345 [M+H]⁺
(111) 1,3-Dimethyl-7-((Z)-2-methyl-2-buten-1-yl)-8-(piperazin-1-yl)-xanthin
   (Durchführung in Tetrahydrofuran bei 60°C)
   R_{f}-Wert: 0.23 (Kieselgel, Methylenchlorid/Methanol = 9:1)
   Massenspektrum (ESI⁺): m/z = 333 [M+H]⁺
(112) 1,3-Dimethyl-7-((E)-2-hexen-1-yl)-8-(3-amino-piperidin-1-yl)-xanthin
   Massenspektrum (ESI⁺): m/z = 361 [M+H]⁺
(113) 1,3-Dimethyl-7-(3-methyl-2-buten-1-yl)-8-((S)-2-aminomethyl-azetidin-1-yl)-xanthin
   R_{f}-Wert: 0.52 (Kieselgel, Methylenchlorid/Methanol/konz. wässriges Ammoniak = 90:10:1)
   Massenspektrum (ESI⁺): m/z = 333 [M+H]⁺
(114) 1,3-Dimethyl-7-((E)-1-buten-1-yl)-8-(3-amino-piperidin-1-yl)-xanthin
   Massenspektrum (ESI⁺): m/z = 333 [M+H]⁺
(115) 1,3,7-Trimethyl-8-(3-amino-piperidin-1-yl)-xanthin
   Durchführung mit Kaliumcarbonat in Dimethylformamid
   Schmelzpunkt: 147°C
   Massenspektrum (ESI⁺): m/z = 293 [M+H]⁺
(116) 1,3-Dimethyl-7-(2-naphthyl)-8-(3-amino-piperidin-1-yl)-xanthin
   Durchführung mit Kaliumcarbonat in Dimethylformamid
   Massenspektrum (ESI⁺): m/z = 405 [M+H]⁺
(117) 1,3-Dimethyl-7-phenyl-8-(3-amino-piperidin-1-yl)-xanthin
   Durchführung mit Kaliumcarbonat in Dimethylformamid
   Massenspektrum (ESI⁺): m/z = 355 [M+H]⁺
(118) 1,3-Dimethyl-7-(3,5-dimethyl-phenyl)-8-(3-amino-piperidin-1-yl)-xanthin
   Durchführung mit Kaliumcarbonat in Dimethylformamid
   Massenspektrum (ESI⁺): m/z = 383 [M+H]⁺
(119) 1,3-Dimethyl-7-[(2-naphthyl)methyl]-8-(3-amino-piperidin-1-yl)-xanthin
   Durchführung mit Kaliumcarbonat in Dimethylformamid
   Massenspektrum (ESI⁺): m/z = 419 [M+H]⁺
(120) 1,3-Dimethyl-7-[(1-naphthyl)methyl]-8-(3-amino-piperidin-1-yl)-xanthin
   Durchführung mit Kaliumcarbonat in Dimethylformamid
   Massenspektrum (ESI⁺): m/z = 419 [M+H]⁺
(121) 1,3-Dimethyl-7-(2-cyano-benzyl)-8-(3-amino-piperidin-1-yl)-xanthin
   Durchführung mit Kaliumcarbonat in Dimethylformamid
   Massenspektrum (ESI⁺): m/z = 394 [M+H]⁺
(122) 1,3-Dimethyl-7-(4-methyl-phenyl)-8-(3-amino-piperidin-1-yl)-xanthin
   Durchführung mit Kaliumcarbonat in Dimethylformamid
   Massenspektrum (ESI⁺): m/z = 369 [M+H]⁺
(123) 1,3-Dimethyl-7-(3-cyano-benzyl)-8-(3-amino-piperidin-1-yl)-xanthin
   Durchführung mit Kaliumcarbonat in Dimethylformamid
   Massenspektrum (ESI⁺): m/z = 394 [M+H]⁺
(124) 1,3-Dimethyl-7-(3,5-difluor-benzyl)-8-(3-amino-piperidin-1-yl)-xanthin
   Durchführung mit Kaliumcarbonat in Dimethylformamid
   Massenspektrum (ESI⁺): m/z = 405 [M+H]⁺
(125) 1,3-Dimethyl-7-(4-cyano-benzyl)-8-(3-amino-piperidin-1-yl)-xanthin
   Durchführung mit Kaliumcarbonat in Dimethylformamid
   Massenspektrum (ESI⁺): m/z = 394 [M+H]⁺
(126) 1,3-Dimethyl-7-(3-nitro-benzyl)-8-(3-amino-piperidin-1-yl)-xanthin
   Durchführung mit Kaliumcarbonat in Dimethylformamid
   Massenspektrum (ESI⁺): m/z = 414 [M+H]⁺
(127) 1,3-Dimethyl-7-(4-nitro-benzyl)-8-(3-amino-piperidin-1-yl)-xanthin
   Durchführung mit Kaliumcarbonat in Dimethylformamid
   Massenspektrum (ESI⁺): m/z = 414 [M+H]⁺
(128) 1,3-Dimethyl-7-(2-nitro-benzyl)-8-(3-amino-piperidin-1-yl)-xanthin
   Durchführung mit Kaliumcarbonat in Dimethylformamid
   Massenspektrum (ESI⁺): m/z = 414 [M+H]⁺
(129) 1,3-Dimethyl-7-(3-trifluormethyl-phenyl)-8-(3-amino-piperidin-1-yl)-xanthin
   Durchführung mit Kaliumcarbonat in Dimethylformamid
   Massenspektrum (ESI⁺): m/z = 423 [M+H]⁺
(130) 1,3-Dimethyl-7-(3-cyano-phenyl)-8-(3-amino-piperidin-1-yl)-xanthin
   Durchführung mit-Kaliumcarbonat in Dimethylformamid
   Massenspektrum (ESI⁺): m/z = 380 [M+H]⁺
(131) 1-(2-Phenyl-propyl)-3-methyl-7-(3-methyl-2-buten-1-yl)-8-(3-amino-piperidin-1-yl)-xanthin
   Durchführung mit Kaliumcarbonat in Dimethylsulfoxid
   R_{f}-Wert: 0.50 (Kieselgel, Methylenchlorid/Methanol/konz. wässriges Ammoniak = 80:20:1)
   Massenspektrum (ESI⁺): m/z = 451 [M+H]⁺
(132) 1,3-Dimethyl-7-(3-fluor-phenyl)-8-(3-amino-piperidin-1-yl)-xanthin
   Durchführung mit Kaliumcarbonat in Dimethylformamid
   R_{f}-Wert: 0.10 (Kieselgel, Methylenchlorid/Methanol = 9:1)
   Massenspektrum (ESI⁺): m/z = 373 [M+H]⁺
(133) 1-(2-Methoxy-2-phenyl-ethyl)-3-methyl-7-(3-methyl-2-buten-1-yl)-8-(3-amino-piperidin-1-yl)-xanthin
   Durchführung mit Kaliumcarbonat in Dimethylsulfoxid
   R_{f}-Wert: 0.20 (Kieselgel, Essigester/Methanol = 8:2)
   Massenspektrum (ESI⁺): m/z = 467 [M+H]⁺
(134) 1,3-Dimethyl-7-(3-methyl-2-buten-1-yl)-8-(2-amino-2-methyl-propylamino)-xanthin
   Durchführung mit Natriumcarbonat in Dimethylsulfoxid
   Schmelzpunkt: 140.5-143°C
   Massenspektrum (ESI⁺): m/z = 335 [M+H]⁺
(135) 1,3-Dimethyl-7-(3-methyl-2-buten-1-yl)-8-((*R*)-2-amino-propylamino)-xanthin
   Durchführung mit Natriumcarbonat in Dimethylsulfoxid
   Schmelzpunkt: 141-144°C
   Massenspektrum (ESI⁺): m/z = 321 [M+H]⁺
(136) 1,3-Dimethyl-7-(3-methyl-2-buten-1-yl)-8-((*S*)-2-amino-propylamino)-xanthin
   Durchführung mit Kalium-tert.-butylat und Natriumcarbonat in Dimethylsulfoxid Schmelzpunkt: 142-145°C
   Massenspektrum (ESI⁺): m/z = 321 [M+H]⁺
(137) 1,3-Dimethyl-7-(2-cyano-benzyl)-8-(homopiperazin-1-yl)-xanthin
   Massenspektrum (ESI⁺): m/z = 394 [M+H]⁺
   R_{f}-Wert: 0.10 (Kieselgel, Methylenchlorid/Methanol = 9:1)
(138) 1,3-Dimethyl-7-(2-iod-benzyl)-8-(3-amino-piperidin-1-yl)-xanthin
   Massenspektrum (ESI⁺): m/z = 495 [M+H]⁺
(139) 1,3-Dimethyl-7-(3-methyl-2-buten-1-yl)-8-[3-amino-3-(pyrrolidin-1-ylcarbonyl)-piperidin-1-yl]-xanthin
   Durchführung in Gegenwart von Natriumcarbonat in Dimethylsulfoxid.
   Schmelzpunkt: 159-160°C
   Massenspektrum (ESI⁺): m/z = 444 [M+H]⁺
(140) 1,3-Dimethyl-7-(3-methyl-2-buten-1-yl)-8-(3-amino-4-hydroxy-piperidin-1-yl)-xanthin
   Durchführung in Gegenwart von Natriumcarbonat in Dimethylsulfoxid.
   R_{f}-Wert: 0.64 (Reversed Phase DC-Fertigplatte (E. Merck), Acetonitril/Wasser/ Trifluoressigsäure = 50:50:1)
   Massenspektrum (ESI⁺): m/z = 363 [M+H]⁺

### Beispiel 2

### (R)-1,3-Dimethl-7-(3-methyl-2-buten-1-yl)-8-(3-amino-piperidin-1-yl)-xanthin

980 mg (*R*)-1,3-Dimethyl-7-(3-methyl-2-buten-1-yl)-8-[3-(tert.-butyloxycarbonyl-amino)-piperidin-1-yl]-xanthin in 12 ml Methylenchlorid werden mit 3 ml Trifluoressigsäure versetzt und 2 Stunden bei Raumtemperatur gerührt. Danach wird mit Methylenchlorid verdünnt und mit 1 M Natronlauge alkalisch gestellt. Die organische Phase wird abgetrennt, getrocknet und zur Trockene eingeengt.
Ausbeute: 680 mg (89 % der Theorie)
Massenspektrum (ESI⁺): m/z = 347 [M+H]⁺
R_{f}-Wert: 0.20 (Aluminiumoxid, Essigester/Methanol = 9:1)

Analog Beispiel 2 werden folgende Verbindungen erhalten:
(1) (*S*)-1,3-Dimethyl-7-(3-methyl-2-buten-1-yl)-8-(3-amino-piperidin-1-yl)-xanthin
   Massenspektrum (ESI⁺): m/z = 347 [M+H]⁺
(2) 1,3-Dimethyl-7-(3-methyl-2-buten-1-yl)-8-(3-amino-hexahydroazepin-1-yl)-xanthin
   Massenspektrum (ESI⁺): m/z = 361 [M+H]⁺
(3) 1,3-Dimethyl-7-(3-methyl-2-buten-1-yl)-8-(4-amino-hexahydroazepin-1-yl)-xanthin
   Massenspektrum (ESI⁺): m/z = 361 [M+H]⁺
(4) 1,3-Dimethyl-7-(3-methyl-2-buten-1-yl)-8-(cis-3-amino-cyclohexyl)-xanthin-hydrochlorid
   Die Reaktion wurde mit Salzsäure durchgeführt.
   ¹H-NMR (400 MHz, 6 mg in 0.5 ml DMSO-d₆, 30°C): charakteristische Signale bei 3.03 ppm (1 H, m, H-1) und 3.15 ppm (1 H, m, H-3)
(5) 1,3-Dimethyl-7-(3-methyl-2-buten-1-yl)-8-(3-aminopropyl)-xanthin
   Die Reaktion wurde mit Salzsäure durchgeführt.
   Massenspektrum (ESI⁺): m/z = 306 [M+H]⁺
(6) 1,3-Dimethyl-7-(3-methyl-2-buten-1-yl)-8-(3-amino-4-methyl-piperidin-1-yl)-xanthin
   Massenspektrum (ESI⁺): m/z = 361 [M+H]⁺
(7) 1-Methyl-3-(4-methoxy-benzyl)-7-benzyl-8-((*S*)-3-amino-piperidin-1-yl)-xanthin
   Massenspektrum (ESI⁺): m/z = 475 [M+H]⁺
   R_{f}-Wert: 0.38 (Kieselgel, Methylenchlorid/Methanol/konz. wässriges Ammoniak = 9:1:0.1)
(8) 1,3-Dimethyl-7-(3-methyl-2-buten-1-yl)-8-[N-(2-aminoethyl)-N-ethyl-amino]-xanthin
   Massenspektrum (ESI⁺): m/z = 335 [M+H]⁺
(9) 1,3-Dimethyl-7-(3-methyl-2-buten-1-yl)-8-(piperidin-4-yl)-xanthin
   Massenspektrum (ESI⁺): m/z = 332 [M+H]⁺
(10) 1,3-Dimethyl-7-(3-methyl-2-buten-1-yl)-8-(trans-2-amino-cydohexyl)-xanthin
   Massenspektrum (ESI⁺): m/z = 346 [M+H]⁺
(11) 1-Methyl-3-hexyl-7-benzyl-8-((*S*)-3-amino-piperidin-1-yl)-xanthin
   R_{f}-Wert: 0.18 (Kieselgel, Methylenchlorid/Methanol/konz. wässriges Ammoniak = 9:1:0.1)
   Massenspektrum (ESI⁺): m/z = 439 [M+H]⁺
(12) 1-Methyl-3-(2-hydroxy-ethyl)-7-benzyl-8-((*S*)-3-amino-piperidin-1-yl)-xanthin
   R_{f}-Wert: 0.19 (Kieselgel, Methylenchlorid/Methanol/konz. wässriges Ammoniak = 9:1:0.1)
   Massenspektrum (ESI⁺): m/z = 399 [M+H]⁺
(13) 1-(2-Phenyl-ethyl)-3-methyl-7-(3-methyl-2-buten-1-yl)-8-((*S*)-3-amino-piperidin-1-yl)-xanthin
   Massenspektrum (ESI⁺): m/z = 437 [M+H]⁺
(14) 1-(2-Phenyl-ethyl)-3-methyl-7-(3-methyl-2-buten-1-yl)-8-((*R*)-3-amino-piperidin-1-yl)-xanthin
   Massenspektrum (ESI⁺): m/z = 437 [M+H]⁺
(15) 1,3-Dimethyl-7-(3-methyl-2-buten-1-yl)-8-[2-(aminomethyl)-piperidin-1-yl)]-xanthin
   R_{f}-Wert: 0.34 (Kieselgel, Methylenchlorid/Methanol/konz. wässriges Ammoniak = 90:10:1)
   Massenspektrum (ESI⁺): m/z = 361 [M+H]⁺
(16) 1,3-Dimethyl-7-(3-methyl-2-buten-1-yl)-8-[(pyrrolidin-3-yl)amino]-xanthin
   Durchführung mit Salzsäure in Dioxan
   R_{f}-Wert: 0.15 (Kieselgel, Methylenchlorid/Methanol/konz. wässriges Ammoniak = 90:10:1)
   Massenspektrum (ESI⁺): m/z = 333 [M+H]⁺
(17) 1,3-Dimethyl-7-(3-methyl-2-buten-1-yl)-8-[N-(piperidin-3-yl)-N-methyl-amino]-xanthin
   R_{f}-Wert: 0.44 (Kieselgel, Methylenchlorid/Methanol/konz. wässriges Ammoniak = 90:10:1)
   Massenspektrum (ESI⁺): m/z = 361 [M+H]⁺
(18) 1-[2-(4-Hydroxy-phenyl)-ethyl]-3-methyl-7-(3-methyl-2-buten-1-yl)-8-((*S*)-3-amino-piperidin-1-yl)-xanthin
   Durchführung in Tetrahydrofuran/Wasser bei 50-80°C
   R_{f}-Wert: 0.58 (Reversed Phase DC-Fertigplatte (E. Merck), Acetonitril/Wasser/ Trifluoressigsäure = 50:50:1)
   Massenspektrum (ESI⁺): m/z = 453 [M+H]⁺
(19) 1-[(Methoxycarbonyl)-methyl]-3-methyl-7-(3-methyl-2-buten-1-yl)-8-((*S*)-3-amino-piperidin-1-yl)-xanthin
   Schmelzpunkt: 102-105°C
   Massenspektrum (ESI⁺): m/z = 405 [M+H]⁺
(20) 1-(3-(Methoxycarbonyl)-propyl]-3-methyl-7-(3-methyl-2-buten-1-yl)-8-((*S*)-3-amino-piperidin-1-yl)-xanthin
   R_{f}-Wert: 0.15 (Kieselgel, Methylenchlorid/Methanol = 9:1)
   Massenspektrum (ESI⁺): m/z = 433 [M+H]⁺
(21) 1-{2-[4-(Ethoxycarbonyl)-phenyl]-ethyl}-3-methyl-7-(3-methyl-2-buten-1-yl)-8-((*S*)-3-amino-piperidin-1-yl)-xanthin
   Schmelzpunkt: 142-144°C
   Massenspektrum (ESI⁺): m/z = 509 [M+H]⁺
(22) 1-[2-(3-Hydroxy-phenyl)-ethyl]-3-methyl-7-(3-methyl-2-buten-1-yl)-8-((*S*)-3-amino-piperidin-1-yl)-xanthin
   Durchführung in Tetrahydrofuran/Wasser bei 80°C
   Schmelzpunkt: 168-170°C
   Massenspektrum (ESI⁺): m/z = 453 [M+H]⁺
(23) 1-[2-(Methoxycarbonyl)-ethyl]-3-methyl-7-(3-methyl-2-buten-1-yl)-8-((*S*)-3-amino-piperidin-1-yl)-xanthin
   R_{f}-Wert: 0.26 (Kieselgel, Methylenchlorid/Methanol = 9:1)
   Massenspektrum (ESI⁺): m/z = 419 [M+H]⁺
(24) 1,3-Dimethyl-7-(3-methyl-2-buten-1-yl)-8-[(piperidin-4-yl)amino]-xanthin
   Massenspektrum (ESI⁺): m/z = 347 [M+H]⁺
   R_{f}-Wert: 0.25 (Kieselgel, Methylenchlorid/Methanol/konz. wässriges Ammoniak = 9:1:0.1)
(25) 1,3-Dimethyl-7-(3-methyl-2-buten-1-yl)-8-[(piperidin-3-yl)amino]-xanthin
   Massenspektrum (ESI⁺): m/z = 347 [M+H]⁺
   R_{f}-Wert: 0.13 (Kieselgel, Methylenchlorid/Methanol = 9:1)
(26) 1-Phenyl-7-(3-methyl-2-buten-1-yl)-8-(3-amino-piperidin-1-yl)-xanthin
   Massenspektrum (ESI⁺): m/z = 395 [M+H]⁺
(27) 1-Phenyl-3-methyl-7-(3-methyl-2-buten-1-yl)-8-(3-amino-piperidin-1-yl)-xanthin
   R_{f}-Wert: 0.70 (Aluminiumoxid, Methylenchlorid/Methanol = 19:1)
   Massenspektrum (ESI⁺): m/z = 409 [M+H]⁺
(28) 1-(2-Phenyl-2-oxo-ethyl)-3-methyl-7-(3-methyl-2-buten-1-yl)-8-(3-amino-piperidin-1-yl)-xanthin
   R_{f}-Wert: 0.16 (Kieselgel, Essigester/Methanol/konz. wässriges Ammoniak = 7:3:0.1)
   Massenspektrum (ESI⁺): m/z = 451 [M+H]⁺
(29) 1,3-Dimethyl-7-(3-methyl-2-buten-1-yl)-8-[N-(pyrrolidin-3-yl)-N-methyl-amino]-xanthin
   R_{f}-Wert: 0.43 (Kieselgel, Methylenchlorid/Methanol/konz. wässriges Ammoniak = 90:10:1)
   Massenspektrum (ESI⁺): m/z = 347 [M+H]⁺
(30) 1,3-Dimethyl-7-(3-methyl-2-buten-1-yl)-8-(3-amino-cyclohexyl)-xanthin (Laut NMR-Spektrum cis/trans-Gemisch = 65:35)
   Massenspektrum (ESI⁺): m/z = 346 [M+H]⁺
(31) 1,3-Bis(2-phenyl-ethyl)-7-(3-methyl-2-buten-1-yl)-8-(3-amino-piperidin-1-yl)-xanthin
   R_{f}-Wert: 0.33 (Kieselgel, Methylenchlorid/Methanol/konz. wässriges Ammoniak = 90:10:1)
   Massenspektrum (ESI⁺): m/z = 527 [M+H]⁺
(32) 1-(2-Phenyl-ethyl)-7-(3-methyl-2-buten-1-yl)-8-(3-amino-piperidin-1-yl)-xanthin
   Massenspektrum (ESI⁺): m/z = 423 [M+H]⁺
(33) 1-(2-Phenyl-ethyl)-3-cyanomethyl-7-(3-methyl-2-buten-1-yl)-8-(3-amino-piperidin-1-yl)-xanthin
   R_{f}-Wert: 0.31 (Kieselgel, Methylenchlorid/Methanol/konz. wässriges Ammoniak = 90:10:1)
   Massenspektrum (ESI⁺): m/z = 462 [M+H]⁺
(34) 1-(2-Phenyl-ethyl)-3-[(methoxycarbonyl)-methyl]-7-(3-methyl-2-buten-1-yl)-8-(3-amino-piperidin-1-yl)-xanthin
   Massenspektrum (ESI⁺): m/z = 495 [M+H]⁺
(35) 1-[2-(2-Nitro-phenyl)-ethyl]-3-methyl-7-(3-methyl-2-buten-1-yl)-8-(3-amino-piperidin-1-yl)-xanthin
   R_{f}-Wert: 0.25 (Kieselgel, Methylenchlorid/Methanol/konz. wässriges Ammoniak = 90:10:1)
   Massenspektrum (ESI⁺): m/z = 482 [M+H]⁺
(36) 1-[2-(3,5-Difluor-phenyl)-ethyl]-3-methyl-7-(3-methyl-2-buten-1-yl)-8-(3-amino-piperidin-1-yl)-xanthin
   Schmelzpunkt: 162-163.5°C
   Massenspektrum (ESI⁺): m/z = 473 [M+H]⁺
(37) 1-[2-(2-Methoxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 481 [M+H]⁺
(38) 1-[2-(Thiophen-3-yl)-2-oxo-ethyl]-3-methyl-7-(3-methyl-2-buten-1-yl)-8-(3-amino-piperidin-1-yl)-xanthin
   Massenspektrum (ESI⁺): m/z = 457 [M+H]⁺
(39) 1-[2-(2,6-Difluor-phenyl)-ethyl]-3-methyl-7-(3-methyl-2-buten-1-yl)-8-(3-amino-piperidin-1-yl)-xanthin
   R_{f}-Wert: 0.35 (Kieselgel, Methylenchlorid/Methanol/konz. wässriges Ammoniak = 90:10:1).
   Massenspektrum (ESI⁺): m/z = 473 [M+H]⁺
(40) 1-[2-(4-Methoxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 481 [M+H]⁺
(41) 1-(2-Phenyl-2-oxo-ethyl)-3-methyl-7-(3-methyl-2-buten-1-yl)-8-((*S*)-3-amino-piperidin-1-yl)-xanthin
   Massenspektrum (ESI⁺): m/z = 451 [M+H]⁺
(42) 1-(2-Phenyl-2-oxo-ethyl)-3-methyl-7-(3-methyl-2-buten-1-yl)-8-((*R*)-3-amino-piperidin-1-yl)-xanthin
   Massenspektrum (ESI⁺): m/z = 451 [M+H]⁺
(43) 1-[2-(3,5-Dimethyl-phenyl)-ethyl]-3-methyl-7-(3-methyl-2-buten-1-yl)-8-(3-amino-piperidin-1-yl)-xanthin
   R_{f}-Wert: 0.15 (Kieselgel, Methylenchlorid/Methanol/konz. wässriges Ammoniak = 90:10:1)
   Massenspektrum (ESI⁺): m/z = 465 [M+H]⁺
(44) 1-(Phenylsulfanylmethyl)-3-methyl-7-(3-methyl-2-buten-1-yl)-8-(3-amino-piperidin-1-yl)-xanthin
   R_{f}-Wert: 0.40 (Kieselgel, Methylenchlorid/Methanol/konz. wässriges Ammoniak = 90:10:1)
   Massenspektrum (ESI⁺): m/z = 455 [M+H]⁺
(45) 1-(Phenylsulfinylmethyl)-3-methyl-7-(3-methyl-2-buten-1-yl)-8-(3-amino-piperidin-1-yl)-xanthin
   R_{f}-Wert: 0.42 (Kieselgel, Methylenchlorid/Methanol/konz. wässriges Ammoniak = 90:10:1)
   Massenspektrum (ESI⁺): m/z = 471 [M+H]⁺
(46) 1,3-Dimethyl-7-(3-methyl-2-buten-1-yl)-8-(cis-2-amino-cyclopropylamino)-xanthin
   Massenspektrum (ESI⁺): m/z = 319 [M+H]⁺
   R_{f}-Wert: 0.55 (Kieselgel, Methylenchlorid/Methanol/konz. wässriges Ammoniak = 90:10:0.1)
(47) 1-[2-(3-Methoxy-phenyl)-2-oxo-ethyl]-3-methyl-7-(3-methyl-2-buten-1-yl)-8-(3-amino-piperidin-1-yl)-xanthin
   R_{f}-Wert: 0.14 (Kieselgel, Methylenchlorid/Methanol/konz. wässriges Ammoniak = 90:10:1)
   Massenspektrum (ESI⁺): m/z = 481 [M+H]⁺
(48) 1-[2-(4-Methyl-phenyl)-2-oxo-ethyl]-3-methyl-7-(3-methyl-2-buten-1-yl)-8-(3-amino-piperidirl-1-yl)-xanthin
   R_{f}-Wert: 0.35 (Kieselgel, Methylenchlorid/Methanol/konz. wässriges Ammoniak = 90:10:1)
   Massenspektrum (ESI+): m/z = 465 [M+H]+
(49) 1-(2-Methoxycarbonyl-2-propen-1-yl)-3-methyl-7-(3-methyl-2-buten-1-yl)-8-(3-amino-piperidin-1-yl)-xanthin
   R_{f}-Wert: 0.30 (Kieselgel, Methylenchlorid/Methanol/konz. wässriges Ammoniak = 90:10:1)
   Massenspektrum (ESI⁺): m/z = 431 [M+H]⁺
(50) 1-(2-Phenyl-ethyl)-3-(2-dimethylamino-ethyl)-7-(3-methyl-2-buten-1-yl)-8-(3-amino-piperidin-1-yl)-xanthin
   R_{f}-Wert: 0.15 (Kieselgel, Methylenchlorid/Methanol/konz. wässriges Ammoniak = 90:10:1)
   Massenspektrum (ESI⁺): m/z = 494 [M+H]⁺
(51) 1-(2-Phenyl-ethyl)-3-(2-propin-1-yl)-7-(3-methyl-2-buten-1-yl)-8-(3-amino-piperidin-1-yl)-xanthin
   R_{f}-Wert: 0.71 (Kieselgel, Methylenchlorid/Methanol/konz. wässriges Ammoniak = 80:20:1)
   Massenspektrum (ESI⁺): m/z = 461 [M+H]⁺
(52) 1-(2-Phenyl-ethyl)-3-((E)-2-phenyl-vinyl)-7-(3-methyl-2-buten-1-yl)-8-(3-amino-piperidin-1-yl)-xanthin
   R_{f}-Wert: 0.27 (Kieselgel, Methylenchlorid/Methanol/konz. wässriges Ammoniak = 90:10:1)
   Massenspektrum (ESI⁺): m/z = 525 [M+H]⁺
(53) 1,3-Dimethyl-7-(3-methyl-2-buten-1-yl)-8-(piperidin-3-yl)-xanthin
   Massenspektrum (ESI⁺): m/z = 332 [M+H]⁺
(54) 1-(2-Phenyl-ethyl)-3-vinyl-7-(3-methyl-2-buten-1-yl)-8-(3-amino-piperidin-1-yl)-xanthin
   R_{f}-Wert: 0.26 (Kieselgel, Methylenchlorid/Methanol/konz. wässriges Ammoniak = 90:10:1)
   Massenspektrum (ESI⁺): m/z = 449 [M+H]⁺
(55) 1-(3-Oxo-3-phenyl-propyl)-3-methyl-7-(3-methyl-2-buten-1-yl)-8-(3-amino-piperidin-1-yl)-xanthin
   Massenspektrum (ESI⁺): m/z = 465 (M+H]⁺
(56) 1-Methyl-3-(2-phenyl-2-oxo-ethyl)-7-(3-methyl-2-buten-1-yl)-8-(3-amino-piperidin-1-yl)-xanthin
   R_{f}-Wert: 0.30 (Kieselgel, Methylenchlorid/Methanol/konz. wässriges Ammoniak = 90:10:1)
   Massenspektrum (ESI⁺): m/z = 451 [M+H]⁺
(57) 1-Methyl-3-cyanomethyl-7-(3-methyl-2-buten-1-yl)-8-(3-amino-piperidin-1-yl)-xanthin
   R_{f}-Wert: 0.23 (Kieselgel, Methylenchlorid/Methanol/konz. wässriges Ammoniak = 90:10:1)

   Massenspektrum (ESI⁺): m/z = 372 [M+H]⁺
(58) 1-Methyl-3-(2-phenyl-ethyl)-7-(3-methyl-2-buten-1-yl)-8-(3-amino-piperidin-1-yl)-xanthin
   R_{f}-Wert: 0.20 (Kieselgel, Methylenchlorid/Methanol/konz. wässriges Ammoniak = 90:10:1)
   Massenspektrum (ESI⁺): m/z = 437 [M+H]⁺
(59) 1-Methyl-3-(2-dimethylamino-ethyl)-7-(3-methyl-2-buten-1-yl)-8-(3-amino-piperidin-1-yl)-xanthin
   R_{f}-Wert: 0.14 (Kieselgel, Methylenchlorid/Methanol/konz. wässriges Ammoniak = 80:20:1)
   Massenspektrum (ESI⁺): m/z = 404 [M+H]⁺
(60) 1-Methyl-3-isopropyl-7-(3-methyl-2-buten-1-yl)-8-(3-amino-piperidin-1-yl)-xanthin
   Schmelzpunkt: 115-117°C
   Massenspektrum (ESI⁺): m/z = 375 [M+H]⁺
(61) 1-(2-Hydroxy-2-phenyl-ethyl)-3-methyl-7-(3-methyl-2-buten-1-yl)-8-(3-amino-piperidin-1-yl)-xanthin
   R_{f}-Wert: 0.20 (Kieselgel, Methylenchlorid/Methanol/konz. wässriges Ammoniak = 90:10:1)
   Massenspektrum (ESI⁺): m/z = 453 [M+H]⁺
(62) 1-Methyl-3-(2-cyano-ethyl)-7-(3-methyl-2-buten-1-yl)-8-(3-amino-piperidin-1-yl)-xanthin
   Schmelzpunkt: 146-149°C
   Massenspektrum (ESI⁺): m/z = 386 [M+H]⁺
(63) 1-Methyl-3-[2-(4-methoxy-phenyl)-ethyl]-7-(3-methyl-2-buten-1-yl)-8-(3-amino-piperidin-1-yl)-xanthin
   R_{f}-Wert: 0.34 (Kieselgel, Methylenchlorid/Methanol/konz. wässriges Ammoniak = 90:10:1)
   Massenspektrum (ESI⁺): m/z = 467 [M+H]⁺
(64) 1-Methyl-3-phenyl-7-(3-methyl-2-buten-1-yl)-8-(3-amino-piperidin-1-yl)-xanthin
   R_{f}-Wert: 0.38 (Kieselgel, Methylenchlorid/Methanol/konz. wässriges Ammoniak = 90:10:1)
   Massenspektrum (ESI⁺): m/z = 409 [M+H]⁺
(65) 1-Methyl-3-[2-(3-methoxy-phenyl)-ethyl]-7-(3-methyl-2-buten-1-yl)-8-(3-amino-piperidin-1-yl)-xanthin
   R_{f}-Wert: 0.35 (Kieselgel, Methylenchlorid/Methanol/konz. wässriges Ammoniak = 90:10:1)
   Massenspektrum (ESI⁺): m/z = 467 [M+H]⁺
(66) 1-Methyl-3-[2-(2-methoxy-phenyl)-ethyl]-7-(3-methyl-2-buten-1-yl)-8-(3-amino-piperidin-1-yl)-xanthin
   R_{f}-Wert: 0.31 (Kieselgel, Methylenchlorid/Methanol/konz. wässriges Ammoniak = 90:10:1)
   Massenspektrum (ESI⁺): m/z = 467 [M+H]⁺
(67) 1-Methyl-3-[2-(3-methyl-phenyl)-ethyl]-7-(3-methyl-2-buten-1-yl)-8-(3-amino-piperidin-1-yl)-xanthin
   R_{f}-Wert: 0.13 (Kieselgel, Methylenchlorid/Methanol/konz. wässriges Ammoniak = 90:10:1)
   Massenspektrum (ESI⁺): m/z = 451 [M+H]⁺
(68) 1-Methyl-3-[2-(4-methyl-phenyl)-ethyl]-7-(3-methyl-2-buten-1-yl)-8-(3-amino-piperidin-1-yl)-xanthin
   R_{f}-Wert: 0.16 (Kieselgel, Methylenchlorid/Methanol/konz. wässriges Ammoniak = 95:5:1)
   Massenspektrum (ESI⁺): m/z = 451 [M+H]⁺
(69) 1-Methyl-3-[2-(2-methyl-phenyl)-ethyl]-7-(3-methyl-2-buten-1-yl)-8-(3-amino-piperidin-1-yl)-xanth in
   R_{f}-Wert: 0.16 (Kieselgel, Methylenchlorid/Methanol/konz. wässriges Ammoniak = 95:5:1)
   Massenspektrum (ESI⁺): m/z = 451 [M+H]⁺
(70) 1-Methyl-3-[2-(2-fluor-phenyl)-ethyl]-7-(3-methyl-2-buten-1-yl)-8-(3-amino-piperidin-1-yl)-xanthin
   R_{f}-Wert: 0.35 (Kieselgel, Methylenchlorid/Methanol/konz. wässriges Ammoniak = 90:10:1)
   Massenspektrum (ESI⁺): m/z = 455 [M+H]⁺
(71) 1-(2-Oxo-propyl)-3-methyl-7-(3-methyl-2-buten-1-yl)-8-(3-amino-piperidin-1-yl)-xanthin x Trifluoressigsäure
   (Das Produkt wird als Trifluoracetat isoliert.)
   Massenspektrum (ESI⁺): m/z = 389 [M+H]⁺
(72) 1-Methyl-3-(4-phenyl-butyl)-7-(3-methyl-2-buten-1-yl)-8-(3-amino-piperidin-1-yl)-xanthin
   R_{f}-Wert: 0.36 (Kieselgel, Methylenchlorid/Methanol/konz. wässriges Ammoniak = 90:10:1)
   Massenspektrum (ESI⁺): m/z = 465 [M+H]⁺
(73) 1-Methyl-3-(3-phenyl-propyl)-7-(3-methyl-2-buten-1-yl)-8-(3-amino-piperidin-1-yl)-xanthin
   R_{f}-Wert: 0.33 (Kieselgel, Methylenchlorid/Methanol/konz. wässriges Ammoniak = 90:10:1)
   Massenspektrum (ESI⁺): m/z = 451 [M+H]⁺
(74) 1-(2-Phenyl-2-oxo-ethyl)-3-methyl-7-(2-cyano-benzyl)-8-(3-amino-piperidin-1-yl)-xanthin
   Massenspektrum (ESI⁺): m/z = 498 [M+H]⁺
(75) 1-(2-Phenyl-ethyl)-3-methyl-7-(2-cyano-benzyl)-8-(3-amino-piperidin-1-yl)-xanthin
   Massenspektrum (ESI⁺): m/z = 484 [M+H]⁺
(76) 1-(3-Methoxycarbonyl-2-propen-1-yl)-3-methyl-7-(3-methyl-2-buten-1-yl)-8-(3-amino-piperidin-1-yl)-xanthin
   R_{f}-Wert: 0.35 (Kieselgel, Methylenchlorid/Methanol/konz. wässriges Ammoniak = 80:20:1)
   Massenspektrum (ESI⁺): m/z = 431 [M+H]⁺
(77) 1-Methyl-3-[2-(4-fluor-phenyl)-ethyl]-7-(3-methyl-2-buten-1-yl)-8-(3-amino-piperidin-1-yl)-xanthin
   R_{f}-Wert: 0.28 (Kieselgel, Methylenchlorid/Methanol/konz. wässriges Ammoniak = 90:10:1)
   Massenspektrum (ESI⁺): m/z = 455 [M+H]⁺
(78) 1-Methyl-3-(2-(3-fluor-phenyl)-ethyl]-7-(3-methyl-2-buten-1-yl)-8-(3-amino-piperidin-1-yl)-xanthin
   R_{f}-Wert: 0.35 (Kieselgel, Methylenchlorid/Methanol/konz. wässriges Ammoniak = 90:10:1)
   Massenspektrum (ESI⁺): m/z = 455 [M+H]⁺
(79) 1-[2-(2,5-Dimethoxy-phenyl)-2-oxo-ethyl]-3-methyl-7-(3-methyl-2-buten-1-yl)-8-(3-amino-piperidin-1-yl)-xanthin
   R_{f}-Wert: 0.29 (Kieselgel, Essigester/Methanol/konz. wässriges Ammoniak = 70:30:1)
   Massenspektrum (ESI⁺): m/z = 511 [M+H]⁺
(80) 1-[2-(4-Fluor-phenyl)-2-oxo-ethyl]-3-methyl-7-(3-methyl-2-buten-1-yl)-8-(3-amino-piperidin-1-yl)-xanthin
   R_{f}-Wert: 0.35 (Kieselgel, Methylenchlorid/Methanol/konz. wässriges Ammoniak = 80:20:1)
   Massenspektrum (ESI⁺): m/z = 469 [M+H]⁺
(81) 1-Phenylcarbonylamino-7-(3-methyl-2-buten-1-yl)-8-(3-amino-piperidin-1-yl)-xanthin
   (Verunreinigt mit 1-Phenylcarbonylamino-7-(3-methyl-butyl)-8-(3-amino-piperidin-1-yl)-xanthin)
   R_{f}-Wert: 0.26 (Kieselgel, Methylenehlorid/Methanol/konz. wässriges Ammoniak = 80:20:1)
   Massenspektrum (ESI⁺): m/z = 438 [M+H]⁺
(82) 1-Amino-7-(3-methyl-2-buten-1-yl)-8-(3-amino-piperidin-1-yl)-xanthin
   (Verunreinigt mit 1-Amino-7-(3-methyl-butyl)-8-(3-amino-piperidin-1-yl)-xanthin)
   R_{f}-Wert: 0.22 (Kieselgel, Methylenchlorid/Methanol/konz. wässriges Ammoniak = 80:20:1)
   Massenspektrum (ESI⁺): m/z = 334 [M+H]⁺
(83) 1-[2-(3-Methansulfonyloxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 545 [M+H]⁺
(84) 1-[2-(3-Allyloxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 507 [M+H]⁺
(85) 1-{2-Oxo-2-[3-(2-propin-1-yloxy)-phenyl]-ethyl}-3-methyl-7-(3-methyl-2-buten-1-yl)-8-(3-amino-piperidin-1-y))-xanthin
   Massenspektrum (ESI⁺): m/z = 505 [M+H]⁺
(86) 1-(3-Methoxycarbonyl-2-propen-1-yl)-3-methyl-7-(2-cyano-benzyl)-8-(3-amino-piperidin-1-yl)-xanthin
   Massenspektrum (ESI⁺): m/z = 478 [M+H]⁺
(87) 1-(2-{3-[(Methoxycarbonyl)methoxy]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 539 [M+H]⁺
(88) 1-[2-(3-Cyanomethoxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 506 [M+H]⁺
(89) 1-[2-(3-Benzyloxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 557 [M+H]⁺
(90) 1-[2-(3-Phenylsulfonyloxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 607 [M+H]⁺
(91) 1-[2-(3-Hydroxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 467 [M+H]⁺
(92) 1-[(Pyridin-2-yl)methyl]-3-methyl-7-(2-cyano-benzyl)-8-(3-amino-piperidin-1-yl)-xanthin
   R_{f}-Wert: 0.20 (Kieselgel, Methylenchlorid/Methanol/konz. wässriges Ammoniak = 90:10:1)
   Massenspektrum (ESI⁺): m/z = 471 [M+H]⁺
(93) 1-[2-(3-Phenyloxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 543 [M+H]⁺
(94) 1-(2-Phenyl-2-oxo-ethyl)-3-[(methoxycarbonyl)methyl]-7-(3-methyl-2-buten-1-yl)-8-(3-amino-piperidin-1-yl)-xanthin
   R_{f}-Wert: 0.29 (Kieselgel, Methylenchlorid/Methanol/konz. wässriges Ammoniak = 90:10:1)
   Massenspektrum (ESI⁺): m/z = 509 [M+H]⁺
(95) 1-(2-Phenyl-2-oxo-ethyl)-3-methyl-7-(3-methyl-2-buten-1-yl)-8-(piperazin-1-yl)-xanthin
   R_{f}-Wert: 0.10 (Kieselgel, Methylenchlorid/Methanol = 90:10)
   Massenspektrum (ESI⁺): m/z = 437 [M+H]⁺
(96) 1-[2-(3-Amino-phenyl)-2-oxo-ethyl]-3-methyl-7-(3-methyl-2-buten-1-yl)-8-(3-amino-piperidin-1-yl)-xanthin
   R_{f}-Wert: 0.25 (Kieselgel, Methylenchlorid/Methanol/konz. wässriges Ammoniak = 80:20:1)
   Massenspektrum (ESI⁺): m/z = 466 [M+H]⁺
(97) 1-(2-{3-[Bis(methansulfonyl)-amino]-phenyl}-2-oxo-ethyl)-3-methyl-7-(3-methyl-2-buten-1-yl)-8-(3-amino-piperidin-1-yl)-xanthin
   R_{f}-Wert: 0.45 (Kieselgel, Methylenchlorid/Methanol/konz. wässriges Ammoniak = 80:20:1)
   Massenspektrum (ESI⁺): m/z = 622 [M+H]⁺
(98) 1-[2-(2-Brom-5-dimethylamino-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 572, 574 [M+H]⁺
(99) 1-[2-(3-Nitro-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 496 [M+H]⁺
(100) 1-[2-(3-Methoxycarbonylamino-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 524 [M+H]⁺
(101) 1-[2-(3-Acetylamino-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 508 [M+H]⁺
(102) 1-[2-(3-{[(Ethoxycarbonylamino)carbonyl]amino}-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 581 [M+H]⁺
(103) 1-(2-Phenyl-2-oxo-ethyl)-3-methyl-7-(3-methyl-2-buten-1-yl)-8-(homopiperazin-1-yl)-xanthin
   R_{f}-Wert: 0.10 (Kieselgel, Methylenchlorid/Methanol = 90:10)
   Massenspektrum (ESI⁺): m/z = 451 [M+H]⁺
(104) 1-[2-(3-Cyanomethylamino-phenyl)-2-oxo-ethyl]-3-methyl-7-(3-methyl-2-buten-1-yl)-8-(3-amino-piperidin-1-yl)-xanthin
   R_{f}-Wert: 0.35 (Kieselgel, Methylenchlorid/Methanol/konz. wässriges Ammoniak = 80:20:1)
   Massenspektrum (ESI⁺): m/z = 505 [M+H]⁺
(105) 1,3-Dimethyl-7-(3-methyl-2-buten-1-yl)-8-(4-aminomethyl-piperidin-1-yl)-xanthin
   Durchführung mit isopropanolischer Salzsäure (5-6M) in Methylenchlorid Schmelzpunkt: 110-112°C
   Massenspektrum (ESI⁺): m/z = 361 [M+H]⁺
(106) 1,3-Dimethyl-7-(3-methyl-2-buten-1-yl)-8-(3-aminomethyl-piperidin-1-yl)-xanthin
   Durchführung mit isopropanolischer Salzsäure (5-6M) in Methylenchlorid.
   R_{f}-Wert: 0.48 (Kieselgel, Methylenchlorid/Methanol/konz. wässriges Ammoniak = 90:10:0.1)
   Massenspektrum (ESI⁺): m/z = 361 [M+H]⁺
(107) 1,3-Dimethyl-7-(3-methyl-2-buten-1-yl)-8-(trans-2-amino-cyclobutylamino)-xanthin
   Durchführung mit isopropanolischer Salzsäure (5-6M) in Methylenchlorid.
   R_{f}-Wert: 0.65 (Kieselgel, Methylenchlorid/Methanol/konz. wässriges Ammoniak = 90:10:0.1)
   Massenspektrum (ESI⁺): m/z = 333 [M+H]⁺
(108) 1,3-Dimethyl-7-(3-methyl-2-buten-1-yl)-8-[N-((S)-2-amino-1-methyl-ethyl)-N-methyl-amino]-xanthin
   Durchführung mit isopropanolischer Salzsäure (5-6M) in Methylenchlorid.
   Schmelzpunkt: 109.5-113°C
   Massenspektrum (ESI⁺): m/z = 335 [M+H]⁺
(109) 1,3-Dimethyl-7-(3-methyl-2-buten-1-yl)-8-[N-((*R*)-2-amino-1-methyl-ethyl)-N-methyl-amino]-xanthin
   Durchführung mit isopropanolischer Salzsäure (5-6M) in Methylenchlorid.
   R_{f}-Wert: 0.50 (Kieselgel, Methylenchlorid/Methanol/konz. wässriges Ammoniak = 90:10:1)
   Massenspektrum (ESI⁺): m/z = 335 [M+H]⁺
(110) 1,3-Dimethyl-7-(3-methyl-2-buten-1-yl)-8-[cis-N-(2-amino-cydohexyl)-N-methyl-amino]-xanthin
   Durchführung mit isopropanolischer Salzsäure (5-6M) in Methylenchlorid.
   R_{f}-Wert: 0.71 (Kieselgel, Methylenchlorid/Methanol/konz. wässriges Ammoniak = 90:10:1)
   Massenspektrum (ESI⁺): m/z = 375 [M+H]⁺
(111)1,3-Dimethyl-7-(3-methyl-2-buten-1-yl)-8-(6-amino-[1,4]diazepan-1-yl)-xanthin Durchführung mit isopropanolischer Salzsäure (5-6M) in Methylenchlorid.
   R_{f}-Wert: 0.41 (Kieselgel, Methylenchlorid/Methanol/konz. wässriges Ammoniak = 90:10:1)
   Massenspektrum (ESI⁺): m/z = 362 [M+H]⁺
(112) 1,3-Dimethyl-7-(3-methyl-2-buten-1-yl)-8-[N-(2-amino-2-methyl-propyl)-N-methyl-amino]-xanthin
   Durchführung mit isopropanolischer Salzsäure (5-6M) in Methylenchlorid.
   Schmelzpunkt: 156.5-159.5°C
   Massenspektrum (ESI⁺): m/z = 349 [M+H]⁺
(113) 1-[(Pyridin-2-yl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   Schmelzpunkt: 136-139.5°C
   Massenspektrum (ESI⁺): m/z = 424 [M+H]⁺
(114) 1-[(Thiazol-2-yl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   Schmelzpunkt: 124-127°C
   Massenspektrum (ESI⁺): m/z = 430 [M+H]⁺
(115) 1,3-Dimethyl-7-(3-methyl-2-buten-1-yl)-8-(trans-2-amino-cyclopentylamino)-xanthin
   Durchführung mit isopropanolischer Salzsäure (5-6M) in Methylenchlorid.
   R_{f}-Wert: 0.25 (Kieselgel, Methylenchlorid/Methanol/konz. wässriges Ammoniak = 95:5:0.1)
   Massenspektrum (ESI⁺): m/z = 347 [M+H]⁺
(116) 1,3-Dimethyl-7-(3-methyl-2-buten-1-yl)-8-(trans-3-amino-cyclohexylamino)-xanthin (mit ca. 25% cis-Verbindung verunreinigt)
   Durchführung mit isopropanolischer Salzsäure (5-6M) in Methylenchlorid.
   R_{f}-Wert: 0.16 (Kieselgel, Methylenchlorid/Methanol/konz. wässriges Ammoniak = 90:10:1)
   Massenspektrum (ESI⁻): m/z = 359 [M-H]⁻
(117) 1,3-Dimethyl-7-(3-methyl-2-buten-1-yl)-8-(cis-3-amino-cyclohexylamino)-xanthin (mit ca. 21 % trans-Verbindung verunreinigt)
   Durchführung mit isopropanolischer Salzsäure (5-6M) in Methylenchlorid.
   R_{f}-Wert: 0.21 (Kieselgel, Methylenchlorid/Methanol/konz. wässriges Ammoniak = 90:10:1)
   Massenspektrum (ESI⁻) m/z = 359 [M-H]⁻
(118) 1,3-Dimethyl-7-(3-methyl-2-buten-1-yl)-8-(cis-2-amino-cyclopentylamino)-xanthin
   Durchführung mit isopropanolischer Salzsäure (5-6M) in Methylenchlorid.
   R_{f}-Wert: 0.25 (Kieselgel, Methylenchlorid/Methanol/konz. wässriges Ammoniak = 95:5:0.1)
   Massenspektrum (ESI⁺): m/z = 347 [M+H]⁺
(119) 1-[(Isochinolin-1-yl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   Schmelzpunkt: 146-149°C
   Massenspektrum (ESI⁺): m/z = 474 [M+H]⁺
(120) 1,3-Dimethyl-7-(3-methyl-2-buten-1-yl)-8-(cis-3-amino-cyclopentylamino)-xanthin
   Durchführung mit isopropanolischer Salzsäure (5-6M) in Methylenchlorid.
   Schmelzpunkt: 146-148°C
   Massenspektrum (ESI⁺): m/z = 347 [M+H]⁺
(121) 1-[(Benzo[*d*]isothiazol-3-yl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   Schmelzpunkt: 129-131 °C
   Massenspektrum (ESI⁺): m/z = 480 [M+H]⁺
(122) 1-[(Pyridin-3-yl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   R_{f}-Wert: 0.42 (Kieselgel, Methylenchlorid/Methanol/konz. wässriges Ammoniak = 90:10:1)
   Massenspektrum (ESI⁺): m/z = 424 [M+H]⁺
(123) 1-[(Pyridin-4-yl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   R_{f}-Wert: 0.48 (Kieselgel, Methylenchlorid/Methanol/konz. wässriges Ammoniak = 90:10:1)
   Massenspektrum (ESI⁺): m/z = 424 [M+H]⁺
(124) 1-[(Isoxazol-3-yl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   Schmelzpunkt: 124-127.5°C
   Massenspektrum (ESI⁺): m/z = 414 [M+H]⁺
(125) 1-[(Isochinofin-1-yl)methyl]-3-methyl-7-(3-methyl-2-buten-1-yl)-8-((*R*)-3-amino-piperidin-1-yl)-xanthin
   Durchführung mit isopropanolischer Salzsäure (5-6M) in Methylenchlorid.
   R_{f}-Wert: 0.50 (Kieselgel, Methylenchlorid/Methanol/konz. wässriges Ammoniak = 90:10:1)
   Massenspektrum (ESI⁺): m/z = 474 [M+H]⁺
(126) 1-[(Isochinolin-1-yl)methyl]-3-methyl-7-(3-methyl-2-buten-1-yl)-8-((*S*)-3-amino-piperidin-1-yl)-xanthin
   Durchführung mit isopropanolischer Salzsäure (5-6M) in Methylenchlorid.
   Massenspektrum (ESI⁺): m/z = 474 [M+H]⁺
(127)1-[(1-Naphthyl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   R_{f}-Wert: 0.51 (Kieselgel, Methylenchlorid/Methanol/konz. wässriges Ammoniak = 90:10:1)
   Massenspektrum (ESI⁺): m/z = 473 [M+H]⁺
(128) 1-[(Benzo[*d*]isoxazol-3-yl)methyl]-3-methyl-7-(3-methyl-2-buten-1-yl)-8-(3-amino-piperidin-1-yl)-xanthin

   R_{f}-Wert: 0.20 (Kieselgel, Methylenchlorid/Methanol = 9:1)
   Massenspektrum (ESI⁺): m/z = 464 [M+H]⁺
(129) 1-(2-Phenyl-2-oxo-ethyl)-3-methyl-7-(3-methyl-2-buten-1-yl)-8-(3-amino-3-methyl-piperidin-1-yl)-xanthin
   R_{f}-Wert: 0.18 (Kieselgel, Essigester/Methanol/konz. wässriges Ammoniak = 90:10:1)
   Massenspektrum (ESI⁺): m/z = 465 [M+H]⁺
(130) 1,3-Dimethyl-7-(3-methyl-2-buten-1-yl)-8-(3-amino-3-methyl-piperidin-1-yl)-xanthin
   R_{f}-Wert: 0.41 (Aluminiumoxid, Methylenchlorid/Methanol = 20:1)
   Massenspektrum (ESI⁺): m/z = 361 [M+H]⁺
(131)1,3-Dimethyl-7-(3-methyl-2-buten-1-yl)-8-[N-(2-amino-3-dimethylamino-3-oxo-propyl)-N-methyl-amino]-xanthin x Trifluoressigsäure
   R_{f}-Wert: 0.31 (Kieselgel, Methylenchlorid/Methanol/konz. wässriges Ammoniak = 40:10:1)
   Massenspektrum (ESI⁺): m/z = 392 [M+H]⁺
(132) 1,3-Dimethyl-7-(3-methyl-2-buten-1-yl)-8-[N-(2,3-diamino-3-oxo-propyl)-N-methyl-amino]-xanthin x Trifluoressigsäure
   R_{f}-Wert: 0.28 (Kieselgel, Methylenchlorid/Methanol/konz. wässriges Ammoniak = 40:10:1)
   Massenspektrum (ESI⁺): m/z = 364 [M+H]⁺
(133) 1-[(Aminocarbonyl)methyl)]-3-methyl-7-(2-cyano-benzyl)-8-(3-amino-piperidin-1-yl)-xanthin
   Hergestellt aus 1-Cyanomethyl-3-methyl-7-(2-cyano-benzyl)-8-[3-(tert.-butyloxycarbonylamino)-piperidin-1-yl]-xanthin. Bei der Behandlung mit Trifluoressigsäure wird sowohl die Schutzgruppe abgespalten als auch die Cyanogruppe zum Amid hydrolysiert.
   R_{f}-Wert: 0.10 (Kieselgel, Methylenchlorid/Methanol/konz. wässriges Ammoniak = 90:10:0.1)
   Massenspektrum (ESI⁺): m/z = 437 [M+H]⁺
(134) 1-[2-(3-Methansulfonylamino-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 544 [M+H]⁺
   R_{f}-Wert: 0.45 (Kieselgel, Methylenchlorid/Methanol/Triethylamin = 90:10:0.1)
(135) 1-[2-(2-Nitro-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 496 [M+H]⁺
(136) 1-[2-(2-Amino-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 466 [M+H]⁺
(137) 1-(2-{3-[(Methylamino)thiocarbonylamino]-phenyl}-2-oxo-ethyl)-3-methyl-7-(3-methyl-2-buten-1-yl)-8-(3-amino-piperidin-1-yl)-xanthin
   R_{f}-Wert: 0.30 (Kieselgel, Methylenchlorid/Methanol/konz. wässriges Ammoniak = 80:20:0.1)
   Massenspektrum (ESI⁺): m/z = 539 [M+H]⁺
(138) 1-[2-(2-Acetylamino-phenyl)-2-oxo-ethyl]-3-methyl-7-(3-methyl-2-buten-l-yl)-8-(3-amino-pipehdin-1-yl)-xanthin
   Massenspektrum (ESI⁺): m/z = 508 [M+H]⁺
(139) 1-[(6-Methyl-pyridin-2-yl)methyl]-3-methyl-7-(3-methyl-2-buten-1-yl)-8-(3-amino-piperidin-1-yl)-xanthin
   Durchführung mit isopropanolischer Salzsäure (5-6M) in Methylenchlorid.
   Schmelzpunkt: 127.5-130°C
   Massenspektrum (ESI⁺): m/z = 438 [M+H]⁺
(140) 1-[(Isochinolin-4-yl)methyl]-3-methyl-7-(3-methyl-2-buten-1-yl)8-(3-amino-piperidin-1-yl)-xanthin
   Durchführung mit isopropanolischer Salzsäure (5-6M) in Methylenchlorid.
   R_{f}-Wert: 0.40 (Kieselgel, Methylenchlorid/Methanol/konz. wässriges Ammoniak = 90:10:1)
   Massenspektrum (ESI⁺): m/z = 474 [M+H]⁺
(141) 1-[(1-Methyl-1*H*-indazol-3-yl)methyl]-3-methyl-7-(3-methyl-2-buten-1-yl)-8-(3-amino-piperidin-1-yl)-xanth in
   Durchführung mit isopropanolischer Salzsäure (5-6M) in Methylenchlorid.
   R_{f}-Wert: 0.31 (Kieselgel, Methylenchlorid/Methanol/konz. wässriges Ammoniak = 90:10:1)
   Massenspektrum (ESI⁺): m/z = 477 [M+H]⁺
(142) 1,3-Dimethyl-7-(3-methyl-2-buten-1-yl)-8-{N-[2-amino-3-oxo-3-(pyrrolidin-1-yl)-propyl]-N-methyl-amino}-xanthin
   Schmelzpunkt: 138°C
   Massenspektrum (ESI⁺): m/z = 418 [M+H]⁺
(143) 1,3-Dimethyl-7-(3-methyl-2-buten-1-yl)-8-[N-(2-amino-3-methylamino-3-oxo-propyl)-N-methyl-amino]-xanthin
   R_{f}-Wert: 0.20 (Kieselgel, Methylenchlorid/Methanol/konz. wässriges Ammoniak = 90:10:1)
   Massenspektrum (ESI⁺): m/z = 378 [M+H]⁺
(144) 1-(2-{3-[(Methoxycarbonyl)methylamino]-phenyl}-2-oxo-ethyl)-3-methyl-7-(3-methyl-2-buten-1-yl)-8-(3-amino-piperidin-1-yl)-xanthin
   R_{f}-Wert: 0.29 (Kieselgel, Methylenchlorid/Methanol/konz. wässriges Ammoniak = 80:20:0.1)
   Massenspektrum (ESI⁺): m/z = 538 [M+H]⁺
(145) 1-Cyanomethyl-3-methyl-7-(2-cyano-benzyl)-8-(3-amino-piperidin-1-yl)-xanthin Durchführung mit isopropanolischer Salzsäure (5-6M) in Methylenchlorid.
   R_{f}-Wert: 0.60 (Kieselgel, Methylenchlorid/Methanol = 9:2)
   Massenspektrum (ESI⁺): m/z = 419 [M+H]⁺
(146) 1-[2-(2-Hydroxy-phenyl)-2-oxo-ethyl]-3-methyl-7-(3-methyl-2-buten-1-yl)-8-(3-amino-piperidin-1-yl)-xanthin x Trifluoressigsäure
   Massenspektrum (ESI⁺): m/z = 467 [M+H]⁺
(147) 1-[2-(2-Methansulfonyloxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 545 [M+H]⁺
(148) 1-(2-{2-[(Methoxycarbonyl)methoxy]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 539 [M+H]⁺
(149) 1-[2-(2-Cyanomethoxy-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 506 [M+H]⁺
(150) 1-(2-{3-[(Dimethylaminocarbonyl)methoxy]-phenyl}-2-oxo-ethyl)-3-methyl-7-(3-methyl-2-buten-1-yl)-8-(3-amino-piperidin-1-yl)-xanthin
   R_{f}-Wert: 0.45 (Kieselgel, Methylenchlorid/Methanol/Triethylamin = 80:20:0.1)
   Massenspektrum (ESI⁺): m/z = 552 [M+H]⁺
(151) 1-(2-(3-[(Methylaminocarbonyl)methoxy]-phenyl}-2-oxo-ethyl)-3-methyl-7-(3-methyl-2-buten-1-yl)-8-(3-amino-piperidin-1-yl)-xanthin
   R_{f}-Wert: 0.55 (Kieselgel, Methylenchlorid/Methanol/Triethylamin = 80:20:0.1)
   Massenspektrum (ESI⁺): m/z = 538 [M+H]⁺
(152) 1-(2-{3-[(Aminocarbonyl)methoxy]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 524 [M+H]⁺
(153) 1-(2-{2-[Bis(methansulfonyl)-amino]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 622 [M+H]⁺
(154) 1-Methyl-3-[2-(4-methoxy-phenyl)-ethyl]-7-(2-cyano-benzyl)-8-(3-amino-piperidin-1-yl)-xanthin
   R_{f}-Wert: 0.35 (Kieselgel, Methylenchlorid/Methanol = 9:1)
   Massenspektrum (ESI⁺): m/z = 514 [M+H]⁺
(155) 1-Methyl-3-(2-phenyl-ethyl)-7-(2-cyano-benzyl)-8-(3-amino-piperidin-1-yl)-xanthin
   Massenspektrum (ESI⁺): m/z = 484 [M+H]⁺
(156) 1-(2-{3-[(Aminocarbonyl)amino]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 509 [M+H]⁺
(157) 1-(2-{3-[(Dimethylaminocarbonyl)amino]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 537 [M+H]⁺
(158) 1-Methyl-3-((E)-2-phenyl-vinyl)-7-(3-methyl-2-buten-1-yl)-8-(3-amino-piperidin-1-yl)-xanthin
   R_{f}-Wert: 0.49 (Kieselgel, Methylenchlorid/Methanol/konz. wässriges Ammoniak = 90:10:1)
   Massenspektrum (ESI⁺): m/z = 435 [M+H]⁺
(159) 1-(4-Oxo-4*H*-chromen-3-yl)-3-methyl-7-(3-methyl-2-buten-1-yl)-8-(3-amino-piperidin-1-yl)-xanthin x Trifluoressigsäure
   Massenspektrum (ESI⁺): m/z = 477 [M+H]⁺
(160) 1-[(3-Methyl-pyridin-2-yl)methyl]-3-methyl-7-(3-methyl-2-buten-1-yl)-8-(3-amino-piperidin-1-yl)-xanthin
   Durchführung mit isopropanolischer Salzsäure (5-6 M) in Methylenchlorid.
   R_{f}-Wert: 0.54 (Kieselgel, Methylenchlorid/Methanol/konz. wässriges Ammoniak = 90:10:1)
   Massenspektrum (ESI⁺): m/z = 438 [M+H]⁺
(161) 1-[(5-Methyl-pyridin-2-yl)methyl]-3-methyl-7-(3-methyl-2-buten-1-yl)-8-(3-amino-piperidin-1-yl)-xanthin
   Durchführung mit isopropanolischer Salzsäure (5-6 M) in Methylenchlorid.
   R_{f}-Wert: 0.35 (Kieselgel, Methylenchlorid/Methanol/konz. wässriges Ammoniak = 90:10:1)
   Massenspektrum (ESI⁺): m/z = 438 [M+H]⁺
(162) 1-[(4-Methyl-pyridin-2-yl)methyl]-3-methyl-7-(3-methyl-2-buten-1-yl)-8-(3-amino-piperidin-1-yl)-xanthin
   Durchführung mit isopropanolischer Salzsäure (5-6 M) in Methylenchlorid.
   R_{f}-Wert: 0.39 (Kieselgel, Methylenchlorid/Methanol/konz. wässriges Ammoniak = 90:10:1)
   Massenspektrum (ESI⁺): m/z = 438 [M+H]⁺
(163) 1-[(Chinolin-4-yl)methyl]-3-methyl-7-(3-methyl-2-buten-1-yl)-8-(3-amino-piperidin-1-yl)-xanthin
   Durchführung mit isopropanolischer Salzsäure (5-6 M) in Methylenchlorid.
   R_{f}-Wert: 0.53 (Kieselgel, Methylenchlorid/Methanol/konz. wässriges Ammoniak = 90:10:1)
   Massenspektrum (ESI⁺): m/z = 474 [M+H]⁺
(164) 1,3-Dimethyl-7-(3-methyl-2-buten-1-yl)-8-(endo-6-amino-2-aza-bicyclo[2.2.2]oct-2-yl)-xanthin
   Durchführung mit isopropanolischer Salzsäure (5-6 M) in Methylenchlorid.
   Schmelzpunkt: 174-179°C
   Massenspektrum (ESI⁺): m/z = 373 [M+H]⁺
(165) 1-[(Chinolin-8-yl)methyl]-3-methyl-7-(3-methyl-2-buten-1-yl)-8-(3-amino-piperidin-1-yl)-xanthin
   Durchführung mit isopropanolischer Salzsäure (5-6 M) in Methylenchlorid.
   Schmelzpunkt: 175-177°C
   Massenspektrum (ESI⁺): m/z = 474 [M+H]⁺
(166) 1-[(5-Nitro-isochinolin-1-yl)methyl]-3-methyl-7-(3-methyl-2-buten-1-yl)-8-(3-amino-piperidin-1-yl)-xanthin
   Durchführung mit isopropanolischer Salzsäure (5-6 M) in Methylenchlorid.
   R_{f}-Wert: 0.47 (Kieselgel, Methylenchlorid/Methanol/konz. wässriges Ammoniak = 90:10:1)
   Massenspektrum (ESI⁺): m/z = 519 [M+H]⁺
(167) 1,3-Dimethyl-7-(3-methyl-2-buten-1-yl)-8-(exo-6-amino-2-aza-bicyclo[2.2.2]oct-2-yl)-xanthin
   Durchführung mit isopropanolischer Salzsäure (5-6 M) in Methylenchlorid.
   R_{f}-Wert: 0.23 (Kieselgel, Methylenchlorid/Methanol/konz. wässriges Ammoniak = 95:5:0.1)
   Massenspektrum (ESI⁺): m/z = 373 [M+H]⁺
(168) 1-[(2-Oxo-1,2-dihydro-chinolin-4-yl)methyl]-3-methyl-7-(3-methyl-2-buten-1-yl)-8-(3-amino-piperidin-1-yl)-xanthin
   Durchführung mit isopropanolischer Salzsäure (5-6 M) in Methylenchlorid.
   R_{f}-Wert: 0.43 (Kieselgel, Methylenchlorid/Methanol/konz. wässriges Ammoniak = 90:10:1)
   Massenspektrum (ESI⁺): m/z = 490 [M+H]⁺
(169) 1-[(5-Amino-isochinolin-1-yl)methyl]-3-methyl-7-(3-methyl-2-buten-1-yl)-8-(3-amino-piperidin-1-yl)-xanthin
   Durchführung mit isopropanolischer Salzsäure (5-6 M) in Methylenchlorid.
   R_{f}-Wert: 0.39 (Kieselgel, Methylenchlorid/Methanol/konz. wässriges Ammoniak = 90:10:1)
   Massenspektrum (ESI⁺): m/z = 489 [M+H]⁺
(170) 1-[2-(3-Cyano-phenyl)-2-oxo-ethyl]-3-methyl-7-(3-methyl-2-buten-1-yl)-8-(3-amino-piperidin-1-yl)-xanthin
   R_{f}-Wert: 0.65 (Kieselgel, Methylenchlorid/Methanol/konz. wässriges Ammoniak = 90:10:1)
   Massenspektrum (ESI⁺): m/z = 476 [M+H]⁺
(171) 1-[2-(3-Aminosulfonyl-phenyl)-2-oxo-ethyl]-3-methyl-7-(3-methyl-2-buten-1-yl)-8-(3-amino-piperidin-1-yl)-xanthin
   R_{f}-Wert: 0.24 (Kieselgel, Methylenchlorid/Methanol/konz. wässriges Ammoniak = 90:10:1)
   Massenspektrum (ESI⁺): m/z = 530 [M+H]⁺
(172) 1-[2-(3-Aminocarbonyl-phenyl)-2-oxo-ethyl]-3-methyl-7-(3-methyl-2-buten-1-yl)-8-(3-amino-piperidin-1-yl)-xanthin
   R_{f}-Wert: 0.10 (Kieselgel, Methylenchlorid/Methanol/konz. wässriges Ammoniak = 90:10:1)
   Massenspektrum (ESI⁺): m/z = 494 [M+H]⁺
(173) 1-(2-Phenoxy-ethyl)-3-methyl-7-(3-methyl-2-buten-1-yl)-8-(3-amino-piperidin-1-yl)-xanthin
   Massenspektrum (ESI⁺): m/z = 453 [M+H]⁺
(174) 1,3-Dimethyl-2-thioxo-7-benzyl-8-(3-amino-piperidin-1-yl)-xanthin x Trifluoressigsäure
   R_{f}-Wert: 0.50 (Aluminiumoxid, Methylenchlorid/Methanol = 20:1)
   Massenspektrum (ESI⁺): m/z = 385 [M+H]⁺

### Beispiel 3

### 1,3-Dimethyl-7-(3-methyl-2-buten-1-yl)-8-(3-methylamino-piperidin-1-yl)-xanthin

154 mg 1,3-Dimethyl-7-(3-methyl-2-buten-1-yl)-8-(3-amino-piperidin-1-yl)-xanthin und 0,032 ml wässrige Formaldehyd-Lösung (37 Gewichtsprozent) in 0,5 ml Methanol werden mit 24 mg Natriumborhydrid versetzt und bei Raumtemperatur gerührt.
Es werden noch zweimal je 0.01 ml Formaldehyd-Lösung und 10 mg Natriumborhydrid zugesetzt und weiter bei Raumtemperatur gerührt. Das Reaktionsgemisch wird mit 1 M Natronlauge versetzt und mehrmals mit Essigester extrahiert. Die organischen Phasen werden vereint, getrocknet und eingeengt. Der Rückstand wird durch Chromatographie über eine Aluminiumoxid-Säule mit Essigester/Methanol gereinigt.
Ausbeute: 160 mg (25% d. Theorie)
Massenspektrum (ESI⁺): m/z = 361 [M+H]⁺
R_{f}-Wert: 0.80 (Aluminiumoxid, Essigester/Methanol = 4:1)

Analog Beispiel 3 wird folgende Verbindung erhalten:
(1) 1,3-Dimethyl-7-(3-methyl-2-buten-1-yl)-8-(3-dimethylamino-piperidin-1-yl)-xanthin
   Massenspektrum (ESI⁺): m/z = 375 [M+H]⁺
   R_{f}-Wert: 0.65 (Aluminiumoxid, Methylenchlorid/Methanol = 100:1)

### Beispiel 4

### (S)-1,3-Dimethyl-7-(3-methyl-2-buten-1-yl)-8-{3-[(2-cyanpyrrolidin-1-ylcarbonyl-methyl)amino]-piperidin-1-yl}-xanthin

hergestellt durch Umsetzung der Verbindung des Beispiels 1 (4) mit (S)-1-(Brom-acetyl)-2-cyan-pyrrolidin in Tetrahydrofuran in Gegenwart von Triethylamin bei Raumtemperatur
Schmelzpunkt: 67-68°C
Massenspektrum (ESI⁺): m/z = 505 [M+Na]⁺

### Beispiel 5

### 1-Methyl-7-benzyl-8-(3-amino-piperidin-1-yl)-xanthin

hergestellt durch Behandeln von 1-Methyl-3-(2-trimethylsilanyl-ethoxymethyl)-7-benzyl-8-(3-amino-piperidin-1-yl)-xanthin mit Trifluoressigsäure in Methylenchlorid bei Raumtemperatur
Massenspektrum (ESI⁺): m/z = 355 [M+H]⁺

### Beispiel 6

### 1-Methyl-3-carboxymethyl-7-benzyl-8-(3-amino-piperidin-1-yl)-xanthin

hergestellt durch Behandeln von 1-Methyl-3-[(methoxycarbonyl)-methyl]-7-benzyl-8-(3-amino-piperidin-1-yl)-xanthin mit 1 N Natronlauge in Methanol Schmelzpunkt: 212-215°C
Massenspektrum (ESI⁺): m/z = 413 [M+H]⁺

Analog Beispiel 6 werden folgende Verbindungen erhalten:
(1) 1-Carboxymethyl-3-methyl-7-(3-methyl-2-buten-1-yl)-8-((*S*)-3-amino-piperidin-1-yl)-xanthin
   R_{f}-Wert: 0.54 (Reversed Phase DC-Fertigplatte (E. Merck), Acetonitril/Wasser/ Trifluoressigsäure = 50:50:1)
   Massenspektrum (ESI⁺): m/z = 391 [M+H]⁺
(2) 1-(3-Carboxy-propyl)-3-methyl-7-(3-methyl-2-buten-1-yl)-8-((*S*)-3-amino-piperidin-1-yl)-xanthin
   R_{f}-Wert: 0.42 (Reversed Phase DC-Fertigplatte (E. Merck), Acetonitril/Wasser/ Trifluoressigsäure = 50:50:1)
   Massenspektrum (ESI⁺): m/z = 419 [M+H]⁺
(3) 1-[2-(4-Carboxy-phenyl)-ethyl]-3-methyl-7-(3-methyl-2-buten-1-yl)-8-((*S*)-3-amino-piperidin-1-yl)-xanthin
   R_{f}-Wert: 0.42 (Reversed Phase DC-Fertigplatte (E. Merck), Acetonitril/Wasser/ Trifluoressigsäure = 50:50:1)
   Massenspektrum (ESI⁺): m/z = 481 [M+H]⁺
(4) 1-(2-Carboxy-ethyl)-3-methyl-7-(3-methyl-2-buten-1-yl)-8-((*S*)-3-amino-piperidin-1-yl)-xanthin
   Schmelzpunkt: 226-228°C
   Massenspektrum (ESI⁺): m/z = 405 [M+H]⁺
(5) 1-(2-Phenyl-ethyl)-3-carboxymethyl-7-(3-methyl-2-buten-1-yl)-8-(3-amino-piperidin-1-yl)-xanthin
   Schmelzpunkt: 228-235°C
   Massenspektrum (ESI⁺): m/z = 481 [M+H]⁺

### Beispiel 7

### 1-[2-(3-Amino-phenyl)-ethyl]-3-methyl-7-(3-methyl-2-buten-1-yl)-8-(3-amino-piperidin-1-yl)-xanthin

hergestellt durch Reduktion von 1-[2-(3-Nitro-phenyl)-ethyl]-3-methyl-7-(3-methyl-2-buten-1-yl)-8-(3-amino-piperidin-1-yl)-xanthin mit Eisen in einem Gemisch aus Ethanol, Wasser und Eisessig (10:5:1).
R_{f}-Wert: 0.45 (Kieselgel, Methylenchlorid/Methanol/konz. wässriges Ammoniak = 9:1:0.1)
Massenspektrum (ESI⁺): m/z = 452 [M+H]⁺

Analog Beispiel 7 werden folgende Verbindungen erhalten:
(1) 1-[2-(2-Amino-phenyl)-ethyl]-3-methyl-7-(3-methyl-2-buten-1-yl)-8-(3-amino-piperidin-1-yl)-xanthin
   R_{f}-Wert: 0.20 (Kieselgel, Methylenchlorid/Methanol/konz. wässriges Ammoniak = 9:1:0.1)
   Massenspektrum (ESI⁺): m/z = 452 [M+H]⁺
(2) 1,3-Dimethyl-7-(3-amino-benzyl)-8-(3-amino-piperidin-1-yl)-xanthin R_{f}-Wert: 0.20 (Kieselgel, Methylenchlorid/Methanol/konz. wässriges Ammoniak = 90:10:1)
   Massenspektrum (ESI⁺): m/z = 384 [M+H]⁺
(3) 1,3-Dimethyl-7-(2-amino-benzyl)-8-(3-amino-piperidin-1-yl)-xanthin
   Massenspektrum (ESI⁺): m/z = 384 [M+H]⁺

### Beispiel 8

### 1,3-Dimethyl-7-(3-methyl-2-buten-1-yl)-8-(1-amino-piperidin-4-yl)-xanthin

hergestellt durch Behandeln von 1,3-Dimethyl-7-(3-methyl-2-buten-1-yl)-8-(1-nitroso-piperidin-4-yl)-xanthin mit Zink in einem Gemisch aus Essigsäure und Wasser (1:1.5) bei 80°C
Massenspektrum (ESI⁺): m/z = 347 [M+H]⁺

Analog Beispiel 8 werden folgende Verbindungen erhalten:
(1) 1,3-Dimethyl-7-(3-methyl-2-buten-1-yl)-8-(1-amino-piperidin-3-yl)-xanthin
   Massenspektrum (ESI⁺): m/z = 347 [M+H]⁺

### Beispiel 9

### 1-(2-Hydroxyimino-2-phenyl-ethyl)-3-methyl-7-(3-methyl-2-buten-1-yl)-8-((R)-3-amino-Piperidin-1-yl)-xanthin

hergestellt durch Umsetzung von 1-(2-Phenyl-2-oxo-ethyl)-3-methyl-7-(3-methyl-2-buten-1-yl)-8-((*R*)-3-amino-piperidin-1-yl)-xanthin mit Hydroxylamin-hydrochlorid in Gegenwart von Kaliumcarbonat in Ethanol bei 85°C.
R_{f}-Wert: 0.54 (Reversed Phase DC-Fertigplatte (E. Merck), Acetonitril/Wasser/ Trifluoressigsäure = 10:10:0.2)
Massenspektrum (ESI⁺): m/z = 466 [M+H]⁺

### Beispiel 10

### 1-[2-(2-Methansulfonylamino-phenyl)-2-oxo-ethyl]-3-methyl-7-(3-methyl-2-buten-1-yl)-8-(3-amino-piperidin-1-yl)-xanthin

hergestellt durch Behandeln von 1-(2-{2-[Bis(methansulfonyl)-amino]-phenyl}-2-oxo-ethyl)-3-methyl-7-(3-methyl-2-buten-1-yl)-8-(3-amino-piperidin-1-yl)-xanthin mit 5 N Natronlauge in Tetrahydrofuran bei Raumtemperatur.
Massenspektrum (ESI⁺): m/z = 544 [M+H]⁺

Analog den vorstehenden Beispielen und anderen literaturbekannten Verfahren können auch die folgenden Verbindungen erhalten werden:
(1) 7-(3-Methyl-2-buten-1-yl)-8-(3-amino-piperidin-1-yl)-xanthin
(2) 1-Methyl-7-(3-methyl-2-buten-1-yl)-8-(3-amino-piperidin-1-yl)-xanthin
(3) 3-Methyl-7-(3-methyl-2-buten-1-yl)-8-(3-amino-piperidin-1-yl)-xanthin
(4) 1-Ethyl-3-methyl-7-(3-methyl-2-buten-1-yl)-8-(3-amino-piperidin-1-yl)-xanthin
(5) 1-Propyl-3-methyl-7-(3-methyl-2-buten-1-yl)-8-(3-amino-piperidin-1-yl)-xanthin
(6) 1-(2-Propyl)-3-methyl-7-(3-methyl-2-buten-1-yl)-8-(3-amino-piperidin-1-yl)-xanthin
(7) 1-Butyl-3-methyl-7-(3-methyl-2-buten-1-yl)-8-(3-amino-piperidin-1-yl)-xanthin
(8) 1-(2-Butyl)-3-methyl-7-(3-methyl-2-buten-1-yl)-8-(3-amino-piperidin-1-yl)-xanthin
(9) 1-(2-Methylpropyl)-3-methyl-7-(3-methyl-2-buten-1-yl)-8-(3-amino-piperidin-1-yl)-xanthin
(10) 1-(2-Propen-1-yl)-3-methyl-7-(3-methyl-2-buten-1-yl)-8-(3-amino-piperidin-1-yl)-xanthin
(11) 1-(2-Propin-1-yl)-3-methyl-7-(3-methyl-2-buten-1-yl)-8-(3-amino-piperidin-1-yl)-xanthin
(12) 1-Cyclopropylmethyl-3-methyl-7-(3-methyl-2-buten-1-yl)-8-(3-amino-piperidin-1-yl)-xanthin
(13) 1-Benzyl-3-methyl-7-(3-methyl-2-buten-1-yl)-8-(3-amino-piperidin-1-yl)-xanthin
(14) 1-(2-Phenylethyl)-3-methyl-7-(3-methyl-2-buten-1-yl)-8-(3-amino-piperidin-1-yl)-xanthin
(15) 1-(2-Hydroxyethyl)-3-methyl-7-(3-methyl-2-buten-1-yl)-8-(3-amino-piperidin-1-yl)-xanthin
(16) 1-(2-Methoxyethyl)-3-methyl-7-(3-methyl-2-buten-1-yl)-8-(3-amino-piperidin-1-yl)-xanthin
(17) 1-(2-Ethoxyethyl)-3-methyl-7-(3-methyl-2-buten-1-yl)-8-(3-amino-piperidin-1-yl)-xanthin
(18) 1-[2-(Dimethylamino)ethyl]-3-methyl-7-(3-methyl-2-buten-1-yl)-8-(3-amino-piperidin-1-yl)-xanthin
(19) 1-[2-(Diethylamino)ethyl]-3-methyl-7-(3-methyl-2-buten-1-yl)-8-(3-amino-piperidin-1-yl)-xanthin
(20) 1-[2-(Pyrrolidin-1-yl)ethyl]-3-methyl-7-(3-methyl-2-buten-1-yl)-8-(3-amino-piperidin-1-yl)-xanthin
(21) 1-[2-(Piperidin-1-yl)ethyl]-3-methyl-7-(3-methyl-2-buten-1-yl)-8-(3-amino-piperidin-1-yl)-xanthin
(22) 1-[2-(Morpholin-4-yl)ethyl]-3-methyl-7-(3-methyl-2-buten-1-yl)-8-(3-amino-piperidin-1-yl)-xanthin
(23) 1-[2-(Piperazin-1-yl)ethyl]-3-methyl-7-(3-methyl-2-buten-1-yl)-8-(3-amino-piperidin-1-yl)-xanthin
(24) 1-[2-(4-Methyl-piperazin-1-yl)ethyl]-3-methyl-7-(3-methyl-2-buten-1-yl)-8-(3-amino-piperidin-1-yl)-xanthin
(25) 1-(3-Hydroxypropyl)-3-methyl-7-(3-methyl-2-buten-1-yl)-8-(3-amino-piperidin-1-yl)-xanthin
(26) 1-(3-Methoxypropyl)-3-methyl-7-(3-methyl-2-buten-1-yl)-8-(3-amino-piperidin-1-yl)-xanthin
(27) 1-(3-Ethoxypropyl)-3-methyl-7-(3-methyl-2-buten-1-yl)-8-(3-amino-piperidin-1-yl)-xanthin
(28) 1-[3-(Dimethylamino)propyl]-3-methyl-7-(3-methyl-2-buten-1-ylr8-(3-amino-piperidin-1-yl)-xanthin
(29) 1-[3-(Diethylamino)propyl]-3-methyl-7-(3-methyl-2-buten-1-yl)-8-(3-amino-piperidin-1-yl)-xanthin
(30) 1-[3-(Pyrrolidin-1-yl)propyl]-3-methyl-7-(3-methyl-2-buten-1-yl)-8-(3-amino-piperidin-1-yl)-xanthin
(31) 1-[3-(Piperidin-1-yl)propyl]-3-methyl-7-(3-methyl-2-buten-1-yl)-8-(3-amino-piperidin-1-yl)-xanthin
(32) 1-[3-(Morpholin-4-yl)propyl]-3-methyl-7-(3-methyl-2-buten-1-yl)-8-(3-amino-piperidin-1-yl)-xanthin
(33) 1-[3-(Piperazin-1-yl)propyl]-3-methyl-7-(3-methyl-2-buten-1-yl)-8-(3-amino-piperidin-1-yl)-xanthin

(34) 1-[3-(4-Methyl-piperazin-1-yl)propyl]-3-methyl-7-(3-methyl-2-buten-1-yl)-8-(3-amino-piperidin-1-yl)-xanthin
(35) 1-(Carboxymethyl)-3-methyl-7-(3-methyl-2-buten-1-yl)-8-(3-amino-piperidin-1-yl)-xanthin
(36) 1-(Methoxycarbonylmethyl)-3-methyl-7-(3-methyl-2-buten-1-yl)-8-(3-amino-piperidin-1-yl)-xanthin
(37) 1-(Ethoxycarbonylmethyl)-3-methyl-7-(3-methyl-2-buten-1-yl)-8-(3-amino-piperidin-1-yl)-xanthin
(38) 1-(2-Carboxyethyl)-3-methyl-7-(3-methyl-2-buten-1-yl)-8-(3-amino-piperidin-1-yl)-xanthin
(39) 1-[2-(Methoxycarbonyl)ethyl]-3-methyl-7-(3-methyl-2-buten-1-yl)-8-(3-amino-piperidin-1-yl)-xanthin
(40) 1-[2-(Ethoxycarbonyl)ethyl]-3-methyl-7-(3-methyl-2-buten-1-yl)-8-(3-amino-piperidin-1-yl)-xanthin
(41) 1-(Aminocarbonylmethyl)-3-methyl-7-(3-methyl-2-buten-1-yl)-8-(3-amino-piperidin-1-yl)-xanthin
(42) 1-(Methylaminocarbonylmethyl)-3-methyl-7-(3-methyl-2-buten-1-yl)-8-(3-amino-piperidin-1-yl)-xanthin
(43) 1-(Dimethylaminocarbonylmethyl)-3-methyl-7-(3-methyl-2-buten-1-yl)-8-(3-amino-piperidin-1-yl)-xanthin
(44) 1-(Pyrrolidin-1-yl-carbonylmethyl)-3-methyl-7-(3-methyl-2-buten-1-yl)-8-(3-amino-piperidin-1-yl)-xanthin
(45) 1-(Piperidin-1-yl-carbonylmethyl)-3-methyl-7-(3-methyl-2-buten-1-yl)-8-(3-amino-piperidin-1-yl)-xanthin
(46) 1-(Morpholin-4-yl-carbonylmethyl)-3-methyl-7-(3-methyl-2-buten-1-yl)-8-(3-amino-piperidin-1-yl)-xanthin
(47) 1-(Cyanmethyl)-3-methyl-7-(3-methyl-2-buten-1-yl)-8-(3-amino-piperidin-1-yl)-xanthin
(48) 1-(2-Cyanethyl)-3-methyl-7-(3-methyl-2-buten-1-yl)-8-(3-amino-piperidin-1-yl)-xanthin
(49) 1-Methyl-3-ethyl-7-(3-methyl-2-buten-1-yl)-8-(3-amino-piperidin-1-yl)-xanthin
(50) 1-Methyl-3-propyl-7-(3-methyl-2-buten-1-yl)-8-(3-amino-piperidin-1-yl)-xanthin
(51) 1-Methyl-3-(2-propyl)-7-(3-methyl-2-buten-1-yl)-8-(3-amino-piperidin-1-yl)-xanthin
(52) 1-Methyl-3-butyl-7-(3-methyl-2-buten-1-yl)-8-(3-amino-piperidin-1-yl)-xanthin
(53) 1-Methyl-3-(2-butyl)-7-(3-methyl-2-buten-1-yl)-8-(3-amino-piperidin-1-yl)-xanthin
(54) 1-Methyl-3-(2-methylpropyl)-7-(3-methyl-2-buten-1-yl)-8-(3-amino-piperidin-1-yl)-xanthin
(55) 1-Methyl-3-(2-propen-1-yl)-7-(3-methyl-2-buten-1-yl)-8-(3-amino-piperidin-1-yl)-xanthin
(56) 1-Methyl-3-(2-propin-1-yl)-7-(3-methyl-2-buten-1-yl)-8-(3-amino-piperidin-1-yl)-xanthin
(57) 1-Methyl-3-cyclopropylmethyl-7-(3-methyl-2-buten-1-yl)-8-(3-amino-piperidin-1-yl)-xanthin
(58) 1-Methyl-3-benzyl-7-(3-methyl-2-buten-1-yl)-8-(3-amino-piperidin-1-yl)-xanthin
(59) 1-Methyl-3-(2-phenylethyl)-7-(3-methyl-2-buten-1-yl)-8-(3-amino-piperidin-1-yl)-xanthin
(60) 1-Methyl-3-(2-hydroxyethyl)-7-(3-methyl-2-buten-1-yl)-8-(3-amino-piperidin-1-yl)-xanthin
(61) 1-Methyl-3-(2-methoxyethyl)-7-(3-methyl-2-buten-1-yl)-8-(3-amino-piperidin-1-yl)-xanthin
(62) 1-Methyl-3-(2-ethoxyethyl)-7-(3-methyl-2-buten-1-yl)-8-(3-amino-piperidin-1-yl)-xanthin
(63) 1-Methyl-3-[2-(dimethylamino)ethyl]-7-(3-methyl-2-buten-1-yl)-8-(3-amino-piperidin-1-yl)-xanthin
(64) 1-Methyl-3-[2-(diethylamino)ethyl]-7-(3-methyl-2-buten-1-yl)-8-(3-amino-piperidin-1-yl)-xanthin
(65) 1-Methyl-3-[2-(pyrrolidin-1-yl)ethyl]-7-(3-methyl-2-buten-1-yl)-8-(3-amino-piperidin-1-yl)-xanthin
(66) 1-Methyl-3-[2-(piperidin-1-yl)ethyl]-7-(3-methyl-2-buten-1-yl)-8-(3-amino-piperidin-1-yl)-xanthin
(67) 1-Methyl-3-[2-(morpholin-4-yl)ethyl]-7-(3-methyl-2-buten-1-yl)-8-(3-amino-piperidin-1-yl)-xanthin
(68) 1-Methyl-3-[2-(piperazin-1-yl)ethyl]-7-(3-methyl-2-buten-1-yl)-8-(3-amino-piperidin-1-yl)-xanthin
(69) 1-Methyl-3-[2-(4-methyl-piperazin-1-yl)ethyl]-7-(3-methyl-2-buten-1-yl)-8-(3-amino-piperidin-1-yl)-xanthin

(70) 1-Methyl-3-(3-hydroxypropyl)-7-(3-methyl-2-buten-1-yl)-8-(3-amino-piperidin-1-yl)-xanthin
(71) 1-Methyl-3-(3-methoxypropyl)-7-(3-methyl-2-buten-1-yl)-8-(3-amino-piperidin-1-yl)-xanthin
(72) 1-Methyl-3-(3-ethoxypropyl)-7-(3-methyl-2-buten-1-yl)-8-(3-amino-piperidin-1-yl)-xanthin
(73) 1-Methyl-3-[3-(dimethylamino)propyl]-7-(3-methyl-2-buten-1-yl)-8-(3-amino-piperidin-1-yl)-xanthin
(74) 1-Methyl-3-[3-(diethylamino)propyl]-7-(3-methyl-2-buten-1-yl)-8-(3-amino-piperidin-1-yl)-xanthin
(75) 1-Methyl-3-[3-(pyrrolidin-1-yl)propyl]-7-(3-methyl-2-buten-1-yl)-8-(3-amino-piperidin-1-yl)-xanthin
(76) 1-Methyl-3-[3-(piperidin-1-yl)propyl]-7-(3-methyl-2-buten-1-yl)-8-(3-amino-piperidin-1-yl)-xanthin
(77) 1-Methyl-3-[3-(morpholin-4-yl)propyl]-7-(3-methyl-2-buten-1-yl)-8-(3-amino-piperidin-1-yl)-xanthin
(78) 1-Methyl-3-[3-(piperazin-1-yl)propyl]-7-(3-methyl-2-buten-1-yl)-8-(3-amino-piperidin-1-yl)-xanthin
(79) 1-Methyl-3-[3-(4-methyl-piperazin-1-yl)propyl]-7-(3-methyl-2-buten-1-yl)-8-(3-amino-piperidin-1-yl)-xanthin
(80) 1-Methyl-3-(carboxymethyl)-7-(3-methyl-2-buten-1-yl)-8-(3-amino-piperidin-1-yl)-xanthin
(81) 1-Methyl-3-(methoxycarbonylmethyl)-7-(3-methyl-2-buten-1-yl)-8-(3-amino-piperidin-1-yl)-xanthin
(82) 1-Methyl-3-(ethoxycarbonylmethyl)-7-(3-methyl-2-buten-1-yl)-8-(3-amino-piperidin-1-yl)-xanthin
(83) 1-Methyl-3-(2-carboxyethyl)-7-(3-methyl-2-buten-1-yl)-8-(3-amino-piperidin-1-yl)-xanthin
(84) 1-Methyl-3-[2-(methoxycarbonyl)ethyl]-7-(3-methyl-2-buten-1-yl)-8-(3-amino-piperidin-1-yl)-xanthin
(85) 1-Methyl-3-[2-(ethoxycarbonyl)ethyl]-7-(3-methyl-2-buten-1-yl)-8-(3-amino-piperidin-1-yl)-xanthin
(86) 1-Methyl-3-(aminocarbonylmethyl)-7-(3-methyl-2-buten-1-yl)-8-(3-amino-piperidin-1-yl)-xanthin
(87) 1-Methyl-3-(methylaminocarbonylmethyl)-7-(3-methyl-2-buten-1-yl)-8-(3-amino-piperidin-1-yl)-xanthin
(88) 1-Methyl-3-(dimethylaminocarbonylmethyl)-7-(3-methyl-2-buten-1-yl)-8-(3-amino-piperidin-1-yl)-xanthin
(89) 1-Methyl-3-(pyrrolidin-1-yl-carbonylmethyl)-7-(3-methyl-2-buten-1-yl)-8-(3-amino-piperidin-1-yl)-xanthin
(90) 1-Methyl-3-(piperidin-1-yl-carbonylmethyl)-7-(3-methyl-2-buten-1-yl)-8-(3-amino-piperidin-1-yl)-xanthin
(91) 1-Methyl-3-(morpholin-4-yl-carbonylmethyl)-7-(3-methyl-2-buten-1-yl)-8-(3-amino-piperidin-1-yl)-xanthin
(92) 1-Methyl-3-(cyanmethyl)-7-(3-methyl-2-buten-1-yl)-8-(3-amino-piperidin-1-yl)-xanthin
(93) 1-Methyl-3-(2-cyanethyl)-7-(3-methyl-2-buten-1-yl)-8-(3-amino-piperidin-1-yl)-xanthin

(94) 1,3,7-Trimethyl-8-(3-amino-piperidin-1-yl)-xanthin
(95) 1,3-Dimethyl-7-ethyl-8-(3-amino-piperidin-1-yl)-xanthin
(96) 1,3-Dimethyl-7-propyl-8-(3-amino-piperidin-1-yl)-xanthin
(97) 1,3-Dimethyl-7-(2-propyl)-8-(3-amino-piperidin-1-yl)-xanthin
(98) 1,3-Dimethyl-7-butyl-8-(3-amino-piperidin-1-yl)-xanthin
(99) 1,3-Dimethyl-7-(2-butyl)-8-(3-amino-piperidin-1-yl)-xanthin
(100) 1,3-Dimethyl-7-(2-methylpropyl)-8-(3-amino-piperidin-1-yl)-xanthin
(101) 1,3-Dimethyl-7-pentyl-8-(3-amino-piperidin-1-yl)-xanthin
(102) 1,3-Dimethyl-7-(2-methylbutyl)-8-(3-amino-piperidin-1-yl)-xanthin
(103) 1,3-Dimethyl-7-(3-methylbutyl)-8-(3-amino-piperidin-1-yl)-xanthin
(104) 1,3-Dimethyl-7-(2,2-dimethylpropyl)-8-(3-amino-piperidin-1-yl)-xanthin
(105) 1,3-Dimethyl-7-cyclopropylmethyl-8-(3-amino-piperidin-1-yl)-xanthin
(106) 1,3-Dimethyl-7-[(1-methylcyclopropyl)methyl]-8-(3-amino-piperidin-1-yl)-xanthin
(107) 1,3-Dimethyl-7-[(2-methylcyclopropyl)methyl]-8-(3-amino-piperidin-1-yl)-xanthin
(108) 1,3-Dimethyl-7-cyclobutylmethyl-8-(3-amino-piperidin-1-yl)-xanthin
(109) 1,3-Dimethyl-7-cyclopentylmethyl-8-(3-amino-piperidin-1-yl)-xanthin
(110) 1,3-Dimethyl-7-cyclohexylmethyl-8-(3-amino-piperidin-1-yl)-xanthin
(111) 1,3-Dimethyl-7-[2-(cyclopropyl)ethyl]-8-(3-amino-piperidin-1-yl)-xanthin
(112) 1,3-Dimethyl-7-(2-propen-1-yl)-8-(3-amino-piperidin-1-yl)-xanthin
(113) 1,3-Dimethyl-7-(2-methyl-2-propen-1-yl)-8-(3-amino-piperidin-1-yl)-xanthin
(114) 1,3-Dimethyl-7-(3-phenyl-2-propen-1-yl)-8-(3-amino-piperidin-1-yl)-xanthin
(115) 1,3-Dimethyl-7-(2-buten-1-yl)-8-(3-amino-piperidin-1-yl)-xanthin
(116) 1,3-Dimethyl-7-(4,4,4-trifluor-2-buten-1-yl)-8-(3-amino-piperidin-1-yl)-xanthin
(117) 1,3-Dimethyl-7-(3-buten-1-yl)-8-(3-amino-piperidin-1-yl)-xanthin
(118) 1,3-Dimethyl-7-(2-chlor-2-buten-1-yl)-8-(3-amino-piperidin-1-yl)-xanthin
(119) 1,3-Dimethyl-7-(2-brom-2-buten-1-yl)-8-(3-amino-piperidin-1-yl)-xanthin
(120) 1,3-Dimethyl-7-(3-chlor-2-buten-1-yl)-8-(3-amino-piperidin-1-yl)-xanthin
(121) 1,3-Dimethyl-7-(3-brom-2-buten-1-yl)-8-(3-amino-piperidin-1-yl)-xanthin
(122) 1,3-Dimethyl-7-(2-methyl-2-buten-1-yl)-8-(3-amino-piperidin-1-yl)-xanthin
(123) 1,3-Dimethyl-7-(2,3-dimethyl-2-buten-1-yl)-8-(3-amino-piperidin-1-yl)-xanthin
(124) 1,3-Dimethyl-7-(3-trifluormethyl-2-buten-1-yl)-8-(3-amino-piperidin-1-yl)-xanthin
(125) 1,3-Dimethyl-7-(3-methyl-3-buten-1-yl)-8-(3-amino-piperidin-1-yl)-xanthin
(126) 1,3-Dimethyl-7-[(2-methyl-1-cyclopenten-1-yl)methyl]-8-(3-amino-piperidin-1-yl)-xanthin
(127) 1,3-Dimethyl-7-(1-cyclohexen-1-yl-methyl)-8-(3-amino-piperidin-1-yl)-xanthin
(128) 1,3-Dimethyl-7-[2-(1-cyclopenten-1-yl)ethyl]-8-(3-amino-piperidin-1-yl)-xanthin
(129) 1,3-Dimethyl-7-(2-propin-1-yl)-8-(3-amino-piperidin-1-yl)-xanthin
(130) 1,3-Dimethyl-7-(3-butin-1-yl)-8-(3-amino-piperidin-1-yl)-xanthin
(131) 1,3-Dimethyl-7-(4-fluorbenzyl)-8-(3-amino-piperidin-1-yl)-xanthin

(132) 1,3-Dimethyl-7-(2-chlorbenzyl)-8-(3-amino-piperidin-1-yl)-xanthin
(133) 1,3-Dimethyl-7-(3-chlorbenzyl)-8-(3-amino-piperidin-1-yl)-xanthin
(134) 1,3-Dimethyl-7-(4-chlorbenzyl)-8-(3-amino-piperidin-1-yl)-xanthin
(135) 1,3-Dimethyl-7-(2-brombenzyl)-8-(3-amino-piperidin-1-yl)-xanthin
(136) 1,3-Dimethyl-7-(3-brombenzyl)-8-(3-amino-piperidin-1-yl)-xanthin
(137) 1,3-Dimethyl-7-(4-brombenzyl)-8-(3-amino-piperidin-1-yl)-xanthin
(138) 1,3-Dimethyl-7-(2-methylbenzyl)-8-(3-amino-piperidin-1-yl)-xanthin
(139) 1,3-Dimethyl-7-(3-methylbenzyl)-8-(3-amino-piperidin-1-yl)-xanthin
(140) 1,3-Dimethyl-7-(4-methylbenzyl)-8-(3-amino-piperidin-1-yl)-xanthin
(141) 1,3-Dimethyl-7-(2-methoxybenzyl)-8-(3-amino-piperidin-1-yl)-xanthin
(142) 1,3-Dimethyl-7-(3-methoxybenzyl)-8-(3-amino-piperidin-1-yl)-xanthin
(143) 1,3-Dimethyl-7-(4-methoxybenzyl)-8-(3-amino-piperidin-1-yl)-xanthin
(144) 1,3-Dimethyl-7-(2-phenylethyl)-8-(3-amino-piperidin-1-yl)-xanthin
(145) 1,3-Dimethyl-7-(3-phenylpropyl)-8-(3-amino-piperidin-1-yl)-xanthin
(146) 1,3-Dimethyl-7-(2-furanylmethyl)-8-(3-amino-piperidin-1-yl)-xanthin
(147) 1,3-Dimethyl-7-(3-furanylmethyl)-8-(3-amino-piperidin-1-yl)-xanthin
(148) 1,3-Dimethyl-7-(3-thienylmethyl)-8-(3-amino-piperidin-1-yl)-xanthin
(149) 1,3-Dimethyl-7-(3-methyl-2-buten-1-yl)-8-(3-methylamino-piperidin-1-yl)-xanthin
(150) 1,3-Dimethyl-7-(3-methyl-2-buten-1-yl)-8-(3-ethylamino-piperidin-1-yl)-xanthin
(151) 1,3-Dimethyl-7-(3-methyl-2-buten-1-yl)-8-(3-dimethylamino-piperidin-1-yl)-xanthin
(152) 1,3-Dimethyl-7-(3-methyl-2-buten-1-yl)-8-(3-diethylamino-piperidin-1-yl)-xanthin
(153) 1,3-Dimethyl-7-(3-methyl-2-buten-1-yl)-8-{3-[(2-hydroxyethyl)amino]-piperidin-1-yl}-xanthin
(154) 1,3-Dimethyl-7-(3-methyl-2-buten-1-yl)-8-{3-[N-methyl-N-(2-hydroxyethyl)-amino]-piperidin-1-yl}-xanthin
(155) 1,3-Dimethyl-7-(3-methyl-2-buten-1-yl)-8-{3-[(3-hydroxypropyl)amino]-piperidin-1-yl}-xanthin
(156) 1,3-Dimethyl-7-(3-methyl-2-buten-1-yl)-8-{3-[N-methyl-N-(3-hydroxypropyl)-amino]-piperidin-1-yl}-xanthin
(157) 1,3-Dimethyl-7-(3-methyl-2-buten-1-yl)-8-{3-[(carboxymethyl)amino]-piperidin-1-yl}-xanthin
(158) 1,3-Dimethyl-7-(3-methyl-2-buten-1-yl)-8-{3-[(methoxycarbonylmethyl)amino]-piperidin-1-yl}-xanthin
(159) 1,3-Dimethyl-7-(3-methyl-2-buten-1-yl)-8-{3-[(ethoxycarbonylmethyl)amino]-piperidin-1-yl}-xanthin
(160) 1,3-Dimethyl-7-(3-methyl-2-buten-1-yl)-8-{3-[N-methyl-N-(methoxycarbonyl-methyl)-amino]-piperidin-1-yl}-xanthin
(161) 1,3-Dimethyl-7-(3-methyl-2-buten-1-yl)-8-{3-[N-methyl-N-(ethoxycarbonyl-methyl)-amino]-piperidin-1-yl}-xanthin
(162) 1,3-Dimethyl-7-(3-methyl-2-buten-1-yl)-8-{3-[(2-carboxyethyl)amino]-piperidin-1-yl}-xanthin
(163) 1,3-Dimethyl-7-(3-methyl-2-buten-1-yl)-8-(3-{[2-(methoxycarbonyl)ethyl]-amino}-piperidin-1-yl)-xanthin
(164) 1,3-Dimethyl-7-(3-methyl-2-buten-1-yl)-8-(3-{[2-(ethoxycarbonyl)ethyl]amino}-piperidin-1-yl)-xanthin
(165) 1,3-Dimethyl-7-(3-methyl-2-buten-1-yl)-8-(3-{N-methyl-N-[2-(methoxycarbonyl)-ethyl]-amino}-piperidin-1-yl)-xanthin
(166) 1,3-Dimethyl-7-(3-methyl-2-buten-1-yl)-8-(3-{N-methyl-N-[2-(ethoxycarbonyl)-ethyl]-amino}-piperidin-1-yl)-xanthin
(167) 1,3-Dimethyl-7-(3-methyl-2-buten-1-yl)-8-{3-[(aminocarbonylmethyl)amino]-piperidin-1-yl}-xanthin
(168) 1,3-Dimethyl-7-(3-methyl-2-buten-1-yl)-8-{3-[(methylaminocarbonylmethyl)-amino]-piperidin-1-yl}-xanthin
(169) 1,3-Dimethyl-7-(3-methyl-2-buten-1-yl)-8-{3-[(dimethylaminocarbonylmethyl)-amino]-piperidin-1-yl}-xanthin

(170) 1,3-Dimethyl-7-(3-methyl-2-buten-1-yl)-8-{3-[(ethylaminocarbonylmethyl)-amino]-piperidin-1-yl}-xanthin
(171) 1,3-Dimethyl-7-(3-methyl-2-buten-1-yl)-8-{3-[(diethylaminocarbonylmethyl)-amino]-piperidin-1-yl}-xanthin
(172) 1,3-Dimethyl-7-(3-methyl-2-buten-1-yl)-8-{3-[(pyrrolidin-1-ylcarbonylmethyl)-amino]-piperidin-1-yl}-xanthin
(173) 1,3-Dimethyl-7-(3-methyl-2-buten-1-yl)-8-{3-[(2-cyanpyrrolidin-1-ylcarbonyl-methyl)amino]-piperidin-1-yl}-xanthin
(174) 1,3-Dimethyl-7-(3-methyl-2-buten-1-yl)-8-{3-[(4-cyanthiazolidin-3-ylcarbonyl-methyl)amino]-piperidin-1-yl}-xanthin
(175) 1,3-Dimethyl-7-(3-methyl-2-buten-1-yl)-8-{3-[(2-aminocarbonylpyrrolidin-1-yl-carbonylmethyl)amino]-piperidin-1-yl}-xanthin
(176) 1,3-Dimethyl-7-(3-methyl-2-buten-1-yl)-8-{3-[(2-carboxypyrrolidin-1-ylcarbonyl-methyl)amino]-piperidin-1-yl}-xanthin
(177) 1,3-Dimethyl-7-(3-methyl-2-buten-1-yl)-8-{3-[(2-methoxycarbonylpyrrolidin-1-ylcarbonylmethyl)amino]-piperidin-1-yl}-xanthin
(178) 1,3-Dimethyl-7-(3-methyl-2-buten-1-yl)-8-{3-[(piperidin-1-ylcarbonylmethyl)-amino]-piperidin-1-yl}-xanthin
(179) 1,3-Dimethyl-7-(3-methyl-2-buten-1-yl)-8-{3-[(morpholin-4-ylcarbonylmethyl)-amino]-piperidin-1-yl}-xanthin
(180) 1,3-Dimethyl-7-(3-methyl-2-buten-1-yl)-8-(2-methyl-3-amino-piperidin-1-yl)-xanthin
(181) 1,3-Dimethyl-7-(3-methyl-2-buten-1-ylr8-(3-methyl-3-amino-piperidin-1-yl)-xanthin
(182) 1,3-Dimethyl-7-(3-methyl-2-buten-1-yl)-8-(4-methyl-3-amino-piperidin-1-yl)-xanthin
(183) 1,3-Dimethyl-7-(3-methyl-2-buten-1-yl)-8-(5-methyl-3-amino-piperidin-1-yl)-xanthin
(184) 1,3-Dimethyl-7-(3-methyl-2-buten-1-yl)-8-(6-methyl-3-amino-piperidin-1-yl)-xanthin
(185) 1,3-Dimethyl-7-(3-methyl-2-buten-1-yl)-8-(2-amino-8-aza-bicyclo[3.2.1]oct-8-yl)-xanthin
(186) 1,3-Dimethyl-7-(3-methyl-2-buten-1-yl)-8-(6-amino-2-aza-bicyclo[2.2.2]oct-2-yl)-xanthin
(187) 1,3-Dimethyl-7-(3-methyl-2-buten-1-yl)-8-(3-amino-cyclopentyl)-xanthin
(188) 1,3-Dimethyl-7-(3-methyl-2-buten-1-yl)-8-(3-methylamino-cyclohexyl)-xanthin
(189) 1,3-Dimethyl-7-(3-methyl-2-buten-1-yl)-8-(3-ethylamino-cyclohexyl)-xanthin
(190) 1,3-Dimethyl-7-(3-methyl-2-buten-1-yl)-8-(3-dimethylamino-cyclohexyl)-xanthin
(191) 1,3-Dimethyl-7-(3-methyl-2-buten-1-yl)-8-(3-diethylamino-cyclohexyl)-xanthin
(192) 1,3-Dimethyl-7-(3-methyl-2-buten-1-yl)-8-(4-amino-cyclohexyl)-xanthin

(193) 1,3-Dimethyl-7-(3-methyl-2-buten-1-yl)-8-[(3-amino-cyclohexyl)amino]-xanthin
(194) 1,3-Dimethyl-7-(3-methyl-2-buten-1-yl)-8-[(2-amino-cyclopentyl)amino]-xanthin
(195) 1,3-Dimethyl-7-(3-methyl-2-buten-1-yl)-8-[(3-amino-cyclopentyl)amino]-xanthin
(196) 1,3-Dimethyl-7-(3-methyl-2-buten-1-yl)-8-[(2-amino-cyclobutyl)amino]-xanthin
(197) 1,3-Dimethyl-7-(3-methyl-2-buten-1-yl)-8-[(3-amino-cyclobutyl)amino]-xanthin
(198) 1,3-Dimethyl-7-(3-methyl-2-buten-1-yl)-8-[(2-amino-cyclopropyl)amino]-xanthin
(199) 1-[2-(4-Hydroxy-phenyl)-ethyl]-3-methyl-7-(3-methyl-2-buten-1-yl)-8-(3-amino-piperidin-1-yl)-xanthin
(200) 1-[2-(3-Fluor-4-hydroxy-phenyl)-ethyl]-3-methyl-7-(3-methyl-2-buten-1-yl)-8-(3-amino-piperidin-1-yl)-xanthin
(201) 1-[2-(4-Methoxy-phenyl)-ethyl]-3-methyl-7-(3-methyl-2-buten-1-yl)-8-(3-amino-piperidin-1-yl)-xanthin
(202) 1-[2-(4-Ethoxy-phenyl)-ethyl]-3-methyl-7-(3-methyl-2-buten-1-yl)-8-(3-amino-piperidin-1-yl)-xanthin
(203) 1-(2-{4-[(Carboxymethyl)oxy]-phenyl}-ethyl)-3-methyl-7-(3-methyl-2-buten-1-yl)-8-(3-amino-piperidin-1-yl)-xanthin
(204) 1-(2-{4-[(Methoxycarbonyl)methyloxy]-phenyl}-ethyl)-3-methyl-7-(3-methyl-2-buten-1-yl)-8-(3-amino-piperidin-1-yl)-xanthin
(205) 1-[2-(3-Hydroxy-phenyl)-ethyl]-3-methyl-7-(3-methyl-2-buten-1-yl)-8-(3-amino-piperidin-1-yl)-xanthin
(206) 1-[2-(2-Fluor-5-hydroxy-phenyl)-ethyl]-3-methyl-7-(3-methyl-2-buten-1-yl)-8-(3-amino-piperidin-1-yl)-xanthin
(207) 1-[2-(3-Methoxy-phenyl)-ethyl]-3-methyl-7-(3-methyl-2-buten-1-yl)-8-(3-amino-piperidin-1-yl)-xanthin
(208) 1-{2-[3-(Carboxymethyloxy)-phenyl]-ethyl}-3-methyl-7-(3-methyl-2-buten-1-yl)-8-(3-amino-piperidin-1-yl)-xanthin
(209) 1-(2-{3-[(Ethoxycarbony)methyloxy]-phenyl}-ethyl)-3-methyl-7-(3-methyl-2-buten-1-yl)-8-(3-amino-piperidin-1-yl)-xanthin
(210) 1-[2-(2-Hydroxy-phenyl)-ethyl]-3-methyl-7-(3-methyl-2-buten-1-yl)-8-(3-amino-piperidin-1-yl)-xanthin
(211) 1-[2-(2-Methoxy-phenyl)-ethyl]-3-methyl-7-(3-methyl-2-buten-1-yl)-8-(3-amino-piperidin-1-yl)-xanthin
(212) 1-{2-[2-(Carboxymethyloxy)-phenyl]-ethyl}-3-methyl-7-(3-methyl-2-buten-1-yl)-8-(3-amino-piperidin-1-yl)-xanthin
(213) 1-(2-{2-[(Methoxycarbonyl)methyloxy]-phenyl}-ethyl)-3-methyl-7-(3-methyl-2-buten-1-yl)-8-(3-amino-piperidin-1-yl)-xanthin
(214) 1-[2-(4-Methyl-phenyl)-ethyl]-3-methyl-7-(3-methyl-2-buten-1-yl)-8-(3-amino-piperidin-1-yl)-xanthin
(215) 1-[2-(4-Hydroxymethyl-phenyl)-ethyl]-3-methyl-7-(3-methyl-2-buten-1-yl)-8-(3-amino-piperidin-1-yl)-xanthin

(216) 1-[2-(4-Carboxy-phenyl)-ethyl]-3-methyl-7-(3-methyl-2-buten-1-yl)-8-(3-amino-piperidin-1-yl)-xanthin
(217) 1-{2-[4-(Methoxycarbonyl)-phenyl]-ethyl}-3-methyl-7-(3-methyl-2-buten-1-yl)-8-(3-amino-piperidin-1-yl)-xanthin
(218) 1-{2-[4-(Carboxymethyl)-phenyl]-ethyl}-3-methyl-7-(3-methyl-2-buten-1-yl)-8-(3-amino-piperidin-1-yl)-xanthin
(219) 1-(2-{4-[(Methoxycarbonyl)methyl]-phenyl}-ethyl)-3-methyl-7-(3-methyl-2-buten-1-yl)-8-(3-amino-piperidin-1-yl)-xanthin
(220) 1-{2-[4-(2-Carboxy-ethyl)-phenyl]-ethyl}-3-methyl-7-(3-methyl-2-buten-1-yl)-8-(3-amino-piperidin-1-yl)-xanthin
(221) 1-(2-{4-[2-(Methoxycarbonyl)-ethyl]-phenyl}-ethyl)-3-methyl-7-(3-methyl-2-buten-1-yl)-8-(3-amino-piperidin-1-yl)-xanthin
(222) 1-[2-(3-Methyl-phenyl)-ethyl]-3-methyl-7-(3-methyl-2-buten-1-yl)-8-(3-amino-piperidin-1-yl)-xanthin
(223) 1-[2-(3-Carboxy-phenyl)-ethyl]-3-methyl-7-(3-methyl-2-buten-1-yl)-8-(3-amino-piperidin-1-yl)-xanthin
(224) 1-{2-[3-(Ethoxycarbonyl)-phenyl]-ethyl}-3-methyl-7-(3-methyl-2-buten-1-yl)-8-(3-amino-piperidin-1-yl)-xanthin
(225) 1-{2-[3-(Carboxymethyl)-phenyl]-ethyl}-3-methyl-7-(3-methyl-2-buten-1-yl)-8-(3-amino-piperidin-1-yl)-xanthin
(226) 1-(2-{3-[(Methoxycarbonyl)methyl]-phenyl}-ethyl)-3-methyl-7-(3-methyl-2-buten-1-yl)-8-(3-amino-piperidin-1-yl)-xanthin
(227) 1-{2-[3-(2-Carboxy-ethyl)-phenyl]-ethyl}-3-methyl-7-(3-methyl-2-buten-1-yl)-8-(3-amino-piperidin-1-yl)-xanthin
(228) 1-(2-{3-[2-(Methoxycarbonyl)-ethyl]-phenyl}-ethyl)-3-methyl-7-(3-methyl-2-buten-1-yl)-8-(3-amino-piperidin-1-yl)-xanthin
(229) 1-[2-(2-Methyl-phenyl)-ethyl]-3-methyl-7-(3-methyl-2-buten-1-yl)-8-(3-amino-piperidin-1-yl)-xanthin
(230) 1-[2-(2-Carboxy-phenyl)-ethyl]-3-methyl-7-(3-methyl-2-buten-1-yl)-8-(3-amino-piperidin-1-yl)-xanthin
(231) 1-{2-[2-(Methoxycarbonyl)-phenyl]-ethyl}-3-methyl-7-(3-methyl-2-buten-1-yl)-8-(3-amino-piperidin-1-yl)-xanthin
(232) 1-[2-(4-Fluor-phenyl)-ethyl]-3-methyl-7-(3-methyl-2-buten-1-yl)-8-(3-amino-piperidin-1-yl)-xanthin
(233) 1-[2-(4-Chlor-phenyl)-ethyl]-3-methyl-7-(3-methyl-2-buten-1-yl)-8-(3-amino-piperidin-1-yl)-xanthin
(234) 1-[2-(4-Brom-phenyl)-ethyl]-3-methyl-7-(3-methyl-2-buten-1-yl)-8-(3-amino-piperidin-1-yl)-xanthin
(235) 1-[2-(4-Cyano-phenyl)-ethyl]-3-methyl-7-(3-methyl-2-buten-1-yl)-8-(3-amino-piperidin-1-yl)-xanthin
(236) 1-[2-(4-Trifluormethoxy-phenyl)-ethyl]-3-methyl-7-(3-methyl-2-buten-1-yl)-8-(3-amino-piperidin-1-yl)-xanthin
(237) 1-[2-(4-Methylsulfanyl-phenyl)-ethyl]-3-methyl-7-(3-methyl-2-buten-1-yl)-8-(3-amino-piperidin-1-yl)-xanthin
(238) 1-[2-(4-Methylsulfinyl-phenyl)ethyl]-3-methyl-7-(3-methyl-2-buten-1-yl)-8-(3-amino-piperidin-1-yl)-xanthin
(239) 1-[2-(4-Methylsulfonyl-phenyl)-ethyl]-3-methyl-7-(3-methyl-2-buten-1-yl)-8-(3-amino-piperidin-1-yl)-xanthin
(240) 1-[2-(4-Trifluormethyl-phenyl)-ethyl]-3-methyl-7-(3-methyl-2-buten-1-yl)-8-(3-amino-piperidin-1-yl)-xanthin
(241) 1-[2-(4-Amino-phenyl)-ethyl]-3-methyl-7-(3-methyl-2-buten-1-yl)-8-(3-amino-piperidin-1-yl)-xanthin
(242) 1-(2-{4-[(Methylcarbonyl)amino]-phenyl}-ethyl)-3-methyl-7-(3-methyl-2-buten-1-yl)-8-(3-amino-piperidin-1-yl)-xanthin
(243) 1-(2-{4-[(Methylsulfonyl)amino]-phenyl}-ethyl)-3-methyl-7-(3-methyl-2-buten-1-yl)-8-(3-amino-piperidin-1-yl)-xanthin
(244) 1-[2-(3-Nitro-phenyl)-ethyl]-3-methyl-7-(3-methyl-2-buten-1-yl)-8-(3-amino-piperidin-1-yl)-xanthin
(245) 1-{2-[4-(Aminocarbonyl)-phenyl]-ethyl}-3-methyl-7-(3-methyl-2-buten-1-yl)-8-(3-amino-piperidin-1-yl)-xanthin
(246) 1-{2-[4-(Methylaminocarbonyl)-phenyl]-ethyl}-3-methyl-7-(3-methyl-2-buten-1-yl)-8-(3-amino-piperidin-1-yl)-xanthin

(247) 1-{2-[4-(Dimethylaminocarbonyl)-phenyl]-ethyl}-3-methyl-7-(3-methyl-2-buten-1-yl)-8-(3-amino-piperidin-1-yl)-xanthin
(248) 1-{2-[4-(Aminosulfonyl)-phenyl]-ethyl}-3-methyl-7-(3-methyl-2-buten-1-yl)-8-(3-amino-piperidin-1-yl)-xanthin
(249) 1-{2-[4-(Methylaminosulfonyl)-phenyl]-ethyl}-3-methyl-7-(3-methyl-2-buten-1-yl)-8-(3-amino-piperidin-1-yl)-xanthin
(250) 1-{2-[4-(Dimethylaminosulfonyl)-phenyl]-ethyl}-3-methyl-7-(3-methyl-2-buten-1-yl)-8-(3-amino-piperidin-1-yl)-xanthin
(251) 1-(3-Carboxy-propyl)-3-methyl-7-(3-methyl-2-buten-1-yl)-8-(3-amino-piperidin-1-yl)-xanthin
(252) 1-[3-(Methoxycarbonyl)-propyl]-3-methyl-7-(3-methyl-2-buten-1-yl)-8-(3-amino-piperidin-1-yl)-xanthin
(253) 1-[3-(Ethoxycarbonyl)-propyl]-3-methyl-7-(3-methyl-2-buten-1-yl)-8-(3-amino-piperidin-1-yl)-xanthin
(254) 1-[2-(3,4-Dimethyl-phenyl)-ethyl]-3-methyl-7-(3-methyl-2-buten-1-yl)-8-(3-amino-piperidin-1-yl)-xanthin
(255) 1-[2-(2-Fluor-5-chlor-phenyl)-ethyl]-3-methyl-7-(3-methyl-2-buten-1-yl)-8-(3-amino-piperidin-1-yl)-xanthin
(256) 1-[2-(3,5-Dimethoxy-phenyl)-ethyl]-3-methyl-7-(3-methyl-2-buten-1-yl)-8-(3-amino-piperidin-1-yl)-xanthin
(257) 1-[2-(Naphthalin-2-yl)-ethyl]-3-methyl-7-(3-methyl-2-buten-1-yl)-8-(3-amino-piperidin-1-yl)-xanthin
(258) 1-[2-(Pyridin-3-yl)-ethyl]-3-methyl-7-(3-methyl-2-buten-1-yl)-8-(3-amino-piperidin-1-yl)-xanthin
(259) 1-[4-Phenyl-butyl]-3-methyl-7-(3-methyl-2-buten-1-yl)-8-(3-amino-piperidin-1-yl)-xanthin
(260) 1-Methyl-3-(3-phenyl-propyl)-7-(3-methyl-2-buten-1-yl)-8-(3-amino-piperidin-1-yl)-xanthin
(261) 1-Methyl-3-(3-carboxy-propyl)-7-(3-methyl-2-buten-1-yl)-8-(3-amino-piperidin-1-yl)-xanthin
(262) 1-Methyl-3-[3-(methoxycarbonyl)-propyl]-7-(3-methyl-2-buten-1-yl)-8-(3-amino-piperidin-1-yl)-xanthin
(263) 1-Methyl-3-[3-(ethoxycarbonyl)-propyl]-7-(3-methyl-2-buten-1-yl)-8-(3-amino-piperidin-1-yl)-xanthin
(264) 1,3-Dimethyl-7-(3-methyl-2-buten-1-yl)-8-(3-amino-1-methyl-prop-1-yl)-xanthin
(265) 1,3-Dimethyl-7-(3-methyl-2-buten-1-yl)-8-(3-amino-1,1-dimethyl-prop-1-yl)-xanthin
(266) 1,3-Dimethyl-7-(3-methyl-2-buten-1-yl)-8-(3-amino-1-methyl-but-1-yl)-xanthin
(267) 1,3-Dimethyl-7-(3-methyl-2-buten-1-yl)-8-[1-(2-amino-ethyl)-cyclopropyl]-xanthin
(268) 1,3-Dimethyl-7-(3-methyl-2-buten-1-yl)-8-[1-(aminomethyl)-cyclopentylmethyl]-xanthin
(269) 1,3-Dimethyl-7-(3-methyl-2-buten-1-yl)-8-[2-(aminomethyl)-cyclopropyl]-xanthin
(270) 1,3-Dimethyl-7-(3-methyl-2-buten-1-yl)-8-[2-(aminomethyl)-cyclopentyl]-xanthin
(271) 1,3-Dimethyl-7-(3-methyl-2-buten-1-yl)-8-(2-amino-cyclopropylmethyl)-xanthin
(272) 1,3-Dimethyl-7-(3-methyl-2-buten-1-yl)-8-[(piperidin-3-yl)methyl]-xanthin
(273) 1,3-Dimethyl-7-(3-methyl-2-buten-1-yl)-8-[2-(pyrrolidin-2-yl)-ethyl]-xanthin
(274) 1,3-Dimethyl-7-(3-methyl-2-buten-1-yl)-8-[N-(2-amino-ethyl)-N-ethyl-amino]-xanthin
(275) 1,3-Dimethyl-7-(3-methyl-2-buten-1-yl)-8-[N-(2-amino-ethyl)-N-isopropyl-amino]-xanthin
(276) 1,3-Dimethyl-7-(3-methyl-2-buten-1-yl)-8-[N-(2-amino-ethyl)-N-cyclopropyl-amino]-xanthin
(277) 1,3-Dimethyl-7-(3-methyl-2-buten-1-yl)-8-[N-(2-amino-ethyl)-N-cyclopropylmethyl-amino]-xanthin
(278) 1,3-Dimethyl-7-(3-methyl-2-buten-1-yl)-8-[N-(2-amino-ethyl)-N-phenyl-amino]-xanthin
(279) 1,3-Dimethyl-7-(3-methyl-2-buten-1-yl)-8-[N-(2-amino-ethyl)-N-benzyl-amino]-xanthin
(280) 1,3-Dimethyl-7-(3-methyl-2-buten-1-yl)-8-[N-(2-amino-1-methyl-ethyl)-N-methyl-amino]-xanthin

(281) 1,3-Dimethyl-7-(3-methyl-2-buten-1-yl)-8-[N-(2-amino-prop-1-yl)-N-methyl-amino]-xanthin
(282) 1,3-Dimethyl-7-(3-methyl-2-buten-1-yl)-8-[N-(2-amino-1-methyl-prop-1-yl)-N-methyl-amino]-xanthin
(283) 1,3-Dimethyl-7-(3-methyl-2-buten-1-yl)-8-[N-(2-amino-2-methyl-propyl)-N-methyl-amino]-xanthin
(284) 1,3-Dimethyl-7-(3-methyl-2-buten-1-yl)-8-[N-(1-amino-cyclopropylmethyl)-N-methyl-amino]-xanthin
(285) 1,3-Dimethyl-7-(3-methyl-2-buten-1-yl)-8-[N-(2-amino-cyclopropyl)-N-methyl-amino]-xanthin
(286) 1,3-Dimethyl-7-(3-methyl-2-buten-1-yl)-8-[N-(2-amino-cyclobutyl)-N-methyl-amino]-xanthin
(287) 1,3-Dimethyl-7-(3-methyl-2-buten-1-yl)-8-[N-(2-amino-cyclopentyl)-N-methyl-amino]-xanthin
(288) 1,3-Dimethyl-7-(3-methyl-2-buten-1-yl)-8-[N-(2-amino-cyclohexyl)-N-methyl-amino]-xanthin
(289) 1,3-Dimethyl-7-(3-methyl-2-buten-1-yl)-8-{N-[(pyrrolidin-2-yl)methyl]-N-methyl-amino}-xanthin
(290) 1,3-Dimethyl-7-(3-methyl-2-buten-1-yl)-8-[N-(pyrrolidin-3-yl)-N-methyl-amino]-xanthin
(291) 1,3-Dimethyl-7-(3-methyl-2-buten-1-yl)-8-[N-(piperidin-3-yl)-N-methyl-amino]-xanthin
(292) 1-(2-Phenyloxy-ethyl)-3-methyl-7-(3-methyl-2-buten-1-yl)-8-(3-amino-piperidin-1-yl)-xanthin
(293) 1-(2-Phenylsulfanyl-ethyl)-3-methyl-7-(3-methyl-2-buten-1-yl)-8-(3-amino-piperidin-1-yl)-xanthin
(294) 1-(2-Phenylsulfinyl-ethyl)-3-methyl-7-(3-methyl-2-buten-1-yl)-8-(3-amino-piperidin-1-yl)-xanthin
(295) 1-(2-Phenylsulfonyl-ethyl)-3-methyl-7-(3-methyl-2-buten-1-yl)-8-(3-amino-piperidin-1-yl)-xanthin
(296) 1-Methyl-3-(2-oxo-2-phenyl-ethyl)-7-(3-methyl-2-buten-1-yl)-8-(3-amino-piperidin-1-yl)-xanthin
(297) 1-Methyl-3-(2-oxo-propyl)-7-(3-methyl-2-buten-1-yl)-8-(3-amino-piperidin-1-yl)-xanthin
(298) 1-Methyl-3-phenyl-7-(3-methyl-2-buten-1-yl)-8-(3-amino-piperidin-1-yl)-xanthin
(299) 1-Methyl-3-cyclopropyl-7-(3-methyl-2-buten-1-yl)-8-(3-amino-piperidin-1-yl)-xanthin
(300) 1-[2-(3-Fluor-phenyl)-2-oxo-ethyl]-3-methyl-7-(3-methyl-2-buten-1-yl)-8-(3-amino-piperidin-1-yl)-xanthin
(301) 1-[2-(3-Chlor-phenyl)-2-oxo-ethyl]-3-methyl-7-(3-methyl-2-buten-1-yl)-8-(3-amino-piperidin-1-yl)-xanthin
(302) 1-[2-(3-Brom-phenyl)-2-oxo-ethyl]-3-methyl-7-(3-methyl-2-buten-1-yl)-8-(3-amino-piperidin-1-yl)-xanthin
(303) 1-[2-(3-Methyl-phenyl)-2-oxo-ethyl]-3-methyl-7-(3-methyl-2-buten-1-yl)-8-(3-amino-piperidin-1-yl)-xanthin
(304) 1-[2-(3-Trifluormethyl-phenyl)-2-oxo-ethyl]-3-methyl-7-(3-methyl-2-buten-1-yl)-8-(3-amino-piperidin-1-yl)-xanthin
(305) 1-[2-(2-Methyl-phenyl)-2-oxo-ethyl]-3-methyl-7-(3-methyl-2-buten-1-yl)-8-(3-amino-piperidin-1-yl)-xanthin
(306) 1-[2-(3-Methoxy-phenyl)-2-oxo-ethyl]-3-methyl-7-(3-methyl-2-buten-1-yl)-8-(3-amino-piperidin-1-yl)-xanthin
(307) 1-[2-(3-Difluormethoxy-phenyl)-2-oxo-ethyl]-3-methyl-7-(3-methyl-2-buten-1-yl)-8-(3-amino-piperidin-1-yl)-xanthin
(308) 1-[2-(3-Trifluormethoxy-phenyl)-2-oxo-ethyl]-3-methyl-7-(3-methyl-2-buten-1-yl)-8-(3-amino-piperidin-1-yl)-xanthin
(309) 1-[2-(3-Ethoxy-phenyl)-2-oxo-ethyl]-3-methyl-7-(3-methyl-2-buten-1-yl)-8-(3-amino-piperidin-1-yl)-xanthin
(310) 1-[2-(3-Isopropyloxy-phenyl)-2-oxo-ethyl]-3-methyl-7-(3-methyl-2-buten-1-yl)-8-(3-amino-piperidin-1-yl)-xanthin
(311) 1-[2-(3-Cyclopropyloxy-phenyl)-2-oxo-ethyl]-3-methyl-7-(3-methyl-2-buten-1-yl)-8-(3-amino-piperidin-1-yl)-xanthin
(312) 1-[2-(3-Cyclopentyloxy-phenyl)-2-oxo-ethyl]-3-methyl-7-(3-methyl-2-buten-1-yl)-8-(3-amino-piperidin-1-yl)-xanthin

(313) 1-[2-(3-Cyclopropylmethoxy-phenyl)-2-oxo-ethyl]-3-methyl-7-(3-methyl-2-buten-1-yl)-8-(3-amino-piperidin-1-yl)-xanthin
(314) 1-{2-[3-(2,2,2-Trifluorethoxy)-phenyl]-2-oxo-ethyl}-3-methyl-7-(3-methyl-2-buten-1-yl)-8-(3-amino-piperidin-1-yl)-xanthin
(315) 1-[2-(4-Hydroxy-phenyl)-2-oxo-ethyl]-3-methyl-7-(3-methyl-2-buten-1-yl)-8-(3-amino-piperidin-1-yl)-xanthin
(316) 1-[2-(3-Nitro-phenyl)-2-oxo-ethyl]-3-methyl-7-(3-methyl-2-buten-1-yl)-8-(3-amino-piperidin-1-yl)-xanthin
(317) 1-[2-(3-Amino-phenyl)-2-oxo-ethyl]-3-methyl-7-(3-methyl-2-buten-1-yl)-8-(3-amino-piperidin-1-yl)-xanthin
(318) 1-{2-[3-(Methylcarbonylamino)-phenyl]-2-oxo-ethyl}-3-methyl-7-(3-methyl-2-buten-1-yl)-8-(3-amino-piperidin-1-yl)-xanthin
(319) 1-{2-[3-(Aminocarbonylamino)-phenyl]-2-oxo-ethyl}-3-methyl-7-(3-methyl-2-buten-1-yl)-8-(3-amino-piperidin-1-yl)-xanthin
(320) 1-{2-[3-(Methylaminocarbonylamino)-phenyl]-2-oxo-ethyl}-3-methyl-7-(3-methyl-2-buten-1-yl)-8-(3-amino-piperidin-1-yl)-xanthin
(321) 1-{2-[3-(Dimethylaminocarbonylamino)-phenyl]-2-oxo-ethyl}-3-methyl-7-(3-methyl-2-buten-1-yl)-8-(3-amino-piperidin-1-yl)-xanthin
(322) 1-{2-[3-(Methylsulfonylamino)-phenyl]-2-oxo-ethyl}-3-methyl-7-(3-methyl-2-buten-1-yl)-8-(3-amino-piperidin-1-yl)-xanthin
(323) 1-{2-[3-(Aminosulfonyl)-phenyl]-2-oxo-ethyl}-3-methyl-7-(3-methyl-2-buten-1-yl)-8-(3-amino-piperidin-1-yl)-xanthin
(324) 1-{2-[3-(Methylaminosulfonyl)-phenyl]-2-oxo-ethyl}-3-methyl-7-(3-methyl-2-buten-1-yl)-8-(3-amino-piperidin-1-yl)-xanthin
(325) 1-{2-[3-(Dimethylaminosulfonyl)-phenyl]-2-oxo-ethyl}-3-methyl-7-(3-methyl-2-buten-1-yl)-8-(3-amino-piperidin-1-yl)-xanthin
(326) 1-[2-(3-Ethinyl-phenyl)-2-oxo-ethyl]-3-methyl-7-(3-methyl-2-buten-1-yl)-8-(3-amino-piperidin-1-yl)-xanthin
(327) 1-[2-(3-Cyano-phenyl)-2-oxo-ethyl]-3-methyl-7-(3-methyl-2-buten-1-yl)-8-(3-amino-piperidin-1-yl)-xanthin
(328) 1-{2-[3-(Aminocarbonyl)-phenyl]-2-oxo-ethyl}-3-methyl-7-(3-methyl-2-buten-1-yl)-8-(3-amino-piperidin-1-yl)-xanthin
(329) 1-{2-[3-(Methylaminocarbonyl)-phenyl]-2-oxo-ethyl}-3-methyl-7-(3-methyl-2-buten-1-yl)-8-(3-amino-piperidin-1-yl)-xanthin
(330) 1-{2-[3-(Dimethylaminocarbonyl)-phenyl]-2-oxo-ethyl}-3-methyl-7-(3-methyl-2-buten-1-yl)-8-(3-amino-piperidin-1-yl)-xanthin
(331) 1-{2-[3-(Methylsulfanyl)-phenyl]-2-oxo-ethyl}-3-methyl-7-(3-methyl-2-buten-1-yl)-8-(3-amino-piperidin-1-yl)-xanthin
(332) 1-{2-[3-(Methylsulfinyl)-phenyl]-2-oxo-ethyl}-3-methyl-7-(3-methyl-2-buten-1-yl)-8-(3-amino-piperidin-1-yl)-xanthin
(333) 1-{2-[3-(Methylsulfonyl)-phenyl]-2-oxo-ethyl}-3-methyl-7-(3-methyl-2-buten-1-yl)-8-(3-amino-piperidin-1-yl)-xanthin
(334) 1-[2-(3,5-Dimethyl-phenyl)-2-oxo-ethyl]-3-methyl-7-(3-methyl-2-buten-1-yl)-8-(3-amino-piperidin-1-yl)-xanthin
(335) 1-[2-(3,5-Dimethoxy-phenyl)-2-oxo-ethyl]-3-methyl-7-(3-methyl-2-buten-1-yl)-8-(3-amino-piperidin-1-yl)-xanthin
(336) 1-[2-(3-Fluor-5-methyl-phenyl)-2-oxo-ethyl]-3-methyl-7-(3-methyl-2-buten-1-yl)-8-(3-amino-piperidin-1-yl)-xanthin
(337) 1-[2-(Pyridin-3-yl)-2-oxo-ethyl]-3-methyl-7-(3-methyl-2-buten-1-yl)-8-(3-amino-piperidin-1-yl)-xanthin
(338) 1-[2-(Furan-2-yl)-2-oxo-ethyl]-3-methyl-7-(3-methyl-2-buten-1-yl)-8-(3-amino-piperidin-1-yl)-xanthin
(339) 1-[2-(Thiophen-2-yl)-2-oxo-ethyl]-3-methyl-7-(3-methyl-2-buten-1-yl)-8-(3-amino-piperidin-1-yl)-xanthin
(340) 1-[2-(Thiazol-2-yl)-2-oxo-ethyl]-3-methyl-7-(3-methyl-2-buten-1-yl)-8-(3-amino-piperidin-1-yl)-xanthin
(341) 1-[2-(Thiazol-5-yl)-2-oxo-ethyl]-3-methyl-7-(3-methyl-2-buten-1-yl)-8-(3-amino-piperidin-1-yl)-xanthin
(342) 1-[2-(Thiazol-4-yl)-2-oxo-ethyl]-3-methyl-7-(3-methyl-2-buten-1-yl)-8-(3-amino-piperidin-1-yl)-xanthin
(343) 1-(2-Phenyl-2-oxo-ethyl)-7-(3-methyl-2-buten-1-yl)-8-(3-amino-piperidin-1-yl)-xanthin
(344) 1-(2-Phenyl-2-oxo-ethyl)-3-methyl-7-[(1-cyclopenten-1-yl)-methyl]-8-(3-amino-piperidin-1-yl)-xanthin

(345) 1-(2-Phenyl-2-oxo-ethyl)-3-methyl-7-[(2-methyl-1-cyclopenten-1-yl)-methyl]-8-(3-amino-piperidin-1-yl)-xanthin
(346) 1-(2-Phenyl-2-oxo-ethyl)-3-methyl-7-(2-butin-1-yl)-methyl]-8-(3-amino-piperidin-1-yl)-xanthin
(347) 1-(2-Phenyl-2-oxo-ethyl)-3-methyl-7-(3-methyl-2-buten-1-yl)-8-(3-amino-cyclohexyl)-xanthin
(348) 1-(2-Phenyl-2-oxo-ethyl)-3-methyl-7-(3-methyl-2-buten-1-yl)-8-[N-(2-amino-ethyl)-N-methyl-amino]-xanthin
(349) 1-(2-Phenyl-2-oxo-ethyl)-3-methyl-7-(3-methyl-2-buten-1-yl)-8-(piperazin-1-yl)-xanthin
(350) 1-(2-Phenyl-2-oxo-ethyl)-3-methyl-7-(3-methyl-2-buten-1-yl)-8-(homopiperazin-1-yl)-xanthin
(351) 1-(2-Phenyl-2-oxo-ethyl)-3-methyl-7-(3-methyl-2-buten-1-yl)-8-(4-aminomethyl-piperidin-1-yl)-xanthin
(352) 1-(2-Phenyl-2-oxo-ethyl)-3-methyl-7-(3-methyl-2-buten-1-yl)-8-(3-aminomethyl-piperidin-1-yl)-xanthin
(353) 1-(2-Phenyl-2-oxo-ethyl)-3-methyl-7-(3-methyl-2-buten-1-yl)-8-(2-amino-cyclohexylamino)-xanthin
(354) 1-(2-Phenyl-2-oxo-ethyl)-3-methyl-7-(3-methyl-2-buten-1-yl)-8-(3-amino-3-methyl-piperidin-1-yl)-xanthin
(355) 1-(2-Phenyl-2-hydroxyimino-ethyl)-3-methyl-7-(3-methyl-2-buten-1-yl)-8-(3-amino-piperidin-1-yl)-xanthin
(356) 1-(2-Phenyl-2-methoxyimino-ethyl)-3-methyl-7-(3-methyl-2-buten-1-yl)-8-(3-amino-piperidin-1-yl)-xanthin
(357) 1-(2-Oxo-propyl)-3-methyl-7-(3-methyl-2-buten-1-yl)-8-(3-amino-piperidin-1-yl)-xanthin
(358) 1-(2-Oxo-butyl)-3-methyl-7-(3-methyl-2-buten-1-yl)-8-(3-amino-piperidin-1-yl)-xanthin
(359) 1-(3-Methyl-2-oxo-butyl)-3-methyl-7-(3-methyl-2-buten-1-yl)-8-(3-amino-piperidin-1-yl)-xanthin
(360) 1-(2-Cyclopropyl-2-oxo-ethyl)-3-methyl-7-(3-methyl-2-buten-1-yl)-8-(3-amino-piperidin-1-yl)-xanthin
(361) 1-(2-Cyclohexyl-2-oxo-ethyl)-3-methyl-7-(3-methyl-2-buten-1-yl)-8-(3-amino-piperidin-1-yl)-xanthin
(362) 1-(3-Dimethylamino-2,3-dioxo-propyl)-3-methyl-7-(3-methyl-2-buten-1-yl)-8-(3-amino-piperidin-1-yl)-xanthin
(363) 1-[3-(Piperidin-1-yl)-2,3-dioxo-propyl]-3-methyl-7-(3-methyl-2-buten-1-yl)-8-(3-amino-piperidin-1-yl)-xanthin
(364) 1-(2-Phenyl-2-hydroxy-ethyl)-3-methyl-7-(3-methyl-2-buten-1-yl)-8-(3-amino-piperidin-1-yl)-xanthin
(365) 1-(2-Phenyl-2-hydroxy-propyl)-3-methyl-7-(3-methyl-2-buten-1-yl)-8-(3-amino-piperidin-1-yl)-xanthin
(366) 1-(2-Phenyl-2-methoxy-ethyl)-3-methyl-7-(3-methyl-2-buten-1-yl)-8-(3-amino-piperidin-1-yl)-xanthin
(367) 1-[(Isochinolin-1-yl)methyl]-3-methyl-7-(3-methyl-2-buten-1-yl)-8-(3-amino-piperidin-1-yl)-xanthin
(368) 1-[(Chinazolin-4-yl)methyl]-3-methyl-7-(3-methyl-2-buten-1-yl)-8-(3-amino-piperidin-1-yl)-xanthin
(369) 1-[(Pyridin-2-yl)methyl]-3-methyl-7-(3-methyl-2-buten-1-yl)-8-(3-amino-piperidin-1-yl)-xanthin
(370) 1-[(5-Methyl-isoxazol-3-yl)methyl]-3-methyl-7-(3-methyl-2-buten-1-yl)-8-(3-amino-piperidin-1-yl)-xanthin
(371) 1-[(Oxazol-2-yl)methyl]-3-methyl-7-(3-methyl-2-buten-1-yl)-8-(3-amino-piperidin-1-yl)-xanthin
(372) 1-[(Thiazol-2-yl)methyl]-3-methyl-7-(3-methyl-2-buten-1-yl)-8-(3-amino-piperidin-1-yl)-xanthin
(373) 1-[(1*H*-Indazol-3-yl)methyl]-3-methyl-7-(3-methyl-2-buten-1-yl)-8-(3-amino-piperidin-1-yl)-xanthin
(374) 1-[(1-Methyl-1*H*-indazol-3-yl)methyl]-3-methyl-7-(3-methyl-2-buten-1-yl)-8-(3-amino-piperidin-1-yl)-xanthin
(375) 1-[(Benzo[*d*]isoxazol-3-yl)methyl]-3-methyl-7-(3-methyl-2-buten-1-yl)-8-(3-amino-piperidin-1-yl)-xanthin

(376) 1-[(Benzo[*d*]isothiazol-3-yl)methyl]-3-methyl-7-(3-methyl-2-buten-1-yl)-8-(3-amino-piperidin-1-yl)-xanthin
(377) 1-[(5-Fluor-benzo[*d*]isothiazol-3-yl)methyl]-3-methyl-7-(3-methyl-2-buten-1-yl)-8-(3-amino-piperidin-1-yl)-xanthin
(378) 1-[(5-Fluor-benzo[*d*]isoxazol-3-yl)methyl]-3-methyl-7-(3-methyl-2-buten-1-yl)-8-(3-amino-piperidin-1-yl)-xanthin
(379) 1-[(5-Methyl-benzo[*d*]isoxazol-3-yl)methyl]-3-methyl-7-(3-methyl-2-buten-1-yl)-8-(3-amino-piperidin-1-yl)-xanthin
(380) 1-[(5-Methyl-benzo[*d*]isothiazol-3-yl)methyl]-3-methyl-7-(3-methyl-2-buten-1-yl)-8-(3-amino-piperidin-1-yl)-xanthin
(381) 1-(2-Phenyl-2-oxo-ethyl)-3-methyl-7-(3-methyl-2-buten-1-yl)-8-(3-imino-piperazin-1-yl)-xanthin
(382) 1-(2-Phenyl-2-oxo-ethyl)-3-methyl-7-(3-methyl-2-buten-1-yl)-8-(6-amino-[1,4]diazepan-1-yl)-xanthin
(383) 1-(2-Cyclohexyl-ethyl)-3-methyl-7-(3-methyl-2-buten-1-yl)-8-(3-amino-piperidin-1-yl)-xanthin
(384) 1-[2-(2-Difluormethoxy-phenyl)-ethyl]-3-methyl-7-(3-methyl-2-buten-1-yl)-8-(3-amino-piperidin-1-yl)-xanthin
(385) 1-[2-(2-Difluormethoxy-phenyl)-2-oxo-ethyl]-3-methyl-7-(3-methyl-2-buten-1-yl)-8-(3-amino-piperidin-1-yl)-xanthin
(386) 1-[2-(2-Trifluormethoxy-phenyl)-2-oxo-ethyl]-3-methyl-7-(3-methyl-2-buten-1-yl)-8-(3-amino-piperidin-1-yl)-xanthin
(387) 1-[2-(Indan-4-yl)-2-oxo-ethyl]-3-methyl-7-(3-methyl-2-buten-1-yl)-8-(3-amino-piperidin-1-yl)-xanthin
(388) 1-[2-(Benzo[1,3]dioxol-4-yl)-2-oxo-ethyl]-3-methyl-7-(3-methyl-2-buten-1-yl)-8-(3-amino-piperidin-1-yl)-xanthin
(389) 1-[2-(2,2-Difluoro-benzo[1,3]dioxol-4-yl)-2-oxo-ethyl]-3-methyl-7-(3-methyl-2-buten-1-yl)-8-(3-amino-piperidin-1-yl)-xanthin
(390) 1-[2-(Naphth-1-yl)-2-oxo-ethyl]-3-methyl-7-(3-methyl-2-buten-1-yl)-8-(3-amino-piperidin-1-yl)-xanthin
(391) 1-[2-(2-Isopropyl-phenyl)-2-oxo-ethyl]-3-methyl-7-(3-methyl-2-buten-1-yl)-8-(3-amino-piperidin-1-yl)-xanthin
(392) 1-[2-(2-Cyclopropyl-phenyl)-2-oxo-ethyl]-3-methyl-7-(3-methyl-2-buten-1-yl)-8-(3-amino-piperidin-1-yl)-xanthin
(393) 1-[2-(2-Cyclopentyl-phenyl)-2-oxo-ethyl]-3-methyl-7-(3-methyl-2-buten-1-yl)-8-(3-amino-piperidin-1-yl)-xanthin
(394) 1-[2-(2-Phenyl-phenyl)-2-oxo-ethyl]-3-methyl-7-(3-methyl-2-buten-1-yl)-8-(3-amino-piperidin-1-yl)-xanthin
(395) 1-[2-(2-Cyclopentylmethoxy-phenyl)-2-oxo-ethyl]-3-methyl-7-(3-methyl-2-buten-1-yl)-8-(3-amino-piperidin-1-yl)-xanthin
(396) 1-(3-Phenyl-2-oxo-propyl)-3-methyl-7-(3-methyl-2-buten-1-yl)-8-(3-amino-piperidin-1-yl)-xanthin

(397) 1-(3-Phenyl-3-oxo-propyl)-3-methyl-7-(3-methyl-2-buten-1-yl)-8-(3-amino-piperidin-1-yl)-xanthin
(398) 1-Methyl-3-cyclopentyl-7-(3-methyl-2-buten-1-yl)-8-(3-amino-piperidin-1-yl)-xanthin
(399) 1-Methyl-3-cyclohexyl-7-(3-methyl-2-buten-1-yl)-8-(3-amino-piperidin-1-yl)-xanthin
(400) 1-Methyl-3-(2-cyclopropyl-ethyl)-7-(3-methyl-2-buten-1-yl)-8-(3-amino-piperidin-1-yl)-xanthin
(401) 1-Methyl-3-(2-cyclohexyl-ethyl)-7-(3-methyl-2-buten-1-yl)-8-(3-amino-piperidin-1-yl)-xanthin
(402) 1-Methyl-3-(4-fluor-phenyl)-7-(3-methyl-2-buten-1-yl)-8-(3-amino-piperidin-1-yl)-xanthin
(403) 1-Methyl-3-(4-methyl-phenyl)-7-(3-methyl-2-buten-1-yl)-8-(3-amino-piperidin-1-yl)-xanthin
(404) 1-Methyl-3-(4-trifluormethyl-phenyl)-7-(3-methyl-2-buten-1-yl)-8-(3-amino-piperidin-1-yl)-xanthin
(405) 1-Methyl-3-(3-methoxy-phenyl)-7-(3-methyl-2-buten-1-yl)-8-(3-amino-piperidin-1-yl)-xanthin
(406) 1-Methyl-3-(3-difluormethoxy-phenyl)-7-(3-methyl-2-buten-1-yl)-8-(3-amino-piperidin-1-yl)-xanthin
(407) 1-Methyl-3-[2-(3-fluor-phenyl)-ethyl]-7-(3-methyl-2-buten-1-yl)-8-(3-amino-piperidin-1-yl)-xanthin
(408) 1-Methyl-3-[2-(3-methyl-phenyl)-ethyl]-7-(3-methyl-2-buten-1-yl)-8-(3-amino-piperidin-1-yl)-xanthin
(409) 1-Methyl-3-[2-(4-methoxy-phenyl)-ethyl]-7-(3-methyl-2-buten-1-yl)-8-(3-amino-piperidin-1-yl)-xanthin
(410) 1-Methyl-3-[2-(4-trifluormethoxy-phenyl)-ethyl]-7-(3-methyl-2-buten-1-yl)-8-(3-amino-piperidin-1-yl)-xanthin
(411) 1-Methyl-3-[2-(4-trifluormethoxy-phenyl)-2-oxo-ethyl]-7-(3-methyl-2-buten-1-yl)-8-(3-amino-piperidin-1-yl)-xanthin
(412) 1-Methyl-3-[2-(4-methoxy-phenyl)-2-oxo-ethyl]-7-(3-methyl-2-buten-1-yl)-8-(3-amino-piperidin-1-yl)-xanthin
(413) 1-Methyl-3-[2-(4-hydroxy-phenyl)-2-oxo-ethyl]-7-(3-methyl-2-buten-1-yl)-8-(3-amino-piperidin-1-yl)-xanthin
(414) 1-Methyl-3-[2-(3-chlor-phenyl)-2-oxo-ethyl]-7-(3-methyl-2-buten-1-yl)-8-(3-amino-piperidin-1-yl)-xanthin
(415) 1-Methyl-3-[2-(pyridin-3-yl)-2-oxo-ethyl]-7-(3-methyl-2-buten-1-yl)-8-(3-amino-piperidin-1-yl)-xanthin
(416) 1-Methyl-3-[2-(thiophen-2-yl)-2-oxo-ethyl]-7-(3-methyl-2-buten-1-yl)-8-(3-amino-piperidin-1-yl)-xanthin
(417) 1-Methyl-3-[3-methyl-2-oxo-butyl]-7-(3-methyl-2-buten-1-yl)-8-(3-amino-piperidin-1-yl)-xanthin
(418) 1-Methyl-3-(2-cyclopentyl-2-oxo-ethyl)-7-(3-methyl-2-buten-1-yl)-8-(3-amino-piperidin-1-yl)-xanthin
(419) 1-Methyl-3-(2-phenyloxy-ethyl)-7-(3-methyl-2-buten-1-yl)-8-(3-amino-piperidin-1-yl)-xanthin
(420) 1-(2-Phenyl-2-oxo-ethyl)-3-methyl-7-(4-fluor-phenyl)-8-(3-amino-piperidin-1-yl)-xanthin
(421) 1-(2-Phenyl-2-oxo-ethyl)-3-methyl-7-(3-trifluormethyl-phenyl)-8-(3-amino-piperidin-1-yl)-xanthin
(422) 1-(2-Phenyl-2-oxo-ethyl)-3-methyl-7-(3-methoxy-phenyl)-8-(3-amino-piperidin-1-yl)-xanthin
(423) 1-(2-Phenyl-2-oxo-ethyl)-3-methyl-7-(3-difluormethoxy-phenyl)-8-(3-amino-piperidin-1-yl)-xanthin
(424) 1-(2-Phenyl-2-oxo-ethyl)-3-methyl-7-(3-trifluormethoxy-phenyl)-8-(3-amino-piperidin-1-yl)-xanthin
(425) 1-(2-Phenyl-2-oxo-ethyl)-3-methyl-7-(3-methyl-2-buten-1-yl)-8-(4-amino-2-aza-bicyclo[3.2.1]oct-2-yl)-xanthin
(426) 1-[2-(2-Methylamino-phenyl)-2-oxo-ethyl]-3-methyl-7-(3-methyl-2-buten-1-yl)-8-(3-amino-piperidin-1-yl)-xanthin
(427) 1-{2-[2-(N-Cyanomethyl-N-methyl-amino)-phenyl]-2-oxo-ethyl}-3-methyl-7-(3-methyl-2-buten-1-yl)-8-(3-amino-piperidin-1-yl)-xanthin
(428) 1-[2-(2-Cyanomethylamino-phenyl)-2-oxo-ethyl]-3-methyl-7-(3-methyl-2-buten-1-yl)-8-(3-amino-piperidin-1-yl)-xanthin

(429) 1-(2-{2-[(Methoxycarbonyl)methylamino]-phenyl}-2-oxo-ethyl)-3-methyl-7-(3-methyl-2-buten-1-yl)-8-(3-amino-piperidin-1-yl)-xanthin
(430) 1-[2-(2-Methylsulfonylamino-phenyl)-2-oxo-ethyl]-3-methyl-7-(3-methyl-2-buten-1-yl)-8-(3-amino-piperidin-1-yl)-xanthin
(431) 1-(2-{3-[(Methoxycarbonyl)methylamino]-phenyl}-2-oxo-ethyl)-3-methyl-7-(3-methyl-2-buten-1-yl)-8-(3-amino-piperidin-1-yl)-xanthin
(432) 1-[2-(3-Methylamino-phenyl)-2-oxo-ethyl]-3-methyl-7-(3-methyl-2-buten-1-yl)-8-(3-amino-piperidin-1-yl)-xanthin
(433) 1-{2-[3-(N-Cyanomethyl-N-methyl-amino)-phenyl]-2-oxo-ethyl}-3-methyl-7-(3-methyl-2-buten-1-yl)-8-(3-amino-piperidin-1-yl)-xanthin
(434) 1-(2-{3-[(Dimethylamino)sulfonylamino]-phenyl}-2-oxo-ethyl)-3-methyl-7-(3-methyl-2-buten-1-yl)-8-(3-amino-piperidin-1-yl)-xanthin
(435) 1-(2-{3-[(Morpholin-4-yl)sulfonylamino]-phenyl}-2-oxo-ethyl)-3-methyl-7-(3-methyl-2-buten-1-yl)-8-(3-amino-piperidin-1-yl)-xanthin
(436) 1-[2-(3-Aminosulfonylamino-phenyl)-2-oxo-ethyl]-3-methyl-7-(3-methyl-2-buten-1-yl)-8-(3-amino-piperidin-1-yl)-xanthin
(437) 1-[2-(3-Ethylsulfonylamino-phenyl)-2-oxo-ethyl]-3-methyl-7-(3-methyl-2-buten-1-yl)-8-(3-amino-piperidin-1-yl)-xanthin
(438) 1-[2-(3-Isopropylsulfonylamino-phenyl)-2-oxo-ethyl]-3-methyl-7-(3-methyl-2-buten-1-yl)-8-(3-amino-piperidin-1-yl)-xanthin
(439) 1-{2-[3-(2-Oxo-imidazolidin-1-yl)-phenyl]-2-oxo-ethyl}-3-methyl-7-(3-methyl-2-buten-1-yl)-8-(3-amino-piperidin-1-yl)-xanthin
(440) 1-{2-[3-(3-Methyl-2-oxo-imidazolidin-1-yl)-phenyl]-2-oxo-ethyl}-3-methyl-7-(3-methyl-2-buten-1-yl)-8-(3-amino-piperidin-1-yl)-xanthin
(441) 1-{2-[3-(3-Methyl-2,5-dioxo-imidazolidin-1-yl)-phenyl]-2-oxo-ethyl}-3-methyl-7-(3-methyl-2-buten-1-yl)-8-(3-amino-piperidin-1-yl)-xanthin
(442) 1-{2-[3-(3-Methyl-2,4-dioxo-imidazolidin-1-yl)-phenyl]-2-oxo-ethyl}-3-methyl-7-(3-methyl-2-buten-1-yl)-8-(3-amino-piperidin-1-yl)-xanthin
(443) 1-[(2-Oxo-1,2-dihydro-chinolin-4-yl)methyl]-3-methyl-7-(3-methyl-2-buten-1-yl)-8-(3-amino-piperidin-1-yl)-xanthin
(444) 1-[(1-Methyl-2-oxo-1,2-dihydro-chinolin-4-yl)methyl]-3-methyl-7-(3-methyl-2-buten-1-yl)-8-(3-amino-piperidin-1-yl)-xanthin
(445) 1-[(2-Oxo-1,2-dihydro-chinazolin-4-yl)methyl]-3-methyl-7-(3-methyl-2-buten-1-yl)-8-(3-amino-piperidin-1-yl)-xanthin
(446) 1-[(1-Methyl-2-oxo-1,2-dihydro-chinazolin-4-yl)methyl]-3-methyl-7-(3-methyl-2-buten-1-yl)-8-(3-amino-piperidin-1-yl)-xanthin
(447) 1-[(2-Cyano-naphthalin-1-yl)methyl]-3-methyl-7-(3-methyl-2-buten-1-yl)-8-(3-amino-piperidin-1-yl)-xanthin
(448) 1-[(6-Cyano-naphthalin-1-yl)methyl]-3-methyl-7-(3-methyl-2-buten-1-yl)-8-(3-amino-piperidin-1-yl)-xanthin
(449) 1-[(5-Cyano-naphthalin-1-yl)methyl]-3-methyl-7-(3-methyl-2-buten-1-yl)-8-(3-amino-piperidin-1-yl)-xanthin
(450) 1-[(8-Methyl-isochinolin-1-yl)methyl]-3-methyl-7-(3-methyl-2-buten-1-yl)-8-(3-amino-piperidin-1-yl)-xanthin
(451) 1-[(5-Cyano-isochinolin-1-yl)methyl]-3-methyl-7-(3-methyl-2-buten-1-yl)-8-(3-amino-piperidin-1-yl)-xanthin
(452) 1-[(5-Aminocarbonyl-isochinolin-1-yl)methyl]-3-methyl-7-(3-methyl-2-buten-1-yl)-8-(3-amino-piperidin-1-yl)-xanthin
(453) 1-[(5-Aminosulfonyl-isochinolin-1-yl)methyl]-3-methyl-7-(3-methyl-2-buten-1-yl)-8-(3-amino-piperidin-1-yl)-xanthin
(454) 1-[(5-Methylsulfonyl-isochinolin-1-yl)methyl]-3-methyl-7-(3-methyl-2-buten-1-yl)-8-(3-amino-piperidin-1-yl)-xanthin
(455) 1-[(5-Methylsulfonylamino-isochinolin-1-yl)methyl]-3-methyl-7-(3-methyl-2-buten-1-yl)-8-(3-amino-piperidin-1-yl)-xanthin
(456) 1-[(5-Methoxy-isochinolin-1-yl)methyl]-3-methyl-7-(3-methyl-2-buten-1-yl)-8-(3-amino-piperidin-1-yl)-xanthin
(457) 1-[(6-Methoxy-isochinolin-1-yl)methyl]-3-methyl-7-(3-methyl-2-buten-1-yl)-8-(3-amino-piperidin-1-yl)-xanthin
(458) 1-[(7-Methylsulfonylamino-isochinolin-1-yl)methyl]-3-methyl-7-(3-methyl-2-buten-1-yl)-8-(3-amino-piperidin-1-yl)-xanthin
(459) 1-[(7-Cyano-isochinolin-1-yl)methyl]-3-methyl-7-(3-methyl-2-buten-1-yl)-8-(3-amino-piperidin-1-yl)-xanthin

(460) 1-[(7-Aminocarbonyl-isochinolin-1-yl)methyl]-3-methyl-7-(3-methyl-2-buten-1-yl)-8-(3-amino-piperidin-1-yl)-xanthin
(461) 1-[2-(2-Hydroxy-phenyl)-2-oxo-ethyl]-3-methyl-7-(3-methyl-2-buten-1-yl)-8-(3-amino-piperidin-1-yl)-xanthin
(462) 1-[2-(2-Cyanomethoxy-phenyl)-2-oxo-ethyl]-3-methyl-7-(3-methyl-2-buten-1-yl)-8-(3-amino-piperidin-1-yl)-xanthin
(463) 1-(2-{2-[(Methoxycarbonyl)methoxy]-phenyl}-2-oxo-ethyl)-3-methyl-7-(3-methyl-2-buten-1-yl)-8-(3-amino-piperidin-1-yl)-xanthin
(464) 1-[2-(2-Allyloxy-phenyl)-2-oxo-ethyl]-3-methyl-7-(3-methyl-2-buten-1-yl)-8-(3-amino-piperidin-1-yl)-xanthin
(465) 1-(2-{3-[(Aminocarbonyl)methoxy]-phenyl}-2-oxo-ethyl)-3-methyl-7-(3-methyl-2-buten-1-yl)-8-(3-amino-piperidin-1-yl)-xanthin
(466) 1-(2-{3-[(Methylaminocarbonyl)methoxy]-phenyl}-2-oxo-ethyl)-3-methyl-7-(3-methyl-2-buten-1-yl)-8-(3-amino-piperidin-1-yl)-xanthin
(467) 1-(2-{3-[(Dimethylaminocarbonyl)methoxy]-phenyl}-2-oxo-ethyl)-3-methyl-7-(3-methyl-2-buten-1-yl)-8-(3-amino-piperidin-1-yl)-xanthin
(468) 1-[2-(3-{[(Morpholin-4-yl)carbonyl]methoxy}-phenyl)-2-oxo-ethyl]-3-methyl-7-(3-methyl-2-buten-1-yl)-8-(3-amino-piperidin-1-yl)-xanthin
(469) 1-[2-(3-Carboxymethoxy-phenyl)-2-oxo-ethyl]-3-methyl-7-(3-methyl-2-buten-1-yl)-8-(3-amino-piperidin-1-yl)-xanthin
(470) 1-[2-(3-Methylsulfanylmethoxy-phenyl)-2-oxo-ethyl]-3-methyl-7-(3-methyl-2-buten-1-yl)-8-(3-amino-piperidin-1-yl)-xanthin
(471) 1-[2-(3-Methylsulfinylmethoxy-phenyl)-2-oxo-ethyl]-3-methyl-7-(3-methyl-2-buten-1-yl)-8-(3-amino-piperidin-1-yl)-xanthin
(472) 1-[2-(3-Methylsulfoylmethoxy-phenyl)-2-oxo-ethyl]-3-methyl-7-(3-methyl-2-buten-1-yl)-8-(3-amino-piperidin-1-yl)-xanthin
(473) 1-[2-(2-Oxo-2,3-dihydro-benzooxazol-4-yl)-2-oxo-ethyl]-3-methyl-7-(3-methyl-2-buten-1-yl)-8-(3-amino-piperidin-1-yl)-xanthin
(474) 1-[2-(2-Oxo-2,3-dihydro-1*H*-benzoimidazol-4-yl)-2-oxo-ethyl]-3-methyl-7-(3-methyl-2-buten-1-yl)-8-(3-amino-piperidin-1-yl)-xanthin
(475) 1-[2-(1-Methyl-2-oxo-2,3-dihydro-1*H*-benzoimidazol-4-yl)-2-oxo-ethyl]-3-methyl-7-(3-methyl-2-buten-1-yl)-8-(3-amino-piperidin-1-yl)-xanthin
(476) 1-[2-(1,3-Dimethyl-2-oxo-2,3-dihydro-1*H*-benzoimidazol-4-yl)-2-oxo-ethyl]-3-methyl-7-(3-methyl-2-buten-1-yl)-8-(3-amino-piperidin-1-yl)-xanthin
(477) 1-[2-(1*H*-Benzoimidazol-4-yl)-2-oxo-ethyl]-3-methyl-7-(3-methyl-2-buten-1-yl)-8-(3-amino-piperidin-1-yl)-xanthin
(478) 1-[2-(2-Methyl-1*H*-benzoimidazol-4-yl)-2-oxo-ethyl]-3-methyl-7-(3-methyl-2-buten-1-yl)-8-(3-amino-piperidin-1-yl)-xanthin
(479) 1-[2-(Benzooxazol-4-yl)-2-oxo-ethyl]-3-methyl-7-(3-methyl-2-buten-1-yl)-8-(3-amino-piperidin-1-yl)-xanthin
(480) 1-[2-(2-Methyl-benzooxazol-4-yl)-2-oxo-ethyl]-3-methyl-7-(3-methyl-2-buten-1-yl)-8-(3-amino-piperidin-1-yl)-xanthin
(481) 1-[2-(3-Oxo-3,4-dihydro-2*H*-benzo[1,4]oxazin-5-yl)-2-oxo-ethyl]-3-methyl-7-(3-methyl-2-buten-1-yl)-8-(3-amino-piperidin-1-yl)-xanthin
(482) 1-[2-(Benzo[1,3]dioxol-4-yl)-2-oxo-ethyl]-3-methyl-7-(3-methyl-2-buten-1-yl)-8-(3-amino-piperidin-1-yl)-xanthin
(483) 1-(2-Phenyl-2-oxo-ethyl)-3-methyl-7-(3-methyl-2-buten-1-yl)-8-(3-amino-3-aminocarbonyl-piperidin-1-yl)-xanthin
(484) 1-(2-Phenyl-2-oxo-ethyl)-3-methyl-7-(3-methyl-2-buten-1-yl)-8-(3-amino-4-aminocarbonyl-piperidin-1-yl)-xanthin
(485) 1-(2-Phenyl-2-oxo-ethyl)-3-methyl-7-(3-methyl-2-buten-1-yl)-8-(3-amino-3-methylaminocarbonyl-piperidin-1-yl)-xanthin
(486) 1-(2-Phenyl-2-oxo-ethyl)-3-methyl-7-(3-methyl-2-buten-1-yl)-8-(3-amino-3-dimethylaminocarbonyl-piperidin-1-yl)-xanthin
(487) 1-(2-Phenyl-2-oxo-ethyl)-3-methyl-7-(3-methyl-2-buten-1-yl)-8-{3-amino-3-[(pyrrohdin-1-yl)carbonyl]-piperidin-1-yl}-xanthin
(488) 1-(2-Phenyl-2-oxo-ethyl)-3-methyl-7-(3-methyl-2-buten-1-yl)-8-{3-amino-3-[(2-cyano-pyrrolidin-1-yl)carbonyl]-piperidin-1-yl}-xanthin
(489) 1-(2-Phenyl-2-oxo-ethyl)-3-methyl-7-(3-methyl-2-buten-1-yl)-8-{3-amino-3-[(thiazolidin-3-yl)carbonyl]-piperidin-1-yl}-xanthin
(490) 1-(2-Phenyl-2-oxo-ethyl)-3-methyl-7-(3-methyl-2-buten-1-yl)-8-{3-amino-3-[(4-cyano-thiazolidin-3-yl)carbonyl]-piperidin-1-yl}-xanthin
(491) 1-(2-Phenyl-2-oxo-ethyl)-3-methyl-7-(3-methyl-2-buten-1-yl)-8-(5-amino-6-oxo-piperidin-3-yl)-xanthin

(492) 1-(2-Phenyl-2-oxo-ethyl)-3-methyl-7-(3-methyl-2-buten-1-yl)-8-(5-amino-1-methyl-6-oxo-piperidin-3-yl)-xanthin
(493) 1-(2-Phenyl-2-oxo-ethyl)-3-methyl-7-(3-methyl-2-buten-1-yl)-8-(3-amino-4-hydroxy-piperidin-1-yl)-xanthin
(494) 1-(2-Phenyl-2-oxo-ethyl)-3-methyl-7-(3-methyl-2-buten-1-yl)-8-(3-amino-4-methoxy-piperidin-1-yl)-xanthin
(495) 1-(2-Phenyl-2-oxo-ethyl)-3-methyl-7-(3-methyl-2-buten-1-yl)-8-(3-amino-5-hydroxy-piperidin-1-yl)-xanthin
(496) 1-(2-Phenyl-2-oxo-ethyl)-3-methyl-7-(3-methyl-2-buten-1-yl)-8-(5-amino-2-oxo-piperidin-1-yl)-xanthin
(497) 1-(2-Phenyl-2-oxo-ethyl)-3-methyl-7-(3-methyl-2-buten-1-yl)-8-(3-amino-2-oxo-piperidin-1-yl)-xanthin
(498) 1-(1-Methoxycarbonyl-1-phenyl-methyl)-3-methyl-7-(3-methyl-2-buten-1-yl)-8-(3-amino-piperidin-1-yl)-xanthin
(499) 1-(1-Carboxy-1-phenyl-methyl)-3-methyl-7-(3-methyl-2-buten-1-yl)-8-(3-amino-piperidin-1-yl)-xanthin
(500) 1-(1-Aminocarbonyl-1-phenyl-methyl)-3-methyl-7-(3-methyl-2-buten-1-yl)-8-(3-amino-piperidin-1-yl)-xanthin
(501) 1-(1-Methoxycarbonyl-2-phenyl-ethyl)-3-methyl-7-(3-methyl-2-buten-1-yl)-8-(3-amino-piperidin-1-yl)-xanthin
(502) 1-(1-Carboxy-2-phenyl-ethyl)-3-methyl-7-(3-methyl-2-buten-1-yl)-8-(3-amino-piperidin-1-yl)-xanthin
(503) 1-(1-Aminocarbonyl-2-phenyl-ethyl)-3-methyl-7-(3-methyl-2-buten-1-yl)-8-(3-amino-piperidin-1-yl)-xanthin
(504) 1-[(Benzofuran-2-yl)methyl]-3-methyl-7-(3-methyl-2-buten-1-yl)-8-(3-amino-piperidin-1-yl)-xanthin
(505) 1-[(2,3-Dihydro-benzofuran-2-yl)methyl]-3-methyl-7-(3-methyl-2-buten-1-yl)-8-(3-amino-piperidin-1-yl)-xanthin
(506) 1-[2-(2-Amino-3-cyano-phenyl)-2-oxo-ethyl]-3-methyl-7-(3-methyl-2-buten-1-yl)-8-(3-amino-piperidin-1-yl)-xanthin
(507) 1-[2-(2-Amino-3-fluor-phenyl)-2-oxo-ethyl]-3-methyl-7-(3-methyl-2-buten-1-yl)-8-(3-amino-piperidin-1-yl)-xanthin
(508) 1-(2-Phenyl-2-oxo-ethyl)-3-(tetrahydrofuran-3-yl)-7-(3-methyl-2-buten-1-yl)-8-(3-amino-piperidin-1-yl)-xanthin
(509) 1-(2-Phenyl-2-oxo-ethyl)-3-(tetrahydropyran-4-yl)-7-(3-methyl-2-buten-1-yl)-8-(3-amino-piperidin-1-yl)-xanthin
(510) 1-(2-Phenyl-2-oxo-ethyl)-3-[(tetrahydrofuran-2-yl)methyl]-7-(3-methyl-2-buten-1-yl)-8-(3-amino-piperidin-1-yl)-xanthin
(511) 1-(2-Phenyl-2-oxo-ethyl)-3-[(tetrahydropyran-4-yl)methyl]-7-(3-methyl-2-buten-1-yl)-8-(3-amino-piperidin-1-yl)-xanthin
(512) 1-Methyl-3-[2-(4-dimethylamino-phenyl)-ethyl]-7-(2-cyano-benzyl)-8-(3-amino-piperidin-1-yl)-xanthin
(513) 1,3-Dimethyl-7-(3-methyl-1-buten-1-yl)-8-(3-amino-piperidin-1-yl)-xanthin
(514) 1-(1,4-Dioxo-1,4-dihydro-naphthalen-2-yl)-3-methyl-7-(3-methyl-2-buten-1-yl)-8-(3-amino-piperidin-1-yl)-xanthin
(515) 1-(4-Oxo-4H-chromen-3-yl)-3-methyl-7-(3-methyl-2-buten-1-yl)-8-(3-amino-piperidin-1-yl)-xanthin
(516) 1-(1-Oxo-indan-2-yl)-3-methyl-7-(3-methyl-2-buten-1-yl)-8-(3-amino-piperidin-1-yl)-xanthin
(517) 1-(1-Methyl-2-phenyl-2-oxo-ethyl)-3-methyl-7-(3-methyl-2-buten-1-yl)-8-(3-amino-piperidin-1-yl)-xanthin
(518) 1-[2-Oxo-2-(3-oxo-3,4-dihydro-2H-benzo[1,4]oxazin-8-yl)-ethyl]-3-methyl-7-(3-methyl-2-buten-1-yl)-8-(3-amino-piperidin-1-yl)-xanthin
(519) 1-[2-Oxo-2-(4-methyl-3-oxo-3,4-dihydro-2H-benzo[1,4]oxazin-8-yl)-ethyl]-3-methyl-7-(3-methyl-2-buten-1-yl)-8-(3-amino-piperidin-1-yl)-xanthin
(520) 1-[(Cinnolin-4-yl)methyl]-3-methyl-7-(3-methyl-2-buten-1-yl)-8-(3-amino-piperidin-1-yl)-xanthin
(521) 1-[(2-Oxo-2H-chromen-4-yl)methyl]-3-methyl-7-(3-methyl-2-buten-1-yl)-8-(3-amino-piperidin-1-yl)-xanthin
(522) 1-[(1-Oxo-1,2-dihydro-isochinolin-4-yl)methyl]-3-methyl-7-(3-methyl-2-buten-1-yl)-8-(3-amino-piperidin-1-yl)-xanthin
(523) 1-[(2-Methyl-1-oxo-1,2-dihydro-isochinolin-4-yl)methyl]-3-methyl-7-(3-methyl-2-buten-1-yl)-8-(3-amino-piperidin-1-yl)-xanthin
(524) 1-[(4-Oxo-3,4-dihydro-phthalazin-1-yl)methyl]-3-methyl-7-(3-methyl-2-buten-1-yl)-8-(3-amino-piperidin-1-yl)-xanthin
(525) 1-[(3-Methyl-4-oxo-3,4-dihydro-phthalazin-1-yl)methyl]-3-methyl-7-(3-methyl-2-buten-1-yl)-8-(3-amino-piperidin-1-yl)-xanthin
(526) 1-[([1,5]Naphthyridin-4-yl)methyl]-3-methyl-7-(3-methyl-2-buten-1-yl)-8-(3-amino-piperidin-1-yl)-xanthin
(527) 1-[([1,7]Naphthyridin-8-yl)methyj-3-methyl-7-(3-methyl-2-buten-1-yl)-8-(3-amino-piperidin-1-yl)-xanthin
(528) 1-[(Chinolin-2-yl)methyl]-3-methyl-7-(3-methyl-2-buten-1-yl)-8-(3-amino-piperidin-1-yl)-xanth in
(529) 1-[(Isochinolin-3-yl)methyl]-3-methyl-7-(3-methyl-2-buten-1-yl)-8-(3-amino-piperidin-1-yl)-xanthin
(530) 1-(2-Oxo-2-[3-(2-oxo-tetrahydro-pyrimidin-1-yl)-phenyl]-ethyl}-3-methyl-7-(3-methyl-2-buten-1-yl)-8-(3-amino-piperidin-1-yl)-xanthin
(531) 1-{2-Oxo-2-[3-(3-methyl-2-oxo-tetrahydro-pyrimidin-1-yl)-phenyl]-ethyl}-3-methyl-7-(3-methyl-2-buten-1-yl)-8-(3-amino-piperidin-1-yl)-xanthin

### Beispiel 11

### Dragées mit 75 mg Wirksubstanz

| 1 Dragéekern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragäes werden mit Bienenwachs geglänzt.
Dragéegewicht: 245 mg.

### Beispiel 12

### Tabletten mit 100 mg Wirksubstanz

Zusammensetzung:

| 1 Tablette enthält: | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2.0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 13

### Tabletten mit 150 mg Wirksubstanz

### Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 14

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 15

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840.0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 16

### Suspension mit 50 mg Wirksubstanz

### 100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 17

### Ampullen mit 10 mg Wirksubstanz

### Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 18

### Ampullen mit 50 mg Wirksubstanz

### Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 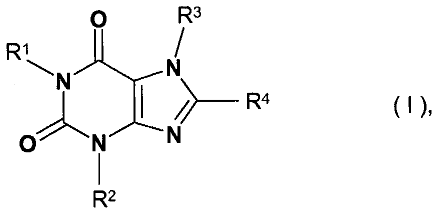 in der
R¹ eine C₁₋₈-Alkylgruppe,
eine C₃₋₈-Alkenylgruppe,
eine C₃₋₄-Alkenylgruppe, die durch eine C₁₋₂-Alkyloxy-carbonyl-, Aminocarbonyl-, C₁₃-Alkylamino-carbonyl-, Di-(C₁₋₃-alkyl)-amino-carbonyl-, Pyrrolidin-1-ylcarbonyl-, Piperidin-1-ylcarbonyl- oder Morpholin-4-ylcarbonyl-gruppe substituiert ist,
eine C₃₋₈-Alkinylgruppe,
eine durch eine Gruppe Rₐ substituierte C₁₋₆-Alkylgruppe, wobei
Rₐ eine C₃₋₇Cycloalkyl-, Heteroaryl-, Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-yl-carbonyl-, Piperazin-1-ylcarbonyl-, 4-Methylpiperazin-1-ylcarbonyl- oder 4-Ethylpiperazin-1-ylcarbonylgruppe bedeutet,
eine durch eine Phenylgruppe substituierte C₁₋₆-Alkylgruppe, wobei der Phenylring durch die Gruppen R¹⁰ bis R¹⁴ substituiert ist und
R¹⁰ ein Wasserstoffatom,
ein Fluor-, Chlor-, Brom- oder lodatom,
eine C₁₋₄-Alkyl-, Hydroxy-, oder C₁₋₄-Alkyloxygruppe,
eine Nitro-, Amino-, C₁₋₃-Alkylamino-, Di-(C₁₋₃-alkyl)amino-, Cyan-C₁₋₃-alkyl-amino-, [N-(Cyan-C₁₋₃-alkyl)-N-C₁₋₃-alkyl-amino]-, C₁₋₃-Alkyloxy-carbonyl-C₁₋₃-alkylamino-, Pyrrolidin-1-yl-, Piperidin-1-yl-, Morpholin-4-yl-, Piperazin-1-yl-, oder 4-(C₁₋₃-Alkyl)-piperazin-1-yl-Gruppe,
eine C₁₋₃-Alkyl-carbonylamino-, Arylcarbonylamino-, Aryl-C₁₋₃-alkyl-carbonyl-amino-, C₁₋₃-Alkyloxy-carbonylamino-, Aminocarbonylamino-, C₁₋₃-Alkyl-aminocarbonylamino-, Di-(C₁₋₃-alkyl)aminocarbonylamino-, Pyrrolidin-1-yl-carbonylamino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonyl-amino-, C₁₋₃-Alkyl-sulfonylamino-, Bis-(C₁₋₃-alkylsulfonyl)-amino-, Aminosulfonylamino-, C₁₋₃-Alkylamino-sulfonylamino-, Di-(C₁₋₃-alkyl)amino-sulfonyl-amino-, Pyrrolidin-1-yl-sulfonylamino-, Piperidin-1-yl-sulfonylamino-, Morpholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)thiocarbonylamino-, (C₁₋₃-Alkyloxy-carbonylamino)carbonylamino-, Arylsulfonylamino- oder Aryl-C₁₋₃-alkyl-sulfonylaminogruppe,
eine N-(C₁₋₃-Alkyl)-C₁₋₃-alkyl-carbonylamino-, N-(C₁₋₃-Alkyl)-arylcarbonylamino-, N-(C₁₋₃-Alkyl)-aryl-C₁₋₃-alkyl-carbonylamino-, N-(C₁₋₃-Alkyl)-C₁₋₃-alkyloxy-carbonylamino-, N-(Aminocarbonyl)-C₁₋₃-alkylamino-, N-(C₁₋₃-Alkyl-aminocarbonyl)-C₁₋₃-alkylamino -, N-[Di-(C₁-₃-alkyl)aminocarbonyl]-C₁₋₃-alkylamino-, N-(C₁₋₃-Alkyl)-C₁₋₃-alkyl-sulfonylamino-, N-(C₁₋₃-Alkyl)-arylsulfonylamino-, oder N-(C₁₋₃-Alkyl)-aryl-C₁₋₃-alkyl-sulfonylaminogruppe,
eine 2-Oxo-imidazolidin-1-yl-, 2,4-Dioxo-imidazolidin-1-yl-, 2,5-Dioxo-imidazolidin-1-yl- oder 2-Oxo-hexahydropyrimidin-1-ylgruppe, in der das Stickstoffatom in 3-Stellung jeweils durch eine Methyl- oder Ethylgruppe substituiert sein kann,
eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl-oder 4-(C₁₋₃-Alkyl)-piperazin-1-yl-carbonylgruppe,
eine C₁₋₃-Alkyl-carbonyl- oder eine Arylcarbonylgruppe,
eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1-yl-carbonyl-C₁₋₃-alkyl- oder 4-(C₁₋₃-Alkyl)-piperazin-1-yl-carbonyl-C₁₋₃-alkylgruppe,
eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy-, C₁₋₃-Alkyl-aminocarbonyl-C₁₋₃-alkyloxy-, Di-(C₁₋₃-alkyl)-aminocarbonyl-C₁₋₃-alkyloxy-, Pyrrolidin-1-yl-carbonyl-C₁₋₃-alkyl-oxy-, Piperidin-1-yl-carbonyl-C₁₋₃-alkyloxy-, Morpholin-4-yl-carbonyl-C₁₋₃-alkyl-oxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
eine Hydroxy-C₁₋₃-alkyl-, C₁₋₃-Alkyloxy-C₁₋₃-alkyl-, Amino-C₁₋₃-alkyl-, C₁₋₃-Alkyl-amino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl-, 4-(C₁₋₃-Alkyl)-piperazin-1-yl-C₁₋₃-alkylgruppe,
eine Hydroxy-C₁₋₃-alkyloxy-, C₁₋₃-Alkyloxy-C₁₋₃-alkyloxy-, C₁₋₃-Alkylsulfanyl-C₁-₃-alkyloxy-, C₁₋₃-Alkylsulfinyl-C₁₋₃-alkyloxy-, C₁₋₃-Alkylsulfonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁-₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy-, 4-(C₁₋₃-Alkyl)-piperazin-1-yl-C₁₋₃-alkyloxygruppe,
eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Arylsulfonyloxy-, Trifluormethylsulfanyl-, Trifluormethyl-sulfinyl- oder Trifluormethylsulfonylgruppe,
eine Sulfo-, Aminosulfonyl-, C₁₋₃-Alkyl-aminosulfonyl-, Di-(C₁₋₃-alkyl)-amino-sulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonyl-gruppe,
eine durch 1 bis 3 Fluoratome substituierte Methyl- oder Methoxygruppe,
eine durch 1 bis 5 Fluoratome substituierte Ethyl- oder Ethoxygruppe,
eine C₂₋₄-Alkenyl- oder C₂₋₄-Alkinylgruppe,
eine C₃₋₄-Alkenyloxy- oder C₃₋₄-Alkinyloxygruppe,
eine C₃₋₆-Cycloalkyl- oder C₃₋₆-Cycloalkyloxygruppe,
eine C₃₋₆-Cycloalkyl-C₁₋₃-alkyl- oder C₃₋₆-Cycloalkyl-C₁₋₃-alkyloxygruppe oder
eine Aryl-, Aryloxy-, Aryl-C₁₋₃-alkyl- oder Aryl-C₁₋₃-alkyloxygruppe,
R¹¹ und R¹², die gleich oder verschieden sein können, jeweils ein Wasserstoffatom, ein Fluor-, Chlor-, Brom- oder lodatom, eine C₁₋₃-Alkyl-, Trifluormethyl-, Hydroxy-, oder C₁₋₃-Alkyloxygruppe oder eine Cyangruppe, oder
R¹¹ zusammen mit R¹², sofern diese an benachbarte Kohlenstoffatome gebunden sind, auch eine Methylendioxy-, Difluormethylendioxy-oder eine geradkettige C₃₋₅-Alkylengruppe,
und
R¹³ und R¹⁴, die gleich oder verschieden sein können, jeweils ein Wasserstoffatom, ein Fluor-, Chlor- oder Bromatom, eine Trifluormethyl-, C₁₋₃-Alkyl- oder C₁₋₃-Alkyloxygruppe bedeuten,
eine Phenyl-C₁₋₄-alkylgruppe, in der der Alkylteil durch eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-Gruppe substituiert ist und der Phenylteil durch die Gruppen R¹⁰ bis R¹⁴ substituiert ist, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴ wie vorstehend erwähnt definiert sind und
A eine Carbonyl-, Cyaniminomethylen-, Hydroxyiminomethylen- oder C₁₋₃-Alkyloxyiminomethylen-Gruppe, m die Zahl 0, 1 oder 2 und n die Zahl 1, 2 oder 3 bedeuten,
eine Phenylcarbonylmethylgruppe, in der der Phenylteil durch R¹⁰ bis R¹⁴ substituiert ist, wobei R¹⁰ bis R¹⁴ wie vorstehend erwähnt definiert sind und der Methylteil durch eine C₁₋₃-Alkylgruppe substituiert ist,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, m und n wie vorstehend erwähnt definiert sind und
B eine Methylengruppe, die durch eine Hydroxy-, C₁₋₃-Alkyloxy-, Amino-, C₁₋₃-Alkylamino-, Di-(C₁₋₃-alkyl)-amino-, Mercapto-, C₁₋₃-Alkylsulfanyl-, C₁₋₃-Alkyl-sulfinyl- oder C₁₋₃-Alkylsulfonylgruppe substituiert ist und gegebenenfalls zusätzlich durch eine Methyl- oder Ethylgruppe substituiert ist, bedeutet,
eine Naphthyl-C₁₋₃-alkylgruppe, in der der Naphthylteil durch die Gruppen R¹⁰ bis R¹⁴ substituiert ist, wobei R¹⁰ bis R¹⁴ wie vorstehend erwähnt definiert sind,
eine Naphthyl-(CH₂)ₘ-A-(CH₂)ₙ-Gruppe, in der der Naphthylteil durch R¹⁰ bis R¹⁴ substituiert ist, wobei R¹⁰ bis R¹⁴, A, m und n wie vorstehend erwähnt definiert sind,
eine Naphthyl-(CH₂)ₘ-B-(CH₂)ₙ-Gruppe, in der der Naphthylteil durch R¹⁰ bis R¹⁴ substituiert ist, wobei R¹⁰ bis R¹⁴, B, m und n wie vorstehend erwähnt definiert sind,
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₃-Alkyloxycarbonyl-, Aminocarbonyl-, C₁₋₃-Alkylaminocarbonyl-, Di-(C₁₋₃-alkyl)aminocarbonyl-, Pyrrolidin-1-yl-carbonyl-, Piperidin-1-yl-carbonyl- oder Morpholin-4-yl-carbonyl-, Piperazin-1-yl-carbonyl-, 4-Methylpiperazin-1-yl-carbonyl- oder 4-Ethylpiperazin-1-yl-carbonyl-Gruppe bedeutet und A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴ und m wie vorstehend erwähnt sind und D eine Sauerstoff- oder Schwefelatom, eine Imino-, C₁₋₃-Alkylimino-, Sulfinyl- oder Sulfonylgruppe bedeutet,
eine Naphthyl-(CH₂)ₘ-D-C₁₋₃-alkylgruppe, in der der Naphthylteil durch die Gruppen R¹⁰ bis R¹⁴ substituiert ist, wobei R¹⁰ bis R¹⁴, D und m wie vorstehend erwähnt sind, oder
eine durch eine Gruppe R_{b} substituierte C₂₋₆-Alkylgruppe, wobei
R_{b} durch mindestens zwei Kohlenstoffatome vom Ringstickstoffatom in 1-Stellung des Xanthingerüstes isoliert ist und R_{b} eine Hydroxy-,C₁₋₃-Alkyloxy-, Mercapto-, C₁₋₃-Alkylsulfanyl-, C₁₋₃-Alkylsulfinyl-, C₁₋₃-Alkylsulfonyl-, Amino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl- oder 4-(C₁₋₃-Alkyl)-piperazin-1-ylgruppe bedeutet,
R² ein Wasserstoffatom,
eine C₁₋₈-Alkylgruppe,
eine C₂₋₆-Alkenylgruppe,
eine C₃₋₆-Alkinylgruppe,
eine durch eine Gruppe Rₐ substituierte C₁₋₆-Alkylgruppe, wobei Rₐ wie vorstehend erwähnt definiert ist,
eine Tetrahydrofuran-3-yl-, Tetrahydropyran-3-yl-, Tetrahydropyran-4-yl-, Tetrahydrofuranyl-C₁₋₃-alkyl- oder Tetrahydropyranyl-C₁₋₃-alkyl-gruppe,
eine durch eine Phenylgruppe substituierte C₁₋₆-Alkylgruppe, wobei der Phenylring durch die Gruppen R¹⁰ bis R¹⁴ substituiert ist und R¹⁰ bis R¹⁴ wie vorstehend erwähnt definiert sind,
eine durch die Gruppen R¹⁰ bis R¹⁴ substituierte Phenylgruppe, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyi-(CH₂)ₘ-A-(CH₂)ₙ-Gruppe, in der der Phenylteil durch R¹⁰ bis R¹⁴ substituiert ist, wobei R¹⁰ bis R¹⁴ , A, m und n wie vorstehend erwähnt definiert sind,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, B, m und n wie vorstehend erwähnt definiert sind,
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹, A und n wie vorstehend erwähnt definiert sind, eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴, m und D wie vorstehend erwähnt sind,
eine durch eine Gruppe R_{b} substituierte C₂₋₆-Alkylgruppe, wobei
R_{b} durch mindestens zwei Kohlenstoffatome vom Ringstickstoffatom in 3-Stellung des Xanthingerüstes isoliert ist und wie vorstehend erwähnt definiert ist,
oder eine C₃₋₆-Cycloalkylgruppe,
R³ eine durch die Gruppe R_{c} substituierte C₁₋₄-Alkylgruppe, wobei
R_{c} eine gegebenenfalls durch eine oder zwei C₁₋₃-Alkylgruppen substituierte C₃₋₇-Cycloalkylgruppe, oder
eine gegebenenfalls durch eine oder zwei C₁₋₃-Alkylgruppen substituierte C₅₋₇-Cycloalkenylgruppe, bedeutet,
eine C₃₋₈-Alkenylgruppe,
eine durch ein Fluor-, Chlor- oder Bromatom, oder eine Trifluormethylgruppe substituierte C₃₋₆-Alkenylgruppe,
eine C₃₋₈-Alkinylgruppe, oder
eine Aryl-C₂₋₄-alkenylgruppe,
und
R⁴ eine Azetidin-1-yl- oder Pyrrolidin-1-ylgruppe, die in 3-Stellung durch eine RₑNR_{d}-Gruppe substituiert ist und zusätzlich durch eine oder zwei C₁₋₃-Alkylgruppen substituiert sein kann, wobei
Rₑ ein Wasserstoffatom oder eine C₁₋₃-Alkylgruppe und
R_{d} ein Wasserstoffatom, eine C₁₋₃-Alkylgruppe, eine R_{f}-C₁₋₃-alkylgruppe oder eine Rg-C₂₋₃-alkylgruppe bedeutet, wobei
R_{f} eine Carboxy-, C₁₋₃-Alkyloxy-carbonyl-, Aminocarbonyl-, C₁₋₃-Alkylaminocarbonyl-, Di-(C₁₋₃-alkyl)-aminocarbonyl-, Pyrrolidin-1-yl-carbonyl-, 2-Cyan-pyrrolidin-1-yl-carbonyl-, 2-Carboxypyrrolidin-1-yl-carbonyl-, 2-Methoxycarbonylpyrrolidin-1-yl-carbonyl-, 2-Ethoxycarbonylpyrrolidin-1-yl-carbonyl-, 2-Aminocarbonylpyrrolidin-1-yl-carbonyl-, 4-Cyanthiazolidin-3-yl-carbonyl-, 4-Carboxythiazolidin-3-yl-carbonyl-, 4-Methoxycarbonylthiazolidin-3-yl-carbonyl-, 4-Ethoxycarbonylthiazolidin-3-yl-carbonyl-, 4-Aminocarbonylthiazolidin-3-yl-carbonyl-, Piperidin-1-yl-carbonyl-, Morpholin-4-yl-carbonyl-, Piperazin-1-yl-carbonyl-, 4-Methyl-piperazin-1-yl-carbonyl- oder 4-Ethyl-piperazin-1-yl-carbonyl-gruppe bedeutet und
R_{g}, das mindestens durch zwei Kohlenstoffatome vom Stickstoffatom der RₑNR_{d}-Gruppe getrennt ist, eine Hydroxy-, Methoxy- oder Ethoxygruppe bedeutet,
eine Piperidin-1-yl- oder Hexahydroazepin-1-ylgruppe, die in 3-Stellung oder in 4-Stellung durch eine RₑNR_{d}-Gruppe substituiert ist und zusätzlich durch eine oder zwei C₁₋₃-Alkylgruppen substituiert sein kann, wobei Rₑ und R_{d} wie vorstehend erwähnt definiert sind,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁-₃-Alkylamino- oder Di-(C₁-₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine R¹⁹-C₃₋₄-alkyl-gruppe, in der der C₃₋₄-Alkylteil geradkettig ist und durch den Rest R¹⁵ substituiert sein kann und zusätzlich durch eine oder zwei C₁₋₃-Alkylgruppen substituiert sein kann, wobei R¹⁵ eine C₁₋₆-Alkylgruppe, eine C₃₋₆-Cycloalkyl-, C₃₋₆-Cycloalkyl-C₁₋₃-alkyl, Aryl- oder Aryl-C₁₋₃-alkylgruppe und R¹⁹ eine Amino-, C₁₋₃-Alkylamino- oder Di-(C₁₋₃-alkyl)-aminogruppe darstellt,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino- oder Di-(C₁₋₃-alkyl)aminogruppe substituiert ist;
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1*H*-benzimidazolyl-, 2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 1,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl- oder 3,4-Dihydro-3-oxo-2H-benzo[1,4]oxazinyl-Gruppe zu verstehen ist,
wobei die vorstehend erwähnten Heteroarylgruppen durch R¹⁰ bis R¹⁴ substituiert sein können, wobei R¹⁰ bis R¹⁴ wie vorstehend erwähnt definiert sind,
wobei, soweit nichts anderes erwähnt wurde, die vorstehend erwähnten Alkyl-, Alkenyl- und Alkinylgruppen geradkettig oder verzweigt sein können,
oder die am Ringstickstoffatom in 9-Stellung des Xanthingerüstes N-oxidierten oder methylierten oder ethylierten Derivate,
oder die Derivate, in denen die 2-Oxo-, die 6-Oxo- oder die 2-Oxo- und die 6-Oxo-gruppe des Xanthingerüstes durch Thioxogruppen ersetzt sind,
deren Tautomere, Enantiomere, Diastereomere, deren Gemische oder deren Salze;
zur Verwendung in der Kombinationstherapie mit anderen Wirkstoffen.

2. Verbindungen zur Verwendung gemäß Anspruch 1, wobei Verbindungen der allgemeinen Formel I gemäß Anspruch 1, in der
R¹ eine C₁₋₆-Alkylgruppe,
eine C₃₋₆-Alkenylgruppe,
eine C₃₋₄-Alkenylgruppe, die durch eine C₁₋₂-Alkyloxy-carbonylgruppe substituiert ist,
eine C₃₋₆-Alkinylgruppe,
eineC₃₋₆-Cycloalkyl-C₁₋₃-alkyl-Gruppe,
eine Phenyl-C₁₋₄-alkyl-Gruppe, in der der Phenylteil durch R¹⁰ bis R¹² substituiert ist, wobei
R¹⁰ ein Wasserstoffatom, ein Fluor-, Chlor- oder Bromatom,
eine C₁₋₄-Alkyl-, Trifluormethyl-, Hydroxymethyl-, C₃₋₆-Cycloalkyl-, Ethinyl-oder Phenylgruppe,
eine Hydroxy-, C₁₋₄-Alkyloxy-, Difluormethoxy-, Trifluormethoxy-, 2,2,2-Tri-fluorethoxy-, Phenoxy-, Benzyloxy-, 2-Propen-1-yloxy-, 2-Propin-1-yloxy-, Cyan-C₁₋₂-alkyloxy-, C₁₋₂-Alkylsulfonyloxy-, Phenylsulfonyloxy-, Carboxy-C₁₋₃-alkyloxy-, C₁₋₃-Alkyloxy-carbonyl-C₁₋₃-alkyloxy-, Aminocarbonyl-C₁₋₃-alkyloxy-, C₁₋₂-Alkyl-aminocarbonyl-C₁₋₃-alkyloxy-, Di-(C₁₋₂-alkyl)aminocarbonyl-C₁₋₃-alkyloxy-, Pyrrolidin-1-yl-carbonyl-C₁₋₃-alkyloxy-, Piperidin-1-ylcarbonyl-C₁₋₃-alkyloxy-, Morpholin-4-ylcarbonyl-C₁₋₃-alkyloxy-, Methylsulfanylmethoxy-, Methylsulfinylmethoxy-, Methylsulfonylmethoxy-, C₃₋₆-Cycloalkyloxy- oder C₃₆-Cycloalkyl-C₁₋₂-alkyloxygruppe,
eine Carboxy-, C₁₋₃-Alkyloxycarbonyl-, Carboxy-C₁₋₃-alkyl-, C₁₋₃-Alkyloxy-carbonyl-C₁₋₃-alkyl-, Aminocarbonyl-, C₁₋₂-Alkylaminocarbonyl-, Di-(C₁₋₂-alkyl)aminocarbonyl-, Morpholin-4-ylcarbonyl- oder Cyanogruppe,
eine Nitro-, Amino-, C₁₋₂-Alkylamino-, Di-(C₁₋₂-alkyl)amino-, Cyan-C₁₋₂-alkylamino-, [N-(Cyan-C₁₋₂-alkyl)-N-C₁₋₂-alkyl-amino]-, C₁₋₂-Alkyloxy-carbonyl-C₁₋₂-alkylamino-, C₁₋₂-Alkyl-carbonylamino-, C₁₋₂-Alkyloxy-carbonylamino-, C₁-₃-Alkylsulfonylamino-, Bis-(C₁₋₂-alkylsulfonyl)-amino-, Aminosulfonylamino-, C₁₋₂-Alkylamino-sulfonylamino-, Di-(C₁₋₂-alkyl)amino-sulfonylamino-, Morpholin-4-yl-sulfonylamino-, (C₁₋₂-Alkylamino)thiocarbonylamino-, (C₁₋₂-Alkyloxy-carbonylamino)carbonylamino-, Aminocarbonylamino-, C₁₋₂-Alkyl-aminocarbonylamino-, Di-(C₁₋₂-alkyl)aminocarbonylamino- oder Morpholin-4-ylcarbonylamino-Gruppe,
eine 2-Oxo-imidazolidin-1-yl-, 3-Methyl-2-oxo-imidazolidin-1-yl-, 2,4-Dioxo-imidazolidin-1-yl-, 3-Methyl-2,4-dioxo-imidazolidin-1-yl-, 2,5-Dioxo-imidazolidin-1-yl-, 3-Methyl-2,5-dioxo-imidazolidin-1-yl-, 2-Oxo-hexahydro-pyrimidin-1-yl- oder 3-Methyl-2-oxo-hexahydropyrimidin-1-yl-Gruppe,
oder
eine C₁₋₂-Alkylsulfanyl-, C₁₋₂-Alkylsulfinyl-, C₁₋₂-Alkylsulfonyl-, Aminosulfonyl-, C₁₋₂-Alkylaminosulfonyl- oder Di-(C₁₋₂-alkyl)aminosulfonylgruppe,
und R¹¹ und R¹², die gleich oder verschieden sein können, ein Wasserstoff-, Fluor-, Chlor- oder Bromatom oder
eine Methyl-, Cyan-, Trifluormethyl- oder Methoxygruppe,
oder, R¹¹ zusammen mit R¹², sofern diese an benachbarte Kohlenstoffatome gebunden sind, auch eine Methylendioxy-, Difluormethylendioxy-, 1,3-Propylen- oder 1,4-Butylen-Gruppe bedeuten,
eine Phenyl-C₁₋₃-alkylgruppe, in der der Alkylteil durch eine Carboxy-, C₁₋₂-Alkyloxy-carbonyl-, Aminocarbonyl-, C₁₋₂-Alkylaminocarbonyl- oder Di-(C₁₋₂-alkyl)aminocarbonyl-Gruppe substituiert ist,
eine Phenyl-C₂₋₃-alkenylgruppe, wobei der Phenylteil durch ein Fluor-, Chlor- oder Bromatom oder durch eine Methyl-, Trifluormethyl- oder Methoxygruppe substituiert sein kann,
eine Phenyl-(CH₂)ₘ-A-(CH₂)ₙ-Gruppe, in der der Phenylteil durch R¹⁰ bis R¹² substituiert ist, wobei R¹⁰ bis R¹² wie vorstehend erwähnt definiert sind und
A eine Carbonyl-, Hydroxyiminomethylen- oder C₁₋₂-Alkyloxyiminomethylen-Gruppe, m die Zahl 0 oder 1 und n die Zahl 1 oder 2 bedeuten,
eine Phenylcarbonylmethylgruppe, in der der Phenylteil durch R¹⁰ bis R¹² substituiert ist, wobei R¹⁰ bis R¹² wie vorstehend erwähnt definiert sind und der Methylteil durch eine Methyl- oder Ethylgruppe substituiert ist,
eine Phenylcarbonylmethylgruppe, in der zwei benachbarte Wasserstoffatome des Phenylteiles durch eine -O-CO-NH-, -NH-CO-NH-, -N=CH-NH-, -N=CH-O- oder -O-CH₂-CO-NH- Brücke ersetzt sind, wobei die vorstehend erwähnten Brücken durch eine oder zwei Methylgruppen substituiert sein können,
eine Phenyl-(CH₂)ₘ-B-(CH₂)ₙ-Gruppe, in der der Phenylteil durch R¹⁰ bis R¹² substituiert ist, wobei R¹⁰ bis R¹², m und n wie vorstehend erwähnt definiert sind und
B eine Methylengruppe, die durch eine Hydroxy- oder C₁₋₂-Alkyloxygruppe substituiert ist und gegebenenfalls zusätzlich durch eine Methylgruppe substituiert ist, bedeutet,
eine Naphthylmethyl- oder Naphthylethylgruppe, wobei der Naphthylteil jeweils durch R¹⁰ bis R¹² substituiert ist, wobei R¹⁰ bis R¹² wie vorstehend erwähnt definiert sind,
eine Heteroaryl-C₁₋₃-alkylgruppe, wobei unter dem Begriff Heteroaryl eine Pyrrolyl-, Imidazolyl-, Triazolyl-, Furanyl-, Thienyl-, Oxazolyl-, Isoxazolyl-, Thiazolyl- Isothiazolyl-, Pyridyl-, Pyridazinyl-, Pyrimidinyl-, Pyrazinyl-, Indolyl-, Benzimidazolyl-, 2,3-Dihydro-2-oxo-1H-benzimidazolyl-, Indazolyl-, Benzofuranyl-, 2,3-Dihydro-benzofuranyl-, Benzoxazolyl-, 2,3-Dihydro-2-oxo-benzoxazolyl-, Benzoisoxazolyl-, Benzothiophenyl-, Benzothiazolyl-, Benzoisothiazolyl-, Chinolinyl-, 1,2-Dihydro-2-oxo-chinolinyl-, Isochinolinyl-, 1,2-Dihydro-1-oxo-isochinolinyl-, Cinnolinyl-, Chinazolinyl-, 1,2-Dihydro-2-oxo-chinazolinyl-, 1,2-Dihydro-1-oxo-phthalazin-4-yl-, Cumarinyl- oder 3,4-Dihydro-3-oxo-2H-benzo[1,4]oxazinyl-Gruppe zu verstehen ist,
wobei die vorstehend erwähnten Heteroarylgruppen an Kohlenstoffatomen durchein Fluor-, Chlor- oder Bromatom, durch eine Methyl- Trifluormethyl-, Cyan-, Aminocarbonyl-, Aminosulfonyl-, Methylsulfonyl-, Nitro-, Amino-, Acetylamino-, Methylsulfonylamino-, Methoxy-, Difluormethoxy- oder Trifluormethoxygruppe substituiert sein können und die Iminogruppen der vorstehend erwähnten Heteroarylgruppen durch Methyl- oder Ethylgruppen substituiert sein können,
eine Furanyl-A-CH₂-, Thienyl-A-CH₂-, Thiazolyl-A-CH₂- oder Pyridyl-A-CH₂-Gruppe, wobei A wie vorstehend erwähnt definiert ist,
eine Furanyl-B-CH₂-, Thienyl-B-CH₂-, Thiazolyl-B-CH₂- oder Pyridyl-B-CH₂-Gruppe, wobei B wie vorstehend erwähnt definiert ist,
eine C₁₋₄-Alkyl-A-(CH₂)ₙ-Gruppe, wobei A und n wie vorstehend erwähnt definiert sind,
eine C₃₋₆-Cycloalkyl-(CH₂)ₘ-A-(CH₂)ₙ-Gruppe, wobei A, m und n wie vorstehend erwähnt definiert sind,
eine C₃₋₆-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₂-Alkyloxycarbonyl-, Aminocarbonyl-, C₁₋₂-Alkylaminocarbonyl-, Di-(C₁₋₂-alkyl)aminocarbonyl-, Pyrrolidin-1-yl-carbonyl-, Piperidin-1-yl-carbonyl- oder Morpholin-4-yl-carbonyl-Gruppe bedeutet und A und n wie vorstehend erwähnt definiert sind,
eine Phenyl-D-C₁₋₃-alkylgruppe, in der der Phenylteil gegebenenfalls durch ein Fluor-, Chlor- oder Bromatom, eine Methyl-, Trifluormethyl- oder Methoxygruppe substituiert ist und D eine Sauerstoff- oder Schwefelatom, eine Sulfinyl- oder Sulfonylgruppe bedeutet,
eine durch eine Gruppe Rₐ substituierte C₁₋₄-Alkylgruppe, wobei
Rₐ eine Cyano-, Carboxy-, C₁₋₃-Alkyloxy-carbonyl-, Aminocarbonyl-, C₁₋₂-Alkyl-aminocarbonyl-, Di-(C₁₋₂-alkyl)aminocarbonyl-, Pyrrolidin-1-yl-carbonyl-, Piperidin-1-ylcarbonyl- oder Morpholin-4-ylcarbonyl-Gruppe bedeutet, oder
eine durch eine Gruppe R_{b} substituierte C₂₋₄-Alkylgruppe, wobei
R_{b} eine Hydroxy-, C₁₋₃-Alkyloxy-, Amino-, C₁₋₃-Alkylamino, Di-(C₁₋₃-alkyl)-amino-, Pyrrolidin-1-yl-, Piperidin-1-yl-, Morpholin-4-yl, Piperazin-1-yl-, 4-Methyl-piperazin-1-yl- oder 4-Ethyl-piperazin-1-yl-Gruppe darstellt und durch mindestens zwei Kohlenstoffatome vom Ringstickstoffatom in 1-Stellung des Xanthingerüstes isoliert ist,
R² ein Wasserstoffatom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetra-hydrofuranylmethyl- oder Tetrahydropyranylmethylgruppe,
eine Phenylgruppe, die gegebenenfalls durch ein Fluor-, Chlor- oder Bromatom oder durch eine Methyl-, Trifluormethyl-, Hydroxy-, Methoxy-, Difluormethoxy- oder Trifluormethoxygruppe substituiert ist,
eine Phenyl-C₁₋₄-alkylgruppe, in der der Phenylteil gegebenenfalls durch ein Fluor-, Chlor- oder Bromatom, eine Methyl-, Trifluormethyl-, Dimethylamino-, Hydroxy-, Methoxy-, Difluormethoxy- oder Trifluormethoxygruppe substituiert ist,
eine Phenyl-C₂₋₃-alkenylgruppe, wobei der Phenylteil durch ein Fluor-, Chlor- oder Bromatom oder durch eine Methyl-, Trifluormethyl- oder Methoxygruppe substituiert sein kann,
eine Phenylcarbonyl-C₁₋₂-alkylgruppe, in der der Phenylteil gegebenenfalls durch ein Fluor-, Chlor- oder Bromatom, eine Methyl-, Trifluormethyl-, Hydroxy-, Methoxy-, Difluormethoxy- oder Trifluormethoxygruppe substituiert ist,
eine Heteroaryl-C₁₋₃-alkylgruppe, wobei der Begriff Heteroaryl wie vorstehend erwähnt definiert ist,
eine Furanylcarbonylmethyl-, Thienylcarbonylmethyl-, Thiazolylcarbonylmethyl- oder Pyridylcarbonylmethylgruppe,
eine C₁₋₄-Alkyl-carbonyl-C₁₋₂-alkyl-Gruppe,
eine C₃₋₆-Cycloalkyl-carbonyl-C₁₋₂-alkyl-Gruppe,
eine Phenyl-D-C₁₋₃-alkylgruppe, in der der Phenylteil gegebenenfalls durch ein Fluor-, Chlor- oder Bromatom, eine Methyl-, Trifluormethyl-, Hydroxy-, Methoxy-, Difluormethoxy- oder Trifluormethoxygruppe substituiert ist, und D wie vorstehend erwähnt definiert ist,
eine durch eine Gruppe Rₐ substituierte C₁₋₄-Alkylgruppe, wobei Rₐ wie vorstehend erwähnt definiert ist oder
eine durch eine Gruppe R_{b} substituierte C₂₋₄-Alkylgruppe, wobei R_{b} wie vorstehend erwähnt definiert ist und durch mindestens zwei Kohlenstoffatome vom Ringstickstoffatom in 3-Stellung des Xanthingerüstes isoliert ist,
R³ eine C₃₋₇-Alkenylgruppe,
eine C₃₋₅-Alkenylgruppe, die durch ein Fluor- Chlor- oder Bromatom oder eine Trifluormethylgruppe substituiert ist,
eine C₃₋₆-Alkinylgruppe,
eine durch die Gruppe R_{c} substituierte C₁₋₃-Alkylgruppe, wobei
R_{c} eine gegebenenfalls durch eine oder zwei Methylgruppen substituierte C₃-₆-Cycloalkylgruppe, oder
eine gegebenenfalls durch eine oder zwei Methylgruppen substituierte C₅₋₆-Cycloalkenylgruppe, bedeutet,
oder eine Phenyl-C₂₋₃-alkenyl-gruppe
und
R⁴ eine Pyrrolidin-1-ylgruppe, die in 3-Stellung durch eine Amino-, Methylamino- oder Dimethylaminogruppe substituiert ist,
eine Azetidin-1-ylgruppe, die durch eine Aminomethylgruppe substituiert ist,
eine Pyrrolidin-1-ylgruppe, die durch eine Aminomethylgruppe substituiert ist,
eine Piperidin-1-ylgruppe, die in 3-Stellung oder in 4-Stellung durch eine Amino-, Methylamino-, Dimethylamino- oder [(2-Cyan-pyrrolidin-1-yl-)carbonylmethyl]-aminogruppe substituiert ist, wobei der Piperidin-1-yl-Teil zusätzlich durch eine Methyl- oder Ethylgruppe substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine 3-Amino-piperidin-1-ylgruppe, in der ein Wasserstoffatom in 2-Stellung zusammen mit einem Wasserstoffatom in 6-Stellung durch eine -CH₂-CH₂-Brücke ersetzt ist,
eine 3-Amino-piperidin-1-ylgruppe, in der ein Wasserstoffatom in 4-Stellung zusammen mit einem Wasserstoffatom in 6-Stellung durch eine -CH₂-CH₂-Brücke ersetzt ist,
eine Piperidin-1-ylgruppe, die durch eine Aminomethylgruppe substituiert ist,
eine Piperidin-3-yl- oder Piperidin-4-ylgruppe,
eine Piperidin-3-yl- oder Piperidin-4-ylgruppe, die in 1-Stellung durch eine Aminogruppe substituiert ist,
eine Hexahydroazepin-1-yl-gruppe, die in 3-Stellung oder in 4-Stellung durch eine Aminogruppe substituiert ist,
eine 3-Amino-propyl-, 3-Methylamino-propyl- oder 3-Dimethylamino-propylgruppe, in der der Propylteil durch eine oder zwei Methylgruppen substituiert sein kann,
eine 4-Amino-butyl-, 4-Methylamino-butyl- oder 4-Dimethylamino-butylgruppe, in der der Butylteil durch eine oder zwei Methylgruppen substituiert sein kann,
eineC₁₋₂-Alkylgruppe, die durch eine 2-Pyrrolidinyl-, 3-Pyrrolidinyl-, 2-Piperidinyl-, 3-Piperidinyl- oder 4-Piperidinylgruppe substituiert ist,
eine 3-Amino-2-oxo-piperidin-5-yl- oder 3-Amino-2-oxo-1-methyl-piperidin-5-yl-Gruppe,
eine C₃₋₆-Cycloalkylgruppe, die durch eine Amino-, Aminomethyl- oder Aminoethylgruppe substituiert ist oder
eine C₃₋₆-Cycloalkyl-C₁₋₂-alkylgruppe, in der der Cycloalkylteil durch eine Amino-, Aminomethyl- oder Aminoethylgruppe substituiert ist, bedeuten,
wobei soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische oder deren Salze;
verwendet werden.

3. Verbindungen zur Verwendung gemäß Anspruch 1, wobei Verbindungen der allgemeinen Formel 1 gemäß Anspruch 1, in der
R¹ wie in Anspruch 1 oder 2 definiert ist, und
R² ein Wasserstoffatom,
eine C₁₋₆-Alkylgruppe,
eine Ethenylgruppe,
eine 2-Propen-1-yl- oder 2-Propin-1-ylgruppe,
eine Phenylgruppe,
eine Phenyl-C₁₋₄-alkylgruppe, wobei der Phenylteil durch ein Fluoratom, eine Methyl- oder Methoxygruppe substituiert sein kann,
eine Phenylcarbonylmethylgruppe,
eine 2-Phenylethenylgruppe,
eine Methylgruppe, die durch eine Cyclopropyl-, Cyan-, Carboxy- oder Methoxycarbonylgruppe substituiert ist, oder
eine Ethylgruppe, die in 2-Stellung durch eine Cyan-, Hydroxy-, Methoxy- oder Dimethylaminogruppe substituiert ist,
R³ eine C₄₋₆-Alkenylgruppe,
eine 1-Cyclopenten-1-ylmethyl- oder 1-Cyclohexen-1-ylmethylgruppe,
eine 2-Propin-1-yl-, 2-Butin-1-yl- oder 2-Pentin-1-ylgruppe,
eine 2-Phenylethenylgruppe, oder
eine Cyclopropylmethylgruppe, und
R⁴ eine Pyrrolidin-1-ylgruppe, die in 3-Stellung durch eine Aminogruppe substituiert ist,
eine Azetidin-1-ylgruppe, die durch eine Aminomethylgruppe substituiert ist,
eine Pyrrolidin-1-ylgruppe, die durch eine Aminomethylgruppe substituiert ist,
eine Piperidin-1-ylgruppe, die in 3-Stellung oder in 4-Stellung durch eine Amino-, Methylamino-, Dimethylamino- oder [(2-Cyan-pyrrolidin-1-yl)carbonylmethyl]-aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Pyrrolidin-1-yl-carbonylgruppe substituiert ist,
eine 3-Amino-piperidin-1-ylgruppe, in der der Piperidin-1-yl-Teil in 4-Stellung zusätzlich durch eine Hydroxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Piperidin-1-ylgruppe, die durch eine Aminomethylgruppe substituiert ist,
eine Piperidin-3-yl- oder Piperidin-4-yl-gruppe,
eine 1-Amino-piperidin-3-yl- oder 1-Amino-piperidin-4-ylgruppe,
eine Hexahydroazepin-1-yl-gruppe, die in 3-Stellung oder in 4-Stellung durch eine Aminogruppe substituiert ist,
eine 3-Aminopropylgruppe, oder
eine Cyclohexylgruppe, die durch eine Aminogruppe substituiert ist, bedeuten,
wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische oder deren Salze; verwendet werden.

4. Verbindungen zur Verwendung gemäß Anspruch 1, wobei Verbindungen der allgemeinen Formel I gemäß Anspruch 1, in der
R¹, R² und R³ wie in Anspruch 1, 2 oder 3 erwähnt definiert sind, und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Pyrrolidin-1-yl-carbonylgruppe substituiert ist,
eine 3-Amino-piperidin-1-ylgruppe, in der der Piperidin-1-yl-Teil in 4-Stellung zusätzlich durch eine Hydroxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist, oder
eine Cyclohexylgruppe, die in 3-Stellung durch eine Aminogruppe substituiert ist, bedeutet,
wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische oder deren Salze; verwendet werden.

5. Verbindungen zur Verwendung gemäß Anspruch 1, wobei Verbindungen der allgemeinen Formel I gemäß Anspruch 1, in der
R¹, R³ und R⁴ wie in Anspruch 1, 2, 3 oder 4 erwähnt definiert sind, und
R² ein Wasserstoffatom oder eine C₁₋₈-Alkylgruppe darstellt,
wobei soweit nichts anderes erwähnt wurde, die vorstehend erwähnte Alkylgruppe geradkettig oder verzweigt sein kann,
deren Tautomere, Enantiomere, Diastereomere, deren Gemische oder deren Salze; verwendet werden.

6. Verbindungen zur Verwendung gemäß Anspruch 1, wobei Verbindungen der allgemeinen Formel I gemäß Anspruch 1, in der
R¹, R³ und R⁴ wie in Anspruch 1, 2, 3 oder 4 erwähnt definiert sind, und
R² eine C₁₋₈-Alkylgruppe darstellt,
wobei soweit nichts anderes erwähnt wurde, die vorstehend erwähnte Alkylgruppe geradkettig oder verzweigt sein kann,
deren Tautomere, Enantiomere, Diastereomere, deren Gemische oder deren Salze; verwendet werden.

7. Verbindungen der allgemeinen Formel I gemäß Anspruch 1, in der R¹ eine C₁₋₆-Alkylgruppe,
eine C₃₋₆-Alkenylgruppe,
eine C₃₋₄-Alkenylgruppe, die durch eine C₁₋₂-Alkyloxy-carbonylgruppe substituiert ist, eine C₃₋₆-Alkinylgruppe,
eine C₃₋₆-Cycloalkyl-C₁₋₃-alkyl-Gruppe,
eine Phenyl-C₁₋₄-alkyl-Gruppe, in der der Phenylteil durch R¹⁰ bis R¹² substituiert ist, wobei
R¹⁰ ein Wasserstoffatom, ein Fluor-, Chlor- oder Bromatom,
eine C₁₋₄-Alkyl-, Trifluormethyl-, Hydroxymethyl-, C₃₋₆-Cycloalkyl-, Ethinyl-oder Phenylgruppe,
eine Hydroxy-, C₁₋₄-Alkyloxy-, Difluormethoxy-, Trifluormethoxy-, 2,2,2-Trifluorethoxy-, Phenoxy-, Benzyloxy-, 2-Propen-1-yloxy-, 2-Propin-1-yloxy-, Cyan-C₁₋₂-alkyloxy-, C₁₋₂-Alkylsulfonyloxy-, Phenylsulfonyloxy-, Carboxy-C₁₋₃-alkyloxy-, C₁₋₃-Alkyloxy-carbonyl-C₁₋₃-alkyloxy-, Aminocarbonyl-C₁₋₃-alkyloxy-, C₁₋₂-Alkyl-aminocarbonyl-C₁₋₃-alkyloxy-, Di-(C₁₋₂-alkyl)aminocarbonyl-C₁₋₃-alkyloxy-, Pyrrolidin-1-yl-carbonyl-C₁₋₃-alkyloxy-, Piperidin-1-ylcarbonyl-C₁₋₃-alkyloxy-, Morpholin-4-ylcarbonyl-C₁₋₃-alkyloxy-, Methylsulfanylmethoxy-, Methylsulfinylmethoxy-, Methylsulfonylmethoxy-, C₃₋₆-Cycloalkyloxy- oder C₃₋₆-Cycloalkyl-C₁₋₂-alkyloxygruppe,
eine Carboxy-, C₁₋₃-Alkyloxycarbonyl-, Carboxy-C₁₋₃-alkyl-, C₁₋₃-Alkyloxy-carbonyl-C₁₋₃-alkyl-, Aminocarbonyl-, C₁₋₂-Alkylaminocarbonyl-, Di-(C₁₋₂-alkyl)aminocarbonyl-, Morpholin-4-ylcarbonyl-oder Cyanogruppe,
eine Nitro-, Amino-, C₁₋₂-Alkylamino-, Di-(C₁₋₂-alkyl)amino-, Cyan-C₁₋₂-alkyl-amino-, [N-(Cyan-C₁₋₂-alkyl)-N-C₁₋₂-alkyl-amino]-, C₁₋₂-Alkyloxy-carbonyl-C₁₋₂-alkylamino-,-C₁₋₂-Alkyl-carbonylamino-, C₁₋₂-Alkyloxy-carbonylamino-, C₁₋₃-Alkylsulfonylamino-, Bis-(C₁₋₂-alkylsulfonyl)-amino-, Aminosulfonylamino-, C₁₋₂-Alkylamino-sulfonylamino-, Di-(C₁₋₂-alkyl)amino-sulfonylamino-, Morpholin-4-yl-sulfonylamino-, (C₁₋₂-Alkylamino)thiocarbonylamino-, (C₁₋₂-Alkyloxy-carbonylamino)carbonylamino-, Aminocarbonylamino-, C₁₋₂-Alkyl-aminocarbonylamino-, Di-(Ci-2-alkyl)aminocarbonylamino- oder Morpholin-4-ylcarbonylamino-Gruppe,
eine 2-Oxo-imidazolidin-1-yl-, 3-Methyl-2-oxo-imidazolidin-1-yl-, 2,4-Dioxo-imidazolidin-1-yl-, 3-Methyl-2,4-dioxo-imidazolidin-1-yl-, 2,5-Dioxo-imidazo-lidin-1-yl-, 3-Methyl-2,5-dioxo-imidazolidin-1-yl-, 2-Oxo-hexahydropyrimidin-1-yl- oder 3-Methyl-2-oxo-hexahydropyrimidin-1-yl-Gruppe,
oder
eine C₁₋₂-Alkylsulfanyl-, C₁₋₂-Alkylsulfinyl-, C₁₋₂-Alkylsulfonyl-, Aminosulfonyl-, C₁₋₂-Alkylaminosulfonyl- oder Di-(C₁₋₂-alkyl)aminosulfonylgruppe,
und R¹¹ und R¹², die gleich oder verschieden sein können, ein Wasserstoff-, Fluor-, Chlor- oder Bromatom oder
eine Methyl-, Cyan-, Trifluormethyl- oder Methoxygruppe,
oder, R¹¹ zusammen mit R¹², sofern diese an benachbarte Kohlenstoffatome gebunden sind, auch eine Methylendioxy-, Difluormethylendioxy-, 1,3-Propylen- oder 1,4-Butylen-Gruppe bedeuten,
eine Phenyl-C₁₋₃-alkylgruppe, in der der Alkylteil durch eine Carboxy-, C₁₋₂-Alkyloxy-carbonyl-, Aminocarbonyl-, C₁₋₂-Alkylaminocarbonyl- oder Di-(C₁₋₂-alkyl)aminocarbonyl-Gruppe substituiert ist,
eine Phenyl-C₂₋₃-alkenylgruppe, wobei der Phenylteil durch ein Fluor-, Chlor- oder Bromatom oder durch eine Methyl-, Trifluormethyl- oder Methoxygruppe substituiert sein kann,
eine Phenyl-(CH₂)ₘ-A-(CH₂)ₙ-Gruppe, in der der Phenylteil durch R¹⁰ bis R¹² substituiert ist, wobei R¹⁰ bis R¹² wie vorstehend erwähnt definiert sind und
A eine Carbonyl-, Hydroxyiminomethylen- oder C₁₋₂-Alkyloxyiminomethylen-Gruppe, m die Zahl 0 oder 1 und n die Zahl 1 oder 2 bedeuten,
eine Phenylcarbonylmethylgruppe, in der der Phenylteil durch R¹⁰ bis R¹² substituiert ist, wobei R¹⁰ bis R¹² wie vorstehend erwähnt definiert sind und der Methylteil durch eine Methyl- oder Ethylgruppe substituiert ist,
eine Phenylcarbonylmethylgruppe, in der zwei benachbarte Wasserstoffatome des Phenylteiles durch eine -O-CO-NH-, -NH-CO-NH-, -N=CH-NH-, -N=CH-O- oder -O-CH₂-CO-NH- Brücke ersetzt sind, wobei die vorstehend erwähnten Brücken durch eine oder zwei Methylgruppen substituiert sein können,
eine Phenyl-(CH₂)ₘ-B-(CH₂)ₙ-Gruppe, in der der Phenylteil durch R¹⁰ bis R¹² substituiert ist, wobei R¹⁰ bis R¹², m und n wie vorstehend erwähnt definiert sind und
B eine Methylengruppe, die durch eine Hydroxy- oder C₁₋₂-Alkyloxygruppe substituiert ist und gegebenenfalls zusätzlich durch eine Methylgruppe substituiert ist, bedeutet,
eine Naphthylmethyl- oder Naphthylethylgruppe, wobei der Naphthylteil jeweils durch R¹⁰ bis R¹² substituiert ist, wobei R¹⁰ bis R¹² wie vorstehend erwähnt definiert sind,
eine Heteroaryl-C₁₋₃-alkylgruppe, wobei unter dem Begriff Heteroaryl eine Pyrrolyl-, Imidazolyl-, Triazolyl-, Furanyl-, Thienyl-, Oxazolyl-, Isoxazolyl-, Thiazolyl-, Isothia-zolyl-, Pyridyl-, Pyridazinyl-, Pyrimidinyl-, Pyrazinyl-, Indolyl-, Benzimidazolyl-, 2,3-Dihydro-2-oxo-1H-benzimidazolyl-, Indazolyl-, Benzofuranyl-, 2,3-Dihydrobenzo-furanyl-, Benzoxazolyl-, Dihydro-2-oxo-benzoxazolyl-, Benzoisoxazolyl-, Benzothiophenyl-, Benzothiazolyl-, Benzoisothiazolyl-, Chinolinyl-, 1,2-Dihydro-2-oxo-chinolinyl-, Isochinolinyl-, 1,2-Dihydro-1-oxo-isochinolinyl-, Cinnolinyl-, Chinazolinyl-, 1,2-Dihydro-2-oxo-chinazolinyl-, 1,2-Dihydro-1-oxo-phthalazin-4-yl-, Cumarinyl- oder 3,4-Dihydro-3-oxo-2H-benzo[1,4]oxazinyl-Gruppe zu verstehen ist,
wobei die vorstehend erwähnten Heteroarylgruppen an Kohlenstoffatomen durch ein Fluor-, Chlor- oder Bromatom, durch eine Methyl- Trifluormethyl-, Cyan-, Aminocarbonyl-, Aminosulfonyl-, Methylsulfonyl-, Nitro-, Amino-, Acetylamino-, Methylsulfonylamino-, Methoxy-, Difluormethoxy- oder Trifluormethoxygruppe substituiert sein können und die Iminogruppen der vorstehend erwähnten Heteroarylgruppen durch Methyl- oder Ethylgruppen substituiert sein können,
eine Furanyl-A-CH₂-, Thienyl-A-CH₂-, Thiazolyl-A-CH₂- oder Pyridyl-A-CH₂-Gruppe, wobei A wie vorstehend erwähnt definiert ist,
eine Furanyl-B-CH₂-, Thienyl-B-CH₂-, Thiazolyl-B-CH₂- oder Pyridyl-B-CH₂-Gruppe, wobei B wie vorstehend erwähnt definiert ist,
eine C₁₋₄-Alkyl-A-(CH₂)ₙ-Gruppe, wobei A und n wie vorstehend erwähnt definiert sind,
eine C₃₋₆-Cycloalkyl-(CH₂)ₘ-A-(CH₂)ₙ-Gruppe, wobei A, m und n wie vorstehend erwähnt definiert sind,
eine C₃₋₆-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₂-Alkyloxycarbonyl-, Aminocarbonyl-, C₁₋₂-Alkylaminocarbonyl-, Di-(C₁₋₂-alkyl)aminocarbonyl-, Pyrrolidin-1-yl-carbonyl-, Piperidin-1-yl-carbonyl- oder Morpholin-4-yl-carbonyl-Gruppe bedeutet und A und n wie vorstehend erwähnt definiert sind,
eine Phenyl-D-C₁₋₃-alkylgruppe, in der der Phenylteil gegebenenfalls durch ein Fluor-, Chlor- oder Bromatom, eine Methyl-, Trifluormethyl- oder Methoxygruppe substituiert ist und D eine Sauerstoff- oder Schwefelatom, eine Sulfinyl- oder Sulfonyl-gruppe bedeutet,
eine durch eine Gruppe Rₐ substituierte C₁₋₄-Alkylgruppe, wobei
Rₐ eine Cyano-, Carboxy-, C₁₋₃-Alkyloxy-carbonyl-, Aminocarbonyl-, C₁₋₂-Alkyl-aminocarbonyl-, Di-(C₁₋₂-alkyl)aminocarbonyl-, Pyrrolidin-1-yl-carbonyl-, Piperidin-1-ylcarbonyl- oder Morpholin-4-ylcarbonyl-Gruppe bedeutet, oder
eine durch eine Gruppe R_{b} substituierte C₂₋₄-Alkylgruppe, wobei
R_{b} eine Hydroxy-, C₁₋₃-Alkyloxy-, Amino-, C₁₋₃-Alkylamino-, Di-(C₁₋₃-alkyl)-amino-, Pyrrolidin-1-yl-, Piperidin-1-yl-, Morpholin-4-yl, Piperazin-1-yl-, 4-Methyl-piperazin-1-yl- oder 4-Ethyl-piperazin-1-yl-Gruppe darstellt und durch mindestens zwei Kohlenstoffatome vom Ringstickstoffatom in 1-Stellung des Xanthingerüstes isoliert ist,
R² ein Wasserstoffatom,
eine C₁₋₆-Alkylgruppe,
eine C₂₋₄-Alkenylgruppe,
eine C₃₋₄-Alkinylgruppe,
eine C₃₋₆-Cycloalkylgruppe,
eine C₃₋₆-Cycloalkyl-C₁₋₃-alkylgruppe,
eine Tetrahydrofuran-3-yl-, Tetrahydropyran-3-yl-, Tetrahydropyran-4-yl-, Tetra-hydrofuranylmethyl- oder Tetrahydropyranylmethylgruppe,
eine Phenylgruppe, die gegebenenfalls durch ein Fluor-, Chlor- oder Bromatom oder durch eine Methyl-, Trifluormethyl-, Hydroxy-, Methoxy-, Difluormethoxy- oder Trifluormethoxygruppe substituiert ist,
eine Phenyl-C₁₋₄-alkylgruppe, in der der Phenylteil gegebenenfalls durch ein Fluor-, Chlor- oder Bromatom, eine Methyl-, Trifluormethyl-, Dimethylamino-, Hydroxy-, Methoxy-, Difluormethoxy- oder Trifluormethoxygruppe substituiert ist,
eine Phenyl-C₂₋₃-alkenylgruppe, wobei der Phenylteil durch ein Fluor-, Chlor- oder Bromatom oder durch eine Methyl-, Trifluormethyl- oder Methoxygruppe substituiert sein kann,
eine Phenylcarbonyl-C₁₋₂-alkylgruppe, in der der Phenylteil gegebenenfalls durch ein Fluor-, Chlor- oder Bromatom, eine Methyl-, Trifluormethyl-, Hydroxy-, Methoxy-, Difluormethoxy- oder Trifluormethoxygruppe substituiert ist,
eine Heteroaryl-C₁₋₃-alkylgruppe, wobei der Begriff Heteroaryl wie vorstehend erwähnt definiert ist,
eine Furanylcarbonylmethyl-, Thienylcarbonylmethyl-, Thiazolylcarbonylmethyl- oder Pyridylcarbonylmethylgruppe,
eine C₁₋₄-Alkyl-carbonyl-C₁₋₂-alkyl-Gruppe,
eine C₃₋₆-Cycloalkyl-carbonyl-C₁₋₂-alkyl-Gruppe,
eine Phenyl-D-C₁₋₃-alkylgruppe, in der der Phenylteil gegebenenfalls durch ein Fluor-, Chlor- oder Bromatom, eine Methyl-, Trifluormethyl-, Hydroxy-, Methoxy-, Difluormethoxy- oder Trifluormethoxygruppe substituiert ist, und D wie vorstehend erwähnt definiert ist, oder
eine durch eine Gruppe Rₐ substituierte C₁₋₄-Alkylgruppe, wobei Rₐ wie vorstehend erwähnt definiert ist, oder
eine durch eine Gruppe R_{b} substituierte C₂₋₄-Alkylgruppe, wobei R_{b} wie vorstehend erwähnt definiert ist und durch mindestens zwei Kohlenstoffatome vom Ringstickstoffatom in 3-Stellung des Xanthingerüstes isoliert ist,
R³ eine durch die Gruppe R_{c} substituierte C₁₋₃-Alkylgruppe, wobei
R_{c} eine gegebenenfalls durch eine oder zwei C₁₋₃-Alkylgruppen substituierte C₃₋₇-Cycloalkylgruppe, oder
eine gegebenenfalls durch eine oder zwei C₁₋₃-Alkylgruppen substituierte C₅₋₇-Cycloalkenylgruppe, bedeutet,
eine C₃₋₈-Alkenylgruppe,
eine durch ein Fluor-, Chlor- oder Bromatom, oder eine Trifluormethylgruppe substituierte C₃₋₆-Alkenylgruppe,
eine C₃₋₈-Alkinylgruppe, oder
eine Aryl-C₂₋₄-alkenylgruppe,
und
R⁴ eine Azetidin-1-yl- oder Pyrrolidin-1-ylgruppe, die in 3-Stellung durch eine RₑNR_{d}-Gruppe substituiert ist und zusätzlich durch eine oder zwei C₁₋₃-Alkylgruppen substituiert sein kann, wobei
Rₑ ein Wasserstoffatom oder eine C₁₋₃-Alkylgruppe und
R_{d} ein Wasserstoffatom oder eine C₁₋₃-Alkylgruppe bedeutet,
eine Piperidin-1-yl- oder Hexahydroazepin-1-ylgruppe, die in 3-Stellung oder in 4-Stellung durch eine RₑNR_{d}-Gruppe substituiert ist und zusätzlich durch eine oder zwei C₁₋₃-Alkylgruppen substituiert sein kann, wobei Rₑ und R_{d} wie vorstehend erwähnt definiert sind,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-amino-gruppe substituierte Piperidin-1-yl- oder Hexahydroazepin-1-yl-gruppe, in denen jeweils zwei Wasserstoffatome am Kohlenstoffgerüst der Piperidin-1-yl- oder Hexa-hydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl-oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine R¹⁹-C₃₋₄-alkyl-gruppe, in der der C₃₋₄-Alkylteil geradkettig ist und durch den Rest R¹⁵ substituiert sein kann und zusätzlich durch eine oder zwei C₁₋₃-Alkylgruppen substituiert sein kann, wobei R¹⁵ eine C₁₋₃-Alkylgruppe und R¹⁹ eine Amino-, C₁₋₃-Alkylamino- oder Di-(C₁₋₃-alkyl)-aminogruppe darstellt,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino-oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt und
wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische oder deren Salze;
zur Verwendung in der Kombinationstherapie mit anderen Wirkstoffen, z.B. ausgewählt aus der Gruppe der Antidiabetika, Lipidsenker, HDL-erhöhende Verbindungen, Wirkstoffe zur Behandlung von Obesitas, und Wirkstoffe zur Beeinflussung des Bluthochdrucks.

8. Verbindungen zur Verwendung gemäß Anspruch 7, wobei Verbindungen der allgemeinen Formel I gemäß Anspruch 1, in der
R¹ eine C₁₋₄-Alkylgruppe,
eine C₃₋₅-Alkenylgruppe,
eine 2-Propen-1-ylgruppe, die durch eine Methoxycarbonylgruppe substituiert ist, eine C₃₋₅-Alkinylgruppe,
eine Phenyl-C₁₋₄-alkyl-Gruppe, in der der Phenylteil durch R¹⁰ bis R¹² substituiert ist, wobei
R¹⁰ ein Wasserstoffatom, ein Fluor-, Chlor- oder Bromatom,
eine Methyl-, Ethyl-, Trifluormethyl-, oder Ethinylgruppe,
eine Hydroxy-, Methoxy-, Ethoxy-, Difluormethoxy-, Trifluormethoxy-, 2,2,2-Trifluorethoxy-, Phenoxy-, Benzyloxy-, 2-Propen-1-yloxy-, 2-Propin-1-yloxy-, Cyan-C₁₋₂-alkyloxy-, C₁₋₂-Alkyl-sulfonyloxy-, Phenylsulfonyloxy-, Carboxy-C₁₋₂-alkyloxy-, C₁₋₂-Alkyloxy-carbonyl-C₁₋₂-alkyloxy-, Aminocarbonyl-C₁₋₂-alkyloxy-, C₁₋₂-Alkyl-aminocarbonyl-C₁₋₂-alkyloxy-, Di-(C₁₋₂-alkyl)aminocarbonyl-C₁₋₂-alkyloxy-, Pyrrolidin-1-ylcarbonyl-C₁₋₂-alkyloxy-, Piperidin-1-ylcarbonyl-C₁₋₂-alkyloxy-, Morpholin-4-ylcarbonyl-C₁₋₂-alkyloxy-gruppe,
eine Carboxy-, C₁₋₂-Alkyloxy-carbonyl-, Aminocarbonyl-, C₁₋₂-Alkylamino-carbonyl-, Di-(C₁₋₂-alkyl)aminocarbonyl-, Morpholin-4-ylcarbonyl- oder Cyanogruppe,
eine Nitro-, Amino-, C₁₋₂-Alkylamino-, Di-(C₁₋₂-alkyl)amino-, Cyan-C₁₋₂-alkylamino-, [N-(Cyan-C₁₋₂-alkyl)-N-methyl-amino]-, C₁₋₂-Alkyloxy-carbonyl-C₁₋₂-alkylamino-, C₁₋₂-Alkyl-carbonylamino-, C₁₋₂-Alkyloxy-carbonylamino-, C₁₋₂-Alkylsulfonylamino-, Bis-(C₁₋₂-alkylsulfonyl)-amino-, Aminosulfonylamino-, C₁₂-Alkylamino-sulfonylamino-, Di-(C₁₋₂-alkyl)amino-sulfonylamino-, Morpholin-4-yl-sulfonylamino-, (C₁₋₂-Alkylamino)thiocarbonylamino-, (C₁₋₂-Alkyloxy-carbon-ylamino)carbonylamino-, Aminocarbonylamino-, C₁₋₂-Alkylaminocarbonyl-amino-, Di-(C₁₋₂-alkyl)aminocarbonylamino- oder Morpholin-4-yl-carbonyl-amino-Gruppe,
eine 2-Oxo-imidazolidin-1-yl-, 3-Methyl-2-oxo-imidazolidin-1-yl-, 2,4-Dioxo-imidazolidin-1-yl-, 3-Methyl-2,4-dioxo-imidazolidin-1-yl-, 2,5-Dioxo-imida-zolidin-1-yl-, 3-Methyl-2,5-dioxo-imidazolidin-1-yl-, 2-Oxo-hexahydropyrimidin-1-yl- oder 3-Methyl-2-oxo-hexahydropyrimidin-1-yl-Gruppe,
oder
eine C₁₋₂-Alkylsulfanyl-, C₁₋₂-Alkylsulfinyl-, C₁₋₂-Alkylsulfonyl-, Aminosulfonyl-, C₁₋₂-Alkylaminosulfonyl- oder Di-(C₁₋₂-alkyl)aminosulfonylgruppe,
und R¹¹ und R¹², die gleich oder verschieden sein können, ein Wasserstoff-, Fluor-, Chlor- oder Bromatom oder
eine Methyl-, Cyan- oder Methoxygruppe,
oder, R¹¹ zusammen mit R¹², sofern diese an benachbarte Kohlenstoffatome gebunden sind, auch eine Methylendioxy-Gruppe bedeuten,
eine Phenylmethylgruppe, in der der Methylteil durch eine Carboxy-, Methoxycarbonyl- oder Aminocarbonylgruppe substituiert ist,
eine 2-Phenylethylgruppe, in der der Ethylteil durch eine Carboxy-, Methoxycarbonyl- oder Aminocarbonylgruppe substituiert ist,
eine 2-Phenylethylgruppe, in der der Ethylteil in 2-Stellung durch eine Hydroxy-, Methoxy-, Hydroxyimino- oder Methoxyiminogruppe substituiert ist,
eine 2-Phenylethylgruppe, in der der Ethylteil in 2-Stellung durch eine Hydroxygruppe und eine Methylgruppe substituiert ist,
eine Phenylcarbonylmethylgruppe, in der der Phenylteil durch R¹⁰ bis R¹² substituiert ist, wobei R¹⁰ bis R¹² wie vorstehend erwähnt definiert sind,
eine 1-(Phenylcarbonyl)ethyl- oder 2-(Phenylcarbonyl)ethylgruppe,
eine 2-Phenylethenylgruppe,
eine Phenylsulfanylmethyl- oder Phenylsulfinylmethylgruppe,
eine 2-(Phenyloxy)ethylgruppe,
eine Naphthylmethyl- oder Naphthylethylgruppe, wobei der Naphthylteil jeweils durch eine Methyl-, Nitro-, Amino-, Acetylamino-, Methylsulfonylamino-, Cyan-, Aminocarbonyl- oder Aminosulfonylgruppe substituiert sein kann,
eine Oxazolylmethyl-, Isoxazolylmethyl-, Thiazolylmethyl-, Pyridylmethyl-, Benzofuranylmethyl-, 2,3-Dihydrobenzofuranylmethyl-, Benzo[d]isoxazolylmethyl-, Benzo[d]isothiazolylmethyl-, (1H-Indazol-3-yl)methyl-, Chinolinylmethyl-, (1,2-Dihydro-2-oxo-chinolin-4-yl)methyl-, Isochinolinylmethyl-, (1,2-Dihydro-1-oxo-isochinolin-4-yl)methyl-, Cinnolinylmethyl-, Chinazolinylmethyl-, (1,2-Dihydro-2-oxo-chinazolin-4-yl)methyl-, (1,2-Dihydro-1-oxo-phthalazin-4-yl)methyl- oder Cumarinyl-methyl-Gruppe, wobei der heterocyclische Teil jeweils durch eine Methylgruppe substituiert sein kann,
eine Chinolinylmethyl- oder Isochinolinylmethylgruppe, wobei der heterocyclische Teil jeweils durch eine Cyan-, Nitro-, Amino-, Acetylamino-, Methylsulfonylamino-, Aminocarbonyl- oder Aminosulfonylgruppe substituiert ist,
eine Pyrrolylethyl-, Triazolylethyl-, Thienylethyl-, Thiazolylethyl- oder Pyridylethylgruppe, wobei der heterocyclische Teil jeweils durch eine Methylgruppe substituiert sein kann,
eine Furanylcarbonylmethyl-, Thienylcarbonylmethyl-, Thiazolylcarbonylmethyl- oder Pyridylcarbonylmethylgruppe,
eine Methylgruppe, die durch eine Cyclopropyl-, Cyan-, Carboxy-, Aminocarbonyl- oder Methoxycarbonylgruppe substituiert ist,
eine Ethylgruppe, die in 2-Stellung durch eine Hydroxy-, Methoxy-, Dimethylamino-, Carboxy- oder Methoxycarbonylgruppe substituiert ist, oder
eine Propylgruppe, die in 3-Stellung durch eine Hydroxy-, Dimethylamino-, Carboxy- oder Methoxycarbonylgruppe substituiert ist, oder
eine 2-Oxopropylgruppe,
R² ein Wasserstoffatom,
eine C₁₋₆-Alkylgruppe,
eine Ethenylgruppe,
eine 2-Propen-1-yl- oder 2-Propin-1-ylgruppe,
eine C₃₋₆-Cycloalkylgruppe,
eine Tetrahydrofuran-3-yl-, Tetrahydropyran-3-yl-, Tetrahydropyran-4-yl-, Tetra-hydrofuranylmethyl- oder Tetrahydropyranylmethylgruppe,
eine Phenylgruppe,
eine Phenyl-C₁₋₄-alkylgruppe, wobei der Phenylteil durch ein Fluor- oder Chloratom, eine Methyl-, Dimethylamino-, Hydroxy-, Methoxy- oder Trifluormethoxygruppe substituiert sein kann,
eine Phenylcarbonylmethylgruppe, wobei der Phenylteil durch ein Fluor- oder Chloratom, eine Hydroxy-, Methoxy- oder Trifluormethoxygruppe substituiert sein kann,
eine 2-Phenylethenylgruppe,
eine 2-(Phenyloxy)ethylgruppe,
eine Pyridylmethyl- oder Pyridylethylgruppe,
eine Methylgruppe, die durch eine C₃₋₆-Cycloalkyl-, Cyan-, Carboxy- oder Methoxy-carbonylgruppe substituiert ist, oder
eine Ethylgruppe, die in 2-Stellung durch eine C₃₋₆-Cycloalkyl-, Cyan-, Carboxy-, Methoxycarbonyl-, Hydroxy-, Methoxy- oder Dimethylaminogruppe substituiert ist,
oder eine Propylgruppe, die in 3-Stellung durch eine C₃₋₆-Cycloalkyl-, Cyan-, Carboxy-, Methoxycarbonyl-, Hydroxy-, Methoxy- oder Dimethylaminogruppe substituiert ist,
R³ eine C₄₋₆-Alkenylgruppe,
eine 1-Cyclopenten-1-ylmethyl- oder 1-Cyclohexen-1-ylmethylgruppe,
eine 1-Cyclopenten-1-ylmethylgruppe, in der der 1-Cyclopenten-1-yl-Teil durch eine Methylgruppe substituiert ist,
eine 2-Propin-1-yl-, 2-Butin-1-yl- oder 2-Pentin-1-ylgruppe,
eine Cyclopropylmethylgruppe oder
eine Cyclopropylmethylgruppe, in der der Cyclopropylteil durch eine Methylgruppe substituiert ist, und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Aminocarbonyl-, Methylaminocarbonyl-, Dimethylaminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist,
eine 3-Amino-2-oxo-piperidin-5-yl- oder 3-Amino-2-oxo-1-methyl-piperidin-5-yl-Gruppe, oder
eine Cyclohexylgruppe, die in 3-Stellung durch eine Aminogruppe substituiert ist, wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische oder deren Salze;
verwendet werden.

9. Verbindungen zur Verwendung gemäß Anspruch 7, wobei Verbindungen der allgemeinen Formel I gemäß Anspruch 1, in der
R¹ eine C₁₋₄-Alkylgruppe,
eine C₃₋₅-Alkenylgruppe,
eine 2-Propen-1-ylgruppe, die durch eine Methoxycarbonylgruppe substituiert ist,
eine C₃₋₅-Alkinylgruppe,
eine Phenyl-C₁₋₄-alkylgruppe, in der der Phenylteil durch ein oder zwei Fluoratome, ein oder zwei Chloratome, ein Bromatom, eine bis drei Methylgruppen, eine Trifluormethyl-, Hydroxy-, Methoxy-, Nitro-, Amino-, Carboxy- oder Ethoxycarbonylgruppe substituiert sein kann,
eine 2-Phenylethylgruppe, in der der Ethylteil in 2-Stellung durch eine Hydroxy-, Methoxy- oder Hydroxyiminogruppe substituiert ist,
eine Phenylcarbonylmethylgruppe, in der der Phenylteil durch ein Fluoratom oder durch eine Methyl-, Aminocarbonyl-, Aminosulfonyl-, Cyan-, Hydroxy-, Methoxy-, Phenoxy-, Benzyloxy-, 2-Propen-1-yloxy-, 2-Propin-1-yloxy-, Cyanmethoxy-, (Methoxycarbonyl)methoxy-, (Aminocarbonyl)methoxy-, (Methylaminocarbonyl)-methoxy-, (Dimethylaminocarbonyl)methoxy-, Methylsulfonyloxy-, Phenylsulfonyloxy-, Nitro-, Amino-, (Methoxycarbonyl)methylamino-, Acetylamino-, Methoxycarbonylamino-, Methylsulfonylamino-, Bis-(methylsulfonyl)-amino-, Aminocarbonylamino-, Dimethylaminocarbonylamino-, (Methylamino)thiocarbonylamino-, (Ethoxycarbonyl-amino)carbonylamino- oder Cyanmethylamino-Gruppe substituiert sein kann, ,
eine Phenylcarbonylmethylgruppe, in der der Phenylteil durch zwei Methoxygruppen oder durch ein Bromatom und durch eine Dimethylaminogruppe substituiert ist,
eine 2-(Phenylcarbonyl)ethylgruppe,
eine 2-Phenylethenylgruppe,
eine 2-(Phenoxy)ethylgruppe,
eine Phenylsulfanylmethyl- oder Phenylsulfinylmethylgruppe,
eine Naphthylmethyl- oder Naphthylethylgruppe,
eine Isoxazolylmethyl-, Thiazolylmethyl-, Pyridylmethyl-, Benzo[d]isoxazolylmethyl-, Benzo[d]isothiazolylmethyl-, (1H-Indazol-3-yl)methyl-, Chinolinylmethyl- oder Isochinolinylmethylgruppe, wobei der heterocyclische Teil jeweils durch eine Methyl-gruppe substituiert sein kann,
eine Isochinolinylmethylgruppe, in der der Isochinolinylteil durch eine Nitro- oder Aminogruppe substituiert ist,
eine (1,2-Dihydro-2-oxo-chinolin-4-yl)methylgruppe,
eine Pyrrolylethyl-, Triazolylethyl-, Thienylethyl-, Thiazolylethyl- oder Pyridylethylgruppe, wobei der heterocyclische Teil jeweils durch eine Methylgruppe substituiert sein kann,
eine Thienylcarbonylmethylgruppe,
eine Methylgruppe, die durch eine Cyclopropyl-, Cyan-, Carboxy-, Aminocarbonyl- oder Methoxycarbonylgruppe substituiert ist,
eine Ethylgruppe, die in 2-Stellung durch eine Hydroxy-, Methoxy-, Dimethylamino-, Carboxy- oder Methoxycarbonylgruppe substituiert ist, oder
eine Propylgruppe, die in 3-Stellung durch eine Hydroxy-, Dimethylamino-, Carboxy- oder Methoxycarbonylgruppe substituiert ist, oder
eine 2-Oxopropylgruppe,
R² ein Wasserstoffatom,
eine C₁₋₆-Alkylgruppe,
eine Ethenylgruppe,
eine 2-Propen-1-yl- oder 2-Propin-1-ylgruppe,
eine Phenylgruppe,
eine Phenyl-C₁₋₄-alkylgruppe, wobei der Phenylteil durch ein Fluoratom, eine Methyl- oder Methoxygruppe substituiert sein kann,
eine Phenylcarbonylmethylgruppe,
eine 2-Phenylethenylgruppe,
eine Methylgruppe, die durch eine Cyclopropyl-, Cyan-, Carboxy- oder Methoxycarbonylgruppe substituiert ist, oder
eine Ethylgruppe, die in 2-Stellung durch eine Cyan-, Hydroxy-, Methoxy- oder Dimethylaminogruppe substituiert ist,
R³ eine C₄₋₆-Alkenylgruppe,
eine 1-Cyclopenten-1-ylmethyl- oder 1-Cyclohexen-1-ylmethylgruppe,
eine 2-Propin-1-yl-, 2-Butin-1-yl- oder 2-Pentin-1-ylgruppe, oder
eine Cyclopropylmethylgruppe und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Pyrrolidin-1-yl-carbonylgruppe substituiert ist,
eine 3-Amino-piperidin-1-ylgruppe, in der der Piperidin-1-yl-Teil in 4-Stellung zusätzlich durch eine Hydroxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist, oder
eine Cyclohexylgruppe, die in 3-Stellung durch eine Aminogruppe substituiert ist, wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische oder deren Salze; verwendet werden.

10. Verbindungen zur Verwendung gemäß Anspruch 7, wobei Verbindungen der allgemeinen Formel I gemäß Anspruch 1, in der
R¹, R² und R³ wie in Anspruch 7 erwähnt definiert sind und
R⁴ eine Azetidin-1-yl- oder Pyrrolidin-1-ylgruppe, die in 3-Stellung durch eine RₑNR_{d}-Gruppe substituiert ist und zusätzlich durch eine oder zwei C₁₋₃-Alkylgruppen substituiert sein kann, wobei
Rₑ ein Wasserstoffatom oder eine C₁₋₃-Alkylgruppe und
R_{d} ein Wasserstoffatom oder eine C₁₋₃-Alkylgruppe bedeutet,
eine Piperidin-1-yl-oder Hexahydroazepin-1-ylgruppe, die in 3-Stellung oder in 4-Stellung durch eine RₑNR_{d}-Gruppe substituiert ist und zusätzlich durch eine oder zwei C₁₋₃-Alkylgruppen substituiert sein kann, wobei Rₑ und R_{d} wie vorstehend erwähnt definiert sind,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-amino-gruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlen-stoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl-oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine R¹⁹-C₃₋₄-alkyl-gruppe, in der der C₃₋₄-Alkylteil geradkettig ist und durch den Rest R¹⁵ substituiert sein kann und zusätzlich durch eine oder zwei C₁₋₃-Alkylgruppen substituiert sein kann, wobei R¹⁵ eine C₁₋₄-Alkylgruppe und R¹⁹ eine Amino-, C₁₋₃-Alkylamino- oder Di-(C₁₋₃-alkyl)-aminogruppe darstellt,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino-oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeutet,
deren Tautomere, Enantiomere, Diastereomere, deren Gemische oder deren Salze;
verwendet werden.

11. Verbindungen zur Verwendung gemäß Anspruch 7, wobei Verbindungen der allgemeinen Formel I gemäß Anspruch 1, in der
R¹, R² und R³ wie in Anspruch 7 oder 8 erwähnt definiert sind und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Aminocarbonyl-, Methylaminocarbonyl-, Dimethylaminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist,
eine 3-Amino-2-oxo-piperidin-5-yl- oder 3-Amino-2-oxo-1-methyl-piperidin-5-yl-Gruppe,
eine Cyclohexylgruppe, die in 3-Stellung durch eine Aminogruppe substituiert ist, wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische oder deren Salze;
verwendet werden.

12. Verbindungen zur Verwendung gemäß Anspruch 7, wobei Verbindungen der allgemeinen Formel I gemäß Anspruch 1, in der
R¹, R² und R³ wie in Anspruch 7, 8 oder 9 erwähnt definiert sind und
R⁴ eine Piperidin-1-ylgruppe, die in 3-Stellung durch eine Aminogruppe substituiert ist, wobei der Piperidin-1-yl-Teil zusätzlich durch eine Methylgruppe substituiert sein kann,
eine 3-Amino-piperidin-1-ylgruppe, in der der Piperidin-1-yl-Teil zusätzlich durch eine Pyrrolidin-1-yl-carbonylgruppe substituiert ist,
eine 3-Amino-piperidin-1-ylgruppe, in der der Piperidin-1-yl-Teil in 4-Stellung zusätzlich durch eine Hydroxygruppe substituiert ist,
eine 3-Amino-piperidin-1-ylgruppe, in der ein Wasserstoffatom in 2-Stellung zusammen mit einem Wasserstoffatom in 5-Stellung durch eine -CH₂-CH₂-Brücke ersetzt ist,
eine Hexahydroazepin-1-yl-gruppe, die in 3-Stellung durch eine Aminogruppe substituiert ist, oder
eine Cyclohexylgruppe, die in 3-Stellung durch eine Aminogruppe substituiert ist, wobei soweit nichts anderes erwähnt wurde, die vorstehend erwähnten Alkyl- und Alkenylgruppen geradkettig oder verzweigt sein können,
deren Tautomere, Enantiomere, Diastereomere, deren Gemische oder deren Salze; verwendet werden.

13. Folgende Verbindungen der allgemeinen Formel I gemäß Anspruch 1:
(1) 1,3-Dimethyl-7-benzyl-8-(3-amino-pyrrolidin-1-yl)-xanthin,
(2) 1,3-Dimethyl-7-(3-methyl-2-buten-1-yl)-8-(3-amino-pyrrolidin-1-yl)-xanthin,
(3) 1,3-Dimethyl-7-benzyl-8-(3-amino-piperidin-1-yl)-xanthin,
(4) 1,3-Dimethyl-7-(3-methyl-2-buten-1-yl)-8-[(trans-2-amino-cyclohexyl)amino]-xanthin,
(5) 1,3-Dimethyl-7-(3-methyl-2-buten-1-yl)-8-(3-amino-piperidin-1-yl)-xanthin,
(6) 1,3-Dimethyl-7-(3-methyl-2-buten-1-yl)-8-(4-amino-piperidin-1-yl)-xanthin,
(7) 1,3-Dimethyl-7-(3-methyl-2-buten-1-yl)-8-[(cis-2-amino-cyclohexyl)amino]-xanthin,
(8) 1,3-Dimethyl-7-(2-butin-1-yl)-8-(3-amino-piperidin-1-yl)-xanthin,
(9) 1,3-Dimethyl-7-[(1-cyclopenten-1-yl)methyl]-8-(3-amino-piperidin-1-yl)-xanthin,
(10) 1,3-Dimethyl-7-(2-thienylmethyl)-8-(3-amino-piperidin-1-yl)-xanthin,
(11) 1,3-Dimethyl-7-(3-fluorbenzyl)-8-(3-amino-piperidin-1-yl)-xanthin,
(12) 1,3-Dimethyl-7-(2-fluorbenzyl)-8-(3-amino-piperidin-1-yl)-xanthin,
(13) 1,3-Dimethyl-7-(4-fluorbenzyl)-8-(3-amino-piperidin-1-yl)-xanthin,
(14) 1,3-Dimethyl-7-(2-buten-1-yl)-8-(3-amino-piperidin-1-yl)-xanthin,
(15) 1,3-Bis-(cyclopropylmethyl)-7-benzyl-8-(3-amino-piperidin-1-yl)-xanthin,
(16) (R)-1,3-Dimethyl-7-(3-methyl-2-buten-1-yl)-8-(3-amino-piperidin-1-yl)-xanthin,
(17) (S)-1,3-Dimethyl-7-(3-methyl-2-buten-1-yl)-8-(3-amino-piperidin-1-yl)-xanthin,
(18) 1,3-Dimethyl-7-(3-methyl-2-buten-1-yl)-8-(3-amino-hexahydroazepin-1-yl)-xanthin,
(19) 1,3-Dimethyl-7-(3-methyl-2-buten-1-yl)-8-(4-amino-hexahydroazepin-1-yl)-xanthin,
(20) 1,3-Dimethyl-7-(3-methyl-2-buten-1-yl)-8-(cis-3-amino-cyclohexyl)-xanthin-hydrochlorid,
(21) 1,3-Dimethyl-7-(3-methyl-2-buten-1-yl)-8-(3-methylamino-piperidin-1-yl)-xanthin,
(22) 1-(2-Phenylethyl)-3-methyl-7-(3-methyl-2-buten-1-yl)-8-(3-amino-piperidin-1-yl)-xanthin,
(23) 1,3-Dimethyl-7-(3-methyl-2-buten-1-yl)-8-[N-(2-aminoethyl)-methylamino]-xanthin,
(24) 1-[2-(Thiophen-2-yl)-ethyl]-3-methyl-7-(3-methyl-2-buten-1-yl)-8-(3-amino-piperidin-1-yl)-xanthin,
(25) 1-[2-(Thiophen-3-yl)-ethyl]-3-methyl-7-(3-methyl-2-buten-1-yl)-8-(3-amino-piperidin-1-yl)-xanthin,
(26) 1-[2-(2-Methyl-phenyl)-ethyl]-3-methyl-7-(3-methyl-2-buten-1-yl)-8-(3-amino-piperidin-1-yl)-xanthin,
(27) 1-[2-(3-Methyl-phenyl)-ethyl]-3-methyl-7-(3-methyl-2-buten-1-yl)-8-(3-amino-piperidin-1-yl)-xanthin,
(28) 1-[2-(3-Methoxy-phenyl)-ethyl]-3-methyl-7-(3-methyl-2-buten-1-yl)-8-(3-amino-piperidin-1-yl)-xanthin,
(29) 1-((E)-2-Phenyl-vinyl)-3-methyl-7-(3-methyl-2-buten-1-yl)-8-(3-amino-piperidin-1-yl)-xanthin,
(30) 1-(2-Phenyl-ethyl)-3-methyl-7-(3-methyl-2-buten-1-yl)-8-((*S*)-3-amino-piperidin-1-yl)-xanthin,
(31) 1-(2-Phenyl-ethyl)-3-methyl-7-(3-methyl-2-buten-1-yl)-8-((*R*)-3-amino-piperidin-1-yl)-xanthin,
(32) 1-[2-(2-Methoxy-phenyl)-2-oxo-ethyl]-3-methyl-7-(3-methyl-2-buten-1-yl)-8-(3-amino-piperidin-1-yl)-xanthin,
(33) 1-[2-(Thiophen-3-yl)-2-oxo-ethyl]-3-methyl-7-(3-methyl-2-buten-1-yl)-8-(3-amino-piperidin-1-yl)-xanthin,
(34) 1-(2-Phenyl-2-oxo-ethyl)-3-methyl-7-(3-methyl-2-buten-1-yl)-8-((S)-3-amino-piperidin-1-yl)-xanthin,
(35) 1-(2-Phenyl-2-oxo-ethyl)-3-methyl-7-(3-methyl-2-buten-1-yl)-8-((R)-3-amino-piperidin-1-yl)-xanthin,
(36) 1-[(Isochinolin-1-yl)methyl]-3-methyl-7-(3-methyl-2-buten-1-yl)-8-((*R*)-3-amino-piperidin-1-yl)-xanthin,
(37) 1-[(Isochinolin-1-yl)methyl]-3-methyl-7-(3-methyl-2-buten-1-yl)-8-((*S*)-3-amino-piperidin-1-yl)-xanthin und
(38) 1-[(1-Naphthyl)methyl]-3-methyl-7-(3-methyl-2-buten-1-yl)-8-(3-amino-piperidin-1-yl)-xanthin
sowie deren Salze;
zur Verwendung in der Kombinationstherapie mit anderen Wirkstoffen, z.B. ausgewählt aus der Gruppe der Antidiabetika, Lipidsenker, HDL-erhöhende Verbindungen, Wirkstoffe zur Behandlung von Obesitas, und Wirkstoffe zur Beeinflussung des Bluthochdrucks.

14. Physiologisch verträgliche Salze der Verbindungen der allgemeinen Formel I nach mindestens einem der Ansprüche 1 bis 13 mit anorganischen oder organischen Säuren oder Basen;
zur Verwendung in der Kombinationstherapie mit anderen Wirkstoffen, z.B. ausgewählt aus der Gruppe der Antidiabetika, Lipidsenker, HDL-erhöhende Verbindungen, Wirkstoffe zur Behandlung von Obesitas, und Wirkstoffe zur Beeinflussung des Bluthochdrucks.

15. Die Verbindungen der allgemeinen Formel I, oder deren physiologisch verträgliche Salze, zur Verwendung gemäß mindestens einem der Ansprüche 1 bis 14, wobei die anderen Wirkstoffen ausgewählt sind aus der Gruppe der Antidiabetika, Lipidsenker, HDL-erhöhende Verbindungen, Wirkstoffe zur Behandlung von Obesitas, und Wirkstoffe zur Beeinflussung des Bluthochdrucks.

16. Arzneimittel, enthaltend eine Verbindung der allgemeinen Formel I nach mindestens einem der Ansprüche 1 bis 13 oder ein physiologisch verträgliches Salz gemäß Anspruch 14 neben gegebenenfalls einem oder mehreren inerten Trägerstoffen und/oder Verdünnungsmitteln;
zur Verwendung in der Kombinationstherapie mit anderen Wirkstoffen, z.B. ausgewählt aus der Gruppe der Antidiabetika, Lipidsenker, HDL-erhöhende Verbindungen, Wirkstoffe zur Behandlung von Obesitas, und Wirkstoffe zur Beeinflussung des Bluthochdrucks.

17. Eine Verbindung der allgemeinen Formel I oder Kombination nach mindestens einem der Ansprüche 1 bis 14 zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können; zum Beispiel, zur Behandlung von Diabetes mellitus Typ I oder Typ II, diabetische Komplikationen, Insulinresistenz, Dyslipidämien, Atherosklerose, Arthritis, Adipositas, Allograft Transplantation oder durch Calcitonin verursachte Osteoporose.

18. Verfahren zur Herstellung eines Arzneimittels zur Verwendung gemäß Anspruch 16, **dadurch gekennzeichnet, daß** auf nichtchemischen Weg eine Verbindung der allgemeinen Formel I nach mindestens einem der Ansprüche 1 bis 14 in einen oder mehrere inerte Trägerstoffe und/oder Verdünnungsmittel eingearbeitet wird.

19. Verfahren zur Herstellung der Verbindungen der allgemeinen Formel I zur Verwendung gemäß den Ansprüchen 1 bis 14, **dadurch gekennzeichnet, daß**
a) zur Herstellung von Verbindungen der allgemeinen Formel I, in der R⁴ einer der in Anspruch 1 erwähnten, über ein Stickstoffatom mit dem Xanthingerüst verknüpften Reste ist
eine Verbindung der allgemeinen Formel 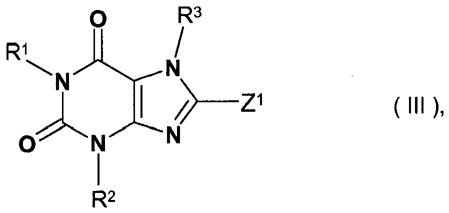 in der
R¹ bis R³ wie in den Ansprüchen 1 bis 14 definiert sind und
Z¹ eine Austrittsgruppe wie ein Halogenatom, eine substituierte Hydroxy-, Mercapto-, Sulfinyl-, Sulfonyl- oder Sulfonyloxygruppe wie ein Chlor- oder Bromatom, eine Methansulfonyl- oder Methansulfonyloxygruppe darstellt, mit einer Verbindung der allgemeinen Formel
H - R⁴' (IV),
umgesetzt wird, in der
R⁴' einen der für R⁴ in den Ansprüchen 1 bis 14 definierten Reste darstellt, der über ein Stickstoffatom mit dem Xanthingerüst der allgemeinen Formel I verknüpft ist,
oder
b) zur Herstellung von Verbindungen der allgemeinen Formel I, in der R⁴ gemäß der Definition nach Anspruch 1 eine Aminogruppe oder eine gegebenenfalls im Alkylteil substituierte Alkylaminogruppe enthält,
eine Verbindung der allgemeinen Formel 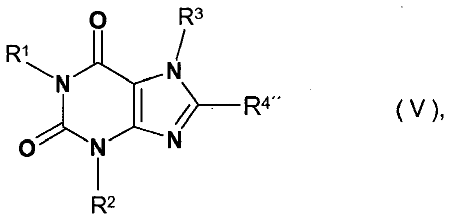
in der R¹, R² und R³ wie in den Ansprüchen 1 bis 14 definiert sind
und
R⁴" eine N-tert.-Butyloxycarbonylaminogruppe oder eine N-tert.-Butyloxycarbonyl-N-alkylaminogruppe enthält, wobei der Alkylteil der N-tert.-Butyloxycarbonyl-N-alkyl-aminogruppe wie in den Ansprüchen 1 bis 4 definiert substituiert sein kann,
entschützt wird,
oder
c) zur Herstellung einer Verbindung der allgemeinen Formel I, in der R² gemäß der Definition nach Anspruch 1 ein Wasserstoffatom darstellt,
eine Verbindung der allgemeinen Formel 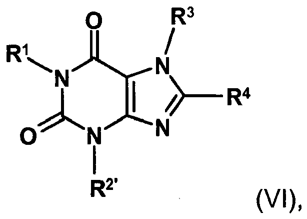
in der R¹, R³ und R⁴ wie eingangs definiert sind und R²' eine Schutzgruppe wie eine Methoxymethyl-, Benzyloxymethyl-, Methoxyethoxymethyl- oder 2-(Trimethylsilyl)-ethyloxymethyl-Gruppe darstellt,
entschützt wird;
wobei eine so erhaltene Verbindung der allgemeinen Formel I, die eine Amino-, Alkylamino- oder Iminogruppe enthält, mittels Acylierung oder Sulfonylierung in eine entsprechende Acyl- oder Sulfonylverbindung der allgemeinen Formel I übergeführt werden kann,
eine so erhaltene Verbindung der allgemeinen Formel I, die eine Amino-, Alkylamino- oder Iminogruppe enthält, mittels Alkylierung oder reduktiver Alkylierung in eine entsprechende Alkylverbindung der allgemeinen Formel I übergeführt werden kann,
eine so erhaltene Verbindung der allgemeinen Formel I, die eine Nitrogruppe enthält, mittels Reduktion in eine entsprechende Aminoverbindung übergeführt werden kann, eine so erhaltene Verbindung der allgemeinen Formel I, die eine Iminogruppe enthält, mittels Nitrosierung und anschließender Reduktion in eine entsprechende N-Amino-iminoverbindung übergeführt werden kann,
eine so erhaltene Verbindung der allgemeinen Formel I, die eine C₁₋₃-Alkyloxy-carbonylgruppe enthält, mittels Esterspaltung in die entsprechende Carboxyverbindung übergeführt werden kann,
eine so erhaltene Verbindung der allgemeinen Formel I, in der R¹ eine Carbonylgruppe enthält, diese beispielsweise mittels Reaktion mit Hydroxylamin in ein entsprechendes Oxim der allgemeinen Formel I übergeführt werden kann,
eine so erhaltene Verbindung der allgemeinen Formel I, die eine Carboxygruppe enthält, mittels Veresterung in einen entsprechenden Ester der allgemeinen Formel I übergeführt werden kann oder
eine so erhaltene Verbindung der allgemeinen Formel I, die eine Carboxy- oder Estergruppe enthält, durch Umsetzung mit einem Amin in ein entsprechendes Amid der allgemeinen Formel I übergeführt werden kann.

20. Arzneimittel, das eine Verbindung der allgemeinen Formel I nach mindestens einem der Ansprüche 1 bis 13 oder ein physiologisch verträgliches Salz gemäß Anspruch 14 sowie einen anderen Wirkstoff, z.B. ausgewählt aus der Gruppe der Antidiabetika, neben gegebenenfalls einem oder mehreren inerten Trägerstoffen und/oder Verdünnungsmitteln aufweist.

21. Arzneimittel gemäß Anspruch 20, wobei der Wirkstoff aus der Gruppe der Antidiabetika ausgewählt ist aus der Gruppe bestehend aus Metformin, Sulfonylharnstoffe, Nateglinide, Repaglinide, Thiazolidindione, PPAR-gamma-Agonisten, alpha-Glucosidasehemmer, alpha2-Anatagonisten, Insulin und Insulinanaloga, GLP-1 und GLP-1 Analoga und Amylin.

22. Verfahren zur Herstellung eines Arzneimittels gemäß Anspruch 20, **dadurch gekennzeichnet, daß** auf nichtchemischen Weg eine Verbindung der allgemeinen Formel I nach mindestens einem der Ansprüche 1 bis 14 in Kombination mit einem anderen Wirkstoff in einen oder mehrere inerte Trägerstoffe und/oder Verdünnungsmittel eingearbeitet wird.

23. Die Verbindung der allgemeinen Formel I nach mindestens einem der Ansprüche 1 bis 14 zur Verwendung gemäß Anspruch 17, wobei die Verbindung der allgemeinen Formel I nach mindestens einem der Ansprüche 1 bis 14 in Kombination mit einem anderen Wirkstoff, z.B. ausgewählt aus der Gruppe der Antidiabetika, Lipidsenker, HDL-erhöhende Verbindungen, Wirkstoffe zur Behandlung von Obesitas, und Wirkstoffe zur Beeinflussung des Bluthochdrucks, verwendet wird.

24. Die Verbindungen der allgemeinen Formel I nach mindestens einem der Ansprüche 1 bis 13 oder ein physiologisch verträgliches Salz gemäß Anspruch 14 zur Verwendung in der Kombinationstherapie mit anderen Antidiabetika.

25. Die Verbindungen der allgemeinen Formel I nach mindestens einem der Ansprüche 1 bis 13 oder ein physiologisch verträgliches Salz gemäß Anspruch 14 zur Verwendung in der Kombinationstherapie mit anderen Wirkstoffen ausgewählt aus der Gruppe der Antidiabetika bestehend aus Metformin, Sulfonylharnstoffe, Nateglinide, Repaglinide, Thiazolidindione, PPAR-gamma-Agonisten, alpha-Glucosidasehemmer, alpha2-Anatagonisten, Insulin und Insulinanaloga, GLP-1 und GLP-1 Analoga und Amylin.

26. Die Verbindungen der allgemeinen Formel I nach mindestens einem der Ansprüche 1 bis 14, worin
die bei der Definition der vorstehend erwähnten Reste erwähnten Carboxygruppen durch eine in-vivo in eine Carboxygruppe überführbare Gruppe oder durch eine unter physiologischen Bedingungen negativ geladene Gruppe ersetzt sein können, und/oder
die bei der Definition der vorstehend erwähnten Reste erwähnten Amino- und Iminogruppen durch einen in-vivo abspaltbaren Rest substituiert sein können.

## Claims

1. Compounds of general formula 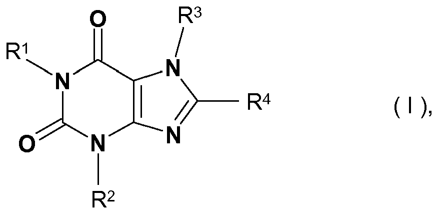 wherein
R¹ denotes a C₁₋₈-alkyl group,
a C₃₋₈-alkenyl group,
a C₃₋₄-alkenyl group which is substituted by a C₁₋₂-alkyloxy-carbonyl, aminocarbonyl, C₁₋₃-alkylamino-carbonyl, di-(C₁₋₃-alkyl)-amino-carbonyl, pyrrolidin-1-ylcarbonyl, piperidin-1-ylcarbonyl or morpholin-4-ylcarbonyl group,
a C₃₋₈-alkynyl group,
a C₁₋₆-alkyl group substituted by a group Rₐ, wherein
Rₐ denotes a C₃₋₇-cycloalkyl, heteroaryl, cyano, carboxy, C₁₋₃-alkyloxy-carbonyl, aminocarbonyl, C₁₋₃-alkylamino-carbonyl, di-(C₁₋₃-alkyl)-amino-carbonyl, pyrrolidin-1-ylcarbonyl, piperidin-1-ylcarbonyl, morpholin-4-ylcarbonyl, piperazin-1-ylcarbonyl, 4-methylpiperazin-1-ylcarbonyl or 4-ethylpiperazin-1-ylcarbonyl group,
a C₁₋₆-alkyl group substituted by a phenyl group, wherein the phenyl ring is substituted by the groups R¹⁰ to R¹⁴ and
R¹⁰ denotes a hydrogen atom,
a fluorine, chlorine, bromine or iodine atom,
a C₁₋₄-alkyl, hydroxy, or C₁₋₄-alkyloxy group,
a nitro, amino, C₁₋₃-alkylamino, di-(C₁₋₃-alkyl)amino, cyano-C₁₋₃-alkylamino, [N-(cyano-C₁₋₃-alkyl)-N-C₁₋₃-alkyl-amino], C₁₋₃-alkyloxy-carbonyl-C₁₋₃-alkylamino, pyrrolidin-1-yl, piperidin-1-yl, morpholin-4-yl, piperazin-1-yl or 4-(C₁₋₃-alkyl)-piperazin-1-yl group,
a C₁₋₃-alkyl-carbonylamino, arylcarbonylamino, aryl-C₁₋₃-alkyl-carbonylamino, C₁₋₃-alkyloxy-carbonylamino, aminocarbonylamino, C₁₋₃-alkyl-aminocarbonylamino, di-(C₁₋₃-alkyl)aminocarbonylamino, pyrrolidin-1-yl-carbonylamino, piperidin-1-yl-carbonylamino, morpholin-4-yl-carbonylamino, piperazin-1-yl-carbonylamino or 4-(C₁₋₃-alkyl)-piperazin-1-yl-carbonylamino, C₁₋₃-alkyl-sulphonylamino, bis-(C₁₋₃-alkylsulphonyl)-amino, aminosulphonylamino, C₁₋₃-alkylamino-sulphonylamino, di-(C₁₋₃-alkyl)amino-sulphonylamino, pyrrolidin-1-yl-sulphonylamino, piperidin-1-yl-sulphonylamino, morpholin-4-yl-sulphonylamino, piperazin-1-yl-sulphonylamino or 4-(C₁₋₃-alkyl)-piperazin-1-yl-sulphonylamino, (C₁₋₃-alkylamino)thiocarbonylamino, (C₁₋₃-alkyloxy-carbonylamino)carbonylamino, arylsulphonylamino or aryl-C₁₋₃-alkyl-sulphonylamino group,
an N-(C₁₋₃-alkyl)-C₁₋₃-alkyl-carbonylamino, N-(C₁₋₃-alkyl)-arylcarbonylamino, N-(C₁₋₃-alkyl)-aryl-C₁₋₃-alkyl-carbonylamino, N-(C₁₋₃-alkyl)-C₁₋₃-alkyloxy-carbonylamino, N-(aminocarbonyl)-C₁₋₃-alkylamino, N-(C₁₋₃-alkyl-aminocarbonyl)-C₁₋₃-alkylamino, N-[di-(C₁₋₃-alkyl)aminocarbonyl]-C₁₋₃-alkylamino, N-(C₁₋₃-alkyl)-C₁₋₃-alkyl-sulphonylamino, N-(C₁₋₃-alkyl)-arylsulphonylamino or N-(C₁₋₃-alkyl)-aryl-C₁₋₃-alkyl-sulphonylamino group,
a 2-oxo-imidazolidin-1-yl, 2,4-dioxo-imidazolidin-1-yl, 2,5-dioxo-imidazolidin-1-yl or 2-oxo-hexahydropyrimidin-1-yl group wherein the nitrogen atom in the 3 position in each case may be substituted by a methyl or ethyl group,
a cyano, carboxy, C₁₋₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl or 4-(C₁₋₃-alkyl)-piperazin-1-yl-carbonyl group,
a C₁₋₃-alkyl-carbonyl or an arylcarbonyl group,
a carboxy-C₁₋₃-alkyl, C₁₋₃-alkyloxy-carbonyl-C₁₋₃-alkyl, cyano-C₁₋₃-alkyl, aminocarbonyl-C₁₋₃-alkyl, C₁₋₃-alkyl-aminocarbonyl-C₁₋₃-alkyl, di-(C₁₋₃-alkyl)-aminocarbonyl-C₁₋₃-alkyl, pyrrolidin-1-yl-carbonyl-C₁₋₃-alkyl, piperidin-1-yl-carbonyl-C₁₋₃-alkyl, morpholin-4-yl-carbonyl-C₁₋₃-alkyl, piperazin-1-yl-carbonyl-C₁₋₃-alkyl or 4-(C₁₋₃-alkyl)-piperazin-1-yl-carbonyl-C₁₋₃-alkyl group,
a carboxy-C₁₋₃-alkyloxy, C₁₋₃-alkyloxy-carbonyl-C₁₋₃-alkyloxy, cyano-C₁₋₃-alkyloxy, aminocarbonyl-C₁₋₃-alkyloxy, C₁₋₃-alkyl-aminocarbonyl-C₁₋₃-alkyloxy, di-(C₁₋₃-alkyl)-aminocarbonyl-C₁₋₃-alkyloxy, pyrrolidin-1-yl-carbonyl-C₁₋₃-alkyl-oxy, piperidin-1-yl-carbonyl-C₁₋₃-alkyloxy, morpholin-4-yl-carbonyl-C₁₋₃-alkyl-oxy, piperazin-1-yl-carbonyl-C₁₋₃-alkyloxy or 4-(C₁₋₃-alkyl)-piperazin-1-yl-carbonyl-C₁₋₃-alkyloxy group,
a hydroxy-C₁₋₃-alkyl, C₁₋₃-alkyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, pyrrolidin-1-yl-C₁₋₃-alkyl, piperidin-1-yl-C₁₋₃-alkyl, morpholin-4-yl-C₁₋₃-alkyl, piperazin-1-yl-C₁₋₃-alkyl, 4-(C₁₋₃-alkyl)-piperazin-1-yl-C₁₋₃-alkyl group,
a hydroxy-C₁₋₃-alkyloxy, C₁₋₃-alkyloxy-C₁₋₃-alkyloxy, C₁₋₃-alkylsulphanyl-C₁₋₃-alkyloxy, C₁₋₃-alkylsulphinyl-C₁₋₃-alkyloxy, C₁₋₃-alkylsulphonyl-C₁₋₃-alkyloxy, amino-C₁₋₃-alkyloxy, C₁₋₃-alkylamino-C₁₋₃-alkyloxy, di-(C₁₋₃-alkyl)-amino-C₁₋₃-alkyloxy, pyrrolidin-1-yl-C₁₋₃-alkyloxy, piperidin-1-yl-C₁₋₃-alkyloxy, morpholin-4-yl-C₁₋₃-alkyloxy, piperazin-1-yl-C₁₋₃-alkyloxy, 4-(C₁₋₃-alkyl)-piperazin-1-yl-C₁₋₃-alkyloxy group,
a mercapto, C₁₋₃-alkylsulphanyl, C₁₋₃-alkysulphinyl, C₁₋₃-alkylsulphonyl, C₁₋₃-alkylsulphonyloxy, arylsulphonyloxy, trifluoromethylsulphanyl, trifluoromethylsulphinyl or trifluoromethylsulphonyl group,
a sulpho, aminosulphonyl, C₁₋₃-alkyl-aminosulphonyl, di-(C₁₋₃-alkyl)-aminosulphonyl, pyrrolidin-1-yl-sulphonyl, piperidin-1-yl-sulphonyl, morpholin-4-yl-sulphonyl, piperazin-1-yl-sulphonyl or 4-(C₁₋₃-alkyl)-piperazin-1-yl-sulphonyl group,
a methyl or methoxy group substituted by 1 to 3 fluorine atoms,
an ethyl or ethoxy group substituted by 1 to 5 fluorine atoms,
a C₂₋₄-alkenyl or C₂₋₄-alkynyl group,
a C₃₋₄-alkenyloxy or C₃₋₄-alkynyloxy group,
a C₃₋₆-cycloalkyl or C₃₋₆-cycloalkyloxy group,
a C₃₋₆-cycloalkyl-C₁₋₃-alkyl or C₃₋₆-cycloalkyl-C₁₋₃-alkyloxy group or
an aryl, aryloxy, aryl-C₁₋₃-alkyl or aryl-C₁₋₃-alkyloxy group,
R¹¹ and R¹², which may be identical or different, each denote a hydrogen atom, a fluorine, chlorine, bromine or iodine atom, a C₁₋₃-alkyl, trifluoromethyl, hydroxy or C₁₋₃-alkyloxy group or a cyano group, or
R¹¹ together with R¹², if they are bound to adjacent carbon atoms, also denote a methylenedioxy, difluoromethylenedioxy or a straight-chain C₃₋₅-alkylene group, and
R¹³ and R¹⁴, which may be identical or different, each denote a hydrogen atom, a fluorine, chlorine or bromine atom, a trifluoromethyl, C₁₋₃-alkyl or C₁₋₃-alkyloxy group,
a phenyl-C₁₋₄-alkyl group wherein the alkyl moiety is substituted by a cyano, carboxy, C₁₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl group and the phenyl moiety is substituted by the groups R¹⁰ to R¹⁴, wherein R¹⁰ to R¹⁴ are as hereinbefore defined,
a phenyl-C₂₋₃-alkenyl group wherein the phenyl moiety is substituted by the groups R¹⁰ to R¹⁴, wherein R¹⁰ to R¹⁴ are as hereinbefore defined,
a phenyl-(CH₂)ₘ-A-(CH₂)ₙ-group wherein the phenyl moiety is substituted by R¹⁰ to R¹⁴, wherein R¹⁰ to R¹⁴ are as hereinbefore defined and
A denotes a carbonyl, cyanoiminomethylene, hydroxyiminomethylene or C₁₋₃-alkyloxyiminomethylene group, m denotes the number 0, 1 or 2 and n denotes the number 1, 2 or 3,
a phenylcarbonylmethyl group wherein the phenyl moiety is substituted by R¹⁰ to R¹⁴, wherein R¹⁰ to R¹⁴ are as hereinbefore defined and the methyl moiety is substituted by a C₁₋₃-alkyl group,
a phenyl-(CH₂)ₘ-B-(CH₂)ₙ group wherein the phenyl moiety is substituted by R¹⁰ to R¹⁴, wherein R¹⁰ to R¹⁴, m and n are as hereinbefore defined and
B denotes a methylene group which is substituted by a hydroxy, C₁₋₃-alkyloxy, amino, C₁₋₃-alkylamino, di-(C₁₋₃-alkyl)-amino, mercapto, C₁₋₃-alkylsulphanyl, C₁₋₃-alkylsulphinyl or C₁₋₃-alkylsulphonyl group and is optionally additionally substituted by a methyl or ethyl group,
a naphthyl-C₁₋₃-alkyl group wherein the naphthyl moiety is substituted by the groups R¹⁰ to R¹⁴, wherein R¹⁰ to R¹⁴ are as hereinbefore defined,
a naphthyl-(CH₂)ₘ-A-(CH₂)ₙ group wherein the naphthyl moiety is substituted by R¹⁰ to R¹⁴, wherein R¹⁰ to R¹⁴, A, m and n are as hereinbefore defined,
a naphthyl-(CH₂)ₘ-B-(CH₂)ₙ group wherein the naphthyl moiety is substituted by R¹⁰ to R¹⁴, wherein R¹⁰ to R¹⁴, B, m and n are as hereinbefore defined,
a heteroaryl-(CH₂)ₘ-A-(CH₂)ₙ group, wherein A, m and n are as hereinbefore defined,
a heteroaryl-(CH₂)ₘ-B-(CH₂)ₙ group, wherein B, m and n are as hereinbefore defined,
a C₁₋₆-alkyl-A-(CH₂)ₙ group, wherein A and n are as hereinbefore defined,
a C₃₋₇-cycloalkyl-(CH₂)ₘ-A-(CH₂)ₙ group, wherein A, m and n are as hereinbefore defined,
a C₃₋₇-cycloalkyl-(CH₂)ₘ-B-(CH₂)ₙ group, wherein B, m and n are as hereinbefore defined,
an R²¹-A-(CH₂)ₙ group wherein R²¹ denotes a C₁₋₃-alkyloxycarbonyl, aminocarbonyl, C₁₋₃-alkylaminocarbonyl, di-(C₁₋₃-alkyl)aminocarbonyl, pyrrolidin-1-yl-carbonyl, piperidin-1-yl-carbonyl or morpholin-4-yl-carbonyl, piperazin-1-yl-carbonyl, 4-methylpiperazin-1-yl-carbonyl or 4-ethylpiperazin-1-yl-carbonyl group and A and n are as hereinbefore defined,
a phenyl-(CH₂)ₘ-D-C₁₋₃-alkyl group wherein the phenyl moiety is substituted by the groups R¹⁰ to R¹⁴, wherein R¹⁰ to R¹⁴ and m are as hereinbefore defined and D denotes an oxygen or sulphur atom, an imino, C₁₋₃-alkylimino, sulphinyl or sulphonyl group,
a naphthyl-(CH₂)ₘ-D-C₁₋₃-alkyl group wherein the naphthyl moiety is substituted by the groups R¹⁰ to R¹⁴, wherein R¹⁰ to R¹⁴, D and m are as hereinbefore defined, or
a C₂₋₆-alkyl group substituted by a group R_{b}, wherein
R_{b} is isolated by at least two carbon atoms from the cyclic nitrogen atom in the 1 position of the xanthine skeleton and R_{b} denotes a hydroxy, C₁₋₃-alkyloxy, mercapto, C₁₋₃-alkylsulphanyl, C₁₋₃-alkylsulphinyl, C₁₋₃-alkylsulphonyl, amino, C₁₋₃-alkylamino, di-(C₁₋₃-alkyl)-amino, pyrrolidin-1-yl, piperidin-1-yl, morpholin-4-yl, piperazin-1-yl or 4-(C₁₋₃-alkyl)-piperazin-1-yl group,
R² denotes a hydrogen atom,
a C₁₋₈-alkyl group,
a C₂₋₆-alkenyl group,
a C₃₋₆-alkynyl group,
a C₁₋₆-alkyl group substituted by a group Rₐ, wherein Rₐ is as hereinbefore defined,
a tetrahydrofuran-3-yl, tetrahydropyran-3-yl, tetrahydropyran-4-yl, tetrahydrofuranyl-C₁₋₃-alkyl or tetrahydropyranyl-C₁₋₃-alkyl group,
a C₁₋₆-alkyl group substituted by a phenyl group, wherein the phenyl ring is substituted by the groups R¹⁰ to R¹⁴ and R¹⁰ to R¹⁴ are as hereinbefore defined,
a phenyl group substituted by the groups R¹⁰ to R^{14,} wherein R¹⁰ to R¹⁴ are as hereinbefore defined,
a phenyl-C₂₋₃-alkenyl group wherein the phenyl moiety is substituted by the groups R¹⁰ to R¹⁴, wherein R¹⁰ to R¹⁴ are as hereinbefore defined,
a phenyl-(CH₂)ₘ-A-(CH₂)ₙ group wherein the phenyl moiety is substituted by R¹⁰ to R¹⁴, wherein R¹⁰ to R¹⁴, A, m and n are as hereinbefore defined,
a phenyl-(CH₂)ₘ-B-(CH₂)ₙ group wherein the phenyl moiety is substituted by R¹⁰ to R¹⁴, wherein R¹⁰ to R¹⁴, B, m and n are as hereinbefore defined,
a heteroaryl-(CH₂)ₘ-A-(CH₂)ₙ group, wherein A, m and n are as hereinbefore defined,
a heteroaryl-(CH₂)ₘ-B-(CH₂)ₙ group, wherein B, m and n are as hereinbefore defined,
a C₁₋₆-alkyl-A-(CH₂)ₙ group, wherein A and n are as hereinbefore defined,
a C₃₋₇-cycloalkyl-(CH₂)ₘ-A-(CH₂)ₙ group, wherein A, m and n are as hereinbefore defined,
a C₃₋₇-cycloalkyl-(CH₂)ₘ-B-(CH₂)ₙ group, wherein B, m and n are as hereinbefore defined,
an R²¹-A-(CH₂)ₙ group wherein R²¹, A and n are as hereinbefore defined,
a phenyl-(CH₂)ₘ-D-C₁₋₃-alkyl group wherein the phenyl moiety is substituted by the groups R¹⁰ to R¹⁴, wherein R¹⁰ to R¹⁴, m and D are as hereinbefore defined,
a C₂₋₆-alkyl group substituted by a group R_{b}, wherein
R_{b} is isolated by at least two carbon atoms from the cyclic nitrogen atom in the 3 position of the xanthine skeleton and is as hereinbefore defined,
or a C₃₋₆-cycloalkyl group,
R³ denotes a C₁₋₄-alkyl group substituted by the group R_{c}, wherein
R_{c} denotes a C₃₋₇-cycloalkyl group optionally substituted by one or two C₁₋₃-alkyl groups, or
a C₅₋₇-cycloalkenyl group optionally substituted by one or two C₁₋₃-alkyl groups,
a C₃₋₈-alkenyl group,
a C₃₋₆-alkenyl group substituted by a fluorine, chlorine or bromine atom or a trifluoromethyl group,
a C₃₋₈-alkynyl group, or
an aryl-C₂₋₄-alkenyl group,
and
R⁴ denotes an azetidin-1-yl or pyrrolidin-1-yl group which is substituted in the 3 position by an RₑNR_{d} group and may additionally be substituted by one or two C₁₋₃-alkyl groups, wherein
Rₑ denotes a hydrogen atom or a C₁₋₃-alkyl group and
R_{d} denotes a hydrogen atom, a C₁₋₃-alkyl group, an R_{f}-C₁₋₃-alkyl group or an R_{g}-C₂₋₃-alkyl group, wherein
R_{f} denotes a carboxy, C₁₋₃-alkyloxy-carbonyl, aminocarbonyl, C₁₋₃-alkylamino-carbonyl, di-(C₁₋₃-alkyl)-aminocarbonyl, pyrrolidin-1-yl-carbonyl, 2-cyanopyrrolidin-1-yl-carbonyl, 2-carboxypyrrolidin-1-yl-carbonyl, 2-methoxycarbonylpyrrolidin-1-yl-carbonyl, 2-ethoxycarbonylpyrrolidin-1-yl-carbonyl, 2-aminocarbonylpyrrolidin-1-yl-carbonyl, 4-cyanothiazolidin-3-yl-carbonyl, 4-carboxythiazolidin-3-yl-carbonyl, 4-methoxycarbonylthiazolidin-3-yl-carbonyl, 4-ethoxycarbonylthiazolidin-3-yl-carbonyl, 4-aminocarbonylthiazolidin-3-yl-carbonyl, piperidin-1-yl-carbonyl, morpholin-4-yl-carbonyl, piperazin-1-yl-carbonyl, 4-methyl-piperazin-1-yl-carbonyl or 4-ethyl-piperazin-1-yl-carbonyl group and
R_{g}, which is separated by at least two carbon atoms from the nitrogen atom of the RₑNR_{d} group, denotes a hydroxy, methoxy or ethoxy group,
a piperidin-1-yl or hexahydroazepin-1-yl group which is substituted in the 3 position or in the 4 position by an RₑNR_{d} group and may additionally be substituted by one or two C₁₋₃-alkyl groups, wherein Rₑ and R_{d} are as hereinbefore defined,
a 3-amino-piperidin-1-yl group wherein the piperidin-1-yl moiety is additionally substituted by an aminocarbonyl, C₁₋₂-alkyl-aminocarbonyl, di-(C₁₋₂-alkyl)aminocarbonyl, pyrrolidin-1-yl-carbonyl, (2-cyano-pyrrolidin-1-yl-)carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety in the 4 position or in the 5 position is additionally substituted by a hydroxy or methoxy group,
a 3-amino-piperidin-1-yl group wherein the methylene group in the 2 position or in the 6 position is replaced by a carbonyl group,
a piperidin-1-yl or hexahydroazepin-1-yl group substituted in the 3 position by an amino, C₁₋₃-alkylamino or di-(C₁₋₃-alkyl)-amino group, wherein in each case two hydrogen atoms at the carbon skeleton of the piperidin-1-yl or hexahydroazepin-1-yl- group are replaced by a straight-chain alkylene bridge, this bridge containing 2 to 5 carbon atoms if the two hydrogen atoms are located on the same carbon atom, or 1 to 4 carbon atoms if the hydrogen atoms are located on adjacent carbon atoms, or 1 to 4 carbon atoms, if the hydrogen atoms are located at carbon atoms separated by one atom, or 1 to 3 carbon atoms if the two hydrogen atoms are located at carbon atoms separated by two atoms,
an azetidin-1-yl, pyrrolidin-1-yl, piperidin-1-yl or hexahydroazepin-1-yl group which is substituted by an amino-C₁₋₃-alkyl, C₁₋₃-alkylamino-C₁₋₃-alkyl or a di-(C₁₋₃-alkyl)amino-C₁₃-alkyl group,
a C₃₋₇-cycloalkyl group which is substituted by an amino, C₁₋₃-alkylamino or di-(C₁₋₃-alkyl)-amino group,
a C₃₋₇-cycloalkyl group which is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl-C₁₋₂-alkyl group wherein the cycloalkyl moiety is substituted by an amino, C₁₋₃-alkylamino or di-(C₁₋₃-alkyl)-amino group,
a C₃₋₇-cycloalkyl-C₁₋₂-alkyl group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
an R¹⁹-C₃₋₄-alkyl group wherein the C₃₋₄-alkyl moiety is straight-chained and may be substituted by the group R¹⁵ and may additionally be substituted by one or two C₁₋₃-alkyl groups, wherein R¹⁵ denotes a C₁₋₆-alkyl group, a C₃₋₆-cycloalkyl, C₃₋₆-cydoalkyl-C₁₋₃-alkyl, aryl or aryl-C₁₋₃-alkyl group and R¹⁹ denotes an amino, C₁₋₃-alkylamino or di-(C₁₋₃-alkyl)-amino group,
a 3-amino-2-oxo-piperidin-5-yl or 3-amino-2-oxo-1-methyl-piperidin-5-yl group,
a pyrrolidin-3-yl, piperidin-3-yl, piperidin-4-yl, hexahydroazepin-3-yl or hexahydroazepin-4-yl group which is substituted in the 1 position by an amino, C₁₋₃-alkylamino or di-(C₁₋₃-alkyl)amino group,
or an azetidin-2-yl-C₁₋₂-alkyl, azetidin-3-yl-C₁₋₂-alkyl, pyrrolidin-2-yl-C₁₋₂-alkyl, pyrrolidin-3-yl, pyrrolidin-3-yl-C₁₋₂-alkyl, piperidin-2-yl-C₁₋₂-alkyl, piperidin-3-yl, piperidin-3-yl-C₁₋₂-alkyl, piperidin-4-yl or piperidin-4-yl-C₁₋₂-alkyl group, wherein the abovementioned groups may each be substituted by one or two C₁₋₃-alkyl groups,
while by the aryl groups mentioned in the definition of the groups mentioned above are meant phenyl or naphthyl groups which may be mono- or disubstituted by Rₕ independently of one another, while the substituents may be identical or different and Rₕ denotes a fluorine, chlorine, bromine or iodine atom, a trifluoromethyl, cyano, nitro, amino, aminocarbonyl, aminosulphonyl, methylsulphonyl, acetylamino, methylsulphonylamino, C₁₋₃-alkyl, cyclopropyl, ethenyl, ethynyl, hydroxy, C₁₋₃-alkyloxy, difluoromethoxy or trifluoromethoxy group,
by the heteroaryl groups mentioned in the definition of the groups mentioned above is meant a pyrrolyl, furanyl, thienyl, pyridyl, indolyl, benzofuranyl, benzothiophenyl, quinolinyl or isoquinolinyl group,
or a pyrrolyl, furanyl, thienyl or pyridyl group wherein one or two methyne groups are replaced by nitrogen atoms,
or an indolyl, benzofuranyl, benzothiophenyl, quinolinyl or isoquinolinyl group wherein one to three methyne groups are replaced by nitrogen atoms,
or a 1,2-dihydro-2-oxo-pyridinyl, 1,4-dihydro-4-oxo-pyridinyl, 2,3-dihydro-3-oxo-pyridazinyl, 1,2,3,6-tetrahydro-3,6-dioxo-pyridazinyl, 1,2-dihydro-2-oxo-pyrimidinyl, 3,4-dihydro-4-oxo-pyrimidinyl, 1,2,3,4-tetrahydro-2,4-dioxo-pyrimidinyl, 1,2-dihydro-2-oxo-pyrazinyl, 1,2,3,4-tetrahydro-2,3-dioxo-pyrazinyl, 2,3-dihydro-2-oxo-indolyl, 2,3-dihydrobenzofuranyl, 2,3-dihydro-2-oxo-1 H-benzimidazolyl, 2,3-dihydro-2-oxo-benzoxazolyl, 1,2-dihydro-2-oxo-quinolinyl, 1,4-dihydro-4-oxo-quinolinyl, 1,2-dihydro-1-oxo-isoquinolinyl, 1,4-dihydro-4-oxo-cinnolinyl, 1,2-dihydro-2-oxo-quinazolinyl, 1,4-dihydro-4-oxo-quinazolinyl, 1,2,3,4-tetrahydro-2,4-dioxo-quinazolinyl, 1,2-dihydro-2-oxoquinoxalinyl, 1,2,3,4-tetrahydro-2,3-dioxo-quinoxalinyl, 1,2-dihydro-1-oxo-phthalazinyl, 1,2,3,4-tetrahydro-1,4-dioxo-phthalazinyl, chromanyl, cumarinyl, 2,3-dihydro-benzo[1,4]dioxinyl or 3,4-dihydro-3-oxo-2H-benzo[1,4]oxazinyl group,
wherein the abovementioned heteroaryl groups may be substituted by R¹⁰ to R¹⁴, wherein R¹⁰ to R¹⁴ are as hereinbefore defined,
while, unless otherwise stated, the abovementioned alkyl, alkenyl and alkynyl groups may be straight-chain or branched,
or the derivatives which are N-oxidised or methylated or ethylated at the cyclic nitrogen atom in the 9 position of the xanthine skeleton,
or the derivatives wherein the 2-oxo, the 6-oxo or the 2-oxo and the 6-oxo group of the xanthine skeleton are replaced by thioxo groups,
the tautomers, enantiomers, diastereomers, mixtures or salts thereof;
for use in combination therapy with other active substances.

2. Compounds for use according to claim 1, where compounds of general formula I according to claim 1 are used, wherein
R¹ denotes a C₁₋₆-alkyl group,
a C₃₋₆-alkenyl group,
a C₃₋₄-alkenyl group which is substituted by a C₁₋₂-alkyloxy-carbonyl group,
a C₃₋₆-alkynyl group,
a C₃₋₆-cycloalkyl-C₁₋₃-alkyl group,
a phenyl-C₁₋₄-alkyl group wherein the phenyl moiety is substituted by R¹⁰ to R¹², wherein
R¹⁰ denotes a hydrogen atom, a fluorine, chlorine or bromine atom,
a C₁₋₄-alkyl, trifluoromethyl, hydroxymethyl, C₃₋₆-cycloalkyl, ethynyl or phenyl group,
a hydroxy, C₁₋₄-alkyloxy, difluoromethoxy, trifluoromethoxy, 2,2,2-trifluoroethoxy, phenoxy, benzyloxy, 2-propen-1-yloxy, 2-propyn-1-yloxy, cyano-C₁₋₂-alkyloxy, C₁₋₂-alkylsulphonyloxy, phenylsulphonyloxy, carboxy-C₁₋₃-alkyloxy, C₁₋₃-alkyloxycarbonyl-C₁₋₃-alkyloxy, aminocarbonyl-C₁₋₃-alkyloxy, C₁₋₂-alkyl-aminocarbonyl-C₁₋₃-alkyloxy, di-(C₁₋₂-alkyl)aminocarbonyl-C₁₋₃-alkyloxy, pyrrolidin-1-yl-carbonyl-C₁₋₃-alkyloxy, piperidin-1-ylcarbonyl-C₁₋₃-alkyloxy, morpholin-4-ylcarbonyl-C₁₋₃-alkyloxy, methylsulphanylmethoxy, methylsulphinylmethoxy, methylsulphonylmethoxy, C₃₋₆-cycloalkyloxy or C₃₋₆-cycloalkyl-C₁₋₂-alkyloxy group,
a carboxy, C₁₋₃-alkyloxycarbonyl, carboxy-C₁₋₃-alkyl, C₁₋₃-alkyloxy-carbonyl-C₁₋₃-alkyl, aminocarbonyl, C₁₋₂-alkylaminocarbonyl, di-(C₁₋₂-alkyl)aminocarbonyl, morpholin-4-ylcarbonyl or cyano group,
a nitro, amino, C₁₋₂-alkylamino, di-(C₁₋₂-alkyl)amino, cyano-C₁₋₂-alkylamino, [N-(cyano-C₁₋₂-alkyl)-N-C₁₋₂-alkyl-amino], C₁₋₂-alkyloxy-carbonyl-C₁₋₂-alkylamino, C₁₋₂-alkylcarbonylamino, C₁₋₂-alkyloxy-carbonylamino, C₁₋₃-alkylsulphonylamino, bis-(C₁₋₂-alkylsulphonyl)-amino, aminosulphonylamino, C₁₋₂-alkylamino-sulphonylamino, di-(C₁₋₂-alkyl)amino-sulphonylamino, morpholin-4-yl-sulphonylamino, (C₁₋₂-alkylamino)thiocarbonylamino, (C₁₋₂-alkyloxy-carbonylamino)carbonylamino, aminocarbonylamino, C₁₋₂-alkylaminocarbonylamino, di-(C₁₋₂-alkyl)aminocarbonylamino or morpholin-4-ylcarbonylamino group,
a 2-oxo-imidazolidin-1-yl, 3-methyl-2-oxo-imidazolidin-1-yl, 2,4-dioxo-imidazolidin-1-yl, 3-methyl-2,4-dioxo-imidazolidin-1-yl, 2,5-dioxo-imidazolidin-1-yl, 3-methyl-2,5-dioxo-imidazolidin-1-yl, 2-oxo-hexahydropyrimidin-1-yl or 3-methyl-2-oxo-hexahydropyrimidin-1-yl group,
or
a C₁₋₂-alkylsulphanyl, C₁₋₂-alkylsulphinyl, C₁₋₂-alkylsulphonyl, aminosulphonyl, C₁₋₂-alkylaminosulphonyl or di-(C₁₋₂-alkyl)aminosulphonyl group,
and R¹¹ and R¹², which may be identical or different, denote a hydrogen, fluorine, chlorine or bromine atom or
a methyl, cyano, trifluoromethyl or methoxy group,
or, R¹¹ together with R¹², if they are bound to adjacent carbon atoms, also denote a methylenedioxy, difluoromethylenedioxy, 1,3-propylene or 1,4-butylene group,
a phenyl-C₁₋₃-alkyl group wherein the alkyl moiety is substituted by a carboxy, C₁₋₂-alkyloxy-carbonyl, aminocarbonyl, C₁₋₂-alkylaminocarbonyl or di-(C₁₋₂-alkyl)amino-carbonyl group,
a phenyl-C₂₋₃-alkenyl group, wherein the phenyl moiety may be substituted by a fluorine, chlorine or bromine atom or by a methyl, trifluoromethyl or methoxy group,
a phenyl-(CH₂)ₘ-A-(CH₂)ₙ group wherein the phenyl moiety is substituted by R¹⁰ to R¹², wherein R¹⁰ to R¹² are as hereinbefore defined and
A denotes a carbonyl, hydroxyiminomethylene or C₁₋₂-alkyloxyiminomethylene group, m denotes the number 0 or 1 and n denotes the number 1 or 2,
a phenylcarbonylmethyl group wherein the phenyl moiety is substituted by R¹⁰ to R¹², wherein R¹⁰ to R¹² are as hereinbefore defined and the methyl moiety is substituted by a methyl or ethyl group,
a phenylcarbonylmethyl group wherein two adjacent hydrogen atoms of the phenyl moiety are replaced by a -O-CO-NH-, -NH-CO-NH-, -N=CH-NH-, -N=CH-O- or -O-CH₂-CO-NH- bridge, wherein the abovementioned bridges may be substituted by one or two methyl groups,
a phenyl-(CH₂)ₘ-B-(CH₂)ₙ group wherein the phenyl moiety is substituted by R¹⁰ to R¹², wherein R¹⁰ to R¹², m and n are as hereinbefore defined and
B denotes a methylene group which is substituted by a hydroxy or C₁₋₂-alkyloxy group and is optionally additionally substituted by a methyl group,
a naphthylmethyl or naphthylethyl group, wherein the naphthyl moiety is substituted in each case by R¹⁰ to R¹², wherein R¹⁰ to R¹² are as hereinbefore defined,
a heteroaryl-C₁₋₃-alkyl group, wherein by the term heteroaryl is meant a pyrrolyl, imidazolyl, triazolyl, furanyl, thienyl, oxazolyl, isoxazolyl, thiazolyl, isothiazolyl, pyridyl, pyridazinyl, pyrimidinyl, pyrazinyl, indolyl, benzimidazolyl, 2,3-dihydro-2-oxo-1H-benzimidazolyl, indazolyl, benzofuranyl, 2,3-dihydrobenzofuranyl, benzoxazolyl, 2,3-dihydro-2-oxo-benzoxazolyl, benzisoxazolyl, benzothiophenyl, benzothiazolyl, benzoisothiazolyl, quinolinyl, 1,2-dihydro-2-oxo-quinolinyl, isoquinolinyl, 1,2-dihydro-1-oxo-isoquinolinyl, cinnolinyl, quinazolinyl, 1,2-dihydro-2-oxo-quinazolinyl, 1,2-dihydro-1-oxo-phthalazin-4-yl, cumarinyl or 3,4-dihydro-3-oxo-2H-benzo[1,4]oxazinyl group,
wherein the abovementioned heteroaryl groups may be substituted at carbon atoms by a fluorine, chlorine or bromine atom, by a methyl, trifluoromethyl, cyano, aminocarbonyl, aminosulphonyl, methylsulphonyl, nitro, amino, acetylamino, methylsulphonylamino, methoxy, difluoromethoxy or trifluoromethoxy group and the imino groups of the abovementioned heteroaryl groups may be substituted by methyl or ethyl groups,
a furanyl-A-CH₂, thienyl-A-CH₂, thiazolyl-A-CH₂ or pyridyl-A-CH₂ group, wherein A is as hereinbefore defined,
a furanyl-B-CH₂, thienyl-B-CH₂, thiazolyl-B-CH₂ or pyridyl-B-CH₂ group, wherein B is as hereinbefore defined,
a C₁₋₄-alkyl-A-(CH₂)ₙ group, wherein A and n are as hereinbefore defined,
a C₃₋₆-cycloalkyl-(CH₂)ₘ-A-(CH₂)ₙ group, wherein A, m and n are as hereinbefore defined,
a C₃₋₆-cycloalkyl-(CH₂)ₘ-B-(CH₂)ₙ group, wherein B, m and n are as hereinbefore defined,
an R²¹-A-(CH₂)ₙ group wherein R²¹ denotes a C₁₋₂-alkyloxycarbonyl, aminocarbonyl, C₁₋₂-alkylaminocarbonyl, di-(C₁₋₂-alkyl)aminocarbonyl, pyrrolidin-1-yl-carbonyl, piperidin-1-yl-carbonyl or morpholin-4-yl-carbonyl group and A and n are as hereinbefore defined,
a phenyl-D-C₁₋₃-alkyl group wherein the phenyl moiety is optionally substituted by a fluorine, chlorine or bromine atom, a methyl, trifluoromethyl or methoxy group and D denotes an oxygen or sulphur atom, a sulphinyl or sulphonyl group,
a C₁₋₄-alkyl group substituted by a group Rₐ, wherein
Rₐ denotes a cyano, carboxy, C₁₋₃-alkyloxy-carbonyl, aminocarbonyl, C₁₋₂-alkyl-aminocarbonyl, di-(C₁₋₂-alkyl)aminocarbonyl, pyrrolidin-1-yl-carbonyl, piperidin-1-ylcarbonyl or morpholin-4-ylcarbonyl group,
a C₂₋₄-alkyl group substituted by a group R_{b}, wherein
R_{b} denotes a hydroxy, C₁₋₃-alkyloxy, amino, C₁₋₃-alkylamino, di-(C₁₋₃-alkyl)-amino, pyrrolidin-1-yl, piperidin-1-yl, morpholin-4-yl, piperazin-1-yl, 4-methyl-piperazin-1-yl or 4-ethyl-piperazin-1-yl group and is isolated by at least two carbon atoms from the cyclic nitrogen atom in the 1 position of the xanthine skeleton,
R² denotes a hydrogen atom,
a C₁₋₆-alkyl group,
a C₂₋₄-alkenyl group,
a C₃₋₄-alkynyl group,
a C₃₋₆-cycloalkyl group,
a C₃₋₆-cycloalkyl-C₁₋₃-alkyl group,
a tetrahydrofuran-3-yl, tetrahydropyran-3-yl, tetrahydropyran-4-yl, tetrahydrofuranylmethyl or tetrahydropyranylmethyl group,
a phenyl group which is optionally substituted by a fluorine, chlorine or bromine atom or by a methyl, trifluoromethyl, hydroxy, methoxy, difluoromethoxy or trifluoromethoxy group,
a phenyl-C₁₋₄-alkyl group wherein the phenyl moiety is optionally substituted by a fluorine, chlorine or bromine atom, a methyl, trifluoromethyl, dimethylamino, hydroxy, methoxy, difluoromethoxy or trifluoromethoxy group,
a phenyl-C₂₋₃-alkenyl group, wherein the phenyl moiety may be substituted by a fluorine, chlorine or bromine atom or by a methyl, trifluoromethyl or methoxy group,
a phenylcarbonyl-C₁₋₂-alkyl group wherein the phenyl moiety is optionally substituted by a fluorine, chlorine or bromine atom, a methyl, trifluoromethyl, hydroxy, methoxy, difluoromethoxy or trifluoromethoxy group,
a heteroaryl-C₁₋₃-alkyl group, wherein the term heteroaryl is as hereinbefore defined,
a furanylcarbonylmethyl, thienylcarbonylmethyl, thiazolylcarbonylmethyl or pyridylcarbonylmethyl group,
a C₁₋₄-alkyl-carbonyl-C₁₋₂-alkyl group,
a C₃₋₆-cycloalkyl-carbonyl-C₁₋₂-alkyl group,
a phenyl-D-C₁₋₃-alkyl group wherein the phenyl moiety is optionally substituted by a fluorine, chlorine or bromine atom, a methyl, trifluoromethyl, hydroxy, methoxy, difluoromethoxy or trifluoromethoxy group, and D is as hereinbefore defined, or
a C₁₋₄-alkyl group substituted by a group Rₐ, wherein Rₐ is as hereinbefore defined, a C₂₋₄-alkyl group substituted by a group R_{b}, wherein R_{b} is as hereinbefore defined and is isolated by at least two carbon atoms from the cyclic nitrogen atom in the 3 position of the xanthine skeleton,
R³ denotes a C₃₋₇-alkenyl group,
a C₃₋₅-alkenyl group which is substituted by a fluorine, chlorine or bromine atom or a trifluoromethyl group,
a C₃₋₆-alkynyl group,
a C₁₋₃-alkyl group substituted by the group R_{c}, wherein
R_{c} denotes a C₃₋₆-cycloalkyl group optionally substituted by one or two methyl groups,
a C₅₋₆-cycloalkenyl group optionally substituted by one or two methyl groups,
or a phenyl-C₂₋₃-alkenyl group
and
R⁴ denotes a pyrrolidin-1-yl group which is substituted in the 3 position by an amino, methylamino or dimethylamino group,
an azetidin-1-yl group which is substituted by an aminomethyl group,
a pyrrolidin-1-yl group which is substituted by an aminomethyl group,
a piperidin-1-yl group which is substituted in the 3 position or in the 4 position by an amino, methylamino, dimethylamino or [(2-cyano-pyrrolidin-1-yl-)carbonylmethyl]-amino group, wherein the piperidin-1-yl moiety may additionally be substituted by a methyl or ethyl group,
a 3-amino-piperidin-1-yl group wherein the piperidin-1-yl moiety is additionally substituted by an aminocarbonyl, C₁₋₂-alkyl-aminocarbonyl, di-(C₁₋₂-alkyl)aminocarbonyl, pyrrolidin-1-yl-carbonyl, (2-cyano-pyrrolidin-1-yl-)carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety in the 4 position or in the 5 position is additionally substituted by a hydroxy or methoxy group,
a 3-amino-piperidin-1-yl group wherein the methylene group in the 2 position or in the 6 position is replaced by a carbonyl group,
a 3-amino-piperidin-1-yl group wherein a hydrogen atom in the 2 position together with a hydrogen atom in the 5 position is replaced by a -CH₂-CH₂- bridge,
a 3-amino-piperidin-1-yl group wherein a hydrogen atom in the 2 position together with a hydrogen atom in the 6 position is replaced by a -CH₂-CH₂- bridge,
a 3-amino-piperidin-1-yl group wherein a hydrogen atom in the 4 position together with a hydrogen atom in the 6 position is replaced by a -CH₂-CH₂- bridge,
a piperidin-1-yl group which is substituted by an aminomethyl group,
a piperidin-3-yl or piperidin-4-yl group,
a piperidin-3-yl or piperidin-4-yl group which is substituted in the 1 position by an amino group,
a hexahydroazepin-1-yl group which is substituted in the 3 position or in the 4 position by an amino group,
a 3-amino-propyl, 3-methylamino-propyl or 3-dimethylamino-propyl group wherein the propyl moiety may be substituted by one or two methyl groups,
a 4-amino-butyl, 4-methylamino-butyl or 4-dimethylamino-butyl group wherein the butyl moiety may be substituted by one or two methyl groups,
a C₁₋₂-alkyl group which is substituted by a 2-pyrrolidinyl, 3-pyrrolidinyl, 2-piperidinyl, 3-piperidinyl or 4-piperidinyl group,
a 3-amino-2-oxo-piperidin-5-yl or 3-amino-2-oxo-1-methyl-piperidin-5-yl group,
a C₃₋₆-cycloalkyl group which is substituted by an amino, aminomethyl or aminoethyl group or
a C₃₋₆-cycloalkyl-C₁₋₂-alkyl group wherein the cycloalkyl moiety is substituted by an amino, aminomethyl or aminoethyl group,
while, unless otherwise stated, the abovementioned alkyl, alkenyl and alkynyl groups may be straight-chain or branched,
the tautomers, enantiomers, diastereomers, mixtures thereof or the salts thereof.

3. Compounds for use according to claim 1, where compounds of general formula I according to claim 1 are used, wherein
R¹ is defined as in claim 1 or 2, and
R² denotes a hydrogen atom,
a C₁₋₆-alkyl group,
an ethenyl group,
a 2-propen-1-yl or 2-propyn-1-yl group,
a phenyl group,
a phenyl-C₁₋₄-alkyl group, wherein the phenyl moiety may be substituted by a fluorine atom, a methyl or methoxy group,
a phenylcarbonylmethyl group,
a 2-phenylethenyl group,
a methyl group which is substituted by a cyclopropyl, cyano, carboxy or methoxycarbonyl group, or
an ethyl group which is substituted in the 2 position by a cyano, hydroxy, methoxy or dimethylamino group,
R³ denotes a C₄₋₆-alkenyl group,
a 1-cyclopenten-1-ylmethyl or 1-cyclohexen-1-ylmethyl group,
a 2-propyn-1-yl, 2-butyn-1-yl or 2-pentyn-1-yl group,
a 2-phenylethenyl group, or
a cyclopropylmethyl group and
R⁴ denotes a pyrrolidin-1-yl group which is substituted in the 3 position by an amino group,
an azetidin-1-yl group which is substituted by an aminomethyl group,
a pyrrolidin-1-yl group which is substituted by an aminomethyl group,
a piperidin-1-yl group which is substituted in the 3 position or in the 4 position by an amino, methylamino, dimethylamino or [(2-cyano-pyrrolidin-1-yl)carbonylmethyl]-amino group, wherein the piperidin-1-yl moiety may additionally be substituted by a methyl group,
a 3-amino-piperidin-1-yl group wherein the piperidin-1-yl moiety is additionally substituted by a pyrrolidin-1-yl-carbonyl group,
a 3-amino-piperidin-1-yl group wherein the piperidin-1-yl moiety in the 4 position is additionally substituted by a hydroxy group,
a 3-amino-piperidin-1-yl group wherein a hydrogen atom in the 2 position together with a hydrogen atom in the 5 position is replaced by a -CH₂-CH₂-bridge,
a piperidin-1-yl group which is substituted by an aminomethyl group,
a piperidin-3-yl or piperidin-4-yl group,
a 1-amino-piperidin-3-yl or 1-amino-piperidin-4-yl group,
a hexahydroazepin-1-yl group which is substituted in the 3 position or in the 4 position by an amino group,
a 3-aminopropyl group,
a cyclohexyl group which is substituted by an amino group,
while, unless otherwise stated, the abovementioned alkyl and alkenyl groups may be straight-chain or branched,
the tautomers, enantiomers, diastereomers, mixtures thereof or the salts thereof.

4. Compounds for use according to claim 1, where compounds of general formula I according to claim 1 are used wherein
R¹, R² and R³ are defined as in claim 1, 2 or 3, and
R⁴ denotes a piperidin-1-yl group which is substituted in the 3 position by an amino group, while the piperidin-1-yl group may additionally be substituted by a methyl group,
a 3-amino-piperidin-1-yl group wherein the piperidin-1-yl group is additionally substituted by a pyrrolidin-1-yl-carbonyl group,
a 3-amino-piperidin-1-yl group wherein the piperidin-1-yl group is additionally substituted in the 4 position by a hydroxy group,
a 3-amino-piperidin-1-yl group wherein a hydrogen atom in the 2 position together with a hydrogen atom in the 5 position is replaced by a -CH₂-CH₂- bridge,
a hexahydroazepin-1-yl group which is substituted in the 3 position by an amino group, or
a cyclohexyl group which is substituted in the 3 position by an amino group,
while unless otherwise stated the above-mentioned alkyl and alkenyl groups may be straight-chain or branched,
the tautomers, enantiomers, diastereomers, mixtures thereof or the salts thereof.

5. Compounds for use according to claim 1, where compounds of general formula I according to claim 1 are used wherein
R¹, R³ and R⁴ are defined as in claim 1, 2, 3 or 4, and
R² denotes a hydrogen atom or a C₁₋₈-alkyl group,
while unless otherwise stated the above-mentioned alkyl group may be straight-chain or branched,
the tautomers, enantiomers, diastereomers, mixtures thereof or the salts thereof.

6. Compounds for use according to claim 1, where compounds of general formula I according to claim 1 are used, wherein
R¹, R³ and R⁴ are defined as in claim 1, 2, 3 or 4, and
R² denotes a C₁₋₈-alkyl group,
while unless otherwise stated the above-mentioned alkyl group may be straight-chain or branched,
the tautomers, enantiomers, diastereomers, mixtures thereof or the salts thereof.

7. Compounds of general formula I according to claim 1, wherein
R¹ denotes a C₁₋₆-alkyl group,
a C₃₋₆-alkenyl group,
a C₃₋₄-alkenyl group which is substituted by a C₁₋₂-alkyloxy-carbonyl group,
a C₃₋₆-alkynyl group,
a C₃₋₆-cycloalkyl-C₁₋₃-alkyl group,
a phenyl-C₁₋₄-alkyl group wherein the phenyl moiety is substituted by R¹⁰ to R¹², wherein
R¹⁰ denotes a hydrogen atom, a fluorine, chlorine or bromine atom,
a C₁₋₄-alkyl, trifluoromethyl, hydroxymethyl, C₃₋₆-cycloalkyl, ethynyl or phenyl group,
a hydroxy, C₁₋₄-alkyloxy, difluoromethoxy, trifluoromethoxy, 2,2,2-trifluoroethoxy, phenoxy, benzyloxy, 2-propen-1-yloxy, 2-propyn-1-yloxy, cyano-C₁₋₂-alkyloxy, C₁₋₂-alkylsulphonyloxy, phenylsulphonyloxy, carboxy-C₁₋₃-alkyloxy, C₁₋₃-alkyloxy-carbonyl-C₁₋₃-alkyloxy, aminocarbonyl-C₁₋₃-alkyloxy, C₁₋₂-alkyl-aminocarbonyl-C₁₋₃-alkyloxy, di-(C₁₋₂-alkyl)aminocarbonyl-C₁₋₃-alkyloxy, pyrrolidin-1-yl-carbonyl-C₁₋₃-alkyloxy, piperidin-1-ylcarbonyl-C₁₋₃-alkyloxy, morpholin-4-ylcarbonyl-C₁₋₃-alkyloxy, methylsulphanylmethoxy, methylsulphinylmethoxy, methylsulphonylmethoxy, C₃₋₆-cycloalkyloxy or C₃₋₆-cycloalkyl-C₁₋₂-alkyloxy group,
a carboxy, C₁₋₃-alkyloxycarbonyl, carboxy-C₁₋₃-alkyl, C₁₋₃-alkyloxy-carbonyl-C₁₋₃-alkyl, aminocarbonyl, C₁₋₂-alkylaminocarbonyl, di-(C₁₋₂-alkyl)aminocarbonyl, morpholin-4-ylcarbonyl or cyano group,
a nitro, amino, C₁₋₂-alkylamino, di-(C₁₋₂-alkyl)amino, cyano-C₁₋₂-alkylamino, [N-(cyano-C₁₋₂-alkyl)-N-C₁₋₂-alkyl-amino], C₁₋₂-alkyloxy-carbonyl-C₁₋₂-alkylamino, C₁₋₂-alkyl-carbonylamino, C₁₋₂-alkyloxy-carbonylamino, C₁₋₃-alkylsulphonylamino, bis-(C₁₋₂-alkylsulphonyl)-amino, aminosulphonylamino, C₁₋₂-alkylamino-sulphonylamino, di-(C₁₋₂-alkyl)amino-sulphonylamino, morpholin-4-yl-sulphonylamino, (C₁₋₂-alkylamino)thiocarbonylamino, (C₁₋₂-alkyloxy-carbonyl-amino)carbonylamino, aminocarbonylamino, C₁₋₂-alkylaminocarbonylamino, di-(C₁₋₂-alkyl)aminocarbonylamino or morpholin-4-ylcarbonylamino group,
a 2-oxo-imidazolidin-1-yl, 3-methyl-2-oxo-imidazolidin-1-yl, 2,4-dioxo-imidazolidin-1-yl, 3-methyl-2,4-dioxo-imidazolidin-1-yl, 2,5-dioxo-imidazolidin-1-yl, 3-methyl-2,5-dioxo-imidazolidin-1-yl, 2-oxo-hexahydropyrimidin-1-yl or 3-methyl-2-oxo-hexahydropyrimidin-1-yl group,
or
a C₁₋₂-alkylsulphanyl, C₁₋₂-alkylsulphinyl, C₁₋₂-alkylsulphonyl, aminosulphonyl, C₁₋₂-alkylaminosulphonyl or di-(C₁₋₂-alkyl)aminosulphonyl group,
and R¹¹ and R¹², which may be identical or different, denote a hydrogen, fluorine, chlorine or bromine atom or
a methyl, cyano, trifluoromethyl or methoxy group,
or, R¹¹ together with R¹², if they are bound to adjacent carbon atoms, also denote a methylenedioxy, difluoromethylenedioxy, 1,3-propylene or 1,4-butylene group,
a phenyl-C₁₋₃-alkyl group wherein the alkyl moiety is substituted by a carboxy, C₁₋₂-alkyloxy-carbonyl, aminocarbonyl, C₁₋₂-alkylaminocarbonyl or di-(C₁₋₂-alkyl)amino-carbonyl group,
a phenyl-C₂₋₃-alkenyl group, wherein the phenyl moiety may be substituted by a fluorine, chlorine or bromine atom or by a methyl, trifluoromethyl or methoxy group,
a phenyl-(CH₂)ₘ-A-(CH₂)ₙ group wherein the phenyl moiety is substituted by R¹⁰ to R¹², wherein R¹⁰ to R¹² are as hereinbefore defined and
A denotes a carbonyl, hydroxyiminomethylene or C₁₋₂-alkyloxyiminomethylene group, m denotes the number 0 or 1 and n denotes the number 1 or 2,
a phenylcarbonylmethyl group wherein the phenyl moiety is substituted by R¹⁰ to R¹², wherein R¹⁰ to R¹² are as hereinbefore defined and the methyl moiety is substituted by a methyl or ethyl group,
a phenylcarbonylmethyl group wherein two adjacent hydrogen atoms of the phenyl moiety are replaced by a -O-CO-NH-, -NH-CO-NH-, -N=CH-NH-, -N=CH-O- or -O-CH₂-CO-NH- bridge, wherein the abovementioned bridges may be substituted by one or two methyl groups,
a phenyl-(CH₂)ₘ-B-(CH₂)ₙ group wherein the phenyl moiety is substituted by R¹⁰ to R¹², wherein R¹⁰ to R¹², m and n are as hereinbefore defined and
B denotes a methylene group which is substituted by a hydroxy or C₁₋₂-alkyloxy group and is optionally additionally substituted by a methyl group,
a naphthylmethyl or naphthylethyl group, wherein the naphthyl moiety is substituted in each case by R¹⁰ to R¹², wherein R¹⁰ to R¹² are as hereinbefore defined,
a heteroaryl-C₁₋₃-alkyl group, wherein the term heteroaryl denotes a pyrrolyl, imidazolyl, triazolyl, furanyl, thienyl, oxazolyl, isoxazolyl, thiazolyl, isothiazolyl, pyridyl, pyridazinyl, pyrimidinyl, pyrazinyl, indolyl, benzimidazolyl, 2,3-dihydro-2-oxo-1*H*-benzimidazolyl, indazolyl, benzofuranyl, 2,3-dihydrobenzofuranyl, benzoxazolyl, dihydro-2-oxo-benzoxazolyl, benzoisoxazolyl, benzothiophenyl, benzothiazolyl, benzoisothiazolyl, quinolinyl, 1,2-dihydro-2-oxo-quinolinyl, isoquinolinyl, 1,2-dihydro-1-oxo-isoquinolinyl, cinnolinyl, quinazolinyl, 1,2-dihydro-2-oxo-quinazolinyl, 1,2-dihydro-1-oxo-phthalazin-4-yl, cumarinyl or 3,4-dihydro-3-oxo-2*H*-benzo[1,4]oxazinyl group,
wherein the abovementioned heteroaryl groups may be substituted at carbon atoms by a fluorine, chlorine or bromine atom, by a methyl, trifluoromethyl, cyano, aminocarbonyl, aminosulphonyl, methylsulphonyl, nitro, amino, acetylamino, methylsulphonylamino, methoxy, difluoromethoxy or trifluoromethoxy group and the imino groups of the abovementioned heteroaryl groups may be substituted by methyl or ethyl groups,
a furanyl-A-CH₂, thienyl-A-CH₂, thiazolyl-A-CH₂ or pyridyl-A-CH₂ group, wherein A is as hereinbefore defined,
a furanyl-B-CH₂, thienyl-B-CH₂, thiazolyl-B-CH₂ or pyridyl-B-CH₂ group, wherein B is as hereinbefore defined,
a C₁₋₄-alkyl-A-(CH₂)ₙ group, wherein A and n are as hereinbefore defined,
a C₃₋₆-cycloalkyl-(CH₂)ₘ-A-(CH₂)ₙ group, wherein A, m and n are as hereinbefore defined,
a C₃₋₆-cycloalkyl-(CH₂)ₘ-B-(CH₂)ₙ group, wherein B, m and n are as hereinbefore defined,
a R²¹-A-(CH₂)ₙ group wherein R²¹ denotes a C₁₋₂-alkyloxycarbonyl, aminocarbonyl, C₁₋₂-alkylaminocarbonyl, di-(C₁₋₂-alkyl)aminocarbonyl, pyrrolidin-1-yl-carbonyl, piperidin-1-yl-carbonyl or morpholin-4-yl-carbonyl group and A and n are as hereinbefore defined, a phenyl-D-C₁₋₃-alkyl group wherein the phenyl moiety is optionally substituted by a fluorine, chlorine or bromine atom, a methyl, trifluoromethyl or methoxy group and D denotes an oxygen or sulphur atom, a sulphinyl or sulphonyl group,
a C₁₋₄-alkyl group substituted by a group Rₐ, wherein
Rₐ denotes a cyano, carboxy, C₁₋₃-alkyloxy-carbonyl, aminocarbonyl, C₁₋₂-alkyl-aminocarbonyl, di-(C₁₋₂-alkyl)aminocarbonyl, pyrrolidin-1-yl-carbonyl, piperidin-1-ylcarbonyl or morpholin-4-ylcarbonyl group,
a C₂₋₄-alkyl group substituted by a group R_{b}, wherein
R_{b} denotes a hydroxy, C₁₋₃-alkyloxy, amino, C₁₋₃-alkylamino, di-(C₁₋₃-alkyl)-amino, pyrrolidin-1-yl, piperidin-1-yl, morpholin-4-yl, piperazin-1-yl, 4-methyl-piperazin-1-yl or 4-ethyl-piperazin-1-yl group and is isolated from the cyclic nitrogen atom in the 1 position of the xanthine skeleton by at least two carbon atoms,
R² denotes a hydrogen atom,
a C₁₋₆-alkyl group,
a C₂₋₄-alkenyl group,
a C₃₋₄-alkynyl group,
a C₃₋₆-cycloalkyl group,
a C₃₋₆-cycloalkyl-C₁₋₃-alkyl group,
a tetrahydrofuran-3-yl, tetrahydropyran-3-yl, tetrahydropyran-4-yl, tetrahydrofuranylmethyl or tetrahydropyranylmethyl group,
a phenyl group which is optionally substituted by a fluorine, chlorine or bromine atom or by a methyl, trifluoromethyl, hydroxy, methoxy, difluoromethoxy or trifluoromethoxy group,
a phenyl-C₁₋₄-alkyl group wherein the phenyl moiety is optionally substituted by a fluorine, chlorine or bromine atom, a methyl, trifluoromethyl, dimethylamino, hydroxy, methoxy, difluoromethoxy or trifluoromethoxy group,
a phenyl-C₂₋₃-alkenyl group, wherein the phenyl moiety may be substituted by a fluorine, chlorine or bromine atom or by a methyl, trifluoromethyl or methoxy group,
a phenylcarbonyl-C₁₋₂-alkyl group wherein the phenyl moiety is optionally substituted by a fluorine, chlorine or bromine atom, a methyl, trifluoromethyl, hydroxy, methoxy, difluoromethoxy or trifluoromethoxy group,
a heteroaryl-C₁₋₃-alkyl group, wherein the term heteroaryl is as hereinbefore defined,
a furanylcarbonylmethyl, thienylcarbonylmethyl, thiazolylcarbonylmethyl or pyridylcarbonylmethyl group,
a C₁₋₄-alkyl-carbonyl-C₁₋₂-alkyl group,
a C₃₋₆-cycloalkyl-carbonyl-C₁₋₂-alkyl group,
a phenyl-D-C₁₋₃-alkyl group wherein the phenyl moiety is optionally substituted by a fluorine, chlorine or bromine atom, a methyl, trifluoromethyl, hydroxy, methoxy, difluoromethoxy or trifluoromethoxy group, and D is as hereinbefore defined, or
a C₁₋₄-alkyl group substituted by a group Rₐ, wherein Rₐ is as hereinbefore defined, or a C₂₋₄-alkyl group substituted by a group R_{b}, wherein R_{b} is as hereinbefore defined and is isolated from the cyclic nitrogen atom in the 3 position of the xanthine skeleton by at least two carbon atoms,
R³ denotes a C₁₋₃-alkyl group substituted by the group R_{c}, wherein
R_{c} denotes a C₃₋₇-cydoalkyl group optionally substituted by one or two C₁₋₃-alkyl groups, or
a C₅₋₇-cycloalkenyl group optionally substituted by one or two C₁₋₃-alkyl groups,
a C₃₋₈-alkenyl group,
a C₃₋₆-alkenyl group substituted by a fluorine, chlorine or bromine atom, or a trifluoromethyl group,
a C₃₋₈-alkynyl group, or
an aryl-C₂₋₄-alkenyl group,
and
R⁴ denotes an azetidin-1-yl or pyrrolidin-1-yl group which is substituted in the 3 position by an RₑNR_{d} group and may additionally be substituted by one or two C₁₋₃-alkyl groups,
wherein
Rₑ denotes a hydrogen atom or a C₁₋₃-alkyl group and
R_{d} denotes a hydrogen atom or a C₁₋₃-alkyl group,
a piperidin-1-yl or hexahydroazepin-1-yl group which is substituted in the 3 position or in the 4 position by an RₑNR_{d} group and may additionally be substituted by one or two C₁₋₃-alkyl groups, wherein Rₑ and R_{d} are as hereinbefore defined,
a 3-amino-piperidin-1-yl group wherein the piperidin-1-yl moiety is additionally substituted by an aminocarbonyl, C₁₋₂-alkyl-aminocarbonyl, di-(C₁₋₂-alkyl)aminocarbonyl, pyrrolidin-1-yl-carbonyl, (2-cyano-pyrrolidin-1-yl-)carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety is additionally substituted in the 4 position or in the 5 position by a hydroxy or methoxy group,
a 3-amino-piperidin-1-yl group wherein the methylene group is replaced in the 2 position or in the 6 position by a carbonyl group,
a piperidin-1-yl or hexahydroazepin-1-yl group substituted in the 3 position by an amino, C₁₋₃-alkylamino or di-(C₁₋₃-alkyl)-amino group, wherein in each case two hydrogen atoms on the carbon skeleton of the piperidin-1-yl or hexahydroazepin-1-yl group are replaced by a straight-chain alkylene bridge, this bridge containing 2 to 5 carbon atoms if the two hydrogen atoms are located on the same carbon atom, or 1 to 4 carbon atoms if the hydrogen atoms are located on adjacent carbon atoms, or 1 to 4 carbon atoms if the hydrogen atoms are located on carbon atoms which are separated by one atom, or 1 to 3 carbon atoms if the two hydrogen atoms are located on carbon atoms separated by two atoms,
an azetidin-1-yl, pyrrolidin-1-yl, piperidin-1-yl or hexahydroazepin-1-yl group which is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl group which is substituted by an amino, C₁₋₃-alkylamino or di-(C₁₋₃-alkyl)-amino group,
a C₃₋₇-cycloalkyl group which is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl-C₁₋₂-alkyl group wherein the cycloalkyl moiety is substituted by an amino, C₁₋₃-alkylamino or di-(C₁₋₃-alkyl)-amino group,
a C₃₋₇-cycloalkyl-C₁₋₂-alkyl group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
a R¹⁹-C₃₋₄-alkyl group wherein the C₃₋₄-alkyl moiety is straight-chained and may be substituted by the group R¹⁵ and may additionally be substituted by one or two C₁₋₃-alkyl groups, wherein R¹⁵ denotes a C₁₋₃-alkyl group and R¹⁹ denotes an amino, C₁₋₃-alkylamino or di-(C₁₋₃-alkyl)-amino group,
a 3-amino-2-oxo-piperidin-5-yl or 3-amino-2-oxo-1-methyl-piperidin-5-yl group,
a pyrrolidin-3-yl, piperidin-3-yl, piperidin-4-yl, hexahydroazepin-3-yl or hexahydroazepin-4-yl group, which is substituted in the 1 position by an amino, C₁₋₃-alkylamino or di-(C₁₋₃-alkyl)amino group,
or an azetidin-2-yl-C₁₋₂-alkyl, azetidin-3-yl-C₁₋₂-alkyl, pyrrolidin-2-yl-C₁₋₂-alkyl, pyrrolidin-3-yl, pyrrolidin-3-yl-C₁₋₂-alkyl, piperidin-2-yl-C₁₋₂-alkyl, piperidin-3-yl, piperidin-3-yl-C₁₋₂-alkyl, piperidin-4-yl or piperidin-4-yl-C₁₋₂-alkyl group, wherein the abovementioned groups may each be substituted by one or two C₁₋₃-alkyl groups,
while by the aryl groups mentioned in the definition of the groups mentioned above are meant phenyl or naphthyl groups which may be mono- or disubstituted independently of one another by Rₕ, while the substituents may be identical or different and Rₕ denotes a fluorine, chlorine, bromine or iodine atom, a trifluoromethyl, cyano, nitro, amino, C₁₋₃-alkyl, cyclopropyl, ethenyl, ethynyl, hydroxy, C₁₋₃-alkyloxy, difluoromethoxy or trifluoromethoxy group and
unless otherwise stated, the abovementioned alkyl and alkenyl groups may be straight-chained or branched,
the tautomers, enantiomers, diastereomers, mixtures thereof or the salts thereof;
for use in combination therapy with other active substances, e.g. selected from among the antidiabetic agents, lipid lowering agents, HDL-raising compounds, active substances for the treatment of obesity, and active substances for influencing high blood pressure.

8. Compounds for use according to claim 7, where compounds of general formula I according to claim 1 are used, wherein
R¹ denotes a C₁₋₄-alkyl group,
a C₃₋₅-alkenyl group,
a 2-propen-1-yl group which is substituted by a methoxycarbonyl group,
a C₃₋₅-alkynyl group,
a phenyl-C₁₋₄-alkyl group wherein the phenyl moiety is substituted by R¹⁰ to R¹², wherein
R¹⁰ denotes a hydrogen atom, a fluorine, chlorine or bromine atom,
a methyl, ethyl, trifluoromethyl or ethynyl group,
a hydroxy, methoxy, ethoxy, difluoromethoxy, trifluoromethoxy, 2,2,2-trifluoroethoxy, phenoxy, benzyloxy, 2-propen-1-yloxy, 2-propyn-1-yloxy, cyano-C₁₋₂-alkyloxy, C₁₋₂-alkyl-sulphonyloxy, phenylsulphonyloxy, carboxy-C₁₋₂-alkyloxy, C₁₋₂-alkyloxy-carbonyl-C₁₋₂-alkyloxy, aminocarbonyl-C₁₋₂-alkyloxy, C₁₋₂-alkyl-aminocarbonyl-C₁₋₂-alkyloxy, di-(C₁₋₂-alkyl)aminocarbonyl-C₁₋₂-alkyloxy, pyrrolidin-1-ylcarbonyl-C₁₋₂-alkyloxy, piperidin-1-ylcarbonyl-C₁₋₂-alkyloxy, morpholin-4-ylcarbonyl-C₁₋₂-alkyloxy group,
a carboxy, C₁₋₂-alkyloxy-carbonyl, aminocarbonyl, C₁₋₂-alkylaminocarbonyl, di-(C₁₂-alkyl)aminocarbonyl, morpholin-4-ylcarbonyl or cyano group,
a nitro, amino, C₁₋₂-alkylamino, di-(C₁₋₂-alkyl)amino, cyano-C₁₋₂-alkylamino, [N-(cyano-C₁₋₂-alkyl)-N-methyl-amino], C₁₋₂-alkyloxy-carbonyl-C₁₋₂-alkylamino, C₁₋₂-alkyl-carbonylamino, C₁₋₂-alkyloxy-carbonylamino, C₁₋₂-alkylsulphonylamino, bis-(C₁₋₂-alkylsulphonyl)-amino, aminosulphonylamino, C₁₋₂-alkylamino-sulphonylamino, di-(C₁₋₂-alkyl)amino-sulphonylamino, morpholin-4-yl-sulphonylamino, (C₁₋₂-alkylamino)thiocarbonylamino, (C₁₋₂-alkyloxy-carbonyl-amino)carbonylamino, aminocarbonylamino, C₁₋₂-alkylaminocarbonylamino, di-(C₁₋₂-alkyl)aminocarbonylamino or morpholin-4-yl-carbonylamino group,
a 2-oxo-imidazolidin-1-yl, 3-methyl-2-oxo-imidazolidin-1-yl, 2,4-dioxo-imidazolidin-1-yl, 3-methyl-2,4-dioxo-imidazolidin-1-yl, 2,5-dioxo-imidazolidin-1-yl, 3-methyl-2,5-dioxo-imidazolidin-1-yl, 2-oxo-hexahydropyrimidin-1-yl or 3-methyl-2-oxo-hexahydropyrimidin-1-yl group,
or
a C₁₋₂-alkylsulphanyl, C₁₋₂-alkylsulphinyl, C₁₋₂-alkylsulphonyl, aminosulphonyl, C₁₋₂-alkylaminosulphonyl or di-(C₁₋₂-alkyl)aminosulphonyl group,
and R¹¹ and R¹², which may be identical or different, denote a hydrogen, fluorine, chlorine or bromine atom or
a methyl, cyano or methoxy group,
or, R¹¹ together with R¹², if they are bound to adjacent carbon atoms, also denote a methylenedioxy group,
a phenylmethyl group wherein the methyl moiety is substituted by a carboxy, methoxycarbonyl or aminocarbonyl group,
a 2-phenylethyl group wherein the ethyl moiety is substituted by a carboxy, methoxycarbonyl or aminocarbonyl group,
a 2-phenylethyl group wherein the ethyl moiety is substituted in the 2 position by a hydroxy, methoxy, hydroxyimino or methoxyimino group,
a 2-phenylethyl group wherein the ethyl moiety is substituted in the 2 position by a hydroxy group and a methyl group,
a phenylcarbonylmethyl group wherein the phenyl moiety is substituted by R¹⁰ to R¹², wherein R¹⁰ to R¹² are as hereinbefore defined,
a 1-(phenylcarbonyl)ethyl or 2-(phenylcarbonyl)ethyl group,
a 2-phenylethenyl group,
a phenylsulphanylmethyl or phenylsulphinylmethyl group,
a 2-(phenyloxy)ethyl group,
a naphthylmethyl or naphthylethyl group, wherein the naphthyl moiety may be substituted in each case by a methyl, nitro, amino, acetylamino, methylsulphonylamino, cyano, aminocarbonyl or aminosulphonyl group,
an oxazolylmethyl, isoxazolylmethyl, thiazolylmethyl, pyridylmethyl, benzofuranylmethyl, 2,3-dihydrobenzofuranylmethyl, benzo[d]isoxazolylmethyl, benzo[d]isothiazolylmethyl, (1*H*-indazol-3-yl)methyl, quinolinylmethyl, (1,2-dihydro-2-oxo-quinolin-4-yl)methyl, isoquinolinylmethyl, (1,2-dihydro-1-oxo-isoquinolin-4-yl)methyl, cinnolinylmethyl, quinazolinylmethyl, (1,2-dihydro-2-oxo-quinazolin-4-yl)methyl, (1,2-dihydro-1-oxo-phthalazin-4-yl)methyl or cumarinylmethyl group, wherein the heterocyclic moiety may be substituted by a methyl group in each case,
a quinolinylmethyl or isoquinolinylmethyl group, wherein the heterocyclic moiety is substituted in each case by a cyano, nitro, amino, acetylamino, methylsulphonylamino, aminocarbonyl or aminosulphonyl group,
a pyrrolylethyl, triazolylethyl, thienylethyl, thiazolylethyl or pyridylethyl group, wherein the heterocyclic moiety may be substituted in each case by a methyl group,
a furanylcarbonylmethyl, thienylcarbonylmethyl, thiazolylcarbonylmethyl or pyridylcarbonylmethyl group,
a methyl group which is substituted by a cyclopropyl, cyano, carboxy, aminocarbonyl or methoxycarbonyl group,
an ethyl group which is substituted in the 2 position by a hydroxy, methoxy, dimethylamino, carboxy or methoxycarbonyl group, or
a propyl group which is substituted in the 3 position by a hydroxy, dimethylamino, carboxy or methoxycarbonyl group,
a 2-oxopropyl group,
R² denotes a hydrogen atom,
a C₁₋₆-alkyl group,
an ethenyl group,
a 2-propen-1-yl or 2-propyn-1-yl group,
a C₃₋₆-cycloalkyl group,
a tetrahydrofuran-3-yl, tetrahydropyran-3-yl, tetrahydropyran-4-yl, tetrahydrofuranylmethyl or tetrahydropyranylmethyl group,
a phenyl group,
a phenyl-C₁₋₄-alkyl group, wherein the phenyl moiety may be substituted by a fluorine or chlorine atom, a methyl, dimethylamino, hydroxy, methoxy or trifluoromethoxy group,
a phenylcarbonylmethyl group, wherein the phenyl moiety may be substituted by a fluorine or chlorine atom, a hydroxy, methoxy or trifluoromethoxy group,
a 2-phenylethenyl group,
a 2-(phenyloxy)ethyl group,
a pyridylmethyl or pyridylethyl group,
a methyl group which is substituted by a C₃₋₆-cycloalkyl, cyano, carboxy or methoxycarbonyl group, or
an ethyl group which is substituted in the 2 position by a C₃₋₆-cycloalkyl, cyano, carboxy, methoxycarbonyl, hydroxy, methoxy or dimethylamino group,
or a propyl group which is substituted in the 3 position by a C₃₋₆-cycloalkyl, cyano, carboxy, methoxycarbonyl, hydroxy, methoxy or dimethylamino group,
R³ denotes a C₄₋₆-alkenyl group,
a 1-cyclopenten-1-ylmethyl or 1-cyclohexen-1-ylmethyl group,
a 1-cyclopenten-1-ylmethyl group wherein the 1-cyclopenten-1-yl moiety is substituted by a methyl group,
a 2-propyn-1-yl, 2-butyn-1-yl or 2-pentyn-1-yl group,
a cyclopropylmethyl group or
a cyclopropylmethyl group wherein the cyclopropyl moiety is substituted by a methyl group, and
R⁴ denotes a piperidin-1-yl group which is substituted in the 3 position by an amino group, wherein the piperidin-1-yl moiety may additionally be substituted by a methyl group,
a 3-amino-piperidin-1-yl group wherein the piperidin-1-yl moiety is additionally substituted by an aminocarbonyl, methylaminocarbonyl, dimethylaminocarbonyl, pyrrolidin-1-yl-carbonyl, (2-cyano-pyrrolidin-1-yl-)carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety in the 4 position or in the 5 position is additionally substituted by a hydroxy or methoxy group,
a 3-amino-piperidin-1-yl group wherein a hydrogen atom in the 2 position together with a hydrogen atom in the 5 position is replaced by a -CH₂-CH₂-bridge,
a hexahydroazepin-1-yl group which is substituted in the 3 position by an amino group, a 3-amino-2-oxo-piperidin-5-yl or 3-amino-2-oxo-1-methyl-piperidin-5-yl group, or
a cyclohexyl group which is substituted in the 3 position by an amino group,
while unless otherwise stated, the abovementioned alkyl and alkenyl groups may be straight-chained or branched,
the tautomers, enantiomers, diastereomers, mixtures thereof or the salts thereof.

9. Compounds for use according to claim 7, where compounds of general formula I according to claim 1 are used, wherein
R¹ denotes a C₁₋₄-alkyl group,
a C₃₋₅-alkenyl group,
a 2-propen-1-yl group which is substituted by a methoxycarbonyl group,
a C₃₋₅-alkynyl group,
a phenyl-C₁₋₄-alkyl group wherein the phenyl moiety may be substituted by one or two fluorine atoms, one or two chlorine atoms, a bromine atom, one to three methyl groups, a trifluoromethyl, hydroxy, methoxy, nitro, amino, carboxy or ethoxycarbonyl group,
a 2-phenylethyl group wherein the ethyl moiety is substituted in the 2 position by a hydroxy, methoxy or hydroxyimino group,
a phenylcarbonylmethyl group wherein the phenyl moiety may be substituted by a fluorine atom or by a methyl, aminocarbonyl, aminosulphonyl, cyano, hydroxy, methoxy, phenoxy, benzyloxy, 2-propen-1-yloxy, 2-propyn-1-yloxy, cyanomethoxy, (methoxycarbonyl)methoxy, (aminocarbonyl)methoxy, (methylaminocarbonyl)methoxy, (dimethylaminocarbonyl)methoxy, methylsulphonyloxy, phenylsulphonyloxy, nitro, amino, (methoxycarbonyl)methylamino, acetylamino, methoxycarbonylamino, methylsulphonylamino, bis-(methylsulphonyl)-amino, aminocarbonylamino, dimethylaminocarbonylamino, (methylamino)thiocarbonylamino, (ethoxycarbonyl-amino)carbonylamino or cyanomethylamino group,
a phenylcarbonylmethyl group wherein the phenyl moiety is substituted by two methoxy groups or by a bromine atom and by a dimethylamino group,
a 2-(phenylcarbonyl)ethyl group,
a 2-phenylethenyl group,
a 2-(phenoxy)ethyl group,
a phenylsulphanylmethyl or phenylsulphinylmethyl group,
a naphthylmethyl or naphthylethyl group,
an isoxazolylmethyl, thiazolylmethyl, pyridylmethyl, benzo[d]isoxazolylmethyl, benzo[d]isothiazolylmethyl, (1*H*-indazol-3-yl)methyl, quinolinylmethyl or iso-quinolinylmethyl group, wherein the heterocyclic moiety may be substituted in each case by a methyl group,
an isoquinolinylmethyl group wherein the isoquinolinyl moiety is substituted by a nitro or amino group,
a (1,2-dihydro-2-oxo-quinolin-4-yl)methyl group,
a pyrrolylethyl, triazolylethyl, thienylethyl, thiazolylethyl or pyridylethyl group, wherein the heterocyclic moiety may be substituted in each case by a methyl group,
a thienylcarbonylmethyl group,
a methyl group which is substituted by a cyclopropyl, cyano, carboxy, aminocarbonyl or methoxycarbonyl group,
an ethyl group which is substituted in the 2 position by a hydroxy, methoxy, dimethylamino, carboxy or methoxycarbonyl group, or
a propyl group which is substituted in the 3 position by a hydroxy, dimethylamino, carboxy or methoxycarbonyl group, or
a 2-oxopropyl group,
R² denotes a hydrogen atom,
a C₁₋₆-alkyl group,
an ethenyl group,
a 2-propen-1-yl or 2-propyn-1-yl group,
a phenyl group,
a phenyl-C₁₋₄-alkyl group wherein the phenyl moiety may be substituted by a fluorine atom, a methyl or methoxy group,
a phenylcarbonylmethyl group,
a 2-phenylethenyl group,
a methyl group which is substituted by a cyclopropyl, cyano, carboxy or methoxycarbonyl group, or
an ethyl group which is substituted in the 2 position by a cyano, hydroxy, methoxy or dimethylamino group,
R³ denotes a C₄₋₆-alkenyl group,
a 1-cyclopenten-1-ylmethyl or 1-cyclohexen-1-ylmethyl group,
a 2-propyn-1-yl, 2-butyn-1-yl or 2-pentyn-1-yl group, or
a cyclopropylmethyl group and
R⁴ denotes a piperidin-1-yl group which is substituted in the 3 position by an amino group, wherein the piperidin-1-yl moiety may additionally be substituted by a methyl group,
a 3-amino-piperidin-1-yl group wherein the piperidin-1-yl moiety is additionally substituted by a pyrrolidin-1-yl-carbonyl group,
a 3-amino-piperidin-1-yl group wherein the piperidin-1-yl moiety is additionally substituted in the 4 position by a hydroxy group,
a 3-amino-piperidin-1-yl group wherein a hydrogen atom in the 2 position together with a hydrogen atom in the 5 position is replaced by a -CH₂-CH₂-bridge,
a hexahydroazepin-1-yl group which is substituted in the 3 position by an amino group, or
a cyclohexyl group which is substituted in the 3 position by an amino group,
while unless otherwise stated, the abovementioned alkyl and alkenyl groups may be straight-chained or branched,
the tautomers, enantiomers, diastereomers, mixtures thereof or the salts thereof.

10. Compounds for use according to claim 7, where compounds of general formula I according to claim 1 are used wherein
R¹, R² and R³ are defined as in claim 7 and
R⁴ denotes an azetidin-1-yl or pyrrolidin-1-yl group which is substituted in the 3 position by a RₑNR_{d} group and may additionally be substituted by one or two C₁₋₃-alkyl groups,
wherein
Rₑ denotes a hydrogen atom or a C₁₋₃-alkyl group and
R_{d} denotes a hydrogen atom or a C₁₋₃-alkyl group,
a piperidin-1-yl or hexahydroazepin-1-yl group which is substituted in the 3 position or in the 4 position by a RₑNR_{d} group and may additionally be substituted by one or two C₁₋₃-alkyl groups, wherein Rₑ and R_{d} are as hereinbefore defined,
a 3-amino-piperidin-1-yl group wherein the piperidin-1-yl moiety is additionally substituted by an aminocarbonyl, C₁₋₂-alkyl-aminocarbonyl, di-(C₁₋₂-alkyl)aminocarbonyl, pyrrolidin-1-yl-carbonyl, (2-cyano-pyrrolidin-1-yl-)carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety is additionally substituted in the 4 position or in the 5 position by a hydroxy or methoxy group,
a 3-amino-piperidin-1-yl group wherein the methylene group in the 2 position or in the 6 position is replaced by a carbonyl group,
a piperidin-1-yl or hexahydroazepin-1-yl group substituted in the 3 position by an amino, C₁₋₃-alkylamino or di-(C₁₋₃-alkyl)-amino group, wherein in each case two hydrogen atoms on the carbon skeleton of the piperidin-1-yl or hexahydroazepin-1-yl group are replaced by a straight-chain alkylene bridge, this bridge containing 2 to 5 carbon atoms if the two hydrogen atoms are located on the same carbon atom, or 1 to 4 carbon atoms, if the hydrogen atoms are located on adjacent carbon atoms, or 1 to 4 carbon atoms, if the hydrogen atoms are located on carbon atoms separated by one atom, or 1 to 3 carbon atoms if the two hydrogen atoms are located on carbon atoms separated by two atoms,
an azetidin-1-yl, pyrrolidin-1-yl, piperidin-1-yl or hexahydroazepin-1-yl group which is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl group which is substituted by an amino, C₁₋₃-alkylamino or di-(C₁₋₃-alkyl)-amino group,
a C₃₋₇-cycloalkyl group which is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl-C₁₋₂-alkyl group wherein the cycloalkyl moiety is substituted by an amino, C₁₋₃-alkylamino or di-(C₁₋₃-alkyl)-amino group,
a C₃₋₇-cycloalkyl-C₁₋₂-alkyl group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
an R¹⁹-C₃₋₄-alkyl group wherein the C₃₋₄-alkyl moiety is straight-chained and may be substituted by the group R¹⁵ and may additionally be substituted by one or two C₁₋₃-alkyl groups, wherein R¹⁵ denotes a C₁₋₄-alkyl group and R¹⁹ denotes an amino, C₁₋₃-alkylamino or di-(C₁₋₃-alkyl)-amino group,
a 3-amino-2-oxo-piperidin-5-yl or 3-amino-2-oxo-1-methyl-piperidin-5-yl group,
a pyrrolidin-3-yl, piperidin-3-yl, piperidin-4-yl, hexahydroazepin-3-yl or hexahydroazepin-4-yl group, which is substituted in the 1 position by an amino, C₁₋₃-alkylamino or di-(C₁₋₃-alkyl)amino group,
or an azetidin-2-yl-C₁₋₂-alkyl, azetidin-3-yl-C₁₋₂-alkyl, pyrrolidin-2-yl-C₁₋₂-alkyl, pyrrolidin-3-yl, pyrrolidin-3-yl-C₁₋₂-alkyl, piperidin-2-yl-C₁₋₂-alkyl, piperidin-3-yl, piperidin-3-yl-C₁₋₂-alkyl, piperidin-4-yl or piperidin-4-yl-C₁₋₂-alkyl group, wherein the abovementioned groups may each be substituted by one or two C₁₋₃-alkyl groups,
the tautomers, enantiomers, diastereomers, mixtures thereof or the salts thereof.

11. Compounds for use according to claim 7, where compounds of general formula I according to claim 1 are used, wherein
R¹, R² and R³ are defined as in claim 7 or claim 8 and
R⁴ denotes a piperidin-1-yl group which is substituted in the 3 position by an amino group, wherein the piperidin-1-yl moiety may additionally be substituted by a methyl group,
a 3-amino-piperidin-1-yl group wherein the piperidin-1-yl moiety is additionally substituted by an aminocarbonyl, methylaminocarbonyl, dimethylaminocarbonyl, pyrrolidin-1-yl-carbonyl, (2-cyano-pyrrolidin-1-yl-)carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety is additionally substituted in the 4 position or in the 5 position by a hydroxy or methoxy group,
a 3-amino-piperidin-1-yl group wherein a hydrogen atom in the 2 position together with a hydrogen atom in the 5 position is replaced by a -CH₂-CH₂-bridge,
a hexahydroazepin-1-yl group which is substituted in the 3 position by an amino group, a 3-amino-2-oxo-piperidin-5-yl or 3-amino-2-oxo-1-methyl-piperidin-5-yl group,
a cyclohexyl group which is substituted in the 3 position by an amino group,
while unless otherwise stated, the abovementioned alkyl- and alkenyl groups may be straight-chained or branched,
the tautomers, enantiomers, diastereomers, mixtures thereof or the salts thereof.

12. Compounds for use according to claim 7, where compounds of general formula I according to claim 1 are used, wherein
R¹, R² and R³ are defined as in claim 7, 8 or 9 and
R⁴ denotes a piperidin-1-yl group which is substituted in the 3 position by an amino group, wherein the piperidin-1-yl moiety may additionally be substituted by a methyl group,
a 3-amino-piperidin-1-yl group wherein the piperidin-1-yl moiety is additionally substituted by a pyrrolidin-1-yl-carbonyl group,
a 3-amino-piperidin-1-yl group wherein the piperidin-1-yl moiety in the 4 position is additionally substituted by a hydroxy group,
a 3-amino-piperidin-1-yl group wherein a hydrogen atom in the 2 position together with a hydrogen atom in the 5 position is replaced by a -CH₂-CH₂-bridge,
a hexahydroazepin-1-yl group which is substituted in the 3 position by an amino group, a cyclohexyl group which is substituted in the 3 position by an amino group,
while unless otherwise stated the abovementioned alkyl- and alkenyl groups may be straight-chained or branched,
the tautomers, enantiomers, diastereomers, mixtures thereof or the salts thereof.

13. The following compounds of general formula I according to claim 1:
(1) 1,3-dimethyl-7-benzyl-8-(3-amino-pyrrolidin-1-yl)-xanthine,
(2) 1,3-dimethyl-7-(3-methyl-2-buten-1-yl)-8-(3-amino-pyrrolidin-1-yl)-xanthine,
(3) 1,3-dimethyl-7-benzyl-8-(3-amino-piperidin-1-yl)-xanthine,
(4) 1,3-dimethyl-7-(3-methyl-2-buten-1-yl)-8-[(trans-2-amino-cyclohexyl)amino]-xanthine,
(5) 1,3-dimethyl-7-(3-methyl-2-buten-1-yl)-8-(3-amino-piperidin-1-yl)-xanthine,
(6) 1,3-dimethyl-7-(3-methyl-2-buten-1-yl)-8-(4-amino-piperidin-1-yl)-xanthine,
(7) 1,3-dimethyl-7-(3-methyl-2-buten-1-yl)-8-[(cis-2-amino-cyclohexyl)amino]-xanthine,
(8) 1,3-dimethyl-7-(2-butyn-1-yl)-8-(3-amino-piperidin-1-yl)-xanthine,
(9) 1,3-dimethyl-7-[(1-cyclopenten-1-yl)methyl]-8-(3-amino-piperidin-1-yl)-xanthine,
(10) 1,3-dimethyl-7-(2-thienylmethyl)-8-(3-amino-piperidin-1-yl)-xanthine
(11) 1,3-dimethyl-7-(3-fluorobenzyl)-8-(3-amino-piperidin-1-yl)-xanthine,
(12) 1,3-dimethyl-7-(2-fluorobenzyl)-8-(3-amino-piperidin-1-yl)-xanthine,
(13) 1,3-dimethyl-7-(4-fluorobenzyl)-8-(3-amino-piperidin-1-yl)-xanthine,
(14) 1,3-dimethyl-7-(2-buten-1-yl)-8-(3-amino-piperidin-1-yl)-xanthine,
(15) 1,3-bis-(cyclopropylmethyl)-7-benzyl-8-(3-amino-piperidin-1-yl)-xanthine,
(16) (R)-1,3-dimethyl-7-(3-methyl-2-buten-1-yl)-8-(3-amino-piperidin-1-yl)-xanthine,
(17) (S)-1,3-dimethyl-7-(3-methyl-2-buten-1-yl)-8-(3-amino-piperidin-1-yl)-xanthine,
(18) 1,3-dimethyl-7-(3-methyl-2-buten-1-yl)-8-(3-amino-hexahydroazepin-1-yl)-xanthine,
(19) 1,3-dimethyl-7-(3-methyl-2-buten-1-yl)-8-(4-amino-hexahydroazepin-1-yl)-xanthine,
(20) 1,3-dimethyl-7-(3-methyl-2-buten-1-yl)-8-(cis-3-amino-cyclohexyl)-xanthine-hydrochloride,
(21) 1,3-dimethyl-7-(3-methyl-2-buten-1-yl)-8-(3-methylamino-piperidin-1-yl)-xanthine,
(22) 1-(2-phenylethyl)-3-methyl-7-(3-methyl-2-buten-1-yl)-8-(3-amino-piperidin-1-yl)-xanthine,
(23) 1,3-dimethyl-7-(3-methyl-2-buten-1-yl)-8-[N-(2-aminoethyl)-methylamino]-xanthine,
(24) 1-[2-(thiophen-2-yl)-ethyl]-3-methyl-7-(3-methyl-2-buten-1-yl)-8-(3-amino-piperidin-1-yl)-xanthine,
(25) 1-[2-(thiophen-3-yl)-ethyl]-3-methyl-7-(3-methyl-2-buten-1-yl)-8-(3-amino-piperidin-1-yl)-xanthine,
(26) 1-[2-(2-methyl-phenyl)-ethyl]-3-methyl-7-(3-methyl-2-buten-1-yl)-8-(3-amino-piperidin-1-yl)-xanthine,
(27) 1-[2-(3-methyl-phenyl)-ethyl]-3-methyl-7-(3-methyl-2-buten-1-yl)-8-(3-amino-piperidin-1-yl)-xanthine,
(28) 1-[2-(3-methoxy-phenyl)-ethyl]-3-methyl-7-(3-methyl-2-buten-1-yl)-8-(3-amino-piperidin-1-yl)-xanthine,
(29) 1-((E)-2-phenyl-vinyl)-3-methyl-7-(3-methyl-2-buten-1-yl)-8-(3-amino-piperidin-1-yl)-xanthine,
(30) 1-(2-phenyl-ethyl)-3-methyl-7-(3-methyl-2-buten-1-yl)-8-((*S*)-3-amino-piperidin-1-yl)-xanthine,
(31) 1-(2-phenyl-ethyl)-3-methyl-7-(3-methyl-2-buten-1-yl)-8-((*R*)-3-amino-piperidin-1-yl)-xanthine,
(32) 1-[2-(2-methoxy-phenyl)-2-oxo-ethyl]-3-methyl-7-(3-methyl-2-buten-1-yl)-8-(3-amino-piperidin-1-yl)-xanthine,
(33) 1-[2-(thiophen-3-yl)-2-oxo-ethyl]-3-methyl-7-(3-methyl-2-buten-1-yl)-8-(3-amino-piperidin-1-yl)-xanthine,
(34) 1-(2-phenyl-2-oxo-ethyl)-3-methyl-7-(3-methyl-2-buten-1-yl)-8-((*S*)-3-amino-piperidin-1-yl)-xanthine,
(35) 1-(2-phenyl-2-oxo-ethyl)-3-methyl-7-(3-methyl-2-buten-1-yl)-8-((*R*)-3-amino-piperidin-1-yl)-xanthine,
(36) 1-[(isoquinolin-1-yl)methyl]-3-methyl-7-(3-methyl-2-buten-1-yl)-8-((*R*)-3-amino-piperidin-1-yl)-xanthine,
(37) 1-[(isoquinolin-1-yl)methyl]-3-methyl-7-(3-methyl-2-buten-1-yl)-8-((*S*)-3-amino-piperidin-1-yl)-xanthine and
(38) 1-[(1-naphthyl)methyl]-3-methyl-7-(3-methyl-2-buten-1-yl)-8-(3-amino-piperidin-1-yl)-xanthine
or the salts thereof;
for use in combination therapy with other active substances, e.g. selected from among the antidiabetic agents, lipid lowering agents, HDL-raising compounds, active substances for the treatment of obesity, and active substances for influencing high blood pressure.

14. Physiologically acceptable salts of the compounds of general formula I according to at least one of claims 1 to 13 with inorganic or organic acids or bases;
for use in combination therapy with other active substances, e.g. selected from among the antidiabetic agents, lipid lowering agents, HDL-raising compounds, active substances for the treatment of obesity, and active substances for influencing high blood pressure.

15. The compounds of general formula I, or the physiologically acceptable salts thereof, for use according to at least one of claims 1 to 14, the other active substances being selected from among the antidiabetic agents, lipid lowering agents, HDL-raising compounds, active substances for the treatment of obesity, and active substances for influencing high blood pressure.

16. Medicaments containing a compound of general formula I according to at least one of claims 1 to 13 or a physiologically acceptable salt according to claim 14 optionally together with one or more inert carriers and/or diluents;
for use in combination therapy with other active substances, e.g. selected from among the antidiabetic agents, lipid lowering agents, HDL-raising compounds, active substances for the treatment of obesity, and active substances for influencing high blood pressure.

17. A compound of general formula I or combination according to at least one of claims 1 to 14 for the prevention or treatment of diseases or conditions which are connected to an increased DPP-IV activity or which can be prevented or alleviated by reducing the DPP-IV activity; for example, for treating type I or type II diabetes mellitus, complications of diabetes, insulin resistance, dyslipidaemias, atherosclerosis, arthritis, obesity, allograft transplantation or osteoporosis caused by calcitonin.

18. Process for preparing a medicament for use according to claim 16, **characterised in that** a compound of general formula I according to at least one of claims 1 to 14 is incorporated in one or more inert carriers and/or diluents by a non-chemical method.

19. Process for preparing the compounds of general formula I for use according to claims 1 to 14, **characterised in that**
a) in order to prepare compounds of general formula I wherein R⁴ is one of the groups mentioned in claim 1 linked to the xanthine skeleton via a nitrogen atom:
a compound of general formula 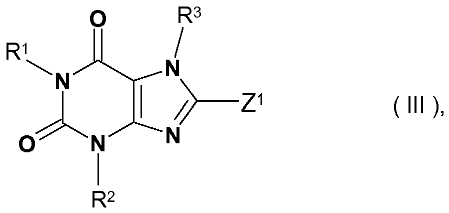 wherein
R¹ to R³ are defined as in claims 1 to 14 and
Z¹ denotes a leaving group such as a halogen atom, a substituted hydroxy, mercapto, sulphinyl, sulphonyl or sulphonyloxy group such as a chlorine or bromine atom, a methanesulphonyl or methanesulphonyloxy group, is reacted with a compound of general formula
H - R⁴, (IV),
wherein
R⁴, denotes one of the groups defined for R⁴ in claims 1 to 14 which is linked to the xanthine skeleton of general formula I via a nitrogen atom,
or
b) In order to prepare compounds of general formula I wherein R⁴ according to the definition in claim 1 contains an amino group or an alkylamino group optionally substituted in the alkyl moiety:
a compound of general formula 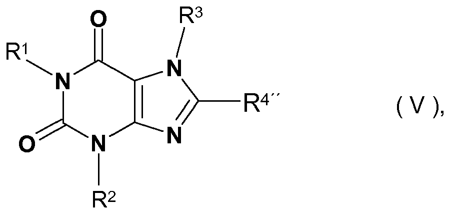
wherein R¹, R² and R³ are defined as in claims 1 to 14 and
R⁴" contains an N-tert.-butyloxycarbonylamino group or an N-tert.-butyloxycarbonyl-N-alkylamino group, wherein the alkyl moiety of the N-tert.-butyloxycarbonyl-N-alkylamino group may be substituted as in claims 1 to 4,
is deprotected,
or
c) In order to prepare a compound of general formula I wherein R² as defined in claim 1 denotes a hydrogen atom:
a compound of general formula 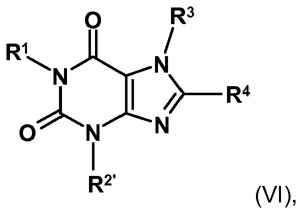
wherein R¹, R³ and R⁴ are as hereinbefore defined and R^{2'} denotes a protecting group such as a methoxymethyl, benzyloxymethyl, methoxyethoxymethyl or 2-(trimethylsilyl)-ethyloxymethyl group,
is deprotected;
while a compound of general formula I thus obtained which contains an amino, alkylamino or imino group may be converted by acylation or sulphonylation into a corresponding acyl or sulphonyl compound of general formula I;
a compound of general formula I thus obtained which contains an amino, alkylamino or imino group may be converted by alkylation or reductive alkylation into a corresponding alkyl compound of general formula I;
a compound of general formula I thus obtained which contains a nitro group may be converted by reduction into a corresponding amino compound;
a compound of general formula I thus obtained which contains an imino group may be converted by nitrosation and subsequent reduction into a corresponding N-amino-imino compound;
a compound of general formula I thus obtained which contains a C₁₋₃-alkyloxycarbonyl group may be converted by cleavage of the ester into the corresponding carboxy compound;
a compound of general formula I thus obtained wherein R¹ contains a carbonyl group may be converted by reaction with hydroxylamine into a corresponding oxime of general formula I;
a compound of general formula I thus obtained which contains a carboxy group may be converted by esterification into a corresponding ester of general formula I; or
a compound of general formula I thus obtained which contains a carboxy or ester group may be converted by reaction with an amine into a corresponding amide of general formula I.

20. Medicament comprising a compound of general formula I according to at least one of claims 1 to 13 or a physiologically acceptable salt according to claim 14 as well as another active substance selected for example from among the antidiabetic agents, optionally together with one or more inert carriers and/or diluents.

21. Medicament according to claim 20, wherein the active substance is selected from among the antidiabetic agents comprising metformin, sulfonylureas, nateglinides, repaglinides, thiazolidinediones, PPAR-gamma-agonists, alpha-glucosidase inhibitors, alpha2-antagonists, insulin and insulin analogues, GLP-1 and GLP-1 analogues and amylin.

22. Process for preparing a medicament according to claim 20, **characterised in that** a compound of general formula I according to at least one of claims 1 to 14 combined with another active substance is incorporated in one or more inert carriers and/or diluents by a non-chemical method.

23. The compound of general formula I according to at least one of claims 1 to 14 for use according to claim 17, wherein the compound of general formula I according to at least one of claims 1 to 14 is used in conjunction with another active substance, selected for example from among the antidiabetic agents, lipid lowering agents, HDL-raising compounds, active substances for treating obesity, and active substances for influencing high blood pressure.

24. The compounds of general formula I according to at least one of claims 1 to 13 or a physiologically acceptable salt according to claim 14 for use in combination therapy with other antidiabetic agents.

25. The compounds of general formula I according to at least one of claims 1 to 13 or a physiologically acceptable salt according to claim 14 for use in combination therapy with other active substances selected from among the antidiabetic agents comprising metformin, sulfonylureas, nateglinides, repaglinides, thiazolidinediones, PPAR-gamma-agonists, alpha-glucosidase inhibitors, alpha2-antagonists, insulin and insulin analogues, GLP-1 and GLP-1 analogues and amylin.

26. The compounds of general formula I according to at least one of claims 1 to 14, wherein
the carboxy groups mentioned in the definition of the above-mentioned groups may be replaced by a group that can be converted *in vivo* into a carboxy group or by a group that is negatively charged under physiological conditions, and/or
the amino and imino groups mentioned in the definition of the above-mentioned groups may be substituted by a group that can be cleaved *in vivo*.

## Revendications

1. Composés de formule générale I 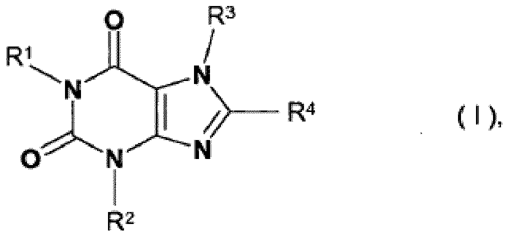 dans laquelle
R¹ est un groupe alkyle en C₁ à C₈,
un groupe alcényle en C₃ à C₈,
un groupe alcényle en C₃ à C₄ qui est substitué par un groupe alkyloxy en C₁ à C₂-carbonyle, aminocarbonyle, alkylamino en C₁ à C₃-carbonyle, di-(alkyle en C₁ à C₃)-aminocarbonyle, pyrrolidin-1-ylcarbonyle, pipéridin-1-ylcarbonyle ou morpholin-4-ylcarbonyle,
un groupe alcinyle en C₃ à C₈,
un groupe alkyle en C₁ à C₆ substitué par un groupe Rₐ, dans lequel
Rₐ désigne un groupe cycloalkyle en C₃ à C₇, hétéroaryle, cyano, carboxy, alkyloxy en C₁ à C₃, carbonyle, aminocarbonyle, alkylamino en C₁ à C₃-carbonyle, di-(alkyle en C₁ à C₃)-aminocarbonyle, pyrrolidin-lylcarbonyle, pipéridin-1-ylcarbonyle, morpholin-4-yl-carbonyle, pipérazin-1-ylcarbonyle, 4-méthylpipérazin-1-ylcarbonyle ou 4-éthylpipérazin-1-ylcarbonyle,
un groupe alkyle en C₁ à C₆ substitué par un groupe phényle, le groupe phényle étant substitué par les groupes R¹⁰ à R¹⁴, et
R¹⁰ est un atome d'hydrogène,
un atome de fluor, de chlore, de brome ou d'iode,
un groupe alkyle en C₁ à C₄, hydroxy ou alkyloxy en C₁ à C₄,
un groupe nitro, amino, alkylamino en C₁ à C₃, di-(alkyle en C₁ à C₃)amino, cyano-alkyle en C₁ à C₃-amino, [N-(cyano-alkyle en C₁ à C₃)-N-alkyle en C₁ à C₃-amino], alkyloxy en C₁ à C₃-carbonyl-alkylamino en C₁ à C₃, pyrrolidin-1-yle, pipéridin-1-yle, morpholin-4-yle, pipérazin-1-yle ou 4-(alkyle en C₁ à C₃) -pipérazin-1-yle,
un groupe alkyle en C₁ à C₃-carbonylamino, arylcarbonylamino, aryl-alkyle en C₁ à C₃-carbonylamino, alkyloxy en C₁ à C₃-carbonylamino, aminocarbonylamino, alkyle en C₁ à C₃-aminocarbonylamino, di-(alkyle en C₁ à C₃)aminocarbonylamino, pyrrolidin-1-yl-carbonylamino, pipéridin-1-yl-carbonylamino, morpholin-4-ylcarbonylamino, pipérazin-1-yl-carbonylamino ou 4-(alkyle en C₁ à C₃)-pipérazin-1-yl-carbonyl-amino, alkyle en C₁ à C₃-sulfonylamino, bis-(alkylsulfonyle en C₁ à C₃)-amino, amino-sulfonylamino, alkylamino en C₁ à C₃-sulfonylamino, di- (alkyle en C₁ à C₃) amino-sulfonylamino, pyrrolidin-1-yl-sulfonylamino, pipéridin-1-yl-sulfonylamino, morpholin-4-yl-sulfonylamino, pipérazin-1-yl-sulfonylamino ou 4-(alkyle en C₁ à C₃)-pipérazin-1-yl-sulfonylamino, (alkylamino en C₁ à C₃)thiocarbonylamino, (alkyloxy en C₁ à C₃-carbonylamino)carbonylamino, arylsulfonylamino ou aryl-alkyle en C₁ à C₃-sulfonylamino,
un groupe N- (alkyle en C₁ à C₃) -alkyle en C₁ à C₃-carbonylamino, N-(alkyle en C₁ à C₃) - arylcarbonylamino, N-(alkyle en C₁ à C₃)-aryl-alkyle en C₁ à C₃-carbonylamino, N-(alkyle en C₁ à C₃) -alkyloxy en C₁ à C₃-carbonylamino, N-(aminocarbonyl)-alkylamino en C₁ à C₃, N-(alkyle en C₁ à C₃)-aminocarbonyl)-alkylamino en C₁ à C₃, N-[di-(alkyle en C₁ à C₃)aminocarbonyl]-alkylamino en C₁ à C₃, N-(alkyle en C₁ à C₃) -alkyle en C₁ à C₃-sulfonylamino, N-(alkyle en C₁ à C₃)-arylsulfonylamino ou N-(alkyle en C₁ à C₃)-aryl-alkyle en C₁ à C₃)-sulfonylamino,
un groupe 2-oxo-imidazolidin-1-yle, 2,4-dioxo-imidazolidin-1-yle, 2,5-dioxo-imidazolidin-1-yle ou 2-oxo-hexahydropyrimidin-1-yle dans lequel l'atome d'azote en position 3 peut être substitué respectivement par un groupe méthyle ou éthyle,
un groupe cyano, carboxy, alkyloxy en C₁ à C₃-carbonyle, aminocarbonyle, alkyle en C₁ à C₃-aminocarbonyle, di- (alkyle en C₁ à C₃) - aminocarbonyle, pyrrolidin-1-yl-carbonyle, pipéridin-1-yl-carbonyle, morpholin-4-yl-carbonyle, pipérazin-1-yl-carbonyle ou 4-(alkyle en C₁ à C₃) - pipérazin-1-yl-carbonyle,
un groupe alkyle en C₁ à C₃-carbonyle ou un groupe arylcarbonyle,
un groupe carboxy-alkyle en C₁ à C₃, alkyloxy en C₁ à C₃-carbonyl-alkyle en C₁ à C₃, cyano-alkyle en C₁ à C₃, aminocarbonyl-alkyle en C₁ à C₃, alkyle en C₁ à C₃-aminocarbonyl-alkyle en C₁ à C₃, di-(alkyle en C₁ à C₃)-aminocarbonyl-alkyle en C₁ à C₃, pyrrolidin-1-yl-carbonyl-alkyle en C₁ à C₃, pipéridin-1-yl-carbonyl-alkyle en C₁ à C₃, morpholin-4-yl-carbonyl-alkyle en C₁ à C₃, pipérazin-1-yl-carbonyl-alkyle en C₁ à C₃ ou 4-(alkyle en C₁ à C₃)-pipérazin-1-yl-carbonyl-alkyle en C₁ à C₃,
un groupe carboxy-alkyloxy en C₁ à C₃, alkyloxy en C₁ à C₃-carbonyl-alkyloxy en C₁ à C₃, cyano-alkyloxy en C₁ à C₃, aminocarbonyl-alkyloxy en C₁ à C₃, alkyle en C₁ à C₃-aminocarbonyl-alkyloxy en C₁ à C₃, di- (alkyle en C₁ à C₃)-aminocarbonyl-alkyloxy en C₁ à C₃, pyrrolidin-1-yl-carbonyl-alkyle en C₁ à C₃-oxy, pipéridin-1-yl-carbonyl-alkyloxy en C₁ à C₃, morpholin-4-yl-carbonyl-alkyle en C₁ à C₃-oxy, pipérazin-1-yl-carbonyl-alkyloxy en C₁ à C₃ ou 4-(alkyle en C₁ à C₃)-pipérazin-1-yl-carbonyl-alkyloxy en C₁ à C₃,
un groupe hydroxy-alkyle en C₁ à C₃, alkyloxy en C₁ à C₃-alkyle en C₁ à C₃, amino-alkyle en C₁ à C₃, alkyle en C₁ à C₃-amino-alkyle en C₁ à C₃, di-(alkyle en C₁ à C₃)-amino-alkyle en C₁ à C₃, pyrrolidin-1-yl-alkyle en C₁ à C₃, pipéridin-1-yl-alkyle en C₁ à C₃, morpholin-4-yl-alkyle en C₁ à C₃, pipérazin-1-yl-alkyle en C₁ à C₃, 4-(alkyle en C₁ à C₃)-pipérazin-1-yl-alkyle en C₁ à C₃,
un groupe hydroxy-alkyloxy en C₁ à C₃, alkyloxy en C₁ à C₃-alkyloxy en C₁ à C₃, alkylsulfanyle en C₁ à C₃-alkyloxy en C₁ à C₃, alkylsulfinyle en C₁ à C₃-alkyloxy en C₁ à C₃, alkylsulfonyle en C₁ à C₃-alkyloxy en C₁ à C₃, amino-alkyloxy en C₁ à C₃, alkylamino en C₁ à C₃-alkyloxy en C₁ à C₃, di-(alkyle en C₁ à C₃) -amino-alkyloxy en C₁ à C₃, pyrrolidin-1-yl-alkyloxy en C₁ à C₃, pipéridin-1-yl-alkyloxy en C₁ à C₃, morpholin-4-yl-alkyloxy en C₁ à C₃, pipérazin-1-yl-alkyloxy en C₁ à C₃, 4-(alkyle en C₁ à C₃)-pipérazin-1-yl-alkyloxy en C₁ à C₃,
un groupe mercapto, alkylsulfanyle en C₁ à C₃, alkylsulfinyle en C₁ à C₃, alkylsulfonyle en C₁ à C₃, alkylsulfonyloxy en C₁ à C₃, arylsulfonyloxy, trifluorométhylsulfanyle, trifluorométhylsulfinyle ou trifluorométhylsulfonyle,
un groupe sulfo, aminosulfonyle, alkyle en C₁ à C₃-aminosulfonyle, di-(alkyle en C₁ à C₃)-amino-sulfonyle, pyrrolidin-1-yl-sulfonyle, pipéridin-1-yl-sulfonyle, morpholin-4-yl-sulfonyle, pipérazin-1-yl-sulfonyle ou 4-(alkyle en C₁ à C₃)-pipérazin-1-yl-sulfonyle,
un groupe méthyle ou méthoxy substitué par 1 à 3 atomes de carbone,
un groupe éthyle ou éthoxy substitué par 1 à 5 atomes de fluor
un groupe alcényle en C₂ à C₄ ou alcinyle en C₂ à C₄,
un groupe alcényloxy en C₃ à C₄ ou alcinyloxy en C₃ à C₄,
un groupe cycloalkyle en C₃ à C₆ ou cycloalkyloxy en C₃ à C₆,
un groupe cycloalkyle en C₃ à C₆-alkyle en C₁ à C₃ ou cycloalkyle en C₃ à C₆-alkyloxy en C₁ à C₃ ou
un groupe aryle, aryloxy, aryl-alkyle en C₁ à C₃ ou aryl-alkyloxy en C₁ à C₃,
R¹¹ et R¹² qui peuvent être identiques ou différents, sont respectivement un atome d'hydrogène, de fluor, de chlore, de brome ou d'iode, un groupe alkyle en C₁ à C₃, trifluorométhyle, hydroxy ou alkyloxy en C₁ à C₃ ou cyano, ou
R¹¹ conjointement à R¹², dans la mesure où ceux-ci sont liés aux atomes de carbone voisins, désignent également un groupe méthylènedioxy, difluorométhylènedioxy ou alkylène en C₃ à C₅ à chaîne linéaire,
et
R¹³ et R¹⁴, qui peuvent être identiques ou différents, désignent respectivement un atome d'hydrogène, de fluor, de chlore ou de brome, un groupe trifluorométhyle, alkyle en C₁ à C₃ ou alkyloxy en C₁ à C₃,
un groupe phényl-alkyle en C₁ à C₄ dans lequel la partie alkyle est substituée par un groupe cyano, carboxy, alkyloxy en C₁ à C₃-carbonyle, aminocarbonyle, alkyle en C₁ à C₃-aminocarbonyle, di-(alkyle en C₁ à C₃)-aminocarbonyle, pyrrolidin-1-yl-carbonyle, pipéridin-1-yl-carbonyle, morpholin-4-yl-carbonyle et la partie phényle est substituée par les groupes R¹⁰ à R¹⁴, R¹⁰ à R¹⁴ étant tels que définis précédemment,
un groupe phényl-alcényle en C₂ à C₃ dans lequel la partie phényle est substituée par les groupes R¹⁰ à R¹⁴, R¹⁰ à R¹⁴ étant tels que définis précédemment,
un groupe phényl- (CH₂)ₘ-A-(CH₂)ₙ dans lequel la partie phényle est substituée par R¹⁰ à R¹⁴, R¹⁰ à R¹⁴ étant tels que définis précédemment et
A représente un groupe carbonyle, cyano-iminométhylène, hydroxy-iminométhylène ou alkyloxy-iminométhylène en C₁ à C₃, m représente le nombre 0, 1 ou 2 et n, le nombre 1, 2 ou 3,
un groupe phénylcarbonylméthyle dans lequel la partie phényle est substituée par R¹⁰ à R¹⁴, R¹⁰ à R¹⁴ étant tels que définis précédemment et la partie méthyle étant substituée par un groupe alkyle en C₁ à C₃,
un groupe phényl-(CH₂)ₘ-B-(CH₂)ₙ dans lequel la partie phényle est substituée par R¹⁰ à R¹⁴, R¹⁰ à R¹⁴, m et n étant tels que définis précédemment et
B désigne un groupe méthylène qui est substitué par un groupe hydroxy, alkyloxy en C₁ à C₃, amino, alkylamino en C₁ à C₃, di-(alkyle en C₁ à C₃)-amino, mercapto, alkylsulfanyle en C₁ à C₃, alkylsulfinyle en C₁ à C₃ ou alkylsulfonyle en C₁ à C₃ et éventuellement, substitué en outre par un groupe méthyle ou éthyle,
un groupe naphtyl-alkyle en C₁ à C₃ dans lequel la partie naphtyle est substituée par les groupes R¹⁰ à R¹⁴, R¹⁰ à R¹⁴ étant tels que définis précédemment,
un groupe naphtyl-(CH₂)ₘ-A-(CH₂)ₙ dans lequel la partie naphtyle est substituée par les groupes R¹⁰ à R¹⁴, R¹⁰ à R¹⁴, A, m et n étant tels que définis précédemment,
un groupe naphtyl-(CH₂)ₘ-B-(CH₂)ₙ dans lequel la partie naphtyle est substituée par les groupes R¹⁰ à R¹⁴, R¹⁰ à R¹⁴, B, m et n étant tels que définis précédemment,
un groupe hétéroaryl-(CH₂)ₘ-A-(CH₂)ₙ, dans lequel A, m et n sont tels que définis précédemment,
un groupe hétéroaryl -(CH₂)ₘ-B-(CH₂)ₙ, dans lequel B, m et n sont tels que définis précédemment,
un groupe alkyle en C₁ à C₆-A-(CH₂)ₙ, dans lequel A et n sont tels que définis précédemment,
un groupe cycloalkyle en C₃ à C₇-(CH₂)ₘ-A-(CH₂)ₙ, dans lequel A, m et n sont tels que définis précédemment,
un groupe cycloalkyle en C₃ à C₇-(CH₂)ₘ-B-(CH₂)ₙ, dans lequel B, m et n sont tels que définis précédemment,
un groupe R²¹-A-(CH₂)ₙ dans lequel R²¹ désigne un groupe alkyloxycarbonyle en C₁ à C₃, aminocarbonyle, alkylaminocarbonyle en C₁ à C₃, di-(alkyle en C₁ à C₃)aminocarbonyle, pyrrolidin-1-yl-carbonyle, pipéridin-1-yl-carbonyle ou morpholin-4-yl-carbonyle, pipérazin-1-yl-carbonyle, 4-méthylpipérazin-1-yl-carbonyle ou 4-éthylpipérazin-1-yl-carbonyle et A et n sont tels que définis précédemment,
un groupe phényl-(CH₂)ₘ-D-alkyle en C₁ à C₃ dans lequel la partie phényle est substituée par les groupes R¹⁰ à R¹⁴, dans lequel R¹⁰ à R¹⁴ et m sont tels que définis précédemment et D désigne un atome d'oxygène ou de soufre, un groupe imino, alkylimino en C₁ à C₃, sulfinyle ou sulfonyle,
un groupe naphtyl-(CH₂)ₘ-D-alkyle en C₁ à C₃ dans lequel la partie naphtyle est substituée par les groupes R¹⁰ à R¹⁴, dans lequel R¹⁰ à R¹⁴, D et m sont tels que définis précédemment,
ou
un groupe alkyle en C₂ à C₆ substitué par un groupe R_{b}, dans lequel
R_{b} est isolé de l'atome d'azote cyclique par au moins deux atomes de carbone en position 1 du squelette de la xanthine et R_{b} est un groupe hydroxy, alkyloxy en C₁ à C₃, mercapto, alkylsulfanyle en C₁ à C₃, alkylsulfinyle en C₁ à C₃, alkylsulfonyle en C₁ à C₃, amino, alkylamino en C₁ à C₃, di-(alkyle en C₁ à C₃)amino, pyrrolidin-1-yle, pipéridin-1-yle, morpholin-4-yle, pipérazin-1-yle ou 4-(alkyle en C₁ à C₃)-pipérazin-1-yle,
R² désigne un atome d'hydrogène,
un groupe alkyle en C₁ à C₈,
un groupe alcényle en C₂ à C₆,
un groupe alcinyle en C₃ à C₆,
un groupe alkyle en C₁ à C₆ substitué par un groupe Rₐ, Rₐ étant tel que défini précédemment,
un groupe tétrahydrofuran-3-yle, tétrahydropyran-3-yle, tétrahydropyran-4-yle, tétra-hydrofuranyl-alkyle en C₁ à C₃ ou tétrahydropyranyl-alkyle en C₁ à C₃,
un groupe alkyle en C₁ à C₆ substitué par un groupe phényle, le cycle phényle étant substitué par les groupes R¹⁰ à R¹⁴ et R¹⁰ à R¹⁴ étant tels que définis précédemment,
un groupe phényle substitué par les groupes R¹⁰ à R¹⁴, R¹⁰ à R¹⁴ étant tels que définis précédemment,
un groupe phényl-alcényle en C₂ à C₃ dans lequel la partie phényle est substituée par les groupes R¹⁰ à R¹⁴, R¹⁰ à R¹⁴ étant tels que définis précédemment,
un groupe phényl-(CH₂)ₘ-A-(CH₂)ₙ, dans lequel la partie phényle est substituée par les groupes R¹⁰ à R¹⁴, dans lequel R¹⁰ à R¹⁴, A, m et n sont tels que définis précédemment,
un groupe phényl-(CH₂)ₘ-B-(CH₂)ₙ, dans lequel la partie phényle est substituée par les groupes R¹⁰ à R¹⁴, dans lequel R¹⁰ à R¹⁴, B, m et n sont tels que définis précédemment,
un groupe hétéroaryl-(CH₂)ₘ-A-(CH₂)ₙ, dans lequel A, m et n sont tels que définis précédemment,
un groupe hétéroaryl-(CH₂)ₘ-B-(CH₂)ₙ, dans lequel B, m et n sont tels que définis précédemment,
un groupe alkyle en C₁ à C₆-A-(CH₂)ₙ, dans lequel A et n sont tels que définis précédemment,
un groupe cycloalkyle en C₃ à C₇-(CH₂)ₘ-A-(CH₂)ₙ, dans lequel A, m et n sont tels que définis précédemment,
un groupe cycloalkyle en C₃ à C₇-(CH₂)ₘ-B-(CH₂)ₙ, dans lequel B, m et n sont tels que définis précédemment,
un groupe R²¹-A-(CH₂)ₙ, dans lequel R²¹, A et n sont tels que définis précédemment,
un groupe phényl-(CH₂)ₘ-D-alkyle en C₁ à C₃, dans lequel la partie phényle est substituée par les groupes R¹⁰ à R¹⁴, R¹⁰ à R¹⁴, m et D étant tels que définis précédemment,
un groupe alkyke en C₂ à C₆ substitué par un groupe R_{b}, dans lequel
R_{b} est isolé de l'atome d'azote cyclique par au moins deux atomes de carbone en position 3 du squelette de la xanthine et est tel que défini précédemment,
ou un groupe cycloalkyle en C₃ à C₆,
R³ désigne un groupe alkyle en C₁ à C₄ substitué par le groupe R_{c}, dans lequel
R_{c} désigne un groupe cycloalkyle en C₃ à C₇ substitué éventuellement par un ou deux groupes alkyle en C₁ à C₃, ou
un groupe cycloalcényle en C₅ à C₇ substitué éventuellement par un ou deux groupes alkyle en C₁ à C₃,
un groupe alcényle en C₃ à C₈,
un groupe alcényle en C₃ à C₆ substitué par un atome de fluor, de chlore ou de brome ou un groupe trifluorométhyle,
un groupe alcinyle en C₃ à C₈ ou
un groupe aryl-alcényle en C₂ à C₄,
et
R⁴ désigne un groupe azétidin-1-yle ou pyrrolidin-1-yle qui est substitué en position 3 par un groupe RₑNR_{d} et en outre par un ou deux groupes alkyle en C₁ à C₃, dans lequel
Rₑ désigne un atome d'hydrogène ou un groupe alkyle en C₁ à C₃ et
R_{d} désigne un atome d'hydrogène, un groupe alkyle en C₁ à C₃, un groupe R_{f}-alkyle en C₁ à C₃ ou un groupe R_{g}-alkyle en C₂ à C₃, dans lequel
R_{f} désigne un groupe carboxy, alkyloxy en C₁ à C₃-carbonyle, aminocarbonyle, alkylamino en C₁ à C₃-carbonyle, di-(alkyle en C₁ à C₃)-aminocarbonyle, pyrrolidin-1-yl-carbonyle, 2-cyano-pyrrolidin-1-ylcarbonyle, 2-carboxypyrrolidin-1-yl-carbonyle, 2-méthoxycarbonylpyrrrolidin-1-yl-carbonyle, 2-éthoxycarbonylpyrrolidin-1-yl-carbonyle, 2-aminocarbonylpyrrolidin-1-yl-carbonyle, 4-cyano-thiazolidin-3-yl-carbonyle, 4-carboxythiazolidin-3-yl-carbonyle, 4-méthoxycarbonylthiazolidin-3-yl-carbonyle, 4-éthoxycarbonylthiazolidin-3-yl-carbonyle, 4-aminocarbonylthiazolidin-3-yl-carbonyle, pipéridin-1-ylcarbonyle, morpholin-4-yl-carbonyle, pipérazin-1-yl-carbonyle, 4-méthyl-pipérazin-1-yl-carbonyle ou 4-éthyl-pipérazin-1-yl-carbonyle et
R_{g} qui est séparé par au moins deux atomes de carbone de l'atome d'azote du groupe RₑNR_{d}, désigne un groupe hydroxy, méthoxy ou éthoxy,
un groupe pipéridin-1-yle ou hexahydroazépin-1-yle qui est substitué en position 3 ou en position 4 par un groupe RₑNR_{d} et peut en outre être substitué par un ou deux groupes alkyle en C₁ à C₃, où Rₑ et R_{d} sont tels que définis précédemment,
un groupe 3-amino-pipéridin-1-yle dans lequel la partie pipéridin-1-yle est substituée en outre par un groupe aminocarbonyle, alkyle en C₁ à C₂-aminocarbonyle, di-(alkyle en C₁ à C₂)aminocarbonyle, pyrrolidin-1-yl-carbonyle, (2-cyano-pyrrolidin-1-yl)carbonyle, thiazolidin-3-yl-carbonyle, (4-cyano-thiazolidin-3-yl)carbonyle, pipéridin-1-ylcarbonyle ou morpholin-4-ylcarbonyle,
un groupe 3-amino-pipéridin-1-yle dans lequel la partie pipéridin-1-yle est substituée en outre en position 4 ou en position 5 par un groupe hydroxy ou méthoxy,
un groupe 3-amino-pipéridin-1-yle dans lequel la partie méthylène est substituée en outre en position 2 ou en position 6 par un groupe carbonyle,
un groupe pipéridin-1-yle ou hexahydro-azépin-1-yle substitué en position 3 par un groupe amino, alkylamino en C₁ à C₃ ou di-(alkyle en C₁ à C₃)-amino dans lesquels respectivement deux atomes d'hydrogène sont remplacés sur le squelette du carbone du groupe pipéridin-1-yle ou hexahydro-azépin-1-yle par un pont alkylène à chaîne linéaire, ce pont contenant 2 à 5 atomes de carbone lorsque les deux atomes d'hydrogène se trouvent sur le même atome de carbone, ou 1 à 4 atomes de carbone lorsque les atomes d'hydrogène se trouvent sur des atomes de carbone voisins, ou contenant 1 à 4 atomes de carbone lorsque les atomes d'hydrogène se trouvent sur des atomes de carbone qui sont séparés par un atome ou contenant 1 à 3 atomes de carbone lorsque les deux atomes d'hydrogène se trouvent sur des atomes de carbone qui sont séparés par deux atomes,
un groupe azétidin-1-yle, pyrrolidin-1-yle, pipéridin-1-yle ou hexahydroazépin-1-yle qui est substitué par un groupe amino-alkyle en C₁ à C₃, alkylamino en C₁ à C₃-alkyle en C₁ à C₃ ou di-(alkyle en C₁ à C₃)amino-alkyle en C₁ à C₃,
un groupe cycloalkyle en C₃ à C₇ qui est substitué par un groupe amino, alkylamino en C₁ à C₃ ou di-(alkyle en C₁ à C₃) -amino,
un groupe cycloalkyle en C₃ à C₇ qui est substitué par un groupe amino-alkyle en C₁ à C₃, alkylamino en C₁ à C₃-alkyle en C₁ à C₃ ou di- (alkyle en C₁ à C₃) amino-alkyle en C₁ à C₃,
un groupe cycloalkyle en C₃ à C₇-alkyle en C₁ à C₂, dans lequel la partie cycloalkyle est substituée par un groupe amino, alkylamino en C₁ à C₃ ou di-(alkyle en C₁ à C₃)-amino,
un groupe cycloalkyle en C₃ à C₇-alkyle en C₁ à C₂ dans lequel la partie cycloalkyle est substituée par un groupe amino-alkyle en C₁ à C₃, alkylamino en C₁ à C₃-alkyle en C₁ à C₃ ou di-(alkyle en C₁ à C₃)amino-alkyle en C₁ à C₃,
un groupe R¹⁹-alkyle en C₃ à C₄ dans lequel la partie alkyle en C₃ à C₄ est à chaîne linéaire et qui peut être substitué par un radical R15 et en outre, peut être substitué par un ou deux groupes alkyle en C₁ à C₃, R¹⁵ représentant un groupe alkyle en C₁ à C₆, cycloalkyle en C₃ à C₆, cycloalkyle en C₃ à C₆-alkyle en C₁ à C₃, aryle ou aryl-alkyle en C₁ à C₃ et R¹⁹ représente un groupe amino, alkylamino en C₁ à C₃ ou di-(alkyle en C₁ à C₃)-amino,
un groupe 3-amino-2-oxo-pipéridin-5-yle ou 3-amino-2-oxo-1-méthyl-pipéridin-5-yle,
un groupe pyrrolidin-3-yle, pipéridin-3-yle, pipéridin-4-yle, hexahydroazépin-3-yle ou hexahydroazépin-4-yle qui est substitué en position 1 par un groupe amino, alkylamino en C₁ à C₃ ou di-(alkyle en C₁ à C₃)amino,
ou un groupe azétidin-2-yl-alkyle en C₁ à C₂, azétidin-3-yl-alkyle en C₁ à C₂, pyrrolidin-2-yl-alkyle en C₁ à C₂, pyrrolidin-3-yle, pyrrolidin-3-yl-alkyle en C₁ à C₂, pipéridin-2-yl-alkyle en C₁ à C₂, pipéridin-3-yle, pipéridin-3-yl-alkyle en C₁ à C₂, pipéridin-4-yle ou pipéridin-4-yl-alkyle en C₁ à C₂, les groupes précédemment cités pouvant être substitués respectivement par un ou deux groupes alkyle en C₁ à C₃,
où les groupes aryle mentionnés dans la définition des radicaux cités précédemment désignent des groupes phényle ou naphtyle qui peuvent être mono- ou disubstitués indépendamment les uns des autres par R_{b}, les substituants pouvant être identiques ou différents et Rₕ représente un atome de fluor, de chlore, de brome ou d'iode, un groupe trifluorométhyle, cyano, nitro, amino, aminocarbonyle, aminosulfonyle, méthyle, sulfonyle, acétylamino, méthylsulfonylamino, alkyle en C₁ à C₃, cyclopropyle, éthényle, éthinyle, hydroxy, alkyloxy en C₁ à C₃, difluorométhoxy ou trifluorométhoxy,
les groupes hétéroaryle mentionnés dans la définition des radicaux cités précédemment désignent un groupe pyrrolyle, furanyle, thiényle, pyridyle, indolyle, benzofuranyle, benzothiophényle, quinolinyle ou isoquinolinyle,
ou un groupe pyrrolyle, furanyle, thiényle ou pyridyle, dans lequel un ou deux groupes méthine sont remplacés par des atomes d'azote,
ou un groupe indolyle, benzofuranyle, benzothiophényle, quinolinyle ou isoquinolinyle, dans lequel un à trois groupes méthine sont remplacés par des atomes d'azote
ou un groupe 1,2-dihydro-2-oxo-pyridinyle, 1,4-dihydro-4-oxo-pyridinyle, 2,3-dihydro-3-oxo-pyridazinyle, 1,2,3,6-tétrahydro-3,6-dioxo-pyridazinyle, 1,2-dihydro-2-oxo-pyrimidinyle, 3,4-dihydro-4-oxo-pyrimidinyle, 1,2,3,4-tétrahydro-2,4-dioxo-pyrimidinyle, 1,2-dihydro-2-oxo-pyrazinyle, 1,2,3,4-tétrahydro-2,3-dioxo-pyrazinyle, 2,3-dihydro-2-oxo-indolyle, 2,3-dihydrobenzofuranyle, 2,3-dihydro-2-oxo-1H-benzimidazolyle, 2,3-dihydro-2-oxo-benzoxazolyle, 1,2-dihydro-2-oxo-quinolinyle, 1,4-dihydro-4-oxo-quinolinyle, 1,2-dihydro-1-oxo-isoquinolinyle, 1,4-dihydro-4-oxo-cinnolinyle, 1,2-dihydro-2-oxo-quinazolinyle, 1,4-dihydro-4-oxo-quinazolinyle, 1,2,3,4-tétrahydro-2,4-dioxo-quinazolinyle, 1,2-dihydro-2-oxoquinoxalinyle, 1,2,3,4-tétrahydro-2,3-dioxo-quinoxalinyle, 1,2-dihydro-1-oxo-phtalazinyle, 1,2,3,4-tétrahydro-1,4-dioxo-phtalazinyle, chromanyle, cumarinyle, 2,3-dihydro-benzo[1,4]dioxinyle ou 3,4-dihydro-3-oxo-2H-benzo[1,4]oxazinyle,
les groupes hétéroaryle mentionnés précédemment pouvant être substitués par R¹⁰ à R¹⁴, R¹⁰ à R¹⁴ étant tels que définis précédemment,
où, sauf indication contraire, les groupes alkyle, alcényle et alcinyle mentionnés précédemment pouvant être à chaîne linéaire ou ramifiée,
ou les dérivés N-oxydés ou méthylés ou éthylés sur l'atome d'azote cyclique en position 9 du squelette de la xanthine,
ou les dérivés dans lesquels les groupes 2-oxo, 6-oxo ou 2-oxo et 6-oxo du squelette de la xanthine sont remplacés par des groupes thioxo,
leurs tautomères, énantiomères, diastéréomères, leurs mélanges et leurs sels ;
pour leur utilisation dans la thérapie combinée avec d'autres substances actives.

2. Composés pour leur utilisation selon la revendication 1, où l'on utilise les composés de formule générale I selon la revendication 1, dans laquelle
R¹ est un groupe alkyle en C₁ à C₆,
un groupe alcényle en C₃ à C₆,
un groupe alcényle en C₃ à C₄ qui est substitué par un groupe alkyloxy en C₁ à C₂-carbonyle,
un groupe alcinyle en C₃ à C₆,
un groupe cycloalkyle en C₃ à C₆-alkyle en C₁ à C₃,
un groupe phényl-alkyle en C₁ à C₄, dans lequel la partie phényle est substituée par R¹⁰ à R¹², où
R¹⁰ est un atome d'hydrogène, un atome de fluor, de chlore ou de brome,
un groupe alkyle en C₁ à C₄, trifluorométhyle, hydroxyméthyle, cycloalkyle en C₃ à C₆, éthinyle ou phényle,
un groupe hydroxy, alkyloxy en C₁ à C₄, difluorométhoxy, trifluorométhoxy, 2,2,2-trifluoroéthoxy, phénoxy, benzyloxy, 2-propén-1-yloxy, 2-propin-1-yloxy, cyano-alkyloxy en C₁ à C₂, alkylsulfonyloxy en C₁ à C₂, phénylsulfonyloxy, carboxy-alkyloxy en C₁ à C₃, alkyloxy en C₁ à C₃-carbonyl-alkyloxy en C₁ à C₃, aminocarbonyl-alkyloxy en C₁ à C₃, alkyle en C₁ à C₂-aminocarbonyl-alkyloxy en C₁ à C₃, di-(alkyle en C₁ à C₂)aminocarbonyl-alkyloxy en C₁ à C₃, pyrrolidin-1-yl-carbonyl-alkyloxy en C₁ à C₃, pipéridin-1-ylcarbonyl-alkyloxy en C₁ à C₃, morpholin-4-ylcarbonyl-alkyloxy en C₁ à C₃, méthylsulfanylméthoxy, méthylsulfinylméthoxy, méthylsulfonylméthoxy, cycloalkyloxy en C₃ à C₆ ou cycloalkyle en C₃ à C₆-alkyloxy en C₁ à C₂,
un groupe carboxy, alkyloxycarbonyle en C₁ à C₃, carboxy-alkyle en C₁ à C₃, alkyloxycarbonyle en C₁ à C₃-alkyle en C₁ à C₃, aminocarbonyle, alkylaminocarbonyle en C₁ à C₂, di-(alkyle en C₁ à C₂)-aminocarbonyle, morpholin-4-ylcarbonyle ou cyano,
un groupe nitro, amino, alkylamino en C₁ à C₂, di-(alkyle en C₁ à C₂) amino, cyano-alkylamino en C₁ à C₂, [N- (cyano-alkyle en C₁ à C₂)-N-alkyle en C₁ à C₂-amino], alkyloxy en C₁ à C₂-carbonyl-alkylamino en C₁ à C₂, alkyle en C₁ à C₂-carbonylamino, alkyloxy en C₁ à C₂-carbonylamino, alkylsulfonylamino en C₁ à C₃, bis- (alkylsulfonyle en C₁ à C₂)amino, aminosulfonylamino, alkylamino en C₁ à C₂-sulfonylamino, di-(alkyle en C₁ à C₂)amino-sulfonylamino, morpholino-4-yl-sulfonylamino, (alkylamino en C₁ à C₂)thiocarbonylamino, (alkyloxy en C₁ à C₂-carbonylamino)carbonylamino, aminocarbonylamino, alkyle en C₁ à C₂-aminocarbonylamino, di-(alkyle en C₁ à C₂)aminocarbonylamino ou morpholin-4-ylcarbonylamino,
un groupe 2-oxo-imidazolidin-1-yle, 3-méthyl-2-oxo-imidazolidin-1-yle, 2,4-dioxo-imidazolidin-1-yle, 3-méthyl-2,4-dioxo-imidazolidin-1-yle, 2,5-dioxo-imidazolidin-1-yle, 3-méthyl-2,5-dioxo-imidazolidin-1-yle, 2-oxo-hexahydro-pyrimidin-1-yle ou 3-méthyl-2-oxo-hexahydropyrimidin-1-yle,
ou
un groupe alkylsulfanyle en C₁ à C₂, alkylsulfinyle en C₁ à C₂, alkylsulfonyle en C₁ à C₂, aminosulfonyle, alkylaminosulfonyle en C₁ à C₂ ou di-(alkyle en C₁ à C₂) aminosulfonyle,
et R¹¹ et R¹² qui peuvent être identiques ou différents, sont un atome d'hydrogène, de fluor, de chlore ou de brome ou
un groupe méthyle, cyano, trifluorométhyle ou méthoxy,
ou R¹¹ conjointement à R¹², dans la mesure où ceux-ci sont liés aux atomes de carbone voisins, désignent également un groupe méthylènedioxy, difluorométhylènedioxy, 1,3-propylène ou 1,4-butylène,
un groupe phényl-alkyle en C₁ à C₃ dans lequel la partie alkyle est substituée par un groupe carboxy, alkyloxy en C₁ à C₂-carbonyle, aminocarbonyle, alkylaminocarbonyle en C₁ à C₂ ou di-(alkyle en C₁ à C₂) -aminocarbonyle,
un groupe phényl-alcényle en C₂ à C₃ dans lequel la partie phényle peut être substituée par un atome de fluor, de chlore ou de brome ou par un groupe méthyle, trifluorométhyle ou méthoxy,
un groupe phényl-(CH₂)ₘ-A-(CH₂)ₙ dans lequel la partie phényle est substituée par R¹⁰ à R¹², R¹⁰ à R¹² étant tels que définis précédemment et
A représente un groupe carbonyle, hydroxy-iminométhylène ou alkyloxy-iminométhylène en C₁ à C₂, m représente le nombre 0 ou 1 et n, le nombre 1 ou 2,
un groupe phénylcarbonylméthyle dans lequel la partie phényle est substituée par R¹⁰ à R¹², R¹⁰ à R¹² étant tels que définis précédemment et la partie méthyle étant substituée par un groupe méthyle ou éthyle,
un groupe phénylcarbonylméthyle dans lequel deux atomes de carbone voisins de la partie phényle sont remplacés par des ponts -O-CO-NH, -NH-CO-NH, -N=CH-NH-, -N=CH-O-ou -O-CH₂-CO-NH-, les ponts mentionnés précédemment pouvant être remplacés par un ou deux groupes méthyle,
un groupe phényl-(CH₂)ₘ-B**-**(CH₂)ₙ dans lequel la partie phényle est substituée par R¹⁰ à R¹², R¹⁰ à R¹², m et n étant tels que définis précédemment et
B désigne un groupe méthylène qui est substitué par un groupe hydroxy, alkyloxy en C₁ à C₂, et éventuellement, substitué en outre par un groupe méthyle,
un groupe naphtyl-méthyle ou naphtyléthyle dans lequel la partie naphtyle est substituée respectivement par les groupes R¹⁰ à R¹², R¹⁰ à R¹² étant tels que définis précédemment,
un groupe hétéroaryl-alkyle en C₁ à C₃, le terme hétéroaryle désignant un groupe pyrrolyle, imidazolyle, triazolyle, furanyle, thiényle, oxazolyle, isoxazolyle, thiazolyle, isothiazolyle, pyridyle, pyridazinyle, pyrimidinyle, pyrazinyle, indolyle, benzimidazolyle, 2,3-dihydro-2-oxo-1*H*-benzimidazolyle, indazolyle, benzofuranyle, 2,3-dihydrobenzofuranyle, benzoxazolyle, 2,3-dihydro-2-oxo-benzoxazolyle, benzo-isoxazolyle, benzothiophényle, benzothiazolyle, benzo-isothiazolyle, quinolinyle, 1,2-dihydro-2-oxo-quinolinyle, isoquinolinyle, 1,2-dihydro-1-oxo-isoquinolinyle, cinnolinyle, quinazolinyle, 1,2-dihydro-2-oxo-quinazolinyle, 1,2-dihydro-1-oxo-phtalazin-4-yle, cumarinyle ou 3,4-dihydro-3-oxo-2H-benzo[1,4]oxazinyle,
les groupes hétéroaryle mentionnés précédemment pouvant être substitués sur les atomes de carbone, par un atome de fluor, de chlore ou de brome, par un groupe méthyle, trifluorométhyle, cyano, aminocarbonyle, aminosulfonyle, méthylsulfonyle, nitro, amino, acétylamino, méthylsulfonylamino, méthoxy, difluorométhoxy ou trifluorométhoxy et les groupes imino des groupes hétéroaryle précédemment cités pouvant être substitués par des groupes méthyle ou éthyle,
un groupe furanyl-A-CH₂-, thiényl-A-CH₂-, thiazolyl-A-CH₂ ou pyridyl-A-CH₂, où A est tel que défini précédemment,
un groupe furanyl-B-CH₂-, thiényl-B-CH₂-, thiazolyl-B-CH₂ ou pyridyl-B-CH₂, où B est tel que défini précédemment,
un groupe alkyle en C₁ à C₄-A-(CH₂)ₙ, dans lequel A et n sont tels que définis précédemment,
un groupe cycloalkyle en C₃ à C₆-(CH₂)ₘ-A-(CH₂)ₙ, dans lequel A, m et n sont tels que définis précédemment,
un groupe cycloalkyle en C₃ à C₆-(CH₂)ₘ-B-(CH₂)ₙ, dans lequel B, m et n sont tels que définis précédemment,
un groupe R²¹-A-(CH₂)ₙ dans lequel R²¹ désigne un groupe alkyloxycarbonyle en C₁ à C₂, aminocarbonyle, alkylaminocarbonyle en C₁ à C₂, di-(alkyle en C₁ à C₂)aminocarbonyle, pyrrolidin-1-yl-carbonyle, pipéridin-1-yl-carbonyle ou morpholin-4-yl-carbonyle, et A et n sont tels que définis précédemment,
un groupe phényl-D-alkyle en C₁ à C₃ dans lequel la partie phényle est substituée éventuellement par un atome de fluor, de chlore ou de brome, un groupe méthyle, trifluorométhyle ou méthoxy et D représente un atome d'oxygène ou de soufre, un groupe sulfinyle ou sulfonyle,
un groupe alkyle en C₁ à C₄ substitué par un groupe Rₐ, dans lequel
Rₐ représente un groupe cyano, carboxy, alkyloxy en C₁ à C₃-carbonyle, aminocarbonyle, alkyle en C₁ à C₂-aminocarbonyle, di-(alkyle en C₁ à C₂)aminocarbonyle, pyrrolidin-1-ylcarbonyle, pipéridin-1-ylcarbonyle ou morpholin-4-ylcarbonyle, ou
un groupe alkyle en C₂ à C₄ substitué par un groupe R_{b}, dans lequel
R_{b} représente un groupe hydroxy, alkyloxy en C₁ à C₃, amino, alkylamino en C₁ à C₃, di-(alkyle en C₁ à C₃)-amino, pyrrolidin-1-yle, pipéridin-1-yle, morpholin-4-yle, pipérazin-1-yle, 4-méthyl-pipérazin-1-yle ou 4-éthyl-pipérazin-1-yle et est isolé par au moins deux atomes de carbone de l'atome d'azote cyclique en position 1 du squelette de la xanthine,
R² désigne un atome d'hydrogène,
un groupe alkyle en C₁ à C₆,
un groupe alcényle en C₂ à C₄,
un groupe alcinyle en C₃ à C₄,
un groupe cycloalkyle en C₃ à C₆,
un groupe cycloalkyle en C₃ à C₆-alkyle en C₁ à C₃,
un groupe tétrahydrofuran-3-yle, tétrahydropyran-3-yle, tétrahydropyran-4-yle, tétra-hydrofuranylméthyle ou tétrahydropyranylméthyle,
un groupe phényle qui est éventuellement substitué par un atome de fluor, de chlore ou de brome ou par un groupe méthyle, trifluorométhyle, hydroxy, méthoxy, difluorométhoxy ou trifluorométhoxy,
un groupe phényl-alkyle en C₁ à C₄ dans lequel la partie phényle est éventuellement substituée par un atome de fluor, de chlore ou de brome, un groupe méthyle, trifluorométhyle, diméthylamino, hydroxy, méthoxy, difluorométhoxy ou trifluorométhoxy,
un groupe phényl-alcényle en C₂ à C₃ dans lequel la partie phényle peut être substituée par un atome de fluor, de chlore ou de brome ou par un groupe méthyle, trifluorométhyle ou méthoxy,
un groupe phénylcarbonyl-alkyle en C₁ à C₂ dans lequel la partie phényle est substituée éventuellement par un atome de fluor, de chlore ou de brome, un groupe méthyle, trifluorométhyle, hydroxy, méthoxy, difluorométhoxy ou trifluorométhoxy,
un groupe hétéroaryl-alkyle en C₁ à C₃, le terme hétéroaryle étant tel que défini précédemment,
un groupe furanylcarbonylméthyle, thiénylcarbonyl-méthyle, thiazolylcarbonylméthyle ou pyridyl-carbonylméthyle,
un groupe alkyle en C₁ à C₄-carbonyl-alkyle en C₁ à C₂,
un groupe cycloalkyle en C₃ à C₆-carbonyl-alkyle en C₁ à C₂,
un groupe phényl-D-alkyle en C₁ à C₃ dans lequel la partie phényle est substituée éventuellement par un atome de fluor, de chlore ou de brome, un groupe méthyle, trifluorométhyle, hydroxy, méthoxy, difluorométhoxy ou trifluorométhoxy et D est tel que défini précédemment,
un groupe alkyle en C₁ à C₄ substitué par un groupe Rₐ, dans lequel Rₐ est tel que défini précédemment ou
un groupe alkyle en C₂ à C₄ substitué par un groupe R_{b}, R_{b} étant tel que défini précédemment et par au moins deux atomes de carbone de l'atome d'azote cyclique en position 3 du squelette de la xanthine,
R³ désigne un groupe alcényle en C₃ à C₇ un groupe alcényle en C₃ à C₅ qui est substitué par un atome de fluor, de chlore ou de brome ou un groupe trifluorométhyle,
un groupe alcinyle en C₃ à C₆,
un groupe alkyle en C₁ à C₃ substitué par le groupe R_{c}, dans lequel
R_{c} désigne un groupe cycloalkyle en C₃ à C₆ substitué éventuellement par un ou deux groupes méthyle, ou
un groupe cycloalcényle en C₅ à C₆ substitué éventuellement par un ou deux groupes méthyle,
ou un groupe phényl-alcényle en C₂ à C₃
et
R⁴ est un groupe pyrrolidin-1-yle qui est substitué en position 3 par un groupe amino, méthylamino ou diméthylamino,
un groupe azétidin-1-yle qui est substitué par un groupe aminométhyle,
un groupe pyrrolidin-1-yle qui est substitué par un groupe aminométhyle,
un groupe pipéridin-1-yle qui est substitué en position 3 ou en position 4 par un groupe amino, méthylamino, diméthylamino ou [(2-cyano-pyrrolidin-1-yl)carbonylméthyl]-amino, dans lequel la partie pipéridin-1-yle peut en outre être substituée par un groupe méthyle ou éthyle,
un groupe 3-amino-pipéridin-1-yle dans lequel la partie pipéridin-1-yle est substituée en outre par un groupe aminocarbonyle, alkyle en C₁ à C₂-aminocarbonyle, di-(alkyle en C₁ à C₂)aminocarbonyle, pyrrolidin-1-ylcarbonyle, (2-cyano-pyrrolidin-1-yl)carbonyle, thiazolidin-3-yl-carbonyle, (4-cyano-thiazolidin-3-yl)carbonyle, pipéridin-1-ylcarbonyle ou morpholin-4-ylcarbonyle,
un groupe 3-amino-pipéridin-1-yle dans lequel la partie pipéridin-1-yle est en outre substituée en position 4 ou en position 5 par un groupe hydroxy ou méthoxy,
un groupe 3-amino-pipéridin-1-yle dans lequel le groupe méthylène est remplacé en position 2 ou en position 6 par un groupe carbonyle,
un groupe 3-amino-pipéridin-1-yle dans lequel un atome d'hydrogène est remplacé en position 2, conjointement avec un atome d'hydrogène en position 5 par un pont - CH₂-CH₂,
un groupe 3-amino-pipéridin-1-yle dans lequel un atome d'hydrogène est remplacé en position 2 conjointement avec un atome d'hydrogène en position 6 par un pont - CH₂-CH₂,
un groupe 3-amino-pipéridin-1-yle dans lequel un atome d'hydrogène est remplacé en position 4 conjointement avec un atome d'hydrogène en position 6 par un pont - CH₂-CH₂,
un groupe pipéridin-1-yle qui est substitué par un groupe aminométhyle,
un groupe pipéridin-3-yle ou pipéridin-4-yle,
un groupe pipéridin-3-yle ou pipéridin-4-yle qui est substitué en position 1 par un groupe amino,
un groupe hexahydroazépin-1-yle qui est substitué en position 3 ou en position 4 par un groupe amino,
un groupe 3-amino-propyle, 3-méthylamino-propyle ou 3-diméthylamino-propyle dans lequel la partie propyle peut être substituée par un ou deux groupes méthyle,
un groupe 4-amino-butyle, 4-méthylamino-butyle ou 4-diméthylamino-butyle dans lequel la partie butyle peut être substituée par un ou deux groupes méthyle,
un groupe alkyle en C₁ à C₂ qui est substitué par un groupe 2-pyrrolidinyle, 3-pyrrolidinyle, 2-pipéridinyle, 3-pipéridinyle ou 4-pipéridinyle,
un groupe 3-amino-2-oxo-pipéridin-5-yle ou 3-amino-2- oxo-1-méthyl-pipéridin-5-yle,
un groupe cycloalkyle en C₃ à C₆ qui est substitué par un groupe amino, aminométhyle ou aminoéthyle ou
un groupe cycloalkyle en C₃ à C₆-alkyle en C₁ à C₂ dans lequel la partie cycloalkyle est substituée par un groupe amino, aminométhyle ou aminoéthyle,
où, sauf indication contraire, les groupes alkyle, alcényle et alcinyle mentionnés précédemment peuvent être à chaîne linéaire ou ramifiée,
leurs tautomères, énantiomères, diastéréomères, leurs mélanges et leurs sels.

3. Composés pour leur utilisation selon la revendication 1, où l'on utilise les composés de formule générale I selon la revendication 1, dans laquelle
R¹ est tel que défini dans la revendication 1 ou 2, et
R² est un atome d'hydrogène,
un groupe alkyle en C₁ à C₆,
un groupe éthényle,
un groupe 2-propén-1-yle ou 2-propin-1-yle,
un groupe phényle,
un groupe phényl-alkyle en C₁ à C₄, dans lequel la partie phényle peut être substituée par un atome de fluor, un groupe méthyle ou méthoxy,
un groupe phénylcarbonylméthyle,
un groupe 2-phényléthényle,
un groupe méthyle qui est substitué par un groupe cyclopropyle, cyano, carboxy ou méthoxycarbonyle, ou
un groupe éthyle qui est substitué en position 2 par un groupe cyano, hydroxy, méthoxy ou diméthylamino,
R³ est un groupe alcényle en C₄ à C₆,
un groupe 1-cyclopentén-1-ylméthyle ou 1-cyclohexén-1-ylméthyle,
un groupe 2-propin-1-yle, 2-butin-1-yle ou 2-pentin-1-yle,
un groupe 2-phényléthényle, ou
un groupe cyclopropylméthyle, et
R⁴ est un groupe pyrrolidin-1-yle qui est substitué en position 3 par un groupe amino,
un groupe azétidin-1-yle qui est substitué par un groupe aminométhyle,
un groupe pyrrolidin-1-yle qui est substitué par un groupe aminométhyle,
un groupe pipéridin-1-yle qui est substitué en position 3 ou en position 4 par un groupe amino, méthylamino, diméthylamino ou [(2-cyano-pyrrolidin-1-yl)carbonylméthyl]-amino dans lequel la partie pipéridin-1-yle peut en outre être substituée par un groupe méthyle,
un groupe 3-amino-pipéridin-1-yle dans lequel la partie pipéridin-1-yle est en outre substituée par un groupe pyrrolidin-1-yl-carbonyle,
un groupe 3-amino-pipéridin-1-yle dans lequel la partie pipéridin-1-yle est substituée en outre en position 4 par un groupe hydroxy,
un groupe 3-amino-pipéridin-1-yle dans lequel un atome d'hydrogène en position 2 conjointement à un atome d'hydrogène en position 5 est remplacé par un pont - CH₂-CH₂-,
un groupe pipéridin-1-yle qui est substitué par un groupe aminométhyle,
un groupe pipéridin-3-yle ou pipéridin-4-yle,
un groupe 1-amino-pipéridin-3-yle ou 1-amino-pipéridin-4-yle,
un groupe hexahydroazépin-1-yle qui est substitué en position 3 ou en position 4 par un groupe amino,
un groupe 3-aminopropyle, ou
un groupe cyclohexyle qui est substitué par un groupe amino,
où, sauf indication contraire, les groupes alkyle, alcényle et alcinyle mentionnés précédemment peuvent être à chaîne linéaire ou ramifiée,
leurs tautomères, énantiomères, diastéréomères, leurs mélanges et leurs sels.

4. Composés pour leur utilisation selon la revendication 1, où l'on utilise les composés de formule générale I selon la revendication 1, dans laquelle
R¹, R² et R³ sont tels que définis dans la revendication 1, 2 ou 3, et
R⁴ désigne un groupe pipéridin-1-yle qui est substitué en position 3 par un groupe amino dans lequel la partie pipéridin-1-yle peut en outre être substituée par un groupe méthyle,
un groupe 3-amino-pipéridin-1-yle dans lequel la partie pipéridin-1-yle est substituée en outre par un groupe pyrrolidin-1-yl-carbonyle,
un groupe 3-amino-pipéridin-1-yle dans lequel la partie pipéridin-1-yle est en outre substituée en position 4 par un groupe hydroxy,
un groupe 3-amino-pipéridin-1-yle dans lequel un atome d'hydrogène en position 2 conjointement avec un atome d'hydrogène en position 5 est remplacé en outre par un pont -CH₂-CH₂-,
un groupe hexahydroazépin-1-yle qui est substitué en position 3 par un groupe amino, ou
un groupe cyclohexyle qui est substitué en position 3 par un groupe amino,
où, sauf indication contraire, les groupes alkyle et, alcényle mentionnés précédemment peuvent être à chaîne linéaire ou ramifiée,
leurs tautomères, énantiomères, diastéréomères, leurs mélanges et leurs sels.

5. Composés pour leur utilisation selon la revendication 1, où l'on utilise les composés de formule générale I selon la revendication 1, dans laquelle R¹, R³ et R⁴ sont tels que définis dans la revendication 1, 2, 3 ou 4 et
R² représente un atome d'hydrogène ou un groupe alkyle en C₁ à C₈,
où, sauf indication contraire, les groupes alkyle mentionnés précédemment peuvent être à chaîne linéaire ou ramifiée,
leurs tautomères, énantiomères, diastéréomères, leurs mélanges et leurs sels.

6. Composés pour leur utilisation selon la revendication 1, où l'on utilise les composés de formule générale I selon la revendication 1, dans laquelle
R¹, R³ et R⁴ sont tels que définis dans la revendication 1, 2, 3 ou 4 et
R² représente un groupe alkyle en C₁ à C₈,
où, sauf indication contraire, les groupes alkyle mentionnés précédemment peuvent être à chaîne linéaire ou ramifiée,
leurs tautomères, énantiomères, diastéréomères, leurs mélanges et leurs sels.

7. Composés de formule générale I selon la revendication 1, dans laquelle
R¹ est un groupe alkyle en C₁ à C₆,
un groupe alcényle en C₃ à C₆,
un groupe alcényle en C₃ à C₄ qui est substitué par un groupe alkyloxy en C₁ à C₂-carbonyle,
un groupe alcinyle en C₃ à C₆,
un groupe cycloalkyle en C₃ à C₆-alkyle en C₁ à C₃,
un groupe phényl-alkyle en C₁ à C₄ dans lequel la partie phényle est substituée par R¹⁰ à R¹², dans lequel
R¹⁰ représente un atome d'hydrogène, de fluor, de chlore ou de brome,
un groupe alkyle en C₁ à C₄, trifluorométhyle, hydroxyméthyle, cycloalkyle en C₃ à C₆, éthinyle ou phényle,
un groupe hydroxy, alkyloxy en C₁ à C₄, difluorométhoxy, trifluorométhoxy, 2,2,2-trifluoroéthoxy, phénoxy, benzyloxy, 2-propén-1-yloxy, 2-propin-1-yloxy, cyanoalkyloxy en C₁ à C₂, alkylsulfonyloxy en C₁ à C₂, phénylsulfonyloxy, carboxy-alkyloxy en C₁ à C₃, alkyloxy en C₁ à C₃-carbonyl-alkyloxy en C₁ à C₃- aminocarbonyl-alkyloxy en C₁ à C₃, alkyle en C₁ à C₂-aminocarbonyl-alkyloxy en C₁ à C₃, di-(alkyle en C₁ à C₂)aminocarbonyl-alkyloxy en C₁ à C₃, pyrrolidin-1-yl-carbonyl-alkyloxy en C₁ à C₃, pipéridin-1-ylcarbonyl-alkyloxy en C₁ à C₃-alkyloxy, morpholin-4-ylcarbonyl-alkyloxy en C₁ à C₃, méthylsulfanylméthoxy, méthylsulfinylméthoxy, méthylsulfonylméthoxy, cycloalkyloxy en C₃ à C₆ ou cycloalkyle en C₃ à C₆-alkyloxy en C₁ à C₂,
un groupe carboxy, alkyloxycarbonyle en C₁ à C₃, carboxy-alkyle en C₁ à C₃, alkyloxy en C₁ à C₃-carbonyl-alkyle en C₁ à C₃, aminocarbonyle, alkylaminocarbonyle en C₁ à C₂, di-(alkyle en C₁ à C₂)aminocarbonyle, morpholin-4-ylcarbonyle ou cyano,
un groupe nitro, amino, alkylamino en C₁ à C₂, di-(alkyle en C₁ à C₂) amino, cyano-alkyle en C₁ à C₂-amino, [N- (cyano-alkyle en C₁ à C₂)-N-alkyle en C₁ à C₂-amino], alkyloxy en C₁ à C₂-carbonyl-alkylamino en C₁ à C₂, alkyle en C₁ à C₂-carbonyalmino, alkyloxy en C₁ à C₂-carbonylamino, alkylsulfonylamino en C₁ à C₃, bis-(alkylsulfonyle en C₁ à C₂)-amino, aminosulfonylamino, alkylamino en C₁ à C₂-sulfonylamino, di-(alkyle en C₁ à C₂)amino-sulfonylamino, morpholin-4-yl-sulfonylamino, (alkylamino en C₁ à C₂)thiocarbonylamino, (alkyloxy en C₁ à C₂-carbonylamino)carbonylamino, aminocarbonylamino, alkyle en C₁ à C₂-aminocarbonylamino, di-(alkyle en C₁ à C₂)aminocarbonylamino ou morpholin-4-ylcarbonylamino,
un groupe 2-oxo-imidazolidin-1-yle, 3-méthyl-2-oxo-imidazolidin-1-yle, 2,4-dioxo-imidazolidin-1-yle, 3-méthyl-2,4-dioxo-imidazolidin-1-yle, 2,5-dioxo-imidazolidin-1-yle, 3-méthyl-2,5-dioxo-imidazolidin-1-yle, 2-oxo-hexahydropyrimidin-1-yle ou 3-méthyl-2-oxo-hexahydropyrimidin-1-yle,
ou
un groupe alkylsulfanyle en C₁ à C₂, alkylsulfinyle en C₁ à C₂, alkylsulfonyle en C₁ à C₂, aminosulfonyle, alkylaminosulfonyle en C₁ à C₂ ou di-(alkyle en C₁ à C₂) aminosulfonyle,
et R¹¹ et R¹² qui peuvent être identiques ou différents, sont respectivement un atome d'hydrogène, de fluor, de chlore ou de brome ou
un groupe méthyle, cyano, trifluorométhyle ou méthoxy,
ou R¹¹ conjointement à R¹², dans la mesure où ceux-ci sont liés aux atomes de carbone voisins, désignent également un groupe méthylènedioxy, difluorométhylènedioxy, 1,3-propylène ou 1,4-butylène,
un groupe phényl-alkyle en C₁ à C₃ dans lequel la partie alkyle est substituée par un groupe carboxy, alkyloxy en C₁ à C₂-carbonyle, aminocarbonyle, alkylaminocarbonyle en C₁ à C₂ ou di- (alkyle en C₁ à C₂)amino-carbonyle,
un groupe phényl-alcényle en C₂ à C₃ dans lequel la partie phényle peut être substituée par un atome de fluor, de chlore ou de brome ou par un groupe méthyle, trifluorométhyle ou méthoxy,
un groupe phényl-(CH₂)ₘ-A-(CH₂)ₙ dans lequel la partie phényle est substituée par R¹⁰ à R¹², R¹⁰ à R¹² étant tels que définis précédemment et
A désigne un groupe carbonyle, hydroxyiminométhylène ou alkyloxy-iminométhylène en C₁ à C₂, m désigne le nombre 0 ou 1 et n, le nombre 1 ou 2,
un groupe phénylcarbonylméthyle dans lequel la partie phényle est substituée par R¹⁰ à R¹², R¹⁰ à R¹² étant tels que définis précédemment et la partie méthyle est substituée par un groupe méthyle ou éthyle,
un groupe phénylcarbonylméthyle dans lequel deux atomes d'hydrogène voisins de la partie phényle sont remplacés par un pont -O-CO-NH-, -NH-CO-NH-, -N=CH-NH-, -N=CH-O-ou -O-CH₂-CO-NH-, les ponts mentionnés précédemment pouvant être substitués par un ou deux groupes méthyle,
un groupe phényl-(CH₂₎ₘ-B-(CH₂)ₙ dans lequel la partie phényle est substituée par R¹⁰ à R¹², R¹⁰ à R¹², m et n étant tels que définis précédemment et
B désigne un groupe méthylène qui est substitué par un groupe hydroxy ou alkyloxy en C₁ à C₂ et est en outre substitué éventuellement par un groupe méthyle,
un groupe naphtylméthyle ou naphtyléthyle dans lequel la partie naphtyle est substituée respectivement par R¹⁰ à R¹², R¹⁰ à R¹² étant tel que défini précédemment,
un groupe hétéroaryl-alkyle en C₁ à C₃, dans lequel le terme hétéroaryle désigne un groupe pyrrolyle, imidazolyle, triazolyle, furanyle, thiényle, oxazolyle, isoxazolyle, thiazolyle, isothiazolyle, pyridyle, pyridazinyle, pyrimidinyle, pyrazinyle, indolyle, benzimidazolyle, 2,3-dihydro-2-oxo-1H-benzimidazolyle, indazolyle, benzofuranyle, 2,3-dihydrobenzo-furanyle, benzoxazolyle, dihydro-2-oxo-benzoxazolyle, benzo-isoxazolyle, benzothiophényle, benzothiazolyle, benzo-isothiazolyle, quinolinyle, 1,2-dihydro-2-oxo-quinolinyle, isoquinolinyle, 1,2-dihydro-1-oxo-isoquinolinyle, cinnolinyle, quinazolinyle, 1,2-dihydro-2-oxo-quinazolinyle, 1,2-dihydro-1-oxo-phtalazin-4-yle, cumarinyle ou 3,4-dihydro-3-oxo-2H-benzo[1,4]oxazinyle,
où les groupes hétéroaryle mentionnés précédemment peuvent être substitués sur des atomes de carbone par un atome de fluor, de chlore ou de brome, par un groupe méthyle, trifluorométhyle, cyano, aminocarbonyle, aminosulfonyle, méthylsulfonyle, nitro, amino, acétylamino, méthylsulfonylamino, méthoxy, difluorométhoxy ou trifluorométhoxy et les groupes imino des groupes hétéroaryle mentionnés précédemment peuvent être substitués par des groupes méthyle ou éthyle,
un groupe furanyl-A-CH₂, thiényl-A-CH₂, thiazolyl-A-CH₂, ou pyridyl-A-CH₂, où A est tel que défini précédemment, un groupe furanyl-B-CH₂-, thiényl-B-CH₂-, thiazolyl-B-CH₂ ou pyridyl-B-CH₂, où B est tel que défini précédemment,
un groupe alkyle en C₁ à C₄-A-(CH₂)ₙ, dans lequel A et n sont tels que définis précédemment,
un groupe cycloalkyle en C₃ à C₆-(CH₂)ₘ-A-(CH₂)ₙ, dans lequel A, m et n sont tels que définis précédemment,
un groupe cycloalkyle en C₃ à C₆-(CH₂)ₘ-B-(CH₂)ₙ, dans lequel B, m et n sont tels que définis précédemment,
un groupe R²¹-A-(CH₂)ₙ dans lequel R²¹ désigne un groupe alkyloxycarbonyle en C₁ à C₂, aminocarbonyle, alkylaminocarbonyle en C₁ à C₂, di-(alkyle en C₁ à C₂)aminocarbonyle, pyrrolidin-1-yl-carbonyle, pipéridin-1-yl-carbonyle ou morpholin-4-yl-carbonyle, et A et n sont tels que définis précédemment,
un groupe phényl-D-alkyle en C₁ à C₃ dans lequel la partie phényle est substituée éventuellement par un atome de fluor, de chlore ou de brome, un groupe méthyle, trifluorométhyle ou méthoxy et D représente un atome d'oxygène ou de soufre, un groupe sulfinyle ou sulfonyle,
un groupe alkyle en C₁ à C₄ substitué par un groupe Rₐ, dans lequel
Rₐ représente un groupe cyano, carboxy, alkyloxy en C₁ à C₃-carbonyle, aminocarbonyle, alkyle en C₁ à C₂-aminocarbonyle, di-(alkyle en C₁ à C₂)aminocarbonyle, pyrrolidin-1-ylcarbonyle, pipéridin-1-ylcarbonyle ou morpholin-4-ylcarbonyle, ou
un groupe alkyle en C₂ à C₄ substitué par un groupe R_{b}, dans lequel
R_{b} représente un groupe hydroxy, alkyloxy en C₁ à C₃, amino, alkylamino en C₁ à C₃, di-(alkyle en C₁ à C₃) -amino, pyrrolidin-1-yle, pipéridin-1-yle, morpholin-4-yle, pipérazin-1-yle, 4-méthyl-pipérazin-1-yle ou 4-éthyl-pipérazin-1-yle et est isolé par au moins deux atomes de carbone de l'atome d'azote cyclique en position 1 du squelette de la xanthine,
R² désigne un atome d'hydrogène,
un groupe alkyle en C₁ à C₆,
un groupe alcényle en C₂ à C₄,
un groupe alcinyle en C₃ à C₄,
un groupe cycloalkyle en C₃ à C₆,
un groupe cycloalkyle en C₃ à C₆-alkyle en C₁ à C₃,
un groupe tétrahydrofuran-3-yle, tétrahydropyran-3-yle, tétrahydropyran-4-yle, tétra-hydrofuranylméthyle ou tétrahydropyranylméthyle,
un groupe phényle qui est éventuellement substitué par un atome de fluor, de chlore ou de brome ou par un groupe méthyle, trifluorométhyle, hydroxy, méthoxy, difluorométhoxy ou trifluorométhoxy,
un groupe phényl-alkyle en C₁ à C₄ dans lequel la partie phényle est éventuellement substituée par un atome de fluor, de chlore ou de brome, un groupe méthyle, trifluorométhyle, diméthylamino, hydroxy, méthoxy, difluorométhoxy ou trifluorométhoxy,
un groupe phényl-alcényle en C₂ à C₃ dans lequel la partie phényle peut être substituée par un atome de fluor, de chlore ou de brome ou par un groupe méthyle, trifluorométhyle ou méthoxy,
un groupe phénylcarbonyl-alkyle en C₁ à C₂ dans lequel la partie phényle est substituée éventuellement par un atome de fluor, de chlore ou de brome, un groupe méthyle, trifluorométhyle, hydroxy, méthoxy, difluorométhoxy ou trifluorométhoxy,
un groupe hétéroaryl-alkyle en C₁ à C₃, le terme hétéroaryle étant tel que défini précédemment,
un groupe furanylcarbonylméthyle, thiénylcarbonylméthyle, thiazolylcarbonylméthyle ou pyridylcarbonylméthyle,
un groupe alkyle en C₁ à C₄-carbonyl-alkyle en C₁ à C₂, un groupe cycloalkyle en C₃ à C₆-carbonyl-alkyle en C₁ à C₂,
un groupe phényl-D-alkyle en C₁ à C₃ dans lequel la partie phényle est substituée éventuellement par un atome de fluor, de chlore ou de brome, un groupe méthyle, trifluorométhyle, hydroxy, méthoxy, difluorométhoxy ou trifluorométhoxy et D est tel que défini précédemment, ou
un groupe alkyle en C₁ à C₄ substitué par un groupe Rₐ, dans lequel Rₐ est tel que défini précédemment ou
un groupe alkyle en C₂ à C₄ substitué par un groupe R_{b}, R_{b} étant tel que défini précédemment et par au moins deux atomes de carbone de l'atome d'azote cyclique en position 3 du squelette de la xanthine,
R³ désigne un groupe alkyle en C₁ à C₃ substitué par le groupe R_{c}, dans lequel
R_{c} représente un groupe cycloalkyle en C₃ à C₇ éventuellement substitué par un ou deux groupes alkyle en C₁ à C₃, ou
un groupe cycloalcényle en C₅ à C₇ substitué éventuellement par un ou deux groupes alkyle en C₁ à C₃,
un groupe alcényle en C₃ à C₈,
un groupe alcényle en C₃ à C₆, substitué par un atome de fluor, de chlore ou de brome ou un groupe trifluorométhyle,
un groupe alcinyle en C₃ à C₈, ou
un groupe aryl-alcényle en C₂ à C₄,
et
R⁴ est un groupe azétidin-1-yle ou pyrrolidin-1-yle qui est substitué en position 3 par un groupe RₑNR_{d} et peut en outre être substitué par un ou deux groupes alkyle en C₁ à C₃, dans lequel
Rₑ désigne un atome d'hydrogène ou un groupe alkyle en C₁ à C₃ et
R_{d} désigne un atome d'hydrogène ou un groupe alkyle en C₁ à C₃,
un groupe pipéridin-1-yle ou hexahydroazépin-1-yle qui est substitué en position 3 ou en position 4 par un groupe RₑNR_{d} et peut en outre être substitué par un ou deux groupes alkyle en C₁ à C₃, Rₑ et R_{d} étant tels que définis précédemment,
un groupe 3-amino-pipéridin-1-yle dans lequel la partie pipéridin-1-yle est substituée en outre par un groupe aminocarbonyle, alkyle en C₁ à C₂-aminocarbonyle, di-(alkyle en C₁ à C₂)aminocarbonyle, pyrrolidin-1-ylcarbonyle, (2-cyano-pyrrolidin-1-yl)carbonyle, thiazolidin-3-yl-carbonyle, (4-cyano-thiazolidin-3-yl)carbonyle, pipéridin-1-ylcarbonyle ou morpholin-4-ylcarbonyle,
un groupe 3-amino-pipéridin-1-yle dans lequel la partie pipéridin-1-yle est en outre substituée en position 4 ou en position 5 par un groupe hydroxy ou méthoxy,
un groupe 3-amino-pipéridin-1-yle dans lequel le groupe méthylène est remplacé en position 2 ou en position 6 par un groupe carbonyle,
un groupe pipéridin-1-yle ou hexahydroazépin-1-yle substitué en position 3 par un groupe amino, alkylamino en C₁ à C₃ ou di-(alkyle en C₁ à C₃) dans lequel respectivement deux atomes d'hydrogène sont remplacés sur le squelette du carbone du groupe pipéridin-1-yle ou hexa-hydroazépin-1-yle par un pont alkylène à chaîne linéaire, ce pont contenant 2 à 5 atomes de carbone, lorsque les deux atomes d'hydrogène se trouvent sur le même atome de carbone, ou 1 à 4 atomes de carbone, lorsque les atomes d'hydrogène se trouvent sur des atomes de carbone voisin ou contenant 1 à 4 atomes de carbone lorsque les atomes d'hydrogène se trouvent sur des atomes de carbone qui sont séparés par un atome ou contenant 1 à 3 atomes de carbone lorsque les deux atomes d'hydrogène se trouvent sur des atomes de carbone qui sont séparés par deux atomes,
un groupe azétidin-1-yle, pyrrolidin-1-yle, pipéridin-1-yle ou hexahydroazépin-1-yle qui est substitué par un groupe amino-alkyle en C₁ à C₃, alkylamino en C₁ à C₃-alkyle en C₁ à C₃ ou di-(alkyle en C₁ à C₃)amino-alkyle en C₁ à C₃,
un groupe cycloalkyle en C₃ à C₇ qui est substitué par un groupe amino, alkylamino en C₁ à C₃ ou di-(alkyle en C₁ à C₃) amino,
un groupe cycloalkyle en C₃ à C₇ qui est substitué par un groupe amino-alkyle en C₁ à C₃, alkylamino en C₁ à C₃-alkyle en C₁ à C₃ ou di- (alkyle en C₁ à C₃) amino-alkyle en C₁ à C₃,
un groupe cycloalkyle en C₃ à C₇-alkyle en C₁ à C₂ dans lequel la partie cycloalkyle est substituée par un groupe amino, alkylamino en C₁ à C₃ ou di-(alkyle en C₁ à C₃)-amino,
un groupe cycloalkyle en C₃ à C₇-alkyle en C₁ à C₂ dans lequel la partie cycloalkyle est substituée par un groupe amino-alkyle en C₁ à C₃, alkylamino en C₁ à C₃-alkyle en C₁ à C₃ ou di-(alkyle en C₁ à C₃)amino-alkyle en C₁ à C₃,
un groupe R¹⁹-alkyle en C₃ à C₄ dans lequel la partie alkyle en C₃ à C₄ est à chaîne linéaire et peut être substituée par un radical R¹⁵ et peut en outre être substituée par un ou deux groupes alkyle en C₁ à C₃, où R¹⁵ représente un groupe alkyle en C₁ à C₃ et R¹⁹, un groupe amino, alkylamino en C₁ à C₃ ou di-(alkyle en C₁ à C₃)-amino,
un groupe 3-amino-2-oxo-pipéridin-5-yle ou 3-amino-2-oxo-1-méthyl-pipéridin-5-yle,
un groupe pyrrolidin-3-yle, pipéridin-3-yle, pipéridin-4-yle, hexahydroazépin-3-yle ou hexahydroazépin-4-yle qui est substitué en position 1 par un groupe amino, alkylamino en C₁ à C₃ ou di-(alkyle en C₁ à C₃)amino,
ou un groupe azétidin-2-yl-alkyle en C₁ à C₂, azétidin-3-yl-alkyle en C₁ à C₂, pyrrolidin-2-yl-alkyle en C₁ à C₂, pyrrolidin-3-yle, pyrrolidin-3-yl-alkyle en C₁ à C₂, pipéridin-2-yl-alkyle en C₁ à C₂, pipéridin-3-yle, pipéridin-3-yl-alkyle en C₁ à C₂, pipéridin-4-yle ou pipéridin-4-yl-alkyle en C₁ à C₂, les groupes mentionnés précédemment pouvant respectivement être substitués par un ou deux groupes alkyle en C₁ à C₃,
où parmi les groupes aryle mentionnés dans la définition des radicaux cités précédemment sont des groupes phényle ou naphtyle qui peuvent être mono- ou disubstitués indépendamment les uns des autres par Rₕ, les substituants pouvant être identiques ou différents et Rₕ représente un atome de fluor, de chlore, de brome ou d'iode, un groupe trifluorométhyle, cyano, nitro, amino, alkyle en C₁ à C₃, cyclopropyle, éthényle, éthinyle, hydroxy, alkyloxy en C₁ à C₃, difluorométhoxy ou trifluorométhoxy et
où, sauf indication contraire, les groupes alkyle et alcényle mentionnés précédemment peuvent être à chaîne linéaire ou ramifiée,
leurs tautomères, énantiomères, diastéréomères, leurs mélanges et leurs sels,
pour leur utilisation dans la thérapie combinée avec d'autres substances actives, par exemple, choisies dans le groupe des antidiabétiques, des hypolipidémiants, des composés augmentant le taux de HDL, des substances actives pour le traitement de l'obésité et des substances actives agissant sur l'hypertension.

8. Composés pour leur utilisation selon la revendication 7, où l'on utilise les composés de formule générale I selon la revendication 1, dans laquelle R¹ est un groupe alkyle en C₁ à C₄,
un groupe alcényle en C₃ à C₅,
un groupe 2-propén-1-yle qui est substitué par un groupe méthoxycarbonyle,
un groupe alcinyle en C₃ à C₅,
un groupe phényl-alkyle en C₁ à C₄ dans lequel la partie phényle est substituée par R¹⁰ à R¹², dans lequel
R¹⁰ est un atome d'hydrogène, de fluor, de chlore ou de brome,
un groupe méthyle, éthyle, trifluorométhyle ou éthinyle,
un groupe hydroxy, méthoxy, éthoxy, difluorométhoxy, trifluorométhoxy, 2,2,2-trifluorométhoxy, phénoxy, benzyloxy, 2-propén-1-yloxy, 2-propin-1-yl-oxy, cyano-alkyloxy en C₁ à C₂, alkyle en C₁ à C₂-sulfonyloxy, phénylsulfonyloxy, carboxy-alkyloxy en C₁ à C₂, alkyloxy en C₁ à C₂-carbonyl-alkyloxy en C₁ à C₂, aminocabonyl-alkyloxy en C₁ à C₂, alkyle en C₁ à C₂-aminocarbonyl-alkyloxy en C₁ à C₂, di-(alkyle en C₁ à C₂) aminocarbonyl-alkyloxy en C₁ à C₂, pyrrolidin-1-ylcarbonyl-alkyloxy en C₁ à C₂, pipéridin-1-ylcarbonyl-alkyloxy en C₁ à C₂, morpholin-4-ylcarbonyl-alkyloxy en C₁ à C₂,
un groupe carboxy, alkyloxy en C₁ à C₂-carbonyle, aminocarbonyle, alkylamino en C₁ à C₂-carbonyle, di-(alkyle en C₁ à C₂)aminocarbonyle, morpholin-4-ylcarbonyle ou cyano,
un groupe nitro, amino, alkylamino en C₁ à C₂, di-(alkyle en C₁ à C₂) amino, cyano-alkyle en C₁ à C₂-amino, [N-(cyano-alkyle en C₁ à C₂)-N-méthylamino], alkyloxy en C₁ à C₂-carbonyl-alkylamino en C₁ à C₂, alkyle en C₁ à C₂-carbonylamino, alkyloxy en C₁ à C₂-carbonylamino, alkylsulfonylamino en C₁ à C₂, bis- (alkylsulfonyle en C₁ à C₂) -amino, aminosulfonylamino, alkylamino en C₁ à C₂-sulfonylamino, di- (alkyle en C₁ à C₂) amino-sulfonylamino, morpholin-4-yl-sulfonylamino, (alkylamino en C₁ à C₂)thiocarbonylamino, (alkyloxy en C₁ à C₂-carbonylamino)carbonylamino, aminocarbonylamino, alkylaminocarbonyle en C₁ à C₂-amino, di-(alkyle en C₁ à C₂) aminocarbonylamino ou morpholin-4-yl-carbonylamino,
un groupe 2-oxo-imidazolidin-1-yle, 3-méthyl-2-oxo-imidazolidin-1-yle, 2,4-dioxo-imidazolidin-1-yle, 3-méthyl-2,4-dioxo-imidazolidin-1-yle, 2,5-dioxo-imidazolidin-1-yle, 3-méthyl-2,5-dioxo-imidazolidin-1-yle, 2-oxo-hexahydropyrimidin-1-yle ou 3-méthyl-2-oxo-hexahydropyrimidin-1-yle,
ou
un groupe alkylsulfanyle en C₁ à C₂, alkylsulfinyle en C₁ à C₂, alkylsulfonyle en C₁ à C₂, aminosulfonyle, alkylaminosulfonyle en C₁ à C₂ ou di-(alkyle en C₁ à C₂) aminosulfonyle,
et R¹¹ et R¹² qui peuvent être identiques ou différents, désignent un atome d'hydrogène, de fluor, de chlore ou de brome ou
un groupe méthyle, cyano ou méthoxy,
ou R¹¹ conjointement avec R¹², dans la mesure où ceux-ci sont liés aux atomes de carbone voisins, désignent également un groupe méthylènedioxy,
un groupe phénylméthyle dans lequel la partie méthyle est substituée par un groupe carboxy, méthoxy, carbonyle ou aminocarbonyle,
un groupe 2-phényléthyle dans lequel la partie éthyle est substituée par un groupe carboxy, méthoxy, carbonyle ou aminocarbonyle,
un groupe 2-phényléthyle dans lequel la partie éthyle en position 2 est substituée par un groupe hydroxy, méthoxy, hydroxy-imino ou méthoxyimino,
un groupe 2-phényléthyle dans lequel la partie éthyle en position 2 est substituée par un groupe hydroxy et un groupe méthyle,
un groupe phénylcarbonylméthyle dans lequel la partie phényle est substituée par R¹⁰ à R¹², R¹⁰ à R¹² étant tels que définis précédemment,
un groupe 1-(phénylcarbonyl)éthyle ou 2-(phénylcarbonyl)éthyle,
un groupe 2-phényléthényle,
un groupe phénylsulfanylméthyle ou phénylsulfinylméthyle,
un groupe 2-(phényloxy)éthyle,
un groupe naphtylméthyle ou naphtyléthyle, la partie naphtyle pouvant être respectivement substituée par un groupe méthyle, nitro, amino, acétylamino, méthylsulfonylamino, cyano, aminocarbonyle ou aminosulfonyle,
un groupe oxazolylméthyle, isoxazolylméthyle, thiazolylméthyle, pyridylméthyle, benzofuranylméthyle, 2,3-dihydrobenzofuranylméthyle, benzo[d]isoxazolylméthyle, benzo[d]isothiazolylméthyle, (1*H*-indazol-3-yl)méthyle, quinolinylméthyle, (1,2-dihydro-2-oxo-quinolin-4-yl)méthyle,
isoquinolinylméthyle, (1,2-dihydro-1-oxo-isoquinolin-4-yl) méthyle, cinnolinylméthyle, quinazolinylméthyle, (1,2-dihydro-2-oxo-quinazolin-4-yl)méthyle, (1,2-dihydro-1-oxo-phtalazin-4-yl)méthyle ou cumarinylméthyle dans lequel la partie hétérocyclique peut être substituée respectivement par un groupe méthyle,
un groupe quinolinylméthyle ou isoquinolinylméthyle, dans lequel la partie hétérocyclique est substituée respectivement par un groupe cyano, nitro, amino, acétylamino, méthylsulfonylamino, aminocarbonyle ou aminosulfonyle,
un groupe pyrrolyléthyle, triazolyléthyle, thiényléthyle, thiazolyléthyle ou pyridyléthyle dans lequel la partie hétérocyclique peut être substituée respectivement par un groupe méthyle,
un groupe furanylcarbonylméthyle, thiénylcarbonylméthyle, thiazolylcarbonylméthyle ou pyridylcarbonylméthyle,
un groupe méthyle qui est substitué par un groupe cyclopropyle, cyano, carboxy, aminocarbonyle ou méthoxycarbonyle,
un groupe éthyle qui est substitué en position 2 par un groupe hydroxy, méthoxy, diméthylamino, carboxy ou méthoxycarbonyle, ou
un groupe propyle qui est substitué en position 3 par un groupe hydroxy, diméthylamino, carboxy ou méthoxycarbonyle, ou
un groupe 2-oxo-propyle,
R² désigne un atome d'hydrogène,
un groupe alkyle en C₁ à C₆,
un groupe éthényle,
un groupe 2-propén-1-yle ou 2-propin-1-yle,
un groupe cycloalkyle en C₃ à C₆,
un groupe tétrahydrofuran-3-yle, tétrahydropyran-3-yle, tétrahydropyran-4-yle, tétrahydrofuranylméthyle ou tétrahydropyranylméthyle,
un groupe phényle
un groupe phényl-alkyle en C₁ à C₄ dans lequel la partie phényle peut être substituée par un atome de fluor ou de chlore, un groupe méthyle, diméthylamino, hydroxy, méthoxy ou trifluorométhoxy,
un groupe phénylcarbonylméthyle dans lequel la partie phényle peut être substituée par un atome de fluor ou de chlore, un groupe hydroxy, méthoxy ou trifluorométhoxy,
un groupe 2-phényléthényle,
un groupe 2-(phényloxy)éthyle,
un groupe pyridylméthyle ou pyridyléthyle,
un groupe méthyle qui est substitué par un groupe cycloalkyle en C₃ à C₆, cyano, carboxy ou méthoxycarbonyle, ou
un groupe éthyle qui est substitué en position 2 par un groupe cycloalkyle en C₃ à C₆, cyano, carboxy, méthoxycarbonyle, hydroxy, méthoxy ou diméthylamino,
ou un groupe propyle qui est substitué en position 3 par un groupe cycloalkyle en C₃ à C₆, cyano, carboxy, méthoxycarbonyle, hydroxy, méthoxy ou diméthylamino,
R³ désigne un groupe alcényle en C₄ à C₆,
un groupe 1-cyclopentén-1-ylméthyle ou 1-cyclohexén-1-ylméthyle,
un groupe 1-cyclopentén-1-ylméthyle dans lequel la partie 1-cyclopentén-1-yle est substituée par un groupe méthyle,
un groupe 2-propin-1-yle, 2-butin-1-yle ou 2-pentin-1-yle,
un groupe cyclopropylméthyle ou
un groupe cyclopropylméthyle dans lequel la partie cyclopropyle est substituée par un groupe méthyle, et
R⁴ est un groupe pipéridin-1-yle qui est substitué en position 3 par un groupe amino, dans lequel la partie pipéridin-1-yle peut être substituée en outre par un groupe méthyle,
un groupe 3-amino-pipéridin-1-yle dans lequel la partie pipéridin-1-yle est substituée en outre par un groupe aminocarbonyle, méthylaminocarbonyle, diméthylaminocarbonyle, pyrrolidin-1-yl-carbonyle, (2-cyano-pyrrolidin-1-yl)carbonyle, thiazolidin-3-yl-carbonyle, (4-cyano-thiazolidin-3-yl)carbonyle, pipéridin-1-ylcarbonyle ou morpholin-4-ylcarbonyle,
un groupe 3-amino-pipéridin-1-yle dans lequel la partie pipéridin-1-yle en position 4 ou en position 5 est substituée en outre par un groupe hydroxy ou méthoxy,
un groupe 3-amino-pipéridin-1-yle dans lequel un atome d'hydrogène en position 2 conjointement avec un atome d'hydrogène en position 5 est remplacé par un pont - CH₂-CH₂-
un groupe hexahydroazépin-1-yle qui est substitué en position 3 par un groupe amino,
un groupe 3-amino-2-oxo-pipéridin-5-yle ou 3-amino-2-oxo-1-méthyl-pipéridin-5-yle, ou
un groupe cyclohexyle qui est substitué en position 3 par un groupe amino,
où, sauf indication contraire, les groupes alkyle et alcényle mentionnés précédemment peuvent être à chaîne linéaire ou ramifiée,
leurs tautomères, énantiomères, diastéréomères, leurs mélanges et leurs sels.

9. Composés pour leur utilisation selon la revendication 7, où l'on utilise les composés de formule générale I selon la revendication 1, dans laquelle
R¹ est un groupe alkyle en C₁ à C₄,
un groupe alcényle en C₃ à C₅,
un groupe 2-propén-1-yle qui est substitué par un groupe méthoxycarbonyle,
un groupe alcinyle en C₃ à C₅,
un groupe phényl-alkyle en C₁ à C₄ dans lequel la partie phényle peut être substituée par un ou deux atomes de fluor, un ou deux atomes de chlore, un atome de brome, un à trois groupes méthyle, un groupe trifluorométhyle, hydroxy, méthoxy, nitro, amino, carboxy ou éthoxycarbonyle,
un groupe 2-phényléthyle dans lequel la partie éthyle en position 2 est substituée par un groupe hydroxy, méthoxy ou hydroxy-imino,
un groupe phénylcarbonylméthyle dans lequel la partie phényle peut être substituée par un atome de fluor ou par un groupe méthyle, aminocarbonyle, aminosulfonyle, cyano, hydroxy, méthoxy, phénoxy, benzyloxy, 2-propén-1-yloxy, 2-propin-1-yloxy, cyanométhoxy, (méthoxycarbonyl)méthoxy, (aminocarbonyl)méthoxy, (méthylaminocarbonyl)-méthoxy, (diméthylaminocarbonyl) méthoxy, méthylsulfonyloxy, phénylsulfonyloxy, nitro, amino, (méthoxycarbonyl)méthylamino, acétylamino, méthoxycarbonyle, amino, méthylsulfonylamino, bis-(méthylsulfonyl)-amino, aminocarbonylamino diméthylaminocarbonylamino, (méthylamino) thiocarbonylamino, (éthoxycarbonylamino)carbonylamino ou cyanométhylamino,
un groupe phénylcarbonylméthyle dans lequel la partie phényle est substituée par deux groupes méthoxy ou par un atome de brome et par un groupe diméthylamino,
un groupe 2-(phénylcarbonyl)éthyle,
un groupe 2-phényléthényle,
un groupe 2-(phénoxy)éthyle,
un groupe phénylsulfanylméthyle ou phénylsulfinyl-méthyle,
un groupe naphtylméthyle ou naphtyléthyle,
un groupe isoxazolylméthyle, thiazolylméthyle, pyridylméthyle, benzo[d]isoxazolylméthyle, benzo[d]isothiazolylméthyle, (*1H*-indazol-3-yl)méthyle, quinolinylméthyle ou isoquinolinylméthyle dans lequel la partie hétérocyclique peut être substituée respectivement par un groupe méthyle,
un groupe isoquinolinylméthyle dans lequel la partie isoquinolinyle est substituée par un groupe nitro ou amino,
un groupe (1,2-dihydro-2-oxo-quinolin-4-yl)méthyle,
un groupe pyrrolyléthyle, triazolyléthyle, thiényléthyle, thiazolyléthyle ou pyridyléthyle dans lequel la partie hétérocyclique peut être substituée respectivement par un groupe méthyle,
un groupe thiénylcarbonyle,
un groupe méthyle qui est substitué par un groupe cyclopropyle, cyano, carboxy, aminocarbonyle ou méthoxycarbonyle,
un groupe éthyle qui est substitué en position 2 par un groupe hydroxy, méthoxy, diméthylamino, carboxy ou méthoxycarbonyle, ou
un groupe propyle qui est substitué en position 3 par un groupe hydroxy, diméthylamino, carboxy ou méthoxycarbonyle, ou
un groupe 2-oxopropyle,
R² désigne un atome d'hydrogène,
un groupe alkyle en C₁ à C₆,
un groupe éthényle,
un groupe 2-propén-1-yle ou 2-propin-1-yle,
un groupe phényle,
un groupe phényl-alkyle en C₁ à C₄ dans lequel la partie phényle peut être substituée par un atome de fluor, un groupe méthyle ou méthoxy,
un groupe phénylcarbonylméthyle,
un groupe 2-phényléthényle,
un groupe méthyle qui est substitué par un groupe cyclopropyle, cyano, carboxy ou méthoxycarbonyle, ou
un groupe éthyle qui est substitué en position 2 par un groupe cyano, hydroxy, méthoxy ou diméthylamino,
R³ désigne un groupe alcényle en C₄ à C₆,
un groupe 1-cyclopentén-1-ylméthyle ou 1-cyclohexén-1-ylméthyle,
un groupe 2-propin-1-yle, 2-butin-1-yle ou 2-pentin-1-yle ou
un groupe cyclopropylméthyle et
R⁴ désigne un groupe pipéridin-1-yle qui est substitué en position 3 par un groupe amino dans lequel la partie pipéridin-1-yle peut en outre être substituée par un groupe méthyle,
un groupe 3-amino-pipéridin-1-yle dans lequel la partie pipéridin-1-yle est substituée en outre par un groupe pyrrolidin-1-yl-carbonyle,
un groupe 3-amino-pipéridin-1-yle dans lequel la partie pipéridin-1-yle est en outre substituée en position 4 par un groupe hydroxy,
un groupe 3-amino-pipéridin-1-yle dans lequel un atome d'hydrogène en position 2 conjointement avec un atome d'hydrogène en position 5 est remplacé par un pont - CH₂-CH₂-,
un groupe hexahydroazépin-1-yle qui est substitué en position 3 par un groupe amino, ou
un groupe cyclohexyle qui est substitué en position 3 par un groupe amino,
où, sauf indication contraire, les groupes alkyle et alcényle mentionnés précédemment peuvent être à chaîne linéaire ou ramifiée,
leurs tautomères, énantiomères, diastéréomères, leurs mélanges et leurs sels.

10. Composés pour leur utilisation selon la revendication 7, où l'on utilise les composés de formule générale I selon la revendication 1, dans laquelle
R¹, R² et R³ sont tels que définis dans la revendication 7 et
R⁴ désigne un groupe azétidin-1-yle ou pyrrolidin-1-yle qui peut être substitué en position 3 par un groupe RₑNR_{d} et peut en outre être substitué par un ou deux groupes alkyle en C₁ à C₃, où
Rₑ désigne un atome d'hydrogène ou un groupe alkyle en C₁ à C₃ et
R_{d} désigne un atome d'hydrogène ou un groupe alkyle en C₁ à C₃,
un groupe pipéridin-1-yle ou hexahydroazépin-1-yle qui est substitué en position 3 ou en position 4 par un groupe RₑNR_{d} et peut en outre être substitué par un ou deux groupes alkyle en C₁ à C₃, où Rₑ et R_{d} sont tels que définis précédemment,
un groupe 3-amino-pipéridin-1-yle dans lequel la partie pipéridin-1-yle est substituée en outre par un groupe aminocarbonyle, alkyle en C₁ à C₂-aminocarbonyle, di-(alkyle en C₁ à C₂)aminocarbonyle, pyrrolidin-1-yl-carbonyle, (2-cyano-pyrrolidin-1-yl)carbonyle, thiazolidin-3-yl-carbonyle, (4-cyano-thiazolidin-3-yl)carbonyle, pipéridin-1-ylcarbonyle ou morpholin-4-ylcarbonyle,
un groupe 3-amino-pipéridin-1-yle dans lequel la partie pipéridin-1-yle est substituée en outre en position 4 ou en position 5 par un groupe hydroxy ou méthoxy,
un groupe 3-amino-pipéridin-1-yle dans lequel la partie méthylène est substituée en outre en position 2 ou en position 6 par un groupe carbonyle,
un groupe pipéridin-1-yle ou hexahydro-azépin-1-yle substitué en position 3 par un groupe amino, alkylamino en C₁ à C₃ ou di-(alkyle en C₁ à C₃)-amino dans lesquels respectivement deux atomes d'hydrogène sont remplacés sur le squelette du carbone du groupe pipéridin-1-yle ou hexahydro-azépin-1-yle par un pont alkylène à chaîne linéaire, ce pont contenant 2 à 5 atomes de carbone lorsque les deux atomes d'hydrogène se trouvent sur le même atome de carbone, ou 1 à 4 atomes de carbone lorsque les atomes d'hydrogène se trouvent sur des atomes de carbone voisins, ou contenant 1 à 4 atomes de carbone lorsque les atomes d'hydrogène se trouvent sur des atomes de carbone qui sont séparés par un atome ou contenant 1 à 3 atomes de carbone lorsque les deux atomes d'hydrogène se trouvent sur des atomes de carbone qui sont séparés par deux atomes,
un groupe azétidin-1-yle, pyrrolidin-1-yle, pipéridin-1-yle ou hexahydroazépin-1-yle qui est substitué par un groupe amino-alkyle en C₁ à C₃, alkylamino en C₁ à C₃-alkyle en C₁ à C₃ ou di-(alkyle en C₁ à C₃)amino-alkyle en C₁ à C₃,
un groupe cycloalkyle en C₃ à C₇ qui est substitué par un groupe amino, alkylamino en C₁ à C₃ ou di-(alkyle en C₁ à C₃)-amino,
un groupe cycloalkyle en C₃ à C₇ qui est substitué par un groupe amino-alkyle en C₁ à C₃, alkylamino en C₁ à C₃-alkyle en C₁ à C₃ ou di- (alkyle en C₁ à C₃)amino-alkyle en C₁ à C₃,
un groupe cycloalkyle en C₃ à C₇-alkyle en C₁ à C₂, dans lequel la partie cycloalkyle est substituée par un groupe amino, alkylamino en C₁ à C₃ ou di-(alkyle en C₁ à C₃)-amino,
un groupe cycloalkyle en C₃ à C₇-alkyle en C₁ à C₂ dans lequel la partie cycloalkyle est substituée par un groupe amino-alkyle en C₁ à C₃, alkylamino en C₁ à C₃-alkyle en C₁ à C₃ ou di-(alkyle en C₁ à C₃)amino-alkyle en C₁ à C₃,
un groupe R¹⁹- alkyle en C₃ à C₄ dans lequel la partie alkyle en C₃ à C₄ est à chaîne linéaire et peut être substituée par un radical R¹⁵ et, en outre, peut être substituée par un ou deux groupes alkyle en C₁ à C₃,
R¹⁵ représentant un groupe alkyle en C₁ à C₄ et R¹⁹ un groupe amino, alkylamino en C₁ à C₃ ou di-(alkyle en C₁ à C₃)-amino,
un groupe 3-amino-2-oxo-pipéridin-5-yle ou 3-amino-2-oxo-1-méthyl-pipéridin-5-yle,
un groupe pyrrolidin-3-yle, pipéridin-3-yle, pipéridin-4-yle, hexahydroazépin-3-yle ou hexahydroazépin-4-yle qui est substitué en position 1 par un groupe amino, alkylamino en C₁ à C₃ ou di-(alkyle en C₁ à C₃)amino,
ou un groupe azétidin-2-yle-alkyle en C₁ à C₂, azétidin-3-yl-alkyle en C₁ à C₂, pyrrolidin-2-yl-alkyle en C₁ à C₂, pyrrolidin-3-yle, pyrrolidin-3-yl-alkyle en C₁ à C₂, pipéridin-2-yl-alkyle en C₁ à C₂, pipéridin-3-yle, pipéridin-3-yl-alkyle en C₁ à C₂, pipéridin-4-yle ou pipéridin-4-yl-alkyle en C₁ à C₂, dans lequel les groupes mentionnés précédemment peuvent être substitués respectivement par un ou deux groupes alkyle en C₁ à C₃,
leurs tautomères, énantiomères, diastéréomères, leurs mélanges et leurs sels.

11. Composés pour leur utilisation selon la revendication 7, où l'on utilise les composés de formule générale I selon la revendication 1, dans laquelle
R¹, R² et R³ sont tels que définis dans la revendication 7 ou 8 et
R⁴ désigne un groupe pipéridin-1-yle qui est substitué en position 3 par un groupe amino dans lequel la partie pipéridin-1-yle peut en outre être substituée par un groupe méthyle,
un groupe 3-amino-pipéridin-1-yle dans lequel la partie pipéridin-1-yle est substituée en outre par un groupe aminocarbonyle, méthylaminocarbonyle, diméthylaminocarbonyle, pyrrolidin-1-yl-carbonyle, (2-cyano-pyrrolidin-1-yl)carbonyle, thiazolidin-3-yl-carbonyle, (4-cyano-thiazolidin-3-yl)carbonyle, pipéridin-1-ylcarbonyle ou morpholin-4-ylcarbonyle,
un groupe 3-amino-pipéridin-1-yle dans lequel la partie pipéridin-1-yle est en outre substituée en position 4 ou en position 5 par un groupe hydroxy ou méthoxy,
un groupe 3-amino-pipéridin-1-yle dans lequel un atome d'hydrogène en position 2 conjointement avec un atome d'hydrogène en position 5 est remplacé en outre par un pont -CH₂-CH₂-,
un groupe hexahydroazépin-1-yle qui est substitué en position 3 par un groupe amino,
un groupe 3-amino-2-oxo-pipéridin-5-yle ou 3-amino-2-oxo-1-méthyl-pipéridin-5-yle,
un groupe cyclohexyle qui est substitué en position 3 par un groupe amino,
où, sauf indication contraire, les groupes alkyle et alcényle mentionnés précédemment peuvent être à chaîne linéaire ou ramifiée,
leurs tautomères, énantiomères, diastéréomères, leurs mélanges et leurs sels.

12. Composés pour leur utilisation selon la revendication 7, où l'on utilise les composés de formule générale I selon la revendication 1, dans laquelle
R¹, R² et R³ sont tels que définis dans la revendication 7, 8 ou 9 et
R⁴ représente un groupe pipéridin-1-yle qui est substitué en position 3 par un groupe amino, dans lequel la partie pipéridin-1-yle peut être substituée en outre par un groupe méthyle
un groupe 3-amino-pipéridin-1-yle dans lequel la partie pipéridin-1-yle est substituée en outre par un groupe pyrrolidin-1-yl-carbonyle,
un groupe 3-amino-pipéridin-1-yle dans lequel la partie pipéridin-1-yle est en outre substituée en position 4 par un groupe hydroxy,
un groupe 3-amino-pipéridin-1-yle dans lequel un atome d'hydrogène en position 2 conjointement avec un atome d'hydrogène en position 5 est remplacé par un pont - CH₂-CH₂-,
un groupe hexahydroazépin-1-yle qui est substitué en position 3 par un groupe amino,
un groupe cyclohexyle qui est substitué en position 3 par un groupe amino,
où, sauf indication contraire, les groupes alkyle et alcényle mentionnés précédemment peuvent être à chaîne linéaire ou ramifiée,
leurs tautomères, énantiomères, diastéréomères, leurs mélanges et leurs sels.

13. Composés suivants de formule générale I selon la revendication 1 :
(1) 1,3-diméthyl-7-benzyl-8-(3-amino-pyrrolidin-1-yl)-xanthine,
(2) 1,3-diméthyl-7-(3-méthyl-2-butén-1-yl)-8-(3-amino-pyrrolidin-1-yl)-xanthine,
(3) 1,3-diméthyl-7-benzyl-8-(3-amino-pipéridin-1-yl)-xanthine,
(4) 1,3-diméthyl-7-(3-méthyl-2-butén-1-yl)-8-[(trans-2-amino-cyclohexyl)amino]-xanthine,
(5) 1,3-diméthyl-7-(3-méthyl-2-butén-1-yl)-8-(3-amino-pipéridin-1-yl)-xanthine,
(6) 1,3-diméthyl-7-(3-méthyl-2-butén-1-yl)-8-(4-amino-pipéridin-1-yl)-xanthine,
(7) 1,3-diméthyl-7-(3-méthyl-2-butén-1-yl)-8-[(cis-2-amino-cyclohexyl)amino]xanthine,
(8) 1,3-diméthyl-7-(2-butin-1-yl)-8-(3-amino-pipéridin-1-yl)-xanthine,
(9) 1,3-diméthyl-7-[(1-cyclopentén-1-yl)méthyl]-8-(3-amino-pipéridin-1-yl)-xanthine,
(10) 1,3-diméthyl-7-(2-thiénylméthyl)-8-(3-amino-pipéridin-1-yl)-xanthine,
(11) 1,3-diméthyl-7-(3-fluorobenzyl)-8-(3-amino-pipéridin-1-yl)-xanthine,
(12) 1,3-diméthyl-7-(2-fluorobenzyl)-8-(3-amino-pipéridin-1-yl)-xanthine,
(13) 1,3-diméthyl-7-(4-fluorobenzyl)-8-(3-amino-pipéridin-1-yl)-xanthine,
(14) 1,3-diméthyl-7-(2-butén-1-yl)-8-(3-amino-pipéridin-1-yl)-xanthine,
(15) 1,3-bis-(cyclopropylméthyl)-7-benzyl-8-(3-amino-pipéridin-1-yl)-xanthine,
(16) (R)-1,3-diméthyl-7-(3-méthyl-2-butén-1yl)-8-(3-amino-pipéridin-1-yl)-xanthine,
(17) (S)-1,3-diméthyl-7-(3-méthyl-2-butén-1yl)-8-(3-amino-pipéridin-1-yl)-xanthine,
(18) 1,3-diméthyl-7-(3-méthyl-2-butén-1yl)-8-(3-amino-hexahydroazépin-1-yl)-xanthine,
(19) 1,3-diméthyl-7-(3-méthyl-2-butén-1yl)-8-(4-amino-hexahydroazépin-1-yl)-xanthine,
(20) chlorhydrate de 1,3-diméthyl-7-(3-méthyl-2-butén-1yl)-8-(cis-3-amino-cyclohexyl)-xanthine,
(21) 1,3-diméthyl-7-(3-méthyl-2-butén-1-yl)-8-(3-méthylamino-pipéridin-1-yl)-xanthine,
(22) 1-(2-phényléthyl)-3-méthyl-7-(3-méthyl-2-butén-1-yl)-8-(3-amino-pipéridin-1-yl)-xanthine,
(23) 1,3-diméthyl-7-(3-méthyl-2-butén-1-yl)-8-[N-(2-aminoéthyl)-méthylamino]-xanthine,
(24) 1-[2-(thiophén-2-yl)-éthyl]-3-méthyl-7-(3-méthyl-2-butén-1-yl)-8-(3-amino-pipéridin-1-yl)-xanthine,
(25) 1-[2-(thiophén-3-yl)-éthyl]-3-méthyl-7-(3-méthyl-2-butén-1-yl)-8-(3-amino-pipéridin-1-yl)-xanthine,
(26) 1-[2-(2-méthyl-phényl)-éthyl]-3-méthyl-7-(3-méthyl-2-butén-1-yl)-8-(3-amino-pipéridin-1-yl)-xanthine,
(27) 1-[2-(3-méthyl-phényl)-éthyl]-3-méthyl-7-(3-méthyl-2-butén-1-yl)-8-(3-amino-pipéridin-1-yl)-xanthine,
(28) 1-[2-(3-méthoxy-phényl)-éthyl]-3-méthyl-7-(3-méthyl-2-butén-1-yl)-8-(3-amino-pipéridin-1-yl)-xanthine,
(29) 1-((E)-2-phényl-vinyl)-3-méthyl-7-(3-méthyl-2-butén-1-yl)-8-(3-amino-pipéridin-1-yl)-xanthine,
(30) 1-(2-phényl-éthyl)-3-méthyl-7-(3-méthyl-2-butén-1-yl)-8-((S)-3-amino-pipéridin-1-yl)-xanthine,
(31) 1-(2-phényl-éthyl)-3-méthyl-7-(3-méthyl-2-butén-1-yl)-8-((R)-3-amino-pipéridin-1-yl)-xanthine,
(32) 1-[2-(2-méthoxy-phényl)-2-oxo-éthyl]-3-méthyl-7-(3-méthyl-2-butén-1-yl)-8-(3-amino-pipéridin-1-yl)-xanthine,
(33) 1-[2-(thiohén-3-yl)-2-oxo-éthyl]-3-méthyl-7-(3-méthyl-2-butén-1-yl)-8-(3-amino-pipéridin-1-yl)-xanthine,
(34) 1-(2-phényl-2-oxo-éthyl)-3-méthyl-7-(3-méthyl-2-butén-1-yl)-8-((S)-3-amino-pipéridin-1-yl)-xanthine,
(35) 1-(2-phényl-2-oxo-éthyl)-3-méthyl-7-(3-méthyl-2-butén-1-yl)-8-((R)-3-amino-pipéridin-1-yl)-xanthine,
(36) 1-[(isoquinolin-1-yl)méthyl]-3-méthyl-7-(3-méthyl-2-butén-1-yl)-8-((R)-3-amino-pipéridin-1-yl)-xanthine,
(37) 1-[(isoquinolin-1-yl)méthyl]-3-méthyl-7-(3-méthyl-2-butén-1-yl)-8-((S)-3-amino-pipéridin-1-yl)-xanthine et
(38) 1-[(1-naphtyl)méthyl]-3-méthyl-7-(3-méthyl-2-butén-1-yl)-8-(3-amino-pipéridin-1-yl)-xanthine,
et leurs sels ;
pour leur utilisation dans la thérapie combinée avec d'autres substances actives, par exemple, choisies dans le groupe des antidiabétiques, des hypolipidémiants, des composés augmentant le taux de HDL, des substances actives pour le traitement de l'obésité et des substances actives agissant sur l'hypertension.

14. Sels physiologiquement acceptables des composés de formule générale 1 selon au moins l'une des revendications 1 à 13 avec des acides ou des bases inorganiques ou organiques ;
pour leur utilisation dans la thérapie combinée avec d'autres substances actives, par exemple, choisies dans le groupe des antidiabétiques, des hypolipidémiants, des composés augmentant le taux de HDL, des substances actives pour le traitement de l'obésité et des substances actives agissant sur l'hypertension.

15. Composés de formule générale I, ou leurs sels physiologiquement acceptables pour leur utilisation selon au moins l'une des revendications 1 à 14, dans lesquels les autres substances actives sont choisies dans le groupe des antidiabétiques, des hypolipidémiants, des composés augmentant le taux de HDL, des substances actives pour le traitement de l'obésité et des substances actives agissant sur l'hypertension.

16. Médicaments contenant un composé de formule générale I selon au moins l'une des revendications 1 à 13 ou un sel physiologiquement acceptable selon la revendication 14, outre éventuellement un ou plusieurs véhicules et/ou diluants inertes ;
pour leur utilisation dans la thérapie combinée avec d'autres substances actives, par exemple, choisies dans le groupe des antidiabétiques, des hypolipidémiants, des composés augmentant le taux de HDL, des substances actives pour le traitement de l'obésité et des substances actives agissant sur l'hypertension.

17. Composé de formule générale I ou combinaison selon au moins l'une des revendications 1 à 14, pour la prévention ou le traitement de maladies ou d'états qui dépendent d'une activité accrue de DPP-IV ou qui peuvent être inhibés ou diminués par la réduction de l'activité de DPP-IV ; par exemple, pour le traitement du diabète sucré de type I ou de type II, des complications diabétiques, de l'insulinorésistance, des dyslipidémies, de l'athérosclérose, de l'arthrite, des adiposités, des allogreffes ou de l'ostéoporose provoquée par la calcitonine.

18. Procédé de production d'un médicament pour l'utilisation selon la revendication 16, **caractérisé en ce que**, de façon non chimique, un composé de formule générale I selon au moins l'une des revendications 1 à 14 est intégré dans un ou plusieurs véhicules et/ou diluants inertes.

19. Procédé de production de composés de formule générale I pour leur utilisation selon les revendications 1 à 14, **caractérisé en ce que**
a) pour la production de composés de formule générale I, dans laquelle R⁴ est l'un des radicaux mentionné dans la revendication 1, relié par un atome d'azote au squelette de la xanthine, on fait réagir
un composé de formule générale 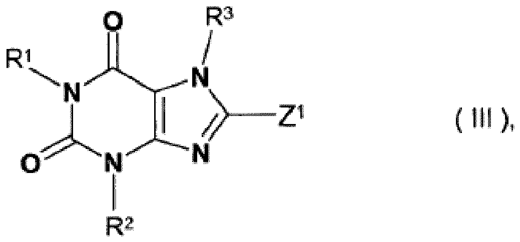 dans laquelle
R¹ à R³ sont tels que définis dans les revendications 1 à 14, et
Z¹ est un groupe partant tel qu'un atome d'halogène, un groupe hydroxy, mercapto, sulfinyle, sulfonyle ou sulfonyloxy substitué tel qu'un atome de chlore ou de brome, un groupe méthanesulfonyle ou méthanesulfonyloxy, avec un composé de formule générale
H-R^{4'} (IV),
dans laquelle
R^{4'} représente l'un des radicaux définis pour R⁴ dans les revendications 1 à 14, qui est relié par un atome d'azote au squelette de la xanthine de formule générale I
ou
b) pour la production de composés de formule générale I, dans laquelle R⁴ contient un groupe amino selon la définition de la revendication 1 ou un groupe alkylamino éventuellement substitué dans la partie alkyle, on déprotège
un composé de formule générale 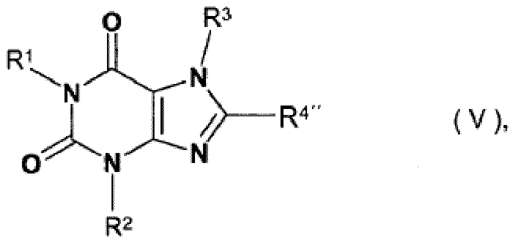
dans laquelle R¹, R² et R³ sont tels que définis dans les revendications 1 à 14
et
R^{4"} contient un groupe N-tert.-butyloxycarbonylamino ou un groupe N-tert-butyloxycarbonyl-N-alkylamino, dans lequel la partie alkyle du groupe N-tert.-butyloxycarbonyl-N-alkyl-amino peut être substituée comme défini dans les revendications 1 à 4,
ou
c) pour la production d'un composé de formule générale I, dans laquelle R² représente un atome d'hydrogène selon la définition de la revendication 1, on déprotège un composé de formule générale 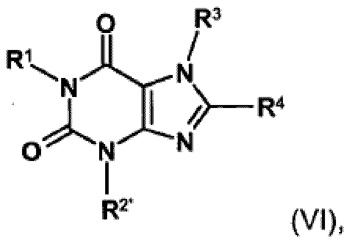
dans laquelle R¹, R³ et R⁴ sont tels que définis précédemment et R^{2'} représente un groupe protecteur tel qu'un groupe méthoxyméthyle, benzyloxyméthyle, méthoxyéthoxyméthyle ou 2-(triméthylsilyl)-éthoxyméthyle,
dans laquelle un composé de formule générale I ainsi obtenu qui contient un groupe amino, alkylamino ou imino peut être converti par acylation ou sulfonylation en un composé acyle ou sulfonyle de formule générale I correspondant,
un composé de formule générale I ainsi obtenu qui contient un groupe amino, alkylamino ou imino peut être converti par alkylation ou alkylation réductrice en un composé alkyle de formule générale I correspondant,
un composé de formule générale I ainsi obtenu qui contient un groupe nitro peut être converti par réduction en un composé amino correspondant,
un composé de formule générale I ainsi obtenu qui contient un groupe imino peut être converti par nitrosation et ensuite, réduction, en un composé N-amino-imino correspondant,
un composé de formule générale I ainsi obtenu qui contient un groupe alkyloxy en C₁ à C₃-carbonyle peut être converti par clivage d'ester en un composé carboxy correspondant,
un composé de formule générale I ainsi obtenu, dans lequel R¹ contient un groupe carbonyle, peut être converti par exemple par une réaction avec une hydroxylamine en un oxime de formule générale I correspondant,
un composé de formule générale I ainsi obtenu qui contient un groupe carboxy peut être converti par estérification en un ester de formule générale I correspondant, ou
un composé de formule générale I ainsi obtenu qui contient un groupe carboxy ou un groupe ester peut être converti par réaction avec une amine en un amide de formule générale I correspondant.

20. Médicament qui présente un composé de formule générale I selon au moins l'une des revendications 1 à 13 ou un sel physiologiquement acceptable selon la revendication 14 et une autre substance active choisie par exemple dans le groupe des antidiabétiques, outre éventuellement un ou plusieurs véhicules et/ou diluants inertes.

21. Médicament selon la revendication 20, dans lequel la substance active est choisie dans le groupe des antidiabétiques du groupe consistant en metformine, sulfonylurée, natéglinide, répaglinide, thiazolidinedione, agonistes PPAR-gamma, inhibiteur de l'alpha-glucosidase, antagonistes alpha 2, insuline et analogues de l'insuline, GLP-1 et analogues de GLP-1 et amyline.

22. Procédé de production d'un médicament selon la revendication 20, **caractérisé en ce que**, de façon non chimique, un composé de formule générale I selon au moins l'une des revendications 1 à 14 en combinaison avec une autre substance active est intégré dans un ou plusieurs véhicules et/ou diluants inertes.

23. Composé de formule générale I selon au moins l'une des revendications 1 à 14, pour l'utilisation selon la revendication 17, le composé de formule générale I selon au moins l'une des revendications 1 à 14 étant utilisé en combinaison avec une autre substance active, par exemple choisie dans le groupe des antidiabétiques, des hypolipidémiants, des composés augmentant le taux de HDL, des substances actives pour le traitement de l'obésité et des substances actives agissant sur l'hypertension.

24. Composés de formule générale I selon au moins l'une des revendications 1 à 13 ou sel physiologiquement acceptable selon la revendication 14, pour leur utilisation dans la thérapie combinée avec d'autres antidiabétiques.

25. Composés de formule générale I selon au moins l'une des revendications 1 à 13 ou sel physiologiquement acceptable selon la revendication 14, pour leur utilisation dans la thérapie combinée avec d'autres substances actives choisies dans le groupe des antidiabétiques consistant en metformine, sulfonylurée, natéglinide, répaglinide, thiazolidinedione, agonistes PPAR-gamma, inhibiteur de l'alpha-glucosidase, antagonistes alpha 2, insuline et analogues de l'insuline, GLP-1 et analogues de GLP-1 et amyline.

26. Composés de formule générale I selon au moins l'une des revendications 1 à 14, dans lesquels les groupes carboxy mentionnés dans la définition des radicaux cités précédemment peuvent être remplacés par un groupe pouvant être converti *in vivo* en un groupe carboxy ou par un groupe à charge négative dans des conditions physiologiques
et/ou
les groupes amino et imino mentionnés dans la définition des radicaux cités précédemment peuvent être substitués par un radical clivable *in vivo.*
